# EUROPEAN PATENT SPECIFICATION

(11) **EP 1 040 103 B1**
(45) Date of publication and mention of the grant of the patent: **14.03.2007**
(21) Application number: 98961383.1
(22) Date of filing: 17.12.1998
(51) Int. Cl.: C07D 295/12, A61K 31/16, A61K 31/33, A61K 31/66, C07C 233/62, C07D 213/20, C07D 213/61, C07D 213/84, C07D 213/85, C07F 9/44, C07F 9/54, C07F 9/6584, C07F 9/6568, C07F 9/655, C07F 9/53, C07D 313/08, C07D 407/12

(54) **ANILIDE DERIVATIVE, PRODUCTION AND USE THEREOF**
ANILIDDERIVATE, DEREN HERSTELLUNG UND VERWENDUNG
DERIVE D'ANILIDE, SA PREPARATION ET SON UTILISATION

(30) Priority: 19.12.1997 JP 35148197
(43) Date of publication of application: 04.10.2000
(73) Proprietor: Takeda Pharmaceutical Company Limited, Osaka-shi, Osaka 541-0045 (JP)
(72) Inventor: SHIRAISHI, Mitsuru, Amagasaki-shi, Hyogo 661-0002 (JP); KITAYOSHI, Takahito, Suita-shi, Osaka 565-0824 (JP); ARAMAKI, Yoshio Takeda Pharmaceutical Company Ltd, Osaka-shi, Osaka 532-8686 (JP); HONDA, Susumu Takeda Pharmaceutical Company Ltd, Ibaraki 300-4293 (JP); ODA, Tsuneo, Osaka 567-0895 (JP)
(74) Representative: Wright, Robert Gordon McRae
(86) International application number: PCT/JP1998/005707
(87) International publication number: WO 1999/032468

(56) References cited:
- PATENT ABSTRACTS OF JAPAN vol. 095, no. 004, 31 May 1995 & JP 07 025757 A (TEIJIN LTD), 27 January 1995 cited in the application
- PATENT ABSTRACTS OF JAPAN vol. 095, no. 004, 31 May 1995 & JP 07 025756 A (TEIJIN LTD), 27 January 1995 cited in the application

## Description

### Technical Field

The present invention relates to an anilide derivative or a salt thereof having antagonistic activity on MCP-1 (monocyte chemoattractant protein-1) receptor, production method and use thereof.

### Background Art

MCP-1 is known to be a monocyte chemotactic factor relating to inflammatory diseases, and belongs to CC chemokine sub-family. MCP-1 is found to express not only from monocyte but also from cardiac muscle cell, blood vessel endothelial cell, fibroblast, chondrocyte, smooth muscle cell, mesangial cell, aveolar cell, Tlymphocyte, macrophage, etc. in various pathosis (specifically, angiostenosis, arteriosclerosis, rheumatic arthritis, diabetic microangiopathy, granulomatous inflammation (tuberculosis, sarcoidosis, etc.), solid cancer, diastolic cardiomyopathy (chronic heart failure, etc.), glomerulonephritis, etc.), and MCP-1 deeply relate to crisis and progression these pathosis. Therefore, MCP-1 receptor antagonists are used as a medicament for the treatment and prophylaxis of these pathosis.

So far, there have been only a little reports on low molecule compounds having antagonistic activity on MCP-1 receptor. For example, it is disclosed that aryloxy-propanolamine derivatives being active as β-blocker show weak inhibitoryactivityonMCP-1 binding to its receptor in JP-A-25756/1995 and that phenylethanolamine derivatives having sympathetic activity and sympatholytic activity show weak inhibitory activity on MCP-1 binding to its receptor in JP-A-25757/1995.

On the other hand, phosphonic acid derivatives having osteogenesis activity is disclosed in JP-A-73476/1996 but there is no description on MCP-1 receptor antagonistic activity.

The present invention is to provide a new anilide derivative or a salt thereof having antagonistic activity on MCP-1 receptor and therapeutic and prophylactic effect on cardiac infarction, myocarditis, cardiomyopathy, chronic heart failure, restenosis after angioplasty, disorder after reperfusion in lung and heart, inflammatory diseases (e.g. arteriosclerosis, arteriosclerosis after heart transplantation, (chronic) rheumatic arthritis, nephritis, etc.), rejection after organ transplantation, fibroid lung, renal insufficiency, diabetic diseases (e.g. diabetes, diabetic nephropathy, diabetic complication, diabetic retinopathy, diabetic retinitis, diabetic microangiopathy, etc.), tumor (e.g. bladder cancer, breast carcinoma, cervical carcinoma, chronic lymphocytic leukemia, chronic myelocytic leukemia, colon carcinoma, multiple myeloma, malignant myeloma, prostatic cancer, lung cancer, stomach cancer, Hodgkin's disease, etc.), infectious diseases (e.g. tuberculosis, invasive staphylococcia, etc.), etc.; production method and use thereof.

### Disclosure of Invention

The present inventors diligently made extensive studies on compounds having MCP-1 receptor antagonistic activity and, as a result, they found that an anilide derivative of the following formula (I) or a salt thereof [hereinafter, referred to as Compound (I)] unexpectedly possesses potent MCP-1 receptor antagonistic activity and clinically desirable pharmaceutical effect. Based on the finding, the present invention was accomplished.

More specifically, the present invention relates to
(1) a compound of the formula: 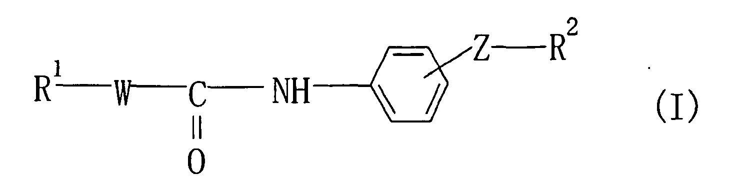 wherein R¹ is an optionally substituted 5- to 6-membered ring, W is a divalent group of the formula: 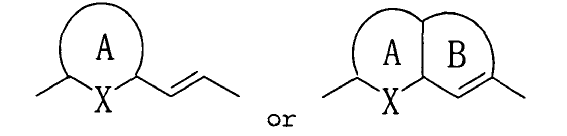 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring, Z is a methylene group, R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 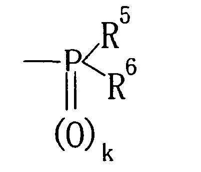 wherein k is 0 or 1, and when k is 0, a phosphorus atom may form a phosphonium; and R⁵ and R⁶ are independently an optionally substituted hydrocarbon group or an optionally substituted amino group, and R⁵ and R⁶ may bind to each other to form a cyclic group together with the adjacent phosphorus atom, or a salt thereof;
(2) a compound of the above (1), wherein R¹ is benzene, furan, thiophene, pyridine, cyclopentane, cyclohexane, pyrrolidine, piperidine, piperazine, morpholine, thiomorpholine or tetrahydropyran, each of which may be substituted;
(3) a compound of the above (1), wherein R¹ is an optionally substituted benzene;
(4) a compound of the above (1), wherein the ring A is furan, thiophene, pyrrole, pyridine or benzene, each of which may be substituted;
(5) a compound of the above (1), wherein the ring A is an optionally substituted benzene;
(6) a compound of the above (1), wherein W is a group of the formula: 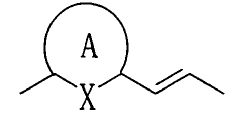 wherein each symbol is as defined in the above (1);
(7) a compound of the above (1), wherein W is a group of the formula: 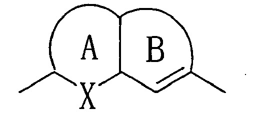 wherein each symbol is as defined in the above (1);
(8) a compound of the above (7), wherein the ring B is a 5- to 7-membered ring group of the formula: 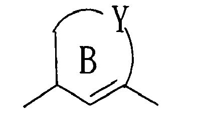 wherein Y is -Y'-(CH₂)ₘ- (Y' is -S-, -O-, -NH- or -CH₂-, and m is an integer of 0-2), -CH=CH- or -N=CH-, which may have a substituent at any possible position;
(9) a compound of the above (8), wherein Y is -Y'-(CH₂)₂-(Y' is -S-, -O-, -NH- or -CH₂-);
(10) a compound of the above (8), wherein Y is -(CH₂)₂-, - (CH₂)₃- or -O-(CH₂)₂-;
(11) a compound of the above (10), wherein the ring A is an optionally substituted benzene;
(12) a compound of the above (1), wherein Z is substituted at para position of the benzene ring;
(13) acompoundof the above (1), wherein R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, or (3) a group of the formula: 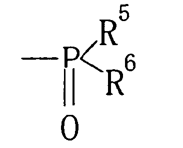 wherein R⁵ and R⁶ are independently an optionally substituted hydrocarbon group, and R⁵ and R⁶ may bind to each other to form a cyclic group together with the adjacent phosphorus atom;
(14) a compound of the above (1) of the formula: 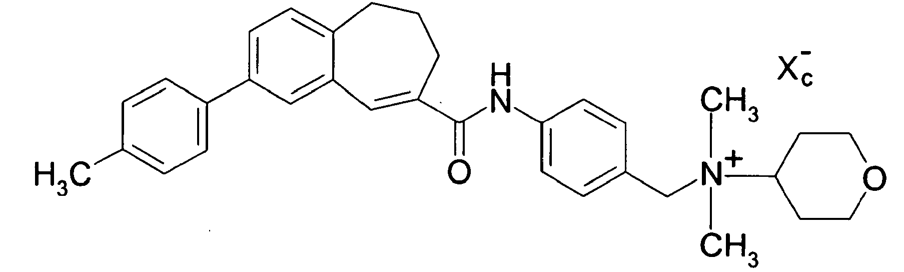 wherein X_{c}⁻ is an anion;
(15) a compound of the above (14), wherein X_{c} is a halogen atom;
(16) a compound of the above (1) selected from the class consisting of
   N-methyl-N-[4-[[[2-(4-methylphenyl)-6,7-dihydro-5H-benz ocyclohepten-8-yl]carbonyl]amino]benzyl]-piperidinium iodide,
   N-methyl-N-[4-[[[7-(4-methylphenyl)-2,3-dihydro-1-benzo xepin-4-yl]carbonyl]amino]benzyl]piperidinium iodide, N-[4-[N-methyl-N-(tetrahydropyran-4-yl)aminomethyl]-phe nyl]-7-(4-methylphenyl)-2,3-dihydro-1-benzoxepine-4-car boxmide,
   N-[4-[N-methyl-N-(tetrahydropyran-4-yl)aminomethyl]-phe nyl]-7-(4-morpholinophenyl)-2,3-dihydro-1-benzoxepine-4 -carboxmide,
   7-(4-ethoxyphenyl)-N-[4-[N-methyl-N-(tetrahydropyran-4-yl)aminomethyl]phenyl]-2,3-dihydro-1-benzoxepine-4-carb oxmide,
   N,N-dimethyl-N-[4-[[[2-(4-methylphenyl)-6,7-dihydro-5H-benzocyclohepten-8-yl]carbonyl]amino]benzyl]-N-(tetrahy dropyran-4-yl)ammonium iodide and
   N-methyl-N-[4-[[[7-(4-methylphenyl)-3,4-dihydro-naphtha len-2-yl]carbonyl]amino]benzyl]piperidinium iodide, or a salt thereof;
(17) a method for producing a compound of the above (1) of the formula: 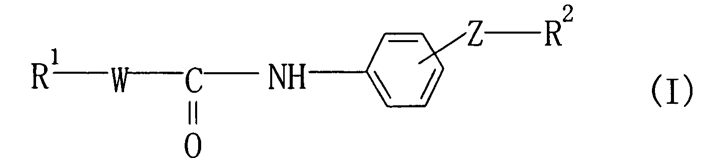 wherein each symbol is as defined above (1) or a salt thereof, which comprises subjecting a compound of the formula: R¹-W-COOH (II)
   wherein each symbol is as defined above (1), a salt or a reactive derivative thereof to condensation reaction with a compound of.the formula: 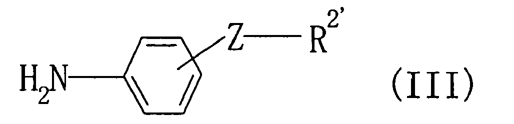 wherein Z is as defined above (1) and R²' is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 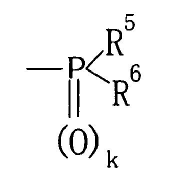 wherein k is 0 or 1, and when k is 0, a phosphorus atom may form a phosphonium; and R⁵ and R⁶ are independently an optionally substituted hydrocarbon group or an optionally substituted amino group, and R⁵ and R⁶ may bind to each other to form a cyclic group together with the adjacent phosphorus atom, the above groups (1) - (3) being optionally protected, or a salt thereof, and, if desired, subjecting the obtained product to deprotection, oxidation, reduction and/or ammoniumation;
(18) A method of the above (17) wherein the compound of the formula R¹-W-COOH is
   3-(4-methylphenyl)-8,9-dihydro-7H-benzocyclo-heptene-6-carboxylic acid or a salt thereof;
(19) a pharmaceutical composition comprising a compound of the above (1) or a salt thereof;
(20) acompositionof the above (19), which is for antagonizing MCP-1 receptor;
(21) a composition of the above (19), which is for the treatment or prophylaxis of cardiac infarction or myocarditis;
(22) a pharmaceutical composition for antagonizing MCP-1 receptor (or a pharmaceutical composition for inhibiting binding of MCP-1 (a ligand) to MCP-1 receptor or a pharmaceutical composition for antagonizing binding of MCP-1 to its receptor), which comprises a compound of the formula: 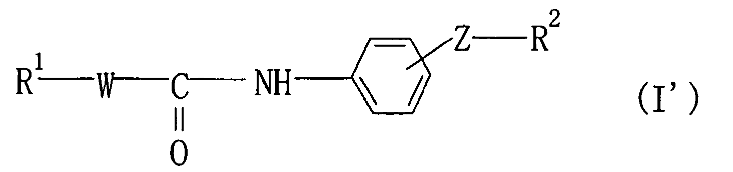 wherein R¹ is an optionally substituted 5- to 6-membered ring, W is a divalent group of the formula: 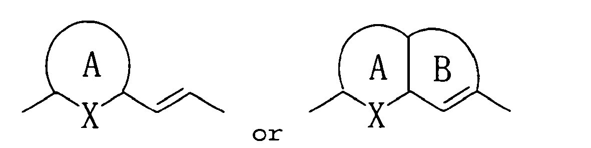 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring, Z is a methylene group, R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 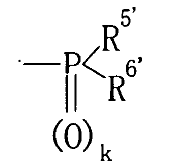 wherein k is 0 or 1, and when k is 0, a phosphorus atom may form a phosphonium; and R⁵' and R⁶' are independently an optionally substituted hydrocarbon group, an optionally substituted hydroxy group or an optionally substituted amino group, and R⁵' and R⁶' may bind to each other to form a cyclic group together with the adjacent phosphorus atom, or a salt thereof ;
(23) use of a compound of the formula: 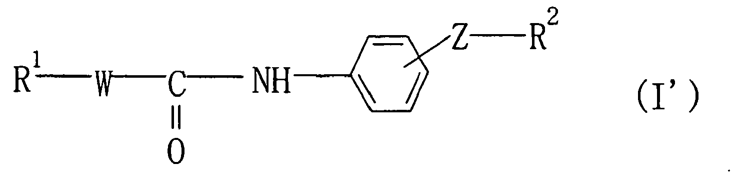 wherein R¹ is an optionally substituted 5- to 6-membered ring;
   W is a divalent group of the formula: 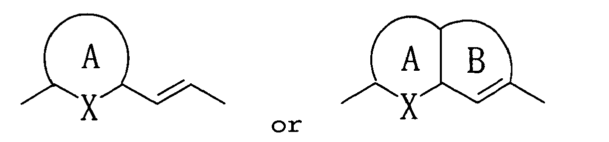 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring; Z is a methylene group; R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 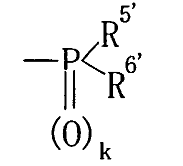 wherein k is 0 or 1, and when k is 0, a phosphorus atom may form a phosphonium; and R⁵' and R⁶' are independently an optionally substituted hydrocarbon group, an optionally substituted hydroxy group or an optionally substituted amino group, and R⁵' and R⁶' may bind to each other to form a cyclic group together with the adjacent phosphorus atom, or a salt thereof, for the manufacture of a medicament for the treatment or prophylaxis of inflammatory disease, cardiac infarction or myocarditis;
(24) use of a compound of the above (1) or a salt thereof for the manufacture of a medicament for the treatment or prophylaxis of inflammatory disease, cardiac infarction or myocarditis.

In the above formula (I), examples of the "5- to 6-membered ring" of the "optionally substituted 5- to 6-membered ring" represented by R¹ include a 6-membered aromatic hydrocarbon such as benzene, etc. ; a 5- to 6-membered aliphatic hydrocarbon such as cyclopentane, cyclohexane, cyclopentene, cyclohexene, cyclopentanediene, cyclohexanediene, etc.; 5- to 6-membered aromatic heterocyclic ring containing 1 to 4 hetero-atoms consisting of 1 to 2 kinds of hetero-atoms selected from oxygen atom, sulfur atom and nitrogen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; 5- to 6-membered non-aromatic heterocyclic ring containing 1 to 4 hetero-atoms consisting of 1 to 2 kinds of hetero-atoms selected from oxygen atom, sulfur atom and nitrogen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; etc. Among others, benzene, furan, thiophene, pyridine, cyclopentane, cyclohexane, pyrrolidine, piperidine, piperazine, morpholine, thiomorpholine, tetrahydropyran (preferably, 6-membered ring), etc. are preferable, and in particular, benzene is preferable.

Example of the "substituents" which the "5- to 6-membered ring" in the "optionally substituted 5- to 6-membered ring" represented by R¹ may have include halogen atom, nitro, cyano, an optionally substituted alkyl, an optionally substituted cycloalkyl, an optionally substituted hydroxy group, an optionally substituted thiol group wherein a sulfur atom may be optionally oxidized to form a sulfinyl group or a sulfonyl group, an optionally substituted amino group, an optionally substituted acyl, an optionally esterified carboxyl group, an optionally substituted aromatic group, etc.

Examples of the halogen as the substituents for R¹ include fluorine, chlorine, bromine, iodine, etc. Among others, fluorine and chlorine are preferable.

Examples of the alkyl in the optionally substituted alkyl as the substituents for R¹ include a straight or branched C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl.

Examples of the substituents in the optionally substituted alkyl include halogen (e.g. fluorine, chlorine, bromine, iodine, etc.), nitro, cyano, hydroxy group, thiol group,amino group,carboxylgroup,an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the cycloalkyl in the optionally substituted cycloalkyl as the substituents for R¹ include C₃₋₇ cycloalkyl, etc. such as cyclopropyl, cyclobutyl, cyclopentyl, cyclohexyl, cycloheptyl, etc.

Examples of the substituents in the optionally substituted cycloalkyl include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted hydroxy group as the substituents for R¹ include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted alkenyl (e.g. C₂₋₁₀ alkenyl such as allyl, crotyl, 2-pentenyl, 3-hexenyl, etc., preferably lower (C₂₋₆) alkenyl, etc.);
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.);
(5) an optionally substituted aralkyl (e.g. phenyl-C₁₋₄ alkyl (e.g. benzyl, phenethyl, etc.), etc.);
(6) an optionally substituted acyl (e.g. C₂₋₄ alkanoyl (e.g. acetyl, propionyl, butyryl, isobutyryl, etc.), C₁₋₄ alkylsulfonyl (e.g. methanesulfonyl, ethanesulfonyl, etc.), etc.);
(7) an optionally substituted aryl (e.g. phenyl, naphthyl, etc.); etc.

Examples of the substituents which the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl may have include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted thiol group as the substituents for R¹ are similar to the above-described substituents in the optionally substituted hydroxy group as the substituents for R¹, and among others,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted aralkyl (e.g. phenyl-C₁₋₄ alkyl (e.g. benzyl, phenethyl, etc.), etc.);
(4) an optionally substituted aryl (e.g. phenyl, naphthyl, etc.); etc. are preferable.

Examples of the substituents which the above-mentioned (1) optionally substituted alkyl, (2) optionally substituted cycloalkyl, (3) optionally substituted aralkyl and (4) optionally substituted aryl may have include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the substituents in the optionally substituted amino group as the substituents for R¹ are similar to the above-described substituents in the optionally substituted hydroxy group as the substituents for R¹, and examples of the optionally substituted amino group as the substituents for R¹ include an amino group which may have one to two substituents selected from the above-described substituents in the optionally substituted hydroxy group as the substituents for R¹, etc. Among others, as the substituents in the optionally substituted amino group as the substituents for R¹,
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇cycloalkyl, etc. such as cyclopropyl, cyclobutyl, cyclopentyl, cyclohexyl, cycloheptyl, etc.);
(3) an optionally substituted alkenyl (e.g. C₂₋₁₀ alkenyl such as allyl, crotyl, 2-pentenyl, 3-hexenyl, etc., preferably lower (C₂₋₆) alkenyl, etc.);
(4) an optionally substituted cycloalkenyl (e.g. C₃₋₇ cycloalkenyl, etc. such as2-cyclopentenyl,2-cyclohexenyl, 2-cyclopentenylmethyl, 2-cyclohexenylmethyl, etc.);
(5) an optionally substituted acyl (e.g. C₂₋₄ alkanoyl (e.g. acetyl, propionyl, butyryl, isobutyryl, etc.), C₁₋₄ alkylsulfonyl (e.g. methanesulfonyl, ethanesulfonyl, etc.), etc.) ;
(6) an optionally substituted aryl (e.g. phenyl, naphthyl, etc.); etc. are preferable.

Examples of the substituents, which each of the above-described (1) optionally substituted alkyl, (2) optionally substituted cycloalkyl, (3) optionally substituted alkenyl, (4) optionally substituted cycloalkenyl, (5) optionally substituted acyl and (6) optionally substituted aryl may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

The substituents in the optionally substituted amino group as the substituents for R¹ may bind to each other to form a cyclic amino group (e.g. 5- to 6-membered cyclic amino, etc. such as tetrahydropyrrole, piperazine, piperidine, morpholine, thiomorpholine, pyrrole, imidazole, etc.). Said cyclic amino group may have a substituent, and examples of the substituents include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally substituted acyl as the substituents for R¹ include a carbonyl group or a sulfonyl group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇cycloalkyl, etc. such as cyclopropyl, cyclobutyl, cyclopentyl, cyclohexyl, cycloheptyl, etc.);
(4) an optionally substituted alkenyl (e.g. C₂₋₁₀ alkenyl such as allyl, crotyl, 2-pentenyl,3-hexenyl, etc., preferably lower (C₂₋₆) alkenyl, etc.);
(5) an optionally substituted cycloalkenyl (e.g. C₃₋₇ cycloalkenyl,etc.such as2-cyclopentenyl,2-cyclohexenyl, 2-cyclopentenylmethyl, 2-cyclohexenylmethyl, etc.);
(6) an optionally substituted 5- to 6-membered monocyclic aromatic group (e.g. phenyl, pyridyl, etc.); etc.

Examples of the acyl include acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.

Examples of the substituents, which the above-mentioned (2) optionallysubstitutedalkyl, (3) optionally substituted cycloalkyl, (4) optionally substituted alkenyl, (5) optionally substituted cycloalkenyl and (6) optionally substituted 5- to 6-membered monocyclic aromatic group may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally esterified carboxyl group as the substituents for R¹ include a carbonyloxy group binding to
(1) hydrogen;
(2) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(3) an optionally substituted cycloalkyl (e.g. C₃₋₇ cycloalkyl, etc. such as cyclopropyl, cyclobutyl, cyclopentyl, cyclohexyl, cycloheptyl, etc.);
(4) an optionally substituted alkenyl (e.g. C₂₋₁₀ alkenyl such as allyl, crotyl, 2-pentenyl,3-hexenyl, etc., preferably lower (C₂₋₆) alkenyl, etc.);
(5) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.);
(6) an optionally substituted aryl (e.g. phenyl, naphthyl, etc.); etc., and preferably carboxyl, lower (C₁₋₆) alkoxycarbonyl, aryloxycarbonyl (e.g. methoxycarbonyl, ethoxycarbonyl, propoxycarbonyl, phenoxycarbonyl, naphthoxycarbonyl, etc.), etc.

Examples of the substituents, which the above-mentioned (2) optionallysubstitutedalkyl, (3) optionally substituted cycloalkyl, (4) optionally substituted alkenyl, (5) optionally substituted cycloalkenyl and (6) optionally substituted aryl may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the aromatic group in the optionally substituted aromatic group as the substituents for R¹ include 5- to 6-membered homocyclic or heterocyclic ring aromatic ring, etc. suchasphenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, etc.

Examples of the substituents for these aromatic group include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

The number of the above-mentioned substituents for R¹ is 1-4 (preferably 1-2) and they may be same or different and present at any possible position on the ring represented by R¹. When two or more substituents are present on the 5- to 6-membered ring in the "an optionally substituted 5- to 6-membered ring" represented by R¹, two substituents among them may bind to each other to form a lower (C₁₋₆) alkylene (e.g. trimethylene, tetramethylene, etc.), a lower (C₁₋₆) alkyleneoxy (e.g. -CH₂-O-CH₂- , -O-CH₂-CH₂- , etc.), a lower (C₁₋₆) alkylenedioxy (e.g. -O-CH₂-O-, -O-CH₂-CH₂-O-, etc.), a lower (C₂₋₆) alkenylene (e.g. -CH₂-CH=CH-, -CH₂-CH₂-CH=CH-, -CH₂-CH=CH-CH₂- , etc.), a lower (C₄₋₆) alkadienylene (e.g. -CH=CH-CH=CH-, etc.), etc.

Preferred examples of the "substituents", which the "5- to 6-membered ring" in the "an optionally substituted 5- to 6-membered ring" represented by R¹ may have, include an optionally halogenated lower (C₁-₄) alkyl (e.g. methyl, ethyl, t-butyl, trifluoromethyl, etc.), an optionally halogenated lower (C₁₋₄) alkoxy (e.g. methoxy, ethoxy, t-butoxy, trifluoromethoxy, etc.), halogen (e.g. fluorine, chlorine, etc.), nitro, cyano, an amino group optionally substituted with 1-2 lower (C₁₋₄) alkyl groups (e.g. amino, methylamino, dimethylamino, etc.), 5- to 6-membered cyclic amino (e.g. 1-pyrrolidinyl, 1-piperazinyl, 1-piperidinyl, 4-morpholino, 4-thiomorpholin, 1-imidazolyl, 4-tetrahydropyranyl, etc.), etc., and when R¹ is a benzene, the "substituent" is preferably present at para position.

In the above formula (I), examples of the "5- to 6-membered aromatic ring" in the "optionally substituted 5- to 6-membered aromatic ring" represented by A include 6-membered aromatic hydrocarbon such as benzene, etc.; 5-to 6-membered aromatic heterocyclic ring containing 1 to 3 hetero-atoms consisting of 1 to 2 kinds of hetero-atoms selected from oxygen atom, sulfur atom and nitrogen atom such as furan, thiophene, pyrrole, imidazole, pyrazole, thiazole, oxazole, isothiazole, isoxazole, pyridine, pyrazine, pyrimidine, pyridazine, triazole, etc.; etc. Among others, benzene, furan, thiophene, pyridine (preferably, 6-membered ring) etc. are preferable, and in particular benzene is preferable.

Examples of the "substituents", which the "5- to 6-membered aromatic ring" in the "optionally substituted 5- to 6-membered aromatic ring" represented by A may have, are similar to the "substituents" which the "5- to 6-membered ring" in the "optionally substituted 5- to 6-membered ring" represented by R¹ may have. The number of said substituents for the ring A is 1-4 (preferably 1-2), and they may be same or different and present at any possible position (e.g. the position of the group X and the other positions) on the ring represented by A.

In the above formula (I), a group of the formula: 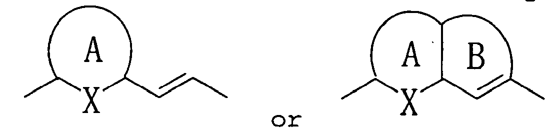 represented by W binds to adjacent groups in the following manner:

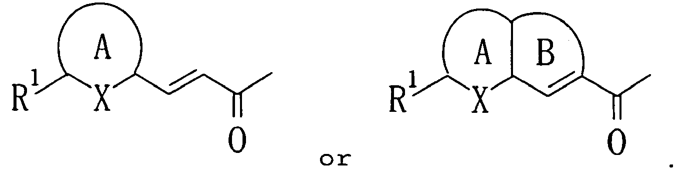

In the above formula (I), examples of the "5- to 7-membered ring" in the "optionally substituted 5- to 7-membered ring" represented by B include a 5- to 7-membered ring group of the formula: 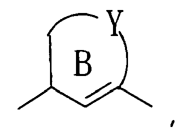 which may have a substituent at any possible position, etc.

In the above formula, the divalent group represented by Y may be any divalent group as far as the ring B forms an optionally substituted 5- to 7-membered ring, and preferred examples of the divalent groups include
(1) - (CH₂)ₐ₁-O- (CH₂)ₐ₂- (a₁ and a₂ are same or different and 0, 1 or 2, provided that the sum of a₁ and a₂ is 2 or less), -O-(CH=CH)-, -(CH=CH)-O-;
(2) -(CH₂)_{b1}-S-(CH₂)_{b2}- (b₁ and b₂ are same or different and 0, 1 or 2, provided that the sum of b₁ and b₂ is 2 or less), -S-(CH=CH)-, -(CH=CH)-S-;
(3) -(CH₂)_{d1}- (d₁ is 1, 2 or 3), -CH₂-(CH=CH)-, -(CH=CH)-CH₂-, -CH=CH-;
(4) -(CH₂)ₑ₁-NH-(CH₂)ₑ₂- (e₁ and e₂ are same or different and 0, 1 or 2, provided that the sum of e₁ and e₂ is 2 or less), -NH-(CH=CH)-, (CH=CH)-NH-, -(CH₂)ₑ₆-(N=CH)-(CH₂)ₑ₇-, -(CH₂)ₑ₇-(CH=N)-(CH₂)ₑ₆- (one of e₆ and e₇ is 0, and the other is 1), -(CH₂)ₑ₈-(N=N)-(CH₂)ₑ₉- (one of e₈ and e₉ is 0, and the other is 1) ; etc. More preferred examples of the divalent groups include -O-, -O-CH₂-, -O-CH₂-CH₂-, -O-CH=CH-, -S-, -S-CH₂-, -S-CH₂-CH₂-, -S-CH=CH-, -CH₂-, -(CH₂)₂-, -(CH₂)₃-, -CH=CH-, -CH=CH-CH₂-, -CH₂-CH=CH-, -NH-, -N=CH-, -CH=N-, -N=N- (in which each of the above formulas represent that it binds to the ring A through its left chemical bond), etc.

The divalent group may have a substituent. Examples of the substituent include those for the "5- to 6-membered ring" in the "optionally substituted 5- to 6-membered ring" represented by R¹ and an oxo group, etc. Among others, a lower (C₁₋₃) alkyl (e.g. methyl, ethyl, propyl, etc.), a phenyl group, an oxo group, a hydroxy group, etc. are preferable. In addition, the divalent group may be -O-C(O)-(in which each of the above formulas represent that it binds to the ring A through its left chemical bond), etc. The number of the substituents are preferably 1 to 4 (preferably, 1-2), and they may be same or different and bind to the divalent group at any possible position.

As the divalent group represented by Y, a group of the formula: -Y'-(CH₂)ₘ- (Y' is -S-, -O-, -NH- or -CH₂-, and m is an integer of 0-2), -CH=CH-, -N=CH-, -(CH₂)ₘ-Y'-(Y' is -S-, -O-, -NH- or -CH₂-, and m is an integer of 0-2), -CH=N- (in which each of the above formulas represent that it binds to the ring A through its left chemical bond), etc. is preferable. Among others, a group of the formula: -Y'-(CH₂)ₘ- (Y' is -S-, -O-, -NH- or -CH₂-, and m is an integer of 0-2), -CH=CH-, -N=CH- (in which each of the above formulas represent that it binds to the ring A through its left chemical bond), etc. is preferable. In particular, Y is preferably a group of the formula: -Y'-(CH₂)₂- (Y' is -S-, -O-, -NH- or -CH₂- (preferably -S-, -O- or -CH₂- more preferably -O- or -CH₂-)) in which the formula binds to the ring A through its left chemical bond, etc.; and the ring B is preferably a 7-membered ring. As the divalent group represented by Y, a group of the formula: -(CH₂)₂-, -(CH₂)₃- or -O-(CH₂)₂- is preferable.

Examples of the "substituents", which the "5- to 7-membered ring" in the "optionally substituted 5- to 7-membered ring" represented by B may have, include those for the "5- to 6-membered ring" in the "optionally substituted 5- to 6-membered ring" represented by R¹ and an oxo group, etc. The number of the substituents are preferably 1 to 4 (preferably, 1-2), and they may be same or different and bind to the divalent group at any possible position.

In a group of the formula: 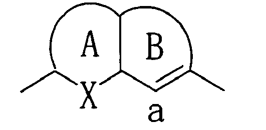 represented by W, a carbon atom at the position a is preferably unsubstituted.

In the above formula (I), the divalent group represented by Z is methylene. The group Z may be bound to any possible position of the benzene ring, and preferably to para position of the benzene ring.

In the above-mentioned formula (I), examples of the "amino group" in the "optionally substituted amino group in which a nitrogen atom may form a quaternary ammonium" represented by R² include an amino group which may have 1-2 substituents, an amino group having 3 substituents wherein the nitrogen atom forms a quaternary ammonium, etc. When the number of the substituents on the nitrogen atom is 2 or more, these substituents may be same or different. When the total number of the substituents and hydrogen atoms on the nitrogen atom is 3, the "amino group" represented by R² may be any type of an amino group represented by the formula: -N⁺R₃, -N⁺R₂R' or -N⁺ RR'R" (R, R' and R" are independently a hydrogen atom or a substituent). Examples of the counter anion of the amino group wherein the nitrogen atom forms a quaternary ammonium include an anion of a halogen atom (e.g. Cl⁻, Br⁻, I⁻, etc.), etc., and also an anion derived from an inorganic acid such as hydrochloric acid, hydrobromic acid, nitric acid, sulfuric acid, phosphoric acid, etc.; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; an anion derived from an acidic amino acid such as aspartic acid, glutamic acid, etc.; etc. Among others, Cl⁻, Br⁻, I⁻, etc. are preferable.

Examples of the substituents for said amino group include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₈cycloalkyl, etc. such as cyclopropyl, cyclobutyl, cyclopentyl, cyclohexyl, cycloheptyl, cyclooctyl, etc.), provided that
(2-1) said cycloalkyl may contain one hetero-atom selected from a sulfur atom, an oxygen atom and a nitrogen atom to form oxirane, thiorane, aziridine, tetrahydrofuran, tetrahydrothiophene, pyrrolidine, tetrahydropyran, tetrahydrothiopyran, tetrahydrothiopyran 1-oxide, piperidine, etc. (preferably, 6-membered ring such as tetrahydropyran, tetrahydrothiopyran, piperidine, etc.) and these groups preferably bind to the amino group at their 3- or 4-position (preferably, 4-position), that
(2-2) said cycloalkyl may be fused with a benzene ring to form indane, tetrahydronaphthalene, etc. (preferably, indane, etc.), and that
(2-3) said cycloalkyl may have a bridging comprising a straight chain constituted by 1-2 carbon atoms to form a bridged hydrocarbon residue such as bicyclo[2.2.1]heptyl, bicyclo[2.2.2]octyl, bicyclo[3.2.1]octyl, bicyclo [3.2.2]nonyl, etc., preferably, a cyclohexyl group, etc. having a bridging comprising a straight chain constituted by 1-2 carbon atoms, and more preferably bicyclo [2.2.1] heptyl, etc.;
(3) an optionally substituted alkenyl (e.g. C₂₋₁₀ alkenyl such as allyl, crotyl, 2-pentenyl,3-hexenyl, etc., preferably lower (C₂₋₆) alkenyl, etc.) ;
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.);
(5) an optionally substituted aralkyl (e.g. phenyl-C₁₋₄ alkyl (e.g. benzyl, phenethyl, etc.), etc.);
(6) an optionally substituted acyl (e.g. C₂₋₄alkanoyl (e.g. acetyl, propionyl, butyryl, isobutyryl, etc.), C₁₋₄ alkylsulfonyl (e.g. methanesulfonyl, ethanesulfonyl, etc.), etc.);
(7) an optionally substituted aryl (e.g. phenyl, naphthyl, etc.);
(8) an optionally substituted heterocyclic ring group (e.g. 5- to 6-membered aromatic heterocyclic ring containing 1 to 4 hetero-atoms consisting of 1 to 2 kinds of hetero-atoms selected from oxygen atom, sulfur atom and nitrogen atom such as furan, thiophene, pyrrole, imidazole, pyrazole, thiazole, oxazole, isothiazole, isoxazole, tetrazole, pyridine, pyrazine, pyrimidine, pyridazine, triazole, etc. ; 5- to 6-membered non-aromatic heterocyclic ring containing 1 to 4 hetero-atoms consisting of 1 to 2 kinds of hetero-atoms selected from oxygen atom, sulfur atom and nitrogen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc. ; etc.; preferably 5- to 6-membered non-aromatic heterocyclic ring, etc.; more preferably 5- to 6-membered non-aromatic heterocyclic ring containing one hetero-atom, etc. such as tetrahydrofuran, piperidine, tetrahydropyran, tetrahydrothiopyran, etc.); etc.

Examples of the substituents, which the above-mentioned (1) optionally substituted alkyl, (2) optionally substituted cycloalkyl, (3) optionally substituted alkenyl, (4) optionally substituted cycloalkenyl, (5) optionally substituted aralkyl, (6) optionally substituted acyl, (7) optionally substituted aryl and (8) optionally substituted heterocyclic ring group may have, include halogen (e.g. fluorine, chlorine, bromine, iodine, etc.), an optionally halogenated lower (C₁₋₄) alkyl, an optionally halogenated C₁₋₄ alkoxy (e.g. methoxy, ethoxy, trifluoromethoxy, trifluoroethoxy, etc.), C₁₋₄ alkylenedioxy (e.g. -O-CH₂-O-, -O-CH₂-CH₂-O-, etc.), C₂₋₄ alkanoyl (e.g. acetyl, propionyl, etc.), C₁₋₄ alkylsulfonyl (e.g. methanesulfonyl, ethanesulfonyl, etc.), phenyl-lower (C₁₋₄) alkyl, C₃₋₇ cycloalkyl, cyano, nitro, hydroxy group, thiol group, amino group, carboxyl group, lower (C₁₋₄) alkoxy-carbonyl (preferably, halogen, an optionally halogenated lower (C₁₋₄) alkyl, an optionally halogenated lower (C₁₋₄) alkoxy, phenyl-lower (C₁₋₄) alkyl, C₃₋₇ cycloalkyl, cyano, hydroxy group, etc.), etc., and the number of the substituents are preferably 1 to 3.

In the above formula (I), preferred examples of the "optionally substituted amino group in which a nitrogen atom may form a quaternary ammonium" represented by R² include an amino group which may have 1-3 substituents selected from
(1) a straight or branched lower (C₁₋₆) alkyl which may have 1 to 3 substituents selected from halogen, cyano, hydroxy group or C₃₋₇ cycloalkyl;
(2) a C₅₋₈ cycloalkyl which may have 1 to 3 substituents selected from halogen, an optionally halogenated lower (C₁₋₄) alkyl or phenyl-lower (C₁₋₄) alkyl, which may contain one hetero-atom selected from a sulfur atom, an oxygen atom and a nitrogen atom, which may be fused with a benzene ring, and which may have a bridging comprising a straight chain constituted by 1-2 carbon atoms (e.g. cyclopentyl, cyclohexyl, cycloheptyl, cyclooctyl, tetrahydropyranyl, tetrahydrothiapyranyl, piperidinyl, indanyl, tetrahydronaphthalenyl, bicyclo[2.2.1]heptyl, etc., each of which may be substituted);
(3) a phenyl-lower (C₁₋₄) alkyl which may have 1 to 3 substituents selected from halogen, an optionally halogenated lower (C₁₋₄) alkyl or an optionally halogenated lower (C₁₋₄) alkoxy;
(4) a phenyl which may have 1 to 3 substituents selected from halogen, an optionally halogenated lower (C₁₋₄) alkyl or an optionally halogenated lower (C₁₋₄) alkoxy; and
(5) a 5- to 6-membered aromatic heterocyclic ring (e.g. furan, thiophene, pyrrole, pyridine, etc.) which may have 1 to 3 substituents selected from halogen, an optionally halogenated lower (C₁₋₄) alkyl, an optionally halogenated lower (C₁₋₄) alkoxy, an optionally halogenated lower (C₁₋₄) alkoxy-lower (C₁₋₄) alkoxy, phenyl-lower (C₁₋₄) alkyl, cyano or hydroxy group.

In the above formula (I), examples of the "nitrogen-containing heterocyclic ring" in the "optionally substituted nitrogen-containing heterocyclic ring group which may contain a sulfur atom or an oxygen atom as ring constituting atoms and wherein a nitrogen atom may form a quaternary ammonium" include a 5- to 6-membered aromatic heterocyclic ring which may contain 1 to 3 hetero-atoms consisting of 1 to 2 kinds of hetero-atoms selected from an oxygen atom, a sulfur atom and a nitrogen atom other than one nitrogen atom such as pyrrole, imidazole, pyrazole, thiazole, oxazole, isothiazole, isoxazole, tetrazole, pyridine, pyrazine, pyrimidine, pyridazine, triazole, etc.; 5-8 membered non-aromatic heterocyclic ring which may contain 1 to 3 hetero-atoms consisting of 1 to 2 kinds of hetero-atoms selected from an oxygen atom, a sulfur atom and a nitrogen atom other than one nitrogen atom such as pyrrolidine, pyrroline, imidazolidine, imidazoline, pyrazolidine, pyrazoline, piperidine, piperazine, oxazine, oxadiazine, thiazine, thiadiazine, morpholine, thio-morpholine, azacycloheptane, azacyclooctane (azocane), etc.; etc. These nitrogen-containing heterocyclic rings may have a bridging comprising a straight chain constituted by 1-2 carbon atoms to form a bridged nitrogen-containing heterocyclic ring azabicyclo[2.2.1]heptane, azabicyclo[2.2.2]octane (quinuclidine), etc. (preferably, piperidine having a bridging comprising a straight chain constituted by 1-2 carbon atoms, etc.).

Among the above-exemplified nitrogen-containing heterocyclic rings, pyridine, imidazole, pyrrolidine, piperidine, piperazine, morpholine, thiomorpholine, azabicyclo[2.2.2]octane (preferably, a 6-membered ring) are preferable.

The nitrogen atom of said "nitrogen-containing heterocyclic ring" may form a quaternary ammonium or may be oxidized. When the nitrogen atom of said "nitrogen-containing heterocyclic ring" forms a quaternary ammonium, examples of the counter anion of the "nitrogen-containing heterocyclic ring wherein the nitrogen atom forms a quaternary ammonium" include an anion of a halogen atom (e.g. Cl⁻, Br⁻, I⁻, etc.), etc., and also an anion derived from an inorganic acid such as hydrochloric acid, hydrobromic acid, nitric acid, sulfuric acid, phosphoric acid, etc.; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; an anion derived from an acidic amino acid such as aspartic acid, glutamic acid, etc.; etc. Among others, Cl⁻, Br⁻, I⁻, etc. are preferable.

Said "nitrogen-containing heterocyclic ring" may bind to the divalent group represented by Z through either a carbon atom or a nitrogen atom, and may be 2-pyridyl, 3-pyridyl, 2-piperidinyl, etc. which binds to the divalent group represented by Z through a carbon atom. Preferably, the "nitrogen-containing heterocyclic ring" binds to the divalent group represented by Z through a nitrogen atom, as exemplified by the following formulas: 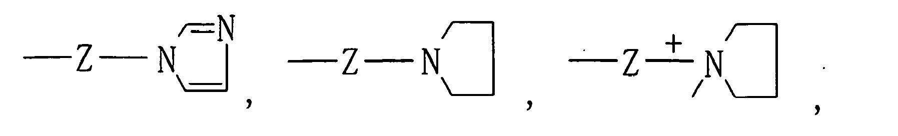 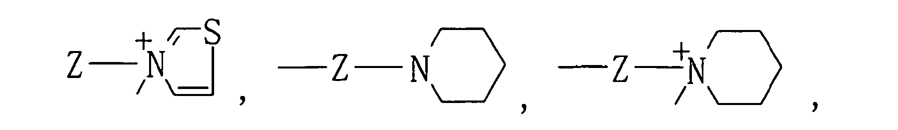 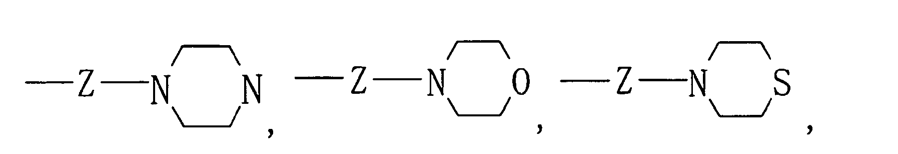 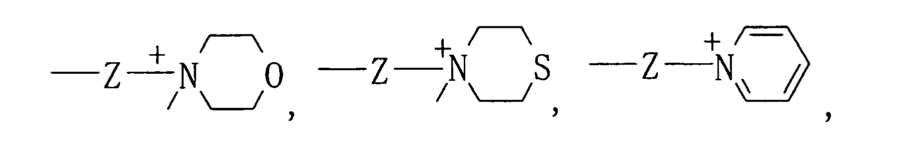 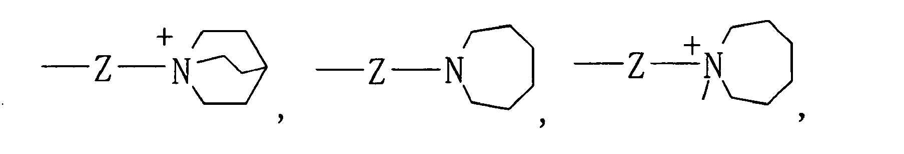 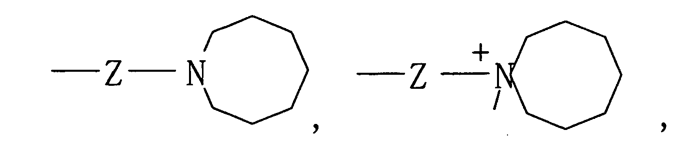 etc.

Examples of the substituents, which said "nitrogen containing heterocyclic ring" may have, include halogen (e.g. fluorine, chlorine, bromine, iodine, etc.), an optionally substituted lower (C₁₋₄) alkyl, an optionally substituted lower (C₁₋₄) alkoxy, an optionally substituted phenyl, an optionally substituted mono- or di-phenyl-lower (C₁₋₄) alkyl, an optionally substituted C₃₋₇ cycloalkyl, cyano, nitro, hydroxy group, thiol group, amino group, carboxyl group, lower (C₁₋₄) alkoxy-carbonyl, lower (C₂₋₄) alkanoyl, lower (C₁₋₄) alkylsulfonyl, an optionally substituted heterocyclic ring group (e.g. 5- to 6-membered aromatic heterocyclic ring containing 1 to 4 hetero-atoms consisting of 1 to 2 kinds of hetero-atoms selected from an oxygen atom, a sulfur atom and a nitrogen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; 5- to 6-membered non-aromatic heterocyclic ring containing 1 to 4 hetero-atoms consisting of 1 to 2 kinds of hetero-atoms selected from an oxygen atom, a sulfur atom and a nitrogen atom such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; etc.), etc., and the number of the substituents is preferably 1-3.

Examples of the substituent, which the "optionally substituted lower (C₁₋₄) alkyl", the "optionally substituted lower (C₁₋₄) alkoxy", the "optionally substituted phenyl", the "optionally substituted mono- or di-phenyl-lower (C₁₋₄) alkyl", the "optionally substituted C₃₋₇ cycloalkyl" and the "optionally substituted heterocyclic ring group" as a substituent for said "nitrogen-containing heterocyclic ring" may have, include halogen (e.g. fluorine, chlorine, bromine, iodine, etc.), an optionally halogenated lower (C₁₋₄) alkyl, an optionally halogenated C₁₋₄ alkoxy (e.g. methoxy, ethoxy, trifluoromethoxy, trifluoroethoxy, etc.), C₂₋₄alkanoyl (e.g. acetyl, propionyl, etc.), C₁₋₄ alkylsulfonyl (e.g. methanesulfonyl, ethanesulfonyl, etc.), C₁₋₃ alkylenedioxy (e.g. methylenedioxy, ethylenedioxy, etc.), cyano, nitro, hydroxy group, thiol group, amino group, carboxyl group, lower (C₁₋₄) alkoxy-carbonyl, etc., and the number of the substituents are preferably 1 to 3.

In the above formula (I), preferred example of the substituents for the "nitrogen-containing heterocyclic ring" in the "optionally substituted nitrogen-containing heterocyclic ring group which may contain a sulfur atom or an oxygen atom as ring constituting atoms and wherein a nitrogen atom may form a quaternary ammonium" include (1) halogen, (2) cyano, (3) hydroxy group, (4) carboxyl group, (5) lower (C₁₋₄) alkoxy-carbonyl, (6) lower (C₁₋₄) alkyl which may be substituted with halogen, hydroxy group or lower (C₁₋₄) alkoxy, (7) lower (C₁₋₄) alkoxy which may be substituted with halogen, hydroxy group or lower (C₁₋₄) alkoxy, (8) phenyl which may be substituted with halogen, lower (C₁₋₄) alkyl, hydroxy group, lower (C₁₋₄) alkoxy or C₁₋₃ alkylenedioxy, (9) mono- or di-phenyl-lower (C₁₋₄) alkyl whose benzene ring may be substituted with halogen, lower (C₁₋₄) alkyl, hydroxy group, lower (C₁₋₄) alkoxy or C₁₋₃ alkylenedioxy, (10) 5- to 6-membered aromatic heterocyclic ring such as furan, thiophene, pyrrole, pyridine, etc., etc.

In the above formula (I), examples of the "hydrocarbon group" in the "optionally substituted hydrocarbon group" represented by R⁵ and R⁶ of the "group of the formula: 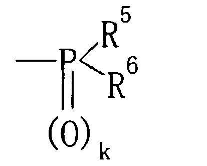 wherein k is 0 or 1, and when k is 0, a phosphorus atom may form a phosphonium; and R⁵ and R⁶ are independently an optionally substituted hydrocarbon group or an optionally substituted amino group, and R⁵ and R⁶ may bind to each other to form a cyclic group together with the adjacent phosphorus atom" represented by R² include
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted alkenyl (e.g. C₂₋₁₀ alkenyl such as allyl, crotyl, 2-pentenyl,3-hexenyl, etc., preferably lower (C₂₋₆) alkenyl, etc.);
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.);
(5) an optionally substituted alkynyl (e.g. C₂₋₁₀ alkynyl such as ethynyl, 1-propynyl, 2-propynyl, 1-butynyl, 2-pentynyl, 3-hexynyl, etc., preferably lower (C₂₋₆) alkynyl, etc.);
(6) an optionally substituted aralkyl (e.g. phenyl-C₁₋₄ alkyl (e.g. benzyl, phenethyl, etc.), etc.);
(7) an optionally substituted aryl (e.g. phenyl, naphthyl, etc.); etc.

Examples of the substituents, which the above-mentioned (1) optionallysubstituted alkyl, (2)optionally substituted cycloalkyl, (3) optionally substituted alkenyl, (4) optionally substituted cycloalkenyl, (5) optionally substituted alkynyl, (6) optionally substituted aralkyl and (7) optionally substituted aryl may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

Examples of the optionally substituted amino group represented by R⁵ and R⁶ include an amino group which may have 1-2 substituents selected from
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇ cycloalkyl, etc. such as cyclopropyl, cyclobutyl, cyclopentyl, cyclohexyl, cycloheptyl, etc.);
(3) an optionally substituted alkenyl (e.g. C₂₋₁₀ alkenyl such as allyl, crotyl, 2-pentenyl,3-hexenyl, etc., preferably lower (C₂₋₆) alkenyl, etc.) ;
(4) an optionally substituted cycloalkenyl (e.g. C₃₋₇ cycloalkenyl such as 2-cyclopentenyl, 2-cyclohexenyl, 2-cyclopentenylmethyl, 2-cyclohexenylmethyl, etc., etc.);
(5) an optionally substituted acyl (e.g. C₂₋₄ alkanoyl (e.g. acetyl, propionyl, butyryl, isobutyryl, etc.), C₁₋₄ alkylsulfonyl (e.g. methanesulfonyl, ethanesulfonyl, etc.), etc.);
(6) an amino group which may have 1-2 optionally substituted aryl groups (e.g. phenyl, naphthyl, etc.); etc.

Examples of the substituent, which the above mentioned (1) optionally substituted alkyl, (2) optionally substituted cycloalkyl, (3) optionally substituted alkenyl, (4) optionally substituted cycloalkenyl, (5) optionally substituted acyl and (6) optionally substituted aryl may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

In the above formula, the groups R⁵ and R⁶ may bind to each other to form a cyclic group (preferably, 5- to 7-membered ring) together with the adjacent phosphorus atom. Said cyclic group may have a substituent. Examples of the substituent include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

In the above formula (I), examples of the counter anion, when the phosphorus atom forms a phosphonium, include an anion of a halogen atom (e.g. Cl⁻, Br⁻, I⁻, etc.), etc., and also an anion derived from an inorganic acid such as hydrochloric acid, hydrobromic acid, nitric acid, sulfuric acid, phosphoric acid, etc. ; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. ; an anion derived from an acidic amino acid such as aspartic acid, glutamic acid, etc.; etc. Among others, Cl⁻, Br⁻, I⁻, etc. are preferable.

As the group R², (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, (3) a group of the formula: 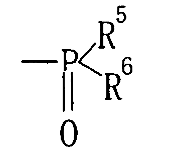 wherein R⁵ and R⁶ are independently an optionally substituted hydrocarbon group, and R⁵ and R⁶ may bind to each other to form a cyclic group together with the adjacent phosphorus atom, etc. are preferable.

In the above formula (I'), examples of the "optionally substituted hydrocarbon group" and the "optionally substituted amino group" represented by R^{5'} and R⁶' in the "group of the formula: 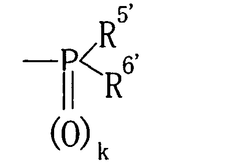 wherein k is 0 or 1, and when k is 0, a phosphorus atom may form a phosphonium; and R⁵' and R⁶' are independently an optionally substituted hydrocarbon group, an optionally substituted hydroxy group or an optionally substituted amino group, and R⁵' and R⁶' may bind to each other to form a cyclic group together with the adjacent phosphorus atom" represented byR² include those exemplified as the "optionally substituted hydrocarbon group" and the "optionally substituted amino group" represented by R⁵ and R⁶, respectively.

In the above formula (I'), examples of the "optionally substituted hydroxy group" represented by R⁵' and R⁶' include a hydroxy group which may have
(1) an optionally substituted alkyl (e.g. C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, etc.);
(2) an optionally substituted cycloalkyl (e.g. C₃₋₇cycloalkyl, etc. such as cyclopropyl, cyclobutyl, cyclopentyl, cyclohexyl, cycloheptyl, etc.);
(3) an optionally substituted alkenyl (e.g. C₂₋₁₀ alkenyl such as allyl, crotyl, 2-pentenyl,3-hexenyl, etc., preferably lower (C₂₋₆)alkenyl, etc.);
(4) an optionally substituted cycloalkenyl (e.g. C₃₋₇ cycloalkenyl, etc. such as 2-cyclopentenyl, 2-cyclohexenyl, 2-cyclopentenylmethyl, 2-cyclohexenylmethyl, etc.);
(5) an optionally substituted aralkyl (e.g. phenyl-C₁₋₄ alkyl (e.g. benzyl, phenethyl, etc.), etc.);
(6) an optionally substituted acyl (e.g. C₂₋₄ alkanoyl (e.g. acetyl, propionyl, butyryl, isobutyryl, etc.), C₁₋₄ alkylsulfonyl (e.g. methanesulfonyl, ethanesulfonyl, etc.), etc.);
(7) an optionally substituted aryl (e.g. phenyl, naphthyl, etc.); etc.

Examples of the substituents, which the above-mentioned (1)optionally substituted alkyl, (2) optionally substituted cycloalkyl, (3) optionally substituted alkenyl, (4) optionally substituted cycloalkenyl, (5) optionally substituted aralkyl, (6) optionally substituted acyl and (7) optionally substituted aryl may have, include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.), C₂₋₄ alkanoyl (e.g. acetyl, propionyl, etc.), C₁₋₄ alkylsulfonyl (e.g. methanesulfonyl, ethanesulfonyl, etc.), etc., and the number of the substituents are preferably 1 to 3.

In the above formula, the groups R⁵' and R⁶' may bind to each other to form a cyclic group (preferably, 5- to 7-membered ring) together with the adjacent phosphorus atom. Said cyclic group may have a substituent. Examples of the substituent include halogen (e.g. fluorine, chlorine, bromine, iodine, etc.), nitro, cyano, hydroxy group, thiol group, amino group, carboxyl group, an optionally halogenated C₁₋₄ alkyl (e.g. trifluoromethyl, methyl, ethyl, etc.), an optionally halogenated C₁₋₄ alkoxy (e.g. methoxy, ethoxy, trifluoromethoxy, trifluoroethoxy, etc.),C₂₋₄alkanoyl (e.g. acetyl, propionyl, etc.), C₁₋₄ alkylsulfonyl (e.g. methanesulfonyl,ethanesulfonyl,etc.),etc.,and the number of the substituents are preferably 1 to 3.

In the above formula (I'), examples of the counter anion, when the phosphorus atom forms a phosphonium, include an anion of a halogen atom (e.g. Cl⁻, Br⁻, I⁻, etc.), etc., and also an anion derived from an inorganic acid such as hydrochloric acid, hydrobromic acid, nitric acid, sulfuric acid, phosphoric acid, etc. ; an anion derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; an anion derived from an acidic amino acid such as aspartic acid, glutamic acid, etc.; etc. Among others, Cl⁻, Br⁻, I⁻, etc. are preferable.

As the group R², (1) an optionally substituted amino group in which a nitrogen atom may form a quaternary ammonium is preferable, and a group of the formula:
-N⁺RR'R" wherein R, R' and R'' are independently an optionally substituted aliphatic hydrocarbon group or an optionally substituted alicyclic heterocyclic ring group is more preferable.

Among the Compounds (I), a compound of the formula: 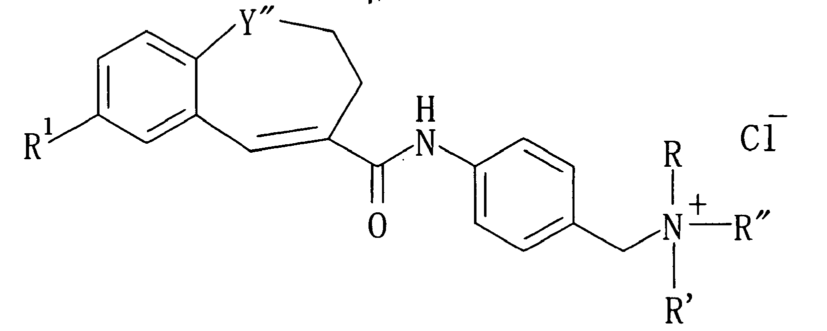 wherein R¹ is an optionally substituted benzene or an optionally substituted thiophene; Y" is -CH₂-, -S- or -O-; and R, R' and R" are independently an optionally substituted aliphatic hydrocarbon group or an optionally substituted alicyclic heterocyclic ring group is preferable.

Examples of the "optionally substituted aliphatic hydrocarbon group" and the" optionally substituted alicyclic heterocyclic ring group" represented by R, R' or R" include those exemplified by the substituents for the "optionally substituted amino" represented by R². Among them, as the group R or R', an optionally substituted acyclic hydrocarbon group is preferable, an optionally substituted C₁₋₆ alkyl group is more preferable, and methyl is most preferable; and as the group R", an optionally substituted alicyclic hydrocarbon group (more preferably, an optionally substituted C₃₋₈ cycloalkyl group; further more preferably, an optionally substituted cyclohexyl) or an optionally substituted alicyclic heterocyclic ring group (more preferably, an optionally substituted saturated alicyclic heterocyclic ring group (preferably 6-membered ring group); further more preferably, an optionally substituted tetrahydropyranyl, an optionally substituted tetrahydrothiopyranyl or an optionally substituted piperidyl; most preferably, an optionally substituted tetrahydropyranyl) is preferable.

Among the Compounds (I), a compound of the formula: 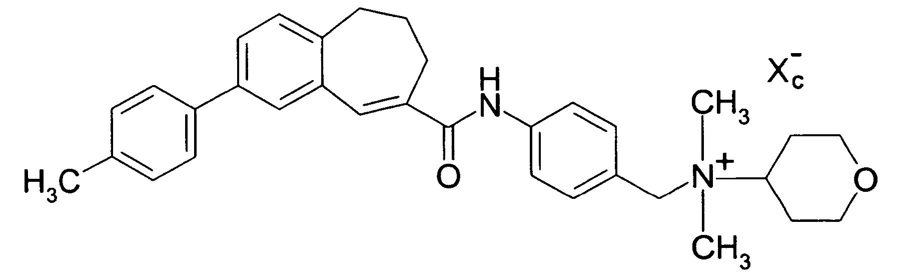 wherein X_{c}⁻ is an anion is preferable.

Examples of the anion include that of a halogen atom; that derived from an inorganic acid such as hydrochloric acid, hydrobromic acid, nitric acid, sulfuric acid, phosphoric acid, etc.; that derived from an organic acid such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; that derived from an acidic amino acid such as aspartic acid, glutamic acid, etc. ; etc. Among others, an anion of a halogen atom is preferable.

Among the Compound (I), the following compounds and their salts are preferable:
N-methyl-N-[4-[[[2-(4-methylphenyl)-6,7-dihydro-5H-benzocyclohepten-8-yl]carbonyl]amino]benzyl]-piperidinium iodide;
N-methyl-N-[4-[[[7-(4-methylphenyl)-2,3-dihydro-1-benzoxepin-4-yl]carbonyl]amino]benzyl]piperidinium iodide;
N- [4- [N-methyl-N- (tetrahydropyran-4-yl) aminomethyl]-phenyl]-7-(4-methylphenyl)-2,3-dihydro-1-benzoxepine-4-carboxmide;
N-[4-[N-methyl-N-(tetrahydropyran-4-yl)aminomethyl]-phenyl]-7-(4-morpholinophenyl)-2,3-dihydro-1-benzoxepine-4 -carboxmide;
7-(4-ethoxyphenyl)-N-[4-[N-methyl-N-(tetrahydropyran-4-yl)aminomethyl]phenyl]-2,3-dihydro-1-benzoxepine-4-carboxmide;
N,N-dimethyl-N-[4-[[[2-(4-methylphenyl)-6,7-dihydro-5H-benzocyclohepten-8-yl]carbonyl]amino]benzyl]-N-(tetrahydropyran-4-yl)ammonium iodide;
N,N-dimethyl-N-[4-[[[7-(4-methylphenyl)-2,3-dihydro-1-benzoxepin-4-yl]carbonyl]amino]benzyl]-N-(4-oxocyclohexyl) ammonium chloride;
N,N-dimethyl-N-[4-[[[7-(4-ethoxyphenyl)-2,3-dihydro-1-benzoxepin-4-yl] carbonyl] amino] benzyl] -N- (tetrahydropyran-4-yl)ammonium chloride;
N-methyl-N-[4-[[[7-(4-methylphenyl)-3,4-dihydro-naphthalen-2-yl]carbonyl]amino]benzyl]piperidinium iodide; etc.

Examples of the salts of the compound represented by the formula (I) [including the formula (I')] include a pharmaceutically acceptable salt such as a salt with inorganic base, a salt with organic base, a salt with inorganic acid, a salt with organic acid, a salt with basic or acidic amino acid, etc. Examples of the salt with the inorganic base include a salt with alkali metal (e.g. sodium, potassium, etc.), alkaline earth metal (e.g. calcium, magnesium, etc.), aluminum, ammonium, etc. Examples of the salt with the organic base include a salt with trimethylamine, triethylamine, pyridine, picoline, ethanolamine, diethanolamine, triethanolamine, dicyclohexylamine, N,N'-dibenzylethylenediamine, etc. Examples of the salt with the inorganic acid include a salt with hydrochloric acid, hydrobromic acid, nitric acid, sulfuric acid, phosphoric acid, etc. Examples of the salt with the organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc. Examples of the salt with the basic amino acid include a salt with arginine, lysine, ornithine, etc. Examples of the salt with the acidic amino acid include a salt with aspartic acid, glutamic acid, etc.

The compound of the formula (I) [including the formula (I')] of the present invention may be hydrated or solvated. When the compound of the formula (I) [including the formula (I') of the present invention exists as configuration isomer, diastereomer, conformer, etc., it is possible to isolate individual isomers with per se known separation and purification method, if desired. When the compound of the formula (I) [including the formula (I')] of the present invention is racemate, it can be separated into (S) -compound and (R) -compound with usual optical resolution and individual optical isomers and a mixture thereof are included in the scope of the present invention.

The present compound of the formula (I) or a salt thereof (hereinafter, "Compound (I)" include the compound of the formula (I) and its salt; and also a compound of the formula (I') and its salt) alone or as an admixture with a pharmaceutically acceptable carrier (e.g. solid formulations such as tablets, capsules, granules, powders, etc.; liquid formulations such as syrups, injections, etc.) may be orally or non-orally administered.

Examples of non-oral formulations include injections, drops, suppositories, pessaryies, etc.

Examples of the carriers include various organic or inorganic carriers which are generally used in this field. For example, an excipient, a lubricant, a binder, an disintegrating agent, etc. are used in the solid formulations, and a solvent, a solubilizer, a suspending agent, a isotonizing agent, a buffer, a soothing agent, etc. are used in the liquid formulations. In addition, if desired, an appropriate additive such as a preservative, an antioxidant, a colorant, a sweetener, etc. may be used in the above formulations.

Examples of the excipient include lactose, sucrose, D-mannitol, starch, crystalline cellulose, light silic acid anhydride, etc. Examples of the lubricant include magnesium stearate, calcium stearate, talc, colloidal silica, etc. Examples of the binder include crystalline cellulose, sucrose, D-mannitol, dextrin, hydroxypropyl cellulose, hydroxypropylmethyl cellulose, polyvinyl-pyrrolidone, etc. Examples of the disintegrating agent include starch, carboxymethyl cellulose, carboxymethyl cellulose calcium, croscarmellose sodium, sodium carboxymethyl starch, etc. Examples of the solvent include water for injection, alcohol, propyleneglycol, macrogol, sesame oil, corn oil, etc. Examples of the solubilizer include polyethyleneglycol, propyleneglycol, D-mannitol, benzyl benzoate, ethanol, trisaminomethane, cholesterol, triethanolamine, sodium carbonate, sodium citrate, etc. Examples of the suspending agent include surfactants such as stearyl triethanolamine, sodium laurylsulfate, laurylaminopropionic acid, lecithin, benzalkonium chloride, benzetonium chloride, glycerin monostearate, etc.; hydrophilic polymers such as polyvinylalcohol, polyvinylpyrrolidone, sodium carboxymethyl cellulose, methyl cellulose, hydroxymethyl cellulose, hydroxyethyl cellulose, hydroxypropyl cellulose, etc. ; etc. Examples of the isotonizing agent include sodium chloride, glycerin, D-mannitol, etc. Examples of the buffer include a buffer solution of phosphate, acetate, carbonate, citrate, etc. Examples of the soothing agent include benzylalcohol, etc. Examples of the preservative include paraoxybenzoic acid esters, chlorobutanol, benzylalcohol, phenethylalcohol, dehydroacetic acid, sorbic acid, etc. Examples of the antioxidant include sulfites, ascorbic acid, etc.

The present invention is further to provide a production method of a compound of the formula (I) or a salt thereof.

The compound of the formula (I) or a salt thereof can be produced in accordance with per se known methods, for example, the methods described below, the methods described in JP-A-73476/1996, or analogous methods thereto.

A salt of the compound of the formulas (I), (II), (III), (IV), (V), (I-1), (I-2) and (I-3) may be similar to that of the compound the formula (I).

In the following reaction steps, when the starting compounds have, as substituents, an amino group, a carboxyl group and/or hydroxy group, these groups may be protected by ordinary protective groups such as those generally employed in peptide chemistry, etc. After the reaction, if necessary, the protective groups may be removed to obtain the desired compound.

Examples of the amino-protective.group include an optionally substituted C₁₋₆ alkylcarbonyl (e.g. formyl, methylcarbonyl, ethylcarbonyl, etc.), phenylcarbonyl, C₁₋₆ alkyloxycarbonyl (e.g. methoxycarbonyl, ethoxycarbonyl, t-butoxycarbonyl, etc.), aryloxycarbonyl (e.g. phenoxycarbonyl, etc.), C₇₋₁₀ aralkyloxycarbonyl (e.g. benzyloxycarbonyl, etc.), trityl, phthaloyl, etc. These protective groups may be substituted by 1 to 3 substituents such as halogen atom (e.g. fluorine, chlorine, bromine, iodine, etc.), C₁₋₆ alkylcarbonyl (e.g. acetyl, propionyl, butyryl, etc.), nitro group, etc.

Examples of the carboxyl-protective group include an optionally substituted C₁₋₆ alkyl (e.g. methyl, ethyl, propyl, isopropyl, butyl, tert-butyl, etc.), phenyl, trityl, silyl, etc. These protective groups may be substituted by 1 to 3 substituents such as halogen atom (e.g. fluorine, chlorine, bromine, iodine, etc.), C₁₋₆ alkylcarbonyl (e.g. formyl, acetyl, propionyl, butyryl, etc.), nitro group, etc.

Examples of the hydroxy-protective group include an optionally substituted C₁₋₆ alkyl. (e.g. methyl, ethyl, propyl, isopropyl, butyl, tert-butyl, etc.), phenyl, C₇₋₁₀ aralkyl (e.g. benzyl, etc.), C₁₋₆ alkylcarbonyl (e.g. formyl, acetyl, propionyl, etc.), phenyloxycarbonyl, C₇₋₁₀ aralkyloxycarbonyl (e.g. benzyloxycarbonyl, etc.), pyranyl, furanyl, silyl, etc. These protective groups may be substituted by 1 to 4 substituents such as halogen atom (e.g. fluorine, chlorine, bromine, iodine, etc.), C₁₋₆ alkyl, phenyl, C₇₋₁₀ aralkyl ,nitro group, etc.

These protective group may be introduced or removed by per se known methods (e.g. a method described in Protective Groups in Organic Chemistry (J. F. W. McOmie et al.; Plenum Press Inc.) or the methods analogous thereto. For example, employable method for removing the protective groups is a method using an acid, a base, reduction, ultraviolet ray, hydrazine, phenylhydrazine, sodium N-methyldithiocarbamate, tetrabutylammonium fluoride, palladium acetate, etc.

### [Method A]

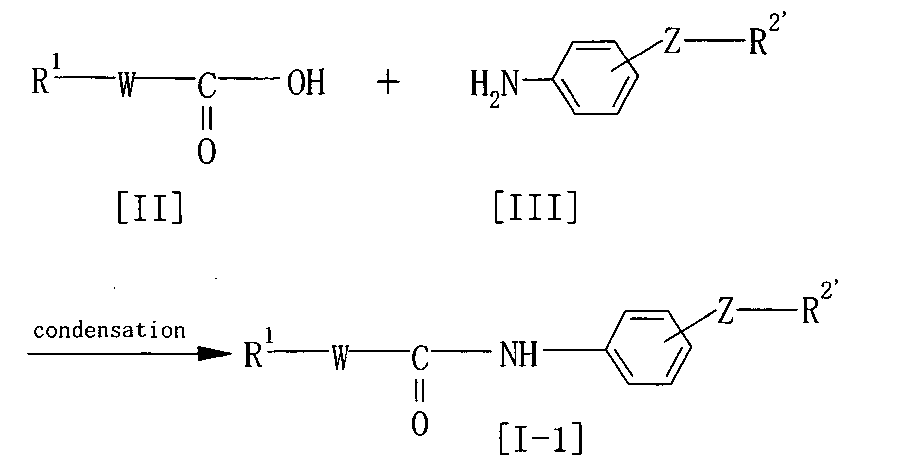

wherein each symbol is as defined above.

This production method is carried out by reacting the compound [II] with the aniline derivative [III] to obtain the anilide Compound [I-1].

The condensation reaction of the compounds [II] and [III] is carried out by usual methods for peptide synthesis. Said methods for peptide synthesis are employed according to optional known methods, for example, methods described in "Peptide Synthesis" written by M. Bodansky and M. A. Ondetti, Interscience, New York, 1966 ; "The Proteins", volume 2, written by F. M. Finn and K. Hofmann, H. Nenrath and R. L. Hill edition, Academic Press Inc., New York, 1976; "peputido-gosei no kiso to jikken (Basis and Experiment of Peptide Synthesis)" written by Nobuo Izumiya et al., Maruzen K.K.,1985; etc., as well as azide method, chloride method, acid anhydride method, mixed acid anhydride method, DCC method, active ester method, method using Woodward reagent K, carbonyldiimidazole method, oxidation-reduction method, DCC/HONB method, etc. and in addition WSC method, method using diethyl cyanophosphate (DEPC), etc.

The condensation reaction can be carried out in a solvent. Examples of the solvents to be employed in the reaction include anhydrous or hydrous N,N-dimethylformamide (DMF), dimethylsulfoxide, pyridine, chloroform, dichloromethane, tetrahydrofuran, dioxane, acetonitrile, or a suitable mixture of these solvents. The reaction temperature is generally about -20°C to about 50°C, preferably about -10°C to about 30°C and the reaction time is generally about 1 to about 100 hours, preferably about 2 to about 40 hours.

The thus obtained anilide derivative [I-1] can be isolated and purified by known separation and purification methods such as concentration, concentration under reduced pressure, extraction, crystallization, recrystallization, solvent convert, chromatography, etc.

### [Method B]

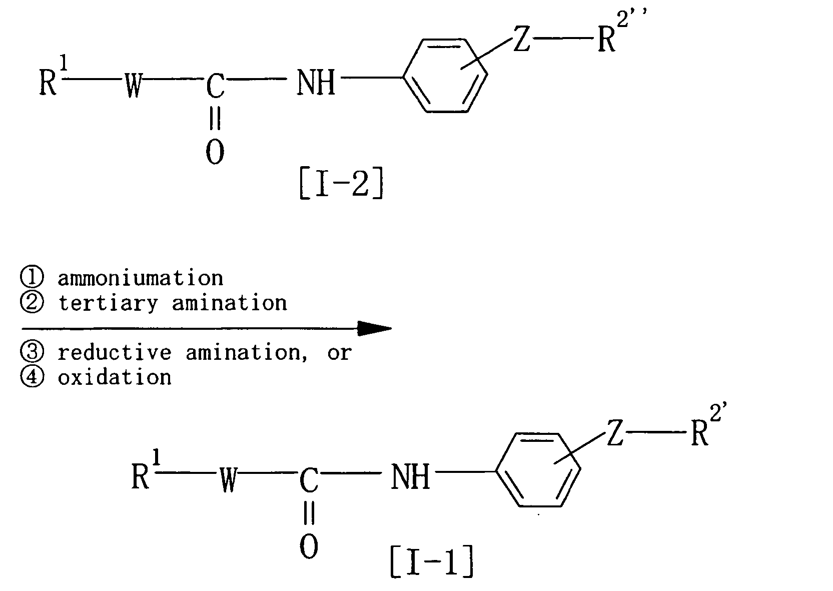

① When the group R²" in Compound [I-2] is, for example, a tertiary amine residue, Compound [I-1] wherein the group R²' is an quaternary ammonium can be produced by reacting Compound [1-2] with halogenated alkyl or halogenated aralkyl. Examples of a halogen atom include chlorine, bromine, iodine, etc. and usually about 1 to 5 moles of the halogenated alkyl (e.g. halogenated lower (C₁₋₆) alkyl, etc.) or halogenated aralkyl (e.g. halogenated lower (C₁₋₄) alkyl-phenyl, etc.) is used per mole of Compound [I-2]. The reaction is carried out in an inert solvent such as toluene, benzene, xylene, dichloromethane, chloroform, 1,2-dichloroethane, dimethylformamide, dimethylacetamide, etc., or a suitable mixture of these solvents. The reaction temperature is generally about 10°C to about 160°C, preferably about 20°C to about 120°C and the reaction time is generally about 1 hour to about 100 hours, preferably about 2 hours to about 40 hours. This reaction is preferably carried out under inert gas (e.g. nitrogen, argon, etc.) atmosphere.
(2) When the group R²" in Compound [1-2] is, for example, a secondary amine residue, Compound [1-1] wherein the group R²' is a tertiary amino can be produced by reacting Compound [1-2] with halogenated alkyl or halogenated aralkyl. Examples of a halogen atom include chlorine, bromine, iodine, etc. and usually about 1 to 2 moles of the halogenated alkyl or halogenated aralkyl is used per mole of Compound [I-2]. If necessary, the reaction smoothly proceeds by addition of about once to thrice moles of a base such as triethylamine, diisopropylethylamine, pyridine, lithium hydride, sodium hydride, sodium methoxide, sodium ethoxide, sodium carbonate, potassium carbonate, sodium hydrogen carbonate and further sodium iodide, potassium iodide, etc.
   This tertiary amination reaction is carried out in an inert solvent such as methanol , ethanol, propanol, isopropanol, n-butanol, tetrahydrofuran, diethylether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a suitable mixture of these solvents. The reaction temperature is generally about 0°C to 180°C, and the reaction time is generally about 1 hour to about 40 hours. This reaction is preferably carried out under inert gas (e.g. nitrogen, argon, etc.) atmosphere.
③ When the group R²" in Compound [I-2] is, for example, a secondary amine residue, Compound [I-1] wherein the group R² is a tertiary amino can be produced by reacting Compound [I-2] with aldehyde compound in the presence of a reductive amination reagent such as triacetoxysodium boron hydride, cyanosodium boron hydride, sodium boron hydride, etc.
   The conditions of this reductive amination reaction varies depending on the reagent to be used. For example, when triacetoxysodium boron hydride is used ,reaction is carried out in an inert solvent such as dichloromethane, chloroform, 1,2-dichloroethane, tetrahydrofuran, diethylether, dioxane, acetonitrile, dimethylformamide (DMF), etc., or a suitable mixture of these solvents. In this case, about 1 to 2 moles of the reagent is used per mole of Compound [I-2]. The reaction temperature is generally about 0°C to about 80°C, and the reaction time is generally about 1 hour to about 40 hours. This reaction is preferably carried out under inert gas (e.g. nitrogen, argon, etc.) atmosphere.
④ When the group R²" in Compound [I-2] is, for example, a tertiary amine residue, Compound [I-1] wherein the group R²' is an amine oxide group can be produced by reacting Compound [I-2] with an oxidizing agent such as m-chloroperbenzoic acid, perbenzoic acid, p-nitroperbenzoic acid, magnesium monoperoxyphthalate, peracetic acid, hydrogen peroxide, sodium periodate, potassium periodate, etc. The conditions of this oxidation reaction varies depending on the oxidizing agent to be used. For example, when m-chloroperbenzoic acid is used, reaction is carried out in an inert solvent such as dichloromethane, chloroform, 1,2-dichloroethane, diethylether, tetrahydrofuran, acetone, ethyl acetate, etc., or a suitable mixture of these solvents. Usually, about 1-3 moles of oxidizing agent is used per mole of Compound [I-2]. The reaction temperature is generally about -25°C to about 80°C (preferably -25°C to 25°C), and the reaction time is generally about 1 hour to about 40 hours.

### [Method C]

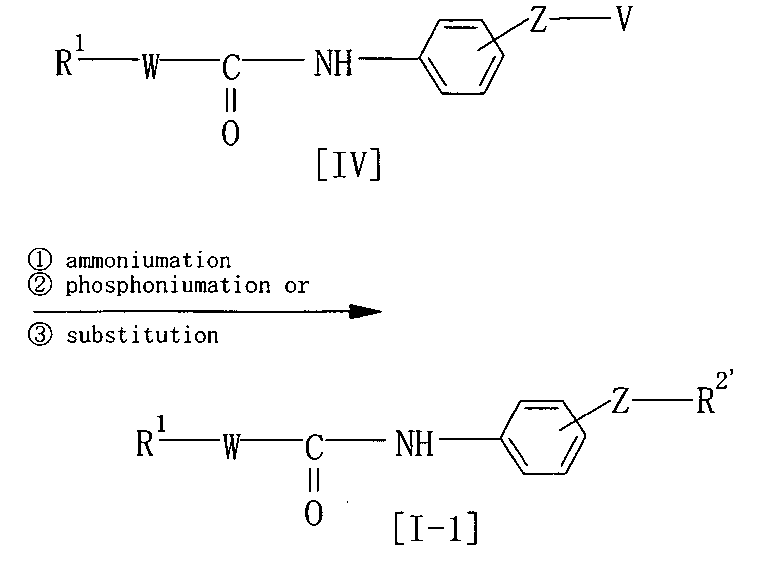

wherein V in the Compound [IV] is a halogen atom (chlorine, bromine, iodine, etc.), or a sulfonyloxy group (methanesulfonyloxy group, trifluoromethanesulfonyloxy group, benzenesulfonyloxy group, toluenesulfonyloxy group, etc.), and the other symbols are as defined above.
① Compound [I-1] wherein the group R²' is a quaternary ammonium can be produced by reacting Compound [IV] and a tertiary amine. The reaction is carried out in an inert solvent such as toluene, benzene, xylene, dichloromethane, chloroform, 1,2-dichloroethane, dimethylformamide (DMF), dimethylacetamide, etc., or a suitable mixture of these solvents. Usually, about 1-3 moles of the tertiary amine is used per mole of Compound [IV]. The reaction temperature is generally about 10°C to about 120°C, and the reaction time is generally about 1 hour to about 40 hours. This reaction is preferably carried out under inert gas (e.g. nitrogen, argon, etc.) atmosphere.
② Compound [I-1] wherein the group R²' is a quaternary phosphonium can be produced by reacting Compound [IV] and a tertiary phosphine. The reaction is carried out in an inert solvent such as toluene, benzene, xylene, dichloromethane, chloroform, 1,2-dichloroethane, acetonitrile, dimethylformamide(DMF), or a suitable mixture of these solvents. Usually, about 1-2 moles of the tertiary phosphine is used per mole of Compound [IV]. The reaction temperature is generally about 20°C to about 150°C, and the reaction time is generally about 1 hour to about 50 hours. This reaction is preferably carried out under inert gas (e.g. nitrogen, argon, etc.) atmosphere.
③ Compound [I-1] wherein the group R²' is a secondary or tertiary amino group can be produced by reacting Compound [IV] and primary or secondary amine compound. Usually, about 1 to 3 moles of the primary or secondary amine compound isusedpermoleof Compound [IV] . If necessary, the reaction smoothly proceeds by addition of about once to thrice moles of a base such as triethylamine, diisopropylethylamine, pyridine, lithium hydride, sodium hydride, sodium methoxide, sodium ethoxide, sodium carbonate, potassium carbonate, sodium hydrogen carbonate and further sodium iodide, potassium iodide, etc. This substitution reaction is carried out in an inert solvent such as methanol, ethanol, propanol, isopropanol, n-butanol, tetrahydrofuran, diethylether, dimethoxyethane, 1,4-dioxane, toluene, benzene, xylene, dichloromethane, chloroform, 1,2-dichloroethane, dimethylformamide (DMF),dimethylsulfoxide (DMSO), pyridine, etc., or a suitable mixture of these solvents. The reaction temperature is generally about -10°C to about 180°C, and the reaction time is generally about 1 hour to about 40 hours. The reaction is carried out preferably under inert gas (e.g. nitrogen, argon, etc.) atmosphere.

### [Method D]

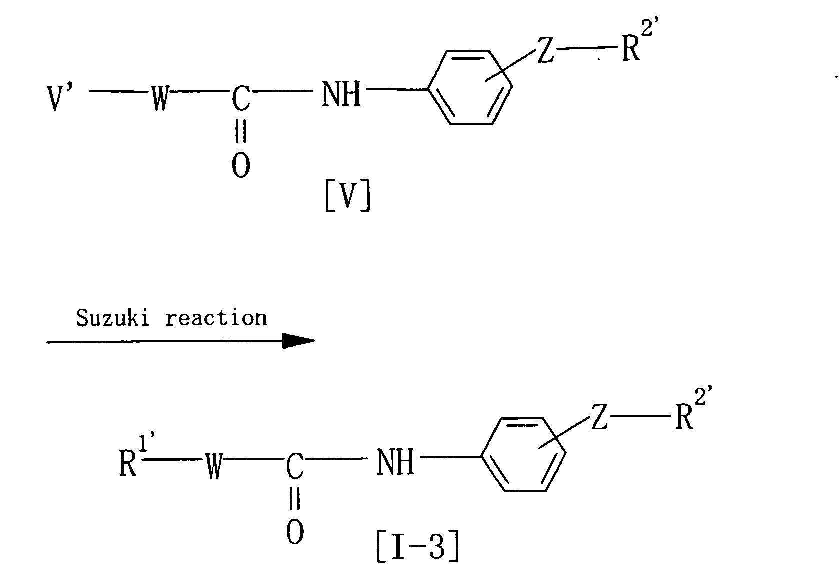

wherein V' is a halogen atom (bromine, iodine, etc.) or a sulfonyloxy group (trifluoromethanesulfonyloxy group, etc.), and the other symbols are as defined above.

Compound [I-3] wherein the group R¹' is a 5- to 6-membered aromatic ring group can be produced by subjecting Compound [V] to, for example, Suzuki reaction [cross condensation reaction of aryl borate with e.g. aryl halide or aryloxytrifluoromethanesulfonate in the presence of palladium catalyst; A. Suzuki et al., Synth. Commun. 1981, 11, 513]. Usually, about 1-1.5 times moles of aryl borate is used per mole of Compound [V].

Compound [II] used as a starting material can be produced by a known method (e.g. method described in JP-A-73476/1996, etc.) or the methods analogous thereto. For example, Compound [II] can be produced by a method described in the following Reaction Scheme I, a method described in the following Reference Examples or the methods analogous thereto. 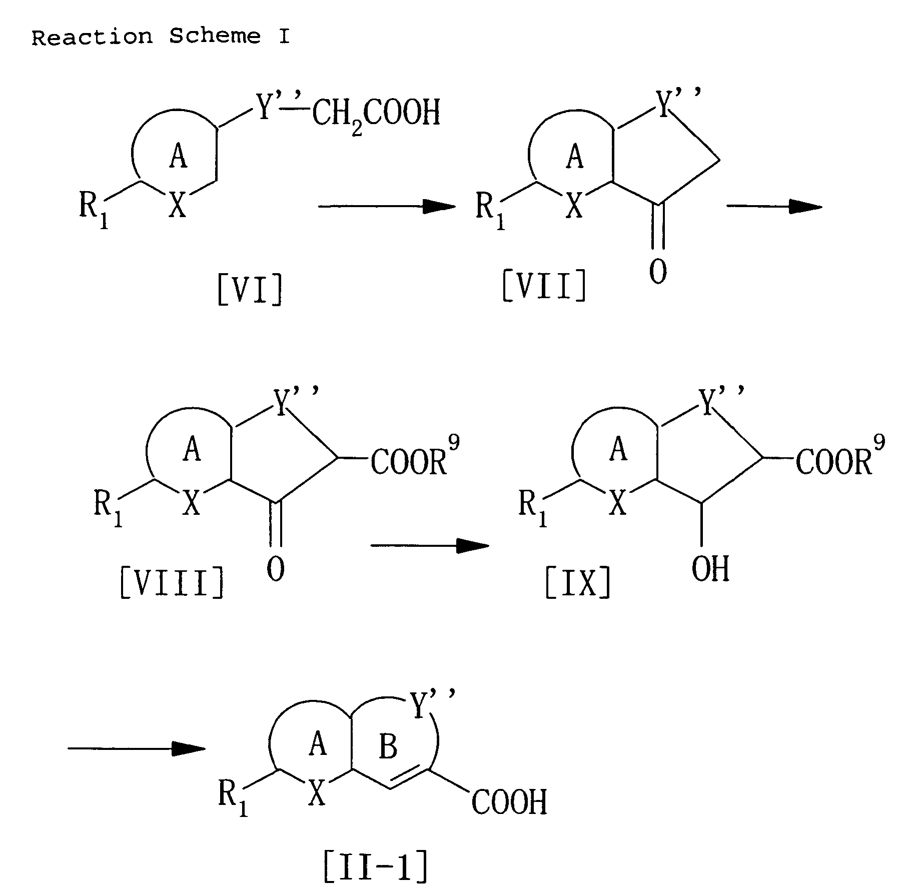 wherein R⁹ is a C₁₋₄ alkyl group, Y" is a divalent group, which does not contain a unsaturated bond and by which the ring B forms a 5- to 7-membered ring, and the other symbols are as defined above.

In this reaction, the compound of the formula [VI] is heated with a polyphosphoric acid, or Compound [VI] is converted to acid chloride with thionyl chloride, oxalyl chloride, phosphorous oxychloride, phosphorous pentachloride, etc., followed by subjecting the resulting acid chloride to usual Friedel-Crafts reaction and cyclizing the same to produce Compound [VII]. Compound [VII] is reacted with carbonate ester in the presence of a base to produce ketoester [VIII]. Compound [VIII] is subjected to reduction with catalytic hydrogenation or sodium boron hydride, etc. to produce Compound [IX]. Compound [IX] is subjected to dehydration and ester hydrolysis by per se known method to produce unsaturated carboxylic acid [II-1].

Compound [III] can be produced by a known method (e.g. method described in JP-A-73476/1996, etc.) or the methods analogous thereto. For example, Compound [III] can be produced by a method described in the following Reaction Scheme II, a method described in the following Reference Examples or the methods analogous thereto.

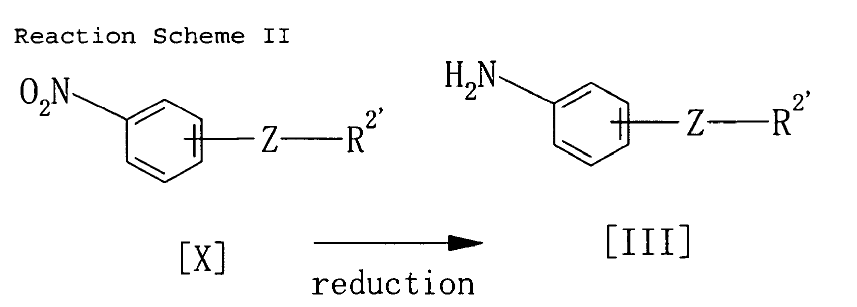

The reduction of Compound [X] can be carried out per se known methods, for example, reduction with metal, reduction with metal hydride, reduction with metal hydride complex compound, reduction with diborane or substituted borane, catalytic hydrogenation, etc. That is, this reaction is carried out by treating Compound [X] with reduction agent. Examples of the reduction agent include metal such as reduced iron, zinc powder, etc. ; alkali metal boron hydride (e.g. sodium boron hydride, lithium boron hydride, etc.); metal hydride complex compound such as aluminum lithium hydride, etc. ; metal hydride such as sodium hydride etc.; organic tin compound (triphenyltin hydride, etc.), metal complex compound and metal salt such as nickel compound, zinc compound etc.; catalytic reduction agent using hydrogen and transit metal catalyst such as palladium, plutinum, rhodium, etc.; diborane; etc. Among others, as the reduction agent, catalytic reduction agent using hydrogen and transit metal catalyst such as palladium, plutinum, rhodium, etc.; reduced iron, etc. are preferable. The reaction is carried out in a solvent which does not affect the reaction. Examples of the solvent include benzene, toluene, xylene, chloroform, carbon tetrachloride, dichloromethane, 1,2-dichloroethane, 1,1,2,2-tetrachloroethane, diethylether, tetrahydrofuran, dioxane, methanol, ethanol, propanol, isopropanol, 2-methoxyethanol, N,N-dimethylformamide, acetic acid, or a suitable mixture of these solvents, etc. The solvent is appropriately selected depending on kind of the reduction agent. The reaction temperature is generally about -20°C to about 150°C, preferably about 0°C to about 100°C, and the reaction time is generally about 1 to about 24 hours.

The resulting Compound [III] can be separated and purified with know separation and purification methods such as concentration, concentration under reduced pressure, extraction, crystallization, was recrystallized with, solvent conversion, chromatography, etc.

The compound of the formula (I) or a salt thereof of the present invention has potent antagonistic activity on MCP-1 receptor and therefore can be used for the treatment or prophylaxis of various inflammatory diseases, cardiac infarction, myocarditis, etc. in human and animals (e.g. mouse, rat, cat, dog, rabbit, bovine, swine, etc.). The compound of the formula (I) or a salt thereof of the present invention is low toxic and safely used as MCP-1 receptor antagonist (e.g. a medicament for the treatment or prophylaxis of cardiac infarction, myocarditis, etc.).

The dose per day of the compound of the formula (I) or a salt thereof varies depending on the condition and body weight of a patient, administration route, etc. Typical daily dose per adult patient (body weight: 50Kg) for oral administration is about 5-1000mg, preferably about 10-600mg, and in particular about 15-150mg, as active ingredient [the compound of the formula (I) or a salt thereof] and the compound of the formula (I) or a salt thereof is administered once or 2-3 times par day.

### Best Mode for Carrying out the Invention

The present invention is hereinafter described in more detail by means of the following Test Example, Reference Example and Working Example, which are mere examples of the present invention and are not construed as limitative to the present invention.

### Test Example 1

### Determination of inhibitory activity on MCP-1 receptor

According to a method described in Working Example 1 of JP-A-238688/1997, human MCP-1 receptor gene was prepared. Said gene was inserted to plasmid pMCR, which was introduced into CHO cell. The resultant transformant [CHO(MCR) ; FERM BP-5446; IFO 50461] was used for the following experiment.

On 96 well culture plate (Packard Instrument Company), 7×10⁴ cell/well of CHO cells expressing human MCP-1 receptor were inoculated, and the cells were cultivated at 37°C overnight. The medium was removed by means of suction. To the residue were added a buffer solution (D-MEM containing 0.5% BSA and 20mM HEPES; pH7.4), Test Compound (1 *µ*M) and ¹²⁵I-human recombinant MCP-1 (Amersham; final concentration: 100pM), and the mixture was allowed to react at room temperature for 40 minutes. The buffer solution was removed by means of suction and washed twice with PBS. To the residue was added MICROSCINT-20 (Packard Instrument Company), radioactivity of ¹²⁵I (cpm) was determined with Topcount (Packard).

The count number (cpm) (non-specific binding) of ¹²⁵I which binds to CHO cells (mock) having a vector was taken from the count number (cpm) of ¹²⁵I which binds to CHO cells expressing human MCP-1 receptor to obtain the amended count number, which was converted into 100%, and inhibition rate of Test Compound (whose number is referred to in the following Examples) against MCP-1 binding to its receptor was calculated. The results are shown in Table 1.

**Table 1**

| Compound Number | Inhibition Rate (%) |
|---|---|
| 14 | 89 |
| 69 | 77 |
| 87 | 92 |
| 90 | 96 |
| 114 | 80 |
| 135 | 80 |
| 151 | 64 |
| 187 | 98 |

### Test Example 2

### Chemotaxis Inhibition Assay

To a lower chamber of 96 well chemotaxis chamber (Neuro Probe, AB96) was added a solution of 20nM MCP-1 (chemotaxis inducer) in buffer (D-MEM containing 0.5% BSA and 20mM HEPES; pH7.4), and the chamber was covered by a filter coated with bovine fibronectin. To its upper chamber were added CHO cells expressing human MCP-1 receptor (2X10⁵ cell/well) and Test Compound (1 *µ*M), followed by incubation at 37°C in 5% CO₂ for 4 hours. The cells migrated under the filter was stained with Diff Quick, and absorbance at 600nm of wave length (O.D at 600nm) was determined by microplate reader. The absorbance in the absence of MCP-1 in the lower chamber was taken from the absorbance in the presence of MCP-1 in the lower chamber to obtain the amended absorbance (ΔO.D, chemotaxis induced by MCP-1), which was converted into 100%, and chemotaxis inhibition rate of Test Compound was calculated.

The results are shown in Table 2.

**Table 2**

| Compound Number | Inhibition Rate (%) |
|---|---|
| 14 | 87 |
| 114 | 89 |

The pharmaceutical composition for antagonizing MCP-1 receptor (e.g. a medicament for the treatment or prophylaxis of cardiac infarction, myocarditis, etc.) comprising the compound of the formula (I) or a salt thereof of the present invention, as an active ingredient, can be prepared, for example, by the following prescriptions:

| 1. Capsule | |
|---|---|
| (1) Compound obtained in Working Example 114 | 40mg |
| (2) lactose | 70mg |
| (3) fine crystalline cellulose | 9mg |
| (4) magnesium stearate | 1mg |
| | 1 capsule 120mg |

| | |
|---|---|
| (1), (2), (3) and 1/2 of (4) are mixed and then granulated. To the granules is added the remainder of (4), and the whole is filled into a gelatin capsule. | |

| 2. Tablet | |
|---|---|
| (1) Compound obtained in Working Example 114 | 40mg |
| (2) lactose | 58mg |
| (3) corn starch | 18mg |
| (4) fine crystalline cellulose | 3.5mg |
| (5) magnesium stearate | 0.5mg |
| 1 tablet 120mg | |

| | |
|---|---|
| (1), (2), (3), 2/3 of (4) and 1/2 of (5) are mixed and then granulated. To the granules are added the remainders of (4) and (5), followed by subjecting the mixture to compression molding. | |

### Working Example

### Reference Example 1

In THF (50ml) was dissolved 4-nitrobenzylchloride (5.00g), and piperidine (6.20g) was added to the mixture. The reaction mixture was stirred at room temperature.for 20 hours. To the mixture was added water (500ml), and the mixture was extracted with ethyl acetate. The organic layer was washed with saturated sodium chloride solution, dried with anhydrous sodium sulfate and concentrated under reduced pressure. The residue was separated and purified with column chromatography (ethyl acetate/hexane= 1/2) to give 1-(4-nitrobenzyl)piperidine (6.41g) as pale yellow oil.
¹H NMR (200MHz, CDCl₃) δ: 1.38-1.70 (6H, m), 2.30-2.45 (4H, m), 3.55 (2H, s), 7.51 (2H, d, J=8.8Hz), 8.17 (2H, d, J=8. 8Hz) .

### Reference Example 2

In ethanol (50ml) was dissolved 1-(4-nitrobenzyl)-piperidine (6.41g), and 10% dried palladium on carbon (0.33g) was added to the mixture. Under hydrogen atmosphere, the mixture was stirred at room temperature under atmospheric pressure for 24 hours. The palladiumwas filtered off, and the filtrate was concentrated. The residue was recrystallized from hexane to give 1-(4-aminobenzyl)piperidine (1.01g) as pale yellow crystals.
mp 87-88°C

| Elemental Analysis for C₁₂H₁₈N₂ | | |
|---|---|---|
| Calcd: C, 75.74; | H, 9.53; | N, 14.72. |
| Found: C, 75.82; | H, 9.58; | N, 14.61. |

IR (KBr) cm⁻¹ : 3417, 2935, 1614, 1518, 1290, 1117, 1038, 991
¹H NMR (200MHz, CDCl₃) δ : 1.35-1.65 (6H, m), 2.28-2.45 (4H, m), 3.37 (2H, s), 3.61 (2H, br s), 6.64 (2H, d, J=8.6Hz), 7.09 (2H, d, J=8.6Hz).

### Reference Example 3

In THF (3ml) was dissolved 7-cyclohexyl-3,4-dihydronaphthalene-2-carboxylic acid (100mg), and oxalyl chloride (41 *µ*l) and a drop of DMF were added to the mixture. The mixture was stirred at room temperature for 1 hour and concentrated under reduced pressure. The residue was dissolved in THF (3ml), and diethyl 4-aminobenzyl-phosphonate (99mg) and triethylamine (60 µ l) were added to the mixture at room temperature. The reaction mixture was stirred at room temperature for 3 hours. To the mixture was added water (100ml), and the mixture was extracted with ethyl acetate. The organic layer was washed with saturated sodium chloride solution, dried with anhydrous sodium sulfate and concentrated under reduced pressure. The residue was separated and purified with column chromatography (ethyl acetate/hexane= 3/1) to give 7-cyclohexyl-N-[4-(diethoxyphosphoryl)benzyl]-3,4-dihyd ronaphthalene-2-carboxamide (85mg) as colorless crystals.
mp 169-170°C

| Elemental Analysis for C₂₇H₃₄NO₄P·0.2H₂O | | | |
|---|---|---|---|
| Calcd: | C, 68.83; | H, 7.32; | N, 2.97. |
| Found: | C, 68.83; | H, 7.34; | N, 3.00. |

IR (KBr) cm⁻¹: 3301, 2927, 1670, 1591, 1522, 1317, 1227, 1136, 1053, 1026, 966
¹H NMR (200MHz, CDCl₃) δ : 1.05-1.95 (16H, m), 2.40-2.56 (1H, m), 2.60-2.73 (2H, m), 2.80-3.00 (2H, m), 4.00-4.22 (4H, m), 7.05-7.15 (3H, m), 7.31 (1H, s), 7.68-7.88 (5H, m).

### Reference Example 4

In thionyl chloride (5.8ml) was dissolved 4-nitro-benzylphosphonic acid (1.50g), and a drop of DMF were added to the mixture. The mixture was refluxed for 5 hours, and thionyl chloride was evaporated under reduced pressure.
The residue was dissolved in THF (15ml), and to the mixture was dropped a solution of ethylamine (excess amount) and pyridine (1.2ml) in acetonitrile (2ml) at -78°C. The reaction mixture was stirred at room temperature for 24 hours. The precipitates was filtered off, and the filtrate was concentrated. The residue was separated and purified with column chromatography (ethyl acetate/methanol=5/1) to give N,N'-diethyl-p-(4-nitrobenzyl)-phosphondiamide (1.88g) as colorless crystals.
mp 102-103°C

| Elemental Analysis for C₁₁H₁₈N₃O₃P | | | |
|---|---|---|---|
| Calcd: | C, 48.71; | H, 6.69; | N, 15.49. |
| Found: | C, 48.51; | H, 6.40; | N, 15.37. |

IR (KBr) cm⁻¹: 3244, 2970, 1520, 1348, 1173, 1128, 966 ¹H NMR (200MHz, DMSO-d₆) δ : 0.99 (6H, t, J=7.1Hz), 2.65-2.85 (4H, m), 3.11 (2H, d, J=18.8Hz), 3.99-4.15 (2H, m), 7.52 (2H, dd, J=2.2, 8.6Hz), 8.15 (2H, d, J=8.6Hz).

### Reference Example 5

In ethanol (20ml) was dissolved N,N'-diethyl-p-(4-nitrobenzyl)phosphondiamide (1.71g), and 10% dried palladium on carbon (0.09g) was added to the solution.
Under hydrogen atmosphere, the mixture was stirred at room temperature under atmospheric pressure for 72 hours. The palladiumwas filteredoff, and the filtrate was concentrated.
The residue was recrystallized from diisopropylether to give p-(4-aminobenzyl)-N,N'-diethylphosphondiamide (1.28g) as colorless crystals.
mp 109-111°C

| Elemental Analysis for C₁₁H₂₀N₃OP · 0.1H₂O | | | |
|---|---|---|---|
| Calcd: | C, 54.35; | H, 8.46; | N, 17.29. |
| Found: | C, 54.39; | H, 8.42; | N, 17.00. |

IR (KBr) cm⁻¹: 3205, 2968, 1518, 1408, 1182, 1122, 1074, 829, 785
¹H NMR (200MHz, CDCl₃) δ : 1.10 (6H, t, J=7.1Hz), 1 .95-2 .10 (2H, m), 2.80-3.03 (6H, m), 3.30-3.90 (2H, br), 6.64 (2H, d, J=8.4Hz), 7.07 (2H, d, J=8.4Hz) .

### Reference Example 6

In xylene (450ml) was dissolved 7-methoxy-1-tetralone (50.0g) under argon atmosphere. To the mixture was added aluminum chloride (75.7g), and the mixture was refluxed for 4.5 hours. The mixture was cooled to room temperature. To the mixture was added 3N hydrochloric acid (500ml), and the mixture was extracted with ethyl acetate. The organic layer was separated and concentrated under reduced pressure. The residue was separated and purified with column chromatography (ethyl acetate) to give 7-hydroxy-1-tetralone (36.4g) as dark green crystals.
mp 162-163°C
¹H NMR (200MHz, CDCl₃) δ: 2.02-2.20 (2H, m), 2.65 (2H, t, J=6.6Hz), 2.90 (2H, t, J=6.0Hz), 6.00-6.20 (1H, br), 7.04 (1H, dd, J=2.8, 8.4Hz), 7.16 (1H, d, J=8.4Hz), 7.61 (1H, d, J=2 . 8Hz).

### Reference Example 7

In dichloromethane (500ml) were dissolved 7-hydroxy-1-tetralone (15.0g) and triethylamine (38.9ml) under argon atmosphere, and to the mixture was added dropwise trifluoromethanesulfonic acid anhydride (15.6ml) at 0°C. The reaction mixture was stirred for 2 hours at 0°C, and to the mixture was added water (500ml). The organic layer was separated, washed with saturated sodium chloride solution, dried with anhydrous sodium sulfate and concentrated under reduced pressure. The residue was separated and purified with column chromatography (ethyl acetate/hexane=1/7) to give 7-(trifluoromethanesulfoxy)-1-tetralone (23.3g) as pale brown oil.
¹H NMR (200MHz, CDCl₃) δ : 2.10-2.25 (2H, m), 2.69 (2H, t, J=6.6Hz), 3.00 (2H, t, J=6.0Hz), 7.37 (2H, s), 7.91 (1H, s).

### Reference Example 8

A mixture of 7-(trifluoromethanesulfoxy)-1-tetralone (23.3g), phenyl borate (11.8g), potassium carbonate (21.9g), toluene (500ml), ethanol (50ml) and water (50ml) was stirred for 30 minutes at room temperature under argon atmosphere, and to the mixture was added tetrakis (triphenylphosphine) palladium (3.66g). The mixture was refluxed for 20 hours and then cooled to room temperature. The organic layer was separated, washed with saturated sodium chloride solution, dried with anhydrous sodium sulfate, and concentrated under reduced pressure. The residue was separated and purified with column chromatography (ethyl acetate/toluene/hexane=1/5/5) to give 7-phenyl-1-tetralone (15.1g) as pale brown oil.
¹H NMR (200MHz, CDCl₃) δ: 2.10-2.25 (2H, m), 2.65-2.75 (2H, m), 2.96-3.05 (2H, m), 7.31-7.50 (4H, m), 7.57-7.67 (2H, m), 7.73 (1H, dd, J=2.2, 8.0Hz), 8.30 (1H, d, J=2.2Hz).

### Reference Example 9

A mixture of sodium methoxide (18.3g), dimethyl carbonate (107ml) and 7-phenyl-1-tetralone (15.1g) was refluxed for 30 minutes. The reaction mixture was cooled to 0°C . To the mixture was gradually added 3N hydrochloric acid (200ml), and the mixture was extracted with ethyl acetate. The organic layer was washed with saturated sodium chloride solution, dried with anhydrous sodium sulfate and concentrated under reduced pressure to give a brown solid. The solid was dissolved in dichloromethane (100ml), and to the mixture was added sodium boron hydride (1.60g) at 0°C . To the mixture was added dropwise methanol (10ml) for 30 minutes, and the reaction mixture was stirred for 4 hours at 0°C. To the mixture was added water (500ml), and the mixture was extracted with ethyl acetate. The organic layer was washed with saturated sodium chloride solution, dried with anhydrous sodium sulfate and concentrated under reduced pressure. The residue was dissolved in methanol (45ml). To the mixture was added 2N sodium hydroxide (50ml), and the mixture was refluxed for 2 hours. The reaction mixture was cooled to room temperature, acidified with concentrated hydro-chloric acid and extracted with ethyl acetate. The organic layer was washed with saturated sodium chloride solution, dried with anhydrous sodium sulfate and concentrated under reduced pressure. The residue was dissolved in Diglyme (1,1'-oxybis[2-methoxyethane]) (50ml), and to the mixture was added concentrated hydrochloric acid (10ml). The mixture was stirred for 2 hours at 100°C, and to the mixture was added water (500ml) . The mixture was extracted with ethyl acetate, and the organic layer was washed with saturated sodium chloride solution and concentrated under reduced pressure. The residue was dissolved in 1N sodium hydroxide (200ml), washed with diethylether, acidified by adding concentrated hydrochloric acid to the aqueous layer and extracted with ethyl acetate. The organic layer was washed with saturated sodium chloride solution, dried with anhydrous sodium sulfate and concentrated under reduced pressure. The residue was recrystallized from ethanol-water to give 7-phenyl-3,4-dihydronaphthalene-2-carboxylic acid (7.47g) as brown crystals.
mp 204-208°C
¹H NMR (200MHz, CDCl₃) δ : 2.61-2.73 (2H, m), 2.88-3.00 (2H, m), 7.23-7.60 (8H, m), 7.74 (1H, s).

### Reference Example 10

In THF (250ml) was dissolved 4-nitrobenzylbromide (25.0g), and to the mixture was added morpholine (25.2ml) at 0°C. The reaction mixture was stirred for 15 hours at room temperature. To the mixture was added water (500ml), and the mixture was extracted with ethyl acetate. The organic layer was washed with saturated sodium chloride solution, dried with anhydrous sodium sulfate and concentrated under reduced pressure. The residue was separated and purified with column chromatography (ethyl acetate) to give 4- (4-nitrobenzyl) morpholine (25. 5g) as pale yellow crystals. A portion of the crystals was recrystallized from diisopropylether to give pale yellow crystals which were used for various analyses. mp 79-80°C

| | | | |
|---|---|---|---|
| Elemental Analysis for C₁₁H₁₄N₂O₃ | | | |
| Calcd: | C, 59.45; | H, 6.35; | N, 12.60. |
| Found: | C, 59.68; | H, 6.25; | N, 12.75. |

IR (KBr) cm⁻¹: 3350, 1518, 1344, 1111, 1009, 864, 744 ¹H NMR (200MHz, CDCl₃) δ : 2.37-2.55 (4H, m), 3.59 (2H, s), 3.65-3.80 (4H, m), 7.53 (2H, d, J=8.4Hz), 8.18 (2H, d, J=8.4Hz).

### Reference Example 11

In ethanol (300ml) was dissolved 4-(4-nitrobenzyl)-morpholine (25.8g), and to the mixture was added dried 10% palladium on carbon (Pd-C) (1.00g) . Under hydrogen atmosphere, the mixture was stirred at room temperature under atmospheric pressure for 20 hours. The palladium was filtered off, and the filtrate was concentrated. The residue was separated and purified with column chromatography (ethyl acetate) to give 4-(4-aminobenzyl)-morpholine (430mg) as pale yellow crystals.
mp 98-99°C

| Elemental Analysis for C₁₁H₁₆N₂O | | | |
|---|---|---|---|
| Calcd: | C, 68.72; | H, 8.39; | N, 14.57. |
| Found: | C, 68.57; | H, 8.25; | N, 14.59. |

IR (KBr) cm⁻¹: 3350, 2804, 1635, 1516, 1282, 1111, 1005, 860
¹H NMR (200MHz, CDCl₃) δ:2.32-2.52 (4H, m), 3.39 (2H, s), 3. 45-3.80 (6H, m), 6.64 (2H, d, J=8.2Hz), 7.09 (2H, d, J=8.2Hz).

### Reference Example 12

In THF (250ml) was dissolved 4-nitrobenzyl bromide (25.0g), and to the mixture was added pyrrolidine (24.1ml) at 0°C. The reaction mixture was stirred at room temperature for 60 hours. To the mixture was added water (500ml), and the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) to give 1-(4-nitrobenzyl)pyrrolidine (23.5g) as orange oil.
¹H NMR (200MHz, CDCl₃) δ : 1.75-1.85 (4H, m), 2.43-2.58 (4H, m), 3.71 (2H, s), 7.51 (2H, d, J=8.6Hz), 8.18 (2H, d, J=8.6Hz).

### Reference Example 13

In ethanol (100ml) was dissolved 1-(4-nitrobenzyl)-pyrrolidine (23.5g), and to the mixture was added dried 10% palladium on carbon (1.00g). Under hydrogen atmosphere, the mixture was stirred at room temperature under atmospheric pressure for 20 hours. The palladium was filtered off, and the filtrate was concentrated. The residue was separated and purified with column chromatography (ethyl acetate/triethylamine =10/1) to give 1-(4-aminobenzyl)pyrrolidine (8.54g) as orange oil.
¹H NMR (200MHz, CDCl₃) δ : 1.60-1.90 (4H, m), 2.35-2.55 (4H, m), 3.45-3.70 (4H, m), 6.64 (2H, d, J=8.4Hz), 7.11 (2H, d, J=8.4Hz).

### Reference Example 14

In THF (250ml) was dissolved 4-nitrobenzyl bromide (25.0g), and to the mixture was added 50% dimethylamine solution (29ml) at 0°C. The reaction mixture was stirred at room temperature for 60 hours. To the mixture was added water (500ml), and the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) to give dimethyl-4-nitrobenzylamine (20.7g) as orange oil.
¹H NMR (200MHz, CDCl₃) δ : 2.26 (6H, s), 3.52 (2H, s), 7.50 (2H, d, J=8.8Hz), 8.19 (2H, d, J=8.8Hz).

### Reference Example 15

In ethanol (100ml) was dissolved dimethyl-4-nitro-benzylamine (20.7g), and to the mixture was added dried 10% palladium on carbon (1.00g). Under hydrogen atmosphere, the mixture was stirred at room temperature under atmospheric pressure for 20 hours. The palladiumwas filtered off, and the filtrate was concentrated. The residue was separated and purified with column chromatography (ethyl acetate) to give 4-aminobenzyl-dimethylamine (8.75g) as pale yellow oil.
¹HNMR (200MHz, CDCl₃) δ : 2.21 (6H, s), 3.31 (2H, s), 3.53-3.70 (2H, br), 6.65 (2H, d, J=8.4Hz), 7.08 (2H, d, J=8.4Hz).

### Reference Example 16

In THF (250ml) was dissolved 3-nitrobenzyl chloride (25.0g), and to the mixture was added piperidine (36ml). The reaction mixture was stirred at room temperature for 20 hours. To the mixture was added water (500ml), and the mixture was extracted with ethyl acetate. The organic layer was washed with saturated sodium chloride solution, dried with anhydrous sodiumsulfate, and concentrated under reduced pressure. The residue was separated and purified with column chromatography (ethyl acetate) to give 1-(3-nitrobenzyl)piperidine (32.2g) as pale yellow oil.
¹H NMR (200MHz, CDCl₃) δ : 1.40-1.66 (6H, m), 2.33-2.44 (4H, m), 3.54 (2H, s), 7.47 (1H, t, J=8.0Hz), 7.67 (1H, d, J=8.0Hz), 8.10 (1H, d, J=8.0Hz), 8.20 (1H, s).

### Reference Example 17

In ethanol (100ml) was dissolved 1-(3-nitrobenzyl)-piperidine (32.2g), and to the mixture was added dried 10% palladium on carbon (1.61g). Under hydrogen atmosphere, the mixture was stirred at room temperature under atmospheric pressure for 24 hours. The palladium was filtered off, and the filtrate was concentrated. The residue was recrystallized from diisopropylether-hexane to give 1-(3-aminobenzyl)piperidine (15.8g) as colorless crystals.
mp 109-110°C

| Elemental Analysis for C₁₂H₁₈N₂ | | | |
|---|---|---|---|
| Calcd: | C, 75.74; | H, 9.53; | N, 14.72. |
| Found: | C, 75.81; | H, 9.13; | N, 14.87. |

IR (KBr) cm⁻¹: 3398, 3184, 2948, 1643, 1606, 1454, 1302, 1101, 995, 795, 775, 698
¹H NMR (200MHz, CDCl₃) δ: 1.35-1.65 (6H, m), 2.25-2.45 (4H, m), 3.38 (2H, s), 3.50-3.75 (2H, br), 6.57 (1H, brd, J=7.9Hz), 6.65-6.75 (2H, m), 7.08 (1H, t, J=7.9Hz).

### Reference Example 18

In DMF (100ml) was dissolved 4-(2-bromoethyl)nitrobenzene (25.0g), and to the solution were added piperidine (12.9ml) and potassium carbonate (18.0g). The mixture was stirred at 70°C for 15 hours, and to the mixture was added water (900ml), and then the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) to give 1-[2-(4-nitro-phenyl)ethyl]piperidine (24.8g) as orange oil.
¹H NMR (200MHz, CDCl₃) δ : 1.39-1.75 (6H, m), 2.35-2.65 (6H, m), 2.85-3.00 (2H, m), 7.36 (2H, d, J=8.8Hz), 8.14 (2H, d, J=8.8Hz).

### Reference Example 19

In ethanol (100ml) was dissolved 1- [2-(4-nitro-phenyl)ethyl]piperidine (24.8g), and to the mixture was added dried 10% palladium on carbon(1.24g). Under hydrogen atmosphere, the mixture was stirred at room temperature under atmospheric pressure for 86 hours. The palladiumwas filtered off, and the filtrate was concentrated to give 1-[2-(4-aminophenyl)ethyl]-piperidine (21.7g) as pale brown oil.
¹H NMR (200MHz, CDCl₃) δ : 1.40-1.80 (6H, m), 2.35-2.60 (6H, m), 2.60-2.80 (2H, m), 3.40-3.70 (2H, br), 6.62 (2H, d, J=8.4Hz), 7.00 (2H, d, J=8.4Hz).

### Reference Example 20

In methanol (35ml) was dissolved 7-phenyl-3,4-dihydro-naphthalene-2-carboxylic acid (1.50g), and to the mixture was added concentrated sulfuric acid (0.1ml), and then the mixture was refluxed for 9 hours. The reaction mixture was cooled to room temperature, and to the mixture was added 5% sodium hydrogen carbonate solution, and then the mixture was extracted with ethyl acetate. The organic layer was washed with saturated sodium chloride solution, dried with anhydrous sodium sulfate, and concentrated under reduced pressure. The residue was dissolved in ethyl acetate (100ml), and to the mixture was added activated manganese dioxide (9g). The mixture was refluxed for 48 hours and then cooled to room temperature. The manganese dioxide was filtered off, and the filtrate was concentrated. The residue was dissolved in methanol (15ml), and to the mixture was added 1N sodium hydroxide (10ml). The mixture was refluxed for 4 hours and then cooled to room temperature. The mixture was acidified with dilute hydrochloric acid, and extracted with ethyl acetate. The organic layer was washed with saturated sodium chloride solution, dried with anhydrous sodium sulfate, and concentrated under reduced pressure. The residue was recrystallized from ethyl acetate-diisopropylether to give 7-phenylnaphthalene-2-carboxylic acid (783mg) as colorless crystals.
mp 244-245°C

| Elemental Analysis for C₁₇H₁₂O₂ | | |
|---|---|---|
| Calcd: | C, 82.24; | H, 4.87. |
| Found: | C, 82.10; | H, 4.85. |

IR (KBr) cm⁻¹: 3053, 1701, 1684, 1429, 1302, 860, 756, 696 ¹H NMR (200MHz, CDCl₃) δ : 7.37-7.57 (3H, m), 7.70-7.77 (2H, m), 7.86-8.02 (3H, m), 8.10-8.20 (2H, m), 8.77 (1H, s).

### Reference Example 21

To a solution of 4-nitrobenzylalcohol (4.59g) in methanol (300ml) was added copper chloride (I) (17.8g) at room temperature, and then was gradually added potassium boron hydride (11.3g) for 40 minutes. The reaction mixture was stirred at room temperature for 2 hours and concentrated under reduced pressure. To the residue was added water, and the mixture was extracted with ethyl acetate. The organic layer was dried with anhydrous sodium sulfate, and concentrated under reduced pressure. The residue was separated and purified with column chromatography (ethyl acetate/hexane=3/1) to give 4-aminobenzylalcohol (1.31g) as pale yellow crystals.
mp 53-55°C

| Elemental Analysis for C₇H₉NO | | | |
|---|---|---|---|
| Calcd: | C, 68.27; | H, 7.37; | N, 11.37. |
| Found: | C, 68.43; | H, 7.43; | N, 11.49. |

IR (KBr) cm⁻¹: 3375, 3219, 1614, 1514, 1470, 1259, 1041, 854, 827, 748, 509
¹H NMR (200MHz, CDCl₃) δ : 3.50-3.85 (2H, br), 4.56 (2H, s), 6.68 (2H, d, J=8.4Hz), 7.17 (2H, d, J=8.4Hz).

### Reference Example 22

In THF (10ml) was dissolved 7-phenyl-3,4-dihydro-naphthalene-2-carboxylic acid (500mg), and to the solution were added oxalyl chloride (262 *µ*l) and a drop of DMF. The mixture was stirred at room temperature for 1 hour and concentrated under reduced pressure. The residue was dissolved in DMF (5ml), and to the mixture was dropwise added a solution of 4-aminobenzylalcohol (246mg) in pyridine (10ml) at 0°C. The reaction mixture was stirred at 0°C for 3 hours. To the mixture was added water (500ml), and then the mixture was extracted with ethyl acetate. The organic layer was washed with saturated sodium chloride solution, dried with anhydrous sodium sulfate, and concentrated under reduced pressure. The residue was recrystallized from ethyl acetate-acetone to give N-[4-(hydroxymethyl)phenyl]-7-phenyl-3,4-dihydronaphthalene-2-carboxamide (486mg) as pale brown crystals.
mp 207-210°C

| Elemental Analysis for C₂₄H₂₁NO₂ · 0.5H₂O | | | |
|---|---|---|---|
| Calcd: | C, 79.10; | H, 6.08; | N, 3.84. |
| Found: | C, 79.35; | H, 5.97; | N, 3.86. |

IR (KBr) cm⁻¹: 3332, 1651, 1618, 1597, 1527, 1412, 1317, 831, 764, 700
¹H NMR (200MHz, DMSO-d₆) δ: 2.50-2.66 (2H, m), 2.80-2.95 (2H, m), 4.46 (2H, s), 7.23-7.72 (13H, m), 9.91 (1H, s).

### Reference Example 23

Under argon atmosphere, a mixture of 7-(trifluoro-methanesulfoxy)-1-tetralone (9.02g), 4-methylphenylborate (5.00g),potassium carbonate (8.46g), toluene (300ml), ethanol (30ml) and water (30ml) was stirred at room temperature for 30 minutes, and to the mixture was added tetrakis(triphenylphosphine)palladium (1.06g). The mixture was refluxed for 14 hours. The reaction mixture was cooled to room temperature. The organic layer was separated, dried with anhydrous sodium sulfate, and concentrated under reduced pressure. The residue was separated and purified with column chromatography (ethyl acetate/toluene=1/10) to give 7-(4-methylphenyl)-1-tetralone (5.23g) as colorless crystals.
mp 86-87°C

| Elemental Analysis for C₁₇H₁₆O | | |
|---|---|---|
| Calcd: | C, 86.41; | H, 6.82. |
| Found: | C, 86.30; | H, 6.69. |

IR (KBr) cm⁻¹: 2947, 1682, 1606, 1489, 1435, 1323, 1223, 1178, 810
¹H NMR (200MHz, CDCl₃) δ : 2.10-2 .24 (2H, m), 2.39 (3H, s), 2.69 (2H, t, J=6.6Hz), 3.00 (2H, t, J=6.0Hz), 7.21-7.35 (3H, m), 7.52 (2H, d, J=8.4Hz), 7.71 (1H, dd, J=2.2, 8.2Hz), 8.27 (1H, d, J=2.2Hz).

### Reference Example 24

Under argon atmosphere, a mixture of 7-(trifluoro-methanesulfoxy)-1-tetralone (17.5g), 4-fluorophenylborate (10.0g), potassium carbonate (16.6g), toluene (500ml), ethanol (50ml) and water (50ml) was stirred at room temperature for 30 minutes, and to the mixture was added tetrakis(triphenylphosphine)palladium (2.08g). The mixture was refluxed for 14 hours. The reaction mixture was cooled to room temperature. The organic layer was separated, dried with anhydrous sodium sulfate, and concentrated under reduced pressure. The residue was separated and purified with column chromatography (ethyl acetate/toluene=1/10) to give 7-(4-fluorophenyl)-1-tetralone (13.8g) as brown oil.
¹H NMR (200MHz, CDCl₃) δ : 2.10-2.24 (2H, m), 2.70 (2H, t, J=6.6Hz), 3.01 (2H, t, J=6.0Hz), 7.07-7.19 (2H, m), 7.30 (1H, d, J=7.6Hz), 7.53-7.62 (2H, m),7.67 (1H, dd, J=2.2, 8.2Hz), 8.23 (1H, d, J=2.2Hz).

### Reference Example 25

A mixture of sodium methoxide (5.63g), dimethyl carbonate (33ml) and 7-(4-methylphenyl)-1-tetralone (4.93g) was refluxed for 30 minutes. The reaction mixture was cooled to 0°C, and to the mixture was gradually added 3N hydrochloric acid (80ml). The mixture was extracted with ethyl acetate. The organic layer was washed with saturated sodium chloride solution, dried with anhydrous sodium sulfate, and concentrated under reduced pressure. The residue was dissolved in THF (30ml), and to the mixture was added sodium boron hydride (494mg) at 0°C and then was dropwise added methanol (3ml) for 30 minutes. The reaction mixture was stirred at 0°C for 4 hours, and to the mixture was added water (500ml). The mixture was extracted with ethyl acetate. The organic layer was washed with saturated sodium chloride solution, dried with anhydrous sodium sulfate, and concentrated under reduced pressure. The residue was dissolved in methanol (20ml), and to the mixture was added 1N sodium hydroxide (20ml). The mixture was refluxed for 4 hours, cooled, acidified with concentrated hydrochloric acid, and extracted with ethyl acetate. The organic layer was washed with saturated sodium chloride solution, dried with anhydrous sodium sulfate, and concentrated under reduced pressure. The residue was dissolved in Diglyme (20ml), and to the mixture was added concentrated hydrochloric acid (4ml). The mixture was stirred at 100°C for 2 hours, and to the mixture was added water (500ml). The mixture was extracted with ethyl acetate. The organic layer was washed with saturated sodium chloride solution, and concentrated under reduced pressure. The residue was dissolved in 0.5N sodium hydroxide (400ml), and the mixture was washed with diethylether. The aqueous layer was separated and acidified with concentrated hydrochloric acid. The mixture was extracted with ethyl acetate. The organic layer was washed with saturated sodium chloride solution, dried with anhydrous sodium sulfate, and concentrated under reduced pressure.

The residue was recrystallized from ethyl acetate-diisopropylether to give 7-(4-methyl-phenyl)-3,4-dihydronaphthalene-2-carboxylic acid (1.96g) as pale brown crystals.
mp 230-231°C

| Elemental Analysis for C₁₈H₁₆O₂ | | |
|---|---|---|
| Calcd: | C, 81.79; | H, 6.10. |
| Found: | C, 81.62; | H, 6.11. |

IR (KBr) cm⁻¹: 3023, 2908, 1697, 1682, 1626, 1431, 1300, 928, 810
¹H NMR (200MHz, CDCl₃) δ : 2.40 (3H, s), 2.61-2.71 (2H, m), 2.89-2.98 (2H, m), 7.22-7.28 (3H, m), 7.45-7.51 (4H, m), 7.73 (1H, s).

### Reference Example 26

A mixture of sodium methoxide (15.5g), dimethyl carbonate (91ml) and 7-(4-fluorophenyl)-1-tetralone (13.8g) was refluxed for 30 minutes. The reaction mixture was cooled to 0°C, and to the mixture was gradually added 3N hydrochloric acid (200ml). The mixture was extracted with ethyl acetate. The organic layer was washed with saturated sodium chloride solution, dried with anhydrous sodium sulfate, and concentrated under reduced pressure. The residue was dissolved in THF (90ml), and to the mixture was added sodium boron hydride (1.36g) at 0°C and then was dropwise added methanol (9ml) for 30 minutes. The reaction mixture was stirred at 0°C for 4 hours, and to the mixture was added water (500ml). The mixture was extracted with ethyl acetate. The organic layer was washed with saturated sodium chloride solution, and concentrated under reduced pressure. The residue was dissolved in methanol (80ml), and to the mixture was added 1N sodium hydroxide (100ml). The mixture was refluxed for 4 hours and cooled to room temperature. The mixture was acidified with concentrated hydrochloric acid and extracted with ethyl acetate. The organic layer was washed with saturated sodium chloride solution, dried with anhydrous sodium sulfate, and concentrated under reduced pressure. The residue was dissolved in Diglyme (50ml), and to the mixture was added concentrated hydrochloric acid (10ml). The mixture was stirred at 100°C for 2 hours, and to the mixture was added water (500ml) . The mixture was extracted with ethyl acetate. The organic layer was washed with saturated sodium chloride solution, and concentrated under reduced pressure. The residue was dissolved in 0.5N sodium hydroxide (400ml), and the mixture was washed with diethylether. The aqueous layer was separated, acidified with concentrated hydrochloric acid and extracted with ethyl acetate. The organic layer was washed with saturated sodium chloride solution, dried with anhydrous sodium sulfate, and concentrated under reduced pressure. The residue was recrystallized from ethyl acetate-diisopropylether to give 7-(4-fluorophenyl)-3,4-dihydronaphthalene-2-carboxylic acid (6.01g) as pale brown crystals.
mp 213-214°C

| Elemental Analysis for C₁₇H₁₃O₂F | | |
|---|---|---|
| Calcd: | C, 76.11; | H, 4.88. |
| Found: | C, 76.02; | H, 4.97. |

IR (KBr) cm⁻¹: 2953, 1695, 1518, 1431, 1300, 1281, 1246, 930, 824
¹H NMR (200MHz, CDCl₃) δ : 2.61-2.72 (2H, m), 2.90-2.99 (2H, m), 7.08-7.19 (2H, m), 7.23-7.29 (1H, m), 7.41-7.58 (4H, m), 7.72 (1H, s).

### Reference Example 27

To a mixture of N-[4-(hydroxymethyl)phenyl]-7-phenyl-3,4-dihydronaphtha lene-2-carboxamide (566mg), lithium chloride (135mg), triethylamine (446 µl) and dichloromethane (50ml) was added methanesulfonyl chloride (172 µl), and the mixture was stirred at room temperature for 2 hours. To the reaction mixture was added dilute hydrochloric acid. The organic layer was separated, washed with saturated sodium chloride solution, dried with anhydrous sodium sulfate, and concentrated under reduced pressure. The residue was recrystallized from ethyl acetate-hexane to give N-[4-(chloromethyl)phenyl]-7-phenyl-3,4-dihydronaphthal ene-2-carboxamide (494mg) as colorless crystals.
mp 176-177°C

| Elemental Analysis for C₂₄H₂₀NOCl | | | |
|---|---|---|---|
| Calcd: | C, 77.10; | H, 5.39; | N, 3.75. |
| Found: | C, 76.95; | H, 5.47; | N, 3.82. |

IR (KBr) cm⁻¹ : 3327, 1649, 1618, 1527, 1412, 1317, 831, 764, 700
¹H NMR (200MHz, DMSO-d₆) δ: 2.55-2.68 (2H, m), 2.85-2.95 (2H, m), 4.74 (2H, s), 7.30-7.80 (13H, m), 10.05 (1H, s).

### Reference Example 28

A mixture of 4-nitrobenzylalcohol(10.0g), tert-butyl-dimethylsilyl chloride (11.8g), imidazole (11.2g) and DMF (50ml) was stirred at room temperature for 1.5 hours. To the mixture was added water (500ml), and the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/hexane= 1/7) to give tert-butyldimethyl-4-nitrobenzyloxysilane (17.5g) as pale yellow oil.
¹H NMR (200MHz, CDCl₃) δ: 0.13 (6H, s), 0.96 (9H, s), 4.83 (2H, s), 7.48 (2H, d, J=8.6Hz), 8.20 (2H, d, J=8.6Hz).

### Reference Example 29

In ethanol (80ml) was dissolved tert-butyldimethyl-4-nitrobenzyloxysilane (16.5g), and to the mixture was added dried 5% palladium on carbon (0.83g). Under hydrogen atmosphere, the mixture was stirred at room temperature under atmospheric pressure for 7.5 hours. The palladium was filtered off, and the filtrate was concentrated. The residue was separated and purified with column chromatography (ethyl acetate/hexane=1/4) to give 4-aminobenzyloxy-tert-butyldimethylsilane (13.8g) as colorless oil.
IR (neat) cm⁻¹ : 3359, 2954, 2856, 1626, 1518, 1471, 1375, 1257, 1072, 837, 777
¹H NMR (200MHz, CDCl₃) δ: 0.07(6H,s), 0.92 (9H, s), 3.50-3.70 (2H, br), 4.62 (2H, s), 6.65 (2H, d, J=8.4Hz), 7.11 (2H, d, J=8.4Hz).

### Reference Example 30

In THF (60ml) was dissolved 7-(4-methylphenyl)-3,4-dihydro-naphthalene-2-carboxylic acid (4.02g). To the solution were added oxalyl chloride (1.99ml) and a drop of DMF, and the mixture was stirred at room temperature for 1 hour and concentrated under reduced pressure. The residue was dissolved in THF (30ml), and to the mixture was dropwise added a solution of 4-amino-benzyloxy-tert-butyldimethylsilane (3.97g) and triethylamine (2.56ml) in THF (30ml) at room temperature. The reaction mixture was stirred at room temperature for 19 hours. To the mixture was added water (300ml), and the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/toluene/ hexane=1/5/5). The resulting oil was dissolved in acetone (60ml), and to the mixture was added 6N hydrochloric acid (2ml). The mixture was stirred at room temperature for 30 minutes. To the reaction mixture were added 0.5% sodium hydroxide (500ml) and diisopropylether (200ml), and the mixture was stirred at room temperature for 5 minutes. The resulting precipitate s was filtered and recrystallized from acetone-diisopropylether to give N-[4-(hydroxy-methyl)phenyl]-7-(4-methylphenyl)-3,4-dih ydro-naphthalene-2-carboxamide (4.54g) as pale brown crystals.
mp 219-220°C

| Elemental Analysis for C₂₅H₂₃NO₂ | | | |
|---|---|---|---|
| Calcd: | C, 81.27; | H, 6.27; | N, 3.79. |
| Found: | C, 81.23; | H,5.99; | N, 3.80. |

IR (KBr) cm⁻¹: 3315, 1647, 1618, 1597, 1531, 1414, 1321, 810
¹H NMR (200MHz, DMSO-d₆) δ : 2.35 (3H, s), 2.55-2.65 (2H, m), 2.83-2.93 (2H, m), 4.46 (2H, d, J=5.6Hz), 5.13 (1H, t, J=5.6Hz), 7.23-7.33 (5H, m), 7.44-7.58 (5H, m), 7.69 (2H, d, J=8.4Hz), 9.93 (1H, s).

### Reference Example 31

To a mixture of N-[4-(hydroxymethyl)phenyl]-7-(4-methylphenyl)-3,4-dihy dronaphthalene-2-carboxamide (2.20g), lithium chloride (505mg), triethylamine (1.67ml), DMAP [4-dimethylaminopyridine] (catalytic amount) and dichloromethane (200ml) was added methanesulfonyl chloride (645 µl), and the mixture was stirred at room temperature for 42 hours and concentrated under reduced pressure. To the residue was added 0.5N hydrochloric acid (200ml), and the mixture was extracted with ethyl acetate. The organic layer was dried with anhydrous sodium sulfate and concentrated under reduced pressure. The residue was recrystallized from ethyl acetate-hexane to give N-[4-(chloromethyl)-phenyl]-7-(4-methylphenyl)-3,4-dihy dronaphthalene-2-carboxamide (973mg) as colorless crystals.
mp 178-179°C

| Elemental Analysis for C₂₅H₂₂NOCl | | | |
|---|---|---|---|
| Calcd: | C, 77.41; | H, 5.72; | N, 3.61. |
| Found: | C, 77.34; | H, 5.89; | N, 3.65. |

IR (KBr) cm⁻¹ : 3332, 1651, 1620, 1529, 1412, 1319, 812
¹H NMR (200MHz, DMSO-d₆) δ : 2.35 (3H, s), 2.55-2.68 (2H, m), 2.83-2.93 (2H, m), 4.74 (2H, s), 7.24-7.60 (10H, m), 7.76 (2H, d, J=8.6Hz), 10.04 (1H, s).

### Reference Example 32

Under argon atmosphere, 6-methoxy-1-indanone (10.0g) was dissolved in xylene (100ml), and to the mixture was added aluminum chloride (16.4g) . The mixture was refluxed for 2 hours and then cooled to room temperature. To the mixture was added 3N hydrochloric acid (100ml), and the mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) to give 6-hydroxy-1-indanone (7.36g) as pale brown crystals.
¹H NMR (200MHz, CDCl₃) δ : 2.67-2.76 (2H, m), 3.02-3.11 (2H, m), 5.61 (1H, s), 7.10-7.21 (2H, m), 7.36 (1H, d, J=8.0Hz).

### Reference Example 33

Under argon atmosphere, 6-hydroxy-1-indanone (7.36g) and triethylamine (20.9ml) were dissolved in dichloro-methane (120ml), and to the mixture was dropwise added trifluoromethanesulfonic acid anhydride (8.78ml) at 0°C. The reaction mixture was stirred at 0°C for 1 hour, and to the mixture was added water (200ml). The organic layer was separated, washed with water, dried with anhydrous sodium sulfate and concentrated under reduced pressure. The residue was separated and purified with column chromatography (ethyl acetate/hexane=1/4) to give 6-(trifluoromethane-sulfoxy)-1-indanone (11.5g) as brown oil.
¹H NMR (200MHz, CDCl₃) δ: 2.75-2.83 (2H, m), 3.17-3.24 (2H, m), 7.50 (1H, dd, J=2.4, 8.4Hz), 7.60 (1H, d, J=8.4Hz), 7.64 (1H, d, J=2.4Hz).

### Reference Example 34

Under argon atmosphere, a mixture of 6-(trifluoro-methanesulfoxy)-1-indanone (11.5g), 4-methylphenylborate (6.69g), potassium carbonate (11.3g), toluene (400ml), ethanol (40ml) and water (40ml) was stirred at room temperature for 30 minutes, and to the mixture was added tetrakis(triphenylphosphine)palladium (1.42g). The mixture was refluxed for 17 hours and cooled to room temperature. The organic layer was separated, dried with anhydrous sodium sulfate, and concentrated under reduced pressure. The residue was separated and purified with column chromatography (ethyl acetate/toluene=1/10) and recrystallized from ethyl acetate-hexane to give 6-(4-methylphenyl)-1-indanone (5.20g) as pale brown crystals.
mp 121-122°C

| Elemental Analysis for C₁₆H₁₄O | | |
|---|---|---|
| Calcd: | C, 86.45; | H, 6.35. |
| Found: | C, 86.46; | H,6.23. |

IR (KBr) cm⁻¹: 1703, 1614, 1483, 1448, 1404, 1304, 814
¹H NMR (200MHz, CDCl₃) δ: 2.40 (3H, s), 2.70-2.79 (2H,m), 3.13-3.22 (2H, m), 7.23-7.29 (2H, m), 7.48-7.57 (3H, m), 7.83 (1H, dd, J=1.8, 8.0Hz), 7.96 (1H, s).

### Reference Example 35

A solution of 6-(4-methylphenyl)-1-indanone (4.97g) in THF (33ml) was dropwise added to a refluxed mixture of 60% sodium hydride (3.26g), potassium hydride (catalytic amount), dimethyl carbonate (6.65ml) and THF (100ml), and the mixture was refluxed for 6 hours. The reaction mixture was cooled to 0°C, and to the mixture was gradually added 2N hydrochloric acid (150ml). The mixture was extracted with ethyl acetate, and the organic layer was washed with saturated sodium chloride solution, dried with anhydrous sodium sulfate, and concentrated under reduced pressure. The residue was separated and purified with column chromatography (ethyl acetate/toluene=1/3) to give a brown solid. The solid was dissolved in dichloromethane (100ml), and to the mixture was added sodium boron hydride (391mg) at 0°C and then was dropwise added methanol (10ml). The reaction mixture was stirred at 0°Cfor 1.5 hours, and to the mixture was added water (500ml). The mixture was extracted with ethyl acetate, and the organic layer was washed with saturated sodium chloride solution, dried with anhydrous sodium sulfate, and concentrated under reduced pressure. The residue was dissolved in methanol (30ml), and to the mixture was added 1N sodium hydroxide (40ml). The mixture was refluxed for 2 hours and cooled to room temperature. To the mixture was added water, and the mixture was washed with diethylether. The aqueous layer was acidified with concentrated hydrochloric acid and extracted with ethyl acetate. The organic layer was washed with saturated sodium chloride solution, dried with anhydrous sodium sulfate, and concentrated under reduced pressure. The residue was dissolved in Diglyme (30ml), and to the mixture was added concentrated hydrochloric acid (6ml). The mixture was stirred at 100°C for 2 hours, and to the solution were added 0.5% sodium hydrogen carbonate solution (500ml) and hexane (500ml) . The resulting precipitate was filtered to give 5-(4-methylphenyl)-indene-2-carboxylic acid (2.72g) as brown crystals.
mp 226-229°C (decomp.)

| Elemental Analysis for C₁₇H₁₄O₂ · 0.1H₂O | | |
|---|---|---|
| Calcd: | C, 80.99; | H, 5.68. |
| Found: | C, 80.92; | H,5.55. |

IR (KBr) cm⁻¹: 2999, 1670, 1572, 1259, 808
¹H NMR (200MHz, DMSO-d₆) δ : 2.35 (3H, s), 3.63-3.70 (2H, m), 7.28 (2H, d, J=8.0Hz), 7.53-7.73 (5H, m), 7.83 (1H, d, J=6.0Hz).

### Reference Example 36

A mixture of hexamethyleneimine (15.0g), ethyl iodide (14.5ml), potassium carbonate (31.3g) and ethanol (300ml) was refluxed for 6 hours and concentrated under reduced pressure. To the residue was added diethylether, and insoluble material was filtered off. The filtrate was under reduced pressure to give 1-ethylperhydroazepine (4.56g) as colorless oil.
bp 73-76°C/70mmHg
IR (neat) cm⁻¹: 2927, 1452, 1352, 1190, 1140, 1093
¹H NMR (200MHz, CDCl₃) δ : 1.05 (3H, t, J=7.2Hz), 1.55-1.72 (8H, m), 2.47-2.65 (6H, m).

### Reference Example 37

A mixture of hexamethyleneimine (15.0g), 1-propyl iodide (29.5ml), potassium carbonate (31.3g) and ethanol (300ml) was refluxed for 42 hours and concentrated under reduced pressure. To the residue was added diethylether, and insoluble material was filtered off. The filtrate was under reduced pressure to give 1-propylperhydroazepine (2.50g) as colorless oil.
bp 70-74°C/50mmHg
IR (neat) cm⁻¹: 2926, 1749, 1458, 1375, 1259, 1184, 1138, 1082
¹H NMR (200MHz, CDCl₃) δ : 0.87 (3H, t, J=7.5Hz), 1.40-1.80 (10H, m), 2.36-2.46 (2H, m), 2.55-2.67 (4H, m).

### Reference Example 38

A mixture of heptamethyleneimine (10.0g), ethyl iodide (8.48ml), potassium carbonate (18.3g) and ethanol (200ml) was refluxed for 13 hours and concentrated under reduced pressure. To the residue was added diethylether, and insoluble material was filtered off. The filtrate was under reduced pressure to give 1-ethylperhydroazocine (2.29g) as colorless oil.
bp 76-78°C/40mmHg
IR (neat) cm⁻¹: 2920, 1475, 1446, 1371, 1252, 1225, 1161, 1093
¹H NMR (200MHz, CDCl₃) δ : 1.03 (3H, t, J=6.9Hz), 1.48-1.72 (10H, m), 2.42-2.60 (6H, m).

### Reference Example 39

Under argon atmosphere, a mixture of methyl (E)-3-(trifluoromethanesulfoxy)cinnamate (9.00g), 4-methyl-phenylborate (4.73g), potassium carbonate (8.02g), toluene (300ml), ethanol (30ml) and water (30ml) was stirred at room temperature for 30 minutes. To the mixture was added tetrakis(triphenylphosphine)palladium (l:Olg), and the mixture was refluxed for 24 hours. The reaction mixture was cooled to room temperature, and the organic layer was separated, dried with anhydrous sodium sulfate, and concentrated under reduced pressure. The residue was separated and purified with column chromatography (ethyl acetate/toluene/hexane=1/5/5) to give colorless oil, which was dissolved in methanol (50ml). To the mixture was added 1N sodium hydroxide (50ml), and the mixture was refluxed for 1 hour. The reaction mixture was cooled to room temperature, acidified with concentrated hydro-chloric acid and extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-3-(4-methyl-phenyl)cinnamic acid (5.15g) as colorless crystals.
mp 192-194°C

| Elemental Analysis for C₁₆H₁₄O₂ · 0.1H₂O | | |
|---|---|---|
| Calcd: | C, 80.04; | H, 5.96. |
| Found: | C, 80.13; | H, 5.94. |

IR (KBr) cm⁻¹: 2922, 1687, 1628, 1435, 1321, 1282, 1225, 798
¹HNMR (200MHz, CDCl₃) δ :2.41 (3H, s), 6.52 (1H, d, J=16.0Hz), 7.23-7.30 (2H, m), 7.40-7.53 (4H, m), 7.56-7.65 (1H, m), 7.73 (1H, s), 7.85 (1H, d, J=16.0Hz).

### Reference Example 40

In THF (50ml) was dissolved (E)-3-(4-methylphenyl)-cinnamic acid (5.00g), and to the solution were added oxalyl chloride (2.38ml) and a drop of DMF. The mixture was stirred at room temperature for 1 hour and concentrated under reduced pressure. The residue was dissolved in THF (50ml), and to the mixture were added 4-aminobenzyloxy-tert-butyldimethylsilane (5.48g) and triethylamine (3.53ml) at room temperature. The reaction mixture was stirred at room temperature for 3 hours, and to the mixture was added water (200ml). The mixture was extracted with ethyl acetate, and the organic layer was washed with saturated sodium chloride solution, dried with anhydrous sodium sulfate, and concentrated under reduced pressure. The residue was separated and purified with column chromatography (ethyl acetate/toluene/hexane=1/5/5) to give oil, which was dissolved in acetone (50ml). To the mixture was added 6N hydrochloric acid (1ml), and the mixture was stirred at room temperature for 30 minutes. To the reaction mixture were added 0.5% sodium hydroxide (500ml) and diisopropylether (200ml), and the mixture was stirred at room temperature for 5 minutes. The resulting precipitate was filtered and recrystallized from acetone-diisopropylether to give (E)-N-[4-(hydroxymethyl)-phenyl]-3-(4-methylphenyl)-cin namamide (6.18g) as pale yellow crystals.
mp 220-223°C

| Elemental Analysis for C₂₃H₂₁NO₂ | | | |
|---|---|---|---|
| Calcd: | C, 80.44; | H, 6.16; | N, 4.08. |
| Found: | C, 80.12; | H, 6.15; | N, 4.00. |

IR (KBr) cm⁻¹: 3294, 1662, 1624, 1603, 1541, 1516, 1414, 1346, 1250, 1184, 999, 787
¹H NMR (200MHz, DMSO-d₆) δ : 2.36 (3H, s), 4.46 (2H, s), 6.93 (1H, d, J=15.4Hz), 7.22-7.33 (4H, m), 7.46-7.71 (8H, m), 7.89 (1H, s), 10.18 (1H, s).

### Reference Example 41

To a mixture of (E)-N-[4-(hydroxymethyl)phenyl]-3-(4-methylphenyl)cinna mamide (3.00g), lithium chloride (741mg), triethylamine (3.06ml), DMAP(catalytic amount) and dichloro-methane (300ml) was added methanesulfonyl chloride (1.15ml), and the mixture was stirred at room temperature for 13 hours. To the reaction mixture was added 4N hydrochloric acid ethyl acetate solution (3.3ml), and the mixture was purified with column chromatography (ethyl acetate) and recrystallized from ethyl acetate-diisopropylether to give (E)-N-[4-(chloromethyl)phenyl]-3-(4-methylphenyl)cinnamamide (2.00g) as colorless crystals.
mp 178-180°C

| Elemental Analysis for C₂₃H₂₀NOCl · 0.1H₂O | | | |
|---|---|---|---|
| Calcd: | C, 75.96; | H, 5.60; | N, 3.85. |
| Found: | C, 75.93; | H, 5.50; | N, 3.88. |

IR (KBr) cm⁻¹: 3344, 3045, 1664, 1628, 1531, 1412, 1338, 1248, 1176, 968, 793, 658
¹H NMR (200MHz, CDCl₃) δ : 2.41 (3H, s), 4.58 (2H, s), 6.61 (1H, d, J=15.6Hz), 7.25-7.31 (2H, m), 7.33-7.53 (7H, m), 7.55-7.67 (3H, m), 7.74 (1H, s), 7.83 (1H, d, J=15.6Hz).

### Reference Example 42

To a solution cooled at -78°C of 2-bromopyridine (10.0g) in diethylether (200ml) was dropwise added 1.6M butyllithium hexane solution (39.6ml) for 10 minutes. The mixture was stirred at -78°C for 1 hour, and to the mixture was dropwise added a solution of 4-nitrobenzaldehyde in THF (50ml). The reaction mixture was stirred at -78°C for 3 hours, and to the mixture was added water (100ml). The mixture was extracted with ethyl acetate, and the organic layer was washed with saturated sodium chloride solution, dried with anhydrous sodium sulfate, and concentrated under reduced pressure. The residue was separated and purified with column chromatography (ethyl acetate/toluene=1/2) and re-crystallized from diisopropylether to give (4-nitro-phenyl)-(2-pyridyl)methanol (4.50g) as orange crystals.
mp 114-115°C

| Elemental Analysis for C₁₂H₁₀N₂O₃ | | | |
|---|---|---|---|
| Calcd: | C, 62.61; | H, 4.38; | N, 12.17. |
| Found: | C, 62.61; | H, 4.27; | N, 12.16. |

IR (KBr) cm⁻¹: 3113, 2852, 1595, 1506, 1437, 1336, 1267, 1068, 1047, 1007, 847, 814, 777, 756, 743, 706
¹H NMR (200MHz, CDCl₃) δ : 5.44 (1H, br s), 5.86 (1H, s), 7.14-7.29 (2H, m), 7.55-7.73 (3H, m), 8.20 (2H, d, J=8.8Hz), 8.59 (1H, d, J=5.0Hz).

### Reference Example 43

In ethanol (50ml) was dissolved (4-nitrophenyl)-(2-pyridyl)methanol (2.30g), and to the mixture was added dried 10% palladium on carbon (0.12g). Under hydrogen atmosphere, the mixture was stirred at room temperature under atmospheric pressure for 19 hours. The palladiumwas filtered off, and the filtrate was concentrated. The residue was recrystallized from ethyl acetate-hexane to give (4 -aminophenyl) (2 -pyridyl) methanol (1.90g) as pale yellow crystals.
mp 139-140°C

| Elemental Analysis for C₁₂H₁₂N₂O | | | |
|---|---|---|---|
| Calcd: | C, 71.98; | H, 6.04; | N, 13.99. |
| Found: | C, 71.76; | H, 6.01; | N, 13.82. |

IR (KBr) cm⁻¹ : 3292, 1612, 1589, 1512, 1473, 1439, 1263, 1055, 816, 752, 569
¹H NMR (200MHz, CDCl₃) δ : 3.65 (2H, br s), 5.14 (1H, br s), 5.65 (1H, s), 6.65 (2H, d, J=8.8Hz), 7.10-7.22 (4H, m), 7.61 (1H, dt, J=1.8, 7.6Hz) 8.55 (1H, d, J=4.8Hz).

### Reference Example 44

Under argon atmosphere, ethyl 3-hydroxycinnamate (mp 88-89°C ; 20.0g) and triethylamine (34.5ml) were dissolved in dichloromethane (200ml), and to the mixture was dropwise added trifluoromethanesulfonic acid anhydride (31.6g) at -5°C for 40 minutes. The reaction mixture was stirred at -5°C to 0°C for 20 minutes, and to the mixture was added water (200ml). The organic layer was separated, washed with saturated sodium chloride solution, dried with anhydrous magnesium sulfate and concentrated under reduced pressure. The residue was separated and purified with column chromatography (ethyl acetate/hexane=1/4) and crystallized from hexane to give ethyl 3-(trifluoro-methane-sulfoxy)cinnamate (33.5g).
mp 52-53°C
¹H NMR (200MHz, CDCl₃) δ : 3.83 (3H, s), 6.48 (1H, d, J=16.0Hz), 7.30 (1H, m), 7.41 (1H, t, J=1.6Hz), 7.51 (2H, m), 7.67 (1H, d, J=16.0Hz).

### Reference Example 45

Under argon atmosphere, a mixture of ethyl 3-(trifluoromethanesulfoxy)cinnamate (3.10g), 4-methyl-phenylborate (1.63g),potassium carbonate (2.76g), toluene (100ml), ethanol (10ml) and water (10ml) was stirred at room temperature for 30 minutes. To the mixture was added tetrakis(triphenylphosphine)palladium (0.46g), and the mixture was refluxed for 18 hours. The reaction mixture was cooled to room temperature. The organic layer was separated, washed with saturated sodium chloride solution, dried with anhydrous magnesium sulfate and concentrated under reduced pressure. The residue was separated and purified with column chromatography (ethyl acetate/hexane=1/6) to give ethyl 3-(4-methylphenyl)-cinnamate (2.21g) as colorless oil. The oil (2.20g) was dissolved in tetrahydrofuran (20ml). To the mixture was added 2N sodium hydroxide (8.7ml), and the mixture was stirred at 50°C for 2 hours. The reaction mixture was cooled, acidified with potassium hydrogen sulfate and extracted with ethyl acetate. The organic layer was washed with saturated sodium chloride solution, dried with anhydrous magnesium sulfate and concentrated under reduced pressure. The residue was washed with isopropylether to give 3-(4-methylphenyl)-cinnamic acid (1.54g) as colorless crystals.
mp 186-187°C
¹H NMR (200MHz, CDCl₃) δ : 2.41 (3H, s), 6.53 (1H, d, J=16.0Hz), 7.28 (2H, d, J=7.4Hz), 7.46-7.52 (4H, m), 7.50 (1H, s), 7.63 (1H, m), 7.86 (1H, d, J=16.0Hz).

### Reference Example 46

Under argon atmosphere, a mixture of ethyl 3-(trifluoromethanesulfoxy)cinnamate (3.10g), 2-methyl-phenyl borate (mp 165-166°C; 1.63g), potassium carbonate (2.76g), toluene (100ml), ethanol (10ml) and water (10ml) was stirred at room temperature for 30 minutes. To the mixture was added tetrakis(triphenyl-phosphine)palladium (0.46g), and the mixture was refluxed for 18 hours. The reaction mixture was cooled to room temperature, and the organic layer was separated, washed with saturated sodium chloride solution, dried with anhydrous magnesium sulfate and concentrated under reduced pressure. The residue was separated and purified with column chromatography (ethyl acetate/hexane= 1/6) to give ethyl 3-(4-methylphenyl)-cinnamate (2.51g) as pale yellow oil. The oil (2.50g) was dissolved in tetrahydrofuran (20ml). To the mixture was added 2N sodium hydroxide (10.0ml), and the mixture was stirred at 50°C for 2 hours. The reaction mixture was cooled, acidified with potassium hydrogen sulfate and extracted with ethyl acetate. The organic layer was washed with saturated sodium chloride solution, dried with anhydrous magnesium sulfate and concentrated under reduced pressure. The residue was washed with isopropylether to give 3-(2-methylphenyl)cinnamic acid (1.96g) as colorless crystals.
mp 124-125°C
¹HNMR (200MHz, CDCl₃) δ: 2.27 (3H, s), 6.49 (1H, d, J=16.0Hz), 7.23-7.30 (4H, m), 7.36-7.57 (4H, m), d, J=7.4Hz), 7.84 (1H, d, J=16.0Hz).

### Reference Example 47

Under argon atmosphere, a mixture of ethyl 3-(trifluoro-methanesulfoxy)cinnamate (3.10g), 2,5-dimethylphenyl borate (mp 184-186°C; 1.80g), potassium carbonate (2.76g), toluene (100ml), ethanol (10ml) and water (10ml) was stirred at room temperature for 30 minutes. To the mixture was added tetrakis(triphenylphosphine)-palladium (0.46g), and the mixture was refluxed for 27 hours. The reaction mixture was cooled to room temperature, and the organic layer was separated, washed with saturated sodium chloride solution, dried with anhydrous magnesium sulfate and concentrated under reduced pressure. The residue was separated and purified with column chromatography (ethyl acetate/hexane= 1/6) to give ethyl 3-(2,5-dimethylphenyl)cinnamate (2.66g) as pale yellow oil. The oil (2.50g) was dissolved in tetrahydrofuran (20ml), and to the mixture was added 2N sodium hydroxide (10.0ml). Themixturewasstirredat50°C for 2 hours, cooled, acidified with potassium hydrogen sulfate and extracted with ethyl acetate. The organic layer was washed with saturated sodium chloride solution, dried with anhydrous magnesium sulfate and concentrated under reduced pressure. The residue was washed with isopropylether to give 3-(2,5-dimethylphenyl)cinnamic acid (1.96g) as colorless crystals.
mp 156-157°C
¹H NMR (200MHz, CDCl₃) δ: 2.23 (3H, s), 2.60 (3H, s), 6.49 (1H, d, J=16.0Hz), 7.06 (1H, s), 7.14 (2H, ABq, J=7.8Hz), 7.35-7.55 (4H, m), 7.36-7.57 (4H, m), 7.84 (1H, d, J=16.0Hz).

### Reference Example 48

Under argon atmosphere, a mixture of ethyl 3-(trifluoromethanesulfoxy)cinnamate (3.10g), 3-nitro-phenylborate (2.00g), potassium carbonate (2.76g), toluene (100ml), ethanol (10ml) and water (10ml) was stirred at room temperature for 30 minutes. To the mixture was added tetrakis(triphenylphosphine)palladium (0.46g), and the mixture was refluxed for 24 hours. The reaction mixture was cooled to room temperature. The organic layer was separated, washed with saturated sodium chloride solution, dried with anhydrous magnesium sulfate and concentrated under reduced pressure. The residue was separated and purified with column chromatography (ethyl acetate/hexane=1/6) to give ethyl 3-(3-nitrophenyl)-cinnamate (2.40g) aspaleyellowcrystals. The crystals (2.40g) were dissolved in tetrahydrofuran (20ml), and to the mixture was added 2N sodium hydroxide (8.5ml). The mixture was stirred at 50°C for 2 hours, cooled, acidified with potassium hydrogen sulfate and extracted with ethyl acetate. The organic layer was washed with saturated sodium chloride solution, dried with anhydrous magnesium sulfate and concentrated under reduced pressure. The residue was washed with isopropylether to give 3-(3-nitrophenyl)cinnamic acid (1.88g) as pale yellow crystals.
mp247-248°C
¹H NMR (200MHz, DMSO-d₆) δ : 6.59 (1H, d, J=16.0Hz), 7.51-7.76 (4H, m), 7.70 (1H, d, J=16.0Hz), 7.96 (1H, d, J=9.0Hz), 8.09 (1H, m), 8.22 (1H, m), 8.49 (1H, d, J=1.8Hz).

### Working Example 1 (Production of Compound 1)

In THF (5ml) was dissolved 7-cyclohexyl-3,4-dihydronaphthalene-2-carboxylic acid (200mg), and to the solution were added oxalyl chloride (82 *µ*l) and a drop of DMF. The mixture was stirred at room temperature for 1 hour and concentrated under reduced pressure. The residue was dissolved in THF (5ml), and to the solution were added 1-(4-aminobenzyl)piperidine (164mg) and triethylamine (484 µl) at room temperature. The reaction mixture was stirred at room temperature for 3 hours, and to the mixture was added water (100ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give 7-cyclohexyl-N-[4-(piperidinomethyl)-phenyl]-3,4-dihydr onaphthalene-2-carboxamide (Compound 1) (223mg) as colorless crystals.
mp 180-181°C

| Elemental Analysis for C₂₉H₃₆N₂O₂ | | | |
|---|---|---|---|
| Calcd: | C, 81.27; | H, 8.47; | N, 6.54. |
| Found: | C, 81.03; | H, 8.42; | N, 6.53. |

IR (KBr) cm⁻¹: 3430, 2931, 1645, 1597, 1514, 1412, 1317, 824
¹H NMR (200MHz, CDCl₃) δ : 1.20-1.90 (16H, m), 2.30-2.57 (5H, m), 2.60-2.72 (2H, m), 2.85-2.97 (2H, m), 3.46 (2H, s), 7.05-7.15 (3H, m), 7.25-7.34 (3H, m), 7.50-7.60 (3H, m).

### Working Example 2 (Production of Compound 2)

In DMF (2ml) was dissolved 7-cyclohexyl-N-[4-(piperidinomethyl)phenyl]-3,4-dihydro naphthalene-2-carboxamide (120mg), and to the mixture was added methyl iodide (45 µl). The mixture was stirred at room temperature for 24 hours and concentrated under reduced pressure. The residue was recrystallized from ethyl acetate to give 1-[4-(7-cyclohexyl-3,4-dihydro-naphthalene-2-carboxamid O)benzyl]-1-methylpiperidinium iodide (Compound 2) (148mg) as colorless crystals.
mp 188-191°C

| Elemental Analysis for C₃₀H₃₉N₂OI | | | | |
|---|---|---|---|---|
| Calcd: | C, 63.15; | H, 6.89; | N, 4.91; | I, 22.24. |
| Found: | C, 63.03; | H, 6.93; | N, 5.03; | I, 22.22. |

IR (KBr) cm⁻¹: 3430, 2929, 1649, 1599, 1520, 1417, 1321, 1248
¹H NMR (200MHz, DMSO-d₆) δ : 1.20-1.90 (16H, m), 2.40-2.65 (3H, m), 2.75-2.95 (5H, m), 3.20-3.45 (4H, m), 4.53 (2H, s), 7.14 (3H, s), 7.38 (1H, s), 7.49 (2H, d, J=8.6Hz), 7.88 (2H, d, J=8.6Hz), 10.12 (1H, s).

### Working Example 3 (Production of Compound 3)

In THF (3ml) was dissolved 7-cyclohexyl-3,4-dihydronaphthalene-2-carboxylic acid (100mg), and to the solution were added oxalyl chloride (41 *µ*l) and a drop of DMF. The mixture was stirred at room temperature for 1 hour and concentrated under reduced pressure. The residue was dissolved in THF (3ml), and to the solution were added p-(4-aminobenzyl)-N,N'-diethyl-phosphondiamide (104mg) and triethylamine (60 µl) at room temperature. The reaction mixture was stirred at room temperature for 72 hours, and to the mixture was added water (100ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/methanol =10/1) and was recrystallized from diisopropylether to give 7-cyclohexyl-N-[4-[bis(ethylamino)phosphorylmethyl]-phe nyl]-3,4-dihydronaphthalene-2-carboxamide (Compound 3) (140mg) as colorless crystals.
mp 163-165°C

| Elemental Analysis for C₂₈H₃₈N₃O₂P | | | |
|---|---|---|---|
| Calcd: | C, 70.12; | H, 7.99; | N, 8.76. |
| Found: | C, 70.01; | H, 7.99; | N, 8.93. |

IR (KBr) cm⁻¹ : 3250, 2926, 1645, 1599, 1514, 1414, 1321, 1250, 1182, 1126
¹H NMR (200MHz, CDCl₃) δ: 1.10 (6H, t, J=7.1Hz), 1.20-1.90 (10H, m), 1.95-2.20 (2H, m), 2.40-2.57 (1H, m), 2.60-2.72 (2H, m), 2.80-3.05 (7H, m), 3.12 (1H, s), 7.05-7.15 (3H, m), 7.22-7.32 (3H, m), 7.59 (2H, d, J=8.2Hz), 7.83 (1H, s).

### Working Example 4 (Production of Compound 4)

In THF (20ml) was dissolved 7-phenyl-3,4-dihydro-naphthalene-2-carboxylic acid (1.00g), and to the solution were added oxalyl chloride (523 µl) and a drop of DMF. The mixture was added at room temperature for 1 hour and concentrated under reduced pressure. The residue was dissolved in THF (20ml), and to the solution were added 1-(4-aminobenzyl)piperidine (837mg) and triethylamine (673 *µ*l) at room temperature.

The reaction mixture was stirred at room temperature for 2 hours, and to the mixture was added water (150ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give 7-phenyl-N-[4-(piperidinomethyl)phenyl]-3,4-dihydro-nap hthalene-2-carboxamide (Compound 4) (1.15g) as pale brown crystals.
mp 163-164°C

| Elemental Analysis for C₂₉H₃₀N₂O · 0.1H₂O | | | |
|---|---|---|---|
| Calcd: | C, 82.08; | H, 7.17; | N, 6.60. |
| Found: | C, 81.94; | H, 7.22; | N, 6.49. |

IR (KBr) cm⁻¹: 3336, 2935, 1651, 1527, 1412, 1317, 762, 698 ¹H NMR (200MHz, CDCl₃) δ: 1.35-1.70 (6H, m), 2.30-2.45 (4H, m), 2.65-2.80 (2H, m), 2.92-3.04 (2H, m), 3.46 (2H, s), 7.23-7.62 (14H, m).

### Working Example 5 (Production of Compound 5)

In DMF (3ml) was dissolved 7-phenyl-N- [4- (piperidino-methyl)phenyl]-3,4-dihydronaphthalene-2-carboxamide (240mg), and to the mixture was added methyl iodide (106 *µ*l). The mixture was stirred at room temperature for 60 hours and concentrated under reduced pressure. The residue was recrystallized from ethyl acetate to give 1-methyl-1-[4-(7-phenyl-3,4-dihydro-naphthalene-2-carbo xamido)benzyl]piperidinium iodide (Compound 5) (247mg) as colorless crystals.
mp 183-186°C

| Elemental Analysis for C₃₀H₃₃N₂OI | | | |
|---|---|---|---|
| Calcd: | C, 63.83; | H, 5.89; | N, 4.96. |
| Found: | C, 63.54; | H, 5.82; | N, 5.05. |

IR (KBr) cm⁻¹: 3450, 1649, 1599, 1520, 1417, 1319
¹H NMR (200MHz, DMSO-d₆) δ: 1.40-2.00 (6H, m), 2.55-2.70 (2H, m), 2.80-3.00 (5H, m), 3.20-3.45 (4H, m), 4.53 (2H, s), 7.30-7.70 (11H, m), 7.89 (2H, d, J=8.6Hz), 10.18 (1H, s) .

### Working Example 6 (Production of Compound 6)

In THF (10ml) was dissolved 7-phenyl-3,4-dihydro-naphthalene-2-carboxylic acid (500mg), and to the solution were added oxalyl chloride (262 µl) and a drop of DMF. The mixture was stirred at room temperature for 1 hour and concentrated under reduced pressure. The residue was dissolved in THF (10ml), and to the solution were added 4-aminobenzyldimethylamine (330mg) and triethylamine (337 *µ*l) at room temperature. The reaction mixture was stirred at room temperature for 3 hours, and to the mixture was added water (100ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/triethylamine=20/1) and recrystallized from ethyl acetate-hexane to give N-[4-(dimethylaminomethyl)-phenyl]-7-phenyl-3,4-dihydro -naphthalene-2-carboxamide (Compound 6) (131mg) as colorless crystals.
mp 182-184°C

| Elemental Analysis for C₂₆H₂₆N₂O · 0.2H₂O | | | |
|---|---|---|---|
| Calcd: | C, 80.88; | H, 6.89; | N, 7.26. |
| Found: | C, 81.00; | H, 6.90; | N, 7.19. |

IR (KBr) cm⁻¹: 3328, 1649, 1529, 1410, 1317, 762, 698
¹H NMR (200MHz, CDCl₃) δ: 2.24 (6H, s), 2.65-2.80 (2H, m), 2.94-3.03 (2H, m), 3.41 (2H, s), 7.25-7.63 (14H, m).

### Working Example 7 (Production of Compound 7)

In THF (10ml) was dissolved 7-phenyl-3,4-dihydro-naphthalene-2-carboxylic acid (500mg), and to the solution were added oxalyl chloride (262 µl) and a drop of DMF. The mixture was stirred at room temperature for 1 hour and concentrated under reduced pressure. The residue was dissolved in THF (10ml), and to the solution were added 1-(4-aminobenzyl)pyrrolidine (388mg) and triethylamine (337 *µ*l) at room temperature. The reaction mixture was stirred at room temperature for 3 hours, and to the mixture was added water (100ml). The mixture was extracted with ethyl acetate. The organic layer was washed with saturated sodium chloride solution, dried with anhydrous sodium sulfate,and concentrated under reduced pressure. The residue was separated and purified with column chromatography (ethyl acetate/ triethylamine=20/1) and recrystallized from ethyl acetate-diisopropylether to give 7-phenyl-N-[4-(1-pyrrolidinylmethyl)phenyl]-3,4-dihydro naphthalene-2-carboxamide (Compound 7) (107mg) as colorless crystals.
mp 186-187°C

| Elemental Analysis for C₂₈H₂₈N₂O · 0.1H₂O | | | |
|---|---|---|---|
| Calcd: | C, 81.96; | H, 6.93; | N, 6.83. |
| Found: | C, 81.78; | H, 6.84; | N, 6.89. |

IR (KBr) cm⁻¹: 3329, 2962, 1649, 1529, 1410, 1319, 762, 698 ¹H NMR (200MHz, CDCl₃) δ: 1.75-1.85 (4H, m), 2.45-2.55 (4H, m), 2.65-2.80 (2H, m), 2.90-3.05 (2H, m), 3.60 (2H, s), 7.25-7.60 (14H, m).

### Working Example 8 (Production of Compound 8)

In THF (10ml) was dissolved 7-phenyl-3,4-dihydro-naphthalene-2-carboxylic acid (500mg), and to the solution were added oxalyl chloride (262 *µ*l) and a drop of DMF. The mixture was stirred at room temperature for 1 hour and concentrated under reduced pressure. The residue was dissolved in THF (10ml), and to the solution were added 1-(4-aminobenzyl)morpholine (423mg) and triethylamine (337 *µ*l) at room temperature. The reaction mixture was stirred at room temperature for 2 hours, and to the mixture was added water (100ml). The mixture was extracted with ethyl acetate. The organic layer was washed with saturated sodium chloride solution, dried with anhydrous sodium sulfate,and concentrated under reduced pressure. The residue was separated and purified with column chromatography (ethyl acetate) and recrystallized from ethyl acetate-hexane to give N-[4-(morpholinomethyl)-phenyl]-7-phenyl-3,4-dihydronap hthalene-2-carboxamide (659mg) as colorless crystals.
mp 186-187°C

| Elemental Analysis for C₂₈H₂₈N₂O₂ | | | |
|---|---|---|---|
| Calcd: | C, 79.22; | H, 6.65; | N, 6.60. |
| Found: | C, 78.89; | H, 6.50; | N, 6.66. |

IR (KBr) cm⁻¹: 3450, 1651, 1620, 1597, 1527, 1412, 1319, 1113, 764, 700
¹H NMR (200MHz, CDCl₃) δ: 2.38-2.47 (4H, m), 2.66-2.78 (2H, m), 2.92-3.03 (2H, m), 3.48 (2H, s), 3.67-3.75 (4H, m), 7.25-7.60 (14H, m).

### Working Example 9 (Production of Compound 9)

In DMF (2ml) was dissolved N-[4-(dimethylamino-methyl)phenyl]-7-phenyl-3,4-dihydro naphthalene-2-carboxamide (80mg), and to the mixture was added methyl iodide (39 *µ*l). The mixture was stirred at room temperature for 17 hours and concentrated under reduced pressure. The residue was recrystallized from methanol-ethyl acetate to give trimethyl[4-(7-phenyl-3,4-dihydronaphthalene-2-carboxamido)benzyl]ammonium iodide (Compound 9) (92mg) as colorless crystals.
mp 190-192°C

| Elemental Analysis for C₂₇H₂₉N₂OI · 0.5H₂O | | | |
|---|---|---|---|
| Calcd: | C, 60.79; | H, 5.67; | N, 5.25. |
| Found: | C, 60.81; | H, 5.59; | N, 5.30. |

IR (KBr) cm⁻¹: 3450, 1662, 1595, 1520, 1483, 1416, 1319, 1250, 764, 700
¹H NMR (200MHz, CDCl₃) δ : 2.65-2.80 (2H, m), 2.80-2.95 (2H, m), 3.23 (9H, s), 4.98 (2H, s), 7.18 (1H, d, J=8.0Hz), 7.30-7.60 (9H, m), 7.69 (1H, s), 7.82-7.90 (2H, m), 8.71 (1H, s).

### Working Example 10 (Production of Compound 10)

In DMF (2ml) was dissolved 7-phenyl-N-[4-(1-pyrrolidinylmethyl)phenyl]-3,4-dihydro naphthalene-2-carboxamide (70mg), and to the mixture was added methyl iodide (32 *µ*l). The mixture was stirred at room temperature for 17 hours and concentrated under reduced pressure. The residue was recrystallized from methanol-ethyl acetate to give 1-methyl-1-[4-(7-phenyl-3,4-dihydronaphthalene-2-carbox amido)benzyl]pyrrolidinium iodide (Compound 10) (78mg) as pale yellow crystals.
mp 156-160°C

| Elemental Analysis for C₂₉H₃₁N₂OI · 1.0H₂O | | | |
|---|---|---|---|
| Calcd: | C, 61.27; | H, 5.85; | N, 4.93. |
| Found: | C, 61.23; | H, 5.89; | N, 5.04. |

IR (KBr) cm⁻¹: 3442, 1655, 1593, 1520, 1416, 1317, 1248, 766, 700
¹H NMR (200MHz, CDCl₃) δ: 2.05-2.40 (4H, m), 2.65-2.76 (2H, m), 2.82-2.95 (2H, m), 3.05 (3H, s), 3.43-3.57 (2H, m), 3.80-4.00 (2H, m), 4.98 (2H, s), 7.18 (1H, d, J=8.0Hz), 7.30-7.56 (9H, m), 7.70 (1H, s), 7.80-7.90 (2H, m), 8.74 (1H, s).

### Working Example 11 (Production of Compound 11)

In DMF (4ml) was dissolved N-[4-(morpholinomethyl)-phenyl]-7-phenyl-3,4-dihydronaphthalene-2-carboxamide (450mg), and to the mixture was added methyl iodide (198 *µ*l). The mixture was stirred at room temperature for 18 hours and concentrated under reduced pressure. The residue was recrystallized from ethyl acetate to give 4-methyl-4-[4-(7-phenyl-3,4-dihydro-naphthalene-2-carbo xamido)benzyl]morpholinium iodide (Compound 11) (575mg) as pale yellow crystals.
mp 166-170°C

| Elemental Analysis for C₂₉H₃₁N₂O₂I · 0.5H₂O | | | |
|---|---|---|---|
| Calcd: | C, 60.53; | H, 5.60; | N, 4.87. |
| Found: | C, 60.41; | H, 5.61; | N, 4.74. |

IR (KBr) cm⁻¹ : 3450, 1653, 1593, 1520, 1481, 1416, 1317, 1246, 1122, 887, 764, 698
¹H NMR (200MHz, CDCl₃) δ : 2.60-2.75 (2H, m), 2.75-2.90 (2H, m), 3.22 (3H, s), 3.35-3.50 (2H, m), 3.55-3.75 (2H, m), 3.80-4.05 (4H, m), 5.13 (2H, s), 7.12 (1H, d, J=7.6Hz), 7.25-7.55 (9H, m), 7.71 (1H, s), 7.80-7.87 (2H, m), 8.95 (1H, s).

### Working Example 12 (Production of Compound 12)

In THF (10ml) was dissolved 7-(4-methylphenyl)-3,4-dihydronaphthalene-2-carboxylic acid (500mg), and to the solution were added oxalyl chloride (248 *µ*l) and a drop of DMF. The mixture was stirred at room temperature for 1 hour and concentrated under reduced pressure. The residue was dissolved in THF (10ml), and to the solution were added 1-(4-aminobenzyl)piperidine (396mg) and triethylamine (318 *µ*l) at room temperature. The reaction mixture was stirred at room temperature for 14 hours, and to the mixture was added water (100ml). The mixture was extracted with ethyl acetate. The organic layer was washed with saturated sodium chloride solution, dried with anhydrous sodium sulfate, andconcentratedunderreduced pressure. The residue was recrystallized from ethyl acetate-diisopropylether to give 7-(4-methylphenyl)-N-[4-(piperidinomethyl)phenyl]-3,4-d ihydronaphthalene-2-carboxamide (Compound 12) (616mg) as pale brown crystals.
mp 187-189°C

| Elemental Analysis for C₃₀H₃₂N₂O | | | |
|---|---|---|---|
| Calcd: | C, 82.53; | H, 7.39; | N, 6.42. |
| Found: | C, 82.26; | H, 7.36; | N, 6.37. |

IR (KBr) cm⁻¹ : 3310, 2931, 1643, 1599, 1527, 1412, 1315, 1255, 806
¹H NMR (200MHz, CDCl₃) δ : 1.38-1.65 (6H, m), 2.32-2.42 (7H, m), 2.65-2.77 (2H, m), 2.92-3.02 (2H, m), 3.46 (2H, s), 7.20-7.34 (6H, m), 7.40-7.58 (7H, m).

### Working Example 13 (Production of Compound 13)

In THF (10ml) was dissolved 7-(4-fluorophenyl)-3,4-dihydronaphthalene-2-carboxylic acid (500mg), and to the solution were added oxalyl chloride (243 µl) and a drop of DMF. The mixture was stirred at room temperature for 1 hour and concentrated under reduced pressure. The residue was dissolved in THF (10ml), and to the solution were added 1-(4-aminobenzyl)piperidine (389mg) and triethylamine (313 µl) at room temperature. The reaction mixture was stirred at room temperature for 14 hours, and to the mixture was added water (100ml). The mixture was extracted with ethyl acetate. The organic layer was washed with saturated sodium chloride solution, dried with anhydrous sodium sulfate,and concentrated under reduced pressure. The residue was recrystallized from ethyl acetate-diisopropylether to give 7-(4-fluorophenyl)-N-[4-(piperidinomethyl)phenyl]-3,4-d ihydronaphthalene-2-carboxamide (Compound 13) (736mg) as pale yellow crystals.
mp 175-176°C

| Elemental Analysis for C₂₉H₂₉N₂OF · 0.2H₂O | | | |
|---|---|---|---|
| Calcd: | C, 78.42; | H, 6.67; | N, 6.31. |
| Found: | C, 78.36; | H, 6.68; | N, 6.23. |

IR (KBr) cm⁻¹: 3329, 2935, 1649, 1595, 1518, 1319, 1244, 824
¹H NMR (200MHz, CDCl₃) δ : 1.35-1.65 (6H, m), 2.34-2.41 (4H, m), 2.67-2.77 (2H, m), 2.92-3.02 (2H, m), 3.46 (2H, s), 7.07-7.58 (13H, m).

### Working Example 14 (Production of Compound 14)

In DMF (3ml) was dissolved 7-(4-methylphenyl)-N-[4-(piperidinomethyl)phenyl]-3,4-d ihydronaphthalene-2-carboxamide (400mg), and to the mixture was added methyl iodide (171 *µ*l). The mixture was stirred at room temperature for 18 hours and concentrated under reduced pressure. The residue was recrystallized from ethyl acetate to give 1-methyl-1-[4-[7-(4-methylphenyl)-3,4-dihydronaphthalen e-2-carboxamido] benzyl] piperidinium iodide (Compound 14) (490mg) as colorless crystals.
mp 202-204°C

| Elemental Analysis for C₃₁H₃₅N₂OI · 0.5H₂O | | | |
|---|---|---|---|
| Calcd: | C, 63.37; | H, 6.18; | N, 4.77. |
| Found: | C, 63.69; | H, 5.98 ; | N, 4.87. |

IR (KBr) cm⁻¹: 3450, 3294, 2941, 1649, 1622, 1599, 1520, 1417, 1319, 1248, 812
¹H NMR (200MHz, DMSO-d₆) δ: 1.40-2.00 (6H, m), 2.35 (3H, s), 2.55-2.67 (2H, m), 2.82-2.95 (5H, m), 3.22-3.35 (4H, m), 4.53 (2H, s), 7.24-7.35 (3H, m), 7.46-7.60 (7H, m), 7.89 (2H, d, J=8.8Hz), 10.15 (1H, s).

### Working Example 15 (Production of Compound 15)

In DMF (3ml) was dissolved 7- (4-fluorophenyl) -N- [4- (piperidinomethyl)phenyl] -3,4-d ihydronaphthalene-2-carboxamide (500mg), and to the mixture was added methyl iodide (212 µl) . The mixture was stirred at room temperature for 18 hours and concentrated under reduced pressure. The residue was recrystallized from ethyl acetate to give 1-[4-[7-(4-fluoro-phenyl)-3,4-dihydro-naphthalene-2-car boxamido]benzyl]-1-methylpiperidiniumiodide (Compound 15) (610mg) as colorless crystals.
mp 177-180°C

| Elemental Analysis for C₃₀H₃₂N₂OFI · 0.2H₂O | | | |
|---|---|---|---|
| Calcd: | C, 61.48; | H, 5.57; | N, 4.78. |
| Found: | C, 61.38; | H, 5.50; | N, 4.81. |

IR (KBr) cm⁻¹: 3450, 3310, 2947, 1651, 1597, 1518, 1416, 1319, 1246, 1225, 824
¹H NMR (200MHz, DMSO-d₆) δ: 1.40-2.00 (6H, m), 2.55-2.67 (2H, m), 2.85-2.96 (5H, m), 3.20-3.38 (4H, m), 4.53 (2H, s), 7.25-7.38 (3H, m), 7.46-7.60 (5H, m), 7.67-7.76 (2H, m), 7.89 (2H, d, J=8.6Hz), 10.17 (1H, s).

### Working Example 16 (Production of Compound 16)

To a mixture of N-[4-(hydroxymethyl)phenyl]-7-phenyl-3,4-dihydronaphtha lene-2-carboxamide (200mg), triethylamine (158 µl) and THF (10ml) was added methanesulfonic acid anhydride (118mg) at 0°C, and the mixture was stirred at room temperature for 3 hours. To the reaction mixture was added dilute hydrochloric acid, and the mixture was extracted with ethyl acetate. The organic layer was washed with saturated sodium chloride solution, dried with anhydrous sodium sulfate, and concentrated under reduced pressure. The residue was dissolved in DMF (3ml), and to the mixture was added pyridine (137 µl). The mixture was stirred at room temperature for 96 hours and concentrated under reduced pressure. The residue was recrystallized from ethyl acetate-methanol to give 1-[4-(7-phenyl-3,4-dihydronaphthalene-2-carboxamido)-be nzyl]pyridinium chloride (Compound 16) (95mg) as colorless crystals.
mp 162-164°C

| Elemental Analysis for C₂₉H₂₅N₂OCl · 1.0H₂O | | | | |
|---|---|---|---|---|
| Calcd: | C, 73.95; | H, 5.78; | N, 5.95; | Cl, 7.53. |
| Found: | C, 74.25; | H, 5.94; | N, 5.92; | Cl, 7.12. |

IR (KBr) cm⁻¹: 3450, 3030, 1653, 1595, 1520, 1416, 1323, 1254, 1213, 762
¹H NMR (200MHz, CDCl₃) δ: 2.50-2.75 (4H, m), 5.92 (2H, br s), 7.00 (1H, d, J=8.0Hz), 7.15-7.40 (9H, m), 7.60-7.85 (5H, m), 8.08-8.25 (1H, br), 9.21 (2H, br s), 9.73 (1H, br s).

### Working Example 17 (Production of Compound 17)

To a mixture of N-[4-(hydroxymethyl)phenyl]-7-phenyl-3,4-dihydronaphtha lene-2-carboxamide (200mg), lithium chloride (95mg), triethylamine (182 µl) and dichloromethane (20ml) was added methanesulfonyl chloride (174 µl), and the mixture was stirred at room temperature for 2 hours. To the reaction mixture was added dilute hydrochloric acid. The organic layer was separated, washed with saturated sodium chloride solution, dried with anhydrous sodium sulfate, and concentrated under reduced pressure. The residue was dissolvedinDMF (3ml), and to the mixture was added 3-picoline (167 µl). The reaction mixture was stirred at room temperature for 17 hours and concentrated under reduced pressure. The residue was recrystallized from ethyl acetate-methanol to give 3-methyl-1-[4-(7-phenyl-3,4-dihydro-naphthalene-2-carbo xamido)benzyl]pyridinium chloride (Compound 17)(90mg) as colorless crystals.
mp 136-140°C

| Elemental Analysis for C₃₀H₂₇N₂OCl · 1.5H₂O | | | |
|---|---|---|---|
| Calcd: | C, 72.94; | H, 6.12; | N, 5.67. |
| Found: | C, 73.19; | H, 6.37; | N, 5.61. |

IR (KBr) cm⁻¹: 3450, 3030, 1653, 1597, 1520, 1416, 1319, 1250, 1213, 764
¹H NMR (200MHz, CDCl₃) δ : 2.48 (3H, s), 2.65-2.90 (4H, m), 6.03 (2H, br s), 7.12-7.20 (1H, m), 7.25-7.55 (9H, m), 7.70-7.82 (4H, m), 7.95-8.07 (1H, m), 9.29 (2H, br s), 9.35-9.50 (1H, br).

### Working Example 18 (Production of Compound 18)

To a mixture of N-[4-(hydroxymethyl)phenyl]-7-phenyl-3,4-dihydronaphtha lene-2-carboxamide (200mg), lithium chloride (48mg), triethylamine (158 *µ*l) and dichloromethane (30ml) was added methanesulfonyl chloride (61 *µ*l), and the mixture was stirred at room temperature for 2 hours. To the reaction mixture was added dilute hydrochloric acid. The organic layer was separated, washed with saturated sodium chloride solution, dried with anhydrous sodium sulfate, and concentrated under reduced pressure. The residue was dissolved in DMF (3ml), and to the mixture was added 3,5-lutidine (193 *µ*l). The reaction mixture was stirred at room temperature for 65 hours and concentrated under reduced pressure. The residue was recrystallized from ethyl acetate-methanol to give 3,5-dimethyl-1-[4-(7-phenyl-3,4-dihydronaphthalene-2-ca rboxamido)benzyl]pyridinium chloride (Compound 18) (186mg) as colorless crystals.
mp 163-165°C

| Elemental Analysis for C₃₁H₂₉N₂OCl · 1.3H₂O | | | |
|---|---|---|---|
| Calcd: | C, 73.81; | H, 6.31; | N, 5.55. |
| Found: | C, 73.85; | H, 6.29; | N, 5.49. |

IR (KBr) cm⁻¹: 3450, 3030, 1655, 1597, 1520, 1483, 1416, 1319, 1252, 766
¹H NMR (200MHz, CDCl₃) δ : 2.44 (6H, s), 2.67-2.92 (4H, m), 5.99 (2H, s), 7.16 (1H, d, J=7.6Hz), 7.25-7.55 (9H, m), 7.77-7.90 (4H, m), 9.20 (1H, s), 9.72 (1H, br s).

### Working Example 19 (Production of Compound 19)

In DMF (3ml) was dissolved N-[4-(chloromethyl)-phenyl]-7-phenyl-3,4-dihydronaphtha lene-2-carboxamide (140mg), and to the mixture was added 4-cyanopyridine (117mg). The mixture was stirred at 70°C for 24 hours and concentrated under reduced pressure. The residue was recrystallized from ethyl acetate-methanol to give 4-cyano-1-[4-(7-phenyl-3,4-dihydro-naphthalene-2-carbox amido)benzyl]pyridinium chloride (Compound 19) (141mg) as pale brown crystals.
mp 163-165°C

| Elemental Analysis for C₃₀H₂₄N₃OCl· 0.5H₂O | | | |
|---|---|---|---|
| Calcd: | C, 73.99; | H, 5.17; | N, 8.63. |
| Found: | C, 73.71; | H, 5.29; | N, 8.47. |

IR (KBr) cm⁻¹: 3430, 3024, 1653, 1597, 1524, 1416, 1319, 1252, 829, 764
¹H NMR (200MHz, DMSO-d₆) δ : 2.50-2.65 (2H, m), 2.82-2.93 (2H, m), 5.92 (2H, s), 7.29-7.67 (11H, m), 7.85 (2H, d, J=8.6Hz), 8.73 (2H, d, J=6.8Hz), 9.54 (2H, d, J=6.8Hz), 10.19 (1H, s).

### Working Example 20 (Production of Compound 20)

In DMF (3ml) was dissolved N-[4-(chloromethyl)-phenyl]-7-phenyl-3,4-dihydronaphtha lene-2-carboxamide (160mg), and to the mixture was added 3-cyanopyridine (133mg). The mixture was stirred at 70°C for 24 hours and concentrated under reduced pressure. The residue was recrystallized from ethyl acetate-methanol to give 3-cyano-1-[4-(7-phenyl-3,4-dihydro-naphthalene-2-carbox amido)benzyl]pyridinium chloride (Compound 20) (58mg) as pale orange crystals.
mp 158-161°C

| Elemental Analysis for C₃₀H₂₄N₃OCl · 1.5H₂O | | | |
|---|---|---|---|
| Calcd: | C, 71.35; | H, 5.39; | N, 8.32. |
| Found: | C, 71.28; | H, 5.49; | N, 8.40. |

IR (KBr) cm⁻¹: 3450, 3028, 1653, 1597, 1520, 1416, 1319, 1252, 766
¹H NMR (200MHz, DMSO-d₆) δ : 2.55-2.68 (2H, m), 2.82-2.95 (2H, m), 5.88 (2H, s), 7.30-7.90 (13H, m), 8.32-8.42 (1H, m), 9.13 (1H, d, J=8.0Hz), 9.47 (1H, d, J=5.8Hz), 10.05 (1H, s), 10.21 (1H, s).

### Working Example 21 (Production of Compound 21)

In DMF (3ml) was dissolved N-[4-(chloromethyl)-phenyl]-7-phenyl-3,4-dihydronaphtha lene-2-carboxamide (160mg), and to the mixture was added 3-chloropyridine (122 µl). The mixture was stirred at 70°C for 24 hours and concentrated under reduced pressure. The residue was recrystallized from ethyl acetate-methanol to give 3-chloro-1-[4-(7-phenyl-3,4-dihydro-naphthalene-2-carbo xamido)benzyl]pyridinium chloride (Compound 21) (110mg) as pale yellow crystals.
mp 136-139°C

| Elemental Analysis for C₂₉H₂₄N₂OCl₂ · 0.5H₂O | | | |
|---|---|---|---|
| Calcd: | C, 70.16; | H, 5.08; | N, 5.64. |
| Found: | C, 70.13; | H, 5.03; | N, 5.68. |

IR (KBr) cm⁻¹: 3450, 3028, 1653, 1597, 1520, 1483, 1416, 1317, 1252, 1213, 1165, 766, 700
¹H NMR (200MHz, DMSO-d₆) δ : 2.55-2.68 (2H, m), 2.82-2.95 (2H, m), 5.85 (2H, s), 7.30-7.70 (11H, m), 7.86 (2H, d, J=8.4Hz), 8.16-8.26 (1H, m), 8.81 (1H, d, J=7.6Hz), 9.24 (1H, d, J=6.0Hz), 9.72 (1H, s), 10.21 (1H, s).

### Working Example 22 (Production of Compound 22)

In DMF (3ml) was dissolved N-[4-(chloromethyl)-phenyl]-7-phenyl-3,4-dihydronaphtha lene-2-carboxamide (140mg), and to the mixture was added 1-ethylpiperidine (154 *µ*l). The mixture was stirred at room temperature for 14 hours and concentrated under reduced pressure. The residue was recrystallized from ethyl acetate-methanol to give 1-ethyl-1-[4-(7-phenyl-3,4-dihydro-naphthalene-2-carbox amido)benzyl]piperidinium chloride (Compound 22) (125mg) as colorless crystals.
mp 153-156°C

| Elemental Analysis for C₃₁H₃₅N₂OCl · 1.5H₂O | | | |
|---|---|---|---|
| Calcd: | C, 72.42; | H, 7.45; | N, 5.45. |
| Found: | C, 72.14; | H, 7.41; | N, 5.32. |

IR (KBr) cm⁻¹: 3450, 2943, 1655, 1595, 1520, 1483, 1416, 1319, 1255, 1217, 766, 700
¹H NMR (200MHz, CDCl₃) δ: 1.30-1.42 (3H, m), 1.60-1.90 (6H, m), 2.68-2.95 (4H, m), 3.27-3.45 (4H, m), 3.55-3.70 (2H, m), 4.75 (2H, s), 7.17 (1H, d, J=7.8Hz), 7.25-7.60(9H, m), 7.90 (1H, s), 8.03 (2H, d, J=8.6Hz), 10.00 (1H, s).

### Working Example 23 (Production of Compound 23)

In DMF (3ml) was dissolved N-[4-(chloromethyl)-phenyl]-7-phenyl-3,4-dihydronaphtha lene-2-carboxamide (160mg), and to the mixture was added triethylamine (180 *µ*l). The mixture was stirred at room temperature for 14 hours and concentrated under reduced pressure. The residue was recrystallized from ethyl acetate to give triethyl[4-(7-phenyl-3,4-dihydronaphthalene-2-carboxami do)benzyl] ammonium chloride (Compound 23) (176mg) as colorless crystals.
mp 205-206°C

| Elemental Analysis for C₃₀H₃₅N₂OCl · 0.2H₂O | | | |
|---|---|---|---|
| Calcd: | C, 75.28; | H, 7.45; | N, 5.85. |
| Found: | C, 75.10; | H, 7.38; | N, 5.91. |

IR (KBr) cm⁻¹: 3450, 3007, 1655, 1599, 1519, 1483, 1416, 1319, 1252, 1215, 768, 704
¹H NMR (200MHz, CDCl₃) δ : 1.37 (9H, t, J=6.9Hz), 2.72-2.96 (4H, m), 3.22 (6H, q, J=6.9Hz), 4.62 (2H, s), 7.15-7.45 (7H, m), 7.50-7. 60 (3H, m), 7.99 (1H, s), 8.12 (2H, d, J=8.6Hz), 10.19 (1H, s).

### Working Example 24 (Production of Compound 24)

In DMF (3ml) was dissolved N-[4-(chloromethyl)-phenyl]-7-phenyl-3,4-dihydronaphtha lene-2-carboxamide (160mg), and to the mixture was added tripropylamine (244 µl). The mixture was stirred at room temperature for 14 hours and concentrated under reduced pressure. The residue was recrystallized from ethyl acetate to give [4-(7-phenyl-3,4-dihydronaphthalene-2-carboxamido)-benz yl]tripropylammonium chloride (Compound 24) (205mg) as colorless crystals.
mp 206-207°C

| Elemental Analysis for C₃₃H₄₁N₂OCl · 0.5H₂O | | | |
|---|---|---|---|
| Calcd: | C, 75.33; | H, 8.05; | N, 5.32. |
| Found: | C, 75.59; | H, 7.88; | N, 5.63. |

IR (KBr) cm⁻¹ : 3450, 2970, 1649, 1595, 1524, 1481, 1417, 1317, 1252, 1217, 770, 708
¹H NMR (200MHz, CDCl₃) δ : 0.94 (9H, t, J=7.2Hz), 1.60-1.90 (6H, m), 2.79-3.10 (10H, m), 4.64 (2H, s), 7.07 (2H, d, J=8.4Hz), 7.20 (1H, d, J=7.8Hz), 7.31-7.45 (4H, m), 7.54-7.60 (3H, m), 8.10 (1H, s), 8.19 (2H, d, J=8.6Hz), 10.43 (1H, s) .

### Working Example 25 (Production of Compound 25)

In DMF (3ml) was dissolved N-[4-(chloromethyl)-phenyl]-7-phenyl-3,4-dihydronaphtha lene-2-carboxamide (160mg), and to the mixture was added 3-ethylpyridine (146 µl). The mixture was stirred at 70°C for 72 hours and concentrated under reduced pressure. The residue was recrystallized from ethyl acetate-methanol to give 3-ethyl-1-[4-(7-phenyl-3,4-dihydro-naphthalene-2-carbox amido)benzyl]pyridinium chloride (Compound 25) (185mg) as colorless crystals.
mp 142-145°C

| Elemental Analysis for C₃₁H₂₉N₂OCl · 0.5H₂O | | | |
|---|---|---|---|
| Calcd: | C, 75.98; | H, 6.17; | N, 5.72. |
| Found: | C, 75.96; | H, 6.13; | N, 5.99. |

IR (KBr) cm⁻¹: 3381, 1657, 1597, 1520, 1416, 1317, 1252, 762
¹H NMR (200MHz, CDCl₃) δ : 1.25 (3H, t, J=7.6Hz), 2.64-2.88 (6H, m), 6.09 (2H, s), 7.14 (1H, d, J=7.8Hz), 7.25-7.52 (9H, m), 7.71-7.88 (4H, m), 8.04 (1H, d, J=8.0Hz), 9.37 (1H, d, J=6.0Hz), 9.43 (1H, s), 9.81 (1H, s).

### Working Example 26 (Production of Compound 26)

In DMF (3ml) was dissolved N-[4-(chloromethyl)-phenyl]-7-phenyl-3,4-dihydronaphtha lene-2-carboxamide (160mg), and to the mixture was added 2-picoline (126 *µ*l). The mixture was stirred at 70°C for 63 hours and concentrated under reduced pressure. The residue was recrystallized from ethyl acetate-methanol to give 2-methyl-1-[4-(7-phenyl-3,4-dihydronaphthalene-2-carbox amido)benzyl]-pyridinium chloride (Compound 26) (140mg) as pale brown crystals.
mp 152-155°C

| Elemental Analysis for C₃₀H₂₇N₂OCl · 1.0H₂O | | | |
|---|---|---|---|
| Calcd: | C, 74.29; | H, 6.03; | N, 5.78. |
| Found: | C, 74.56; | H, 5.93; | N, 5.80. |

IR (KBr) cm⁻¹: 3402, 1630, 1597, 1520, 1414, 1319, 1250, 764, 700
¹H NMR (200MHz, CDCl₃) δ : 2.60-2.90 (7H, m), 6.07 (2H, s), 7.04-7.15 (3H, m), 7.25-7.50 (7H, m), 7.65 (1H, d, J=7.8Hz), 7.72-7.92 (4H, m), 8.12-8.22 (1H, m), 9.63 (1H, d, J=6.2Hz), 9.86 (1H, s).

### Working Example 27 (Production of Compound 27)

In DMF (3ml) was dissolved N-[4-(chloromethyl)-phenyl]-7-phenyl-3,4-dihydronaphtha lene-2-carboxamide (160mg), and to the mixture was added thiazole (91 µl). The mixture was stirred at 100°C for 48 hours and concentrated under reduced pressure. The residue was recrystallized from ethyl acetate-methanol to give 3-[4-(7-phenyl-3,4-dihydronaphthalene-2-carboxamido)ben zyl]thiazolium chloride (Compound 27) (133mg) as pale brown crystals.
mp 149-152°C

| Elemental Analysis for C₂₇H₂₃N₂OSCl · 0.5H₂O | | | |
|---|---|---|---|
| Calcd: | C, 69.29; | H, 5.17; | N, 5.99. |
| Found: | C, 69.43; | H, 4.88; | N, 6.12. |

IR (KBr) cm⁻¹ : 3419, 3026, 1649, 1597, 1520, 1414, 1317, 1252, 764, 698
¹H NMR (200MHz, DMSO-d₆) δ : 2.55-2.67 (2H, m), 2.82-2.96 (2H, m), 5.78 (2H, s), 7.29-7.71 (11H, m), 7.84 (2H, d, J=8.2Hz), 8.33-8.40 (1H, m), 8.58-8.66 (1H, m), 10.18 (1H, s), 10.42 (1H, s).

### Working Example 28 (Production of Compound 28)

In DMF (3ml) was dissolved N-[4-(chloromethyl)-phenyl]-7-phenyl-3,4-dihydronaphtha lene-2-carboxamide (160mg), and to the mixture was added quinuclidine (285mg). The mixture was stirred at 100°C for 24 hours and concentrated under reduced pressure. The residue was recrystallized from ethyl acetate-methanol to give 1-[4-(7-phenyl-3,4-dihydronaphthalene-2-carboxamide)-benzyl]quinuclidium chloride (Compound 28) (62mg) as colorless crystals.
mp 250-252°C

| Elemental Analysis for C₃₁H₃₃N₂OCl · 0.9H₂O | | | |
|---|---|---|---|
| Calcd: | C, 74.28; | H, 7.00; | N, 5.59. |
| Found: | C, 74.48; | H,7.01; | N, 5.56. |

IR (KBr) cm⁻¹: 3425, 2945, 1655, 1595, 1520, 1416, 1319, 1255, 833, 766, 700
¹H NMR (200MHz, CDCl₃) δ : 1.75-2. 15 (7H, m), 2.68-2.90 (4H, m), 3.40-3.70 (6H, m), 4.73 (2H, s), 7.15 (1H, d, J= 7. 8Hz), 7.25-7.56 (9H, m), 7.88 (1H, s), 7.96 (2H, d, J=8.0Hz), 9.93 (1H, s).

### Working Example 29 (Production of Compound 29)

In DMF (3ml) was dissolved N-[4-(chloromethyl)-phenyl]-7-phenyl-3,4-dihydronaphtha lene-2-carboxamide (150mg), and to the mixture was added ethyl 1-methyl-piperidine-4-carboxylate (206mg). The mixture was stirred at room temperature for 15 hours and concentrated under reduced pressure. The residue was recrystallized from ethyl acetate-methanol to give 4-ethoxycarbonyl-1-methyl-1-[4-(7-phenyl-3,4-dihydronap hthalene-2-carboxamido)benzyl]piperidinium chloride (Compound 29) (185mg, ratio of isomers=37:63) as colorless crystals.
mp 153-156°C

| Elemental Analysis for C₃₃H₃₇N₂O₃Cl · 0.5H₂O | | | |
|---|---|---|---|
| Calcd: | C, 71.53; | H, 6.91; | N, 5.06. |
| Found: | C, 71.69; | H,6.76; | N, 5.11. |

IR (KBr) cm⁻¹: 3388, 1726, 1655, 1595, 1520, 1483, 1416, 1319, 1254, 1214, 766, 700
¹H NMR (200MHz, CDCl₃) δ : 1.15-1.30 (3H, m), 2.05-2.22 (3H, m), 2.65-2.92 (6H, m), 3.02 (1.11H, s), 3.13 (1.89H, s), 3.38-3.75 (3.26H, m), 3.88-4.22 (2.74H, m), 4.76 (1.26H, s), 5.09 (0.74H, s), 7.15 (1H, dd, J=4.4, 7.6Hz), 7.25-7.55 (9H, m), 7.83 (1H, s), 7.94 (1H, d, J=8.4Hz), 8.00 (1H, d, J=8.4Hz), 9.74 (0.63H, s), 9.84 (0.37H, s).

### Working Example 30 (Production of Compound 30)

In THF (10ml) was dissolved N-[4-(chloromethyl)-phenyl]-7-phenyl-3,4-dihydronaphtha lene-2-carboxamide (300mg), and to the mixture was added hexamethyleneimine (270 µl). The mixture was refluxed for 3.5 hours. The reaction mixture was cooled to room temperature, and to the mixture was added water (30ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/triethylamine=20/1) and recrystallized from ethyl acetate-hexane to give N-[4-(1-perhydroazepinylmethyl)-phenyl]-7-phenyl-3,4-di hydronaphthalene-2-carboxamide (Compound 30) (257mg) as colorless crystals.
mp 168-170°C

| Elemental Analysis for C₃₀H₃₂N₂O | | | |
|---|---|---|---|
| Calcd: | C, 82.53; | H, 7.39; | N, 6.42. |
| Found: | C, 82.28; | H, 7.26; | N, 6.37. |

IR (KBr) cm⁻¹: 3304, 2924, 1645, 1601, 1520, 1410, 1317, 1254, 831, 762, 698
¹H NMR (200MHz, CDCl₃) δ : 1.61 (8H, s), 2.56-2.76 (6H, m), 2.92-3.03 (2H, m), 3.61 (2H, s), 7.23-7.61 (14H, m).

### Working Example 31 (Production of Compound 31)

In DMF (3ml) was dissolved N-[4-(1-perhydroazepinylmethyl)phenyl]-7-phenyl-3,4-dih ydronaphthalene-2-carboxamide (150mg), and to the mixture was added methyl iodide (64µl). The mixture was stirred at room temperature for 12 hours and concentrated under reduced pressure. The residue was recrystallized from ethyl acetate-methanol to give 1-methyl-1-[4-(7-phenyl-3,4-dihydronaphthalene-2-carbox amido)benzyl]perhydroazepinium iodide (Compound 31) (180mg) as colorless crystals.
mp 197-199°C

| Elemental Analysis for C₃₁H₃₅N₂OI · 0.5H₂O | | | |
|---|---|---|---|
| Calcd: | C, 63.37; | H, 6.18; | N, 4.77. |
| Found: | C, 63.39; | H, 6.31; | N, 4.71. |

IR (KBr) cm⁻¹: 3427, 3267, 2937, 1660, 1593, 1520, 1481, 1417, 1313, 1250, 694
¹H NMR (200MHz, DMSO-d₆) δ : 1.50-1.70 (4H, m), 1.80-1.96 (4H, m), 2.55-2.68 (2H, m), 2.83-2.97 (5H, m), 3.22-3.36 (2H, m), 3.40-3.60 (2H, m), 4.50 (2H, s), 7.30-7.70 (11H, m), 7.89 (2H, d, J=8.4Hz), 10.19 (1H, s).

### Working Example 32 (Production of Compound 32)

In DMF (3ml) was dissolved N-[4-(chloromethyl)-phenyl]-7-(4-methylphenyl)-3,4-dihy dronaphthalene-2-carboxamide (150mg), and to the mixture was added 1-ethylpiperidine (159 *µ*l). The mixture was stirred at room temperature for 20 hours. To the reaction mixture was added ethyl acetate (100ml), and the resulting precipitate was filtered to give 1-ethyl-1-[4-[7-(4-methylphenyl)-3,4-dihydronaphthalene -2-carboxamido]benzyl]piperidinium chloride (Compound 32) (156mg) as colorless crystals.
mp 207-209°C

| Elemental Analysis for C₃₂H₃₇N₂OCl | | | |
|---|---|---|---|
| Calcd: | C, 76.70; | H, 7.44; | N, 5.59. |
| Found: | C, 76.33; | H, 7.22; | N, 5.67. |

IR (KBr) cm⁻¹ : 3440, 2945, 1651, 1595, 1520, 1416, 1321, 1248, 808
¹H NMR (200MHz, CDCl₃) δ : 1.36 (3H, t, J=6.0Hz), 1.60-1.90 (6H, m), 2.37 (3H, s), 2.68-2.92 (4H, m), 3.26-3.42 (4H, m), 3.52-3.70 (2H, m), 4.76 (2H, s), 7.11-7.23 (3H, m), 7.31-7.52 (6H, m), 7.90 (1H, s), 8.04 (2H, d, J=8.4Hz), 10.07 (1H, s).

### Working Example 33 (Production of Compound 33)

In THF (15ml) was dissolved N-[4-(chloromethyl)-phenyl]-7-(4-methylphenyl)-3,4-dihy dronaphthalene-2-carboxamide (300mg), and to the mixture was added 4-benzylpiperidine (408 µl). The mixture was refluxed for 19 hours. The reaction mixture was cooled to room temperature, and to the mixture was added water (100ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) and recrystallized from ethyl acetate-hexane to give N-[4-(4-benzyl-piperidinomethyl)phenyl]-7-(4-methylphen yl)-3,4-dihydronaphthalene-2-carboxamide (Compound 33) (259mg) as colorless crystals.
mp 199-201°C

| Elemental Analysis for C₃₇H₃₈N₂O | | | |
|---|---|---|---|
| Calcd: | C, 84.37; | H, 7.27; | N, 5.32. |
| Found: | C, 84.34; | H, 7.18; | N, 5.39. |

IR (KBr) cm⁻¹: 3439, 2920, 1647, 1520, 1412, 1315, 808, 700 ¹H NMR (200MHz, CDCl₃) δ : 1.20-1.70 (5H, m), 1.80-1.97 (2H, m), 2.40 (3H, s), 2.53 (2H, d, J=6.2Hz), 2.65-2.78 (2H, m), 2.80-3.02 (4H, m), 3.45 (2H, s), 7.09-7.36 (11H, m), 7.40-7.63 (7H, m).

### Working Example 34 (Production of Compound 34)

In DMF (3ml) was dissolved N- [4- (4-benzyl-piperidino-methyl)phenyl]-7-(4-methylphenyl)-3,4-dihydro-naphthale ne-2-carboxamide (150mg), and to the mixture was added methyl iodide (53 *µ*l). The mixture was stirred at room temperature for 23 hours. To the reaction mixture was added ethyl acetate (100ml), and the resulting precipitate was filtered to give 4-benzyl-1-methyl-1-[4-[7-(4-methyl-phenyl)-3,4-dihydro naphthalene-2-carboxamido]benzyl]-piperidinium iodide (Compound 34) (141mg, ratio of isomers=19:81) as colorless crystals.
mp 209-212°C

| Elemental Analysis for C₃₈H₄₁N₂OI · 0.5H₂O | | | |
|---|---|---|---|
| Calcd: | C, 67.35; | H, 6.25; | N, 4.13. |
| Found: | C, 67.28; | H, 6.33; | N, 4.08. |

IR (KBr) cm⁻¹ : 3439, 1659, 1593, 1520, 1416, 1317, 1250, 812
¹H NMR (200MHz, DMSO-d₆) δ : 1.55-2.00 (5H, m), 2.35 (3H, s), 2.52-2.75 (4H, m), 2.80-3.00 (5H, m), 3.20-3.40 (4H, m), 4.49 (1.62H, s), 4.60 (0.38H, s), 7.13-7.60 (15H, m), 7.80-7.90 (2H, m), 10.15 (1H, s).

### Working Example 35 (Production of Compound 35)

In DMF (3ml) was dissolved N-[4-(chloromethyl)-phenyl]-7-(4-methylphenyl)-3,4-dihy dronaphthalene-2-carboxamide (150mg), and to the mixture was added 1-ethylperhydroazepine (98mg). The mixture was stirred at room temperature for 15 hours. To the reaction mixture was added ethyl acetate (100ml), and the resulting precipitate was filtered and recrystallized from ethyl acetate-methanol to give 1-ethyl-1-[4-[7-(4-methyl-phenyl)-3,4-dihydronaphthalen e-2-carboxamido]benzyl]perhydroazepinium chloride (Compound 35) (137mg) as colorless crystals.
mp 207-210°C

| Elemental Analysis for C₃₃H₃₉N₂OCl · 0.5H₂O | | | |
|---|---|---|---|
| Calcd: | C, 75.62; | H, 7.69; | N, 5.34. |

Found: C, 75.82; H, 7.69; N, 5.42.
IR (KBr) cm⁻¹: 3431, 2931, 1653, 1597, 1520, 1325, 1255, 808
¹H NMR (200MHz, DMSO-d₆) δ : 1.40 (3H, t, J=7.1Hz), 1.50-1.65 (4H, m), 1.70-1.90 (4H, m), 2.35 (3H, s), 2.55-2.67 (2H, m), 2.80-2.93 (2H, m), 3.12-3.35 (4H, m), 3.40-3.57 (2H, m), 4.47 (2H, s), 7.23-7.35 (3H, m), 7.50-7.60 (7H, m), 7.91 (2H, d, J=8.4Hz), 10.26 (1H, s).

### Working Example 36 (Production of Compound 36)

In DMF (3ml) was dissolved N-[4-(chloromethyl)-phenyl]-7-(4-methylphenyl)-3,4-dihy dronaphthalene-2-carboxamide (150mg), and to the mixture was added 1-propylperhydroazepine (109mg). The mixture was stirred at room temperature for 15 hours. To the reaction mixture was added ethyl acetate (100ml), and the resulting precipitate was filtered to give 1-[4-[7-(4-methylphenyl)-3,4-dihydronaphthalene-2-carbo xamido]benzyl]-1-propyl-perhydroazepinium chloride (Compound 36) (163mg) as colorless crystals.
mp 195-199°C

| Elemental Analysis for C₃₄H₄₁N₂OCl · 0.5H₂O | | | |
|---|---|---|---|
| Calcd: | C, 75.88; | H, 7.87; | N, 5.21. |
| Found: | C, 76.07; | H, 7.83; | N, 5.21. |

IR (KBr) cm⁻¹: 3423, 2937, 1651, 1595, 1520, 1317, 1250, 814
¹H NMR (200MHz, DMSO-d₆) δ : 0.93 (3H, t, J=7.2Hz), 1.52-1.65 (4H, m), 1.75-1.93 (6H, m), 2.35 (3H, s), 2.55-2.68 (2H, m), 2.80-2.95 (2H, m), 3.00-3.13 (2H, m), 3.22-3.40 (2H, m), 3.40-3.58 (2H, m), 4.49 (2H, s), 7.23-7.35 (3H, m), 7.46-7.60 (7H, m), 7.90 (2H, d, J=8.0Hz), 10.22 (1H, s).

### Working Example 37 (Production of Compound 37)

In DMF (3ml) was dissolved N-[4-(chloromethyl)-phenyl]-7-(4-methylphenyl)-3,4-dihy dronaphthalene-2-carboxamide (150mg), and to the mixture was added 1-ethylperhydroazocine (109mg). The mixture was stirred at room temperature for 14 hours. To the reaction mixture was added ethyl acetate (100ml), and the resulting precipitate was filtered and recrystallized from ethyl acetate-methanol to give 1-ethyl-1-[4-[7-(4-methyl-phenyl)-3,4-dihydronaphthalen e-2-carboxamido]benzyl]perhydro-azocinium chloride (Compound 37) (142mg) as colorless crystals.
mp 197-199°C

| Elemental Analysis for C₃₄H₄₁N₂OCl · 0.5H₂O | | | |
|---|---|---|---|
| Calcd: | C, 75,88; | H, 7.87; | N, 5.21. |
| Found: | C, 75.67; | H, 7.88; | N, 5.30. |

IR (KBr) cm⁻¹: 3437, 2926, 1655, 1595, 1520, 1489, 1416, 1321, 1252, 812
¹H NMR (200MHz, DMSO-d₆) δ : 1.30-2.00 (13H, m), 2.35 (3H, s), 2.55-2.70 (2H, m), 2.85-3.00 (2H, m), 3.05-3.50 (6H, m), 4.44 (2H, s), 7.20-7.37 (3H, m), 7.40-7.60 (7H, m), 7.92 (2H, d, J=8.6Hz), 10.28 (1H, s).

### Working Example 38 (Production of Compound 38)

In THF (7ml) was dissolved N-[4-(chloromethyl)-phenyl]-7-(4-methylphenyl)-3,4-dihy dro-naphthalene-2-carboxamide (150mg), and to the mixture was added 1-methylpiperazine (129µl). The mixture was refluxed for 24 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/triethylamine=20/1) and recrystallized from ethyl acetate-hexane to give 7-(4-methylphenyl)-N-[4-(4-methyl-1-piperazinylmethyl)phenyl]-3,4-dihydronaphthalene-2-ca rboxamide (Compound 38) (105mg) as colorless crystals.
mp 174-175°C

| Elemental Analysis for C₃₀H₃₃N₃O | | | |
|---|---|---|---|
| Calcd: | C, 79.79; | H, 7.37; | N, 9.30. |
| Found: | C, 79.43; | H, 7.41; | N, 9.28. |

IR (KBr) cm⁻¹ : 3327, 2941, 2794, 1643, 1524, 1315, 1163, 1011, 808
¹H NMR (200MHz, CDCl₃) δ : 2.29 (3H, s), 2.35-2.60 (8H, m), 2.40 (3H, s), 2.65-2.78 (2H, m), 2.90-3.02 (2H, m), 3.48 (2H, s), 7.20-7.35 (6H, m), 7.39-7.63 (7H, m).

### Working Example 39 (Production of Compound 39)

In DMF (3ml) was dissolved N-[4-(chloromethyl)-phenyl]-7-(4-methylphenyl)-3,4-dihy dronaphthalene-2-carboxamide (150mg), and to the solution were added 1-(2-methoxyphenyl)piperazine (97mg) and potassium carbonate (268mg). The mixture was stirred at room temperature for 13 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give N-[4-[1-(2-methoxyphenyl)-4-piperazinylmethyl]phenyl]-7 -(4-methylphenyl)-3,4-dihydronaphthalene-2-carboxamide (Compound 39) (142mg) as colorless crystals.
mp 202-205°C

| Elemental Analysis for C₃₆H₃₇N₃O₂ | | | |
|---|---|---|---|
| Calcd: | C, 79.53; | H, 6.86; | N, 7.73. |
| Found: | C, 79.28; | H, 6.68; | N, 7.66. |

IR (KBr) cm⁻¹: 3350, 2933, 2812, 1649, 1595, 1520, 1500, 1313, 1240, 812, 746
¹H NMR (200MHz, CDCl₃) δ : 2.40 (3H, s), 2.60-2.75 (6H, m), 2.90-3.12 (6H, m), 3.57 (2H, s), 3.86 (3H, s), 6.80-7.03 (4H, m), 7.20-7.28 (3H, m), 7.30-7.38 (3H, m), 7.40-7.51 (4H, m), 7.53-7.63 (3H, m).

### Working Example 40 (Production of Compound 40)

In THF (7ml) was dissolved N-[4-(chloromethyl)-phenyl]-7-(4-methylphenyl)-3,4-dihy dronaphthalene-2-carboxamide (150mg), and to the mixture was added 1-(2-pyrimidyl)piperazine (190mg). The mixture was refluxed for 24 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) and recrystallized from ethyl acetate-hexane to give 7-(4-methylphenyl)-N-[4-[1-(2-pyrimidyl)-4-piperazinylm ethyl]-phenyl]-3,4-dihydronaphthalene-2-carboxamide (Compound 40) (166mg) as colorless crystals.
mp 203-204°C

| Elemental Analysis for C₃₃H₃₃N₅O | | | |
|---|---|---|---|
| Calcd: | C, 76.87; | H, 6.45; | N, 13.58. |
| Found: | C, 76.77; | H, 6.40; | N, 13.60. |

IR (KBr) cm⁻¹: 3367, 2935, 1649, 1585, 1516, 1448, 1358, 1313, 1255, 984, 808
¹H NMR (200MHz, CDCl₃) δ : 2.40 (3H, s), 2.47-2.54 (4H, m), 2.65-2.78 (2H, m), 2.93-3.03 (2H, m), 3.53 (2H, s), 3.79-3.87 (4H, m), 6.47 (1H, t, J=4.8Hz), 7.23-7.28 (3H, m), 7.30-7.38 (3H, m), 7.42-7.52 (4H, m), 7.54-7.62 (3H, m), 8.30 (2H, d J=4.8Hz).

### Working Example 41 (Production of Compound 41)

In DMF (3ml) was dissolved N-[4-(chloromethyl)-phenyl]-7-(4-methylphenyl)-3,4-dihy dronaphthalene-2-carboxamide (150mg), and to the solution were added 1-benzhydrylpiperazine (127mg) and potassium carbonate (268mg). The mixture was stirred at room temperature for 24 hours, and to the mixture was added water (50ml) . The mixture was extracted with ethyl acetate. The organic layer was washed with saturated sodium chloride solution, dried with anhydrous sodium sulfate, and concentrated under reduced pressure. The residue was recrystallized from acetone-diisopropylether to give N-[4-(4-benzhydryl-1-piperazinyl-methyl)phenyl]-7-(4-me thylphenyl)-3,4-dihydronaphthalene-2-carboxamide (Compound 41) (140mg) as colorless crystals.
mp 217-218°C

| Elemental Analysis for C₄₂H₄₁N₃O | | | |
|---|---|---|---|
| Calcd: | C, 83.55; | H, 6.84; | N, 6.96. |
| Found: | C, 83.25; | H, 6.86; | N, 7.06. |

IR (KBr) cm⁻¹: 3417, 2954, 2812, 1659, 1618, 1520, 1410, 1313, 1007, 810, 706
¹H NMR (200MHz, DMSO-d₆) δ: 2.20-2.65 (13H, m), 2.80-2.93 (2H, m), 3.42 (s, 2H), 4.26 (1H, s), 7.10-7.70 (22H, m), 9.90 (1H, s).

### Working Example 42 (Production of Compound 42)

In DMF (3ml) was dissolved N-[4-(chloromethyl)-phenyl]-7-(4-methylphenyl)-3,4-dihy dronaphthalene-2-carboxamide (150mg), and to the solution were added 1-(2-furoyl)piperazine hydrochloride (109mg) and potassium carbonate (268mg). The mixture was stirred at room temperature for 18 hours, and to the mixture was added water (50ml). The mixture was extracted with ethyl acetate. The organic layer was washed with saturated sodium chloride solution, dried with anhydrous sodium sulfate, and concentrated under reduced pressure. The residue was purified with ethyl acetate-diisopropylether to give N-[4-[1-(2-furoyl)-4-piperazinylmethyl]phenyl]-7-(4-met hylphenyl)-3,4-dihydronaphthalene-2-carboxamide (Compound 42) (112mg) as colorless amorphous.
IR (KBr) cm⁻¹: 3309, 2920, 1618, 1518, 1489, 1437, 1313, 1184, 1001, 812, 754

| Elemental Analysis for C₃₄H₃₃N₃O₃ | | | |
|---|---|---|---|
| Calcd: | C, 76.81; | H, 6.26; | N, 7.90. |
| Found: | C, 76.60; | H, 6.02; | N, 7.61. |

¹H NMR (200MHz, CDCl₃) δ: 2.40 (3H, s), 2.43-2.55 (4H, m), 2.65-2.78 (2H, m), 2.90-3.03 (2H, m), 3.52 (2H, s), 3.73-3.87 (4H, m), 6.44-6.49 (1H, m), 6.98 (1H, d, J=3.2Hz), 7.20-7.68 (14H, m).

### Working Example 43 (Production of Compound 43)

In DMF (3ml) was dissolved N-[4-(chloromethyl)-phenyl]-7-(4-methylphenyl)-3,4-dihy dronaphthalene-2-carboxamide (150mg), and to the solution were added 1-(3,4,5-trimethoxybenzyl)piperazine (138mg) and potassium carbonate (268mg). The mixture was stirred at room temperature for 48 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give N-[4-[1-(3,4,5-trimethoxybenzyl)-4-piperazinylmethyl]-p henyl]-7-(4-methylphenyl)-3,4-dihydronaphthalene-2-carb oxamide (Compound 43) (155mg) as pale yellow crystals.
mp 143-144°C

| Elemental Analysis for C₃₉H₄₃N₃O₄ | | | |
|---|---|---|---|
| Calcd: | C, 75.82; | H, 7.02; | N, 6.80. |
| Found: | C, 75.74; | H, 6.85; | N, 6.75. |

IR (KBr) cm⁻¹: 3425, 2935, 2806, 1649, 1593, 1520, 1458, 1421, 1313, 1236, 1128, 1009, 810
¹H NMR (200MHz, CDCl₃) δ: 2.40 (3H, s), 2.40-2.55 (8H, m), 2.65-2.77 (2H, m), 2.90-3.03 (2H, m), 3.45 (2H, s), 3.51 (2H, s), 3.84 (3H, s), 3.86 (6H, s), 6.56 (2H, s), 7.20-7.36 (6H, m), 7.40-7.62 (7H, m).

### Working Example 44 (Production of Compound 44)

In THF (7ml) was dissolved N-[4-(chloromethyl)-phenyl]-7-(4-methylphenyl)-3,4-dihy dronaphthalene-2-carboxamide (150mg), and to the mixture was added 1-(2-hydroxyethyl)piperazine (142 *µ*l). The mixture was refluxed for 22 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-hexane to give N-[4-[1-(2-hydroxyethyl)-4-piperazinylmethyl]phenyl]-7-(4-methylphenyl)-3,4-dihydronaphthalene-2-carboxamide (Compound 44) (158mg) as colorless crystals.
mp 185-187°C

| Elemental Analysis for C₃₁H₃₅N₃O₂ · 0.3H₂O | | | |
|---|---|---|---|
| Calcd: | C, 76.45; | H, 7.37; | N, 8.63. |
| Found: | C, 76.64; | H, 7.13; | N, 8.35. |

IR (KBr) cm⁻¹: 3319, 2937, 2816, 1649, 1597, 1520, 1412, 1317, 812
¹H NMR (200MHz, CDCl₃) δ : 2.40 (3H, s), 2.43-2.61 (10H, m), 2.65-2.78 (2H, m), 2.92-3.03 (2H, m), 3.50 (2H, s), 3.61 (2H, t, J=5.5Hz), 7.21-7.36 (6H, m), 7.40-7.63 (7H, m) .

### Working Example 45 (Production of Compound 45)

In THF (7ml) was dissolved N-[4-(chloromethyl)-phenyl]-7-(4-methylphenyl)-3,4-dihy dronaphthalene-2-carboxamide (150mg), and to the mixture was added3-aminopyridine (109mg). The mixture was refluxed for 45 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/hexane=3/1) and recrystallized from ethyl acetate-hexane to give 7-(4-methylphenyl)-N-[4-[N-(3-pyridyl)aminomethyl]pheny 1]-3,4-dihydronaphthalene-2-carboxamide (Compound 45) (14mg) as colorless crystals.
mp 212-214°C
IR (KBr) cm⁻¹: 3383, 3022, 1655, 1591, 1516, 1412, 1315, 1254, 808, 708
¹H NMR (200MHz, CDCl₃) δ : 2.40 (3H, s), 2.66-2.78 (2H, m), 2.92-3.03 (2H, m), 4.05-4.18 (1H, br), 4.30-4.37 (2H, m), 6.88 (1H, ddd, J=1.4, 2.8, 8.0Hz), 7.08 (1H, dd, J=4.8, 8.0Hz), 7.23-7.30 (3H, m), 7.32-7.39 (3H, m), 7.41-7.51 (4H, m), 7.58-7.65 (3H, m), 7.98 (1H, dd, J=1.4, 4.8Hz), 8.09 (1H, d, J=2.8Hz).

### Working Example 46 (Production of Compound 46)

In DMF (3ml) was dissolved N-[4-(chloromethyl)-phenyl]-7-(4-methylphenyl)-3,4-dihy dronaphthalene-2-carboxamide (150mg), and to the mixture was added 2-amino-1,3-propanediol (106mg). The mixture was stirred at room temperature for 72 hours, and to the mixture was added water (50ml). The mixture was extracted with ethyl acetate. The organic layer was washed with saturated sodium chloride solution, driedwith anhydrous sodium sulfate, and concentrated under reduced pressure. The residue was recrystallized from ethyl acetate-diisopropylether to give N-[4-[(1,3-dihydroxy-2-propyl)aminomethyl]phenyl]-7-(4-methyl-phenyl)-3,4-dihydronaphthalene-2-carboxamide
(Compound 46) (60mg) as colorless crystals.
mp 189-193°C

| Elemental Analysis for C₂₈H₃₀N₂O₃ | | | |
|---|---|---|---|
| Calcd: | C, 75.99; | H, 6.83; | N, 6.33. |
| Found: | C, 75.64; | H, 6.86; | N, 6.11. |

IR (KBr) cm⁻¹: 3332, 2931, 1649, 1620, 1597, 1520, 1412, 1319, 1255, 1045, 812
¹H NMR (200MHz, DMSO-d₆) δ : 2.35 (3H, s), 2.53-2.65 (2H, m), 2.80-2.93 (2H, m), 3.28-3.45 (5H, m), 3.73 (2H, s), 4.43 (2H, s), 7.20-7.35 (5H, m), 7.43-7.59 (5H, m), 7.67 (2H, d, J=8.4Hz), 9.90 (1H, s).

### Working Example 47 (Production of Compound 47)

In THF (10ml) was dissolved N-[4-(chloromethyl)-phenyl]-7-(4-methylphenyl)-3,4-dihy dronaphthalene-2-carboxamide (300mg), and to the mixture was added 4-hydroxypiperidine (235mg). The mixture was refluxed for 24 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure.

The residue was recrystallized from ethyl acetate-hexane to give N-[4-(4-hydroxypiperidinomethyl)phenyl]-7-(4-methylphen yl)-3,4-dihydronaphthalene-2-carboxamide (Compound 47) (271mg) as colorless crystals.
mp 223-224°C

| Elemental Analysis for C₃₀H₃₂N₂O₂ | | | |
|---|---|---|---|
| Calcd: | C, 79.61; | H, 7.13; | N, 6.19. |
| Found: | C, 79.54; | H, 7.00; | N, 6.15. |

IR (KBr) cm⁻¹: 3321, 2937, 1651, 1622, 1597, 1520, 1412, 1319, 1070, 812
¹H NMR (200MHz, DMSO-d₆) δ : 1.28-1.47 (2H, m), 1.63-1.78 (2H, m), 1.88-2.08 (2H, m), 2.25-2.70 (7H, m), 2.80-2.92 (2H, m), 3.23-3.50 (2H, m), 4.50-4.58 (1H, m), 7.17-7.33 (5H, m), 7.45 (1H, s), 7.48-7.60 (4H, m), 7.67 (2H, d, J=8.0Hz), 9.92 (1H, s).

### Working Example 48 (Production of Compound 48)

In THF (10ml) was dissolved N-[4-(chloromethyl)-phenyl]-7-(4-methylphenyl)-3,4-dihy dro-naphthalene-2-carboxamide (300mg), and to the mixture was added thiomorpholine (233 *µ*l). The mixture was refluxed for 20 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-hexane to give 7-(4-methylphenyl)-N-[4-(thiomorpholinomethyl)phenyl]-3 ,4-dihydro-naphthalene-2-carboxamide (Compound 48) (309mg) as colorless crystals.
mp 178-180°C

| Elemental Analysis for C₂₉H₃₀N₂OS | | | |
|---|---|---|---|
| Calcd: | C, 76.61; | H, 6.65; | N, 6.16. |
| Found: | C, 76.39; | H, 6.71; | N, 5.94. |

IR (KBr) cm⁻¹: 3307, 2910, 2810, 1648, 1599, 1520, 1412, 1315, 1257, 806
¹H NMR (200MHz, CDCl₃) δ : 2.40 (3H, s), 2.57-2.75 (10H, m), 2.90-3.03 (2H, m), 3.50 (2H, s), 7.22-7.62 (13H, m).

### Working Example 49 (Production of Compound 49)

In THF (10ml) was dissolved N-[4-(chloromethyl)-phenyl]-7-(4-methylphenyl)-3,4-dihy dronaphthalene-2-carboxamide (300mg), and to the mixture was added diethanolamine (222 µl). The mixture was refluxed for 34 hours. The reaction mixture was cooled to room temperature, and to the mixture was added 5% sodium hydrogen carbonate solution (50ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate/triethylamine=10/1) and recrystallized from ethyl acetate-hexane to give N-[4-[N,N-bis(2-hydroxyethyl)-aminomethyl]phenyl]-7-(4-methylphenyl)-3,4-dihydro-naphthalene-2-carboxamide (Compound 49) (148mg) as colorless crystals.
mp 150-151°C

| Elemental Analysis for C₂₉H₃₂N₂O₃ | | | |
|---|---|---|---|
| Calcd: | C, 76.29; | H, 7.06; | N, 6.14. |
| Found: | C, 75.90; | H, 7.10; | N, 6.18. |

IR (KBr) cm⁻¹: 3307, 2943, 1645, 1599, 1524, 1412, 1321, 1255, 1036, 804
¹H NMR (200MHz, CDCl₃) δ : 2.40 (3H, s), 2.64-2.75 (6H, m), 2.90-3.00 (2H, m), 3.58-3.70 (6H, m), 7.20-7.37 (6H, m), 7.40-7.51 (4H, m), 7.58 (2H, d, J=8.4Hz), 7.67-7.77 (1H, m).

### Working Example 50 (Production of Compound 50)

In DMF (5ml) was dissolved N-[4-(chloromethyl)-phenyl]-7-(4-methylphenyl)-3,4-dihy dronaphthalene-2-carboxamide (150mg), and to the mixture was added pyridine (94 µl*)*. The mixture was stirred at 70°C for 24 hours, and to the mixture was added water (50ml). The mixture was washed with ethyl acetate. The aqueous layer was allowed to stand at room temperature for 3 hours. The resulting precipitate was filtered and purified with ethyl acetate-methanol to give 1-[7-(4-methylphenyl)-3,4-dihydronaphthalene-2-carboxam ido)benzyl]pyridinium chloride (Compound 50) (74mg) as colorless amorphous.

| Elemental Analysis for C₃₀H₂₇N₂OCl · 0.5H₂O | | | |
|---|---|---|---|
| Calcd: | C, 75.70; | H, 5.93; | N, 5.88. |
| Found: | C, 75.83; | H, 6.02; | N, 5.63. |

IR (KBr) cm⁻¹: 3413, 1655, 1595, 1518, 1414, 1317, 1248, 810
¹H NMR (200MHz, DMSO-d₆) δ : 2.35 (3H, s), 2.55-2.67 (2H, m), 2.80-2.93 (2H, m), 5.85 (2H, s), 7.24-7.34 (3H, m), 7.50-7.60 (7H, m), 7.85 (2H, d, J=8.6Hz), 8.14-8.25 (2H, m), 8.64 (1H, t, J=7.7Hz), 9.20-9.30 (2H, m), 10.18 (1H, s).

### Comparative Example 1

A solution of N-(4-chloromethylphenyl)-7-(4-methylphenyl)-2,3-dihydro-1-benzoxepine-4-carboxamide (0.2g) and sodium cyclohexylsulfide (0.08g) in dimethylformamide (10ml) was stirred at room temperature for 2.5 hours. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-(cyclohexylthiomethyl)-phenyl)-7-(4-methylphenyl)-2,3-dihydro-1-benzoxepine-4-carboxamide (0.19g) as colorless crystals.
mp 161-162°C.
¹H-NMR (δ ppm, CDCl₃) : 1.23-1.42 (6H, m), 1.63-1.75 (2H, m), 1.92-2.05 (2H, m), 2.39 (3H, s), 2.49-2.59 (1H, m), 3.07 (2H, t, J=4.5Hz), 3.73 (2H, s), 4.36 (2H, t, J=4.5Hz), 7.06 (1H, d, J=8.2Hz), 7.22-7.34 (5H, m), 7.44-7.59 (7H, m).
IR(KBr) ν : 2928, 2851, 1651cm⁻¹.

| Anal. for C₃₁H₃₃NO₂S: | | | |
|---|---|---|---|
| Calcd. | C,76.98; | H,6.88; | N,2.90. |
| Found | C,76.65; | H,6.59; | N,3.09. |

### Working Example 51 (Production of Compound 51)

In DMF (3ml) was dissolved 3,4-dihydro-N-[4-(4-hydroxypiperidinomethyl)phenyl]-7-( 4-methylphenyl)-naphthalene-2-carboxamide (130mg), and to the mixture was added methyl iodide (54 µl). The mixture was stirred at room temperature for 17 hours, and to the mixture was added ethyl acetate (100ml). The resulting precipitate was filtered and recrystallized from ethyl acetate-methanol to give 4-hydroxy-1-methyl-1-[4-[7-(4-methylphenyl)-3,4-dihydro naphthalene-2-carboxamido]benzyl]-piperidinium iodide (Compound 51) (138mg, ratio of isomers=58:42) as colorless crystals.
mp 157-161°C

| Elemental Analysis for C₃₁H₃₅N₂O₂I · 0.5H₂O | | | |
|---|---|---|---|
| Calcd: | C, 61.69; | H, 6.01; | N, 4.64. |
| Found: | C, 61.75; | H, 5.84; | N, 4.64. |

IR (KBr) cm⁻¹: 3396, 1655, 1595, 1520, 1416, 1319, 1250, 812
¹H NMR (200MHz, DMSO-d₆) δ: 1.65-1.90 (2H, m), 1.96-2.20 (2H, m), 2.35 (3H, s), 2.55-2.68 (2H, m), 2.82-3.00 (5H, m), 3.10-3.57 (4H, m), 3.70-3.90 (1H, m), 4.50-4.60 (2H, m), 5.05 (0.42H, d, J=2.8Hz), 5.12 (0.58H, d, J=3.6Hz), 7.22-7.35 (3H, m), 7.42-7.60 (7H, m), 7.83-7.93 (2H, m), 10.18 (1H, s).

### Working Example 52 (Production of Compound 52)

In DMF (3ml) was dissolved 7-(4-methylphenyl)-N-[4-(thiomorpholinomethyl)phenyl]-3 ,4-dihydro-naphthalene-2-carboxamide (160mg), and to the mixture was added methyl iodide (66 µl). The mixture was stirred at room temperature for 17 hours, and to the mixture was added ethyl acetate (100ml). The resulting precipitate was filtered and recrystallized from ethyl acetate-methanol to give 4-methyl-4- [4- [7- (4-methyl-phenyl) -3,4-dihydro-naphthal ene-2-carboxamido] benzyl]-thiomorpholinium iodide (Compound 52) (165mg) as colorless crystals.
mp 183-185°C

| Elemental Analysis for C₃₀H₃₃N₂OSI · 0.2H₂O | | | |
|---|---|---|---|
| Calcd: | C, 60.04; | H, 5.61; | N, 4.67. |
| Found: | C, 59.91; | H, 5.52; | N, 4.66. |

IR (KBr) cm⁻¹: 3423, 1651, 1597, 1520, 1416, 1319, 1250, 812
¹H NMR (200MHz, DMSO-d₆) δ: 2.35 (3H, s), 2.55-2.68 (2H, m), 2.83-3.30 (9H, m), 3.40-3.65 (4H, m), 4.62 (2H, s), 7.25-7.35 (3H, m), 7.45-7.61 (7H, m), 7.90 (2H, d, J=8.6Hz), 10.19 (1H, s).

### Working Example 53 (Production of Compound 53)

In DMF (3ml) was dissolved N-[4-[N,N-bis(2-hydroxyethyl)aminomethyl]phenyl]-7-(4-methylphenyl)-3,4-dihydr onaphthalene-2-carboxamide (100mg), and to the mixture was added methyl iodide (41 µl). The mixture was stirred at room temperature for 22 hours. The solvent was evaporated and the residue was purified with ethyl acetate-methanol to give bis(2-hydroxyethyl)methyl[4-[7-(4-methylphenyl)-3,4-dih ydronaphthalene-2-carboxamido]-benzyl]ammonium iodide (Compound 53) (101mg) as colorless amorphous.

| Elemental Analysis for C₃₀H₃₅N₂O₃I · 0.5H₂O | | | |
|---|---|---|---|
| Calcd: | C, 59.31; | H, 5.97; | N, 4.61. |
| Found: | C, 59.19; | H, 5.74; | N, 4.68. |

IR (KBr) cm⁻¹: 3365, 1651, 1593, 1520, 1416, 1319, 1250, 810
¹H NMR (200MHz, DMSO-d₆) δ: 2.35 (3H, s), 2.55-2.67 (2H, m), 2.84-3.01 (5H, m), 3.27-3.55 (4H, m), 3.88-3.98 (4H, m), 4.62 (2H, s), 5.33 (2H, t, J=4.8Hz), 7.25-7.35 (3H, m), 7.47-7.60 (7H, m), 7.88 (2H, d, J=8.4Hz), 10.18 (1H, s) .

### Working Example 54 (Production of Compound 54)

In DMF (3ml) was dissolved (E)-N-[4-(chloromethyl)-phenyl] -3- (4-methylphenyl) cinnamamide (200mg), and to the solution were added 1-(3,4-methylenedioxybenzyl)-piperazine (158mg) and potassium carbonate (382mg). The mixture was stirred at room temperature for 16 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[1-(3,4-methylenedioxybenzyl)-4-piperazinylmet hyl]phenyl]-3-(4-methylphenyl)cinnamamide (Compound 54) (266mg) as colorless crystals.
mp 204-207°C

| Elemental Analysis for C₃₅H₃₅N₃O₃ · 0.5H₂O | | | |
|---|---|---|---|
| Calcd: | C, 75.79; | H, 6.54; | N, 7.58. |
| Found: | C, 76.19; | H, 6.48; | N, 7.83. |

IR (KBr) cm⁻¹: 2939, 2806, 1664, 1626, 1524, 1491, 1246, 1041, 1007, 970, 824, 795
¹H NMR (200MHz, CDCl₃) δ : 2.30-2.60 (8H, m), 2.41 (3H, s), 3.41 (2H, s), 3.48 (2H, s), 5.93 (2H, s), 6.61 (1H, d, J=15.6Hz), 6.73 (2H, s), 6.84 (1H, s), 7.23-7.32 (4H, m), 7.35-7.60 (8H, m), 7.72 (1H, s), 7.81 (1H, d, J=15.6Hz).

### Working Example 55 (Production of Compound 55)

In THF (10ml) was dissolved 7-phenylnaphthalene-2-carboxylic acid (350mg), and to the solution were added oxalyl chloride (184 *µ*l) and a drop of DMF. The mixture was stirred at room temperature for 1 hour and concentrated under reduced pressure. The residue was dissolved in THF (10ml), and to the solution were added 1-(4-aminobenzyl)-piperidine (295mg) and triethylamine (237 *µ*l) at room temperature. The reaction mixture was stirred at room temperature for 2 hours, and to the mixture was added water (100ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give N-[4-(piperidinomethyl)phenyl]-7-phenylnaphthalene-2-ca rboxamide (Compound 55) (491mg) as pale yellow crystals.
mp 177-178°C

| Elemental Analysis for C₂₉H₂₈N₂O · 0.2H₂O | | | |
|---|---|---|---|
| Calcd: | C, 82.12; | H, 6.75; | N, 6.60. |
| Found: | C, 82.26; | H, 6.80; | N, 6.62. |

IR (KBr) cm⁻¹: 3313, 2933, 1649, 1527, 1317, 849, 754, 692 ¹H NMR (200MHz, CDCl₃) δ: 1.37-1.65 (6H, m), 2.35-2.45 (4H, m), 3.48 (2H, s), 7.33-7.57 (5H, m), 7.62-7.77 (4H, m), 7.83-8.01 (5H, m), 8.15 (1H, s), 8.44 (1H, s).

### Working Example 56 (Production of Compound 56)

In DMF (3ml) was dissolved N-[4-(piperidinomethyl)-phenyl]-7-phenylnaphthalene-2-carboxamide (300mg), and to the mixture was added methyl iodide (133 µl). The mixture was stirred at room temperature for 16 hours and concentrated under reduced pressure. The residue was recrystallized from ethyl acetate to give 1-[4-(7-phenylnaphthalene-2-carboxamido)benzyl]-1-methy lpiperidinium iodide (Compound 56) (374mg) as pale yellow crystals.
mp 203-207°C

| Elemental Analysis for C₃₀H₃₁N₂OI · 1.0H₂O | | | |
|---|---|---|---|
| Calcd: | C, 62.07; | H, 5.73; | N, 4.83. |
| Found: | C, 61.82; | H, 5.43; | N, 4.87. |

IR (KBr) cm⁻¹: 3450, 1655, 1597, 1520, 1417, 1317, 1250, 700
1H NMR (200MHz, DMSO-d₆) δ : 1.40-2.00 (6H, m), 2.94 (3H, s), 3.25-3.40 (4H, m), 4.56 (2H, s), 7.40-7.60 (5H, m), 7.84-7.89 (2H, m), 7.95-8.17 (6H, m), 8.40 (1H, s), 8.66 (1H, s), 10.68 (1H, s).

### Working Example 57 (Production of Compound 57)

In THF (15ml) was dissolved 5-(4-methylphenyl)-indene-2-carboxylic acid (500mg), and to the solution were added oxalyl chloride (262 µl) and a drop of DMF. The mixture was stirred at room temperature for 1 hour and concentrated under reduced pressure. The residue was dissolved in THF (15ml), and to the solution were added 1-(4-aminobenzyl)piperidine (419mg) and triethylamine (336 µl) at room temperature. The reaction mixture was stirred at room temperature for 16 hours, and to the mixture was added water (100ml). The mixture was extracted with ethyl acetate. The organic layer was washed with saturated sodium chloride solution, dried with anhydrous sodium sulfate, and concentrated under reduced pressure. The residue was recrystallized from ethyl acetate-hexane to give N-[4-(piperidinomethyl)phenyl]-5-(4-methylphenyl)-inden e-2-carboxamide (Compound 57) (549mg) as colorless crystals.
mp 219-220°C

| Elemental Analysis for C₂₉H₃₀N₂O | | | |
|---|---|---|---|
| Calcd: | C, 82.43; | H, 7.16; | N, 6.63. |
| Found: | C, 82.17; | H, 7.13; | N, 6.56. |

IR (KBr) cm⁻¹ : 3346, 2935, 1645, 1597, 1516, 1408, 1315, 1250, 808
¹H NMR (200MHz, DMSO-d₆) δ: 1.34-1.57 (6H, m), 2.25-2.40 (7H, m), 3.30-3.43 (2H, m), 3.80-3.90 (2H, m), 7.20-7.32 (4H, m), 7.56-7.68 (4H, m), 7.72 (2H, d, J=8.4Hz), 7.83 (2H, s), 9.96 (1H, s).

### Working Example 58 (Production of Compound 58)

In DMF (10ml) was dissolved N- [4-(piperidinomethyl)-phenyl]-5-(4-methylphenyl)indene-2-carboxamide (400mg), and to the mixture was added methyl iodide (177 *µ*l). The mixture was stirred at room temperature for 86 hours and concentrated under reduced pressure. The residue was recrystallized from ethyl acetate to give 1-[4-[5-(4-methylphenyl)indene-2-carboxamido]-benzyl]-1 -methyl-piperidinium iodide (Compound 58) (516mg) as pale yellow crystals.
mp 199-201°C

| Elemental Analysis for C₃₀H₃₃N₂OI · 0.5H₂O | | | |
|---|---|---|---|
| Calcd: | C, 62.83; | H, 5.98; | N, 4.88. |
| Found: | C, 62.56; | H, 5.87; | N, 4.97. |

IR (KBr) cm⁻¹: 3450, 2947, 1651, 1595, 1520, 1416, 1322, 1246, 808
¹H NMR (200MHz, DMSO-d₆) δ: 1.40-2.00 (6H, m), 2.36 (3H, s), 2.92 (3H, s), 3.20-3.40 (4H, m), 3.80-3.90 (2H, m), 4.54 (2H, s), 7.30 (2H, d, J=8.0Hz), 7.52 (2H, d, J=8.0Hz), 7.55-7.70 (4H, m), 7.85-7.97 (4H, m), 10.20-10.25 (1H, m).

### Working Example 59 (Production of Compound 59)

In DMF (3ml) was dissolved (E)-N-[4-(chloromethyl)-phenyl]-3-(4-methylphenyl)cinnamamide (200mg), and to the solution were added 1-(4-methoxyphenyl)piperazine dihydrochloride (190mg) and potassium carbonate (382mg). The mixture was stirred at room temperature for 14 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[1-(4-methoxyphenyl)-4-piperazinylmethyl]phenyl]-3-(4-methylphenyl)-cinnamamide (Compound 59) (224mg) as colorless crystals.
mp 207-208°C

| Elemental Analysis for C₃₄H₃₅N₃O₂ | | | |
|---|---|---|---|
| Calcd: | C, 78.89; | H, 6.81; | N, 8.12. |
| Found: | C, 78.59; | H, 6.65; | N, 8.13. |

IR (KBr) cm⁻¹: 2937, 2812, 1662, 1626, 1512, 1248, 820, 795 ¹H NMR (200MHz, CDCl₃) δ : 2.41 (3H, s), 2.56-2.65 (4H, m), 3.04-3.13 (4H, m), 3.54 (2H, s), 3.76 (3H, s), 6.61 (1H, d, J=15.6Hz), 6.78-6.-94 (4H, m), 7.23-7.63 (12H, m), 7.73 (1H, s), 7.82 (1H, d, J=15. 6Hz) .

### Working Example 60 (Production of Compound 60)

In DMF (3ml) was dissolved (E)-N-[4-(chloromethyl)-phenyl]-3-(4-methylphenyl)cinnamamide (200mg), and to the solution were added 2-(3,4-dimethoxyphenyl)ethylmethyl-amine (132µl) and potassium carbonate (382mg). The mixture was stirred at room temperature for 12 hours, and to the mixture was added water (50ml). The mixture was extracted with ethyl acetate. The organic layer was washed with saturated sodium chloride solution, dried with anhydrous sodium sulfate, and concentrated under reduced pressure. The residue was separated and purified with column chromatography (ethyl acetate) to give colorless amorphous, which was dissolved in ethyl acetate (50ml), and to the mixture was added 4N hydrochloric acid ethyl acetate solution (0.5ml). The resulting precipitate was filtered and recrystallized from ethyl acetate-methanol to give (E)-N-[4-[N-[2-(3,4-dimethoxyphenyl)ethyl]-N-methyl-ami nomethyl]phenyl]-3-(4-methylphenyl)cinnamamide hydrochloride (Compound 60) (245mg) as colorless crystals.
mp 214-217°C

| Elemental Analysis for C₃₄H₃₆N₂O₃ · 1.0HCl | | | | |
|---|---|---|---|---|
| Calcd: | C, 73.30; | H, 6.69; | N, 5.03; | Cl, 6.36. |
| Found: | C, 73.00; | H, 6.66; | N, 4.99; | Cl, 6.20. |

IR (KBr) cm⁻¹: 3427, 2941, 1682, 1601, 1518, 1417, 1344, 1259, 1174, 1026, 793
¹H NMR (200MHz, DMSO-d₆) δ : 2.37 (3H, s), 2.66-2.75 (3H, m), 2.95-3.40 (4H, m), 3.73 (3H, s), 3.75 (3H, s), 4.15-4.28 (1H, m), 4.32-4.46 (1H, m), 6.77 (1H, dd, J=1.8, 8.2Hz), 6.84-6.94 (2H, m), 7.02 (1H, d, J=16.0Hz), 7.31 (2H, d, J=7.8Hz), 7.48-7.75 (8H, m), 7.79-7.93 (3H, m), 10.56 (2H, s).

### Working Example 61 (Production of Compound 61)

In DMF (3ml) was dissolved (E)-N-[4-(chloromethyl)-phenyl]-3-(4-methylphenyl)cinnamamide (200mg), and to the solution were added methylaminoacetonitrile hydrochloride (77mg) and potassium carbonate (382mg). The mixture was stirred at room temperature for 14 hours, and to the mixture was added water (50ml). The mixture was extracted with ethyl acetate. The organic layer was washed with saturated sodium chloride solution,dried with anhydrous sodium sulfate, and concentrated under reduced pressure. The residue was recrystallized from ethyl acetate-diisopropylether to give (E)-N-[4-[N-(cyanomethyl)-N-methylaminomethyl]phenyl]-3 -(4-methylphenyl)-cinnamamide (Compound 61) (129mg) as colorless crystals.
mp 163-165°C

| Elemental Analysis for C₂₆H₂₅N₃O · 0.1H₂O | | | |
|---|---|---|---|
| Calcd: | C, 78.60; | H, 6.39; | N, 10.58. |
| Found: | C, 78.44; | H, 6.32; | N, 10.35. |

IR (KBr) cm⁻¹: 3250, 3055, 1662, 1626, 1599, 1535, 1516, 1412, 1344, 1184, 982, 822, 791
¹H NMR (200MHz, CDCl₃) δ : 2.42 (3H, s), 2.44 (3H, s), 3.46 (2H, s), 3.59 (2H, s), 6.61 (1H, d, J=15.4Hz), 7.23-7.65 (12H, m), 7.74 (1H, s), 7.83 (1H, d, J=15.4Hz).

### Working Example 62 (Production of Compound 62)

In DMF (3ml) was dissolved (E)-N-[4-(chloromethyl)-phenyl]-3-(4-methylphenyl)cinnamamide (200mg), and to the solution were added imidazole (49mg) and potassium carbonate (382mg). The mixture was stirred at room temperature for 18 hours, and to the mixture was added water. The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[(imidazol-1-yl)methyl]phenyl]-3-(4-methylphen yl)-cinnamamide (Compound 62) (90mg) as colorless crystals.
mp 198-200°C

| Elemental Analysis for C₂₆H₂₃N₃O · 0.3H₂O | | | |
|---|---|---|---|
| Calcd: | C, 78.29; | H, 5.96; | N, 10.53. |
| Found: | C, 78.26; | H, 5.92; | N, 10.17. |

IR (KBr) cm⁻¹ : 3026, 1674, 1628, 1601, 1539, 1518, 1416, 1342, 1182, 1080, 787
¹H NMR (200MHz, CDCl₃) δ : 2.41 (3H, s), 5.08 (2H, s), 6. 67 (1H, d, J=15.4Hz), 6.91 (1H, s), 7.09-7.16 (3H, m), 7.23-7.30 (2H, m), 7.35-7.66 (8H, m), 7.72 (1H, s), 7.82 (1H, d, J=15.4Hz), 8.00 (1H, br s).

### Working Example 63 (Production of Compound 63)

In DMF (3ml) was dissolved (E)-N-[4-(chloromethyl)-phenyl]-3-(4-methylphenyl)cinnamamide (200mg), and to the solution were added 3-(hydroxymethyl)piperidine (191mg). The mixture was stirred at room temperature for 72 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[3-(hydroxy-methyl)piperidinomethyl]phenyl]-3-(4-methylphenyl)-cinnamamide (Compound 63) (160mg) as colorless crystals.
mp 153-154°C

| Elemental Analysis for C₂₉H₃₂N₂O₂ · 0.1H₂O | | | |
|---|---|---|---|
| Calcd: | C, 78.74; | H, 7.34; | N, 6.33. |
| Found: | C, 78.51; | H, 7.32; | N, 6.25. |

IR (KBr) cm⁻¹: 3290, 2924, 1664, 1626, 1603, 1543, 1514, 1412, 1346, 1186, 789
¹H NMR (200MHz, CDCl₃) δ : 1.50-1.90 (3H, m), 2.05-2.35 (4H, m), 2.41 (3H, s), 2.50-2.63 (1H, m), 2.70-2.80 (1H, m), 3.46 (2H, s), 3.50-3.71 (2H, m), 6.65 (1H, d, J=15.6Hz), 7.23-7.31 (4H, m), 7.36-7.61 (7H, m), 7.70-7.87 (3H, m).

### Working Example 64 (Production of Compound 64)

In DMF (3ml) was dissolved (E)-N-[4-(chloromethyl)-phenyl]-3-(4-methylphenyl)cinnamamide (200mg), and to the mixture was added 3-hydroxypiperidine (168mg). The mixture was stirred at room temperature for 13 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-(3-hydroxypiperidino-methyl)phenyl]-3-(4-methy lphenyl)cinnamamide (Compound 64) (174mg) as colorless crystals.
mp 132-134°C

| Elemental Analysis for C₂₈H₃₀N₂O₂ | | | |
|---|---|---|---|
| Calcd: | C, 78.84; | H, 7.09; | N, 6.57. |
| Found: | C, 78.58; | H, 7.08; | N, 6.54. |

IR (KBr) cm⁻¹: 3427, 2937, 1660, 1628, 1601, 1539, 1412, 1344, 1184, 791
¹H NMR (200MHz, DMSO-d₆) δ : 1.28-1.90 (6H, m), 2.36 (3H, s), 2.59-2.68 (1H, m), 2.72-2.85 (1H, m), 3.33 (2H, s), 4.56 (1H, d, J=4.8Hz), 6.93 (1H, d, J=15.8Hz), 7.20-7.35 (4H, m), 7.46-7.71 (8H, m), 7.89 (1H, s), 10.19 (1H, s).

### Working Example 65 (Production of Compound 65)

In DMF (3ml) was dissolved (E)-N-[4-(chloromethyl)-phenyl]-3-(4-methylphenyl)cinnamamide (200mg), and to the mixture was added 2-piperidinemethanol (191mg). Themixture was stirred at room temperature for 13 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[2-(hydroxy-methyl)piperidinomethyl]phenyl]-3-(4-methylphenyl)-cinnamamide (Compound 65) (120mg) as colorless crystals.
mp 137-139°C

| Elemental Analysis for C₂₉H₃₂N₂O₂ | | | |
|---|---|---|---|
| Calcd: | C, 79.06; | H, 7.32; | N, 6.36. |
| Found: | C, 78.73; | H, 7.38; | N, 6.37. |

IR (KBr) cm⁻¹: 3325, 2922, 1664, 1630, 1601, 1531, 1412, 1338, 1174, 974, 793
¹H NMR (200MHz, CDCl₃) δ : 1.30-1.80 (6H, m), 2.10-2.25 (1H, m), 2.40-2.57 (1H, m), 2.41 (3H, s), 2.82-2.93 (1H, m), 3.33 (1H, d, J=13.5Hz), 3.53 (1H, dd, J=4.0, 10.8Hz), 3.88 (1H, dd, J=4.0, 10.8Hz), 4.04 (1H, d, J=13.5Hz), 6.61 (1H, d, J=15.4Hz), 7.23-7.33 (4H, m), 7.37-7.62 (8H, m), 7.74 (1H, s), 7.82 (1H, d, J=15.4Hz).

### Working Example 66 (Production of Compound 66)

In DMF (3ml) was dissolved (E)-N-[4-(chloromethyl)-phenyl]-3-(4-methylphenyl)cinnamamide (200mg), and to the mixture was added 2-(2-hydroxyethyl)piperidine (214mg).

The mixture was stirred at room temperature for 18 hours, and to the mixture was added water (50ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give (E)-N-[4-[2-(2-hydroxyethyl)piperidinomethyl]phenyl]-3-(4-methyl-phenyl)cinnamamide (Compound 66) (202mg) as colorless crystals.
mp 142-143°C

| Elemental Analysis for C₃₀H₃₄N₂O₂ | | | |
|---|---|---|---|
| Calcd: | C, 79.26; | H, 7.54; | N, 6.16. |
| Found: | C, 79.00; | H, 7.27; | N, 6.19. |

IR (KBr) cm⁻¹: 3300, 2935, 1666, 1628, 1603, 1541, 1516, 1412, 1344, 1182, 789
¹H NMR (200MHz, CDCl₃) δ : 1.30-2.13 (8H, m), 2.20-2.35 (1H, m), 2.41 (3H, s), 2.73-2.87 (1H, m), 2.92-3.07 (1H, m), 3.48 (1H, d, J=13.0Hz), 3.70-3.83 (1H, m), 3.90-4.02 (1H, m), 4.14 (1H, d, J=13.0Hz), 6.65 (1H, d, J=15.4Hz), 7.23-7.33 (4H, m), 7.38-7.64 (7H, m), 7.72-7.87 (3H, m).

### Working Example 67 (Production of Compound 67)

In THF (10ml) was dissolved 3-(4-methylphenyl)-cinnamic acid (0.48g), and to the solution were added oxalyl chloride (0-35ml) and a drop of DMF. The mixture was stirred at room temperature for 1 hour and concentrated under reduced pressure . The residue was dissolved in THF (20ml), and to the solution were added 1-(4-aminobenzyl)piperidine (0.38g) and triethylamine (0.34ml) at room temperature. The reaction mixture was stirred at room temperature for 2 hours, and to the mixture was added water (150ml). The mixture was extracted with ethyl acetate. The organic layer was washed with saturated sodium chloride solution, dried with anhydrous magnesium sulfate and concentrated under reduced pressure. The residue was recrystallized from ethyl acetate-diisopropylether to give (E) -N- [4- (piperidinomethyl) -phenyl] -3- (4-methylphenyl) - cinnamamide (Compound 67) (0.60g) as pale yellow crystals.
mp 154-156°C

| Elemental Analysis for C₂₈H₃₀N₂O·0.4H₂O | | | |
|---|---|---|---|
| Calcd: | C, 80.50; | H, 7.43; | N, 6.71. |
| Found: | C, 80.60; | H, 7.28; | N, 6.52. |

¹H NMR (200MHz, CDCl₃) δ : 1.44 (2H, m), 1.58 (4H, m), 2.39 (4H, m), 2.41 (3H, s), 3.47 (2H, s), 6.61 (1H, d, J=15.6Hz), 7.25-7.60 (12H, m), 7.73 (1H, s), 7.82 (1H, d, J=15.6Hz).

### Working Example 68 (Production of Compound 68)

In THF (10ml) was dissolved 3-(2-methylphenyl)-cinnamic acid (0.48g), and to the solution were added oxalyl chloride (0.35ml) and a drop of DMF. The mixture was stirred at room temperature for 1 hour and concentrated under reduced pressure. The residue was dissolved in THF (20ml), and to the solution were added 1-(4-aminobenzyl)piperidine (0.38g) and triethylamine (0.34ml) at room temperature. The reaction mixture was stirred at room temperature for 2 hours, and to the mixture was added water (50ml). The mixture was extracted with ethyl acetate. The organic layer was washed with saturated sodium chloride solution, dried with anhydrous magnesium sulfate and concentrated under reduced pressure. The residue was washed with ethyl acetate-diisopropylether to give (E)-N-[4-(piperidino-methyl)phenyl]-3-(2-methyl-phenyl) -cinnamamide (Compound 68) (0.75g) as pale yellow amorphous.

| Elemental Analysis for C₂₈H₃₀N₂O · 0.5H₂O | | | |
|---|---|---|---|
| Calcd: | C, 80.16; | H, 7.45; | N, 6.68. |
| Found: | C, 80.15; | H, 7.38; | N, 6.64. |

¹H NMR (200MHz, CDCl₃) δ : 1.45 (2H, m), 1. 58 (4H, m), 2.27 (3H, s), 2.39 (2H, m), 3.47 (2H, s), 6.58 (1H, d, J=15.4Hz), 7.24-7.35 (7H, m), 7.39-7.58 (6H, m), 7.80 (1H, d, J=15.6Hz).

### Working Example 69 (Production of Compound 69)

In DMF (4ml) was dissolved (E)-N-[4-(piperidino-methyl)phenyl]-3-(4-methylphenyl)c innamamide (0.41g), and to the mixture was added methyl iodide (0.43g). The mixture was stirred at room temperature for 20 hours and concentrated under reduced pressure. The residue was crystallized from ethyl acetate to give (E)-1-methyl-1-[4-(3-(4-methyl-phenyl)cinnamamido)benzy 1]-piperidinium iodide (Compound 69) (0.51g) as pale yellow crystals.
mp 176-178°C

| Elemental Analysis for C₂₉H₃₃N₂OI ·1.5H₂O | | | |
|---|---|---|---|
| Calcd: | C, 60.10; | H, 6.26; | N, 4.83. |
| Found: | C, 60.19; | H, 6.25; | N, 4.95. |

¹H NMR (200MHz, DMSO-d₆) δ: 1.62 (2H, m), 1.88 (4H, m), 2.37 (3H, s), 2.93 (3H, s), 3.36 (4H, m), 4.55 (2H, s), 6.97 (1H, d, J=15.8Hz), 7.31 (2H, d, J=7.6Hz), 7.50-7.90 (11H, m), 10.44 (1H, s).

### Working Example 70 (Production of Compound 70)

In DMF (6ml) was dissolved (E)-N-[4-(piperidino-methyl)phenyl]-3-(2-methylphenyl)c innamamide (0.62g), and to the mixture was added methyl iodide (0.64g). The mixture was stirred at room temperature for 20 hours and concentrated under reduced pressure. The residue was solidified with ethyl acetate to give (E)-1-methyl-1-[4-(3-(2-methyl-phenyl)cinnamamido)benzy 1]-piperidinium iodide (Compound 70) (0.79g) as pale yellow amorphous.

| Elemental Analysis for C₂₉H₃₃N₂OI · 1.5H₂O | | | |
|---|---|---|---|
| Calcd: | C, 60.10; | H, 6.26; | N, 4.83. |
| Found: | C, 60.00; | H, 6.11; | N, 5.00. |

¹H NMR (200MHz, DMSO-d₆) δ : 1.62 (2H, m), 1.88 (4H, m), 2.27 (3H, s), 2.93 (3H, s), 3.32 (4H, m), 4.56 (2H, s), 6.94 (1H, d, J=15.6Hz), 7.27-7.73 (11H, m), 7.84 (2H, d, J=8.4Hz), 10.40 (1H, s).

### Working Example 71 (Production of Compound 71)

In THF (10ml) was dissolved 3-(2,5-dimethylphenyl)-cinnamic acid (0.50g), and to the solution were added oxalyl chloride (0.35ml) and a drop of DMF. The mixture was stirred at room temperature for 1 hour and concentrated under reduced pressure. The residue was dissolved in THF (20ml), and to the solution were added 1-(4-aminobenzyl)piperidine (0.38g) and triethylamine (0.34ml) at room temperature. The reaction mixture was stirred at room temperature for 2 hours, and to the mixture was added water (50ml). The mixture was extracted with ethyl acetate. The organic layer was washed with saturated sodium chloride solution, dried with anhydrous magnesium sulfate and concentrated under reduced pressure. The residue was washed with ethyl acetate-diisopropylether to give (E)-N-[4-(piperidino-methyl)phenyl]-3-(2,5-dimethylphen yl)cinnamamide (Compound 71) (0.75g) as pale yellow amorphous.

| Elemental Analysis for C₂₉H₃₂N₂O · 0.5H₂O | | | |
|---|---|---|---|
| Calcd: | C, 80.33; | H, 7.67; | N, 6.46. |
| Found: | C, 80.25; | H, 7.34; | N, 6.68. |

¹H NMR (200MHz, CDCl₃) δ: 1.44 (2H, m), 1.61 (4H, m), 2.22 (3H, s), 2.36 (3H, s), 2.47 (4H, m), 3.55 (2H, s), 6.61 (1H, d, J=15.4Hz), 7.05-7.20 (3H, m), 7.28-7.60 (8H, m), 7.71 (1H, s), 7.79 (1H, d, J=15.4Hz).

### Working Example 72 (Production of Compound 72)

In THF (10ml) was dissolved 3-(3-nitrophenyl)cinnamic acid (0.54g), and to the solution were added oxalyl chloride (0.35ml) and a drop of DMF. The mixture was stirred at room temperature for 1 hour and concentrated under reduced pressure. The residue was dissolved in THF (20ml), and to the solution were added 1-(4-aminobenzyl)piperidine (0.38g) and triethylamine (0.34ml) at room temperature. The reaction mixture was stirred at room temperature for 2 hours, and to the mixture was added water (50ml). The mixture was extracted with ethyl acetate. The organic layer was washed with saturated sodium chloride solution, dried with anhydrous magnesium sulfate and concentrated under reduced pressure. The residue was recrystallized from ethyl acetate to give (E)-N-[4-(piperidinomethyl)-phenyl]-3-(3-nitrophenyl)ci nnamamide (Compound 72) (0.65g) as pale yellow crystals.
mp 178-179°C

| Elemental Analysis for C₂₇H₂₇N₃O₃ · 0.5H₂O | | | |
|---|---|---|---|
| Calcd: | C, 71.98; | H, 6.26; | N, 9.33. |
| Found: | C, 71.69; | H, 6.38; | N, 9.44. |

¹H NMR (200MHz, DMSO-d₆) δ: 1.51 (6H, m), 2.33 (4H, m), 3.39 (2H, s), 6.96 (1H, d, J=15.8Hz), 7.24 (2H, d, J=8.0Hz), 7.59-7.83 (7H, m), 8.02 (1H, s), 8.18-8.30 (2H, m), 8.52 (1H, s), 10.18 (1H, s).

### Working Example 73 (Production of Compound 73)

In DMF (6ml) was dissolved (E)-N-[4-(piperidino-methyl)phenyl]-3-(2,5-dimethylphen yl)cinnamamide (0.60g), and to the mixture was added methyl iodide (0.60g). The mixture was stirred at room temperature for 20 hours and concentrated under reduced pressure. The residue was crystallized from ethyl acetate to give (E)-1-methyl-1-[4-(3-(2,5-dimethylphenyl)cinnamamido)be nzyl]-piperidinium iodide (Compound 73) (0.66g) as pale yellow crystals.
mp 145-147°C

| Elemental Analysis for C₃₀H₃₅N₂OI · 1.5H₂O | | | |
|---|---|---|---|
| Calcd: | C, 60.71; | H, 6.45; | N, 4.72. |
| Found: | C, 61.06; | H, 6.10; | N, 4.74. |

¹H NMR (200MHz, DMSO-d₆) δ : 1.62 (2H, m), 1.88 (4H, m), 2.22 (3H, s), 2.33 (3H, s), 2.93 (3H, s), 3.33 (4H, m), 4.55 (2H, s), 6.92 (1H, d, J=15.8Hz), 7.07 (1H, s), 7.15 (2H, ABq, J=7.6Hz), 7.37 (1H, d, J=7.4Hz), 7.48-7.60 (5H, m), 7.67 (1H, d, J=15.6Hz), 7.84 (2H, d, J=8.4Hz), 10.39 (1H, s).

### Working Example 74 (Production of Compound 74)

In DMF (6ml) was dissolved (E) -N- [4- (piperidino-methyl) phenyl] -3- (3-nitrophenyl)cinnamamide (0.59g), and to the mixture was added methyl iodide (0.57g). The mixture was stirred at room temperature for 20 hours and concentrated under reduced pressure. The residue was crystallized from ethyl acetate to give (E)-1-methyl-1-[4-(3-(3-nitro-phenyl)cinnamamido)benzyl ]-piperidinium iodide (Compound 74) (0.75g) as pale yellow crystals.
mp 188-190°C

| Elemental Analysis for C₂₈H₃₀N₃O₃I · 1.5H₂O | | | |
|---|---|---|---|
| Calcd: | C, 55.09; | H, 5.45; | N, 6.88. |
| Found: | C, 54.91; | H, 5.40; | N, 7.23. |

¹H NMR (200MHz, DMSO-d₆) δ : 1.65 (2H, m), 1.90 (4H, m), 2.94 (3H, s), 3.35 (4H, m), 4.56 (2H, s), 6.99(1H, d, J=15.8Hz), 7.49-7.88 (9H, m), 8.04 (1H, s), 8.18-8.29 (2H, m), 8.53 (1H, s), 10.45 (1H, s).

### Working Example 75 (Production of Compound 75)

In toluene(10ml) was dissolved (E) -N- [4- (chloro-methyl) phenyl] -3- (4-methylphenyl)cinnamamide (300mg), and to the mixture was added tributylphosphine (248 *µ*l). The mixture was stirred at 80°C for 3 days and cooled to room temperature. The resulting precipitate was filtered and recrystallized from ethyl acetate-methanol to give (E)-tributyl[4-[3-(4-methylphenyl)cinnamamido]benzyl]-p hosphonium chloride (Compound 75) (389mg) as colorless crystals.
mp 216-217°C .

| Elemental Analysis for C₃₅H₄₇NOClP | | | |
|---|---|---|---|
| Calcd: | C, 74.51; | H, 8.40; | N, 2.48. |
| Found: | C, 74.40; | H, 8.33; | N, 2.63. |

IR (KBr) cm⁻¹: 3429, 2966, 1674, 1630, 1601, 1537, 1516, 1344, 1180, 789
¹H NMR (200MHz, DMSO-d₆) δ : 0.85-1.00 (9H, m), 1.30-1.60 (12H, m), 2.05-2.25 (6H, m), 2.37 (3H, s), 3.79 (2H, d, J=15.2Hz), 7.05 (1H, d, J=15.8Hz), 7.25-7.35 (4H, m), 7.48-7.90 (9H, m), 10.61 (1H, s).

### Working Example 76 (Production of Compound 76)

To 3-phenylcinnamic acid (0.62g) were added thionyl chloride (5ml) and dimethylformamide (catalytic amount), and the mixture was refluxed for 4 hours. The solvent was evaporated, and the residue was dissolved in tetrahydrofuran. The mixture was dropwise added to a suspension of 1-(4-aminobenzyl)piperidine (0.5g) and diisopropylethylamine (1.2ml) in tetrahydrofuran (5ml) under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (methanol/triethylamine/ethyl acetate). The resulting crude crystals was recrystallized from ethyl acetate-hexane to give 1-(4-(3-phenylcinnamoylamino)-benzyl)piperidine (Compound 76) (0.45g) as pale yellow crystals.
mp 159-160°C.
¹H-NMR (δ ppm, CDCl₃) : 1.37-1.48 (2H, m), 1.49-1.63 (4H, m), 2.34-2.42 (4H, m), 3.45 (2H, s), 6.62 (1H, d, J=15.4Hz), 7.23-7.63 (13H, m), 7.76 (1H, s), 7.83 (1H, d, J=15.4Hz). IR (KBr) ν : 2934, 1659, 1624 cm⁻¹.

| Anal. for C₂₇H₂₈N₂O · 0.5H₂O: | | | |
|---|---|---|---|
| Calcd. | C,79.97; | H,7.21; | N,6.91. |
| Found | C,81.09; | H,7.02 ; | N,6.94. |

### Comparative Example 2

A solution of N-(4-chloromethylphenyl)-7-(4-methylphenyl)-2,3-dihydro-1-benzoxepine-4-carboxamide (0.15g) andsodiumphenylsulfide (0.05g) indimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give 7-(4-methylphenyl)-N-(4-(phenyl-thiomethyl)phenyl)-2,3-dihydro-1-benzoxepine-4-carboxamide (0.13g) as colorless crystals.
mp 176-177°C.
¹H-NMR (δ ppm, CDCl₃): 2.39 (3H, s), 3.07 (2H, t, J=4.5Hz), 4.10 (2H, s), 4.35 (2H, t, J=4.5Hz), 7.06 (1H, d, J=8.2Hz), 7.18-7.33 (9H, m), 7.43-7.53 (6H, m), 7.58 (1H, s).
IR(KBr) ν : 1652, 1515cm⁻¹.

| Anal. for C₃₁H₂₇NO₂S: | | | |
|---|---|---|---|
| Calcd. | C,77.96; | H,5.70; | N,2.93. |
| Found | C,77.72; | H,5.57; | N,3.07. |

### Working Example 77 (Production of Compound 77)

A suspension of 1-(4-(3-bromocinnamoylamino)-benzyl)piperidine (0.4g), 4-fluorophenyl borate (0.14g), 1M potassium carbonate (2ml) and ethanol (1ml) in toluene (5ml) was stirred under argon atmosphere at room temperature for 30 minutes. To the suspension was added tetrakistriphenylphosphinepalladium (0.05g), and the mixture was refluxed over night. The mixture was extracted with ethyl acetate, and the organic layer was washed with water and saturated sodium chloride solution, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give 1-(4-(3-(4-fluoro-phenyl)-cinnamoylamino)benzyl)piperid ine (Compound 77) (0.35g) as colorless crystals.
mp 166-167°C.
¹H-NMR (δ ppm, CDCl₃): 1.38-1.50 (2H, m), 1.52-1.65 (4H, m), 2.34-2.39 (4H, m), 3.45 (2H, s), 6.61 (1H, d, J=15.4Hz), 7.10-7.19 (2H, m), 7.30 (2H, d, J=8.0Hz), 7.40-7.58 (8H, m), 7.68 (1H, s), 7.81 (1H, d, J=15.4Hz).
IR(KBr) ν : 3262, 2936, 1663cm⁻¹.

| Anal. for C₂₇H₂₇FN₂O·0.2H₂O: | | | |
|---|---|---|---|
| Calcd. | C,77.56; | H,6.61; | N,6.70. |
| Found | C,77.72; | H,6.49; | N,6.79. |

### Working Example 78 (Production of Compound 78)

A suspension of 1-(4-(3-bromocinnamoylamino)-benzyl)piperidine (0.4g), 4-methoxyphenyl borate (0.14g), 1M potassium carbonate (2ml) and ethanol (1ml) in toluene (5ml) was stirred under argon atmosphere at room temperature for 30 minutes. To the suspension was added tetrakistriphenylphosphinepalladium (0.05g), and the mixture was refluxed over night. The mixture was extracted with ethyl acetate, and the organic layer was washed with water and saturated sodium chloride solution, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give 1-(4-(3-(4-methoxyphenyl)-cinnamoylamino)benzyl)piperid ine (Compound 78) (0.38g) as colorless crystals.
mp 150-151°C.
¹H-NMR(δppm, CDCl₃): 1.38-1.50 (2H, m), 1.51-1.62 (4H, m), 2.35-2.40 (4H, m), 3.46 (2H, s), 3.87 (3H, s), 6.61 (1H, d, J=15.4Hz), 7.00 (2H, d, J=9.0Hz), 7.29-7.36 (3H, m), 7.43-7.58 (7H, m), 7.71 (1H, s), 7.82 (1H, d, J=15.4Hz).
IR (KBr) ν : 3264, 2936, 1663cm⁻¹.

| Anal. for C₂₈H₃₀N₂O₂ : | | | |
|---|---|---|---|
| Calcd. | C, 78.84; | H,7.09; | N,6.57. |
| Found | C,79.07; | H,7.12; | N,6.69. |

### Working Example 79 (Production of Compound 79)

A solution of 1-(4-(3-phenylcinnamoylamino)-benzyl)piperidine (0.32g) and methyl iodide (0.15ml) in dimethylformamide (5ml) was stirred over night under nitrogen atmosphere at room temperature. The solvent was evaporated, and to the residue was added ethyl acetate. Precipitated crude crystal was filtered, which were recrystallized from ethanol to give 1-methyl-1-(4-(3-phenylcinnamoylamino)-benzyl)piperidin ium iodide (Compound 79) (0.26g) as colorless crystals.
mp 194-195°C.
¹H-NMR(δ ppm, DMSO-d₆) : 1.45-1.65 (2H, m), 1.75-1.95 (4H, m), 2.92 (3H, s), 3.24-3.28 (4H, m), 4.54 (2H, s), 6.97 (1H, d, J=15.8Hz), 7.41-7.93 (14H, m), 10.44 (1H,s).
IR (KBr) ν : 3241, 1682cm⁻¹.

| | | | |
|---|---|---|---|
| Anal. for C₂₈H₃₁IN₂O: | | | |
| Calcd. | C, 62.46; | H,5.80; | N,5.20. |
| Found | C,62.19; | H,5.74; | N,5.10. |

### Comparative Example 3

A solution of N-(4-chloromethylphenyl)-7-(4-methylphenyl)-2,3-dihydro-1-benzoxepine-4-carboxamide (0.15g) and sodium benzyl sulfide (0.055g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-(benzylthiomethyl)phenyl)-7-(4-methylphenyl)-2,3-d ihydro-1-benzoxepine-4-carboxamide (0.17g) as colorless crystals.
mp 145-146°C.
¹H-NMR(δ ppm, CDCl₃) : 2.39 (3H, s), 3.07 (2H, t, J=4.7Hz), 3.59 (2H, s), 3.60 (2H, s), 4.35 (2H, t, J=4.7Hz), 7.06 (1H, d, J=8.0Hz), 7.22-7.32 (9H, m), 7.43-7.57 (6H, m), 7.61 (1H, s).
IR(KBr) ν : 3028, 1646, 1515cm⁻¹.

| Anal. for C₃₂H₂₉NO₂S·0.5H₂O: | | | |
|---|---|---|---|
| Calcd. | C,76.77; | H,6.04; | N,2.80. |
| Found | C,77.07; | H,5.96; | N,2.95. |

### Working Example 80 (Production of Compound 80)

A solution of 1-(4-(3-(4-fluoro-phenyl)-cinnamoylamino)benzyl)piperidine (0.25g) and methyl iodide (0.2ml) in dimethylformamide (5ml) was stirred at room temperature over night. The solvent was evaporated, and to the residue was added ethyl acetate. Precipitated crude crystal was filtered, which were recrystallized from ethanol to give 1-methyl-1-(4-(3-(4-fluorophenyl)cinnamoylamino)-benzyl)piperidinium iodide (Compound 80) (0.27g) as pale brown crystals.
mp 204-205°C.
¹H-NMR (δ ppm, DMSO-d₆): 1.42-1.75 (2H, m), 1.78-1.95 (4H, m), 2.91 (3H, s), 3.22-3.32 (4H, m), 4.52 (2H, s), 6.95 (1H, d, J=15.8 Hz), 7.29-7.38 (2H, m), 7.48-7.91 (11H, m), 10.44 (1H, s).
IR(KBr) ν : 3237, 1682cm⁻¹.

| Anal. for C₂₈H₃₀FIN₂O·0.5H₂O: | | | |
|---|---|---|---|
| Calcd. | C,59.47; | H,5.53; | N,4.95. |
| Found | C,59.49; | H,5.35; | N,4.98. |

### Working Example 81 (Production of Compound 81)

A solution of 1-(4-(3-(4-methoxyphenyl)cinnamoylamino)benzyl)piperidine (0.32g) and methyl iodide (0.2ml) in dimethylformamide (5ml) was stirred at room temperature over night. The solvent was evaporated, and to the residue was added ethyl acetate. Precipitated crude crystal was filtered, which were recrystallized from ethanol-hexane to give 1-methyl-1-(4-(3-(4-methoxyphenyl)cinnamoylamino)-benzy 1)piperidinium iodide (Compound 81) (0.33g) as pale brown crystals.
mp 208-209°C.
¹H-NMR (δ ppm, DMSO-d₆): 1.45-1.68 (2H, m), 1.78-1.95 (4H, m), 2.91 (3H, s), 3.24-3.34 (4H, m), 3.82 (3H, s), 4.53 (2H, s), 6.95 (1H, d, J=15.8Hz), 7.06 (2H, d, J=8.6Hz), 7.43-7.57 (4H, m), 7.61-7.74 (4H, m), 7.84 (2H, d, J=8.6Hz), 7.88 (1H, s), 10.45 (1H, s).
IR (KBr) ν : 3243, 1682 cm⁻¹.

| Anal. for C₂₉H₃₃IN₂O₂ : | | | |
|---|---|---|---|
| Calcd. | C,61.27; | H,5.85; | N,4.93. |
| Found | C,60.87; | H,5.83; | N,4.88. |

### Working Example 82 (Production of Compound 82)

To 3,4-dihydro-7-phenylnaphthalene-2-carboxylic acid (0.25g) were added thionyl chloride (5ml) and dimethylformamide (catalytic amount), and the mixture was refluxed for 3 hours. The solvent was evaporated, and the residue was dissolved in tetrahydrofuran. The mixture was dropwise added to a suspension of 2-(4-aminobenzyl)-1,3-dimethyl-1,3,2-diazaphosphorinane-2-oxide (0.25g) and diisopropylethylamine (0.5ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated. Precipitated crude crystal was recrystallized from ethanol-hexane to give 2-(4-(3,4-dihydro-7-phenyl-naphthalene-2-carbonyl-amino )benzyl)-1,3-dimethyl-1,3,2-diazaphosphorinane-2-oxide (Compound 82) (0.35g) as colorless crystals.
mp 249-250°C.
¹H-NMR(δ ppm, CDCl₃) : 1.10-1.30 (1H, m), 1.65-1.85 (1H, m), 2.65 (3H, s), 2.69 (3H, s), 2.73-3.07 (8H, m), 3.17 (2H, d, J=17.4Hz), 7.18 (2H, dd, J=2.6, 8.8Hz), 7.29-7.60 (11H, m), 7.70 (1H, s).
IR(KBr) ν : 3283, 2940, 2886, 2832, 1655cm⁻¹.

| Anal. for C₂₉H₃₂N₃O₂P · 0.2H₂O: | | | |
|---|---|---|---|
| Calcd. | C,71.21; | H,6.68; | N,8.59. |
| Found | C,71.12; | H,6.57; | N,8.52. |

### Working Example 83 (Production of Compound 83)

To 3,4-dihydro-7-phenylnaphthalene-2-carboxylic acid (0.35g) were added thionyl chloride (10ml) and dimethylformamide (catalytic amount), and the mixture was refluxed for 2.5 hours. The solvent was evaporated, and the residue was dissolved in tetrahydrofuran. The mixture was dropwise added a suspension of 2-(4-aminobenzyl)-1,3-dimethyl-1,3,2-diazaphosphorane-2 -oxide (0.33g) and diisopropylethylamine (0.75ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, anddriedwithanhydrousmagnesiumsulfate. Under reduced pressure, the solvent was evaporated. Precipitated crude crystal was recrystallized from ethanol-hexane to give 2-(4-(3,4-dihydro-7-phenyl-naphthalene-2-carbonyl-amino )benzyl)-1,3-dimethyl-1,3,2-diaza-phosphorane-2-oxide (Compound 83) (0.24g) as colorless crystals.
mp 212-213°C.
¹H-NMR (δ ppm, CDCl₃) : 2.61 (3H, s), 2.65-2.76 (2H, m), 2.66 (3H, s), 2.94-3.07 (2H, m), 3.22 (2H, d, J=18.6Hz), 7.19 (2H, dd, J=2.6, 8.6Hz), 7.29-7.60 (11H, m), 7.72 (1H, s).
IR(KBr) ν : 3254, 2928, 2897, 1655cm⁻¹.

| Anal. for C₂₈H₃₀N₃O₂P · 0.5H₂O : | | | |
|---|---|---|---|
| Calcd. | C,69.98 ; | H,6.50; | N,8.74. |

| | | | |
|---|---|---|---|
| Found | C,70.27; | H,6.32; | N,8.53. |

### Working Example 84 (Production of Compound 84)

To a solution of 2-(4-methylphenyl)-6,7-dihydro-5H-benzocycloheptene-8-c arboxylic acid (0.25g) in dichloromethane (5ml) were added oxalyl chloride (0.4ml) and dimethylformamide (catalytic amount) under ice-cooling, and the mixture was stirred at 40°C for 1 hour. The solvent was evaporated, and the residue was dissolved in tetra-hydrofuran. The mixture was dropwise added to a solution of 1- (4-aminobenzyl) piperidine (0.17g) and diisopropyl-ethylamine (0.5ml) in tetrahydrofuran (10ml), under ice-cooling. Under nitrogen atmosphere, the mixture was stirred at room temperature over night. The solvent was evaporated, and to the residue was added water. The mixture was extracted with dichloromethane, and the organic layer was washed with water and dried with anhydrous magnesium sulfate. Under reduced pressure, the solvent was evaporated, and precipitated crude crystal was recrystallized from dichloromethane-hexane to give 2-(4-methylphenyl)-N-(4-piperidinomethylphenyl)-6,7-dih ydro-5H-benzocycloheptene-8-carboxamide (Compound 84) (0.36g) as colorless crystals.
mp 192-193°C.
¹H-NMR (δ ppm, CDCl₃) : 1.38-1.50 (2H, m), 1.50-1.63 (4H, m), 2.13-2.22 (2H, m), 2.35-2.39 (4H, m), 2.40 (3H, s), 2.72 (2H, t, J=6.4Hz), 2.85-2.91 (2H, m), 3.46 (2H, s), 7.21-7.33 (5H, m), 7.41-7.57 (6H, m), 7.63 (1H, s).
IR(KBr) ν : 3352, 2932, 1647cm⁻¹.

| Anal. for C₃₁H₃₄N₂O · 0.2H₂O: | | | |
|---|---|---|---|
| Calcd. | C,81.97; | H,7.63; | N,6.17. |
| Found | C,81.88; | H,7.52; | N,6.22. |

### Working Example 85 (Production of Compound 85)

A solution of 2-(4-methylphenyl)-N-(4-piperidino-methylphenyl)-6,7-di hydro-5H-benzocycloheptene-8-carboxamide (0.26g) and methyl iodide (0.15ml) in dimethylformamide (15ml) was stirred at room temperature over night. The solvent was evaporated, and to the residue was added ethyl acetate. Precipitated crude crystal was filtered, which were recrystallized from ethanol-ethyl acetate to give 1-(N-(2-(4-methylphenyl)-6,7-dihydro-5H-benzocyclohepte ne-8-carbonyl)-4-aminobenzyl)-1-methylpiperidinium iodide (Compound 85) (0.3g) as colorless crystals.
mp 220-221°C (dec.).
¹H-NMR (δ ppm, DMSO-d₆) : 1.45-1.65 (2H, m), 1.80-1.94 (4H, m), 1.99-2.09 (2H, m), 2.35 (3H, s), 2.64 (2H, t, J=6. 1Hz), 2.83-2.88 (2H, m), 2.91 (3H, s), 3.23-3.29 (4H, m), 4.53 (2H, s), 7.26-7.38 (4H, m), 7.48-7.68 (6H, m), 7.87 (2H, d, J=8.6Hz), 10.23 (1H, s).
IR(KBr) v : 3285, 2946, 1651cm⁻¹.

| Anal. for C₃₂H₃₇IN₂O·0.5H₂O : | | | |
|---|---|---|---|
| Calcd. | C,63.89; | H,6.37; | N,4.66. |
| Found | C,63.94; | H,6.33; | N,4.60. |

### Working Example 86 (Production of Compound 86)

To a solution of 7-(4-methylphenyl)-N-(4-hydroxy-methylphenyl)-2,3-dihyd ro-1-benzothiepine-4-carboxamide (0.2g), triethylamine (0.21ml) and dimethylaminopyridine (catalytic amount) in tetrahydrofuran (10ml) was dropwise added methane-sulfonylchloride (0.06ml) under ice-cooling, and the mixture was stirred for 10 minutes. To the mixture was added piperidine (0.15ml), and the mixture was stirred at room temperature for 2 hours. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give 7-(4-methylphenyl)-N-(4-piperidinomethylphenyl)-2,3-dih ydro-1-benzothiepine-4-carboxamide (Compound 86) (0.19g) as colorless crystals.
mp 203-204°C.
¹H-NMR (δ ppm, CDCl₃) : 1 . 35-1 . 50 (2H, m), 1.55-1.63 (4H, m), 2.38-2.40 (4H, m), 2.40 (3H, s), 3.08 (2H, t, J=5.7Hz), 3.29 (2H, t, J=5.7Hz), 3.47 (2H, s), 7.24-7.46 (7H, m), 7.50-7.58 (5H, m), 7.68 (1H, s).
IR (KBr) ν : 2934, 1651cm⁻¹.

| Anal. for C₃₀H₃₂N₂OS·0.2H₂O : | | | |
|---|---|---|---|
| Calcd. | C,76.30; | H,6.92; | N,5.93. |
| Found | C,76.27; | H,6.77; | N,6.06. |

### Working Example 87 (Production of Compound 87)

A solution of 7-(4-methylphenyl)-N-(4-piperidino-methyl-phenyl)-2,3-d ihydro-1-benzothiepine-4-carboxamide (0.08g) and methyl iodide (0.013ml) in dimethylformamide (20ml) was stirred at room temperature over night. The solvent was evaporated, and to the residue was added ethyl acetate. Precipitated crude crystal was filtered, which were recrystallized from ethanol-hexane to give 1-(N-(7-(4-methylphenyl)-2,3-dihydro-1-benzo-thiepine-4 -carbonyl)-4-aminobenzyl)-1-methyl-piperidinium iodide (Compound 87) (0.077g) as colorless crystals.
mp 196-197°C.
¹H-NMR(δppm, DMSO-d₆): 1.45-1.65 (2H, m), 1.80-1.95 (4H, m), 2.35 (3H, s), 2.91 (3H, s), 2.99-3.05 (2H, m), 3.15-3.29 (6H, m), 4.53 (2H, s), 7.29 (2H, d, J=8.2Hz), 7.46-7.63 (7H, m), 7.82-7.89 (3H, m), 10.34 (1H, s).
IR(KBr) ν : 3284, 2947, 1652cm⁻¹.

| Anal. for C₃₁H₃₅IN₂OS·0.5H₂O : | | | |
|---|---|---|---|
| Calcd. | C,60.09; | H, 5.86 ; | N,4.52. |
| Found | C,60.03; | H,5.57; | N,4.44. |

### Working Example 88 (Production of Compound 88)

To a suspension of 7-(4-methylphenyl)-2,3-dihydro-1-benzoxepine-4-carboxyl ic acid (1.0g) in dichloromethane (30ml) were added oxalyl chloride (0.93ml) and dimethylformamide (catalytic amount), under ice-cooling, and the mixture was stirred at room temperature for 2 hours. The solvent was evaporated, and the residue was dissolved in tetrahydrofuran. The mixture was dropwise added to a solution of 1-(4-amino-benzyl)piperidine (0.75g) and triethylamine (1.5ml) in tetra-hydrofuran (50ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals which were recrystallized from ethyl acetate-hexane to give 7-(4-methyl-phenyl)-N-(4-((piperidinomethyl)phenyl)-2,3 -dihydro-1-benzoxepine-4-carboxamide (Compound 88) (1.45g) as colorless crystals.
mp 188-189°C.
¹H-NMR(δ ppm, CDCl₃) : 1.40-1.47 (2H, m), 1.52-1.60 (4H, m), 2.34-2.39 (4H, m), 2.39 (3H, s), 3.07 (2H, t, J=4.4Hz), 3.46 (2H, s), 4.36 (2H, t, J=4.4Hz), 7.06 (1H, d, J=8.4Hz), 7.22-7.33 (5H, m), 7.43-7.58 (6H, m).
IR (KBr) ν : 2935, 1652cm⁻¹.

| Anal. for C₃₀H₃₂N₂O₂: | | | |
|---|---|---|---|
| Calcd. | C, 79.61; | H,7.13; | N,6.19. |
| Found | C,79.53; | H,6.91; | N,6.22: |

### Working Example 89 (Production of Compound 89)

A solution of 7-(4-methylphenyl)-N-(4-(piperidino-methyl)phenyl)-2,3-dihydro-1-benzoxepine-4-carboxamide (1.4g) and methyl iodide (0.58ml) in dimethylformamide (50ml) was stirred at room temperature over night. The solvent was evaporated, and to the residue was added ethyl acetate. Precipitated crude crystal was filtered, which were recrystallized from ethanol-ethyl acetate to give 1-(N-(7-(4-methylphenyl)-2,3-dihydro-1-benzoxepin-4-car bonyl)-4-aminobenzyl)-1-methylpiperidinium iodide (Compound 89) (1.6g) as colorless crystals.
mp 227-228°C (dec.).
¹H-NMR (δ ppm, DMSO-d₆) : 1.45-1.70 (2H, m), 1.70-1.95 (4H, m), 2.34 (3H, s), 2.91 (3H, s), 3.00 (2H, br), 3.24-3.34 (4H, m), 4.31 (2H, br), 4.53 (2H, s), 7.06 (1H, d, J=8.4Hz), 7.27 (2H, d, J=8.0Hz), 7.36 (1H, s), 7.48-7.59 (5H, m), 7.75 (1H, s), 7.86 (2H, d, J=8.8Hz), 10.19 (1H, s).
IR(KBr) ν : 3289, 2938, 1649cm⁻¹.

| Anal. for C₃₁H₃₅IN₂O₂ : | | | |
|---|---|---|---|
| Calcd. | C,62.63; | H,5.93; | N,4.71. |
| Found | C,62.43; | H,5.91; | N,4.52. |

### Working Example 90 (Production of Compound 90)

A solution of N-(4-chloromethylphenyl)-7-(4-methylphenyl)-2,3-dihydro-1-benzoxepine-4-carboxamide (0.15g) and 1-methylpiperidine (0.14ml) indimethylformamide(15ml) was stirred at room temperature over night. The solvent was evaporated, and to the residue was added ethyl acetate. Precipitated crude crystal was filtered, which were recrystallized from ethanol-diethylether to give 1-(N-(7-(4-methylphenyl)-2,3-dihydro-1-benzoxepin-4-car bonyl)-4-aminobenzyl)-1-methylpiperidinium chloride (Compound 90) (0.15g) as colorless crystals.
mp 231-232°C.
¹H-NMR(δppm, DMSO-d₆) : 1.45-1.65 (2H, m), 1.80-1.95 (4H, m), 2.34 (3H, s), 2.91 (3H, s), 2.97-3.05 (2H, m), 3.23-3.30 (4H, m), 4.25-4.35 (2H, m), 4.53 (2H, s), 7.06 (1H, d, J=8.4Hz), 7.27 (2H, d, J=8.4Hz), 7.38 (1H, s), 7.48-7.59 (5H, m), 7.75 (1H, s), 7.86 (2H, d, J=8.8Hz), 10.23 (1H, s).
IR(KBr) ν : 3227, 2969, 1665cm⁻¹.

| Anal. for C₃₁H₃₅ClN₂O₂·0.5H₂O: | | | |
|---|---|---|---|
| Calcd. | C,72.71; | H,7.09; | N,5.47. |
| Found | C,72.85; | H,6.93; | N,5.48. |

### Working Example 91 (Production of Compound 91)

A solution of N-(4-chloromethylphenyl)-7-(4-methylphenyl)-2,3-dihydro-1-benzoxepine-4-carboxamide (0.18g) and 1-ethylpiperidine (0.31ml) in dimethylformamide (5ml) were stirred at 50°C over night. The solvent was evaporated, and to the residue was added ethyl acetate. Precipitated crude crystal was filtered, which were recrystallized from ethanol-ethyl acetate to give 1-(N-(7-(4-methylphenyl)-2,3-dihydro-1-benzoxepin-4-car bonyl)-4-amino-benzyl)-1-ethylpiperidinium chloride (Compound 91) (0.17g) as colorless crystals.
mp 209-210°C.
¹H-NMR(δppm, DMSO-d₆): 1.34 (3H, t, J=6.9Hz), 1.38-1.66 (2H, m), 1.80-1.99 (4H, m), 2.34 (3H, s), 3.00 (2H, t, J=4.2Hz), 3.13-3.31 (6H, m), 4.30 (2H, t, J=4.2Hz), 4.50 (2H, s), 7.06 (1H, d, J=8.4Hz), 7.27 (2H, d, J=8.0Hz), 7.39 (1H, s), 7.46-7.59 (5H, m), 7.76 (1H, d, J=2.2Hz), 7.87 (2H, d, J=8.8Hz), 10.24 (1H, s).
IR(KBr) ν : 3202, 2946, 1645cm⁻¹.

| Anal. for C₃₂H₃₇ClN₂O₂·0.3H₂O : | | | |
|---|---|---|---|
| Calcd. | C,73.56; | H,7.25; | N,5.36. |
| Found | C,73.59; | H,7.26; | N,5.32. |

### Working Example 92 (Production of Compound 92)

To a suspension of 7-(4-methylphenyl)-2-phenyl-2,3-dihydro-1-benzoxepine-4 -carboxylic acid (0.1g) in dichloro-methane (10ml) were added oxalyl chloride (0.1ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-(N-methyl-N-(tetrahydropyran-4-yl)amino-methyl)anilin e (0.06g) and triethylamine (0.12ml) in tetrahydrofuran (5ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from ethyl acetate-hexane to give 7-(4-methylphenyl)-2-phenyl-N-(4-((N-tetrahydropyran-4-yl-N-methylamino)methyl)phenyl)-2,3-dihydro-1-benzoxepi ne-4-carboxamide (Compound 92) (0.11g) as colorless crystals.
mp 178-179°C.
¹H-NMR (δ ppm, CDCl₃) : 1.63-1.74 (4H, m), 2.20 (3H, s), 2.40 (3H, s), 2.56-2.66 (1H, m), 3.15-3.43 (4H, m), 3.56 (2H, s), 4.01-4.05 (2H, m), 5.09 (1H, dd, J=2.2, 8.4Hz), 7.10 (1H, d, J=8.4Hz), 7.17-7.57 (16H, m).
IR(KBr) ν : 2949, 2844, 1652cm⁻¹.

| Anal. for C₃₇H₃₈N₂O₃ : | | | |
|---|---|---|---|
| Calcd. | C,79.54; | H,6.86; | N,5.01. |
| Found | C,79.28; | H,6.96; | N,4.97. |

### Working Example 93 (Production of Compound 93)

To a suspension of 7-(4-methylphenyl)-2-phenyl-2,3-dihydro-1-benzoxepine-4 -carboxylic acid (0.1g) in dichloro-methane (10ml) were added oxalyl chloride (0.1ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 1-(4-amino-benzyl)piperidine (0.06g) and triethylamine (0.12ml) in tetrahydrofuran (5ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from ethyl acetate-hexane to give 7-(4-methylphenyl)-2-phenyl-N-(4-(piperidinomethyl)phen yl)-2,3-dihydro-1-benzoxepine-4-carboxamide (Compound 93) (0.12g) as colorless crystals.
mp 210-211°C.
¹H-NMR (δ ppm, CDCl₃) : 1.40-1.47 (2H, m), 1.52-1.62 (4H, m), 2.34-2.40 (4H, m), 2.40 (3H, s), 3.23-3.31 (2H, m), 3.45 (2H, s), 5.09 (1H, dd, J=2.0, 8.8Hz), 7.10 (1H, d, J=8.4Hz), 7.23-7.56 (16H, m).
IR(KBr) ν : 2935, 1652cm⁻¹.

| Anal. for C₃₆H₃₆N₂O₂: | | | |
|---|---|---|---|
| Calcd. | C,81.79; | H,6.86; | N,5.30. |
| Found | C,81.45; | H,6.82; | N,5.28. |

### Working Example 94 (Production of Compound 94)

A solution of 7-(4-methylphenyl)-2-phenyl-N-(4-(piperidinomethyl)phen yl)-2,3-dihydro-1-benzoxepine-4-carboxamide (0.08g) and methyl iodide (0.05ml) in dimethylformamide (15ml) was stirred at room temperature over night.

The solvent was evaporated, and to the residue was added ethyl acetate. Precipitated crude crystal was filtered, which were recrystallized from ethanol-ethyl acetate to give 1-(N-(7-(4-methylphenyl)-2-phenyl-2,3-dihydro-1-benzoxe pin-4-carbonyl)-4-aminobenzyl)-1-methyl-piperidinium iodide (Compound 94) (0.057g) as colorless crystals.
mp 232-233°C (dec.).
¹H-NMR(δppm, DMSO-d₆): 1.45-1.70 (2H, m), 1.75-1.95 (4H, m), 2.35 (3H, s), 2.91 (3H, s), 3.25-3.44 (6H, m), 4.53 (2H, s), 5.12 (1H, t, J=5.0Hz), 7.09 (1H, d, J=8.4Hz), 7.28 (2H, d, J=8.2Hz), 7.37-7.61 (11H, m), 7.81-7.87 (3H, m), 10.20 (1H, s).
IR (KBr) ν : 2949, 1650cm⁻¹.

| Anal. for C₃₇H₃₉IN₂O₂ · 0.2H₂O : | | | |
|---|---|---|---|
| Calcd. | C,65.91; | H,5.89; | N,4.15. |
| Found | C,65.80; | H,5.84; | N,4.17. |

### Working Example 95 (Production of Compound 95)

To a suspension of 7-(4-methylphenyl)-2-methyl-2,3-dihydro-1-benzoxepine-4 -carboxylic acid (0.1g) in dichloro-methane (5ml) were added oxalyl chloride (0.1ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-(N-methyl-N-(tetrahydropyran-4-yl)amino-methyl)anilin e (0.08g) and triethylamine (0.14ml) in tetrahydrofuran (5ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give 7-(4-methylphenyl)-2-methyl-N-(4-((N-tetrahydropyran-4-yl-N-methylamino)methyl)phenyl)-2,3-dihydro-1-benzoxepi ne-4-carboxamide (Compound 95) (0.12g) as colorless crystals.
mp 170-171°C.
¹H-NMR(δ ppm, CDCl₃): 1.54 (3H, d, J=6.4Hz), 1.60-1.78 (4H, m), 2.22 (3H, s), 2.39 (3H, s), 2.63-2.68 (1H, m), 2.85 (1H, ddd, J=2.6, 9.2, 17.6Hz), 3.14 (1H, d, J=17.6Hz), 3.37 (2H, dt, J=2.8, 11.3Hz), 3.58 (2H, s), 4.01-4.07 (2H, m), 4.24-4.30 (1H, m), 7.05 (1H, d, J=8.4Hz), 7.22-7.34 (4H, m), 7.43-7.56 (7H, m).
IR(KBr) ν : 2951, 2845, 1651cm⁻¹.

| Anal. for C₃₂H₃₆N₂O₃: | | | |
|---|---|---|---|
| Calcd. | C,77.39; | H,7.31; | N,5.64. |
| Found | C,77.21; | H,7.43; | N,5.51. |

### Working Example 96 (Production of Compound 96)

To a suspension of 7-(4-methylphenyl)-2-methyl-2,3-dihydro-1-benzoxepine-4 -carboxylic acid (0.1g) in dichloro-methane (5ml) were added oxalyl chloride (0.1ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 1-(4-aminobenzyl)piperidine (0.07g) and triethylamine (0.14ml) intetrahydrofuran (5ml), underice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give 7-(4-methylphenyl)-2-methyl-N-(4-(piperidinomethyl)-phe nyl)-2,3-dihydro-1-benzoxepine-4-carboxamide (Compound 96) (0.12g) as colorless crystals.
mp 175-176°C.
¹H-NMR (δ ppm, CDCl₃) : 1.40-1.45 (2H, m), 1.54 (3H, d, J=6.2Hz), 1.53-1.61 (4H, m), 2.30-2.40 (4H, m), 2.39 (3H, s), 2.85 (1H, ddd, J=2.6, 8.8, 18.0Hz), 3.14 (1H, d, J=18.0Hz), 3.47 (2H, s), 4.23-4.30 (1H, m), 7.05 (1H, d, J=8.8Hz), 7.16-7.36 (4H, m), 7.43-7.55 (7H, m).
IR(KBr) ν : 2936, 1651cm⁻¹.

| Anal. for C₃₁H₃₄N₂O₂: | | | |
|---|---|---|---|
| Calcd. | C,79.79; | H,7.34; | N,6.00. |
| Found | C,79.53; | H,7.35; | N,5.82. |

### Working Example 97 (Production of Compound 97)

A solution of N-(4-chloromethylphenyl)-7-(4-methylphenyl)-2,3-dihydro-1-benzoxepine-4-carboxamide (0.1g) and N-methylcyclopentylamine (0.07g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethanol-hexane to give N-(4-((N-cyclopentyl-N-methyl)amino-methyl)phenyl)-7-(4 -methylphenyl)-2,3-dihydro-1-benzoxepine-4-carboxamide (Compound 97) (0.1g) as colorless crystals.
mp 171-172°C.
¹H-NMR(δ ppm, CDCl₃) : 1.45-1.75 (6H, m), 1.80-1.95 (2H, m), 2.13 (3H, s), 2.39 (3H, s), 2.70-2.80 (1H, m), 3.08 (2H, t, J=4.6Hz), 3.50 (2H, s), 4.35 (2H, t, J=4.6Hz), 7.06 (1H, d, J=8.0Hz), 7.22-7.33 (4H, m), 7.43-7.58 (7H, m).
IR(KBr) ν: 3340, 2958, 1646cm⁻¹.

| Anal. for C₃₁H₃₄N₂O₂·0.2H₂O : | | | |
|---|---|---|---|
| Calcd. | C,79.18; | H,7.37; | N,5.96. |
| Found | C,79.15; | H,7.18; | N,5.96. |

### Working Example 98 (Production of Compound 98)

To a solution of N-(4-hydroxymethylphenyl)-7-(4-methylphenyl)-2,3-dihydr o-1-benzoxepine-4-carboxamide (0.15g), triethylamine (0.14ml) and 4-dimethylamino-pyridine (catalytic amount) in dichloromethane was dropwise added methanesulfonyl chloride (0.04ml) under ice-cooling, and the mixture was stirred for 15 minutes. To the mixture was added N-methylcyclohexylamine (0.15ml), and the mixture was stirred at room temperature over night. The solvent was evaporated, and the residue was purified with silica gel column (ethyl acetate/methanol/triethylamine) to give crude crystals, which were recrystallized from ethyl acetate-hexane to give N-(4-((N-cyclohexyl-N-methyl)-aminomethyl)phenyl)-7-(4-methylphenyl)-2,3-dihydro-1-benzoxepine-4-carboxamide (Compound 98) (0.03g) as colorless crystals.
mp 176-177°C.
¹H-NMR (δ ppm, CDCl₃): 1.15-1.35 (6H, m), 1.70-1.95 (4H, m), 2.23 (3H, s), 2.39 (3H, s), 2.39-2.55 (1H, m), 3.08 (2H, t, J=4.6Hz), 3.59 (2H, s), 4.37 (2H, t, J=4.6Hz), 7.06 (1H, d, J=8.0Hz), 7.23-7.35 (5H, m), 7.44-7.58 (7H, m).
IR(KBr) ν : 2930, 2853, 1651cm⁻¹.

| Anal. for C₃₂H₃₆N₂O₂·0.4H₂O: | | | |
|---|---|---|---|
| Calcd. | C,78.78; | H,7.60; | N,5.74. |
| Found | C,78.97; | H,7.49; | N,5.94. |

### Working Example 99 (Production of Compound 99)

A solution of N-(4-chloromethylphenyl)-7-(4-methylphenyl)-2,3-dihydro-1-benzoxepine-4-carboxamide (0.09g), N-methylcycloheptylamine (0.04g) and potassium carbonate (0.1g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-cycloheptyl-N-methyl)aminomethyl)phenyl)-7-(4-methylphenyl)-2,3-dihydro-1-benzoxepine-4-carboxamide (Compound 99) (0.08g) as colorless crystals.
mp 167-168°C.
¹H-NMR (δ ppm, CDCl₃) : 1.35-1.55 (8H, m), 1.55-1.80 (2H, m), 1.80-1.95 (2H, m), 2.16 (3H, s), 2.39 (3H, s), 2.55-2.70 (1H, m), 3.08 (2H, t, J=4.6Hz), 3.49 (2H, s), 4.35 (2H, t, J=4.6Hz), 7.05 (1H, d, J=8.4Hz), 7.22-7.33 (4H, m), 7.43-7.58 (7H, m).
IR (KBr) ν : 2927, 1650cm⁻¹.

| Anal. for C₃₃H₃₈N₂O₂·0.1H₂O : | | | |
|---|---|---|---|
| Calcd. | C,79.83; | H,7.76; | N,5.64. |
| Found | C,79.62; | H,7.43; | N,5.53. |

### Working Example 100 (Production of Compound 100)

A solution of N-(4-chloromethylphenyl)-7-(4-methylphenyl)-2,3-dihydro-1-benzoxepine-4-carboxamide (0.15g) and cyclohexylamine (0.17ml) in dimethylformamide (10ml) was stirred at room temperature for 2.5 hours. The solvent was evaporated, and the residue was purified with silica gel column (ethyl acetate/methanol/triethylamine) to give crude crystals, which were recrystallized from ethanol-hexane to give N-(4-((cyclohexylamino)methyl)phenyl)-7-(4-methylphenyl )-2,3-dihydro-1-benzoxepine-4-carboxamide (Compound 100) (0.09g) as colorless crystals.
mp 183-184°C .
¹H-NMR(δ ppm, CDCl₃): 1.17-1.30 (6H, m), 1.58-1.82 (4H, m), 2.39 (3H, s), 2.45-2.60 (1H, m), 3.08 (2H, t, J=4.6Hz), 3.81 (2H, s), 4.35 (2H, t, J=4.6Hz), 7.05 (1H, d, J=8.4Hz), 7.22-7.34 (5H, m), 7.43-7.55 (6H, m), 7.72 (1H, s).
IR(KBr) ν : 2928, 2853, 1647cm⁻¹.

| Anal. for C₃₁H₃₄N₂O₂ · 0.5H₂O: | | | |
|---|---|---|---|
| Calcd. | C,78.28; | H,7.42; | N,5.89. |
| Found | C,78.56; | H,7.12; | N,6.01. |

### Working Example 101 (Production of Compound 101)

A solution of N-(4-chloromethylphenyl)-7-(4-methylphenyl)-2,3-dihydro-l-benzoxepine-4-carboxamide (0.15g) and aniline (0.1ml) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give crude crystals, which were recrystallized from ethanol-hexane to give N-(4-((phenylamino)methyl)-phenyl)-7-(4-methyl-phenyl)-2,3-dihydro-1-benzoxepine-4-carboxamide (Compound 101) (0.1g) as colorless crystals.
mp 157-158°C.
¹H-NMR (δppm, CDCl₃) : 2.39 (3H, s), 3.07 (2H, t, J=4.8Hz), 4.31 (2H, s), 4.35 (2H, t, J=4.8Hz), 6.62-6.76 (3H, m), 7.06 (1H, d, J=8.4Hz), 7.18-7.22 (5H, m), 7.36 (2H, d, J=8.4Hz), 7.43-7.60 (6H, m).
IR(KBr) ν : 1652, 1602cm⁻¹.

| Anal. for C₃₁H₂₈N₂O₂ : | | | |
|---|---|---|---|
| Calcd. | C,80.84; | H,6.13; | N,6.08. |
| Found | C,80.57; | H,6.09; | N,6.06. |

### Working Example 102 (Production of Compound 102)

A suspension of N-(4-chloromethylphenyl)-7-(4-methylphenyl)-2,3-dihydro -1-benzoxepine-4-carboxamide (0.15g), N-methylaniline (0.06ml) and potassium carbonate (0.15g) in dimethylformamide (10ml) was stirred at room temperature over night. The solvent was evaporated, and to the residue was added water. The mixture was extracted with ethyl acetate. The organic layer was washed with water and saturated sodium chloride solution, and driedwith anhydrous magnesium sulfate. Under reduced pressure, the solvent was evaporated to give crude crystals, which were recrystallized from ethyl acetate-hexane to give N-(4-((N-methyl-N-phenyl)aminomethyl)phenyl)-7-(4-methy l-phenyl)-2,3-dihydro-1-benzoxepine-4-carboxamide (Compound 102) (0.15g) as colorless crystals.
mp 164-165°C.
¹H-NMR (δ ppm, CDCl₃) : 2.39 (3H, s), 3.00 (3H, s), 3.06 (2H, t, J=4.6Hz), 4.34 (2H, t, J=4.6Hz), 4.51 (2H, s), 6.68-6.77 (3H, m), 7.05 (1H, d, J=8.4Hz), 7.19-7.26 (6H, m), 7.43-7.54 (6H, m), 7.60 (1H, s).
IR(KBr) ν : 3344, 3020, 1644cm⁻¹.

| Anal. for C₃₂H₃₀N₂O₂: | | | |
|---|---|---|---|
| Calcd. | C, 80.98 ; | H,6.37; | N,5.90. |
| Found | C,80.64; | H, 6.32 ; | N,5.85. |

### Working Example 103 (Production of Compound 103)

A suspension of N-(4-chloromethylphenyl)-7-(4-methylphenyl)-2,3-dihydro -1-benzoxepine-4-carboxamide (0.1g), benzylamine hydrochloride (0.5g) and potassium carbonate (0.6g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/ triethylamine) to give crude crystals, which were recrystallized from ethyl acetate-hexane to give N-(4-((benzylamino)methyl)phenyl)-7-(4-methylphenyl)-2, 3-dihydro-1-benzoxepine-4-carboxamide (Compound 103) (0.08g) as colorless crystals.
mp 147-148°C.
¹H-NMR(δppm, CDCl₃) : 2.39 (3H, s), 3.08 (2H, t, J=4.6Hz), 3.80 (2H, s), 3.81 (2H, s), 4.35 (2H, t, J=4.6Hz), 7.06 (1H, d, J=8.4Hz), 7.22-7.36 (9H, m), 7.43-7.61 (7H, m).
IR(KBr) δ : 3028, 1652cm⁻¹.

| Anal. for C₃₂H₃₀N₂O₂·0.1H₂O : | | | |
|---|---|---|---|
| Calcd. | C,80.68; | H,6.39; | N,5.88. |
| Found | C,80.43; | H,6.23; | N,5.95. |

### Working Example 104 (Production of Compound 104)

A suspension of N-(4-chloromethylphenyl)-7-(4-methylphenyl)-2,3-dihydro -1-benzoxepine-4-carboxamide (0.1g), N-methylbenzylamine (0.05ml) and potassium carbonate (0.1g) in dimethylformamide (5ml) was stirred at room temperature for 2 hours. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-benzyl-N-methyl)aminomethyl)phenyl)-7-(4-methy lphenyl)-2,3-dihydro-1-benzoxepine-4-carboxamide (Compound 104) (0.09g) as colorless crystals.
mp 157-158°C.
¹H-NMR (δ ppm, CDCl₃) : 2.18 (3H, s), 2.39 (3H, s), 3.06 (2H, t, J=4. 6Hz), 3.50 (2H, s), 3.52 (2H, s), 4.34 (2H, t, J=4.6Hz), 7.05 (1H, d, J=8.0Hz), 7.22-7.30 (3H, m), 7.33-7.37 (5H, m), 7.43-7.57 (7H, m), 7.63 (1H, s).
IR(KBr) ν : 3336, 1643cm⁻¹.

| Anal. for C₃₃H₃₂N₂O₂·0.2H₂O : | | | |
|---|---|---|---|
| Calcd. | C,80.52; | H,6.63; | N,5.69. |
| Found | C,80.61; | H,6.49; | N,5.54. |

### Working Example 105 (Production of Compound 105)

A solution of N-(4-chloromethylphenyl)-7-(4-methylphenyl)-2,3-dihydro-1-benzoxepine-4-carboxamide (0.1g) and diisopropylamine (0.1ml) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((diisopropylamino)methyl)-phenyl)-7-(4-methyl-phe nyl)-2,3-dihydro-1-benzoxepine-4-carboxamide (Compound 105) (0.11g) as colorless crystals.
mp 152-153°C.
¹H-NMR(δ ppm, CDCl₃) : 1.02 (12H, d, J=6.6Hz), 2.39 (3H, s), 2.98-3.10 (4H, m), 3.62 (2H, s), 4.35 (2H, t, J=4.8Hz), 7.05 (1H, d, J=8.6Hz), 7.24 (2H, d, J=8.0Hz), 7.35-7.55 (9H, m).
IR (KBr) ν : 2964, 1646cm⁻¹.

| Anal. for C₃₁H₃₆N₂O₂: | | | |
|---|---|---|---|
| Calcd. | C,79.45 ; | H,7.74; | N,5.98. |
| Found | C,79.18 ; | H,7.66; | N,5.93. |

### Working Example 106 (Production of Compound 106)

A solution of N-(4-chloromethylphenyl)-7-(4-methylphenyl)-2,3-dihydro-1-benzoxepine-4-carboxamide (0.1g) and N-ethylcyclohexylamine (0.11ml) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-cyclohexyl-N-ethyl)-aminomethyl)phenyl)-7-(4-m ethylphenyl)-2,3-dihydro-1-benzoxepine-4-carboxamide (Compound 106) (0.1g) as colorless crystals.
mp 166-167°C.
¹H-NMR (δ ppm, CDCl₃) : 0.98 (3H, t, J=7.2Hz), 1.02-1.26 (6H, m), 1.60-1.80 (4H, m), 2.39 (3H, s), 2.48-2.59 (3H, m), 3.08 (2H, t, J=4.5Hz), 3.59 (2H, s), 4.36 (2H, t, J=4.5Hz), 7.05 (1H, d, J=8.4Hz), 7.24 (2H, d, J=7.6Hz), 7.35 (2H, d, J=8.4Hz), 7.43-7.56 (7H, m).
IR (KBr) ν : 2929, 1648cm⁻¹.

| Anal. for C₃₃H₃₈N₂O₂·0.2H₂O: | | | |
|---|---|---|---|
| Calcd. | C,79.55; | H,7.77; | N,5.62. |
| Found | C, 79.65; | H,7.63; | N,5.66. |

### Working Example 107 (Production of Compound 107)

A suspension of N-(4-chloromethylphenyl)-7-(4-methylphenyl)-2,3-dihydro -1-benzoxepine-4-carboxamide (0.1g), 4-ethyl-amino-1-benzylpiperidine (0.11g) and potassium carbonate (0.05g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from diethyl ether-hexane to give N-(4-((N-(1-benzylpiperidin-4-yl)-N-ethyl)amino-methyl) phenyl)-7-(4-methylphenyl)-2,3-dihydro-1-benzoxepine-4-carboxamide (Compound 107) (0.13g) as colorless crystals.
mp 121-122°C.
¹H-NMR (δ ppm, CDCl₃) : 0.98 (3H, t, J=7.1Hz), 1.55-1.75 (4H, m), 1.87-2.00 (2H, m), 2.39 (3H, s), 2.49-2.60 (3H, m), 2.90-2.96 (2H, m), 3.08 (2H, t, J=4.4Hz), 3.48 (2H, s), 3.60 (2H, s), 4.36 (2H, t, J=4.4Hz), 7.06 (1H, d, J=8.2Hz), 7.23-7.35 (9H, m), 7.44-7.55 (7H, m).
IR (KBr) ν : 2939, 1652cm⁻¹.

| Anal. for C₃₉H₄₃N₃O₂: | | | |
|---|---|---|---|
| Calcd. | C, 79.97; | H, 7.40; | N,7.17. |
| Found | C,79.95; | H,7.50; | N,7.28. |

### Working Example 108 (Production of Compound 108)

A suspension of N-(4-chloromethylphenyl)-7-(4-methylphenyl)-2,3-dihydro -1-benzoxepine-4-carboxamide (0.1g), amino-methylcyclohexane (0.05g) and potassium carbonate (0.1g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/ triethylamine) to give crude crystals, which were recrystallized from ethyl acetate-hexane to give N-(4-((cyclohexylmethyl)aminomethyl)phenyl)-7-(4-methyl -phenyl)-2,3-dihydro-1-benzoxepine-4-carboxamide (Compound 108) (0.06g) as colorless crystals.
mp 154-155°C.
¹H-NMR (δ ppm, CDCl₃): 0.88-0.99 (2H, m), 1.17-1.26 (4H, m), 1.43-1.56 (1H, m), 1.65-1.78 (4H, m), 2.39 (3H, s), 2.45 (2H, d, J=6.6Hz), 3.07 (2H, t, J=4.5Hz), 3.76 (2H, s), 4.35 (2H, t, J=4.5Hz), 7.05 (1H, d, J=8.4Hz), 7.22-7.33 (5H, m), 7.43-7.61 (6H, m).
IR (KBr) ν : 3357, 2918, 1648cm⁻¹.

| Anal. for C₃₂H₃₆N₂O₂·0.2H₂O: | | | |
|---|---|---|---|
| Calcd. | C,79.37; | H, 7.58; | N,5.78. |
| Found | C, 79.58; | H,7.50; | N,5.80. |

### Working Example 109 (Production of Compound 109)

A solution of N-(4-chloromethylphenyl)-7-(4-methylphenyl)-2,3-dihydro-1-benzoxepine-4-carboxamide (0.1g) and 1-methyl-4-methylaminopiperidine (0.1ml) in dimethylformamide (5ml) was stirred at room temperature over night. The solvent was evaporated, and to the residue was added water. The mixture was extracted with ethyl acetate. The organic layer was washed with water and saturated sodium chloride solution, anddriedwithanhydrousmagnesiumsulfate. Under reduced pressure, the solvent was evaporated to give crude crystals, which were recrystallized from ethyl acetate-hexane to give N-(4-((N-methyl-N-(1-methylpiperidin-4-yl))aminomethyl) phenyl)-7-(4-methylphenyl)-2,3-dihydro-1-benzoxepine-4-carboxamide (Compound 109) (0.03g) as colorless crystals.
mp 183-184°C.
¹H-NMR (δ ppm, CDCl₃): 1.67-2.05 (6H, m), 2.20 (3H, s), 2.28 (3H, s), 2.39 (3H, s), 2.38-2.45 (1H, m), 2.91-2.96 (1H, m), 3.08 (2H, t, J=4.6Hz), 3.56 (2H, s), 4.36 (2H, t, J=4.5Hz), 7.06 (1H, d, J=8.0Hz), 7.22-7.33 (4H, m), 7.44-7.59 (7H, m) .
IR(KBr) ν : 2939, 2785, 1652cm⁻¹.

| Anal. for C₃₂H₃₇N₃O₂ : | | | |
|---|---|---|---|
| Calcd. | C,77.54; | H,7.52; | N,8.48. |
| Found | C,77.34; | H,7.57; | N,8.56. |

### Working Example 110 (Production of Compound 110)

To a solution of 7-(4-(4-methylpiperazin-1-yl)-phenyl)-2,3-dihydro-1-ben zoxepine-4-carboxylic acid (0.12g), 4-(N-methyl-N-(tetrahydropyran-4-yl)amino-methyl)anilin e (0.08g) and 1-hydroxybenzotriazole (0.05g) in dimethylformamide (15ml) was added 1-ethyl-3-(3-dimethylaminopropyl)carbodiimide hydro-chloride (0.1g), under ice-cooling. Under nitrogen atmosphere, the mixture was cooled to room temperature. To the mixture were added 4-dimethylaminopyridine (catalytic amount) and triethylamine (0.14ml), and the mixture was stirred over night. The solvent was evaporated, and to the residue was added water. The mixture was extracted with ethyl acetate. The organic layer was washed with water and saturated sodium chloride solution, and driedwith anhydrous magnesium sulfate. Under reduced pressure, the solvent was evaporated, and the residue was purified with silica gel column (ethyl acetate/methanol/triethylamine) to give crude crystals, which were recrystallized from ethyl acetate-hexane to give 7-(4-(4-methylpiperazin-1-yl)phenyl)-N-(4-((N-tetrahydr o-pyran-4-yl-N-methylamino)methyl)phenyl)-2,3-dihydro-1 -benzoxepine-4-carboxamide (Compound 110) (0.15g) as colorless crystals.
mp 220-221°C.
¹H-NMR (δ ppm, CDCl₃) : 1.64-1.75 (4H, m), 2.22 (3H, s), 2.37 (3H, s), 2.58-2.71 (5H, m), 3.08 (2H, t, J=4.6Hz), 3.25-3.32 (4H, m), 3.37 (2H, dt, J=2.8, 11.4Hz), 3.58 (2H, s), 4.01-4.07 (2H, m), 4.35 (2H, t, J=4.6Hz), 6.97-7.06 (3H, m), 7.32 (2H, d, J=8.4Hz), 7.41-7.58 (7H, m).
IR(KBr) ν : 2946, 2841, 1663cm⁻¹.

| Anal. for C₃₅H₄₂N₄O₃·0.5H₂O : | | | |
|---|---|---|---|
| Calcd. | C,73.01; | H,7.53; | N,9.73. |
| Found | C,73.25; | H,7.46; | N,9.72. |

### Working Example 111 (Production of Compound 111)

A solution of N-(4-((N-(1-t-butoxycarbonyl-piperidin-4-yl)-N-methylamino)methyl)phenyl)-7-(4-methy lphenyl)-2,3-dihydro-1-benzoxepine-4-carboxamide (0.14g) and trifluoro-acetic acid (5ml) in dichloromethane (20ml) was stirred at room temperature for 1.5 hours. The reaction mixture was neutralized with sodium hydrogen carbonate solution, and the solvent was evaporated. To the residue was added water, and the mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethanol-hexane to give N-(4-((N-methyl-N-(piperidin-4-yl))aminomethyl)phenyl)-7-(4-methyl-phenyl)-2,3-dihydro-1-benzoxepine-4-carboxa mide (Compound 111) (0.08g) as colorless crystals.
mp 129-130°C.
¹H-NMR (δ ppm, CDCl₃): 1.68-1.95 (4H, m), 2.22 (3H, s), 2.39 (3H, s), 2.61-2.79 (3H, m), 3.08 (2H, t, J=4.5Hz), 3.25-3.33 (2H, m), 3.58 (2H, s), 4.36 (2H, t, J=4.5Hz), 7.06 (1H, d, J=8.4Hz), 7.23-7.33 (4H, m), 7.44-7.60 (7H, m).
IR(KBr) ν : 2929, 1683cm⁻¹.

### Working Example 112 (Production of Compound 112) and Working Example 113 (Production of Compound 113)

A suspension of N-(4-chloromethylphenyl)-7-(4-methylphenyl)-2,3-dihydro -1-benzoxepine-4-carboxamide (0.1g), N,4-dimethylcyclohexylamine hydrochloride (0.08g) and potassium carbonate (0.17g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give each of crude crystals, which was recrystallized from ethyl acetate-hexane to give each isomer of N-(4-((N-methyl-N-(4-methylcyclohexyl))amino-methyl)phe nyl)-7-(4-methylphenyl)-2,3-dihydro-1-benzoxepine-4-car boxamide (Compound 112 (0.05g), Compound 113(0.03g)) as colorless crystals.

### (Compound 112):

mp 144-145°C.
¹H-NMR (δ ppm, CDCl₃) : 0.96 (3H, d, J=6.8Hz), 1.40-1.80 (9H, m), 2.17 (3H, s), 2.20-2.40 (1H, m), 2.39 (3H, s), 3.08 (2H, t, J=4.5Hz), 3.55 (2H, s), 4.36 (2H, t, J=4.5Hz), 7.05 (1H, d, J=8.4Hz), 7.22-7.34 (4H, m), 7.43-7.58 (7H, m).
IR (KBr) ν : 2927, 1650 cm⁻¹.

| Anal. for C₃₃H₃₈N₂O₂ · 0.2H₂O: | | | |
|---|---|---|---|
| Calcd. | C,79.55; | H,7.77; | N,5.62. |
| Found | C,79.59; | H,7.68; | N,5.84. |

### (Compound 113):

mp 183-184°C.
¹H-NMR (δ ppm, CDCl₃) : 0.87 (3H, d, J=6.6Hz), 0.89-1.02 (2H, m), 1.26-1.89 (7H, m), 2.20 (3H, s), 2.20-2.40 (1H, m), 2.39 (3H, s), 3.08 (2H, t, J=4.6Hz), 3.56 (2H, s), 4.36 (2H, t, J=4.6Hz), 7.06 (1H, d, J=8.4Hz), 7.22-7.34 (5H, m), 7.44-7.55 (6H, m).
IR (KBr) ν : 2925, 1654cm⁻¹.

| Anal. for C₃₃H₃₈N₂O₂·0.2H₂O : | | | |
|---|---|---|---|
| Calcd. | C,79.55; | H,7.77; | N,5.62. |
| Found | C,79.48; | H, 7.70; | N,5.83. |

### Working Example 114 (Production of Compound 114)

To a suspension of 7-(4-methylphenyl)-2,3-dihydro-1-benzoxepine-4-carboxyl ic acid (0.15g) in dichloro-methane (7ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-(N-methyl-N-(tetrahydropyran-4-yl)amino-methyl)anilin e (0.12g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-(N-methyl-(N-tetrahydropyran-4-yl)aminomethyl)phen yl)-7-(4-methylphenyl)-2,3-dihydro-1-benzoxepine-4-carb oxamide (Compound 114) (0.19g) as colorless crystals.
mp 162-163°C.
¹H-NMR(δ ppm, CDCl₃) : 1.59-1.74 (4H, m), 2.20 (3H, s), 2.39 (3H, s), 2.58-2.66 (1H, m), 3.07 (2H, t, J=4.5Hz), 3.37 (2H, dt, J=2.8, 11.0Hz), 3.56 (2H, s), 4.01-4.06 (2H, m), 4.35 (2H, t, J=4.5Hz), 7.05 (1H, d, J=8.4Hz), 7.22-7.33 (4H, m), 7.43-7.56 (6H, m), 7.62 (1H, s).
IR(KBr) ν : 3296, 2950, 1654cm⁻¹.

| Anal. for C₃₁H₃₄N₂O₃ · 0.2H₂O: | | | |
|---|---|---|---|
| Calcd. | C, 76.58; | H,7.13; | N,5.76. |
| Found | C, 76.51; | H,7.07; | N,5.53. |

### Working Example 115 (Production of Compound 115)

To a suspension of 7-(4-methylphenyl)-2,3-dihydro-1-benzoxepine-4-carboxyl ic acid (0.15g) in dichloro-methane (5ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-(N-methyl-N-(tetrahydropyran-3-yl)amino-methyl)anilin e (0.13g) and triethylamine (0.23ml) in tetrahydrofuran (10ml), under ice-cooling, and the mixture was stirred under nitrogen atmosphere at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from ethyl acetate-hexane to give N-(4-((N-tetrahydropyran-3-yl-N-methyl)aminomethyl)-phe nyl)-7-(4-methylphenyl)-2,3-dihydro-1-benzoxepine-4-car boxamide (Compound 115) (0.18g) as colorless crystals.
mp 158-159°C.
¹H-NMR(δppm, CDCl₃) : 1.57-1.75 (3H, m), 2.00-2.05 (1H, m), 2.21 (3H, s), 2.39 (3H, s), 2.55-2.68 (1H, m), 3.08 (2H, t, J=4.7Hz), 3.22-3.39 (2H, m), 3.59 (2H, s), 3.84-3.90 (1H, m), 4.04-4.07 (1H, m), 4.37 (2H, t, J=4.7Hz), 7.06 (1H, d, J=8.0Hz), 7.23-7.32 (4H, m), 7.44-7.55 (7H, m).
IR(KBr) ν: 2941, 1652cm⁻¹.

| Anal. for C₃₁H₃₄N₂O₃: | | | |
|---|---|---|---|
| Calcd. | C,77.15; | H,7.10; | N,5.80. |
| Found | C,77.12; | H,7.02; | N,5.88. |

### Working Example 116 (Production of Compound 116)

To a suspension of 7-(4-methylphenyl)-2,3-dihydro-1-benzoxepine-4-carboxyl ic acid (0.15g) in dichloro-methane (7ml) were added oxalyl chloride (0.14ml) and dimethylformamide (catalytic amount), under ice-cooling, and the mixture was stirred at room temperature for 2 hours. The solvent was evaporated, and the residue was dissolved in tetrahydrofuran. The mixture was dropwise added to a solution of 4-((N-indan-2-yl-N-methyl)aminomethyl)-aniline (0.14g) and triethyl-amine (0.23ml) in tetrahydrofuran (15ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and driedwith anhydrous magnesium sulfate. Under reduced pressure, the solvent was evaporated to give crude crystals, which were recrystallized from ethyl acetate-ethanol-hexane to give N-(4-((N-indan-2-yl-N-methyl)amino-methyl)phenyl)-7-(4-methylphenyl)-2,3-dihydro-1-benzoxepine-4-carboxamide (Compound 116) (0.23g) as colorless crystals.
mp 204-205°C.
¹H-NMR(δppm, CDCl₃) : 2.19 (3H, s), 2.39 (3H, s), 2.94-3.18 (6H, m), 3.41-3.48 (1H, m), 3.57 (2H, s), 4.36 (2H, t, J=4.7Hz), 7.06 (1H, d, J=8.4Hz), 7.16-7.22 (6H, m), 7.33-7.57 (9H, m).
IR (KBr) ν : 1654cm⁻¹.

| Anal. for C₃₅H₃₄N₂O₂ · 0.2H₂O: | | | |
|---|---|---|---|
| Calcd. | C,81.11; | H,6.69; | N,5.41. |
| Found | C,81.06; | H,6.57; | N,5.49. |

### Working Example 117 (Production of Compound 117)

To a suspension of 7-(4-methylphenyl)-2,3-dihydro-1-benzoxepine-4-carboxyl ic acid (0.15g) in dichloro-methane (6ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of (E)-4-((N-4-t-butylcyclohexyl-N-methyl)-aminomethyl)ani line (0.15g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give (E)-N-(4-((N-(4-t-butylcyclohexyl)-N-methyl)aminomethyl )-phenyl)-7-(4-methylphenyl)-2,3-dihydro-1-benzoxepine-4-carboxamide (Compound 117) (0.22g) as colorless crystals.
mp 225-226°C.
¹H-NMR(δppm, CDCl₃): 0.84 (9H, s), 0.95-1.05 (2H, m), 1.22-1.33 (2H, m), 1.82-1.95 (5H, m), 2.20 (3H, s), 2.30-2.45 (1H, m), 2.39 (3H, s), 3.08 (2H, t, J=4.6Hz), 3.55 (2H, s), 4.36 (2H, t, J=4.6Hz), 7.06 (1H, d, J=8.0Hz), 7.22-7.34 (4H, m), 7.44-7.55 (7H, m).
IR(KBr) ν : 2943, 1652cm⁻¹.

| Anal. for C₃₆H₄₄N₂O₂ : | | | |
|---|---|---|---|
| Calcd. | C,80.56; | H,8.26; | N,5.22. |
| Found | C,80.30; | H,8.42; | N,5.32. |

### Working Example 118 (Production of Compound 118)

To a suspension of 7-(4-methylphenyl)-2,3-dihydro-1-benzoxepine-4-carboxyl ic acid (0.15g) in dichloro-methane (6ml) were added oxalyl chloride (0.14ml) and dimethylformamide (catalytic amount), under ice-cooling, and the mixture was stirred at room temperature for 2 hours. The solvent was evaporated, and the residue was dissolved in tetrahydrofuran. The mixture was dropwise added to a solution of (Z)-4-((N-4-t-butylcyclohexyl-N-methyl)-aminomethyl)ani line (0.15g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from diethyl ether-hexane to give (Z)-N-(4-((N-(4-t-butylcyclohexyl)-N-methyl)aminomethyl )-phenyl)-7-(4-methylphenyl)-2,3-dihydro-1-benzoxepine-4-carboxamide (Compound 118) (0.2g) as colorless crystals. mp 169-170°C.
¹H-NMR(δ ppm, CDCl₃) : 0.89 (9H, s), 1.05-1.20 (1H, m), 1.36-1.50 (6H, m), 2.06 (3H, s), 2.06-2.14 (2H, m), 2.30-2.32 (1H, m), 2.39 (3H, s), 3.09 (2H, t, J=4.8Hz), 3.50 (2H, s), 4.37 (2H, t, J=4.8Hz), 7.06 (1H, d, J=8.4Hz), 7.23-7.35 (4H, m), 7.44-7.54 (7H, m).
IR(KBr) ν : 2941, 1648cm⁻¹.

| Anal. for C₃₆H₄₄N₂O₂ · 0.2H₂O: | | | |
|---|---|---|---|
| Calcd. | C,80.02; | H,8.28; | N,5.18. |
| Found | C,80.23; | H,8.30; | N,5.22. |

### Working Example 119 (Production of Compound 119)

To a suspension of 7-(4-methylphenyl)-2,3-dihydro-1-benzoxepine-4-carboxyl ic acid (0.15g) in dichloro-methane (6ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-(3,5-dimethylcyclohexyl)-N-methyl)-aminomethyl)an iline (0.13g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from diethyl ether-hexane to give N-(4-((N-methyl-N-(3,5-dimethylcyclohexyl))aminomethyl) phenyl)-7-(4-methylphenyl)-2,3-dihydro-1-benzoxepine-4-carboxamide (Compound 119) (0.22g) as colorless crystals.
mp 135-136°C.
¹H-NMR(δppm, CDCl₃): 0.45-0.68 (1H, m), 0.84 (3H, s), 0.87 (3H, s), 0.96-1.03 (2H, m), 1.65-2.05 (5H, m), 2.06 (3H, s), 2.39 (3H, s), 2.39-2.42 (1H, m), 3.08 (2H, t, J=4.7Hz), 3.50 (2H, s), 4.36 (2H, t, J=4.7Hz), 7.06 (1H, d, J=8.4Hz), 7.16-7.32 (4H, m), 7.44-7.54 (7H, m).
IR (KBr) ν : 2947, 1652cm⁻¹.

| Anal. for C₃₄H₄₀N₂O₂: | | | |
|---|---|---|---|
| Calcd. | C,80.28; | H,7.93; | N,5.51. |
| Found | C,80.19; | H,7.95; | N,5.54. |

### Working Example 120 (Production of Compound 120)

To a suspension of 7-(4-methylphenyl)-2,3-dihydro-1-benzoxepine-4-carboxyl ic acid (0.15g) in dichloro-methane (6ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-(3,5-dimethylcyclohexyl)-N-methyl)-aminomethyl)an iline (0.13g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-methyl-N-(3,5-dimethylcyclohexyl))aminomethyl) phenyl)-7-(4-methylphenyl)-2,3-dihydro-1-benzoxepine-4-carboxamide (Compound 120) (0.2g) as colorless crystals.
mp 173-174°C.
¹H-NMR(δppm, CDCl₃) : 0.43-0.60 (1H, m), 0.81-0.99 (2H, m), 0.91 (3H, s), 0.95 (3H, s), 1.30-1.58 (3H, m), 1.79-1.84 (2H, m), 2.19 (3H, s), 2.39 (3H, s), 2.48-2.60 (1H, m), 3.08 (2H, t, J=4.6Hz), 3.55 (2H, s), 4.36 (2H, t, J=4.6Hz), 7.06 (1H, d, J=8.4Hz), 7.22-7.33 (4H, m), 7.44-7.55 (7H, m) .
IR(KBr) ν : 2950, 1652cm⁻¹.

| Anal. for C₃₄H₄₀N₂O₂ · 0.2H₂O : | | | |
|---|---|---|---|
| Calcd. | C, 79.71; | H,7.95; | N,5.47. |
| Found | C, 79.83; | H,7.83; | N,5.54. |

### Working Example 121 (Production of Compound 121)

To a suspension of 7-(4-methylphenyl)-2,3-dihydro-1-benzoxepine-4-carboxyl ic acid (0.12g) in dichloro-methane (5ml) were added oxalyl chloride (0.11ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-(3,5-dimethylcyclohexyl)-N-methyl)-aminomethyl)an iline (0.1g) and triethylamine (0.17ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from diethyl ether-hexane to give N-(4-((N-methyl-N-(3,5-dimethylcyclohexyl))aminomethyl) -phenyl)-7-(4-methylphenyl)-2,3-dihydro-1-benzoxepine-4 -carboxamide (Compound 121) (0.08g) as pale yellow crystals.
mp 99-100°C.
¹H-NMR (δ ppm, CDCl₃): 0.82-1.13 (8H, m), 1.40-1.53 (2H, m), 1.64-1.85 (3H, m), 2.08-2.18 (1H, m), 2.18 (3H, s), 2.39 (3H, s), 2.69-2.81 (1H, m), 3.08 (2H, t, J=4.8Hz), 3.54 (2H,s), 4.35 (2H, t, J=4.8Hz), 7.05 (1H, d, J=8.2Hz), 7.22-7.33 (4H, m), 7.43-7.58 (7H, m).
IR(KBr) ν : 2923, 1652cm⁻¹.

| Anal. for C₃₄H₄₀N₂O₂ · 0.5H₂O: | | | |
|---|---|---|---|
| Calcd. | C,78.88; | H,7.98; | N,5.41. |
| Found | C, 78.88; | H,7.74; | N,5.50. |

### Working Example 122 (Production of Compound 122)

To a suspension of 7-(4-methylphenyl)-2,3-dihydro-1-benzoxepine-4-carboxyl ic acid (0.15g) in dichloro-methane (5ml) were added oxalyl chloride (0.14ml) anddimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-methyl-N-n-propyl)aminomethyl)aniline (0.1g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give crude crystals, which were recrystallized from diethyl ether-hexane to give N-(4-((N-methyl-N-n-propyl)aminomethyl)phenyl)-7-(4-met hyl-phenyl)-2,3-dihydro-1-benzoxepine-4-carboxamide (Compound 122) (0.1g) as colorless crystals.
mp 142-143°C.
¹H-NMR(δppm, CDCl₃): 0.90 (3H, t, J=7.3Hz), 1.48-1.59 (2H, m), 2.19 (3H, s), 2.29-2.37 (2H, m), 2.39 (3H, s), 3.08 (2H, t, J=4.4Hz), 3.47 (2H, s), 4.36 (2H, t, J=4.4Hz), 7.06 (2H, d, J=8.4Hz), 7.22-7.33 (4H, m), 7.43-7.57 (7H, m).
IR(KBr) ν : 2962, 1652, 1517cm⁻¹.

| Anal. for C₂₉H₃₂N₂O₂ · 0.2H₂O: | | | |
|---|---|---|---|
| Calcd. | C,78.42; | H,7.35; | N,6.31. |
| Found | C,78.41; | H,7.21; | N,6.26. |

### Working Example 123 (Production of Compound 123)

A solution of N-(4-chloromethylphenyl)-7-(4-methylphenyl)-2,3-dihydro-1-benzoxepine-4-carboxamide (0.1g) and N-methyl-n-butylamine (0.06g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-n-butyl-N-methyl)aminomethyl)phenyl)-7-(4-methylphenyl)-2,3-dihydro-1-benzoxe pine-4-carboxamide (Compound 123) (0.09g) as colorless crystals.
mp 138-139°C.
¹H-NMR (δ ppm, CDCl₃) : 0.91 (3H, t, J=7.2Hz), 1.27-1.55 (4H, m), 2.19 (3H, s), 2.33-2.39 (2H, m), 2.39 (3H, s), 3.08 (2H, t, J=4.5Hz), 3.47 (2H, s), 4.36 (2H, t, J=4.5Hz), 7.06 (1H, d, J=8.2Hz), 7.22-7.33 (4H, m), 7.44-7.58 (7H, m).
IR (KBr) ν : 2956, 2931, 1652cm⁻¹.

| Anal. for C₃₀H₃₄N₂O₂·0.2H₂O: | | | |
|---|---|---|---|
| Calcd. | C,78.64; | H, 7.57; | N,6.11. |
| Found | C,78.83; | H,7.44; | N,6.19. |

### Working Example 124 (Production of Compound 124)

To a suspension of 7-(4-methylphenyl)-2,3-dihydro-1-benzoxepine-4-carboxyl ic acid (0.15g) in dichloro-methane (5ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-isopropyl-N-methyl)aminomethyl)aniline (0.1g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-isopropyl-N-methyl)-aminomethyl)phenyl)-7-(4-m ethylphenyl)-2,3-dihydro-1-benzoxepine-4-carboxamide (Compound 124) (0.18g) as colorless crystals.
mp 181-182°C.
¹H-NMR (δ ppm, CDCl₃) : 1.07 (6H, d, J=6.6Hz), 2.15 (3H, s), 2.39 (3H, s), 2.83-2.96 (1H, m), 3.08 (2H, t, J=4.7Hz), 3.49 (2H, s), 4.36 (2H, t, J=4.7Hz), 7.06 (1H, d, J=8.4Hz), 7.22-7.34 (4H, m), 7.44-7.55 (7H, m).
IR (KBr) ν: 2968, 1652cm⁻¹.

| Anal. for C₂₉H₃₂N₂O₂ : | | | |
|---|---|---|---|
| Calcd. | C, 79.06 ; | H,7.32; | N,6.36. |
| Found | C,78.87; | H,7.30; | N,6.33. |

### Working Example 125 (Production of Compound 125)

To a suspension of 7-(4-methylphenyl)-2,3-dihydro-1-benzoxepine-4-carboxyl ic acid (0.15g) in dichloro-methane (5ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-((N-sec-butyl-N-methyl)aminomethyl)aniline (0.12g) and triethylamine (0.23ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from ethyl acetate-hexane to give N-(4-((N-sec-butyl-N-methyl)-aminomethyl)phenyl)-7-(4-m ethylphenyl)-2,3-dihydro-1-benzoxepine-4-carboxamide (Compound 125) (0.12g) as colorless crystals.
mp 152-153°C.
¹H-NMR (δ ppm, CDCl₃) : 0.89-1.01 (6H, m), 1.22-1.39 (1H, m), 1.50-1.67 (1H, m), 2.13 (3H, s), 2.39 (3H, s), 2.54-2.65 (1H, m), 3.08 (2H, t, J=4.7Hz), 3.44 (1H, d, J=13.2Hz), 3.56 (1H, d, J=13.2Hz), 4.36 (2H, t, J=4.7Hz), 7.06 (2H, d, J=8.0Hz), 7.22-7.35 (4H, m), 7.44-7.54 (7H, m).
IR(neat) ν : 2964, 1652cm⁻¹.

| Anal. for C₃₀H₃₄N₂O₂ · 0.2H₂O: | | | |
|---|---|---|---|
| Calcd. | C, 78.64; | H,7.57; | N,6.11. |
| Found | C,78.88; | H,7.39; | N,6.16. |

### Working Example 126 (Production of Compound 126)

A solution of N-(4-chloromethylphenyl)-7-(4-methylphenyl)-2,3-dihydro-1-benzoxepine-4-carboxamide (0.1g) and N-methylisobutylamine (0.06g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-isobutyl-N-methyl)aminomethyl)phenyl)-7-(4-methyl-phenyl)-2,3-dihydro-1-benzox epine-4-carboxamide (Compound 126) (0.08g) as colorless crystals.
mp 137-138°C.
¹H-NMR (δ ppm, CDCl₃) : 0.90 (6H, d, J=6.6Hz), 1.78-1.88 (1H, m), 2.10 (2H, d, J=7.4Hz), 2.16 (3H, s), 2.39 (3H, s), 3.08 (2H, t, J=4.6Hz), 3.44 (2H, s), 4.36 (2H, t, J=4.6Hz), 7.06 (1H, d, J=8.0Hz), 7.23-7.34 (4H,m), 7.44-7.57 (7H, m).
IR(KBr) ν: 2954, 1652cm⁻¹.

| Anal. for C₃₀H₃₄N₂O₂: | | | |
|---|---|---|---|
| Calcd. | C,79.26; | H,7.54; | N,6.16. |
| Found | C,78.99; | H,7.38; | N,6.21. |

### Working Example 127 (Production of Compound 127)

To a suspension of 7-(4-methylphenyl)-2,3-dihydro-1-benzoxepine-4-carboxyl ic acid (0.1g) in dichloromethane (5ml) were added oxalyl chloride (0.1ml) and dimethylformamide (catalytic amount) under ice-cooling, and the mixture was stirred at room temperature for 2 hours. The solvent was evaporated, and the residue was dissolved in tetra-hydrofuran. The mixture was dropwise added to a solution of 4-((N-t-butyl-N-methyl)amino-methyl)aniline (0.08g) and triethylamine (0.12ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-t-butyl-N-methyl)amino-methyl)phenyl)-7-(4-met hylphenyl)-2,3-dihydro-1-benzoxepine-4-carboxamide (Compound 127) (0.12g) as colorless crystals.
mp 122-123°C.
¹H-NMR(δ ppm, CDCl₃): 1.16 (9H, s), 2.09 (3H, s), 2.39 (3H, s), 3.08 (2H, t, J=4.7Hz), 3.49 (2H, s), 4.36 (2H, t, J=4.7Hz), 7.06 (1H, d, J=8.4Hz), 7.23-7.36 (4H, m), 7.44-7.54 (7H, m).
IR(KBr) ν : 2971, 1651, 1599, 1516cm⁻¹.

| Anal. for C₃₀H₃₄N₂O₂: | | | |
|---|---|---|---|
| Calcd. | C,79.26; | H,7.54; | N,6.16. |
| Found | C,79.16; | H,7.55; | N,5.98. |

### Working Example 128 (Production of Compound 128)

To a suspension of 7-(4-methylphenyl)-2,3-dihydro-1-benzoxepine-4-carboxyl ic acid (0.1g) in dichloromethane (5ml) were added oxalyl chloride (0.1ml) and dimethylformamide (catalytic amount) under ice-cooling, and the mixture was stirred at room temperature for 2 hours. The solvent was evaporated, and the residue was dissolved in tetra-hydrofuran. The mixture was dropwise added to a solution of 4-((N-methyl-N-(pentan-3-yl))aminomethyl)aniline (0.08g) and triethylamine (0.12ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-methyl-N-(pentan-3-yl))aminomethyl)phenyl)-7-( 4-methyl-phenyl)-2,3-dihydro-1-benzoxepine-4-carboxamid e (Compound 128) (0.12g) as colorless crystals.
mp 133-134°C.
¹H-NMR(δppm, CDCl₃) : 0.94 (6H, t, J=7.5Hz), 1.26-1.53 (4H, m), 2.13 (3H, s), 2.24-2.31 (1H, m), 2.40 (3H, s), 3.09 (2H, t, J=4.4Hz), 3.55 (2H, s), 4.37 (2H, t, J=4.4Hz), 7.06 (1H, d, J=8.4Hz), 7.17-7.36 (4H, m), 7.44-7.54 (7H, m).
IR(KBr) ν : 2930, 1649, 1597, 1518cm⁻¹.

| Anal. for C₃₁H₃₆N₂O₂: | | | |
|---|---|---|---|
| Calcd. | C, 79.45; | H,7.74; | N,5.98. |
| Found | C,79.06; | H,7.56; | N,5.98. |

### Working Example 129 (Production of Compound 129)

To a suspension of 7-(4-methylphenyl)-2,3-dihydro-1-benzoxepine-4-carboxyl ic acid (0.1g) in dichloromethane (5ml) were added oxalyl chloride (0.1ml) and dimethylformamide (catalytic amount) under ice-cooling, and the mixture was stirred at room temperature for 2 hours. The solvent was evaporated, and the residue was dissolved in tetra-hydrofuran. The mixture was dropwise added to a solution of 4-((N-methyl-N-(norbornan-2-yl))aminomethyl)aniline (0.09g) and triethylamine (0.12m1) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane). The purified product was dissolved in ethyl acetate (10ml), and to the mixture was added 4N hydrochloric acid-ethyl acetate solution (0.2ml) under ice-cooling. The solvent was evaporated to give crude crystals, which were recrystallized from ethanol-hexane to give N-(4-((N-methyl-N-(norbornan-2-yl))aminomethyl)-phenyl) -7-(4-methylphenyl)-2,3-dihydro-1-benzoxepine-4-carboxa mide hydrochloride (Compound 129) (0.16g) as colorless crystals.
mp 268-269°C (dec.).
¹H-NMR(δppm, DMSO-d₆) : 1.24-1.55 (6H, m), 1.99-2.15 (3H, m), 2.28 (1H, br), 2.34 (3H, s), 2.51-2.63 (3H, m), 2.82 (1H, br), 3.00 (2H, br), 4.04-4.45 (4H, m), 7.06 (1H, d, J=8.4Hz), 7.33 (2H, d, J=7.8Hz), 7.38 (1H, s), 7.48-7.59 (5H, m), 7.75-7.85 (3H, m), 9.52 (0.5H, br), 9.83 (0.5H, br), 10.18 (1H, s).
IR(KBr) ν : 2957, 2492, 1661cm⁻¹.

| Anal. for C₃₃H₃₇ClN₂O₂·0.2H₂O : | | | |
|---|---|---|---|
| Calcd. | C,74.40; | H,7.08 ; | N,5.26. |
| Found | C,74.34; | H,7.05 ; | N,5.19. |

### Working Example 130 (Production of Compound 130)

To a solution of 7-(3-pyridyl)-2,3-dihydro-1-benzoxepine-4-carboxylic acid (0.15g), 4-(N-methyl-N-(tetra-hydropyran-4-yl)aminomethyl)anilin e (0.12g) and triethylamine (0.16ml) in dimethylformamide (50ml) was added diethyl cyano-phosphate (0.1ml) under ice-cooling, and the mixture was stirred under nitrogen atmosphere at room temperature over night. The solvent was evaporated, and the residue was purified with silica gel column (methanol/ethyl acetate/triethylamine) to give crude crystals, which were recrystallized from ethanol-hexane to give 7-(3-pyridyl)-N-(4-((N-tetrahydropyran-4-yl-N-methylami no)-methyl)phenyl)-2,3-dihydro-1-benzoxepine-4-carboxam ide (Compound 130) (0.06g) as colorless crystals.
mp 158-159°C.
¹H-NMR(δ ppm, CDCl₃): 1.64-1.71 (4H, m), 2.23 (3H, s), 2.65-2.75 (1H, m), 3.11 (2H, t, J=4.8Hz), 3.37 (2H, dt, J=2.4, 11.0Hz), 3.60 (2H, s), 4.01-4.07 (2H, m), 4.38 (2H, t, J=4.8Hz), 7.12 (1H, d, J=8.4Hz), 7.31-7.40 (3H, m), 7.44-7.58 (4H, m), 7.66 (1H, br), 7.84 (1H, d, J=7.6Hz), 8.58 (1H, d, J=4.8Hz), 8.82 (1H, d, J=2.2Hz).
IR(KBr) ν: 2949, 2845, 1661cm⁻¹.

| Anal. for C₂₉H₃₁N₃O₃·0.5H₂O : | | | |
|---|---|---|---|
| Calcd. | C,72.78; | H,6.74; | N,8.78. |
| Found | C,72.72; | H,6.72; | N,8.95. |

### Working Example 131 (Production of Compound 131)

To a solution of 7-(4-pyridyl)-2,3-dihydro-1-benzoxepine-4-carboxylic acid (0.15g), 4-(N-methyl-N-(tetrahydropyran-4-yl)aminomethyl)aniline (0.12g) and triethylamine (0.16ml) in dimethylformamide (50ml) was added diethyl cyano-phosphate (0.1ml) under ice-cooling, and the mixture was stirred under nitrogen atmosphere at room temperature over night. The solvent was evaporated, and the residue was purified with silica gel column (methanol/ethyl acetate/triethylamine) to give crude crystals, which were recrystallized from ethanol-hexane to give 7-(4-pyridyl)-N-(4-((N-tetrahydropyran-4-yl-N-methylami no)methyl)phenyl)-2,3-dihydro-1-benzoxepine-4-carboxami de (Compound 131) (0.07g) as pale brown crystals.
mp 186-187°C.
¹H-NMR(δ ppm, CDCl₃) : 1.67-1.73 (4H, m), 2.23 (3H, s), 2.60-2.75 (1H, m), 3.11 (2H, t, J=4.6Hz), 3.37 (2H, dt, J=3.0, 11.0Hz), 3.60 (2H, s), 4.01-4.07 (2H, m), 4.38 (2H, t, J=4.6Hz), 7.12 (1H, d, J=8.0Hz), 7.34 (2H, d, J=8.4Hz), 7.45-7.51 (3H, m), 7.55-7.59 (3H, m), 7.82 (1H, br), 8.64 (2H, d, J=5.8Hz).
IR(KBr) ν: 2948, 1659cm⁻¹.

| Anal. for C₂₉H₃₁N₃O₃· 0.5H₂O : | | | |
|---|---|---|---|
| Calcd. | C,72.78; | H, 6.74 ; | N,8.78. |
| Found | C,72.64; | H, 6.51 ; | N,8.85. |

### Working Example 132 (Production of Compound 132)

To a solution of 7-(2-furyl)-2,3-dihydro-1-benzoxepine-4-carboxylic acid (0.15g), 4-(N-methyl-N-(tetrahydropyran-4-yl)aminomethyl)aniline (0.15g) and triethylamine (0.25ml) in dimethylformamide (10ml) was added diethyl cyanophosphate (0.13ml) under ice-cooling, and the mixture was stirred under nitrogen atmosphere at room temperature over night. The solvent was evaporated, and the residue was purified with silica gel column (methanol/ethyl acetate/triethylamine) to give crude crystals, which were recrystallized from ethyl acetate-hexane to give 7-(2-furyl)-N-(4-((N-tetrahydropyran-4-yl-N-methylamino )methyl)phenyl)-2,3-dihydro-1-benzoxepine-4-carboxamide (Compound 132) (0.1g) as brown crystals.
mp 166-167°C (dec.).
¹H-NMR(δ ppm, CDCl₃) : 1.64-1.78 (4H, m), 2.22 (3H, s), 2.60-2.75 (1H, m), 3.06 (2H, t, J=4.6Hz), 3.37 (2H, dt, J=3.0, 11.1Hz), 3.59 (2H, s), 4.02-4.07 (2H, m), 4.33 (2H, t, J=4.6Hz), 6.46 (1H, dd, J=1.8, 3.3Hz), 6.56 (1H, d, J=3.3Hz), 7.01 (2H, d, J=8.4Hz), 7.21 (1H, s), 7.32 (2H, d, J=8.6Hz), 7.44 (1H, d, J=1.8Hz), 7.50-7.62 (4H, m), 7.73 (1H, s).
IR (KBr) ν : 2951, 1659cm⁻¹.

| Anal. for C₂₈H₃₀N₂O₄·0.5H₂O : | | | |
|---|---|---|---|
| Calcd. | C,71.93; | H,6.68; | N,5.99. |
| Found | C, 71.97 ; | H,6.52; | N,6.08. |

### Working Example 133 (Production of Compound 133)

To a solution of 7-(4-dimethylaminophenyl)-2,3-dihydro-1-benzoxepine-4-c arboxylic acid (0.15g), 4-(N-methyl-N-(tetrahydropyran-4-yl)aminomethyl)aniline (0.11g) and triethylamine (0.2ml) in dimethylformamide (15ml) was added diethyl cyano-phosphate (0.11ml) under ice-cooling, and the mixture was stirred under nitrogen atmosphere at room temperature over night. The solvent was evaporated, and the residue was purified with silica gel column (methanol/ethyl acetate/triethylamine) to give crude crystals, which were recrystallized from ethyl acetate-hexane to give 7-(4-dimethylaminophenyl)-N-(4-((N-tetrahydropyran-4-yl -N-methylamino)methyl)phenyl)-2,3-dihydro-1-benzoxepine -4-carboxamide (Compound 133) (0.07g) as pale brown crystals.
mp 208-209°C (dec.).
¹H-NMR(δ ppm, CDCl₃) : 1.63-1.78 (4H, m), 2.20 (3H, s), 2.59-2.70 (1H, m), 2.98 (6H, s), 3.04 (2H, t, J=4.5Hz), 3.36 (2H, dt, J=2.6, 11.0Hz), 3.56 (2H, s), 4.00-4.06 (2H, m), 4.31 (2H, t, J=4.5Hz), 6.78 (2H, d, J=8.8Hz), 7.01 (1H, d, J=8.0Hz), 7.24-7.31 (3H, m), 7.39-7.46 (4H, m), 7.55 (2H, d, J=8.4Hz), 7.79 (1H, s).
IR (KBr) ν : 2949, 2845, 1659cm⁻¹.

| Anal. for C₃₂H₃₇N₃O₃ · 0.3H₂O: | | | |
|---|---|---|---|
| Calcd. | C,74.33; | H,7.33; | N,8.13. |
| Found | C,74-11; | H,7.22; | N,8.21. |

### Working Example 134 (Production of Compound 134)

To a solution of 7-(4-(pyrrolidin-1-yl)phenyl)-2,3-dihydro-1-benzoxepine -4-carboxylic acid (0.15g), 4-(N-methyl-N-(tetrahydropyran-4-yl)aminomethyl)aniline (0.1g) and 1-hydroxybenzotriazole (0.07g) in dimethyl-formamide (10ml) was added 1-ethyl-3-(3-dimethylamino-propyl)carbodiimide hydro-chloride (0.13g) under ice-cooling, and the mixture was stirred under nitrogen atmosphere at room temperature for 3 hours. To the mixture were added 4-dimethylaminopyridine (catalytic amount) and 1,8-diazabicyclo[5.4.0]-7-undecene (0.2ml), and the mixture was stirred over night. The solvent was evaporated, and the residue was purified with silica gel column (methanol/ethyl acetate/triethylamine) to give crude crystals, which were recrystallized from ethanol-hexane to give 7-(4-(pyrrolidin-1-yl)phenyl)-N-(4-((N-tetrahydro-pyran -4-yl-N-methylamino)-methyl)phenyl)-2,3-dihydro-1-benzo xepine-4-carboxamide (Compound 134) (0.08g) as colorless crystals.
mp 210-211°C.
¹H-NMR(δppm, CDCl₃) : 1.69-1.78 (8H, m), 1.99-2.06 (4H, m), 2.21 (3H, s), 2.55-2.70 (1H, m), 3.07 (2H, t, J=4.5Hz), 3.30-3.38 (4H, m), 3.38-3.57 (2H, m), 3.57 (2H, s), 4.01-4.06 (2H, m), 4.35 (2H, t, J=4.5Hz), 6.63 (2H, d, J=8.8Hz), 7.02 (1H, d, J=8.4Hz), 7.31 (2H, d, J=8.4Hz), 7.40-7.48 (4H, m), 7.54 (2H, d, J=8.4Hz), 7.61 (1H, s).
IR (KBr) ν : 2951, 2841, 1653cm⁻¹.

| Anal. for C₃₄H₃₉N₃O₃: | | | |
|---|---|---|---|
| Calcd. | C,75.95; | H,7.31; | N,7.81. |
| Found | C,75.70; | H,7.10; | N,7.83. |

### Working Example 135 (Production of Compound 135)

To a solution of 7-(4-piperidinophenyl)-2,3-dihydro-1-benzoxepine-4-carboxylic acid (0.15g), 4-(N-methyl-N-(tetrahydropyran-4-yl)aminomethyl)aniline (0.1g) and 1-hydroxy-benzotriazole (0.07g) in dimethylformamide (10ml) was added 1-ethyl-3-(3-dimethylaminopropyl)-carbodiimide hydrochloride (0.13g) under ice-cooling. Under nitrogen atmosphere, the mixture was warmed to room temperature. To the mixture were added4-dimethylaminopyridine (catalytic amount) and triethylamine (0.18ml), and the mixture was stirred over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give 7-(4-piperidino-phenyl)-N-(4-((N-methyl-N-tetrahydro-py ran-4-yl)amino)-methyl)phenyl)-2,3-dihydro-1-benzoxepin e-4-carboxamide (Compound 135) (0.18g) as colorless crystals.
mp 197-198°C.
¹H-NMR (δ ppm, CDCl₃): 1.58-1.70 (2H, m), 1.70-1.73 (4H, m), 2.21 (3H, s), 2.55-2.70 (1H, m), 3.08 (2H, t, J=4.6Hz), 3.18-3.23 (4H, m), 3.37 (2H, dt, J=2.4, 11.0Hz), 3.57 (2H, s), 4.01-4.07 (2H, m), 4.35 (2H, t, J=4.6Hz), 6.63 (2H, d, J=8.8Hz), 6.97-7.05 (3H, m), 7.31 (2H, d, J=8.4Hz), 7.43-7.57 (7H, m).
IR (KBr) ν : 2938, 2847, 1651cm⁻¹.

| Anal. for C₃₅H₄₁N₃O₃·0.5H₂O: | | | |
|---|---|---|---|
| Calcd. | C,74.97; | H,7.55; | N,7.49. |
| Found | C,75.26; | H,7.53; | N,7.63. |

### Working Example 136 (Production of Compound 136)

To a solution of 7-(4-morpholinophenyl)-2,3-dihydro-1-benzoxepine-4-carboxylic acid- (0.15g), 4-(N-methyl-N-(tetrahydropyran-4-yl)aminomethyl)aniline (0.1g) and 1-hydroxybenzotriazole (0.06g) in dimethylformamide (15ml) was added 1-ethyl-3-(3-dimethylaminopropyl)-carbodiimide hydrochloride (0.12g) under ice-cooling. Under nitrogen atmosphere, the mixture was warmed to room temperature. To the mixture were added4-dimethylaminopyridine (catalytic amount) and triethylamine (0.18ml), and the mixture was stirred over night. The mixture was poured into water and was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-methyl-N-(tetrahydropyran-4-yl)aminomethyl)-ph enyl)-7-(4-morpholinophenyl)-2,3-dihydro-1-benzoxepine-4-carboxamide (Compound 136) (0.17g) as pale brown crystals.
mp 238-239°C (dec.).
¹H-NMR(δ ppm, CDCl₃): 1.58-1.77 (4H, m), 2.21 (3H, s), 2.55-2.75 (1H, m), 3.08 (2H, t, J=4.6Hz), 3.19-3.24 (4H, m), 3.37 (2H, dt, J=3.0, 11.3Hz), 3.57 (2H, s), 3.87-3.91 (4H, m), 4.01-4.11 (2H, m), 4.36 (2H, t, J=4.6Hz), 6.98 (2H, d, J=9.0Hz), 7.05 (1H, d, J=8.4Hz), 7.27-7.34 (3H, m), 7.42-7.57 (6H, m).
IR (KBr) ν : 2961, 2847, 1660cm⁻¹.

| Anal. for C₃₄H₃₉N₃O₄·0.5H₂O: | | | |
|---|---|---|---|
| Calcd. | C,72.57; | H,7.16; | N,7.47. |
| Found | C,72.79; | H,7.08; | N,7.35. |

### Working Example 137 (Production of Compound 137)

To a solution of 7-(4-(1-imidazolyl)phenyl)-2,3-dihydro-1-benzoxepine-4-carboxylic acid (0.13g), 4-(N-methyl-N-(tetrahydropyran-4-yl)aminomethyl)aniline (0.11g) and 1-hydroxybenzotriazole (0.07g) in dimethylformamide (20ml) was added 1-ethyl-3-(3-dimethylamino-propyl)carbodiimide hydrochloride (0.13g) under ice-cooling. Under nitrogen atmosphere, the mixture was warmed to room temperature. To the mixture were added 4-dimethylaminopyridine (catalytic amount) and triethylamine (0.2ml), and the mixture was stirred over night. The solvent was evaporated, and the residue was extracted with ethyl acetate. The organic layer was washed with saturated sodium chloride solution and dried with anhydrous magnesium sulfate. Under reduced pressure, the solvent was evaporated, and the residue was purified with silica gel column (ethyl acetate/methanol/triethylamine) to give crude crystals, which were recrystallized from ethanol-hexane to give 7-(4-(1-imidazolyl)phenyl)-N-(4-((N-tetra-hydropyran-4-yl-N-methylamino)methyl)phenyl)-2,3-dihydro-1-benzoxepi ne-4-carboxamide (Compound 137) (0.11g) as pale yellow crystals.
mp 194-195°C.
¹H-NMR(δppm, CDCl₃) : 1.63-1.80 (4H, m), 2.21 (3H, s), 2.59-2.70 (1H, m), 3.10 (2H, t, J=4.6Hz), 3.37 (2H, dt, J=2.6, 11.8Hz), 3.58 (2H, s), 4.00-4.08 (2H, m), 4.39 (2H, t, J=4.6Hz), 7.11 (1H, d, J=8.2Hz), 7.23-7.24 (1H, m), 7.30-7.34 (4H, m), 7.42-7.46 (3H, m), 7.51 (1H, s), 7.57 (2H, d, J=8.6Hz), 7.65 (2H, d, J=8.6Hz), 7.84 (1H, br), 7.91 (1H, s).
IR (KBr) ν : 2949, 2843, 1651cm⁻¹.

| Anal. for C₃₃H₃₄N₄O₃·0.2H₂O: | | | |
|---|---|---|---|
| Calcd. | C,73.64; | H,6.44; | N,10.41. |
| Found | C,73.63; | H,6.23; | N,10.46. |

### Working Example 138 (Production of Compound 138)

To a solution of 7-(4-dimethylaminophenyl)-2,3-dihydro-1-benzoxepine-4-c arboxylic acid (0.1g), 1-(4-aminobenzyl)phosphorinane-1-oxide (0.08g) and 1-hydroxybenzotriazole (0.05g) in dimethylformamide (7ml) was added 1-ethyl-3-(3-dimethylaminopropyl)carbodiimide hydrochloride (0.1g) under ice-cooling. Under nitrogen atmosphere, the mixture was warmed to room temperature. To the mixture were added 4-dimethylaminopyridine (catalytic amount) and triethylamine (0.15ml), and the mixture was stirred over night. The solvent was evaporated, and the residue was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give crude crystals, which were recrystallized from ethanol-hexane to give 7-(4-dimethylaminophenyl)-N-(4-((1-oxophosphorinan-1-yl)methyl)-phen yl)-2,3-dihydro-1-benzoxepine-4-carboxamide (Compound 138) (0.12g) as colorless crystals.
mp 293-294°C (dec.).
¹H-NMR(δppm, CDCl₃) : 1.35-1.55 (2H, m), 1.60-1.75 (6H, m), 1.75-2.05 (2H, m), 3.00 (6H, s), 3.09 (2H, t, J=4.7Hz), 3.13 (2H, d, J=13.6Hz), 4.35 (2H, t, J=4.7Hz), 6.80 (2H, d, J=8.8Hz), 7.03 (1H, d, J=8.4Hz), 7.21-7.27 (3H, m), 7.41-7.51 (4H, m), 7.60 (2H, d, J=8.2Hz), 8.24 (1H, br). IR (KBr) ν : 2940, 1665cm⁻¹.

| Anal. for C₃₁H₃₅N₂O₃P : | | | |
|---|---|---|---|
| Calcd. | C, 72.35 ; | H,6.86; | N,5.44. |
| Found | C,72.00; | H,6.84; | N,5.45. |

### Working Example 139 (Production of Compound 139)

To a solution of 7-(4-dimethylaminophenyl)-N-(4-((1-oxophosphorinan-1-yl )methyl)phenyl)-2,3-dihydro-1-benzoxepine-4-carboxamide (0.1g) in ethanol was added 4N hydrochloric acid-ethyl acetate (0.2ml) under ice-cooling. The solvent was evaporated, and the residue was crystallized from ethanol and diethylether to give 7-(4-dimethylaminophenyl)-N-(4-((1-oxophosphorinan-1-yl)methyl)phenyl)-2, 3-dihydro-1-benzoxepine-4-carboxamide hydrochloride (Compound 139) (0.1g) as colorless crystals.
mp 162-163°C.
¹H-NMR(δppm, DMSO-d₆): 1.40-1.50 (2H, m), 1.50-1.90 (8H, m), 2.99 (2H, br), 3.04 (6H, s), 3.16 (2H, d, J=13.6Hz), 4.30 (2H, br), 7.05 (1H, d, J=8.8Hz), 7.20-7.25 (4H, m), 7.35 (1H, s), 7.54 (1H, dd, J=2.2, 8.2, 8.8Hz), 7.63-7.69 (4H, m), 7.74 (1H, d, J=2.2Hz), 9.97 (1H, s).

| Anal. for C₃₁H₃₅N₂O₃P·HCl·2H₂O: | | | |
|---|---|---|---|
| Calcd. | C, 63.42; | H,6.87; | N,4.77. |
| Found | C,63.45; | H,6.99; | N,4.39. |

### Comparative Example 4

In methanol (40ml) was dissolved N-(4-(1-methyl-piperidin-2-ylcarbonyl)phenyl)-7-(4-methylphenyl)-2,3-d ihydro-1-benzoxepine-4-carboxamide (0.1g) under ice-cooling, and to the mixture was added sodium boron hydride (10mg). The mixture was stirred for 15 minutes, and to the mixture was added water. The mixture was concentrated and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give crude crystals, which were recrystallized from ethanol-ethyl acetate-hexane to give N-(4-(hydroxy(1-methylpiperidin-2-yl)methyl)phenyl)-7-( 4-methylphenyl)-2,3-dihydro-1-benzoxepine-4-carboxamide (0.07g) as colorless crystals. mp 195-196.
¹H-NMR (δppm, CDCl₃) : 0.95-1.05 (2H, m), 1.25-1.40 (2H, m), 2.04-2.30 (4H, m), 2.39 (3H, s), 2.50 (3H, s), 2.95-3.01 (1H, m), 3.08 (2H, t, J=4 .6Hz), 4.36 (2H, t, J=4.6Hz), 5.16 (1H, d, J=3.0Hz), 7.06 (1H, d, J=8.4Hz), 7.24 (2H, d, J=8.0Hz), 7.33 (2H, d, J=8.4Hz), 7.43-7.52 (4H, m), 7.56 (2H, d, J=8.4Hz), 7.61 (1H, s).
IR(KBr) ν : 3287, 2938, 1647 cm⁻¹.

| Anal. for C₃₁H₃₄N₂O₃·0.6H₂O : | | | |
|---|---|---|---|
| Calcd. | C,75.46; | H,7.19 ; | N,5.68. |
| Found | C,75.36; | H,7.33; | N,5.76. |

### Working Example 140 (Production of Compound 140)

Under nitrogen atmosphere, oxalyl chloride (0.19ml) was added to a solution of 7-(4-methylphenyl)-2,3-dihydro-1-benzoxepine-4-carboxyl ic acid (0.40g) in tetra-hydrofuran (10ml) at room temperature. To the mixture was added a drop of DMF, and the mixture was stirred for 1 hour. Under reduced pressure, the solvent was evaporated, and the residue was dissolved in tetrahydrofuran (6ml). To the solution were added triethylamine (0.40ml) and a solution of 2 - (4-aminobenzyl)pyridine (0.29g) in tetrahydrofuran (5ml) at 0°C, and the mixture was stirred at room temperature for 2 hours. The reaction mixture was added to vigorously stirredwater to stop the reaction. The mixture was extracted with ethyl acetate. The organic layer was washed with saturated sodium chloride solution, dried with magnesium sulfate, concentrated and recrystallized from ethyl acetate to give N- [4- (2-pyridylmethyl) -phenyl] -7- (4-methylphenyl) -2,3-d ihydro-1-benzoxepine-4-carboxamide (Compound 140) (303mg) as colorless crystals.
m.p. 189-190°C
¹H-NMR (200MHz, CDCl₃) δ 2.39 (3H, s), 3.06 (2H, t, J=4.6 Hz), 4.14 (2H, s), 4.35 (2H, t, J=4.6 Hz), 7.03-7.16 (3H, m), 7.18-7.31 (5H, m), 7.40-7.64 (8H, m), 8.52-8.58 (1H, m) .
IR (KBr) 3338, 1645, 1510, 1493, 1414, 1313, 1252, 1234, 816, 750 cm⁻¹

| Elemental Analysis for C₃₀H₂₆N₂O₂ | | | |
|---|---|---|---|
| Calcd. | C, 80.69 ; | H, 5.87 ; | N, 6.27 : |
| Found. | C, 80.63 ; | H, 5.80 ; | N, 6.37. |

### Working Example 141 (Production of Compound 141)

To a solution of N-[4-(2-pyridylmethyl)phenyl]-7-(4-methylphenyl)-2,3-di hydro-1-benzoxepine-4-carboxamide (200mg) in tetrahydrofuran (10ml) was added 3-chloro-perbenzoic acid (70%, 0.18g) at 0°C, and the mixture was stirred at room temperature for 17 hours. To the reaction mixture was added sodium thio-sulfate solution, and the mixture was stirred for a few minutes. The mixture was extracted with ethyl acetate. The organic layer was washed with saturated sodium bicarbonate solution and saturated sodium chloride solution, dried with magnesium sulfate and concentrated to give crystals, which were collected by filtration and was recrystallized from ethanol to give N-[4-(1-oxidopyridin-2-ylmethyl)phenyl]-7-(4-methylphen yl)-2,3-dihydro-1-benzoxepine-4-carboxamide (Compound 141) (124mg) as colorless crystals.
m.p. 188-190°C
¹H-NMR (200MHz, CDCl₃) δ 2.39 (3H, s), 3.09 (2H, t, J=4.6 Hz), 4.24 (2H, s), 4.36 (2H, t, J=4.6 Hz), 6.90-7.01 (1H, m), 7.06 (1H, d, J=8.4 Hz), 7.11-7.16 (2H, m), 7.22-7.29 (5H, m), 7.43-7.51 (4H, m), 7.54-7.76 (3H, m), 8.24-8.31 (1H, m).
IR (KBr) 3319, 1666, 1601, 1517, 1491, 1412, 1319, 1246, 813 cm⁻¹

| Elemental Analysis for C₃₀H₂₆N₂O₃ · 0.3H₂O | | | |
|---|---|---|---|
| Calcd. | C, 77.00 ; | H, 5.73 ; | N, 5.99 : |
| Found. | C, 76.98 ; | H, 5.59 ; | N, 6.10. |

### Working Example 142 (Production of Compound 142)

Under nitrogen atmosphere, oxalyl chloride (0.07ml) was added to a solution of 7-(4-methylphenyl)-2,3-dihydro-1-benzoxepine-4-carboxyl ic acid (144.8mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.14ml) and a solution of 4-aminobenzyldiethylphosphine oxide (120mg) in tetrahydrofuran (5ml) at 0°C and the mixture was stirred at room temperature for 1 hour. The reaction mixture was added to vigorously stirred water to stop the reaction. The mixture was extracted with ethyl acetate. The organic layer was washed with saturated sodium chloride solution, dried with magnesium sulfate, concentrated and recrystallized from ethanol-tetrahydrofuran to give N-(4-diethylphosphoryl-methylphenyl)-7-(4-methylphenyl) -2,3-dihydro-1-benzoxepine-4-carboxamide (Compound 142) (157mg) as colorless crystals.
m.p. 240-241°C
¹H-NMR (200MHz, CDCl₃) δ 1.13 (6H, dt, J=16.4, 8.0 Hz), 1.53-1.72 (4H, m), 2.39 (3H, s), 3.06-3.13 (4H, m), 4.36 (2H, t, J=4.8 Hz), 7.06 (1H, d, J=8.4 Hz), 7.22-7.27 (5H, m), 7.44-7.52 (4H, m), 7.58 (2H, d, J=8.4 Hz), 7.98 (1H, s).
IR (KBr) 3263, 1653, 1599, 1516, 1491, 1410, 1319, 1250, 1173, 1132, 843, 808 cm⁻¹

| Elemental Analysis for C₂₉H₃₂NO₃P | | | | |
|---|---|---|---|---|
| Calcd. | C, 73.55 ; | H, 6.81 ; | N, 2.96 ; | P, 6.54 : |
| Found. | C, 73.23 ; | H, 6.64 ; | N, 3.01 ; | P, 6.63. |

### Working Example 143 (Production of Compound 143)

Under nitrogen atmosphere, oxalyl chloride (0.2ml) was added to a solution of 7-(4-methylphenyl)-2,3-dihydro-1-benzoxepine-4-carboxyl ic acid (400mg) in tetra-hydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.4ml) and 4-aminobenzyldipropylphosphine oxide (0.38g) at 0°C, and the mixture was stirred at room temperature for 5 hours. The reaction mixture was added to vigorously stirred water to stop the reaction. The mixture was extracted with ethyl acetate. The organic layer was washed with saturated sodium chloride solution, dried with magnesium sulfate and concentrated. The residue was separated and purified with column chromatography (ethanol/ethyl acetate=1:5), and recrystallized from ethanol to give N-(4-dipropyl-phosphorylmethylphenyl)-7-(4-methylphenyl )-2,3-dihydro-1-benzoxepine-4-carboxamide (Compound 143) (456mg) as colorless crystals.
m.p. 219-220°C
¹H-NMR (200MHz, CDCl₃) δ 0.84-0.98 (6H, m), 1.41-1.63 (8H, m), 2.39 (3H, s), 3.02 (2H, d, J=13.2 Hz), 3.09 (2H, t, J=4.4 Hz), 4.35 (2H, t, J=4.4 Hz), 7.06 (1H, d, J=8.0 Hz), 7.13-7.29 (5H, m), 7.44-7.48 (3H, m), 7.53 (1H, d, J=2.2 Hz), 7.61 (2H, d, J=8.0 Hz), 8.64 (1H, s).
IR (KBr) 3386, 2960, 1653, 1518, 1491, 1319, 1248, 1185, 1128, 849 cm⁻¹

| Elemental Analysis for C₃₁H₃₆NO₃P · 0.3H₂O | | | | |
|---|---|---|---|---|
| Calcd. | C, 73.44 ; | H, 7.28 ; | N, 2.76 ; | P, 6.11 : |
| Found. | C, 73.35 ; | H, 7.40 ; | N, 2.62 ; | P, 6.35. |

### Working Example 144 (Production of Compound 144)

Under nitrogen atmosphere, oxalyl chloride (0.12ml) was added to a solution of 7-(4-methylphenyl)-2,3-dihydro-1-benzoxepine-4-carboxyl ic acid (250mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.25ml) and 1-(4-aminobenzyl)-phosphorane-1-oxide (204.8mg) at 0°C, and the mixture was stirred at room temperature 18 hours. The reaction mixture was added to vigorously stirred water to stop the reaction. The mixture was extracted with ethyl acetate, and the organic layer was washed with saturated sodium chloride solution, concentrated and recrystallized from ethanol to give N-(4-(tetramethylene)phosphoryl-methylphenyl)-7-(4-meth ylphenyl)-2,3-dihydro-benzoxepine-4-carboxamide (Compound 144) (316mg) as colorless crystals.
m.p. 273-275°C
¹H-NMR (200MHz, CDCl₃) δ 1.43-1.97 (8H, m), 2.40 (3H, s), 3.09 (2H, t, J=4.4 Hz), 3.20 (2H, d, J=14.4 Hz), 4.40 (2H, t, J=4.4 Hz), 7.06 (1H, d, J=8.4 Hz), 7.18-7.29 (5H, m), 7.44-7.54 (4H, m), 7.60 (2H, d, J=8.0 Hz), 8.12-8.23 (1H, m).
IR (KBr) 3223, 2952, 1653, 1518, 1491, 1321, 1254, 1186, 810 cm⁻¹

| Elemental Analysis for C₂₉H₃₀NO₃P | | | | |
|---|---|---|---|---|
| Calcd. | C, 73.87 ; | H, 6.41 ; | N, 2.97 ; | P, 6.57 : |
| Found. | C, 73.79 ; | H, 6.33 ; | N, 3.00 ; | P, 6.59. |

### Working Example 145 (Production of Compound 145)

Under nitrogen atmosphere, oxalyl chloride (0.47ml) was added to a solution of 7-(4-methylphenyl)-2,3-dihydro-1-benzoxepine-4-carboxyl ic acid (1.0g) in tetrahydrofuran (20ml) at room temperature. To the mixture was added a drop of DMF, and the mixture was stirred for 1 hour. Under reduced pressure, the solvent was evaporated, and the residue was dissolved in tetrahydrofuran (20ml) at 0°C. To the solution were added triethylamine (1.0ml) and 2-(4-aminobenzyl)-3-methoxymethoxypyridine (0.96g), and the mixture was stirred at room temperature for 4 hours. The reaction mixture was added to vigorously stirred water to stop the reaction. The mixture was extracted with ethyl acetate. The organic layer was washed with saturated sodium chloride solution, dried with magnesium sulfate and concentrated. The residue was separated and purified with column chromatography (ethyl acetate/hexane=2:1) to give N-[4-(3-methoxymethoxy-pyridin-2-ylmethyl)phenyl]-7-(4-methylphenyl)-2,3-dihydro-1-benzoxepine-4-carboxamide (Compound 145) (1.63g) as orange crystals.
¹H-NMR (200MHz, CDCl₃) δ 2.39 (3H, s), 3.03 (2H, t, J=4.4 Hz), 3.37 (3H, s), 4.18 (2H, s), 4.32 (2H, t, J=4.4 Hz), 5.17 (2H, s), 7.03 (1H, d, J=8.0 Hz), 7.10 (1H, dd, J=8.4, 4.8 Hz), 7.19-7.51 (12H, m), 7.62 (1H, br s), 8.20 (1H, dd, J=4.8, 1.2 Hz).
IR (KBr) 3275, 2945, 1659, 1516, 1444, 1406, 1491, 1313, 1240, 1153, 982. 814 cm⁻¹

### Working Example 146 (Production of Compound 146)

To a solution of N-[4-(3-methoxymethoxypyridin-2-ylmethyl)phenyl]-7-(4-m ethylphenyl)-2,3-dihydro-1-benzoxepine-4-carboxamide (300mg) in tetrahydrofuran (10ml) was added 3-chloroperbenzoic acid (70%, 0.22g) at 0°C, and the mixture was stirred at room temperature for 18 hours. To the mixture was added sodium thiosulfate, and the mixture was stirred for a few minutes. The mixture was extracted with ethyl acetate, and the organic layer was washed with saturated sodium bicarbonate solution and saturated sodium chloride solution, dried with magnesium sulfate and concentrated under reduced pressure. The residue was separated and purified with column chromatography (ethanol/ethyl acetate=1:15→1:10), and recrystallized from ethanol to give N-[4-(1-oxido-3-methoxymethoxypyridin-2-ylmethyl)phenyl ]-7-(4-methyl-phenyl)-2,3-dihydro-1-benzoxepine-4-carbo xamide (Compound 146) (203mg) as colorless crystals.
m.p. 206-208°C
¹H-NMR (200MHz, CDCl₃) δ 2.39 (3H, s), 3.06 (2H, t, J=4.6 Hz), 3.44 (3H, s), 4.35 (2H, t, J=4.6 Hz), 4.37 (2H, s), 5.24 (2H, s), 6.96-7.08 (3H, m), 7.19-7.27 (4H, m), 7.38-7.52 (7H, m), 7.62 (1H, br s), 7.99 (1H, dd, J=5.0, 2.2 Hz).
IR (KBr) 3305, 1653, 1601, 1516, 1491, 1321, 1244, 1053, 818 cm⁻¹

| Elemental Analysis for C₃₂H₃₀N₂O₅ · 0.2H₂O | | | |
|---|---|---|---|
| Calcd. | C, 73.04 ; | H, 5.82 ; | N, 5.32 : |
| Found. | C, 72.96 ; | H, 5.72 ; | N, 5.30. |

### Working Example 147 (Production of Compound 147)

To a solution of N-[4-(3-methoxymethoxypyridin-2-ylmethyl)phenyl]-7-(4-m ethylphenyl)-2,3-dihydro-1-benzoxepine-4-carboxamide (1.00g) in ethanol (20ml) was added concentrated hydrochloric acid (5.0ml), and the mixture was stirred at room temperature for 4 days. To the mixture was added saturated sodium bicarbonate solution at 0°C to make the solution pH 6-7, and precipitated crystal was collected by filtration to give N-[4-(3-hydroxy-pyridin-2-ylmethyl)phenyl]-7-(4-methylp henyl)-2,3-dihydro-1-benzoxepine-4-carboxamide (Compound 147) (693mg) as pale yellow crystals.
m.p. 285-288°C
¹H-NMR (200MHz, DMSO-d₆) δ 2.34 (3H, s), 2.97 (2H, t, J=4.4 Hz), 4.00 (2H, s), 4.28 (2H, t, J=4.4 Hz), 7.02-7.32 (8H, m), 7.49-7.64 (5H, m), 7.73 (1H, d, J=2.2 Hz), 7.95 (1H, dd, J=4.4, 1.4 Hz), 9.86 (1H, br s).
IR (KBr) 3390, 3028, 1651, 1510, 1408, 1284, 1236, 808 cm⁻¹

| Elemental Analysis for C₃₀H₂₆N₂O₃ . 0.2H₂O | | | |
|---|---|---|---|
| Calcd. | C, 77.30 ; | H, 5.71 ; | N, 6.01 : |
| Found. | C, 77.20 ; | H, 5.63 ; | N, 5.89. |

### Working Example 148 (Production of Compound 148)

To a suspension of N-[4-(3-hydroxypyridin-2-ylmethyl)phenyl]-7-(4-methylph enyl)-2,3-dihydro-1-benzoxepine-4-carboxamide (400mg) in tetrahydrofuran (30ml) was added 3-chloroperbenzoic acid (70%, 0.32g) at 0°C, and the mixture was stirred at room temperature for 15 hours. To the mixture was added sodium thiosulfate, and the mixture was stirred for a few minutes and extracted with ethyl acetate. The organic layer was washed with saturated sodium bicarbonate solution and saturated sodium chloride solution, dried with magnesium sulfate, concentrated under reduced pressure and recrystallized from ethanol to give N-[4-(1-oxido-3-hydroxypyridin-2-ylmethyl)phenyl]-7-(4-methylphenyl)-2,3-dihydro-1-benzoxepine-4-carboxamide (Compound 148) (262mg) as pale yellow crystals.
m.p. 254°C (dec.)
¹H-NMR (200MHz, DMSO-d₆) δ 2.34 (3H, s), 2.92-3.02 (2H, m), 4.14 (2H, s), 4.23-4.34 (2H, m), 6.87 (1H, d, J=7.4 Hz), 7. 04 (1H, d, J=8.6 Hz), 7.11 (1H, dd, J=8.4, 6.6 Hz), 7.18-7.36 (5H, m), 7.48-7.61 (5H, m), 7.73 (1H, d, J=2.2 Hz), 7.83 (1H, dd, J=6.4, 1.0 Hz), 9.88 (1H, s).
IR (KBr) 3180, 3102, 1651, 1601, 1537, 1516, 1493, 1437, 1227, 1036, 816 cm⁻¹

| Elemental Analysis for C₃₀H₂₆N₂O₄ · 0.2H₂O | | | |
|---|---|---|---|
| Calcd. | C, 74.73 ; | H, 5.52 ; | N, 5.81 : |
| Found. | C, 74.63 ; | H, 5.35 ; | N, 5.55. |

### Working Example 149 (Production of Compound 149)

Under nitrogen atmosphere, oxalyl chloride (0.12ml) was added to a solution of 7-(4-methylphenyl)-2,3-dihydro-1-benzoxepine-4-carboxyl ic acid (250mg) in tetrahydrofuran (10ml) at room temperature. To the mixture was added a drop of DMF, and the mixture was stirred for 1 hour. Under reduced pressure, the solvent was evaporated. The residue was dissolved in tetrahydrofuran (15ml), and to the solution were added triethylamine (0.25ml) and 1-(4-aminobenzyl)phosphorinane-1-oxide (219.0mg) at 0°C. The mixture was stirred at room temperature for 4 hours, added to vigorously stirred water to stop the reaction and extracted with chloroform. The organic layer was washed with saturated sodium chloride solution, dried with magnesium sulfate, concentrated and recrystallized from ethanol to give N-(4-(pentamethylene)phosphorylmethyl-phenyl)-7-(4-meth ylphenyl)-2,3-dihydro-1-benzoxepine-4-carboxamide (Compound 149) (253mg) as colorless crystals.
m.p. 283-286°C
¹H-NMR (200MHz, CDCl₃) δ 1.32-2.09 (10H, m), 2.39 (3H, s), 3.04-3.18 (4H, m), 4.36 (2H, t, J=4.6 Hz), 7.06 (1H, d, J=8.4 Hz), 7.19-7.29 (5H, m), 7.44-7.48 (3H, m), 7.53 (1H, d, J=2.6 Hz), 7.59 (2H, d, J=8.4 Hz), 8.09 (1H, br s).
IR (KBr) 3217, 2927, 1655, 1599, 1516, 1493, 1321, 1255, 1236, 1167, 1134, 847, 810 cm⁻¹

| Elemental Analysis for C₃₀H₃₂NO₃P | | | | |
|---|---|---|---|---|
| Calcd. | C, 74.21 ; | H, 6.64 ; | N, 2.88 ; | P, 6.38 : |
| Found. | C, 73.96 ; | H, 6.53 ; | N, 3.11 ; | P, 6.56. |

### Working Example 150 (Production of Compound 150)

Under nitrogen atmosphere, oxalyl chloride (0.06ml) was added to a solution of 7-(4-ethylphenyl)-2,3-dihydro-1-benzoxepine-4-carboxyli c acid (120mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.12ml) and 4-[N-methyl-N-(tetrahydro-pyran-4-yl)aminomethyl]-anili ne (99mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:5) and recrystallized from ethyl acetate to give N-[4-[N-methyl-N-(tetrahydropyran-4-yl)aminomethyl]-phe nyl]-7-(4-ethylphenyl)-2,3-dihydro-1-benzoxepine-4-carb oxamide (Compound 150) (99mg) as colorless crystals.
m.p. 181-182°C
¹H-NMR (200MHz, CDCl₃) δ 1.28 (3H, t, J=7.6 Hz), 1.60-1.82 (4H, m), 2.21 (3H, s), 2.57-2.61 (1H, m), 2.69 (2H, q, J=7.6 Hz), 3.09 (2H, t, J=4.6 Hz), 3.37 (2H, dt, J=3.3, 11.1 Hz), 3.58 (2H, s), 3.98-4.09 (2H, m), 4.37 (2H, t, J=4.6 Hz), 7.06 (1H, d, J=8.4 Hz), 7.23-7.36 (5H, m), 7.44-7.58 (7H, m).
IR (KBr) 3305, 2960, 1647, 1539, 1514, 1491, 1321, 820 cm⁻¹

| Elemental Analysis for C₃₂H₃₆N₂O₃ | | | |
|---|---|---|---|
| Calcd. | C, 77.39 ; | H, 7.31 ; | N, 5.64 : |
| Found. | C, 77.38 ; | H, 7.24 ; | N, 5.66. |

### Working Example 151 (Production of Compound 151)

Under nitrogen atmosphere, oxalyl chloride (0.06ml) was added to a solution of 7-(4-ethylphenyl)-2,3-dihydro-1-benzoxepine-4-carboxyli c acid (120mg) in tetrahydrofuran (10ml) at room temperature. To the mixture was added a drop of DMF, and the mixture was stirred for 1 hour. Under reduced pressure, the solvent was evaporated. The residue was dissolved in tetrahydrofuran (20ml), and to the solution were added triethylamine (0.12ml) and 1-(4-aminobenzyl)phosphorinane-1-oxide (100mg) at 0°C, and the mixture was stirred at room temperature for 5 hours. The reaction mixture was added to vigorously stirred water to stop the reaction, and the mixture was extracted with chloroform. The organic layer was washed with saturated sodium chloride solution, dried with magnesium sulfate and concentrated. The residue was purified with column chromatography (ethanol/ethyl acetate=1:5→1:4) and recrystallized from ethanol to give N- (4- (pentamethylene) - phosphorylmethylphenyl)-7-(4-ethylphenyl)-2,3-dihydro-1 -benzoxepine-4-carboxamide (Compound 151) (88mg) as colorless crystals.
m.p. 287-288°C
¹H-NMR (200MHz, CDCl₃) δ 1.28 (3H, t, J=7.4 Hz), 1.42-2.16 (10H, m), 2.70 (2H, q, J=7.4 Hz), 3.05-3.19 (4H, m), 4.37 (2H, t, J=4.6 Hz), 7.06 (1H, d, J=8.4 Hz), 7.21-7.31 (5H, m), 7.43-7.62 (6H, m), 7.84 (1H, br s).
IR (KBr) 3392, 1655, 1599, 1533, 1516, 1493, 1321, 1255, 1167, 847, 824 cm⁻¹

| Elemental Analysis for C₃₁H₃₄NO₃P | | | | |
|---|---|---|---|---|
| Calcd. | C, 74.53 ; | H, 6.86 ; | N, 2.80 ; | P, 6.20 : |
| Found. | C, 74.23 ; | H, 6.78 ; | N, 2.89 ; | P, 6.07. |

### Working Example 152 (Production of Compound 152)

Under nitrogen atmosphere, oxalyl chloride (0.06ml) was added to a solution of 7-(4-tert-butylphenyl)-2,3-dihydro-1-benzoxepine-4-carb oxylic acid (130mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.12ml) and 4-[N-methyl-N-(tetrahydro-pyran-4-yl)aminomethyl]-anili ne (98mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethyl acetate to give N-[4-[N-methyl-N-(tetrahydropyran-4-yl)aminomethyl]-phe nyl]-7-(4-tert-butylphenyl)-2,3-dihydro-1-benzoxepine-4 -carboxamide (Compound 152) (126mg) as colorless crystals. m.p. 193-194°C
¹H-NMR (200MHz, CDCl₃) δ 1.37 (9H, s), 1.60-1.82 (4H, m), 2.21 (3H, s), 2.56-2.75 (1H, m), 3.09 (2H, t, J=4.6 Hz), 3.29-3.45 (2H, m), 3.58 (2H, s), 3.97-4.09 (2H, m), 4.37 (2H, t, J=4.6 Hz), 7.06 (1H, d, J=8.0 Hz), 7.23-7.35 (3H, m), 7.41-7.58 (9H, m).
IR (KBr) 3342, 2949, 1647, 1512, 1406, 1313, 1240, 1136, 822 cm⁻¹

| Elemental Analysis for C₃₄H₄₀N₂O₃ | | | |
|---|---|---|---|
| Calcd. | C, 77.83 ; | H, 7.68 ; | N, 5.34 : |
| Found. | C, 77.69 ; | H, 7.71 ; | N, 5.39. |

### Working Example 153 (Production of Compound 153)

Under nitrogen atmosphere, oxalyl chloride (0.06ml) was added to a solution of 7-(4-tert-butylphenyl)-2,3-dihydro-1-benzoxepine-4-carb oxylic acid (130mg) in tetrahydrofuran (10ml) at room temperature. To the mixture was added a drop of DMF, and the mixture was stirred for 1 hour. Under reduced pressure, the solvent was evaporated. The residue was dissolved in dichloromethane (10ml), and to the solution were added triethylamine (0.12ml) and 1-(4-aminobenzyl)phosphorinane-1-oxide (99mg) at 0°C, and the mixture was stirred at room temperature for 4 hours. The reaction mixture was added to vigorously stirred water to stop the reaction, and the mixture was extracted with dichloromethane. The organic layer was washed with saturated sodium chloride solution, dried with magnesium sulfate and concentrated. The residue was purified with column chromatography (ethanol/ethyl acetate=1:4) and recrystallized from ethanol to give N- (4- (pentamethylene) - phosphorylmethylphenyl)-7-(4-tert-butylphenyl)-2,3-dihy dro-1-benzoxepine-4-carboxamide (Compound 153) (106mg) as colorless crystals.
m.p. 292-294°C
¹H-NMR (200MHz, CDCl₃) δ 1.36 (9H, s), 1.39-2.10 (10H, m), 3.04-3.19 (4H, m), 4.36 (2H, t, J=4.6 Hz), 7.06 (1H, d, J=8.2 Hz), 7.19-7.30 (3H, m), 7.41-7.63 (8H, m), 8.24 (1H, br s).
IR (KBr) 3236, 1664, 1516, 1491, 1311, 1252, 1232, 1163, 1132, 845, 824 cm⁻¹

| Elemental Analysis for C₃₃H₃₈NO₃P | | | | |
|---|---|---|---|---|
| Calcd. | C, 75.12 ; | H, 7.26 ; | N, 2.65 ; | P, 5.87 : |
| Found. | C, 74.82 ; | H, 7.25 ; | N, 2.73 ; | P, 5.99. |

### Working Example 154 (Production of Compound 154)

Under nitrogen atmosphere, oxalyl chloride (0.06ml) was added to a solution of 7-(4-chlorophenyl)-2,3-dihydro-1-benzoxepine-4-carboxyl ic acid (120mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.12ml) and 4-[N-methyl-N-(tetrahydropyran-4-yl)aminomethyl]aniline (97mg) at 0°C, and the mixture was stirred at room temperature for 3 hours. The reaction mixture was added to vigorously stirred water to stop the react ion. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethyl acetate-diethylether to give N-[4-[N-methyl-N-(tetrahydropyran-4-yl)aminomethyl]-phe nyl]-7-(4-chlorophenyl)-2,3-dihydro-1-benzoxepine-4-car boxamide (Compound 154) (67mg) as colorless crystals. m.p. 191-192°C
¹H-NMR (200MHz, CDCl₃) δ 1.61-1.83 (4H, m), 2.21 (3H, s), 2.54-2.74 (1H, m), 3.09 (2H, t, J=4.7 Hz), 3.31-3.44 (2H, m), 3.58 (2H, s), 3.97-4.09 (2H, m), 4.37 (2H, t, J=4.7 Hz), 7.08 (1H, d, J=8.2 Hz), 7.23-7.58 (12H, m).
IR (KBr) 3309, 1643, 1520, 1485, 1319, 1246, 816 cm⁻¹

| Elemental Analysis for C₃₀H₃₁N₂O₃Cl | | | | |
|---|---|---|---|---|
| Calcd. | C, 71.63 ; | H, 6.21 ; | N, 5.57 ; | Cl, 7.05 _{:} |
| Found. | C, 71.32 ; | H, 6.21 ; | N, 5.60 ; | Cl, 6.81. |

### Working Example 155 (Production of Compound 155)

Under nitrogen atmosphere, oxalyl chloride (0.06ml) was added to a solution of 7-(4-chlorophenyl)-2,3-dihydro-1-benzoxepine-4-carboxyl ic acid (120mg) in tetrahydrofuran (10ml) at room temperature. To the mixture was added a drop of DMF, and the mixture was stirred for 1 hour. Under reduced pressure, the solvent was evaporated. The residue was dissolved in dichloromethane (10ml). To the solution were added triethylamine (0.12ml) and 1-(4-aminobenzyl)phosphorinane-1-oxide (98mg) at 0°C, and the mixture was stirred at room temperature for 3 hours. The reaction mixture was added to vigorously stirred water to stop the reaction, and the mixture was extracted with dichloro-methane. The organic layer was washed with saturated sodium chloride solution, dried with magnesium sulfate and concentrated. The residue was purified with column chromatography (ethanol/ethyl acetate=1:4) and recrystallized from ethanol to give N-(4-pentamethylenephosphorylmethylphenyl)-7-(4-chlorophenyl)-2,3-dihydro-1-benzoxepine-4-carboxamide (Compound 155) (69mg) as colorless crystals.
m.p. 270-272°C
¹H-NMR (200MHz, CDCl₃) δ 1.31-2.10 (10H, m), 3.04-3.18 (4H, m), 4.37 (2H, t, J=4.6 Hz), 7.07 (1H, d, J=8.4 Hz), 7.19-7.29 (3H, m), 7.38-7.52 (6H, m), 7.58 (2H, d, J=8.4 Hz), 8.07 (1H, br s).
IR (KBr) 3230, 2935, 1655, 1599, 1516, 1483, 1317, 1254, 1230, 1157, 824 cm⁻¹

| Elemental Analysis for C₂₉H₂₉NO₃ClP · 0.5H₂O | | | | | |
|---|---|---|---|---|---|
| Calcd. | C, 67.64 ; | H, 5.87 ; | N, 2.72 ; | Cl, 6.88 ; | P, 6.01 : |
| Found. | C, 67.55 ; | H, 5.81 ; | N, 2.79 ; | Cl, 6.67 ; | P, 6.11. |

### Working Example 156 (Production of Compound 156)

Under nitrogen atmosphere, oxalyl chloride (0.05ml) was added to a solution of 7-(4-trifluoromethylphenyl)-2,3-dihydro-1-benzoxepine-4 -carboxylic acid (130mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.1ml) and 4-[N-methyl-N-(tetrahydropyran-4-yl)amino-methyl]anilin e (95mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethyl acetate-hexane to give N-[4-[N-methyl-N-(tetrahydropyran-4-yl)aminomethyl]phen yl]-7-(4-trifluoromethylphenyl)-2,3-dihydro-1-benzoxepi ne-4-carboxamide (Compound 156) (91mg) as colorless crystals.
m.p. 205-209°C
¹H-NMR (200MHz, CDCl₃) δ 1.69-1.82 (4H, m), 2.21 (3H, s), 2.55-2.74 (1H, m), 3.10 (2H, t, J=4.7 Hz), 3.31-3.44 (2H, m), 3.58 (2H, s), 3.99-4.11 (2H, m), 4.39 (2H, t, J=4.7 Hz), 7.11 (1H, d, J=8.4 Hz), 7.25-7.34 (3H, m), 7.46-7.58 (5H, m), 7.62-7.71 (4H, m).
IR (KBr) 3315, 2958, 2846, 1643, 1522, 1327, 1165, 1115, 1072, 835, 822 cm⁻¹

| Elemental Analysis for C₃₁H₃₁N₂O₃F₃ | | | | |
|---|---|---|---|---|
| Calcd. | C, 69.39 ; | H, 5.82; | N, 5.22 ; | F, 10.62 : |
| Found. | C, 69.21 ; | H, 5.79 ; | N, 5.24 ; | F, 10.60. |

### Working Example 157 (Production of Compound 157)

Under nitrogen atmosphere, oxalyl chloride (0.05ml) was added to a solution of 7-(4-trifluoromethylphenyl)-2,3-dihydro-1-benzoxepine-4 -carboxylic acid (130mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.1ml) and 1-(4-aminobenzyl)phosphorinane-1-oxide (94.5mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethyl acetate-hexane to give N-(4-(pentamethylene)phosphorylmethyl-phenyl)-7-(4-trif luoromethylphenyl)-2,3-dihydro-1-benzoxepine-4-carboxam ide (Compound 157) (111mg) as colorless crystals.
m.p. 269°C (dec.)
¹H-NMR (200MHz, CDCl₃) δ 1.19-2.08 (10H, m), 3.03-3.16 (4H, m), 4.38 (2H, t, J=4.6 Hz), 7.10 (1H, d, J=8.4 Hz), 7. 15-7.30 (3H, m), 7.48 (1H, dd, J=8.4, 2.2 Hz), 7.52-7.73 (7H, m), 8.39-8.46 (1H, m).
IR (KBr) 3221, 2937, 1657, 1533, 1516, 1327, 1257, 1167, 1128, 1072, 849, 825 cm⁻¹

| Elemental Analysis for C₃₀H₂₉NO₃F₃P · 0.2H₂O | | | |
|---|---|---|---|
| Calcd. | C, 66.34 ; | H, 5.46 ; | N, 2.58 : |
| Found. | C, 66.21 ; | H, 5.62 ; | N, 2.61. |

### Working Example 158 (Production of Compound 158)

Under nitrogen atmosphere, oxalyl chloride (0.08ml) was added to a solution of 7-(4-ethoxyphenyl)-2,3-dihydro-1-benzoxepine-4-carboxyl ic acid (154.8mg) in tetrahydro-furan (10ml) at room temperature. To the mixture was added a drop of DMF, and the mixture was stirred for 1 hour. Under reduced pressure, the solvent was evaporated. The residue was dissolved in tetrahydrofuran (20ml), and to the solution were added triethylamine (0.2ml) and 4-[N-methyl-N-(tetrahydropyran-4-yl)aminomethyl]-anilin e (121mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethanol to give 7-(4-ethoxyphenyl)-N-[4-[N-methyl-N-(tetrahydropyran-4-yl)aminomethyl]phenyl]-2,3-dihydro-1-benzoxepine-4-carb oxamide (Compound 158) (202mg) as colorless crystals.
m.p. 174-176°C
¹H-NMR (200MHz, CDCl₃) δ 1.44 (3H, t, J=7.0 Hz), 1.62-1.82 (4H, m), 2.21 (3H, s), 2.55-2.72 (1H, m), 3.08 (2H, t, J=4.8 Hz), 3.31-3.44 (2H, m), 3.57 (2H, s), 3.97-4.10 (2H, m), 4.08 (2H, q, J=7.0 Hz), 4.36 (2H, t, J=4.8 Hz), 6.96 (2H, d, J=8.8 Hz), 7.05 (1H, d, J=8.4 Hz), 7.24-7.58 (10H, m).

IR (KBr) 3327, 2947, 1645, 1608, 1514, 1495, 1240, 1180, 1051, 822 cm⁻¹

| Elemental Analysis for C₃₂H₃₆N₂O₄ | | | |
|---|---|---|---|
| Calcd. | C, 74.97 ; | H, 7.08 ; | N, 5.46 : |
| Found. | C, 74.88 ; | H, 7.27 ; | N, 5.50. |

### Working Example 159 (Production of Compound 159)

Under nitrogen atmosphere, oxalyl chloride (0.06ml) was added to a solution of 7-(4-trifluoromethoxyphenyl)-2,3-dihydro-1-benzoxepine-4-carboxylic acid (150mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.12ml) and 4-[N-methyl-N-(tetrahydropyran-4-yl)aminomethyl]aniline (104mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was separated and purified with column chromatography (ethanol/ethyl acetate=1:4), and recrystallized from ethyl acetate-hexane to give N-[4-[N-methyl-N-(tetrahydro-pyran-4-yl)aminomethyl]phe nyl]-7-(4-trifluoromethoxy-phenyl)-2,3-dihydro-1-benzox epine-4-carboxamide (Compound 159) (143mg) as colorless crystals.
m.p. 187-188°C
¹H-NMR (200MHz, CDCl₃) δ 1.62-1.82 (4H, m), 2.21 (3H, s), 2.55-2.74 (1H, m), 3.10 (2H, t, J=4.7 Hz), 3.29-3.45 (2H, m), 3.57 (2H, s), 3.99-4.10 (2H, m), 4.38 (2H, t, J=4.7 Hz), 7.09 (1H, d, J=8.4 Hz), 7.22-7.35 (3H, m), 7.40-7.60 (9H, m).
IR (KBr) 3319, 2960, 2845, 1643, 1520, 1493, 1319, 1261, 1205, 1163, 835, 810 cm⁻¹

| Elemental Analysis for C₃₁H₃₁N₂O₄F₃ | | | | |
|---|---|---|---|---|
| Calcd. | C, 67.38 ; | H, 5.65 ; | N, 5.07 ; | F, 10.31 : |
| Found. | C, 67.39 ; | H, 5.38 ; | N, 5.07 ; | F, 10.18. |

### Working Example 160 (Production of Compound 160)

Under nitrogen atmosphere, oxalyl chloride (0.07ml) was added to a solution of (E)-3- (4-methylphenyl) cinnamic acid (125mg) in tetrahydrofuran (10ml) at room temperature. To the mixture was added a drop of DMF, and the mixture was stirred for 1 hour. Under reduced pressure, the solvent was evaporated, and the residue was dissolved in tetra-hydrofuran (10ml). To the solution were added triethylamine (0.14ml) and (4-aminobenzyl)diethylphosphine oxide (120mg) in tetrahydrofuran (5ml) at 0°C, and the mixture was stirred at room temperature for 1.5 hours. The reaction mixture was added to vigorously stirred water to stop the reaction. The mixture was extracted with ethyl acetate. The organic layer was washed with saturated sodium chloride solution, dried with magnesium sulfate, concentrated and recrystallized from ethanol-ethyl acetate to give (E)-N-(4-diethylphosphorylmethylphenyl)-3-(4-methylphen yl)-cinnamamide (Compound 160) (125mg) as pale yellow crystals.
m.p. 197-198°C
¹H-NMR (200MHz, CDCl₃) δ 1.13 (6H, dt, J=16.6, 8.0 Hz), 1.55-1.71 (4H, m), 2.41 (3H, m), 3.08 (2H, d, J=13.2 Hz), 6.81 (1H, d, J=15.4 Hz), 7.15-7.30 (4H, m), 7.41-7.62 (7H, m), 7.74-7.84 (2H, m), 8.93-9.02 (1H, m).
IR (KBr) 3242, 1678, 1630, 1603, 1541, 1514, 1409, 1344, 1250, 1165, 1130, 985, 847, 791 cm⁻¹

| Elemental Analysis for C₂₇H₃₀NO₂P · 0.3H₂O | | | | |
|---|---|---|---|---|
| Calcd. | C, 74,22 ; | H, 7.06 ; | N, 3.21 ; | P, 7.09 : |
| Found. | C, 73.96 ; | H, 6.77 ; | N, 3.34 ; | P, 7.01. |

### Working Example 161 (Production of Compound 161)

Under nitrogen atmosphere, oxalyl chloride (0.22ml) was added to a solution of (E)-3-(4-methylphenyl)cinnamic acid (0.40g) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.50ml) and 2-(4-amino-benzyl)pyridine (0.34g) in tetrahydrofuran (5ml) at 0°C, and the mixture was stirred at room temperature for 2 hours. The reaction mixture was added to vigorously stirred water to stop the reaction. The mixture was extracted with ethyl acetate. The organic layer was washed with saturated sodium chloride solution, dried with magnesium sulfate, concentrated and recrystallized from ethyl acetate-hexane to give (E)-N-[4-(2-pyridylmethyl)-phenyl]-3-(4-methylphenyl)-cinnamamide (Compound 161) (490mg) as yellow crystals.
m.p. 169-171°C
¹H-NMR (200MHz, CDCl₃) δ 2.41 (3H, s), 4.14 (2H, s), 6.60 (1H, d, J=15.4 Hz), 7.10-7.15 (2H, m), 7.22-7.28 (4H, m), 7.42-7.63 (9H, m), 7.71 (1H, br s), 7.80 (1H, d, J=15.4 Hz), 8.53-8.58 (1H, m).
IR (KBr) 3238, 1673, 1630, 1601, 1539, 1512, 1348, 1248, 1174, 976, 791, 760 cm⁻¹

| Elemental Analysis for C₂₈H₂₄N₂O · 0.1H₂O | | | |
|---|---|---|---|
| Calcd. | C, 82.77 ; | H, 6.00 ; | N, 6.89 : |
| Found. | C, 82.73 ; | H, 5.89 ; | N, 6.97. |

### Working Example 162 (Production of Compound 162)

To a solution of (E)-N-[4-(2-pyridylmethyl)phenyl]-3-(4-methylphenyl)cinnamamide (302mg) in tetrahydrofuran (10ml) was added 3-chloroperbenzoic acid (70%, 0.27g) at 0°C, and the mixture was stirred at room temperature for 18 hours. To the reaction mixture was added sodium thiosulfate solution, and the mixture was stirred for a few minutes. The mixture was extracted with ethyl acetate. The organic layer was washed with saturated sodium bicarbonate solution and saturated sodium chloride solution, dried with magnesium sulfate and concentrated. The residue was recrystallized from ethanol to give (E)-N-[4-(1-oxidopyridin-2-ylmethyl)-phenyl]-3-(4-methylphenyl)cinnamamide (Compound 162) (180mg) as pale yellow crystals.
m.p. 183-185°C
¹H-NMR (200MHz, CDCl₃) δ 2.41 (3H, s), 4.24 (2H, s), 6.64 (1H, d, J=15.4 Hz), 6.96-7.01 (1H, m), 7.12-7.17 (2H, m), 7.22-7.30 (4H, m), 7.40-7.51 (4H, m), 7.54-7.63 (3H, m), 7.66-7.74 (2H, m), 7.82 (1H, d, J=15.4 Hz), 8.29-8.31 (1H, m).
IR (KBr) 3255, 1684, 1605, 1541, 1514, 1412, 1346, 1244, 839, 785 cm⁻¹

| Elemental Analysis for C₂₈H₂₄N₂O₂ | | | |
|---|---|---|---|
| Calcd. | C, 79.98 ; | H, 5.75 ; | N, 6.66 : |
| Found. | C, 80.18 ; | H, 5.63 ; | N, 6.69. |

### Working Example 163 (Production of Compound 163)

Under nitrogen atmosphere, oxalyl chloride (0.19ml) was added to a solution of (E)-3-(4-methylphenyl)cinnamic acid (340mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.4ml) and 4-aminobenzyl-dipropylphosphine oxide (0.38g) at 0°C, and the mixture was stirred at room temperature for 18 hours. The reaction mixture was added to vigorously stirred water to stop the reaction. The mixture was extracted with ethyl acetate. The organic layer was concentrated. The residue was recrystallized from ethanol to give (E)-N-(4-dipropylphosphorylmethyl-phenyl)-3-(4-methylphenyl)cinnamamide (Compound 163) (489mg) as colorless crystals.
m.p. 225-227°C
¹H-NMR (200MHz, DMSO-d₆) δ 0.87-1.00 (6H, m), 1.37-1.63 (8H, m), 2.37 (3H, s), 3.07 (2H, d, J=15.0 Hz), 6.93 (1H, d, J=16.0 Hz), 7.16-7.25 (2H, m), 7.30 (2H, d, J=8.0 Hz), 7.50-7.71 (9H, m), 7.89 (1H, br s).
IR (KBr) 3232, 1676, 1624, 1605, 1545, 1512, 1338, 1151 cm⁻¹

| Elemental Analysis for C₂₉H₃₄NO₂P | | | | |
|---|---|---|---|---|
| Calcd. | C, 75.79 ; | H, 7.46 ; | N, 3.05 ; | P, 6.74 : |
| Found. | C, 75.60 ; | H, 7.68 ; | N, 2.99 ; | P, 6.83. |

### Working Example 164 (Production of Compound 164)

Under nitrogen atmosphere, oxalyl chloride (0.11ml) was added to a solution of (E)-3- (4-methylphenyl) cinnamic acid (200mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.25ml) and 1-(4-aminobenzyl)phosphorane-1-oxide (193mg) at 0°C, and the mixture was stirred at room temperature for 18 hours. The reaction mixture was added to vigorously stirred water to stop the reaction. The mixture was extracted with ethyl acetate. The organic layer was washed with saturated sodium chloride solution and concentrated. The residue was recrystallized from ethanol to give (E)-N-(4-(tetra-methylene)phosphoryl-methylphenyl)-3-(4 -methylphenyl)-cinnamamide (Compound 164) (221mg) as colorless crystals.
m.p. 273-275°C
¹H-NMR (200MHz, CDCl₃) δ 1.48-2.04 (8H, m), 2.41 (3H, s), 3.19 (2H, d, J=13.6 Hz), 6.78 (1H, d, J=15.8 Hz), 7.14-7.31 (4H, m), 7.43-7.59 (7H, m), 7.73-7.76 (1H, m), 7.79 (1H, d, J=15.8 Hz), 8.75-8.84 (1H, m).
IR (KBr) 3232, 1676, 1628, 1603, 1543, 1512, 1410, 1341, 1171, 985, 868, 793 cm⁻¹

| Elemental Analysis for C₂₇H₂₈NO₂P · 0.3H₂O | | | | |
|---|---|---|---|---|
| Calcd. | C, 74.56 ; | H, 6.62 ; | N, 3.22 ; | P, 7.12 : |
| Found. | C, 74.36 ; | H, 6.64 ; | N, 3.20 ; | P, 7.06. |

### Working Example 165 (Production of Compound 165)

Under nitrogen atmosphere, oxalyl chloride (0.12ml) was added to a solution of (E)-3-(4-methylphenyl)cinnamic acid (220mg) in tetrahydrofuran (10ml) at room temperature. To the mixture was added a drop of DMF, and the mixture was stirred for 1 hour. Under reduced pressure, the solvent was evaporated. The residue was dissolved in tetrahydrofuran (20ml), and to the solution were added triethylamine (0.26ml) and 1-(4-amino-benzyl)phosphorinane-1-oxide (226mg) at 0°C. The mixture was stirred at room temperature for 20 hours. The reaction mixture was added to vigorously stirred water to stop the reaction, and the mixture was extracted with chloroform. The organic layer was washed with saturated sodium chloride solution, dried with magnesium sulfate and concentrated. The residue was recrystallized from ethanol to give (E)-N-(4-(penta-methylene)phosphorylmethylphenyl)-3-(4-methylphenyl)-cinnamamide (Compound 165) (271mg) as colorless crystals.
m.p. 273-276°C
¹H-NMR (200MHz, CDCl₃) δ 1.43-2.08 (10H, m), 2.41 (3H, s), 3.13 (2H, d, J=12.8 Hz), 6.81 (1H, d, J=15.8 Hz), 7.14-7.30 (4H, m), 7.41-7.61 (7H, m), 7.76 (1H, s), 7.80 (1H, d, J=15.8 Hz), 8.72-8.87 (1H, m).
IR (KBr) 3242, 1676, 1628, 1603, 1539, 1514, 1344, 1174, 1155, 1126, 991, 789 cm⁻¹

| Elemental Analysis for C₂₈H₃₀NO₂P · 1.5H₂O | | | | |
|---|---|---|---|---|
| Calcd. | C, 71.47 ; | H, 7.06 ; | N, 2.98 ; | P, 6.58 : |
| Found. | C, 71.53 ; | H, 6.99 ; | N, 2.87 ; | P, 6.76. |

### Working Example 166 (Production of Compound 166)

Under nitrogen atmosphere, oxalyl chloride (0.20ml) was added to a solution of 6-(4-methylphenyl)-2H-1-benzopyran-3-carboxylic acid (300mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.31ml) and 1- (4-aminobenzyl) -piperidine (0.24g) at 0°C, and the mixture was stirred at room temperature for 3 hours. The reaction mixture was added to vigorously stirred water to stop the reaction. The mixture was extracted with ethyl acetate. The organic layer was concentrated. The residue was separated and purified with column chromatography (ethanol/ethyl acetate=1:5) to give N-[4-(1-piperidinylmethyl)phenyl]-6-(4-methyl-phenyl)-2 H-1-benzopyran-3-carboxamide (Compound 166) (324mg) as yellow crystals.
m.p. 196-197°C
¹H-NMR (200MHz, CDCl₃) δ 1.41-1.71 (6H, m), 2.34-2.43 (7H, m), 3.46 (2H, s), 5.12 (2H, d, J=1.4 Hz), 6.95 (1H, d, J=8.0 Hz), 7.14 (1H, br s), 7.23-7.29 (3H, m), 7.31-7.38 (2H, m), 7.40-7.46 (6H, m).
IR (KBr) 3361, 1643, 1601, 1529, 1485, 1317, 1254, 810 cm⁻¹

| Elemental Analysis for C₂₉H₃₀N₂O₂ · 0.1H₂O | | | |
|---|---|---|---|
| Calcd. | C, 79.10 ; | H, 6.91 ; | N, 6.36 : |
| Found. | C, 78.85 ; | H, 6.90 ; | N, 6.26. |

### Working Example 167 (Production of Compound 167)

To a solution of N-[4-(1-piperidinylmethyl)phenyl]-6-(4-methylphenyl)-2H-1-benzopyran-3-carboxamide (200mg) in DMF (3ml) was added methyl iodide (0.1ml) at room temperature, and the mixture was stirred for 20 hours. To the mixture was added ethyl acetate. Precipitated crystal was collected by filtration and recrystallized from chloroform-ethanol to give 1-[4-[N-[6-(4-methylphenyl)-2H-1-benzopyran-3-carbonyl] -amino]benzyl]-1-methyl-piperidinium iodide (Compound 167) (188mg) as yellow crystals.
m.p. 210°C (dec.)
¹H-NMR (200MHz, CDCl₃) δ 1.62-2.01 (6H, m), 2.36 (3H, s), 3.06 (3H, br s), 3.34-3.49 (2H, m), 3.60-3.76 (2H, m), 4.97 (2H, br s), 5.04 (2H, br s), 6.85 (1H, d, J=8.4 Hz), 7.17 (2H, d, J=8.2 Hz), 7.37-7.42 (3H, m), 7.47-7.52 (3H, m), 7.83-7.91 (3H, m), 9.00 (1H, br s).
IR (KBr) 3246, 1668, 1527, 1483, 1319, 1248, 808 cm⁻¹

| Elemental Analysis for C₃₀H₃₃N₂O₂I · 0.2H₂O | | | |
|---|---|---|---|
| Calcd. | C, 61.69 ; | H, 5.76 ; | N, 4.80 : |
| Found. | C, 61.53 ; | H, 5.72 ; | N, 4.85. |

### Working Example 168 (Production of Compound 168)

Under nitrogen atmosphere, oxalyl chloride (0.26ml) was added to a solution of 6-(4-methylphenyl)-2H-1-benzopyran-3-carboxylic acid (0.52g) in tetrahydrofuran (10ml) at room temperature. To the mixture was added a drop of DMF, and the mixture was stirred for 1 hour. Under reduced pressure, the solvent was evaporated. The residue was dissolved in tetrahydrofuran (6ml), and to the solution were added triethylamine (0.60ml) and 2-(4-aminobenzyl)-pyridine (0.40g) in tetrahydrofuran (5ml), and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated under reduced pressure. The residue was separated and purified with column chromatography (ethyl acetate/hexane= 2:1) and concentrated to give crystals, which were recrystallized from ethanol-ethyl acetate) to give N-[4-(2-pyridylmethyl)phenyl]-6-(4-methyl-phenyl)-2H-1-benzopyran-3-carboxamide (Compound 168) (353.2mg) as yellow crystals, which were similarly recrystallized to give the second crystals (208mg).
m.p. 184-187°C
¹H-NMR (200MHz, CDCl₃) δ 2.39 (3H, m), 4.14 (2H, s), 5.10 (2H, d, J=1.4 Hz), 6.93 (1H, d, J=8.4 Hz), 7.09-7.15 (3H, m), 7.19-7.32 (5H, m), 7.37-7.66 (7H, m), 8.53-8.57 (1H, m).
IR (KBr) 3296, 1639, 1599, 1531, 1514, 1473, 1325, 1259 cm⁻¹

| Elemental Analysis for C₂₉H₂₄N₂O₂ | | | |
|---|---|---|---|
| Calcd. | C, 80.53 ; | H, 5.59 ; | N, 6.48 : |
| Found. | C, 80.24 ; | H, 5.75 ; | N, 6.43. |

### Working Example 169 (Production of Compound 169)

To a solution of N-[4-(2-pyridylmethyl)phenyl]-6-(4-methylphenyl)-2H-1-b enzopyran-3-carboxamide (250mg) in tetrahydrofuran (10ml) was added 3-chloroperbenzoic acid (70%, 0.21g) at 0°C, and the mixture was stirred at room temperature for 14 hours. To the reaction mixture was added sodium thiosul fate solution, and the mixture was stirred for a few minutes. The mixture was extracted with ethyl acetate. The organic layer was washed with saturated sodium bicarbonate solution and saturated sodium chloride solution, dried with magnesium sulfate and concentrated. The residue was separated and purified with column chromatography (ethanol/ethyl acetate=1:3) concentrated to give crystals, which were recrystallized from chloroform-ethanol to give N-[4-(1-oxidopyridin-2-ylmethyl)phenyl]-6-(4-methyl-phe nyl)-2H-1-benzopyran-3-carboxamide (Compound 169) (191mg) as pale yellow crystals.
m.p. 261-263°C
¹H-NMR (200MHz, CDCl₃) δ 2.40 (3H, s), 4.25 (2H, s), 5.11 (2H, s), 6.92-7.01 (2H, m), 7.13-7.67 (14H, m), 8.29 (1H, t, J=4.2 Hz).
IR (KBr) 3302, 1660, 1605, 1537, 1520, 1250 cm⁻¹

| Elemental Analysis for C₂₉H₂₄N₂O₃ | | | |
|---|---|---|---|
| Calcd. | C, 77.66 ; | H, 5.39 ; | N, 6.25 : |
| Found. | C, 77.90 ; | H, 5.37; | N, 6.21. |

### Working Example 170 (Production of Compound 170)

Under nitrogen atmosphere, oxalyl chloride (0.19ml) was added to a solution of 6-(4-methylphenyl)-2H-1-benzopyran-3-carboxylic acid (380mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.4ml) and 4-aminobenzyldipropylphosphine oxide (0.38g) at 0°C, and the mixture was stirred at room temperature for 3 hours. The reaction mixture was added to vigorously stirred water to stop the reaction. The mixture was extracted with ethyl acetate . The organic layer was concentrated, and the residue was recrystallized from ethanol to give N-(4-dipropylphosphoryl-methyl-phenyl)-6-(4-methylpheny 1)-2H-1-benzopyran-3-carboxamide (Compound 170) (460mg) as pale yellow crystals.
m.p. 192-194°C
¹H-NMR (200MHz, CDCl₃) δ 0.83-0.97 (6H, m), 1.39-1.68 (8H, m), 2.39 (3H, s), 3.05 (2H, d, J=13.2 Hz), 5.12 (2H, d, J=0.8 Hz), 6.94 (1H, d, J=8.4 Hz), 7.11-7.28 (4H, m), 7.31-7.50 (5H, m), 7.61 (2H, d, J=8.4 Hz), 9.13-9.24 (1H, m).
IR (KBr) 3265, 1664, 1628, 1603, 1539, 1514, 1487, 1325, 1252, 1167, 851 cm⁻¹

| Elemental Analysis for C₃₀H₃₄NO₃P | | | | |
|---|---|---|---|---|
| Calcd. | C, 73.90 ; | H, 7.03 ; | N, 2.87 ; | P, 6.35 : |
| Found. | C, 73.95 ; | H, 6.87 ; | N, 2.84 ; | P, 6.41. |

### Working Example 171 (Production of Compound 171)

Under nitrogen atmosphere, oxalyl chloride (0.11ml) was added to a solution of 6-(4-methylphenyl)-2H-1-benzopyran-3-carboxylic acid (230mg) in tetrahydrofuran (10ml) at room temperature. To the mixture was added a drop of DMF, and the mixture was stirred for 1 hour. Under reduced pressure, the solvent was evaporated. The residue was dissolved in tetra-hydrofuran (20ml), and to the solution were added triethylamine (0.25ml) and 1-(4-aminobenzyl)-phosphorane-1-oxide (200mg) at 0°C, and the mixture was stirred at room temperature for 20 hours. The reaction mixture was added to vigorously stirred water to stop the reaction. Precipitated crystal was collected by filtration to give N-(4-tetramethylenephosphorylmethyl-phenyl)-6-(4-methyl phenyl)-2H-1-benzopyran-3-carboxamide (Compound 171) (181mg) as colorless crystals.
m.p. >300°C
¹H-NMR (200MHz, CDCl₃) δ 1.49-2.04 (8H, m), 2.40 (3H, s), 3.22 (2H, d, J=14.4 Hz), 5.12 (2H, s), 6.94 (1H, d, J=8.4 Hz), 7.21-7.29 (4H, m), 7.34-7.50 (5H, m), 7.58 (2H, d, J=8.4 Hz), 8.04-8.07 (1H, m).
IR (KBr) 3236, 1657, 1601, 1535, 1518, 1487, 1323, 1255, 1180, 810 cm⁻¹

| Elemental Analysis for C₂₈H₂₈NO₃P · 0.3H₂O | | | | |
|---|---|---|---|---|
| Calcd. | C, 72.65 ; | H, 6.23 ; | N, 3.03 ; | P, 6.69 : |
| Found. | C, 72.30 ; | H, 5.90 ; | N, 3.00 ; | P, 6.98. |

### Working Example 172 (Production of Compound 172)

Under nitrogen atmosphere, oxalyl chloride (0.12ml) was added to a solution of 6-(4-methylphenyl)-2H-1-benzopyran-3-carboxylic acid (240mg) in tetrahydrofuran (10ml) at room temperature. To the mixture was added a drop of DMF, and the mixture was stirred for 1 hour. Under reduced pressure, the solvent was evaporated. The residue was dissolved in tetra-hydrofuran (20ml), and to the solution were added triethylamine (0.25ml) and 1-(4-aminobenzyl)-phosphorinane-1-oxide (221mg) at 0°C, and the mixture was stirred at room temperature for 3 hours. The reaction mixture was added to vigorously stirred water to stop the reaction. The mixture was extracted with chloroform. The organic layer was washed with saturated sodium chloride solution, dried with magnesium sulfate and concentrated under reduced pressure. The residue was recrystallized from ethanol to give N-(4-(pentamethylene)phosphorylmethylphenyl)-6-(4-methy lphenyl)-2H-1-benzo-pyran-3-carboxamide (Compound 172) (257mg) as yellow crystals.
m.p. 268°C (dec.)
¹H-NMR (200MHz, CDCl₃) δ 1.39-2.15 (10H, m), 2.40 (3H, s), 3.14 (2H, d, J=12.8 Hz), 5.12 (2H, s), 6.94 (1H, d, J=8.0 Hz), 7.18-7.49 (9H, m), 7.59 (2H, d, J=8.4 Hz), 8.54 (1H, br s) .
IR (KBr) 3296, 1660, 1533, 1514, 1323, 1255, 1163, 845, 812 cm⁻¹

| Elemental Analysis for C₂₉H₃₀NO₃P | | | | |
|---|---|---|---|---|
| Calcd. | C, 73.87; | H, 6.41 ; | N, 2.97 ; | P, 6.57 : |
| Found. | C, 74.20 ; | H, 6.39 ; | N, 2.78 ; | P, 6.45. |

### Working Example 173 (Production of Compound 173)

Under nitrogen atmosphere, oxalyl chloride (0.06ml) was added to a solution of 6-(4-methylphenyl)-2H-1-benzopyran-3-carboxylic acid (120mg) in tetrahydrofuran (10ml) at room temperature. To the mixture was added a drop of DMF, and the mixture was stirred for 1 hour. Under reduced pressure, the solvent was evaporated. The residue was dissolved in tetra-hydrofuran (20ml). To the solution were added triethylamine (0.2ml) and 4-[N-methyl-N-(tetrahydropyran-4-yl)aminomethyl]-aniline (109mg) at 0°C, and the mixture was stirred at room temperature for 4 hours. The reaction mixture was added to vigorously stirred water to stop the reaction. The mixture was extracted with ethyl acetate. The organic layer was washed with saturated sodium chloride solution, dried with magnesium sulfate and concentrated under reduced pressure. The residue was separated and purified with column chromatography (ethanol/ethyl acetate=1:4), and recrystallized from ethyl acetate-hexane to give N-[4-[N-methyl-N-(tetrahydro-pyran-4-yl)aminomethyl]-ph enyl]-6-(4-methylphenyl)-2H-1-benzopyran-3-carboxamide (Compound 173) (117mg) as pale yellow crystals.
m.p. 143-145°C
¹H-NMR (200MHz, CDCl₃) δ 1.62-1.84 (4H, m), 2.21 (3H, s), 2.40 (3H, s), 2.56-2.74 (1H, m), 3.28-3.45 (2H, m), 3.57 (2H, s), 3.98-4.11 (2H, m), 5.12 (2H, d, J=1.0 Hz), 6.94 (1H, d, J=8.4Hz), 7.15 (1H,brs), 7.21-7.37 (5H, m), 7.39-7.59 (6H, m).
IR (KBr) 3280, 2937, 2848, 1649, 1597, 1539, 1489, 1336, 1257, 1138, 1007, 810 cm⁻¹

| Elemental Analysis for C₃₀H₃₂N₂O₃ | | | |
|---|---|---|---|
| Calcd. | C, 76.90 ; | H, 6.88 ; | N, 5.98 : |
| Found. | C, 76.56 ; | H, 6.87 ; | N, 6.00. |

### Working Example 174 (Production of Compound 174)

Under nitrogen atmosphere, oxalyl chloride (0.06ml) was added to a solution of 6-(4-methylphenyl)-2H-1-benzopyran-3-carboxylic acid (120m) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (20ml). To the solution were added triethylamine (0.13ml) and 4-[N-methyl-N-(tetra-hydrothiopyran-4-yl)amino-methyl]a niline (117mg) at 0°C, and the mixture was stirred at room temperature for 4 hours. The reaction mixture was added to vigorously stirred water to stop the reaction. The mixture was extracted with ethyl acetate. The organic layer was washed with saturated sodium chloride solution, dried with magnesium sulfate and concentrated under reduced pressure. The residue was separated and purified with column chromatography (ethanol/ethyl acetate=1:4), and recrystallized from ethyl acetate-hexane to give N-[4-[N-methyl-N-(tetrahydrothiopyran-4-yl)aminomethyl]phenyl]-6-(4-methylphenyl)-2H-1-benzopyran-3-carboxamide (Compound 174) (125mg) as pale yellow crystals.
m.p. 169-171°C
¹H-NMR (200MHz, CDCl₃) δ 1.63-1.80 (2H, m), 2.09-2.24 (2H, m), 2.21 (3H, s), 2.40 (3H, s), 2.42-2.56 (1H, m), 2.64-2.74 (4H, m), 3.57 (2H, s), 5.12 (2H, d, J=1.0 Hz), 6.94 (1H, d, J=8.8 Hz), 7.15 (1H, br s), 7.23-7.36 (5H, m), 7.39-7.57 (6H, m).
IR (KBr) 3286, 2922, 1649, 1597, 1539, 1336, 1319, 1261, 808 cm⁻¹

| C₃₀H₃₂N₂O₂S | | | | |
|---|---|---|---|---|
| Calcd. | C, 74.35 ; | H, 6.65 ; | N, 5.78 ; | S, 6.62 : |
| Found. | C, 74.25 ; | H, 6.47 ; | N, 5.91 ; | S, 6.52. |

### Working Example 175 (Production of Compound 175)

To a solution of (E)-3-[5-(4-methylphenyl)thiophen-2-yl]acrylic acid (400mg) in tetrahydrofuran (10ml) was added oxalyl chloride (0.22ml) at room temperature. To the mixture was added a drop of DMF, and the mixture was stirred for 1 hour. Under reduced pressure, the solvent was evaporated, and the residue was dissolved in tetrahydrofuran (20ml). To the solution were added triethylamine (0.46ml) and 4-[N-methyl-N-(tetrahydropyran-4-yl)amino-methyl]anilin e (0.40g) at 0°C, and the mixture was stirred at room temperature for 18 hours. The reaction mixture was added to vigorously stirred water to stop the reaction. The mixture was extracted with chloroform. The organic layer was washed with saturated sodium chloride solution, dried with magnesium sulfate and concentrated under reduced pressure. The residue was recrystallized from ethanol to give (E)-N-[4-[N-methyl-N-(tetrahydropyran-4-yl)amino-methyl ]phenyl]-3-[5-(4-methylphenyl)thiophen-2-yl]-acrylic amide (Compound 175) (293mg) as yellow crystal.
m.p. 199-201°C
¹H-NMR (200MHz, CD₃OD) δ 1.57-1.95 (4H, m), 2.32 (3H, s), 2.36 (3H, s), 2.74-2.96 (1H, m), 3.32-3.47 (2H, m), 3.76 (2H, s), 3.96-4.09 (2H, m), 6.55 (1H, d, J=15.2 Hz), 7.23 (2H, d, J=8.4 Hz), 7.29-7.36 (4H, m), 7.56 (2H, d, J=8.0 Hz), 7.66 (2H, d, J=8.4 Hz), 7.75 (1H, d, J=15.2Hz).
IR (KBr) 3359, 1668, 1608, 1554, 1512, 1363, 802 cm⁻¹

| Elemental Analysis for C₂₇H₃₀N₂O₂S · 1.2H₂O | | | |
|---|---|---|---|
| Calcd. | C, 69.26 ; | H, 6.97 ; | N, 5.98 : |
| Found. | C, 69.28 ; | H, 6.90 ; | N, 6.06. |

### Working Example 176 (Production of Compound 176)

To a solution of (E)-3-[5-(4-methylphenyl)thiophen-2-yl]acrylic acid (150mg) in tetrahydrofuran (10ml) was added oxalyl chloride (0.1ml) at room temperature. To the mixture was added a drop of DMF, and the mixture was stirred for 1 hour. Under reduced pressure, the solvent was evaporated, and the residue was dissolved in tetrahydrofuran (30ml). To the solution were added triethylamine (0.2ml) and 1-(4-aminobenzyl)phosphorinane-1-oxide (150mg) at 0°C, and the mixture was stirred at room temperature for 16 hours. The reaction mixture was added to vigorously stirred water to stop the reaction. The mixture was extracted with ethyl acetate. The organic layer was washed with saturated sodium chloride solution, dried with magnesium sulfate and concentrated under reduced pressure. The residue was recrystallized from ethanol to give (E)-N-(4-penta-methylenephosphorylmethylphenyl)-3-[5-(4 -methylphenyl)-thiophen-2-yl]acrylic amide (Compound 176) (172mg) as yellow crystals.
m.p. 294-297°C
¹H-NMR (200MHz, CDCl₃) δ 1.35-2.13 (10H, m), 2.29 (3H, s), 3.06 (2H, d, J=13.0 Hz), 6.36-6.48 (1H, m), 7.06-7.17 (6H, m), 7.38-7.49 (4H, m), 7.73 (1H, d, J=15.0 Hz).
IR (KBr) 3048, 1672, 1606, 1541, 1512, 1348, 1151, 804 cm⁻¹

| Elemental Analysis for C₂₆H₂₈NO₂SP | | | | |
|---|---|---|---|---|
| Calcd. | C, 69.47 ; | H, 6.28 ; | N, 3.12 ; | P, 6.89 : |
| Found. | C, 69.48 ; | H, 6.23 ; | N, 3.20 ; | P, 7.17. |

### Working Example 177 (Production of Compound 177)

To a solution of (E) -3- [5- (4-methylphenyl) furan-2-yl] acrylic acid (200mg), 4-[N-methyl-N-(tetrahydropyran-4-yl)aminomethyl]aniline (212mg) and triethylamine (0.15ml) in DMF (10ml) was added diethyl cyanophosphate (0.16ml) at 0°C, and the mixture was stirred at room temperature for 3 hours. To the mixture was added ethyl acetate, and the mixture was washed with water and saturated sodium chloride solution, dried with magnesium sulfate and concentrated. The residue was separated and purified with column chromatography (ethanol/ethyl acetate=1:50→:25→ 1:10) to give (E)-N-[4-[N-methyl-N-(tetrahydropyran-4-yl)aminomethyl] phenyl]-3-[5-(4-methylphenyl)furan-2-yl]acrylic amide (Compound 177) (87mg) as brown amorphous.
¹H-NMR (200MHz, CDCl₃) δ 1.53-1.85 (4H, m), 2.21 (3H, s), 2.38(3H, s), 2.54-2.72 (1H, m), 3.31-3.44 (2H, m), 3.56 (2H, s), 3.98-4.11 (2H, m), 6.52 (1H, d, J=15.4 Hz), 6.67-6.69 (2H, m), 7.22 (2H, d, J=8.0 Hz), 7.29 (2H, d, J=8.4 Hz), 7.41 (1H, s), 7.48-7.64 (5H, m).

### Working Example 178 (Production of Compound 178)

To a solution of (E)-3-[5-(4-methylphenyl)furan-2-yl]acrylic acid (150mg), 1-(4-aminobenzyl)-phosphorinane-1-oxide (161mg) and triethylamine (0.11ml) in DMF (10ml) was added diethyl cyanophosphate (0.12ml) at 0°C, and the mixture was stirred at room temperature for 3 hours. To the mixture was added ethyl acetate, and the mixture was washed with water and saturated sodium chloride solution, dried with magnesium sulfate and concentrated. The residue was separated and purified with column chromatography (ethanol/ethyl acetate=1:10→1:5→1:4) to give (E) -N- (4- (pentamethylene)phosphorylmethylphenyl) -3- [5- ( 4-methylphenyl)furan-2-yl]acrylic amide (Compound 178) (53mg) as brown crystals.
¹H-NMR (200MHz, CDCl₃) δ 1.43-2.09 (10H, m), 2.39 (3H, s), 3.15 (2H, d, J=13.2 Hz), 6.58-6.70 (3H, m), 7.16-7.29 (4H, m), 7.48-7.65 (5H, m), 8.24-8.35 (1H, m).
IR (KBr) 3292, 1672, 1614, 1541, 1512, 1489, 1412, 1335, 1244, 1120, 787 cm⁻¹

### Working Example 179 (Production of Compound 179)

Under nitrogen atmosphere, oxalyl chloride (0.16ml) was added to a solution of (E)-3-[4-(4-methylphenyl)-thiophen-2-yl]acrylic acid (300mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (10ml). To the solution were added triethylamine (0.4ml) and 4-[N-methyl-N-(tetrahydropyran-4-yl)aminomethyl]-aniline (298mg) at 0°C, and the mixture was stirred at room temperature for 3 hours. The reaction mixture was added to vigorously stirred water to stop the reaction. The mixture was extracted with chloroform. The organic layer was washed with saturated sodium chloride solution, dried with magnesium sulfate and concentrated under reduced pressure. The residue was separated and purified with column chromatography (ethanol/ethyl acetatel:4), and recrystallized from ethanol to give pale yellow crystals, which were recrystallized from tetrahydrofuran-hexane to give (E)-N-[4-[N-methyl-N-(tetrahydropyran-4-yl)aminomethyl] -phenyl]-3-[4-(4-methylphenyl)thiophen-2-yl]acrylamide (Compound 179) (261mg) as pale yellow crystals.
m.p. 188-190°C
¹H-NMR (200MHz, CDCl₃) δ 1.45-1.83 (4H, m), 2.20 (3H, s), 2.38 (3H, s), 2.55-2.73 (1H, m), 3.31-3.44 (2H, m), 3.56 (2H, s), 3.99-4.10 (2H, m), 6.38 (1H, d, J=15.2 Hz), 7.20-7.32 (5H, m), 7.41-7.58 (6H, m), 7.89 (1H, d, J=15.2 Hz).
IR (KBr) 3329, 2954, 1668, 1608, 1554, 1512, 1412, 1360, 1342, 1254, 1174, 1159, 984, 816 cm⁻¹

| Elemental Analysis for C₂₇H₃₀N₂O₂S1.0H₂O | | | |
|---|---|---|---|
| Calcd. | C, 69.80 ; | H, 6.94 ; | N, 6.03 : |
| Found. | C, 69.94 ; | H, 6.85 ; | N, 5.98. |

### Working Example 180 (Production of Compound 180)

Under nitrogen atmosphere, oxalyl chloride (0.08ml) was added to a solution of (E)-3-[4-(4-methylphenyl)-thiophen-2-yl]acrylic acid (150mg) in tetrahydrofuran (10ml) at room temperature. To the mixture was added a drop of DMF, and the mixture was stirred for 1 hour. Under reduced pressure, the solvent was evaporated, and the residue was dissolved in tetrahydrofuran (20ml). To the solution were added triethylamine (0.2ml) and 1-(4-aminobenzyl)-phosphorinane-1-oxide (150mg) at 0°C, and the mixture was stirred at room temperature for 4 hours. The reaction mixture was added to vigorously stirred water to stop the reaction. The mixture was extracted with ethyl acetate. The organic layer was washed with saturated sodium chloride solution, dried with magnesium sulfate and concentrated under reduced pressure. The residue was recrystallized from ethanol to give (E)-N-(4-(penta-methylene)phosphorylmethylphenyl)-3-[4-(4-methyl-phenyl)thiophen-2-yl]acrylic amide (Compound 180) (138mg) as pale yellow crystals.
m.p. 279°C (dec.)
¹H-NMR (200MHz, CDCl₃) δ 1.49-2.23 (10H, m), 2.38 (3H, s), 3.15 (2H, d, J=12.8 Hz), 6.61 (1H, d, J=15.2 Hz), 7.13-7.28 (4H, m), 7.38-7.57 (6H, m), 7.86 (1H, d, J=15.2 Hz), 9.09-9.20 (1H, m).
IR (KBr) 3392, 2935, 1672, 1618, 1543, 1512, 1336, 1250, 1161, 818 cm⁻¹

| Elemental Analysis for C₂₆H₂₈NO₂SP · 0.3H₂O | | | | |
|---|---|---|---|---|
| Calcd. | C, 68.64 ; | H, 6.34 ; | N, 3.08 ; | P, 6.81 : |
| Found. | C, 68.44 ; | H, 6.30 ; | N, 3.06 ; | P, 6.65. |

### Working Example 181 (Production of Compound 181)

Under nitrogen atmosphere, oxalyl chloride (0.12ml) was added to a solution of 2- (4-methylphenyl)-7,8-dihydro-6H-cyclohepta[b]thiophene-5-carboxylic acid (250mg) in tetrahydrofuran (10ml) at room temperature. To the mixture was added a drop of DMF, and the mixture was stirred for 2 hours. Under reduced pressure, the solvent was evaporated, and the residue was dissolved in tetrahydrofuran (20ml). To the solution were added triethylamine (0.25ml) and 4-[N-methyl-N-(tetrahydropyran-4-yl)aminomethyl]aniline (215mg) at 0°C, and the mixture was stirred at room temperature for 4 hours. The reaction mixture was added to vigorously stirred water to stop the reaction. The mixture was extracted with chloroform. The organic layer was washed with saturated sodium chloride solution, dried with magnesium sulfate and concentrated. The residue was purified with column chromatography (ethanol/ethyl acetate=1:4) and recrystallized from ethanol to give N-[4-[N-methyl-N-(tetrahydropyran-4-yl)aminomethyl]-phe nyl]-2-(4-methylphenyl)-7,8-dihydro-6H-cyclohepta-[b]th iophene-5-carboxamide (Compound 181) (319mg) as colorless crystals.
m.p. 201-203°C
¹H-NMR (200MHz, CDCl₃) δ 1.62-1.84 (4H, m), 2.06-2.18 (2H, m), 2.21 (3H, s), 2.36 (3H, s), 2.53-2.71 (1H, m), 2.79-2.87 (2H, m), 3.06-3.15 (2H, m), 3.31-3.44 (2H, m), 3.57 (2H, s), 3.97-4.08 (2H, m), 7.08 (1H, s), 7.14-7.22 (3H, m), 7.30 (2H, d, J=8.8 Hz), 7.43 (2H, d, J=8.0 Hz), 7.50-7.56 (3H, m).
IR (KBr) 3311, 2943, 1649, 1518, 1408, 1311, 810 cm⁻¹

| Elemental Analysis for C₃₀H₃₄N₂O₂S | | | | |
|---|---|---|---|---|
| Calcd. | C, 74.04 ; | H, 7.04 ; | N, 5.76 ; | S, 6.59 : |
| Found. | C, 73.92 ; | H, 6.85 ; | N, 5.70 ; | S, 6.53. |

### Working Example 182 (Production of Compound 182)

To a solution of (E)-3-[5-(4-methylphenyl)pyridin-3-yl]acrylic acid (150mg), 4-[N-methyl-N-(tetrahydro-pyran-4-yl)aminomethyl]anilin e (168mg) and triethylamine (0.10ml) in DMF (10ml) was added diethyl cyanophosphate (0.12ml) at 0°C, and the mixture was stirred at room temperature for 3 hours and concentrated under reduced pressure. To the residue was added water, the mixture was extracted with chloroform. The organic layer was washed with saturated sodium chloride solution, dried with magnesium sulfate and concentrated under reduced pressure. The residue was separated and purified with column chromatography (ethanol/ethyl acetate=1:2) to give (E)-N-[4-[N-methyl-N-(tetrahydropyran-4-yl)aminomethyl] -phenyl]-3-[5-(4-methylphenyl)pyridin-3-yl]acrylic amide (Compound 182) (24mg) as yellow solid.
¹H-NMR (200MHz, CDCl₃) δ 1.66-1.83 (4H, m), 2.21 (3H, s), 2.43 (3H, s), 2.53-2.74 (1H, m), 3.30-3.45 (2H, m), 3.57 (2H, s), 3.99-4.10 (2H, m), 6.69 (1H, d, J=15.5 Hz), 7.24-7.37 (4H, m), 7.41-7.63 (5H, m), 7.82 (1H, d, J=15.5Hz), 7.95-8.01 (1H, m), 8.74 (1H, d, J=1.8 Hz), 8.81 (1H, d, J=2.2 Hz).
IR (KBr) 3242, 3190, 1678, 1606, 1545, 1514, 1348, 976, 816 cm⁻¹

### Working Example 183 (Production of Compound 183)

To a solution of 6-(4-methylphenyl)-2-methyl-quinoline-3-carboxylic acid (120mg) and 1-hydroxy-benzotriazole (88mg) in DMF (5ml) was added 1-ethyl-3-(3'-dimethylaminopropyl)carbodiimide hydrochloride (125mg) at room temperature, and the mixture was stirred for 2 hours. To the mixture was added a solution of 4-[N-methyl-N-(tetrahydropyran-4-yl)aminomethyl]aniline (105mg) and triethylamine (0.2ml) in DMF (5ml), and the mixture was stirred for 18 hours and concentrated under reduced pressure. To the residue was added water, and the mixture was extracted with chloroform. The organic layer was washed with saturated sodium chloride solution, dried with magnesium sulfate and concentrated under reduced pressure. The residue was separated and purified with column chromatography (ethanol/ethyl acetate=1:2), and recrystallized from ethyl acetate-hexane to give N-[4-[N-methyl-N-(tetrahydropyran-4-yl)aminomethyl]phen yl]-6-(4-methylphenyl)-2-methylquinoline-3-carboxamide (Compound 183) (82mg) as pale yellow crystals.
m.p. 157-160°C
¹H-NMR (200MHz, CDCl₃) δ 1.49-1.85 (4H, m), 2.23 (3H, s), 2.43 (3H, s), 2.54-2.76 (1H, m), 2.89 (3H, s), 3.31-3.47 (2H, m), 3.60 (2H, s), 4.00-4.11 (2H, m), 7.25-7.41 (4H, m), 7.55-7.71 (4H, m), 7.83 (1H, br s), 7.88 (1H, d, J=1.8 Hz), 8.01 (1H, dd, J=8.8, 1.8 Hz), 8.09 (1H, d, J=8.8 Hz), 8.21 (1H, s).
IR (KBr) 3311, 2958, 1657, 1520, 1313, 110, 847, 812 cm⁻¹

| Elemental Analysis for C₃₁H₃₃N₃O₂· 0.3H₂O | | | |
|---|---|---|---|
| Calcd. | C, 76.76 ; | H, 6.98 ; | N, 8.66 : |
| Found. | C, 76.68 ; | H, 7.07 ; | N, 8.80. |

### Working Example 184 (Production of Compound 184)

In THF (20ml) was dissolved 7-phenyl-3,4-dihydro-naphthalene-2-carboxylic acid (1.00g), and to the solution were added oxalyl chloride (523 µl) and a drop of DMF. The mixture was stirred at room temperature for 1 hour and concentrated under reduced pressure. The residue was dissolved in THF (20ml), and to the solution were added 1-(3-aminobenzyl)piperidine (837mg) and triethylamine (673µl) at room temperature. The reaction mixture was stirred at room temperature for 2 hours, and to the mixture was added water (100ml). The mixture was extracted with ethyl acetate. The organic layer was washed with saturated sodium chloride solution, dried with anhydrous sodium sulfate, and concentrated under reduced pressure. The residue was recrystallized from ethyl acetate-diisopropylether to give 7-phenyl-N-[3-(piperidinomethyl)phenyl]-3,4-dihydro-nap hthalene-2-carboxamide(Compound 184) (1.29g) as pale yellow crystals.
mp 152-153°C

| Elemental Analysis for C₂₉H₃₀N₂O · 0.1H₂O | | | |
|---|---|---|---|
| Calcd: | C, 82.08; | H, 7.17; | N, 6.60. |
| Found: | C, 81.97; | H, 7.27; | N, 6.47. |

IR (KBr) cm⁻¹: 3373, 2933, 1645, 1543, 1487, 1439, 770, 696 ¹H NMR (200MHz, CDCl₃) δ : 1.35-1.70 (6H, m), 2.32-2.45 (4H, m), 2.65-2.80 (2H, m), 2.92-3.03 (2H, m), 3.48 (2H, s), 7.08 (1H, d, J=7.6Hz), 7.25-7.50 (10H, m), 7.52-7.67 (3H, m).

### Working Example 185 (Production of Compound 185)

In DMF (3ml) was dissolved 7-phenyl-N- [3- (piperidino-methyl)phenyl]-3,4-dihydronaphthalene-2-carboxamide (200mg), and to the mixture was added methyl iodide (88 µl). The mixture was stirred at room temperature for 15 hours and concentrated under reduced pressure. The residue was recrystallized from methanol-ethyl acetate to give 1-methyl-1-[3-(7-phenyl-3,4-dihydronaphthalene-2-carbox amido)benzyl]-piperidinium iodide (Compound 185) (211mg) as colorless crystals.
mp 208-209°C

| Elemental Analysis for C₃₀H₃₃N₂OI | | | |
|---|---|---|---|
| Calcd: | C, 63.83; | H, 5.89; | N, 4.96. |
| Found: | C, 63.58; | H, 5.89; | N, 4.95. |

IR (KBr) cm⁻¹: 3450, 1657, 1520, 1483, 1439, 1250, 1215, 766, 702 ¹H NMR (200MHz, DMSO-d₆) δ: 1.40-2.00 (6H, m), 2.55-2.70 (2H, m), 2.80-3.00 (5H, m), 3.20-3.40 (4H, m), 4.57 (2H, s), 7.20-7.82 (12H, m), 8.03 (1H, s), 10.14 (1H, s).

### Working Example 186 (Production of Compound 186)

To a solution of 2-(4-methylphenyl)-6,7-dihydro-5H-benzocycloheptene-8-c arboxylic acid (0.2g) in dichloromethane (5ml) were added oxalyl chloride (0.19ml) and dimethylformamide (catalytic amount) under ice-cooling, and the mixture was stirred at room temperature for 2 hours. The solvent was evaporated, and the residue was dissolved in tetrahydrofuran. The mixture was added to a solution of 4-(N-methyl-N-(tetrahydropyran-4-yl)aminomethyl)aniline (0.17g) and triethylamine (0.3ml) in tetrahydrofuran (10ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and precipitated crude crystal was recrystallized from ethyl acetate-hexane to give 2-(4-methylphenyl)-N-(4-((N-tetrahydropyran-4-yl-N-meth yl-amino)methyl)phenyl)-6,7-dihydro-5H-benzo-cyclohepte ne-8-carboxamide (Compound 186) (0.29g) as colorless crystals.
mp 161-162°C.
¹H-NMR(δ ppm, CDCl₃) : 1.59-1.77 (4H, m), 2.13-2.21 (2H, m), 2.21 (3H, s), 2.40 (3H, s), 2.55-2.75 (3H, m), 2.86-2.92 (2H, m), 3.37 (2H, dt, J=2.8, 10.9Hz), 3.57 (2H, s), 4.01-4.07 (2H, m), 7.21-7.33 (4H, m), 7.41-7.58 (7H, m), 7.63 (1H, s).
IR (KBr) ν : 2938, 1651cm⁻¹.

| Anal. for C₃₂H₃₆N₂O₂ : | | | |
|---|---|---|---|
| Calcd. | C,79.97; | H,7.55; | N,5.83. |
| Found | C,79.63; | H,7.43; | N,5.64. |

### Working Example 187 (Production of Compound 187)

A solution of 2-(4-methylphenyl)-N-(4-((N-tetra-hydropyran-4-yl-N-met hylamino)methyl)phenyl)-6,7-dihydro-5H-benzocyclohepten e-8-carboxamide (0.11g) and methyl iodide (0.02ml) in dimethylformamide (4ml) was stirred at room temperature over night. The solvent was evaporated, and to the residue was added ethyl acetate. Precipitated crude crystal was filtered, which was recrystallized from ethanol-ethyl acetate to give N,N-dimethyl-N-(4-((2-(4-methylphenyl)-6,7-dihydro-5H-b enzocyclohepten-8-yl)carbonyl)aminobenzyl)-N-(4-tetrahy dropyranyl) ammonium iodide (Compound 187) (0.13g) as pale yellow crystals.
mp 157-158°C.
¹H-NMR(δ ppm, DMSO-d₆) : 1.80-2.20 (6H, m), 2.35 (3H, s), 2.64 (2H, t, J=6.6Hz), 2.80-2.88 (2H, m), 2.88 (6H, s), 3.33-3.40 (2H, m), 3.50-3.65 (1H, m), 4.02-4.09 (2H, m), 4.47 (2H, s), 7.26-7.37 (4H, m), 7.50-7.60 (5H, m), 7.66 (1H, s), 7.88 (2H, d, J=8.8Hz), 10.22 (1H, s).
IR(KBr) ν : 1659cm⁻¹.

| Anal. for C₃₃H₃₉IN₂O₂·0.5H₂O: | | | |
|---|---|---|---|
| Calcd. | C,62.76; | H,6.38; | N,4.44. |
| Found | C,62.69; | H,6.38; | N,4.21. |

### Working Example 188 (Production of Compound 188)

A solution of 7-(4-piperidinophenyl)-N-(4-((N-tetrahydropyran-4-yl-N-methylamino)methyl)phenyl)-2,3-dihydro-1-benzoxepine-4-carboxamide (0.2g) and methyl iodide (0.025ml) in dimethylformamide (5ml) was stirred at room temperature over night. The solvent was evaporated, and to the residue was added ethyl acetate. Precipitated crude crystal was filtered, which were recrystallized from ethanol-ethyl acetate to give dimethyl(N-(7-(4-piperidinophenyl)-2,3-dihydro-1-benzox epin-4-carbonyl)-4-aminobenzyl)-4-tetrahydropyranylammo nium iodide (Compound 188) (0.1g) as yellow crystals.
mp 189-190°C.
¹H-NMR (δ ppm, DMSO-d₆) : 1.50-1.70 (6H, m), 1.75-2.00 (2H, m), 2.05-2.25 (2H, m), 2.88 (6H, s), 2.99 (2H, br), 3.16-3.19 (4H, m), 3.26-3.33 (2H, m), 3.50-1.70 (1H, m), 4.01-4.15 (2H, m), 4.29 (2H, br), 4.47 (2H, s), 7.00 (2H, d, J=8.8Hz), 7.03 (1H, d, J=8.4Hz), 7.35 (1H, s), 7.50-7.57 (5H, m), 7.68 (1H, d, J=2.6Hz), 7.86 (2H, d, J=8.4Hz), 10.19 (1H, s).
IR (KBr) ν : 2936, 1659cm⁻¹.

| Anal. for C₃₆H₄₄IN₃O₃·H₂O: | | | |
|---|---|---|---|
| Calcd. | C,60.76; | H,6.51; | N,5.90. |
| Found | C,60.57; | H,6.60; | N,5.85. |

### Working Example 189 (Production of Compound 189)

To a suspension of 7-(4-methylphenyl)-2,3-dihydro-1-benzoxepine-4-carboxyl ic acid (0.3g) in dichloromethane (10ml) were added oxalyl chloride (0.28ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 4-(N-methyl-N-(tetrahydrothiopyran-4-yl)-aminomethyl)an iline (0.26g) and triethylamine (0.5ml) in tetrahydrofuran (20ml), under ice-cooling. Under nitrogen atmosphere, the mixture was stirred at room temperature for 7 hours. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give crude crystals, which were recrystallized from ethyl acetate-hexane to give N-(4-((N-tetrahydrothiopyran-4-yl-N-methyl)amino-methyl )-phenyl)-7-(4-methylphenyl)-2,3-dihydro-1-benzoxepine-4-carboxamide (Compound 189) (0.47g) as colorless crystals.
mp 180-181°C.
¹H-NMR (δ ppm, CDCl₃): 1.60-1.85 (2H, m), 2.10-2.15 (2H, m), 2.21 (3H, s), 2.39 (3H, s), 2.40-2.50 (1H, m), 2.66-2.72 (4H, m), 3.08 (2H, t, J=4.6Hz), 3.57 (2H, s), 4.36 (2H, t, J=4.6Hz), 7.06 (1H, d, J=8.4Hz), 7.24 (2H, d, J=8.0Hz), 7.31 (2H, d, J=8.4Hz), 7.43-7.57 (7H, m).
IR(KBr) ν : 2934, 1653cm⁻¹.

| Anal. for C₃₁H₃₄N₂O₂S: | | | |
|---|---|---|---|
| Calcd. | C,74.66; | H,6.87; | N,5.62. |
| Found | C,74.46; | H,6.72; | N,5.42. |

### Working Example 190 (Production of Compound 190)

A solution of N-(4-((N-tetrahydrothiopyran-4-yl-N-methyl)aminomethyl) phenyl)-7-(4-methylphenyl)-2,3-dihydro-1-benzoxepine-4-carboxamide (0.11g) and methyl iodide (0.025ml) in dimethylformamide (5ml) was stirred at room temperature over night. The solvent was evaporated, and the residue was purified with silica gel column (chloroform/methanol) to give dimethyl-(N-(7-(4-methyl-phenyl)-2,3-dihydro-1-benzoxep in-4-carbonyl)-4-amino-benzyl)-4-tetrahydrothiopyranyla mmonium iodide (Compound 190) (0.09g) as colorless crystals. mp 185-186°C (dec.) .
¹H-NMR (δ ppm, DMSO-d₆) : 1.75-2.00 (2H, m), 2.34 (3H, s), 2.55-2.75 (4H, m), 2.75-2.85 (2H, m), 2.90 (6H, s), 3.00 (2H, br), 3.14-3.25 (1H, m), 4.31 (2H, br), 4.47 (2H, s), 7.07 (1H, d, J=8.4Hz), 7.27 (2H, d, J=7.8Hz), 7.36 (1H, s), 7.50-7.59 (5H, m), 7.74 (1H, d, J=2.2Hz), 7.86 (2H, d, J=8.8Hz), 10.19 (1H, s).
IR (KBr) ν : 2901, 1659cm⁻¹.

| Anal. for C₃₂H₃₇N₂O₂SI·H₂O : | | | |
|---|---|---|---|
| Calcd. | C,58.36; | H,5.97; | N,4.25. |
| Found | C,58.62; | H,6.04; | N,4.29. |

### Working Example 191 (Production of Compound 191)

To a solution of 2-(4-piperidinophenyl)-6,7-dihydro-5H-benzocycloheptene-8-carboxylic acid (0.45g), 4-(N-methyl-N-(tetrahydropyran-4-yl)aminomethyl)aniline (0.31g) and 1-hydroxybenzotriazole (0.18g) in dimethyl-formamide (20ml) was added 1-ethyl-3-(3-dimethylamino-propyl)carbodiimide hydro-chloride (0.37g) under ice-cooling. Under nitrogen atmosphere, the mixture was warmed to room temperature. To the mixture were added 4-dimethylaminopyridine (catalytic amount) and triethylamine (0.54ml), and the mixture was stirred over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give crude crystals, which were recrystallized from ethyl acetate-hexane to give 2-(4-piperidinophenyl)-N-(4-((N-tetrahydropyran-4-yl-N-methylamino)methyl)phenyl)-6,7-dihydro-5H-benzocyclo-he pten-8-carboxamide (Compound 191) (0.44g) as pale orange crystals.
mp 170-171°C.
¹H-NMR(δ ppm, CDCl₃): 1.59-1.65 (2H, m), 1.65-1.80 (8H, m), 2.05-2.21 (2H, m), 2.21 (3H, s), 2.55-2.68 (1H, m), 2.71 (2H, t, J=6.3Hz), 2.84-2.90 (2H, m), 3.19-3.24 (4H, m), 3.37 (2H, dt, J=2.8, 11.2Hz), 4.01-4.11 (2H, m), 7.00 (2H, d, J=8.8Hz), 7.20 (1H, d, J=7.6Hz), 7.31 (2H, d, J=8.4Hz), 7.41-7.51 (4H, m), 7.56 (2H, d, J=8.4Hz), 7.63 (1H, s).
IR(KBr) ν : 2936, 1661cm⁻¹.

| Anal. for C₃₆H₄₃N₃O₂·0.2H₂O : | | | |
|---|---|---|---|
| Calcd. | C,78.14; | H,7.91; | N,7.59. |
| Found | C,78.09; | H,7.93; | N,7.55. |

### Working Example 192 (Production of Compound 192)

A solution of 2-(4-piperidinophenyl)-N-(4-((N-tetrahydropyran-4-yl-N-methylamino)methyl)phenyl)-6,7-dihydro-5H-benzocyclohep tene-8-carboxamide (0.2g) and methyl iodide (0.025ml) in dimethylformamide (10ml) was stirred at room temperature over night. The solvent was evaporated, and the residue was purified with silica gel column (chloroform/methanol) to give crude crystals, which were recrystallized from ethanol-hexane to give dimethyl-(N-(2-(4-piperidinophenyl)-6,7-dihydro-5H-benzocyclo-he ptene-8-carbonyl)-4-aminobenzyl)-4-tetrahydropyranyl-am monium iodide (Compound 192) (0.15g) as pale brown crystals. mp 177-178 °C.
¹H-NMR (δ ppm, DMSO-d₆) : 1.50-1.70 (6H, m), 1.80-1.95 (2H, m), 2.00-2.10 (2H, m), 2.10-2.20 (2H, m), 2.60-2.70 (2H, m), 2.75-2.87 (2H, m), 2.88 (6H, s), 3.14-3.24 (6H, m), 3.53-3.65 (1H, m), 4.00-4.15 (2H, m), 4.46 (2H, s), 7.00 (2H, d, J=8.8Hz), 7.26 (1H, d, J=8.0Hz), 7.36 (1H, s), 7.46-7.62 (6H, m), 7.87 (2H, d, J=8.8Hz), 10.22 (1H, s). IR(KBr) ν : 2934, 1655cm⁻¹.

| Anal. for C₃₇H₄₆IN₃O₂·H₂O: | | | |
|---|---|---|---|
| Calcd. | C, 62.62; | H, 6.82; | N,5.92. |
| Found | C,62.32; | H,6.71; | N,5.92. |

### Working Example 193 (Production of Compound 193)

Under nitrogen atmosphere, oxalyl chloride (0.05ml) was added to a solution of 7-(4-methylthiophenyl)-2,3-dihydro-1-benzoxepine-4-carb oxylic acid (80.6mg) in tetrahydrofuran (10ml) at room temperature. To the mixture was added a drop of DMF, and the mixture was stirred for 1 hour. Under reduced pressure, the solvent was evaporated. The residue was dissolved in tetrahydrofuran (20ml). To the solution were added triethylamine (0.1ml) and 4-[N-methyl-N-(tetrahydropyran-4-yl)aminomethyl]aniline (62.5mg) at 0°C, and the mixture was stirred at room temperature for 3 hours. The reaction mixture was added to vigorously stirred water to stop the reaction. The mixture was extracted with ethyl acetate. The organic layer was washed with saturated sodium chloride solution, dried with magnesium sulfate and concentrated. The residue was purified with column chromatography (ethanol/ethyl acetate=1:4) and recrystallized from ethanol to give N- [4- [N-methyl-N- (tetrahydropyran-4-yl) aminomethyl]-phenyl]-7-(4-methylthiophenyl)-2,3-dihydro-1-benzoxepine-4 -carboxamide (Compound 193) (85mg) as colorless crystals. m.p. 180-186°C
¹H-NMR (200MHz, CDCl₃) δ 1.53-1.81 (4H, m), 2.21 (3H, s), 2.52 (3H, s), 2.54-2.73 (1H, m), 3.08 (2H, t, J=4.6 Hz), 3.31-3.43 (2H, m), 3.57 (2H, s), 3.98-4.10 (2H, m), 4.36 (2H, t, J=4.6 Hz), 7.06 (1H, d, J=8.4 Hz), 7.23-7.36 (4H, m), 7.41-7.63 (8H, m).
IR (KBr) 3319, 2947, 1645, 1516, 1485, 1315, 1248, 1140, 1086, 812 cm⁻¹

| Elemental Analysis for C₃₁H₃₄N₂O₃S · 0.2H₂O | | | | |
|---|---|---|---|---|
| Calcd. | C, 71.84 ; | H, 6.69 ; | N, 5.40 ; | S, 6.19 : |
| Found. | C, 71.75 ; | H, 6.70 ; | N, 5.38 ; | S, 6.24. |

### Reference Example 49

To 3-bromocinnamic acid (2.0g) were added thionyl chloride (25ml) and dimethylformamide (catalytic amount), and the mixture was refluxed for 1.5 hours. The solvent was evaporated, and the residue was dissolved in tetrahydrofuran. The mixture was dropwise added to a suspension of 1-(4-aminobenzyl)piperidine (1.7g) and diisopropylethylamine (4ml) in tetrahydrofuran (5ml) under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give 1-(4-(3-bromocinnamoylamino)-benzyl)piperidine (1.8g) as colorless crystals.
mp 144-145°C.
¹H-NMR(δ ppm, CDCl₃) : 1.37-1.49 (2H, m), 1.52-1.63 (4H, m), 2.34-2.39 (4H, m), 3.45 (2H, s), 6.54 (1H, d, J=15.5Hz), 7.21-7.33 (3H, m), 7.41-7.57 (5H, m), 7.67 (1H, d, J=15.5Hz), 7.69 (1H, s).
IR (KBr) ν : 3270, 2934, 1663cm⁻¹.

| Anal. for C₂₁H₂₃BrN₂O · 0.2H₂O: | | | |
|---|---|---|---|
| Calcd. | C,62.60; | H,5.85; | N,6.95. |
| Found | C,62.67; | H,5.79; | N,6.93. |

### Reference Example 50

To 3-phenylcinnamic acid (0.24g) were added thionyl chloride (10ml) and dimethylformamide (catalytic amount), and the mixture was refluxed for 2 hours. The solvent was evaporated, and the residue was dissolved in tetrahydrofuran. The mixture was dropwise added to a suspension of 2-(4-aminobenzyl)-1,3,2-dioxaphosphorinane-2-oxide (0.2g) and diisopropylethylamine (0.8ml) in tetrahydro-furan(20ml), under ice-cooling. Undernitrogen atmosphere, the mixture was stirred at room temperature over night. The solvent was evaporated, and to the residue wasaddedwater. The mixture was extracted with ethyl acetate. The organic layer was washed with water and saturated sodium chloride solution, anddriedwithanhydrousmagnesiumsulfate. Under reduced pressure, the solvent was evaporated, and precipitated crude crystal was recrystallized from ethanol-hexane to give 2-(4-(3-phenylcinnamoylamino)-benzyl)-1,3,2-dioxaphosph orinane-2-oxide (0.32g) as colorless crystals.
mp 204-205°C.
¹H-NMR(δ ppm, CDCl₃): 1.84-1.88 (2H, m), 3.24 (2H, d, J=21.2Hz), 4.07-4.22 (2H, m), 4.34-4.44 (2H, m), 6.74 (1H, d, J=15.8Hz), 7.23 (2H, dd, J=2.6, 8.8Hz), 7.38-7.63 (10H, m), 7.77 (1H, s), 7.81 (1H, d, J=15.8Hz), 8.16 (1H, br).
IR (KBr) ν : 3059, 1680cm⁻¹.

| Anal. for C₂₅H₂₄NO₄P: | | | |
|---|---|---|---|
| Calcd. | C,69.28; | H,5.58; | N,3.23. |
| Found | C,68.82; | H,5.58; | N,3.30. |

### Reference Example 51

To a suspension of 7-(4-methylphenyl)-2,3-dihydro-1-benzoxepine-4-carboxyl ic acid (0.15g) in dichloro-methane (7ml) were added oxalyl chloride (0.14ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of 2-(4-aminobenzyl)-1,3,2-dioxaphosphorinane-2-oxide (0.13g) and triethylamine (0.23ml) in tetrahydrofuran (20ml), underice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-ethanol-hexane to give 2-(4-(7-(4-methylphenyl)-2,3-dihydro-1-benzoxepin-4-carbonylamino)benzyl)-1,3,2-dioxaphosphorinane-2-oxide (0.23g) as colorless crystals.
mp 268-269°C.
¹H-NMR (δ ppm, CDCl₃) : 1.75-1.87 (2H, m), 2.40 (3H, s), 3.09 (2H, t, J=4.5Hz), 3.24 (2H, d, J=21.6Hz), 4.02-4.19 (2H, m), 4.34-4.50 (4H, m), 7.06 (1H, d, J=8.4Hz), 7.23-7.32 (4H, m), 7.44-7.60 (6H, m), 7.81 (1H, s).
IR (KBr) ν : 1652cm⁻¹.

| Anal. for C₂₈H₂₈NO₅P: | | | |
|---|---|---|---|
| Calcd. | C,68.70; | H,5.77; | N,2.86. |
| Found | C,68.54; | H,5.71; | N,2.86. |

### Reference Example 52

A suspension of N-(4-chloromethylphenyl)-7-(4-methylphenyl)-2,3-dihydro -1-benzoxepine-4-carboxamide (0.18g), 1-t-butoxycarbonyl-4-methylaminopiperidine (0.19g) and potassium carbonate (0.18g) in dimethylformamide (10ml) was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-((N-(1-t-butoxy-carbonylpiperidin-4-yl)-N-methyl)a minomethyl)phenyl)-7-(4-methylphenyl)-2,3-dihydro-1-ben zoxepine-4-carboxamide (0.25g) as colorless crystals.
mp 203-204°C.
¹H-NMR (δ ppm, CDCl₃) : 1.37-1.70 (4H, m), 1.46 (9H, s), 1.77-1.83 (2H, m), 2.19 (3H, s), 2.39 (3H, s), 2.52-2.74 (3H, m), 3.08 (2H, t, J=4.6Hz), 3.56 (2H, s), 4.18 (1H, br), 4.36 (2H, t, J=4.6Hz), 7.06 (1H, d, J=8.4Hz), 7.22-7.33 (5H, m), 7.43-7.61 (6H, m).
IR(KBr) ν: 2977, 2933, 1695, 1668cm⁻¹.

| Anal. for C₃₆H₄₃N₃O₄ : | | | |
|---|---|---|---|
| Calcd. | C,74.33 ; | H,7.45; | N,7.22. |
| Found | C,74.00 ; | H,7.41; | N,7.26. |

### Reference Example 53

To a suspension of 7-(4-methylphenyl)-2,3-dihydro-1-benzoxepine-4-carboxyl ic acid (0.6g) in dichloromethane (25ml) were added oxalyl chloride (0.56ml) and dimethylformamide (catalytic amount) under ice-cooling, and the mixture was stirred at room temperature for 2 hours. The solvent was evaporated, and the residue was dissolved in tetrahydrofuran. The mixture was dropwise added to a solution of (4-aminophenyl)[1-(tert-butoxycarbonyl)-piperidin-2-yl] methanone (0.72g) and triethylamine (0.9ml) in tetrahydrofuran (50ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give crude crystals, which were recrystallized from ethyl acetate-hexane to give N-(4-(1-(tert-butoxycarbonyl)piperidin-2-ylcarbonyl)-ph enyl)-7-(4-methylphenyl)-2,3-dihydro-1-benzoxepine-4-ca rboxamide (1.1g) as pale yellow crystals.
mp 223-224°C.
¹H-NMR(δ ppm, CDCl₃): 1.44 (9H, br), 1.44-1.65 (4H, m), 1.70-1.95 (1H, m), 2.00-2.20 (1H, m), 2.39 (3H, s), 3.08 (2H, t, J=4.4Hz), 5.60 (1H, br), 7.06 (1H, d, J=8.4Hz), 7.25 (2H, d, J=11.8Hz), 7.44-7.53 (4H, m), 7.65 (1H, br), 7.69 (1H, br), 7.82 (1H, br), 7.94 (2H, d, J=8.8Hz).
IR (KBr) ν : 2942, 1678cm⁻¹.

| Anal. for C₃₅H₃₈N₂O₅·0.3H₂O : | | | |
|---|---|---|---|
| Calcd. | C,73.48 ; | H,6.80; | N,4.90. |
| Found | C,73.51; | H,6.60; | N,4.68. |

### Reference Example 54

To a mixture of 3-bromobenzaldehyde (10g) and methoxy-carbonylmethylenetriphenylphosphine (20g) was added toluene (150ml), and the mixture was refluxed under nitrogen atmosphere for 2 hours. The solvent was evaporated, and the organic layer was washed with water and saturated sodium chloride solution, and dried with anhydrous magnesium sulfate. Under reduced pressure, the solvent was evaporated, and the residue was purified with silica gel column (ethyl acetate/hexane) to give methyl 3-bromo-cinnamate (10.7g) as colorless crystals.
¹H-NMR (δppm, CDCl₃) : 3.82 (3H, s), 6.44 (1H, d, J=16.0Hz), 7.27 (1H, d, J=15.6Hz), 7.43-7.54 (2H, m), 7.62 (1H, d, J=16.0Hz), 7.66-7.68 (1H, m).
IR(KBr) ν : 1734, 1717cm⁻¹.

| Anal. for C₁₀H₉BrO₂: | | |
|---|---|---|
| Calcd. | C,49.82; | H,3.76. |
| Found | C,49.90; | H,3.90. |

### Reference Example 55

In a solutionof methanol (200ml) and 2N sodium hydroxide (50ml) was dissolved methyl 3-bromocinnamate (10.7g), and the mixture was stirred at room temperature over night, concentrated and neutralized with 1N hydrochloric acid. The mixture was extracted with ethyl acetate. The organic layer was washed with water and saturated sodium chloride solution, and dried with anhydrous magnesium sulfate. Under reduced pressure, the solvent was evaporated to give 3-bromophenylcinnamic acid (9.2g) as colorless crystals. ¹H-NMR (δppm, CDCl₃) : 6.45 (1H, d, J=15. 8Hz), 7.28 (1H, t, J=7.7Hz), 7.45-7.56 (2H, m), 7.67-7.75 (2H, m).
IR (KBr) ν : 1688cm⁻¹.

| Anal. for C₉H₇BrO₂: | | |
|---|---|---|
| Calcd. | C,47.61; | H,3.11. |
| Found | C,47.57; | H,3.10. |

### Reference Example 56

A suspension of methyl 3-bromocinnamate (3.8g), phenyl borate (2.0g), 1M potassium carbonate (20ml) and ethanol (10ml) in toluene (100ml) was stirred under argon atmosphere at room temperature for 30 minutes. To the reaction mixture was added tetrakistriphenyl-phosphinepalladium (0.9g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (3.6g), 1.8g of which was dissolved in a solution of methanol (100ml) and 1N sodium hydroxide (20ml). The mixture was stirred at room temperature over night, concentrated, neutralized with 1N hydrochloric acid and extracted with ethyl acetate. The organic layer was washed with water and saturated sodium chloride solution, and dried with anhydrous magnesium sulfate. Under reduced pressure, the solvent was evaporated to give 3-phenylcinnamic acid (1.5g) as colorless crystals. ¹H-NMR (δ ppm, CDCl₃): 6.54 (1H, d, J=16. 0Hz), 7.39-7.67 (8H, m), 7.76-7.77 (1H,m), 7.87 (1H,d,J=16.0Hz).
IR (KBr) ν 1709cm⁻¹.

| Anal. for C₁₅H₁₂O₂: | | |
|---|---|---|
| Calcd. | C,80.34; | H,5.39. |
| Found | C,80.62; | H,5.40. |

### Reference Example 57

To 4-nitrobenzylphosphonic acid (0.5g) were added thionyl chloride (5ml) and dimethylformamide (catalytic amount), and the mixture was refluxed under nitrogen atmosphere for 4 hours. The solvent was evaporated, and to the residue was added toluene. The solvent was evaporated. The residue was dissolved in tetrahydrofuran (15ml), and the mixture was cooled to -78°C under nitrogen atmosphere. To the mixture was dropwise added dimethylpropanediamine (0.3ml) dissolved in tetrahydrofuran (2ml) and then triethylamine (1.6ml), and the mixture was gradually warmed to room temperature and stirred at room temperature over night. The solvent was evaporated, and the residue was purified with silica gel column (ethyl acetate/methanol/ triethylamine) to give colorless crystals, which were dissolved in ethanol (15ml). To the mixture was added 10% palladium on carbon (0.04g), and catalytic hydrogenation was carried out at room temperature for 3.5 hours. The catalyst was filtered off, and the solvent was evaporated to give 2-(4-aminobenzyl)-1,3-dimethyl-1,3,2-diaza-phosphorinan e-2-oxide (0.3g) as colorless crystals.
¹H-NMR (δ ppm, CDCl₃) : 1.09-1.27 (1H, m), 1.68-1.85 (1H, m), 2.65 (3H, s), 2.69 (3H, s), 2.72-3.01 (4H, m), 3.08 (2H, d, J=17.4Hz), 6.65 (2H, d, J=8.1Hz), 6.96 (2H, dd, J=2.4, 8.1Hz).
IR(KBr) ν : 3339, 2897, 1615cm⁻¹.

| Anal. for C₁₂H₂₀N₃OP·0.3H₂O : | | | |
|---|---|---|---|
| Calcd. | C,55.72; | H,8.03; | N,16.24. |
| Found | C,55.69; | H, 7.98 ; | N,16.13. |

### Reference Example 58

To 4-nitrobenzylphosphonic acid (0.5g) were added thionyl chloride (5ml) and dimethylformamide (catalytic amount), and the mixture was refluxed for 3 hours under nitrogen atmosphere. The solvent was evaporated, and to the residue was added toluene. The solvent was evaporated. The residue was dissolved in tetrahydrofuran (5ml), and the mixture was cooled to -78°C under nitrogen atmosphere. To the mixture was dropwise added dimethylethylenediamine (0.25ml) dissolved in tetrahydrofuran (2ml), and then triethylamine (1.5ml), and the mixture was gradually warmed to room temperature and stirred at room temperature over night. The solvent was evaporated, and the residue was purified with silica gel column (ethyl acetate/methanol/triethylamine) to give colorless crystals, which were dissolved in ethanol (15ml). To the mixture was added 10% palladium on carbon (0.05g), and catalytic hydrogenation was carried out at room temperature for 3 hours. The catalyst was filtered off, and the solvent was evaporated to give 2-(4-aminobenzyl)-1,3-dimethyl-1,3,2-diaza-phosphorane-2-oxide (0.3g) as yellow crystals.
¹H-NMR (δ ppm, CDCl₃) : 2.61 (3H, s), 2.63-2.71 (2H, m), 2. 66 (3H, s), 3.00-3.07 (2H, m), 3.13 (2H, d, J=18.2Hz), 6.63 (2H, d, J=8.5Hz), 6.97 (2H, dd, J=2.4, 8.5Hz).
IR (KBr) ν: 3341, 2895, 1632cm⁻¹.

| Anal. for C₁₁H₁₈N₃OP·0.5H₂O: | | | |
|---|---|---|---|
| Calcd. | C,53.22; | H, 7.71; | N,16.93. |
| Found | C,53.23; | H,7.53; | N,16.83. |

### Reference Example 59

A suspension of 3-bromo-6,7,8,9-tetrahydro-5H-benzocycloheptan-5-one (4.6g; L. A. M. Cornelius and D. W. Combs, Synth. Commun. (1994), 24(19), 2777-2788), 4-methylphenyl borate (3.8g), 2M potassium carbonate (30ml) and ethanol(30ml) in toluene(100ml) was stirred under argon atmosphere at room temperature for 30 minutes. To the reaction mixture was added tetrakistriphenylphosphine-palladium (1.5g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale brown oil (5.7g), to which were added sodium methoxide (6.2g) and dimethyl carbonate (100ml). The mixture was refluxed under nitrogen atmosphere for 8 hours and poured into 1N hydrochloric acid under ice-cooling. The mixture was extracted with ethyl acetate. The organic layer was washed with water and saturated sodium chloride solution, and dried with anhydrous magnesium sulfate. The solvent was evaporated, and the residue was purified with silica gel column (ethyl acetate/hexane) to give brown oil (5.5g), which was dissolved in dichloromethane (20ml). To the mixture was dropwise added sodium boron hydride dissolved in methanol, under ice-cooling. After starting materials disappeared, water was added to the reaction mixture, and the mixture was concentrated and extracted with ethyl acetate. The organic layer was washed with water and saturated sodium chloride solution, and driedwith anhydrous magnesium sul fate. The solvent was evaporated, and to the residue were added 1N sodium hydroxide (40ml), methanol (40ml) and diethylether (100ml). The mixture was heated to 50°C for 30 minutes and concentrated. To the residue was added 1N sodium hydroxide, and the mixture was extracted with water, washed with ethyl acetate and acidified with hydrochloric acid. The mixture was extracted with ethyl acetate. The organic layer was washed with water and saturated sodium chloride solution, and dried with anhydrous magnesium sulfate. The solvent was evaporated, and the residue was dissolved in Diglyme (20ml). To the mixture was added hydrochloric acid (5ml), and the mixture was heated to 100°C for 6 hours and poured into water. The mixture was extracted with ethyl acetate. The organic layer was washed with water and saturated sodium chloride solution, and dried with anhydrous magnesium sulfate. The solvent was evaporated to give 2-(4-methylphenyl)-6,7-dihydro-5H-benzocycloheptene-8-c arboxylic acid (0.3g) as colorless crystals.
¹H-NMR(δppm, CDCl₃) : 2.07-2.16 (2H, m), 2.40 (3H, s), 2.70 (2H, t, J=6.6Hz), 2.86-2.91 (2H, m), 7.21-7.28 (3H, m), 7.44-7.56 (4H, m), 7.91 (1H, s).
IR(KBr) ν : 2930, 1678 cm⁻¹.

| Anal. for C₁₉H₁₈O₂: | | |
|---|---|---|
| Calcd. | C,81.99; | H,6.52. |
| Found | C,81.64; | H,6.41. |

### Reference Example 60

In dimethylformamide (100ml) was added 4-bromo-thiophenol (25g) . To the solution were added ethyl 4-bromobutyrate (30g) and potassium carbonate (36g), and the mixture was stirred at room temperature over night. The solvent was evaporated, and to the residue was added water. The mixture was extracted with ethyl acetate. The organic layer was washed with and saturated sodium chloride solution, and dried with anhydrous magnesium sulfate. The solvent was evaporated, and to the residue were added 1N sodium hydroxide (240ml) and methanol (120ml). The mixture was stirred at room temperature over night and concentrated. The residue was dissolved in water, and the mixture was washed with ethyl acetate. The aqueous layer was acidified with hydrochloric acid under ice-cooling. The mixture was extracted with ethyl acetate. The organic layer was washed with and saturated sodium chloride solution, and dried with anhydrous magnesium sulfate. The solvent was evaporated to give colorless crystals (32g), to which was added polyphosphoric acid (250g), and the mixture was stirred at 100°C for 1 hour and poured into ice-water. The mixture was extracted with ethyl acetate. The organic layer was washed with water, sodium hydrogen carbonate solution, water and saturated sodium chloride solution, and dried with anhydrous magnesium sulfate. The solvent was evaporated to give brown crystals (13.6g), to which were added sodium methoxide (14.2g) and dimethyl carbonate (200ml), and the mixture was refluxed under nitrogen atmosphere for 8 hours. Under ice-cooling, the mixture was poured into 1N hydrochloric acid. The mixture was extracted with ethyl acetate. The organic layer was washed with and saturated sodium chloride solution, and dried with anhydrous magnesium sulfate. the solvent was evaporated to give brown crystals (11.5g), which were dissolved in dichloromethane (100ml). To the mixture was dropwise added sodium boron hydride dissolved in methanol, under ice-cooling. After starting materials disappeared, water was added to the reaction mixture, and the mixture was concentrated and extracted with ethyl acetate. The organic layer was washed with and saturated sodium chloride solution, and dried with anhydrous magnesium sulfate. The solvent was evaporated, and to the residue were added 1N sodium hydroxide (100ml), methanol (100ml) and diethylether (500ml). The mixture was stirred at room temperature for 1.5 hours and concentrated. To the residue was added 1N sodium hydroxide, and the mixture was extracted with water, washed with diethylether and acidified with hydrochloric acid. The mixture was extracted with ethyl acetate. The organic layer was washed with and saturated sodium chloride solution, and dried with anhydrous magnesium sulfate. The solvent was evaporated, and the residue was dissolved in Diglyme (100ml). To the mixture was added hydrochloric acid (20ml), and the mixture was heated to 110°C for 2.5 hours and poured into water. The mixture was extracted with ethyl acetate. The organic layer was washed with and saturated sodium chloride solution, and dried with anhydrous magnesium sulfate. The solvent was evaporated to give colorless crystal (1.1g), 1g of which was suspended dichloromethane (15ml). To the suspension were added oxalyl chloride (1ml) and dimethylformamide (catalytic amount) under ice-cooling, and the mixture was stirred at room temperature for 2.5 hours. The solvent was evaporated, and the residue was dissolved in tetrahydrofuran. The mixture was dropwise added to a solution of 4-(tert-butyldimethylsilyloxy)aniline (0.76g) and triethylamine (1.6ml) in tetrahydrofuran (20ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give brown oil (1.8g), to which were added 4-methylphenyl borate (0.5g), 1M potassium carbonate (15ml), ethanol (15ml) and toluene(500ml), and the mixture was stirred under argon atmosphere at room temperature for 30 minutes. To the mixture was added tetrakistriphenyl-phosphinepalladium (0.2g), and the mixture was refluxed over night. The mixture was extracted with ethyl acetate, and the organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (1.3g), which were dissolved in ethyl acetate (50ml). To the mixture was added hydrochloric acid (5ml), and the mixture was stirred at room temperature for 1. 5 hours, washed with sodium hydrogen carbonate solution, water, saturated sodium chloride solution, and dried with anhydrous magnesium sulfate. Under reduced pressure, the solvent was evaporated to give 7-(4-methylphenyl)-N-(4-hydroxy-methylphenyl)-2,3-dihyd ro-1-benzothiepine-4-carboxamide (1.0g) as colorless crystals.
¹H-NMR(δ ppm, CDCl₃): 2.40 (3H, s), 3.08 (2H, t, J=5.8Hz), 3.29 (2H, t, J=5.8Hz), 4.69 (2H, s), 7.24-7.28 (2H, m), 7.35-7.62 (10H, m), 7.71 (1H, br).
IR(KBr) ν: 3314, 2928, 1649cm⁻¹.

| Anal. for C₂₅H₂₃NO₂S·0.2H₂O : | | | |
|---|---|---|---|
| Calcd. | C,74.12; | H,5.82; | N,3.46. |
| Found | C,74.10; | H,5.65; | N,3.47. |

### Reference Example 61

In dimethylformamide (100ml) was dissolved 4-bromophenol (17.3g). To the solution were added ethyl 4-bromo-butyrate (21.2g) and potassium carbonate (25g), and the mixture was stirred at room temperature over night. The solvent was evaporated, and to the residue was added water. The mixture was extracted with ethyl acetate. The organic layer was washed with and saturated sodium chloride solution, and dried with anhydrous magnesium sulfate. The solvent was evaporated, and to the residue were added 3N sodium hydroxide (100ml) and methanol (60ml). The mixture was stirred at 70°C for 30 minutes and concentrated. The residue was dissolved in water, and the mixture was washed with diethylether. The aqueous layer was acidified with hydrochloric acid under ice-cooling, and the mixture was extracted with ethyl acetate. The organic layer was washed with and saturated sodium chloride solution, and dried with anhydrous magnesium sulfate. The solvent was evaporated to give colorless crystal (23. 9g), to 10g of which was added polyphosphoric acid (120g). The mixture was stirred at 100°C for 45 minutes and poured into ice-water. The mixture was extracted with ethyl acetate. The organic layer was washed with water, sodium hydrogen carbonate solution, water and saturated sodium chloride solution, and dried with anhydrous magnesium sulfate. The solvent was evaporated, and the residue was purified with silica gel column (ethyl acetate/hexane) to give 7-bromo-2,3,4,5-tetrahydrobenzoxepin-5-one as yellow oil (6.5g).
¹H-NMR (δ ppm, CDCl₃): 2.15-2.29 (2H, m), 2.89 (2H, t, J=7.0Hz), 4.24 (2H, t, J=6.6Hz), 6.97 (1H, d, J=8.8Hz), 7.50 (1H, dd, J=2.6, 8.1Hz), 7.87 (1H, d, J=2.6Hz).
IR(neat) ν: 2969, 1686cm⁻¹.

### Reference Example 62

To 7-bromo-2,3,4,5-tetrahydrobenzoxepin-5-one (6.5g) were added 4-methylphenyl borate (4.1g), 2M potassium carbonate (30ml), ethanol (30ml) and toluene (100ml), and the mixture was stirred under argon atmosphere at room temperature for 30 minutes. To the mixture was added tetrakistriphenylphosphinepalladium (1.3g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow crystal (5.7g), to 3.6g of which was added sodium methoxide (3.9g) and dimethyl carbonate (50ml). Under nitrogen atmosphere, the mixture was refluxed for 8 hours and poured into 1N hydrochloric acid under ice-cooling. The mixture was extracted with ethyl acetate. The organic layer was washed with and saturated sodium chloride solution, and dried with anhydrous magnesium sulfate, and the solvent was evaporated. The residue was purified with silica gel column (ethyl acetate/hexane) to give colorless crystal (3.5g), 1.8g of which was dissolved in dichloromethane (25ml). To the mixture was dropwise added sodium boron hydride dissolved in methanol, under ice-cooling. After starting materials disappeared, water was added to the reaction mixture, and the mixture was concentrated and extracted with ethyl acetate. The organic layer was washed with and saturated sodium chloride solution, and dried with anhydrous magnesium sulfate, and the solvent was evaporated. To the residue were added 1N sodium hydroxide (50ml), methanol (25ml) and diethylether (25ml), and the mixture was stirred at room temperature for 30 minutes and concentrated. To the mixture was added 1N sodium hydroxide, and the mixture was extracted with water, washed with diethylether and acidified with hydrochloric acid. The mixture was extracted with ethyl acetate. The organic layer was washed with and saturated sodium chloride solution, and dried with anhydrous magnesium sulfate. The solvent was evaporated, and the residue was dissolved in Diglyme (25ml). To the mixture was added hydrochloric acid (5ml), and the mixture was heated at 100°C for 40minutes and poured into water. The mixture was extracted with ethyl acetate. The organic layer was washed with and saturated sodium chloride solution, and dried with anhydrous magnesium sulfate. The solvent was evaporated to give 7-(4-methylphenyl)-2,3-dihydro-1-benzoxepine-4-carboxyl ic acid (1.2g) as colorless crystals.
mp 255-256°C.
¹H-NMR(δ ppm, CDCl₃): 2.40 (3H, s), 3.02 (2H, t, J=4.6Hz), 4.33 (2H, t, J=4.6Hz), 7.05 (1H, d, J=8.6Hz), 7.24 (2H, d, J=8.2Hz), 7.46 (2H, d, J=8.2Hz), 7.47-7.56 (2H, m), 7.78 (1H, s).
IR (KBr) ν : 2996, 1694cm⁻¹.

| Anal. for C₁₈H₁₆O₃: | | |
|---|---|---|
| Calcd. | C,77.12; | H,5.75. |
| Found | C,76.91; | H,5.75. |

### Reference Example 63

In dichloromethane (10ml) was suspended 7-(4-methylphenyl)-2,3-dihydro-1-benzoxepine-4-carboxylic acid (1.0g) and to the suspension were added oxalyl chloride (1ml) and dimethylformamide (catalytic amount) under ice-cooling. The mixture was stirred at room temperature for 3 hours. The solvent was evaporated, and the residue was dissolved in tetrahydrofuran. The mixture was dropwise added to a solution of 4-(tert-butyldimethyl-silyloxy)aniline (0.93g) and triethylamine (1.5ml) in tetrahydrofuran (15ml), under ice-cooling. Under nitrogen atmosphere, the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless oil (1.88g), which was dissolved in ethyl acetate (20ml). To the mixture was added hydrochloric acid (5ml), and the mixture was stirred at room temperature 1.5 hours. The mixture was washed with sodium hydrogen carbonate solution, water and saturated sodium chloride solution, and dried with anhydrous magnesium sulfate. Under reduced pressure, the solvent was evaporated, and the residue was purified with silica gel column (ethyl acetate/hexane) to give colorless crystals (0.9g), which was suspended in dichloromethane (60ml). To the suspension were added lithium chloride (0.1g) and triethylamine (1ml). To the mixture was dropwise added methanesulfonylchloride (0.3ml) under ice-cooling, and the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give N-(4-chloromethylphenyl)-7-(4-methyl-phenyl)-2,3-dihydr o-1-benzoxepine-4-carboxamide (0.4g).
¹H-NMR (δppm, CDCl₃) : 2.39 (3H, s), 3.08 (2H, t, J=4.6Hz), 4.36 (2H, t, J=4.6Hz), 4.59 (2H, s), 7.06 (1H, d, J=8.4Hz), 7.22-7.26 (2H, m), 7.36-7.53 (6H, m), 7.60 (2H, d, J=8.4Hz), 7.65 (1H, s).
IR (KBr) ν : 3025, 1649 cm⁻¹.

### Reference Example 64

In tetrahydrofuran (50ml) were suspended p-nitro-phenethylbromide (2.3g) and sodium iodide (1.5g). To the suspension was added piperidine (4ml), and the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated to give yellow oil (2.3g), whichwasdissolvedinethanol (50ml). To the mixture was added 10% palladium on carbon (0.23g), and catalytic hydrogenation was carried out at room temperature over night. The catalyst was filtered off, and the solvent was evaporated to give 1-(2-(4-aminophenyl)ethyl)-piperidine (2.0g) as yellow oil.
¹H-NMR(δ ppm, CDCl₃) : 1.43-1.50 (2H, m), 1.56-1.67 (4H, m), 2.42-2.53 (6H, m), 2.67-2.75 (2H, m), 3.55 (2H, br), 6.62 (2H, d, J=8.4Hz), 6.99 (2H, d, J=8.4Hz).
IR(neat) ν : 2935, 1623cm⁻¹.

### Reference Example 65

To 5'-bromo-2'-hydroxyacetophenone (10g) were added 4-methylphenylborate (6.7g),2M potassium carbonate (70ml), ethanol (70ml) and toluene (200ml), and the mixture was stirred under argon atmosphere at room temperature for 30 minutes. To the mixture was added tetrakistriphenyl-phosphinepalladium (2.1g), and the mixture was refluxed over night. The mixture was extracted with ethyl acetate, and the organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow crystal (7.4g), 2. 3g of which was dissolved in pyridine (15ml). To the mixture was added benzoyl chloride (1.4ml), and the mixture was stirred at room temperature for 30 minutes. The solvent was evaporated, and to the residue was added water. The mixture was extracted with ethyl acetate, and the organic layer was washed with water and saturated sodium chloride solution, and dried with anhydrous magnesium sulfate. Under reduced pressure, the solvent was evaporated to give colorless crystals (3.0g), 2.9g of which was dissolved in pyridine (25ml). To the mixture was added potassium hydroxide (0.7g) little by little at 50°C. The mixture was stirred at 50°C for 1 hour, and the solvent was evaporated. To the residue was added 10% acetic acid under ice-cooling, and the mixture was extracted with ethyl acetate. The organic layer was washed with water and saturated sodium chloride solution, and dried with anhydrous magnesium sulfate. Under reduced pressure, the solvent was evaporated to give yellow crystal (2.3g), to which was added sulfuric acid (0.37ml) and acetic acid (15ml). The mixture was refluxed for 1 hour and poured into ice-water. The mixture was extracted with ethyl acetate. The organic layer was washed with water and saturated sodium chloride solution, and dried with anhydrous magnesium sulfate. Under reduced pressure, the solvent was evaporated to give colorless crystal (2.1g), which was dissolved in dimethylsulfoxide (150ml). To the mixture was dropwise added a solution which was prepared by adding a solution of trimethylsulfoxonium iodide (2.3g) in dimethylsulfoxide (60ml) dropwise to a suspension of sodium hydride (60%, 0.44g) in dimethylsulfoxide (10ml) and stirring the mixture under nitrogen atmosphere at room temperature for 40 minutes. The mixture was stirred at room temperature for 3 hours and further stirred at 50°C for 2 hours. The mixture was poured into water, and the mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow crystals (1.7g), to which were added tributyltin hydride (2.1ml), 2,2'-azobis(isobutyro-nitrile) (0.64g) and toluene (50ml) . The mixture was stirred under nitrogen atmosphere at 100°C for 1 hour, washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.65g), to which were added sodium methoxide (0.54g) and dimethyl carbonate (25ml). The mixture was refluxed under nitrogen atmosphere for 8 hours and poured into 1N hydrochloric acid under ice-cooling. The mixture was extracted with ethyl acetate. The organic layer was washed with and saturated sodium chloride solution, and dried with anhydrous magnesium sulfate. The solvent was evaporated to give pale brown oil (0.76g), which was dissolved in dichloromethane (50ml). To the mixture was dropwise added the solution of sodium boron hydride in methanol at -10°C. After starting materials disappeared, water was added to the reaction mixture, and the mixture was concentrated extracted with ethyl acetate. The organic layer was washed with and saturated sodium chloride solution, and dried with anhydrous magnesium sulfate, and the solvent was evaporated. To the residue were added 1N sodiumhydroxide (20ml) and methanol (200ml), and the mixture was stirred at room temperature for 3 hours, concentrated and acidified with hydrochloric acid. The mixture was extracted with ethyl acetate. The organic layer was washed with and saturated sodium chloride solution, and dried with anhydrous magnesium sulfate, and the solvent was evaporated. The residue was dissolved in Diglyme (50ml), and to the mixture was added hydrochloric acid (10ml) . The mixture was stirred at 100°C for 30 minutes and poured into water. The mixture was extracted with ethyl acetate. The organic layer was washed with and saturated sodium chloride solution, and dried with anhydrous magnesium sulfate. The solvent was evaporated to give 7-(4-methylphenyl)-2-phenyl-2,3-dihydro-1-benzoxepine-4 -carboxylic acid (0.4g) as colorless crystals.
mp 296-297°C.
¹H-NMR (δ ppm, CDCl₃) : 2.40 (3H, s), 3 . 10-3 . 39 (2H, m), 5.02 (1H, dd, J=1.8, 8.8Hz), 7.10 (1H, d, J=8.4Hz), 7.12-7.27 (2H, m), 7.35-7.53 (8H, m), 7.58 (1H, d, J=2.2Hz), 7.86 (1H, d, J=2.0Hz).
IR (KBr) ν : 1673cm⁻¹.

| Anal. for C₂₄H₂₀O₃·0.1H₂O : | | |
|---|---|---|
| Calcd. | C,80.47; | H,5.68. |
| Found | C,80.41; | H,5.73. |

### Reference Example 66

In 1,2-dichloroethane (100ml) were suspended p-nitrobenzylamine hydrochloride(7.5g),4H-tetrahydropyran-4-one (4.0g) and triethylamine (5.6ml), and to the suspension was added sodium triacetoxy boron hydride (11.8g) under ice-cooling. The mixture was stirred under nitrogen atmosphere at room temperature for 5 hours. To the mixture were added 37% formalin (3.6ml) and sodium triacetoxy boron hydride (11.8g) under ice-cooling, and the mixture was stirred under nitrogen atmosphere at room temperature for 4 hours. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated to give brown oil (10g), to which were added reduced iron (9g) and acetic acid (200ml). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated to give 4- (N-methyl-N- (tetrahydropyran-4-yl)aminomethyl)aniline (7.3g) as colorless crystals.
mp 93-94°C.
¹H-NMR (δ ppm, CDCl₃) : 1.65-1.76 (4H, m), 2.19 (3H, s), 2.58-2.68 (1H, m), 3.36 (2H, dt, J=3.2, 11.3Hz), 3.48 (2H, s), 3.60 (2H, br), 4.00-4.05 (2H, m), 6.65 (2H, d, J=8.4Hz), 7.09 (2H, d, J=8.4Hz).
IR(KBr) ν : 2952, 2844, 2788, 1613cm⁻¹.

| Anal. for C₁₃H₂₀N₂O·0.1H₂O: | | | |
|---|---|---|---|
| Calcd. | C,70.30; | H,9.17; | N,12.61. |
| Found | C,70.21; | H,8.85; | N,12.64. |

### Reference Example 67

In methanol (20ml) was dissolved ethyl levulinate (10g), and to the mixture was added sodium boron hydride (0.7g) at -78°C. The mixture was warmed to room temperature, and to the mixture was added ammonium chloride solution. The mixture was concentrated, extracted with diethylether, and dried with anhydrous magnesium sulfate. The solvent was evaporated to give colorless oil (9.3g), which was dissolved in tetrahydrofuran (50ml). To the mixture was added triethylamine (10. 6ml) under ice-cooling, and to the mixture was dropwise added methane-sulfonylchloride (4.9ml). The mixture was warmed to room temperature, and the solvent was evaporated. To the residue were added sodium iodide (11.4g) and acetone (50ml), and the mixture was stirred at 50°C for 2 hours. The solvent was evaporated, and to the residue was added ethyl acetate. The precipitate was filtered off, and the solvent was evaporated. The residue was purified with silica gel column (ethyl acetate/hexane) to give colorless oil (7.0g), which was dissolved in dimethylformamide (20ml). The mixture was dropwise added to a solution of methyl 5-bromosalicylate (1.8g) and sodium hydride (60%, 0.33g) in dimethylformamide (20ml), under ice-cooling, and the mixture was stirred at 50°C over night. The solvent was evaporated, and to the residue was added water. The mixture was extracted with ethyl acetate. The organic layer was washed with water and saturated sodium chloride solution, anddriedwithanhydrousmagnesiumsulfate. Under reduced pressure, the solvent was evaporated, and the residue was purified with silica gel column (ethyl acetate/hexane) to give colorless oil (1.1g), which was dissolved in tetrahydrofuran (20ml). The mixture was dropwise added to a solution of lithium diisopropylamine, which was prepared by diisopropylamine (0.37g) and a solution of n-butyl lithium in hexane (1.6M, 2.1ml), in tetrahydrofuran, at -78°C. The mixture was stirred at room temperature under argon atmosphere over night and poured into water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless oil (0.3g), which was dissolved in dichloromethane (25ml). The mixture was dropwise added to a solution of sodium boron hydride in methanol at -10°C. After starting materials disappeared, water was added to the reaction mixture, and the mixture was concentrated and extracted with ethyl acetate. The organic layer was washed with and saturated sodium chloride solution, and dried with anhydrous magnesium sulfate. The solvent was evaporated, and the residue was dissolved in dichloromethane (25ml). To the mixture was added triethylamine (0.74ml), and to the mixture was dropwise added methanesulfonylchloride (0.15ml) under ice-cooling. The mixture was stirred at room temperature under nitrogen atmosphere over night, washed with water and dried with anhydrous magnesium sulfate. The solvent was evaporated, and the residue was purified with silica gel column (ethyl acetate/hexane) to give colorless crystals (0.2g), to which were added 4-methylphenyl borate (0.1g), 1M potassium carbonate (2.5ml), ethanol (2.5ml) and toluene (15ml). The mixture was stirred under argon atmosphere at room temperature for 30 minutes, and to the mixture was added tetrakistriphenylphosphinepalladium (0.03g). The mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.2g), to which were added 1N sodium hydroxide (5ml) and methanol (50ml). The mixture was refluxed for 30 minutes, concentrated, acidified with hydrochloric acid and extracted with ethyl acetate. The organic layer was washed with and saturated sodium chloride solution, and dried with anhydrous magnesium sulfate. The solvent was evaporated to give 7-(4-methylphenyl)-2-methyl-2,3-dihydro-1-berizoxepine-4 -carboxylic acid (0.2g) as colorless crystals.
mp 224-225°C.
¹H-NMR (δ ppm, CDCl₃): 1.53 (3H, d, J=6.2Hz), 2.40 (3H, s), 2.81 (1H, ddd, J=2.2, 8.8, 18.0Hz), 3.08 (1H, d, J=18.0Hz), 4.17-4.27 (1H, m), 7.04 (1H, d, J=8.2Hz), 7.24 (2H, d, J=7.4Hz), 7.44-7.52 (4H, m), 7.77 (1H, d, J=2.2Hz).
IR(KBr) ν : 2973, 1674cm⁻¹.

| Anal. for C₁₉H₁₈O₃: | | |
|---|---|---|
| Calcd. | C,77.53; | H,6.16. |
| Found | C,77.60; | H,6.14. |

### Reference Example 68

Inethanol (10ml) and ethyl acetate (60ml) was dissolved 4-methylphenyl 4-nitrobenzyl sulfone (0.5g; G. Bram et al., Synthesis, 1987, 56-59). To the mixture was added 10% palladium on carbon (0.05g) and catalytic hydrogenation was carried out at room temperature over night. The catalyst was filtered off, and the solvent was evaporated to give 4-aminobenzyl 4-methylphenyl sulfone (0.4g) as colorless crystals.
¹H-NMR(δppm, CDCl₃) : 2.42 (3H, s), 4.18 (2H, s), 6.56 (2H, d, J=8.4Hz), 6.86 (2H, d, J=8.4Hz), 7.24 (2H, d, J=8.2Hz), 7.52 (2H, d, J=8.2Hz).
IR(KBr) ν : 3443, 3370, 2926, 1612cm⁻¹.

| Anal. for C₁₄H₁₅NO₂S·0.2H₂O: | | | |
|---|---|---|---|
| Calcd. | C, 63.47; | H,5.86; | N,5.29. |
| Found | C,63.63; | H,5.86; | N,5.09. |

### Reference Example 69

In 1,2-dichloroethane (50ml) were suspended cyclo-pentanone (1g), methylamine hydrochloride (1.6g) and triethylamine (3.4ml), and to the suspension was added sodium triacetoxy boron hydride (3.5g) under ice-cooling. Under nitrogen atmosphere, the mixture was stirred at room temperature over night. The mixture was neutralized with sodium hydroxide, concentrated and extracted with water. The aqueous layer was washed with ethyl acetate. The aqueous layer was saturated with sodium chloride and extracted with diethylether. The organic layer was dried with anhydrous magnesium sulfate. Under reduced pressure, the solvent was evaporated to give N-methylcyclopentylamine (0.5g) as colorless oil.
¹H-NMR(δ ppm, CDCl₃) : 1.21-1.86 (8H, m), 2.40 (3H, s), 2.94-3.01 (1H, m).

### Reference Example 70

In 1,2-dichloroethane (50ml) were suspended cyclo-heptanone (2g), methylamine hydrochloride (3g) and triethylamine (6.2ml), and to the suspension was added sodium triacetoxy boron hydride (5.3g) under ice-cooling. Under nitrogen atmosphere, the mixture was stirred at room temperature over night. The solvent was evaporated, and the residue was neutralized with sodium hydroxide. The mixture was extracted with ethyl acetate. The organic layer was washed with saturated sodium chloride solution and dried with anhydrous magnesium sulfate. Under reduced pressure, the solvent was evaporated to give N-methylcycloheptylamine (1.8g) as colorless oil.
¹H-NMR (δ ppm, CDCl₃) : 1.26-1.70 (10H, m), 1.77-1.89 (2H, m), 2.40 (3H, s), 2.47-2.58 (1H, m).
IR (KBr) ν : 2933, 2860cm⁻¹.

### Reference Example 71

In tetrahydrofuran (100ml) were added 4-amino-1-benzyl-piperidine (10g) and triethylamine (36ml), and to the mixture was dropwise added acetyl chloride (4.1ml) under ice-cooling. The mixture was stirred at room temperature for 1 hour, and the solvent was evaporated. To the residue was added water, and the mixture was extracted with ethyl acetate. The organic layer was washed with saturated sodium chloride solution and dried with anhydrous magnesium sulfate. Under reduced pressure, the solvent was evaporated to give colorless crystal (2.6g), which was dissolved in tetrahydrofuran (10ml). Under ice-cooling, borane methylsulfide (2.2ml) was dropwise added to the solution.Under nitrogen atmosphere,the mixture was refluxed for 5 hours. Under ice-cooling, methanol (10ml) was added to the mixture, and the mixture was stirred at room temperature for 1 hour. To the mixture was added 4N hydrochloric acid-ethyl acetate, and the mixture was refluxed for 1 hour. The solvent was evaporated, and to the residue was added 1N sodium hydroxide. The mixture was extracted with ethyl acetate. The organic layer was washed with water and saturated sodium chloride solution, and dried with anhydrous magnesium sulfate. Under reduced pressure, the solvent was evaporated to give 4-ethylamino-1-benzylpiperidine (1.2g) as colorless oil.
¹H-NMR(δ ppm, CDCl₃): 1.10 (3H, t, J=7.2Hz), 1.28-1.47 (2H, m), 1.82-1.88 (2H, m), 1.95-2.07 (2H, m), 2.40-2.51 (1H, m), 2.66 (2H, q, J=7.2Hz), 2.82-2.88 (2H, m), 3.50 (2H, s), 7.20-7.33 (5H, m).

### Reference Example 72

To a mixture of ethyl 7-bromo-2,3-dihydro-1-benzoxepine-4-carboxylate (0.5g), 4-(4-methylpiperazin-1-yl)phenyl borate (0.44g), 1M potassium carbonate (6ml) and ethanol (6ml) was added toluene (50ml), and the mixture was stirred under argon atmosphere at room temperature for 30 minutes. To the mixture was added tetrakistriphenylphosphinepalladium (0.07g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give colorless crystals (0.39g), which were dissolved in 1N sodium hydroxide (15ml) and methanol (100ml). The mixture was refluxed for 2 hours, concentrated and neutralized with hydrochloric acid to precipitate 7-(4-(4-methylpiperazin-1-yl)phenyl)-2,3-dihydro-1-benz oxepine-4-carboxylic acid (0.33g) as colorless crystals.
mp 278-279°C(dec.).
¹H-NMR (δ ppm, DMSO-d₆) : 2.24 (3H, s), 2.45-2.52 (4H, m), 2.87 (2H, t, J=4.0Hz), 3.15-3.20 (4H, m), 4.23 (2H, t, J=4.8Hz), 6.97-7.01 (3H, m), 7.49-7.62 (4H, m), 7.70 (1H, d, J=2.2Hz).
IR (KBr) ν : 1692cm⁻¹.

| Anal. for C₂₂H₂₄N₂O₃·0.5H₂O: | | | |
|---|---|---|---|
| Calcd. | C,70.76; | H,6.75; | N,7.50. |
| Found | C,70.87; | H,6.50; | N,7.56. |

### Reference Example 73

In 1,2-dichloroethane (35ml) were suspended 4-methylcyclohexanone (2.5g), methylamine hydrochloride (1.6g) and triethylamine (3.3ml), and to the suspension was added sodium triacetoxy boron hydride (6.6g) under ice-cooling. The mixture was stirred under nitrogen atmosphere at room temperature over night. The solvent was evaporated, and the residue was neutralized with sodium hydroxide. The mixture was extracted with ethyl acetate. The organic layer was washed with saturated sodium chloride solution and dried with anhydrous magnesium sulfate. Under reduced pressure, the solvent was evaporated. To the residue was added 4N hydrochloric acid-ethyl acetate, and the solvent was evaporated to give N,4-dimethyl-cyclohexylamine hydrochloride (2.6g) as colorless crystals.
¹H-NMR (δ ppm, CDCl₃) : 0.90 (1.5H, d, J=6.6Hz), 1.01 (1.5H, d, J=6.6Hz), 1.45-2.10 (8H, m), 2.19-2.26 (1H, m), 2.61-2.68 (3H, m), 3.03 (1H, br).

| Anal. for C₈H₁₈ClN: | | | |
|---|---|---|---|
| Calcd. | C,58.70; | H,11.08; | N, 8.56. |
| Found | C,58.42; | H,10.91; | N,8.48. |

### Reference Example 74

In 1,2-dichloroethane (25ml) were suspended p-nitrobenzylamine hydrochloride (1.2g), tetrahydropyran-3-one (0.6g; Numata et al., JP-A-63-170372) and triethylamine (0.9ml), and to the suspension was added sodium triacetoxy boron hydride (1.8g) under ice-cooling. Under nitrogen atmosphere, the mixture was stirred at room temperature over night. Under ice-cooling, to the mixture were added 37% formalin (0.6ml) and sodium triacetoxy boron hydride (1.8g). Under nitrogen atmosphere, the mixture was stirred at room temperature over night, and the solvent was evaporated. The residue was neutralized with sodium hydroxide, and the mixture was extracted with ethyl acetate. The organic layer was washed with water and saturated sodium chloride solution,and dried with anhydrous magnesium sulfate. Under reduced pressure, the solvent was evaporated, and the residue was purified with silica gel column (ethyl acetate/hexane) to give pale yellow oil (1.0g), to which was added reduced iron (0.6g) and acetic acid (50ml). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated to give 4-(N-methyl-N-(tetrahydropyran-3-yl)-aminomethyl)aniline (0.3g) as brown oil.
¹H-NMR(δppm, CDCl₃): 1.46-1.75 (3H, m), 1.95-2.01 (1H, m), 2.19 (3H, s), 2.55-2.68 (1H, m), 3.21-3.40 (2H, m), 3.49 (2H, s), 3.59 (2H, br), 3.83-3.89 (1H, m), 4.00-4.08 (1H, m), 6.64 (2H, d, J=8.4Hz), 7.07 (2H, d, J=8.4Hz).
IR(neat) ν : 2941, 2846, 1615cm⁻¹.

### Reference Example 75

In 1,2-dichloroethane (50ml) were suspended 2-amino-indane hydrochloride (1.0g), p-nitrobenzaldehyde (0.9g) and triethylamine (0.9ml), and to the mixture was added sodium triacetoxy boron hydride (1.8g) under ice-cooling. Under nitrogen atmosphere, the mixture was stirred at room temperature over night. Under ice-cooling, to the mixture were added 37% formalin (0.6ml) and sodium triacetoxy boron hydride (1.8g). Under nitrogen atmosphere, the mixture was stirred at room temperature over night, and the solvent was evaporated. The residue was neutralized with sodium hydroxide, and the mixture was extracted with ethyl acetate. The organic layer was washed with water and saturated sodium chloride solution, and dried with anhydrous magnesium sulfate. Under reduced pressure, the solvent was evaporated to give colorless crystals (1.7g), which was dissolved in ethanol (50ml) and ethyl acetate (50ml) . To the mixture was added 10% palladium on carbon (0.15g), and catalytic hydrogenation was carried out at room temperature for 1 hour. The catalyst was filtered off, and the solvent was evaporated. The residue was purified with silica gel column (ethyl acetate) to give 4-((N-indan-2-yl-N-methyl)aminomethyl)aniline (0.6g) as colorless crystals.
mp 95-96°C .
¹H-NMR(δ ppm, CDCl₃): 2.17 (3H, s), 2.91-3.16 (4H, m), 3.32-3.43 (1H, m), 3.47 (2H, s), 3.61 (2H, br), 6.66 (2H, d, J=8.8Hz), 7.10-7.22 (6H, m).
IR(KBr) ν : 2782, 1623cm⁻¹.

| Anal. for C₁₇H₂₀N₂·0.2H₂O: | | | |
|---|---|---|---|
| Calcd. | C,79.77; | H,8.03; | N,10.94. |
| Found | C,79.87; | H,8.04; | N,10.75. |

### Reference Example 76

In 1,2-dichloroethane (50ml) were suspended p-nitrobenzylamine hydrochloride (1.9g), 4-t-butylcyclohexanone (1.5g) and triethylamine (1.4ml), and to the suspension was added sodium triacetoxy boron hydride (3g) under ice-cooling. Under nitrogen atmosphere, the mixture was stirred at room temperature over night. Under ice-cooling, to the mixture were added 37% formalin (0.9ml) and sodium triacetoxy boron hydride (3g). Under nitrogen atmosphere, the mixture was stirred at room temperature over night, and the solvent was evaporated. The residue was neutralized with sodium hydroxide, and the mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give (E)-N-(4-t-butylcyclohexyl)-N-methyl-N-(4-nitro-benzyl) amine (0.3g) as colorless crystals and (Z)-N-(4-t-butylcyclohexyl)-N-methyl-N-(4-nitrobenzyl)a mine (2.4g) as yellow oil.
(E)-N-(4-t-butylcyclohexyl)-N-methyl-N-(4-nitrobenzyl)-amine :
mp 96-97°C.
¹H-NMR(δ ppm, CDCl₃) : 0.85 (9H, s), 0.94-1.05 (3H, m), 1.20-1.40 (2H, m), 1.80-2.00 (4H, m), 2.19 (3H, s), 2.29-2.44 (1H, m), 3.65 (2H, s), 7.51 (2H, d, J=8.4Hz), 8.17 (2H, d, J=8.4Hz).
IR (KBr) ν : 2941, 1604, 1513cm⁻¹.

| Anal . for C₁₈H₂₈N₂O₂: | | | |
|---|---|---|---|
| Calcd. | C,71.02; | H,9.27; | N,9.20. |
| Found | C,70.77; | H,9.26; | N,9.32. |

(Z)-N-(4-t-butylcyclohexyl)-N-methyl-N-(4-nitrobenzyl)-amine :
¹H-NMR (δ ppm, CDCl₃) : 0.89 (9H, s), 1.15-1.20 (1H, m), 1.30-1.54 (6H, m), 1.97-2.10 (2H, m), 2.08 (3H, s), 2.38 (1H, br), 3.61 (2H, s), 7.52 (2H, d, J=8.4Hz), 8.18 (2H, d, J=8.4H2).
IR(neat) ν: 2943, 1606, 1521cm⁻¹.

### Reference Example 77

Inethanol (25ml) and ethyl acetate (25ml) was dissolved (E)-N-(4-t-butylcyclohexyl)-N-methyl-N-(4-nitrobenzyl)a mine (0.3g). To the mixture was added 10% palladium on carbon (0.03g) and catalytic hydrogenation was carried out at room temperature for 1 hour. The catalyst was filtered off, and the solvent was evaporated. The residue was purified with silica gel column (ethyl acetate/methanol/triethylamine) to give (E)-4-((N-4-t-butyl-cyclohexyl-N-methyl)aminomethyl)ani line (0.2g) as colorless crystals.
mp 87-88°C.
¹H-NMR(δ ppm, CDCl₃): 0.84 (9H, s), 0.93-1.03 (2H, m), 1.15-1.40 (2H, m), 1.81-1.96 (5H, m), 2.19 (3H, s), 2.30-2.45 (1H, m), 3.48 (2H, s), 3.60 (2H, br), 6.65 (2H, d, J=8.4Hz), 7.10 (2H, d, J=8.4Hz).
IR (KBr) ν: 2927, 1614, 1517cm⁻¹.

| Anal. for C₁₈H₃₀N₂·0.2H₂O: | | | |
|---|---|---|---|
| Calcd. | C,77.75; | H,11.02; | N,10.07. |
| Found | C,77.87; | H,10.93; | N,10.16. |

### Reference Example 78

In acetic acid (70ml) was dissolved (Z)-N-(4-t-butylcyclohexyl)-N-methyl-N-(4-nitrobenzyl)amine (1.2g), and to the mixture was added reduced iron (1.1g). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated, and the residue was purified with silica gel column (ethyl acetate to give (Z)-4-((N-4-t-butyl-cyclohexyl-N-methyl)aminomethyl)ani line (0.7g) as yellow oil.
¹H-NMR(δ ppm, CDCl₃) : 0.87 (9H, s), 1.00-1.20 (1H, m), 1.25-1.56 (6H, m), 2.04 (3H, s), 2.04-2.13 (2H, m), 2.26-2.29 (1H, m), 3.40 (2H, s), 3.58 (2H, br), 6.65 (2H, d, J=8.4Hz), 7.10 (2H, d, J=8.4Hz).
IR(neat) ν: 2941, 1623, 1515cm⁻¹.

### Reference Example 79

In 1,2-dichloroethane (70ml) were suspended p-nitrobenzylamine hydrochloride (3.8g), 3,5-dimethylcyclo-hexanone (2.5g) and triethylamine (2.8ml) . Under ice-cooling, to the mixture was added sodium triacetoxy boron hydride(5.9g). Under nitrogen atmosphere, the mixture was stirred at room temperature over night. Under ice-cooling, to the mixture were added 37% formalin (1.8ml) and sodium triacetoxy boron hydride (5. 9g) . Under nitrogen atmosphere, the mixture was stirred at room temperature over night. The solvent was evaporated, and the residue was neutralized with sodium hydroxide. The mixture was extracted with ethyl acetate . The organic layer was washed with water and saturated sodium chloride solution, and dried with anhydrous magnesium sulfate. Under reduced pressure, the solvent was evaporated, and the residue was purified with silica gel column (ethyl acetate/hexane) to give 3 isomers of N-methyl-N-(3,5-dimethylcyclohexyl)-N-(4-nitrobenzyl)-a mine (4.3g; (31-a), 0.7g; (31-b), 0.2g; (31-c)) as each yellow oil.
31-a: ¹H-NMR(δ ppm, CDCl₃) : 0.53-0.74 (1H, m), 0.84 (3H, s), 0.87 (3H, s), 0.93-1.07 (2H, m), 1.73-1.99 (5H, m), 2.06 (3H, s), 2.49 (1H, t, J=2.8Hz), 3.60 (2H, s), 7.50 (2H, d, J=8.8Hz), 8.17 (2H, d, J=8.8Hz).
IR(neat) ν : 2949, 1606, 1521cm⁻¹.
31-b: ¹H-NMR (δ ppm, CDCl₃) : 0.51 (1H, q, J=12.0Hz), 0.80-1.02 (2H, m), 0.92 (3H, s), 0.95 (3H, s), 1.34-1.53 (2H, m), 1.58-1.66 (1H, m), 1.78-1.84 (2H, m), 2.19 (3H, s), 2.53 (1H, tt, J=3.3, 11.7Hz), 3.65 (2H, s), 7.51 (2H, d, J=8.8Hz), 8.17 (2H, d, J=8.8Hz).
IR(neat) ν : 2949, 1606, 1519cm⁻¹.
31-c: ¹H-NMR (δ ppm, CDCl₃) : 0.80-1.13 (8H, m), 1.38-1.52 (2H, m), 1.62-1.68 (2H, m), 1.80-1.86 (1H, m), 2.08-2.17 (1H, m), 2.18 (3H, s), 2.74 (1H, tt, J=3.5, 11.9Hz), 3.64 (2H, s), 7.51 (2H, d, J=8.4Hz), 8.17 (2H, d, J=8.4Hz).
IR(neat) ν: 2920, 1606, 1521cm⁻¹.

### Reference Example 80

In ethanol(50ml) and ethyl acetate (50ml) was dissolved N-methyl-N-(3,5-dimethylcyclohexyl)-N-(4-nitrobenzyl)am ine (2.0g ; (31-a)). To the mixture was added 10% palladium on carbon (0.2g) and catalytic hydrogenation was carried out at room temperature for 1 hour. The catalyst was filtered off, and the solvent was evaporated. The residue was purified with silica gel column (ethyl acetate/methanol/triethylamine) to give 4-((N-(3,5-dimethylcyclohexyl)-N-methyl)aminomethyl)ani line (0.2g) as pale yellow oil.
¹H-NMR(δppm, CDCl₃): 0.58 (1H, q, J=11.7Hz), 0.83 (3H, s), 0.86 (3H, s), 0.93-1.00 (2H, m), 1.69-2.04 (5H, m), 2.04 (3H, s), 2.24-2.40 (1H, m), 3.41 (2H, s), 3.50 (2H, br), 6.64 (2H, d, J=8.6Hz), 7.08 (2H, d, J=8.6Hz).
IR(neat) ν : 2947, 1623cm⁻¹.

### Reference Example 81

In acetic acid (30ml) was dissolved N-methyl-N-(3,5-dimethylcyclohexyl)-N-(4-nitrobenzyl)am ine (0.7g; (31-b)), and to the mixture was added reduced iron (0.7g). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated, and the residue was purified with silica gel column (ethyl acetate/methanol/triethylamine) to give 4-((N-(3,5-dimethylcyclo-hexyl)-N-methyl)aminomethyl)an iline (0.4g) as yellow oil.
¹H-NMR(δppm, CDCl₃) : 0.50 (1H, q, J=12.0Hz), 0.80-1.03 (1H, m), 0.91 (3H, s), 0.94 (3H, s), 1.22-1.50 (3H, m), 1.55-1.64 (1H, m), 1.78-1.84 (2H, m), 2.17 (3H, s), 2.53 (1H, tt, J=3.3, 11.8Hz), 3.46 (2H, s), 3.58 (2H, br), 6.64 (2H, d, J=8.6Hz), 7.09 (2H, d, J=8.6Hz).
IR(neat) ν : 2949, 1621cm⁻¹.

### Reference Example 82

In acetic acid (15ml) was dissolved N-methyl-N-(3,5-dimethylcyclohexyl)-N-(4-nitrobenzyl)am ine (0.2g; (31-c)), and to the mixture was added reduced iron (0.2g). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated, and the residue was purified with silica gel column (ethyl acetate/methanol/triethylamine) to give 4-((N-(3,5-dimethylcyclo-hexyl)-N-methyl)aminomethyl)an iline (0.1g) as brown oil.
¹H-NMR (δ ppm, CDCl₃) : 0.87-1.15 (7H, m), 1.35-1.55 (2H, m), 1.60-1.70 (2H, m), 1.75-1.90 (1H, m), 2.05-2.19 (2H, m), 2.17 (3H, s), 2.75 (1H, tt, J=3.3, 12.1Hz), 3.45 (2H, s), 3.60 (2H, br), 6.64 (2H, d, J=8.3Hz), 7. 09 (2H, d, J=8.3Hz).

### Reference Example 83

In 1,2-dichloroethane (50ml) were dissolved n-propylamine (1.1g) and p-nitrobenzaldehyde (2.3g). Under ice-cooling, to the mixture was added sodium triacetoxy boron hydride (4.5g). Under nitrogen atmosphere, the mixture was stirred at room temperature over night. Under ice-cooling, to the mixture were added 37% formalin (1.7ml) and sodium triacetoxy boron hydride (4.5g) . Under nitrogen atmosphere, the mixture was stirred at room temperature over night, and the solvent was evaporated. The residue was neutralized with sodium hydroxide, and the mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow oil (2.3g), which was dissolved in tetrahydrofuran (10ml). The mixture was dropwise added to a solution, which was prepared by adding dropwise lithium aluminum hydride (0.5g) to a solution of titanium tetrachloride (2ml) in tetrahydrofuran (50ml), under ice-cooling, and stirring the mixture at room temperature for 15 minutes, under ice-cooling. The mixture was stirred at room temperature for 30 minutes, and to the mixture were added water (50ml) and ammonia solution (50ml). The mixture was concentrated and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give 4-((N-methyl-N-n-propyl)aminomethyl)aniline (0.25g) as yellow oil.
¹H-NMR(δ ppm, CDCl₃) : 0.88 (3H, t, J=7.3Hz), 1.43-1.61 (2H, m), 2.16 (3H, s), 2.30 (2H, t, J=7.7Hz), 3.37 (2H, s), 3.59 (2H, br), 6.64 (2H, d, J=8.0Hz), 7.08 (2H, d, J=8.0Hz).
IR(neat) ν : 2960, 1623, 1517cm⁻¹.

### Reference Example 84

In 1,2-dichloroethane (50ml) were dissolved isopropylamine (1g) and p-nitrobenzaldehyde (2.3g), and to the mixture was added sodium triacetoxy boron hydride (4.5g) under ice-cooling. Under nitrogen atmosphere, the mixture was stirred at room temperature over night. Under ice-cooling, to the mixture were added 37% formalin (1.5ml) and sodium triacetoxy boron hydride (4.5g). Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give yellow oil (2.8g), 1.5g of which was dissolved in ethanol (25ml) and ethyl acetate (25ml). To the mixture was added 10% palladium on carbon (0.15g), and catalytic hydrogenation was carried out at room temperature for 1 hour. The catalyst wasfilteredoff, and the solvent was evaporated. The residue was purified with silica gel column (ethyl acetate/methanol/triethylamine) to give 4-((N-isopropyl-N-methyl)aminomethyl)aniline (0.17g) as pale yellow oil.
¹H-NMR(δppm, CDCl₃): 1.05 (6H, d, J=6.6Hz), 2.13 (3H, s), 2.81-2.95 (1H, m), 3.40 (2H, s), 3.60 (2H, br), 6.65 (2H, d, J=8.4Hz), 7.10 (2H, d, J=8.4Hz).
IR(neat) ν : 2966, 1623, 1517cm⁻¹.

### Reference Example 85

In 1,2-dichloroethane (50ml) were dissolved 1-methylpropylamine (1.3g) and p-nitrobenzaldehyde (2.3g), and to the mixture was added sodium triacetoxy boron hydride (4 . 5g) under ice-cooling. Under nitrogen atmosphere, the mixture was stirred at room temperature over night. Under ice-cooling, to the mixture were added 37% formalin (1.7ml) and sodium triacetoxy boron hydride (4.5g) . Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated to give brown oil (3.4g), 2.0g of which was dissolved in tetra-hydrofuran (20ml). The mixture was dropwise added to a solution, which was prepared by adding dropwise lithium-aluminum hydride (0.7g) to a solution of titanium tetrachloride (3ml) in tetrahydrofuran (50ml) under ice-cooling and stirring the mixture at room temperature for 15 minutes, under ice-cooling. The mixture was stirred at room temperature over night, and, to the mixture were added water (75ml) and ammonia solution (75ml). The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give 4-((N-sec-butyl-N-methyl)aminomethyl)aniline (0.8g) as yellow oil.
¹H-NMR(δ ppm, CDCl₃): 0.87-0.99 (6H, m), 1.22-1.37 (1H, m), 1.53-1.63 (1H, m), 2.11 (3H, s), 2.53-2.63 (1H, m), 3.34 (1H, d, J=12.8Hz), 3.46 (1H, d, J=12.8Hz), 3.57 (2H, br), 6.64 (2H, d, J=8.4Hz), 7.11 (2H, d, J=8.4Hz).
IR(neat) ν: 2962, 2933, 2873, 1617, 1517cm⁻¹.

### Reference Example 86

In 1,2-dichloroethane (70ml) were dissolved t-butylamine (1.6g) and p-nitrobenzaldehyde (3.0g), and to the mixture was added sodium triacetoxy boron hydride (5.9g) under ice-cooling. Under nitrogen atmosphere, the mixture was stirred at room temperature over night. Under ice-cooling, to the mixture were added 37% formalin (2ml) and sodium triacetoxy boron hydride (5.9g) . Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated, to give brown oil (4.4g), which was dissolved in acetic acid (50ml). To the mixture was added reduced iron (3.2g), and the mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate . Under reduced pressure, the solvent was evaporated to give 4-((N-t-butyl-N-methyl)aminomethyl)-aniline (2.2g) as brown oil.
¹H-NMR (δ ppm, CDCl₃) : 1.14 (9H, s), 2.07 (3H, s), 3.38 (2H, s), 3.57 (2H,br), 6.64 (2H, d, J=8.4Hz), 7.11 (2H, d, J=8.4Hz).
IR(neat) ν : 2971, 1622, 1516cm⁻¹.

### Reference Example 87

In 1,2-dichloroethane (70ml) were suspended p-nitrobenzylamine hydrochloride (3.8g) and 3-pentanone (1.7g), and to the suspension was added triethylamine (2.8ml) . Under ice-cooling, to the mixture was added sodium triacetoxy boron hydride (5.9g) . Under nitrogen atmosphere, the mixture was stirred at room temperature over night. Under ice-cooling, to the mixture were added 37% formalin (1.8ml) and sodium triacetoxy boron hydride (5.9g). Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated to give pale yellow oil (4.6g), which was dissolved in acetic acid (100ml). To the mixture was added reduced iron (4.7g), and the mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, anddriedwithanhydrousmagnesiumsulfate. Under reduced pressure, the solvent was evaporated to give 4-((N-methyl-N-(pentan-3-yl))-amino-methyl)aniline (3.3g) as pale brown oil.
¹H-NMR(δ ppm, CDCl₃): 0.92 (6H, t, J=7.3Hz), 1.20-1.59 (4H, m), 2.10 (3H, s), 2.18-2.29 (1H, m), 3.44 (2H, s), 3.57 (2H, br), 6.64 (2H, d, J=8.4Hz), 7. 11 (2H, d, J=8.4Hz).
IR(neat) ν : 2959, 1622, 1516cm⁻¹.

### Reference Example 88

In 1,2-dichloroethane (70ml) were suspended p-nitrobenzylamine hydrochloride (3.8g) and norcamphor (2.2g), and to the suspension was added triethylamine (2.8ml) . Under ice-cooling, to the mixture was added sodium triacetoxy boron hydride (5.9g) . Under nitrogen atmosphere, the mixture was stirred at room temperature over night. Under ice-cooling, to the mixture were added 37% formalin (1.8ml) and sodium triacetoxy boron hydride (5.9g) . Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated to give pale yellow oil (5.2g), which was dissolved in acetic acid (100ml) . To the mixture was added reduced iron (5g), and the mixture was stirred at room temperature over night. The solvent was evaporated, and to the residue was added ethyl acetate. The precipitate was filtered off, and the filtrate was washed with sodium hydrogen carbonate solution, water and saturated sodium chloride solution,and dried with anhydrous magnesium sulfate. Under reduced pressure, the solvent was evaporated to give 4-((N-methyl-N-(norbornan-2-yl))amino-methyl)aniline (4.0g) as pale brown oil.
¹H-NMR(δppm, CDCl₃): 0.94-1.04 (1H, m), 1.22-1.55 (5H, m), 1.68-1.97 (2H, m), 2.00 (3H, s), 2.16 (1H, br), 2.37 (2H, br), 3.22 (1H, d, J=12.8Hz), 3.42 (1H, d, J=12.8Hz), 3.58 (2H, br), 6.64 (2H, d, J=8.4Hz), 7.09 (2H, d, J=8.4Hz).
IR(neat) ν : 2949, 1622, 1516cm⁻¹.

### Reference Example 89

To a mixture of p-nitrophenethylbromide (2.3g), N-methylcyclohexylamine (2.8g), potassium carbonate (6.6g) and sodium iodide (1.5g) was added dimethylformamide (50ml), and the mixture was stirred at 50°C over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/methanol/triethylamine) to give yellow oil (2.2g), which was dissolved in ethanol (50ml). To the mixture was added 10% palladium on carbon (0.2g), and catalytic hydrogenation was carried out at room temperature over night. The catalyst was filtered off, and the solvent was evaporated to give 4-(2-(N-cyclohexyl-N-methyl)aminoethyl)aniline (1.9g) as pale yellow oil.
¹H-NMR(δ ppm, CDCl₃) : 1.05-1.30 (6H, m), 1.60-1.79 (4H, m), 2.33 (3H, s), 2.33-2.45 (1H, m), 2.61-2.63 (4H, m), 3.55 (2H, br), 6.63 (2H, d, J=8.4Hz), 6.99 (2H, d, J=8.4Hz).
IR(neat) ν : 2929, 1625, 1517cm⁻¹.

### Reference Example 90

In ethanol (15ml) were dissolved p-nitrostyreneoxide (0.5g; E. Borredon et al., J. Org. Che., 1990, 55, 501-504) and piperidine (0.36ml), and the mixture was refluxed for 1 hour. The solvent was evaporated to give yellow crystals (0.53g), whichwasdissolvedinethanol (50ml) . To the mixture was added 5% palladium on carbon (0.05g), and catalytic hydrogenation was carried out at room temperature 1.5 hours. The catalyst was filtered off, and the solvent was evaporated,4-(1-hydroxy-2-piperidino-ethyl)aniline (0.4g) as colorless crystals.
mp 75-76°C.
¹H-NMR(δ ppm, CDCl₃) : 1.40-1.50 (2H, m), 1.55-1.70 (4H, m), 2.31-2.41 (4H, m), 2.62-2.75 (2H, m), 3.61 (2H, br), 4.61 (1H, dd, J=6.2, 8.0Hz), 6.66 (2H, d, J=8.4Hz), 7.15 (2H, d, J=8.4Hz).
IR (KBr) ν : 2936, 1622, 1518cm⁻¹.

| Anal. for C₁₃H₂₀N₂O: | | | |
|---|---|---|---|
| Calcd. | C,70.87; | H,9.15; | N,12.72. |
| Found | C, 71.02; | H,9.10; | N,13.01. |

### Reference Example 91

In dimethylformamide (50ml) were dissolved methyl 5-bromosalicylate (5g), ethyl 4-bromobutyrate (4.2g) and potassium carbonate (7.5g), and the mixture was stirred at room temperature over night. The solvent was evaporated, and to the residue was added water. The mixture was extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless oil (6.5g), which was dissolved in tetrahydrofuran (20ml). The mixture was dropwise added to a solution of lithium diisopropylamine in tetrahydrofuran prepared by diisopropylamine (3.2ml) and n-butyllithium in hexane (1.6M, 13ml), at -78°C. The mixture was stirred at room temperature under argon atmosphere over night and poured into water. The mixture was extracted with ethyl acetate. The organic layer was washed with water and saturated sodium chloride solution, and dried with anhydrous magnesium sulfate. Under reduced pressure, the solvent was evaporated to give oil, which was dissolved in dichloromethane (100ml). The mixture was dropwise added to a solution of sodium boron hydride in methanol at -15°C. After starting materials disappeared, water was added to the reaction mixture, and the mixture was concentrated and extracted with ethyl acetate. The organic layer was washed with and saturated sodium chloride solution, and dried with anhydrous magnesium sulfate. The solvent was evaporated, and the residue was dissolved in dichloromethane (100ml). To the mixture was added triethylamine (7.9ml), and to the mixture was dropwise added methanesulfonylchloride (2.2ml) under ice-cooling. The mixture was stirred at room temperature under nitrogen atmosphere over night, and to the mixture was added water. The mixture was concentrated and extracted with ethyl acetate. The organic layer was washed with and saturated sodium chloride solution, and dried with anhydrous magnesium sulfate. The solvent was evaporated, and the residue was purified with silica gel column (ethyl acetate/hexane) to give ethyl 7-bromo-2,3-dihydro-1-benzoxepine-4-carboxylate (2.3g) as colorless crystals.
mp 86-87°C.
¹H-NMR(δ ppm, CDCl₃) : 1.35 (3H, t, J=7.2Hz), 2.98 (2H, t, J=4.7Hz), 4.23-4.33 (4H, m), 6.86 (1H, d, J=8.8Hz), 7.32 (1H, dd, J=2.6, 8.8Hz), 7.46-7.47 (2H, m).

### Reference Example 92

To a mixture of ethyl 7-bromo-2,3-dihydro-1-benzoxepine-4-carboxylate (0.5g), diethyl(3-pyridyl)-borane (0.26g), 1M potassium carbonate (6ml) and ethanol (6ml) was added toluene (50ml), and the mixture was stirred under argon atmosphere at room temperature for 30 minutes. To the mixture was added tetrakistriphenyl-phosphinepalladium (0.07g), and the mixture was refluxed over night. The mixture was extracted with ethyl acetate, and the organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.28g), which were dissolved in 1N sodium hydroxide (10ml) and methanol (50ml). The mixture was stirred at room temperature over night, concentrated and neutralized with hydrochloric acid to precipitate 7-(3-pyridyl)-2,3-dihydro-1-benzoxepine-4-carboxylic acid (0.3g) as colorless crystals.
mp >300°C.
¹H-NMR (δ ppm, DMSO-d₆) : 2.89 (2H, t, J=4.6Hz), 4.27 (2H, t, J=4.6Hz), 7.09 (1H, d, J=8.4Hz), 7.46 (1H, dd, J=4.6, 7.8Hz), 7.64-7.69 (2H, m), 7.90 (1H, d, J=2.2Hz), 8.10 (1H, dt, J=7.8, 1.5Hz), 8.54 (1H, dd, J=1.5, 4.6Hz), 8.92 (1H, d, J=2.2Hz).
IR (KBr) ν : 1699cm⁻¹.

| Anal. for C₁₆H₁₃NO₃·0.2H₂O : | | | |
|---|---|---|---|
| Calcd. | C,70.94; | H,4.99; | N,5.17. |
| Found | C,70.71; | H,5.00; | N,5.17. |

### Reference Example 93

To a mixture of ethyl 7-bromo-2,3-dihydro-1-benzoxepine-4-carboxylate (1.0g), 4-pyridyl borate (0.46g), 1M potassium carbonate (11ml) and ethanol (11ml) was added toluene (80ml), and the mixture was stirred under argon atmosphere at room temperature for 30 minutes. To the mixture was added tetrakistriphenylphosphinepalladium (0.16g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless oil (0.52g), which was dissolved in 1N sodium hydroxide (18ml) and methanol (100ml). The mixture was stirred at room temperature over night, concentrated and neutralized with hydrochloric acid to precipitate 7-(4-pyridyl)-2,3-dihydro-1-benzoxepine-4-carboxylic acid (0.34g) as colorless crystals.
mp 277-278°C (dec.).
¹H-NMR (δ ppm, DMSO-d₆) : 2.89 (2H, t, J=4.8Hz), 4.28 (2H, t, J=4.8Hz), 7.10 (1H, d, J=8.6Hz), 7.68 (1H, s), 7.74-7.79 (3H, m), 8.02 (1H, d, J=2.2Hz), 8.61 (2H, d, J=5.6Hz).

| Anal. for C₁₆H₁₃NO₃·0.1H₂O : | | | |
|---|---|---|---|
| Calcd. | C,71.42 ; | H, 4.94; | N,5.21. |
| Found | C,71.30 ; | H, 4.80 ; | N,5.05. |

### Reference Example 94

To a mixture of ethyl 7-bromo-2,3-dihydro-l-benzoxepine-4-carboxylate (0.5g), 2-furyl borate (0.22g), 1M potassium carbonate (6ml) and ethanol (6ml) was added toluene (50ml) and, the mixture was stirred under argon atmosphere at room temperature for 30 minutes. To the mixture was added tetrakistriphenylphosphinepalladium (0.07g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.37g), which were dissolved in 1N sodium hydroxide (10ml) and methanol (50ml). The mixture was stirred at room temperature over night, concentrated and acidified with hydrochloric acid. The mixture was extracted with ethyl acetate. The organic layer was washed with water and saturated sodium chloride solution, and dried with anhydrous magnesium sulfate. Under reduced pressure, the solvent was evaporated to give 7-(2-furyl)-2,3-dihydro-1-benzoxepine-4-carboxylic acid (0.3g) as colorless crystals.
mp 234-235°C (dec.).
¹H-NMR(δppm, CDCl₃) : 3.02 (2H, t, J=4.7Hz), 4.32 (2H, t, J=4.7Hz), 6.47 (1H, dd, J=1.5, 3.2Hz), 6.58 (1H, dd, J=0.7, 3.2Hz), 7.02 (1H, d, J=8.6Hz), 7.46 (1H, dd, J=0.7, 1.5Hz), 7.57 (1H, dd, J=2.2, 8.6Hz), 7.68 (1H, d, J=2.2Hz), 7.77 (1H, s).
IR (KBr) ν : 1686cm⁻¹.

| Anal. for C₁₅H₁₂O₄: | | |
|---|---|---|
| Calcd. | C,70.31; | H,4.72. |
| Found | C,70.31; | H,4.73. |

### Reference Example 95

To a mixture of ethyl 7-bromo-2,3-dihydro-1-benzoxepine-4-carboxylate (0.5g), 4 -dimethylaminophenyl borate (0.3g), 1M potassium carbonate (6ml) and ethanol (6ml) was added toluene (50ml), and the mixture was stirred under argon atmosphere at room temperature for 30 minutes. To the mixture was added tetrakistriphenylphosphine-palladium (0.07g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow crystals (0.45g), which were dissolved in 1N sodium hydroxide (15ml), methanol (100ml) and tetrahydrofuran (25ml). The mixture was stirred at room temperature over night, concentrated and neutralized with hydrochloric acid to precipitate 7-(4-dimethylamino-phenyl)-2,3-dihydro-1-benzoxepine-4-carboxylic acid (0.4g) as pale yellow crystals.
mp 281-282°C (dec.).
¹H-NMR(δppm, DMSO-d₆) : 2.87 (2H, t, J=4.6Hz), 2.93 (6H, s), 4.23 (2H, t, J=4.6Hz), 6.78 (2H, d, J=8.8Hz), 6.99 (1H, d, J=8.4Hz), 7.47-7.54 (3H, m), 7.62 (1H, s), 7.67 (1H, d, J=2.2Hz).
IR(KBr) ν : 1676 cm⁻¹.

| Anal. for C₁₉H₁₉NO₃ : | | | |
|---|---|---|---|
| Calcd. | C,73.77 ; | H,6.19; | N,4.53. |
| Found | C,73.57; | H,6.22; | N,4.64. |

### Reference Example 96

To a mixture of ethyl 7-bromo-2,3-dihydro-1-benzoxepine-4-carboxylate (0.5g),4-(pyrrolidin-1-yl)phenyl borate (0.35g), 1M potassium carbonate (6ml) and ethanol (6ml) was added toluene (50ml), and the mixture was stirred under argon atmosphere at room temperature for 30 minutes. To the mixture was added tetrakistriphenylphosphinepalladium (0.07g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give pale yellow crystals (0.55g), which were dissolved in 1N sodium hydroxide (15ml), methanol (25ml) and tetrahydrofuran (25ml). The mixture was stirred at room temperature over night, concentrated and neutralized with hydrochloric acid to precipitate 7-(4-(pyrrolidin-1-yl)phenyl)-2,3-dihydro-1-benzoxepine -4-carboxylic acid (0.5g) as pale yellow crystals.
mp 266-267°C (dec.).
¹H-NMR (δ ppm, DMSO-d₆) : 1.94-2.00 (4H, m), 2.87 (2H, t, J=4.4Hz), 3.25-3.30 (4H, m), 4.22 (2H, t, J=4.4Hz), 6.59 (2H, d, J=8.8Hz), 6.98 (1H, d, J=8.4Hz), 7.45-7.52 (3H, m), 7.61 (1H, s), 7.65 (1H, d, J=2.2Hz).
IR (KBr) ν : 1678cm⁻¹.

| Anal. for C₂₁H₂₁NO₃·0.2H₂O : | | | |
|---|---|---|---|
| Calcd. | C,74.40; | H,6.36; | N,4.13. |
| Found | C,74.49; | H,6.39; | N,4.47. |

### Reference Example 97

To a mixture of ethyl 7-bromo-2,3-dihydro-1-benzoxepine-4-carboxylate (0.5g), 4-piperidinophenyl borate (0.38g), 1M potassium carbonate (6ml) and ethanol (6ml) was added toluene (50ml), and the mixture was stirred under argon atmosphere at room temperature for 30 minutes. To the mixture was added tetrakistriphenylphosphine-palladium (0.07g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.62g), which were dissolved in 1N sodium hydroxide (10ml), methanol (25ml) and tetrahydrofuran (25ml). The mixture was stirred at room temperature over night, concentrated and neutralized with hydrochloric acid to precipitate 7-(4-piperidino-phenyl)-2,3-dihydro-1-benzoxepine-4-car boxylic acid (0.6g) as pale yellow crystals.
mp 262-263°C (dec.).
¹H-NMR (δ ppm, DMSO-d₆): 1.50-1.75 (6H, m), 2.87 (2H, t, J=4.8Hz), 3.15-3.19 (4H, m), 4.23 (2H, t, J=4.8Hz), 6.96 (2H, d, J=8.8Hz), 7.00 (1H, d, J=8.4Hz), 7.51 (1H, dd, J=2.4, 8.4Hz), 7.52 (2H, d, J=8.8Hz), 7.62 (1H, s), 7.68 (1H, d, J=2.4Hz).
IR (KBr) ν : 2932, 1690cm⁻¹.

### Reference Example 98

To a mixture of ethyl 7-bromo-2,3-dihydro-1-benzoxepine-4-carboxylate (0.5g), 4-morpholinophenyl borate (0.39g), 1M potassium carbonate (6ml) and ethanol (6ml) was added toluene (50ml), and the mixture was stirred under argon atmosphere at room temperature for 30 minutes. To the mixture was added tetrakistriphenylphosphine-palladium (0.07g), and the mixture was refluxed for 4 hours and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.54g), which were dissolved in 1N sodium hydroxide (15ml), methanol (100ml) and tetrahydrofuran (100ml). The mixture was stirred at room temperature over night, concentrated and neutralized with hydrochloric acid to precipitate 7-(4-morpholino-phenyl)-2,3-dihydro-1-benzoxepine-4-car boxylic acid (0.44g) as colorless crystals.
mp 291-292°C (dec.) .
¹H-NMR(δ ppm, DMSO-d₆): 2.87 (2H, t, J=4 . 8Hz), 3 . 12-3 . 17 (4H, m), 3.73-3.78 (4H, m), 4.23 (2H, t, J=4.8Hz), 7.00 (3H, d, J=8.4Hz), 7.51 (1H, dd, J=2.4, 8.4Hz), 7.56 (2H, d, J=8.8Hz), 7.60 (1H, s), 7.69 (1H, d, J=2.4Hz).

| Anal. for C₂₁H₂₁NO₄ : | | | |
|---|---|---|---|
| Calcd. | C,71.78; | H,6.02; | N,3.99. |
| Found | C,71.42; | H, 6.19; | N,4.16. |

### Reference Example 99

To a mixture of ethyl 7-bromo-2,3-dihydro-1-benzoxepine-4-carboxylate (0.5g), 4-(1-imidazolyl)phenyl borate (0.38g), 1M potassium carbonate (7ml) and ethanol (7ml) was added toluene (50ml), and the mixture was stirred under argon atmosphere at room temperature for 30 minutes. To the mixture was added tetrakistriphenylphosphine-palladium (0.07g), and the mixture was refluxed for 4 hours and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give colorless crystals (0.53g), which were dissolved in 1N sodium hydroxide (10ml) and methanol (50ml). The mixture was stirred at room temperature over night, concentrated and neutralized with hydrochloric acid to precipitate 7-(4-(1-imidazolyl)phenyl)-2,3-dihydro-1-benzoxepine-4-carboxylic acid (0.44g) as colorless crystals.
mp >300°C.
¹H-NMR (δ ppm, DMSO-d₆) : 2.89 (2H, t, J=4.5Hz), 4.26 (2H, t, J=4.5Hz), 7.07 (1H, d, J=8.4Hz), 7.13 (1H, s), 7.55-7.68 (3H, m), 7.73 (2H, d, J=8.8Hz), 7.81 (1H, s), 7.85 (2H, d, J=8.8Hz), 8.33 (1H, s).

| Anal. for C₂₀H₁₆N₂O₃·0.3H₂O: | | | |
|---|---|---|---|
| Calcd. | C,71.12; | H,4.95; | N,8.29. |
| Found | C,71.15; | H,4.84; | N,8.21. |

### Reference Example 100

In 1,2-dichloroethane (100ml) was suspended p-nitrobenzylamine hydrochloride (8.1g), 4H-tetrahydrothio-pyran-4-one (5.0g) and triethylamine (6ml), and to the suspension was added sodium triacetoxy boron hydride (12.8g) under ice-cooling. Under nitrogen atmosphere, the mixture was stirred at room temperature for 9 hours. Under ice-cooling, to the mixture were added 37% formalin (3.9ml) and sodium triacetoxy boron hydride (12.8g). Under nitrogen atmosphere, the mixture was stirred at room temperature over night. The solvent was evaporated, and the residue was neutralized with sodium hydroxide. The mixture was extracted with ethyl acetate. The organic layer was washed with water and saturated sodium chloride solution, and dried with anhydrous magnesium sulfate. Under reduced pressure, the solvent was evaporated to give yellow oil (11.5g), to which were added reduced iron (12g) and acetic acid (200ml). The mixture was stirred at room temperature over night. The solvent was evaporated, and to the residue was added ethyl acetate. The precipitate was filtered off, and the filtrate was washed with sodium hydrogen carbonate solution, water and saturated sodium chloride solution, and dried with anhydrous magnesium sulfate. Under reduced pressure, the solvent was evaporated, and the residue was purified with silica gel column (ethyl acetate/methanol/triethylamine) to give 4-(N-methyl-N-(tetrahydrothiopyran-4-yl)amino-methyl)an iline (8.8g) as pale yellow crystals.
mp 88-89°C.
¹H-NMR(δppm, CDCl₃) : 1.65-1.84 (2H, m), 2.10-2.18 (2H, m), 2.19 (3H, s), 2.45 (1H, tt, J=3.2, 13.0Hz), 2.65-2.71 (4H, m), 3.47 (2H, s), 3.61 (2H, br), 6.64 (2H, d, J=8.4Hz), 7.08 (2H, d, J=8.4Hz).
IR(KBr) ν : 2932, 1620cm⁻¹.

| Anal. for C₁₃H₂₀N₂S : | | | |
|---|---|---|---|
| Calcd. | C,66.06; | H,8.53; | N,11.85. |
| Found | C,66.03; | H,8.35; | N,11.78. |

### Reference Example 101

A mixture of sodium methoxide (12.5g) and dimethyl carbonate (150ml) was added to 3-bromo-6,7,8,9-tetra-hydro-5H-benzocycloheptan-5-one (10.8g), and the mixture was refluxed for 8 hours under nitrogen atmosphere. Under ice-cooling, the mixture was poured into 1N hydrochloric acid, and the mixture was extracted with ethyl acetate. The organic layer was washed with and saturated sodium chloride solution, and dried with anhydrous magnesium sulfate. The solvent was evaporated to give brown oil (13.1g), which was dissolved in dichloromethane (150ml). To the mixture was dropwise added sodium boron hydride dissolved in methanol,underice-cooling. After starting materials disappeared, water was added to the reaction mixture, and the mixture was concentrated and extracted with ethyl acetate. The organic layer was washed with and saturated sodium chloride solution, and dried with anhydrous magnesium sulfate. The solvent was evaporated, and the residue was dissolved in dichloromethane (150ml). To the mixture was added triethylamine (29ml), and to the mixture was dropwise added methane-sulfonylchloride (5.3ml) under ice-cooling. The mixture was stirred at room temperature under nitrogen atmosphere over night, and to the mixture was added water. The mixture was concentrated and extracted with ethyl acetate. The organic layer was washed with and saturated sodium chloride solution, and dried with anhydrous magnesium sulfate. The solvent was evaporated, and the residue was purified with silica gel column (ethyl acetate/hexane) to give methyl 2-bromo-6,7-dihydro-5H-benzo-cycloheptene-8-carboxylate (1.7g) as colorless crystals.
mp 83-84°C.
¹H-NMR (δ ppm, CDCl₃) : 1.97-2.10 (2H, m), 2.62 (2H, t, J=6.6Hz), 2.72-2.78 (2H, m), 3.82 (3H, s), 7.02 (1H, d, J=8.0Hz), 7.32 (1H, dd, J=2.2, 8.0Hz), 7.45 (1H, d, J=2.2Hz), 7.60 (1H, s).
IR(KBr) ν : 2946, 1713cm⁻¹.

| Anal. for C₁₃H₁₃BrO₂ : | | |
|---|---|---|
| Calcd. | C,55.54; | H,4.66. |
| Found | C,55.56; | H,4.75. |

### Reference Example 102

To a mixture of methyl 2-bromo-6,7-dihydro-5H-benzocycloheptene-8-carboxylate (0.5g), 4-piperidinophenyl borate (0.4g), 1M potassium carbonate (6ml) and ethanol (6ml) was added toluene (50ml), and the mixture was stirred under argon atmosphere at room temperature for 30 minutes. To the mixture was added tetrakistriphenyl-phosphinepalladium (0.08g), and the mixture was refluxed over night and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate/hexane) to give colorless crystals (0.45g), which were dissolved in 1N sodium hydroxide (15ml), methanol (50ml) and tetrahydrofuran (50ml). The mixture was refluxed at room temperature for 2 hours, concentrated and neutralized with hydrochloric acid to precipitate 2-(4-piperidino-phenyl)-6,7-dihydro-5H-benzocyclohepten e-8-carboxylic acid (0.46g) as colorless crystals.
mp 219-220°C (dec.).
¹H-NMR(δppm, DMSO-d₆): 1.50-1.70 (6H, m), 1.85-2.05 (2H, m), 2.56 (2H, t, J=6.4Hz), 2.80-2.82 (2H, s), 3.13-3.25 (4H, m), 6.99 (2H, d, J=8.7Hz), 7.23 (1H, d, J=8.0Hz), 7.47 (1H, dd, J=1.8, 8.0Hz), 7.54 (2H, d, J=8.7Hz), 7.60 (1H, d, J=1.8Hz), 7.70 (1H, s).

| Anal. for C₂₃H₂₅NO₂·0.2H₂O: | | | |
|---|---|---|---|
| Calcd. | C,78.69; | H,7.29; | N,3.99. |
| Found | C,78.82; | H,7.38; | N,3.89. |

### Reference Example 103

To a mixture of N-t-butoxycarbonylpiperidin-4-one (3g ; M. S. Ashwood et al., J. Chem. Soc. Perkin Trans. 1, 1995, 641-644) and methylamine hydrochloride (1g) were added triethylamine (2.1ml) and 1,2-dichloroethane(50ml). Under ice-cooling, to the mixture was added sodium triacetoxy boron hydride (4.5g), and the mixture was stirred under nitrogen atmosphere at room temperature for 4 hours. The mixture was neutralized with sodium hydroxide, concentrated and extracted with ethyl acetate. The organic layer was washed with saturated sodium chloride solution and dried with anhydrous magnesium sulfate. Under reduced pressure, the solvent was evaporated to give 1-t-butoxy-carbonyl-4-methylaminopiperidine (3.1g) as colorless oil.
¹H-NMR(δ ppm, CDCl₃): 1.13-1.33 (3H, m), 1.33-1.54 (3H, m), 1.45 (9H, s), 1.83-1.88 (2H, m), 2.44 (3H, s), 2.44-2.56 (1H, m), 2.73-2.87 (2H, m), 4.01 (1H, br).

### Reference Example 104

In chlorobenzene (100ml) was dissolved 2-bromo-4'-acetophenone (25.1g), and the mixture was dropwise added to a suspension of hexamethylenetetramine (15.9g) in chlorobenzene (100ml). The mixture was stirred under nitrogen atmosphere at 60°C for 4 hours and cooled to precipitate crystals, which were filtered and washed with ethanol and diethylether. The resulting crystals were added little by little to a mixture of 95% ethanol (100ml) and hydrochloric acid (50ml), and the mixture was stirred at room temperature over night. Precipitated crystal was filtered and washed with diethylether. To the crystal was added di-t-butyl bicarbonate (32g), triethylamine (29ml) and dichloromethane (500ml), and the mixture was stirred at room temperature for 2 hours, washed with water, 10% citric acid and water, and dried with anhydrous magnesium sulfate. Under reduced pressure, the solvent was evaporated, and the residue was purified with silica gel column (ethyl acetate/hexane) to give yellow solid (24.9g), 12g of which was dissolved in ethanol (200ml) and ethyl acetate (50ml). To the mixture was added 10% palladium on carbon (1.2g) and catalytic hydrogenation was carried out at room temperature for 6 hours. The catalyst was filtered off, and the solvent was evaporated to give colorless crystals (6.5g), 4g of which was dissolved in dimethylformamide (50ml). To the mixture was added sodium hydride (60%, 1.4g) at -3°C, and the mixture was stirred for 20 minutes. To the mixture was dropwise added 1,4-dibromobutane (2.1ml), and the mixture was stirred under ice-cooling for 1.5 hours. To the mixture was ammonium chloride solution, and the mixture was extracted with ethyl acetate. The organic layer was washed with water and saturated sodium chloride solution, and dried with anhydrous magnesium sulfate. Under reduced pressure, the solvent was evaporated, (4-aminophenyl)[1-(tert-butoxy-carbonyl)piperidin-2-yl] methanone (2.1g) as pale yellow crystals.
mp 187-188°C.
¹H-NMR(δppm, CDCl₃) : 1.42 (9H, br), 1.43 (2H, br), 1.80 (1H, br), 2.05 (1H, br), 3.22 (1H, br), 3.95 (1H, br), 4.09 (2H, br), 5.55 (1H, br), 6.63 (2H, d, J=8.4Hz), 7.79 (2H, d, J=8.4Hz).
IR(KBr) ν : 3362, 2942, 1682cm⁻¹.

| Anal. for C₁₇H₂₄N₂O₃·0.1H₂O: | | | |
|---|---|---|---|
| Calcd. | C,66.69; | H,7.97; | N,9.15. |
| Found | C,66.60; | H,7.91; | N,8.87. |

### Reference Example 105

A mixture of 2- (4-nitrobenzyl) pyridine (J. Chem. Soc., p549, 1929) (1.50g) and 5% Pd-C (0.15g) in ethanol (30ml) was vigorously stirred under hydrogen atmosphere for 8 hours, and the Pd-C was filtered off. The filtrate was concentrated under reduced pressure, and the residue was separated and purified with column chromatography (ethyl acetate/hexane =1:1→2:1) to give 2-(4-aminobenzyl)-pyridine (1.09g) as yellow oil.
¹H-NMR (200MHz, CDCl₃) δ 3.41-3.75 (2H, m), 4.05 (2H, s), 6.50-6.69 (2H, m), 6.97-7.16 (4H, m), 7.51-7.60 (1H, m), 8.48-8.57 (1H, m).
IR (neat) 3338, 3213, 3008, 1622, 1593, 1516, 1471, 1433, 1281, 754 cm⁻¹

### Reference Example 106

Under nitrogen atmosphere, to a solution of ethyl magnesium chloride in tetrahydrofuran (1.58M, 95ml) was added diethyl phosphite (6.91g) under ice-cooling, and the mixture was stirred at room temperature for 1 hour. To the mixture was added benzyl bromide (7.2ml), and the mixture was refluxed for 4 hours. The reaction mixture was vigorously stirred and concentrated hydrochloric acid-ice was added to the mixture to stop the reaction. The mixture was extracted with diethylether and concentrated. To the residue was added chloroform, and the mixture was washed with water and concentrated under reduced pressure. The residue was separated and purified with column chromatography (ethyl acetate/ethanol=3:1→2:1) to give benzyldiethylphosphine oxide (1.45g) as colorless crystals.
¹H-NMR (200MHz, CDCl₃) δ 1.17 (6H, dt, J=16.6, 8.0 Hz), 1.57-1.75 (4H, m), 3.14 (2H, d, J=14.4 Hz), 7.19-7.40 (4H, m).
IR (KBr) 3396, 2974, 16445, 1495, 1458, 1410, 1242, 1159, 1124, 1034, 829, 789, 702 cm⁻¹

### Reference Example 107

To a mixture of nitric acid (0.4ml) and concentrated sulfuric acid (3ml) was added benzyldiethylphosphine oxide (1.05g) at 0°C, and the mixture was stirred at 50°C for 1 hour. The reaction mixture was added to ice-water, and ammonia solution was added to the solution to neutralize the solution, which was extracted with ethyl acetate. The organic layer was washed with saturated sodium chloride solution, dried with magnesium sulfate and concentrated. The residue was separated and purified with column chromatography (ethyl acetate/ethanol=3:2→1:1) to give 4-nitrobenzyldiethylphosphine oxide (518mg) as pale yellow crystals.
¹H-NMR (200MHz, CDCl₃) δ 1.18 (6H, dt, J=17.0, 8.0 Hz), 1.64-1.86 (4H, m), 3.23 (2H, d, J=13.6 Hz), 7.49 (2H, dd, J=8.8, 1.6 Hz), 8.20 (2H, d, J=8.8 Hz).
IR (KBr) 1599, 1506, 1340, 1169, 864, 773, 694, 501 cm⁻¹

### Reference Example 108

A mixture of 4-nitrobenzyldiethylphosphine oxide (0.4g) and 10% Pd-C (0.06g) in ethanol (10ml) was vigorously stirred under hydrogen atmosphere for 16 hours, and the Pd-C was filtered off. The filtrate was concentrated under reduced pressure to give 4-aminobenzyldiethylphosphine oxide (349mg) as brown oil.
¹H-NMR (200MHz, CDCl₃) δ 1.16 (6H, dt, J=16.6, 7.8 Hz), 1.56-1.76 (4H, m), 3.02 (2H, d, J=14.4 Hz), 6.64 (2H, d, J=8.4 Hz), 7.03 (2H, dd, J=8.4, 1.8 Hz).
IR (neat) 3336, 1630, 1614, 1516, 1460, 1408, 1284, 1157, 1126, 841, 791, 768, 540 cm⁻¹

### Reference Example 109

Under nitrogen atmosphere, to a solution of propyl magnesium bromide in tetrahydrofuran (2M, 250g) was added diethyl phosphite (18.0g) under ice-cooling, and the mixture was stirred at room temperature for 3 hours. To the reaction mixture was added benzyl bromide (24.7ml), and the mixture was refluxed for 5 hours. The reaction mixture was vigorously stirred and added to concentrated hydrochloric acid-ice to stop the reaction. The mixture was extracted with ethyl acetate and concentrated. The residue was separated and purified with column chromatography (ethyl acetate→ethyl acetate/ethanol=3:1) to give benzyldipropylphosphine oxide (25.33g) as colorless crystals.
¹H-NMR (200MHz, CDCl₃) δ 0.94-1.09 (6H, m), 1.49-1.75 (8H, m), 3.15 (2H, d, J=14.6 Hz), 7.19-7.39 (5H, m).
IR (KBr) 3425, 2964, 1645, 1603, 1497, 1456, 1242, 1161, 1126, 1080, 1030, 843 cm⁻¹

### Reference Example 110

To a mixture of nitric acid (3.6ml) and concentrated sulfuric acid (22ml) was added benzyldipropylphosphine-oxide (10.75g) at 0°C, and the mixture was stirred at 60°C for 1.5 hours. The reaction mixture was added to ice-water, and ammonia solution was added to the mixture to neutralize the solution, which was extracted with ethyl acetate. The organic layer was washed with saturated sodium chloride solution, dried with magnesium sulfate and concentrated. The residue was separated and purified with column chromatography (ethyl acetate/ethanol= 9:1→14:1) to give 4-nitrobenzyldipropylphosphine oxide (3.77g) as pale yellow crystals.
¹H-NMR (200MHz, CDCl₃) δ 0.96-1.09 (6H, m), 1.51-1.75 (8H, m), 3.20 (2H, d, J=13.6 Hz), 7.47 (2H, dd, J=8.8, 2.0 Hz), 8.21 (2H, d, J=8.8 Hz).
IR (KBr) 1527, 1431, 1352, 1028 cm⁻¹

### Reference Example 111

A mixture of 4-nitrobenzyldipropylphosphine oxide (3.0g) and 5% Pd-C (0.3g)in ethanol (50ml) was vigorously stirred under hydrogen atmosphere for 16 hours, and the Pd-C was filtered off. The filtrate was concentrated under reduced pressure. The residue was separated and purified with column chromatography (ethanol/ethyl acetate=1:5→1:4) and recrystallized from ethanol-ethyl acetate to give 4-aminobenzyldipropylphosphine oxide (1.78g) as colorless crystals.
m.p. 104-106°C
¹H-NMR (200MHz, CDCl₃) δ 0.88-1.12 (6H, m), 1.43-1.72 (8H, m), 3.01 (2H, d, J=14.8 Hz), 3.52-3.76 (2H, m), 6.65 (2H, d, J=8.6 Hz), 7.01 (2H, dd, J=8.6, 2.0 Hz).
IR (KBr) 3348, 3209, 2058, 1608, 1512, 1155, 1126, 852 cm⁻¹

| Elemental Analysis for C₁₃H₂₂NOP | | | | |
|---|---|---|---|---|
| Calcd. | C, 65.25 ; | H, 9.27 ; | N, 5.85 ; | P, 12.94 : |
| Found. | C, 65.16 ; | H, 9.04 ; | N, 5.91 ; | P, 12.94. |

### Reference Example 112

Under nitrogen atmosphere, to a solution of 2-bromo-3-hydroxypyridine (10.00g) in DMF (100ml) was added sodium hydride (60% oil, 2.5g) at 0°C, and the mixture was stirred for 30 minutes. To the reaction mixture was added methyl iodide (4.0ml), and the mixture was stirred at room temperature for 2 hours. To the reaction mixture was added water, and the mixture was extracted with ethyl acetate. The organic layer was washed with saturated sodium chloride solution, dried with magnesium sulfate and concentrated. Under reduced pressure, the residue was separated and purified with column chromatography (ethyl acetate/hexane= 1:2) to give 2-bromo-3-methoxypyridine (9.24g) as colorless crystals.
m.p.41-43°C
¹H-NMR (200MHz, CDCl₃) δ 3.92 (3H, s), 7.15 (1H, dd, J=8.0, 1.4 Hz), 7.24 (1H, dd, J=8.0, 4.4 Hz), 7.99 (1H, dd, J=4.4, 1.4 Hz).
IR (KBr) 3055, 1562, 1468, 1414, 1298, 1205, 1078, 1049, 791, 667 cm⁻¹

| Elemental Analysis for C₆H₆NO | | | |
|---|---|---|---|
| Calcd. | C, 38.33 ; | H, 3.22 ; | N, 7.45 : |
| Found. | C, 38.35; | H, 3.07 ; | N, 7.28. |

### Reference Example 113

To a solution of 2-bromo-3-methoxypyridine (1.00g) in diethylether (20ml) was added a solution of n-butyllithium in hexane (1.6M, 3.7ml) at-78°C, and the mixture was stirred for 1 hour to prepare the lithium salt, which was dropwise added to a solution of 4-nitrobenzaldehyde (0.81g) in tetrahydrofuran (10ml) cooled at -78°C. The mixture was stirred at -78°C. To the reaction mixture was added water to stop the reaction, and the mixture was extracted with ethyl acetate. The organic layer was washed with saturated sodium chloride solution, dried with magnesium sulfate and concentrated. Under reduced pressure, the residue was separated and purified with column chromatography (ethyl acetate/hexane=1:3→1:1) to give 3-methoxypyridin-2-yl)-(4-nitrophenyl)methanol (742mg) as pale yellow crystals. m.p.137-138°C
¹H-NMR (200MHz, CDCl₃) δ 3.81 (3H, s), 5.64 (1H, d, J=6.8 Hz), 6.02 (1H, d, J=6.8 Hz), 7.17 (1H, dd, J=8.4, 1.4 Hz), 7.27 (1H, dd, J=8.4, 4.6 Hz), 7.58 (2H, dd, J=7.0, 2.0 Hz), 8.15 (2H, dd, J=7.0, 2.0 Hz), 8.21 (1H, dd, J=4.6, 1.4 Hz) .
IR (KBr) 3348, 1524, 1464, 1344, 1284, 1053, 1020, 837, 797, 744, 689 cm⁻¹

| Elemental Analysis for C₁₃H₁₂N₂O₄ | | | |
|---|---|---|---|
| Calcd. | C, 60.00 ; | H, 4.65 ; | N, 10.76 : |
| Found. | C, 59.97 ; | H, 4.57 ; | N, 10.82. |

### Reference Example 114

A mixture of (3-methoxypyridin-2-yl)-(4-nitro-phenyl)methanol (600mg) and 5% Pd-C (0.06g) in ethanol (20ml) was vigorously stirred under hydrogen atmosphere for 3 hours, and the Pd-C was filtered off. The filtrate was concentrated under reduced pressure to give (4-amino-phenyl)-(3-methoxypyridin-2-yl)-methanol (483mg) as pale yellow crystals.
¹H-NMR (200MHz, CDCl₃) δ 3.51-3.65 (2H, m), 3.75 (3H, s), 5.33 (1H, d, J=7.1 Hz), 5.85 (1H, d, J=7.1 Hz), 6.60 (2H, dd, J=6.6, 1.8 Hz), 7.08-7.23 (4H, m), 8.17 (1H, dd, J=4.6, 1.4 Hz) .
IR (KBr) 3458, 3463, 3323, 1626, 1614, 1518, 1454, 1427, 1279, 1178, 1038, 835, 804 cm-¹

### Reference Example 115

A solution of diethyl benzylphosphonate (25g) in methanol (10ml) and concentrated hydrochloric acid (500ml) solution was refluxed for 4 days. The mixture was cooled to room temperature, and precipitated crystal was collected by filtration to give benzylphosphonic acid (11.17g) as colorless crystals.
m.p. 171-173°C
¹H-NMR (200MHz, DMSO-d₆) δ 2.96 (2H, d, J=21.6 Hz), 7.13-7.34 (5H, m).
IR (KBr) 2779, 2330, 1497, 1458, 1263, 1074, 993, 943, 781, 694, 527, 428 cm⁻¹

| Elemental Analysis for C₇H₉O₃P | | | |
|---|---|---|---|
| Calcd. | C, 48.85 ; | H, 5.27 ; | P, 18.00 : |
| Found. | C, 48.75 ; | H, 5.01 ; | P, 17.78. |

### Reference Example 116

Under nitrogen atmosphere, to a mixture of magnesium (3.39g) and a piece of iodine in diethylether (16ml) was dropwise added a solution of 1,4-dibromobutane (5.55ml) and 1,2-dibromoethane (2ml) in diethylether (80ml) at 40°C for 1 hour. The mixture was refluxed for 1 hour, cooled to room temperature and allowed to stand for 2 hours. The upper layer of diethylether was removed through cannula, to obtain the di-Grignard reagent, which was dissolved in dichloro-methane (210ml). The resulting di-Grignard reagent as it is was used for the following reaction. To benzyl phosphonate (8.0g) was added thionyl chloride (40ml) and then 2 drops of DMF, and the mixture was refluxed for 4 hours and concentrated under reduced pressure. The residue was dissolved in dichloromethane (210ml), and the mixture was cooled to 0°C. To the mixture was dropwise added a solution of the above di-Grignard reagent in dichloromethane, which was cooled to 0°C, through cannula for 1 hour, and the mixture was stirred at room temperature for 16 hours. To the reaction mixture were added 10% ammonium chloride solution (100ml) and saturated sodium chloride solution, and the mixture was extracted with dichloromethane. The organic layer was washed with saturated sodium chloride solution, dried with magnesium sulfate and concentrated under reduced pressure. The residue was separated and purified with column chromatography (ethanol/ethyl acetate=1:4) to give 1-benzyl-phosphorane-1-oxide (4.83g) as colorless crystals.
¹H-NMR (200MHz, CDCl₃) δ 1.40-2.08 (8H, m), 3.27 (2H, d, J=15.0 Hz), 7.11-7.42 (5H, m).
IR (KBr) 2951, 1643, 1495, 1454, 1406, 1265, 1236, 1165, 1120, 702 cm⁻¹

### Reference Example 117

To 1-benzylphosphorane-1-oxide (4.17g) were added nitric acid (1.7ml) and sulfuric acid (11ml) at 0°C, and the mixture was stirred at 50-60°C for 2 hours. The reaction mixture was added to crushed ice and neutralized with ammonia solution. The mixture was extracted with ethyl acetate. The organic layer was washed with saturated sodium chloride solution, dried with magnesium sulfate and concentrated. Under reduced pressure, The residue was separated and purified with column chromatography (ethanol/ethyl acetate=1:4→1:1) to givel-(4-nitro-benzyl)phosphorane-l-oxide (2.22g) as yellow crystals.
¹H-NMR (200MHz, CDCl₃) δ 1.55-2.13 (8H, m), 3.32 (2H, d, J=13.8 Hz), 7.50 (2H, dd, J=8.8, 1.8 Hz), 8.22 (2H, d, J=8.8 Hz).
IR (KBr) 3402, 2954, 1514, 1346, 1171, 860, 700 cm⁻¹

### Reference Example 118

A mixture of 1-(4-nitrobenzyl)phosphorane-1-oxide (1.80g) and 10% Pd-C (0.2g) in ethanol (30ml) was vigorously stirred under hydrogen atmosphere for 24 hours, and the catalyst was filtered off. The filtrate was concentrated and purified with column chromatography (ethanol/ethyl acetate=1:2) and recrystallized from ethanol-diethylether to give 1-(4-aminobenzyl)phosphorane-1-oxide (0.90g) as colorless crystals.
¹H-NMR (200MHz, CDCl₃) δ 1.32-2.02 (8H, m), 3.16 (2H, d, J=14.6 Hz), 3.52-3.74 (2H, m), 6.65 (2H, d, J=8.4 Hz), 7.04 (2H, dd, J=8.4, 2.2 Hz).
IR (KBr) 3386, 3338, 3228, 1641, 1612, 1516, 1296, 1263, 1174, 1124, 833 cm⁻¹

### Reference Example 119

Under nitrogen atmosphere, to a solution of 2-bromo-3-methoxymethoxypyridine (10.00g) in diethylether (150ml) was added a solution of n-butyllithium in hexane (1.6M, 31.5ml) at -78°C, and the mixture was stirred for 1 hour to prepare the lithium salt. The resulting lithium salt was dropwise added to a solution of 4-nitrobenzaldehyde (6.93g) in tetrahydrofuran (100ml) cooled at -78°C, and the mixture was stirred at the same temperature for 3 hours. To the reaction mixture was added water to stop the reaction, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=1:3→1:2) to give (3-methoxymethoxypyridin-2-yl)-(4-nitrophenyl)-methanol (11.78g) as yellow oil.
¹H-NMR (200MHz, CDCl₃) δ 3.27 (3H, s), 5.12 (1H, d, J=7.0 Hz), 5.20 (1H, d, J=7.0 Hz), 5.70 (1H, d, J=7.0 Hz), 6.02 (1H, d, J=7.0 Hz), 7.25 (1H, dd, J=8.4, 4.4 Hz), 7.42 (1H, dd, J=8.4, 1.4 Hz), 7.58 (2H, d, J=8.8 Hz), 8.15 (2H, d, J=8.8 Hz), 8.27 (1H, dd, J=4.4, 1.4 Hz).
IR (neat) 3390, 1522, 1448, 1348, 1155, 1084, 1055, 980, 824, 849, 800, 744, 700 cm⁻¹

### Reference Example 120

A mixture of (3-methoxymethoxypyridin-2-yl)-(4-nitrophenyl)methanol (11.78g) and 10% Pd-C (1.2g) inethanol (100ml) was vigorously stirred under hydrogen atmosphere for 24 hours. The catalyst was filtered of, and the filtrate was concentrated under reduced pressure. The residue was separated and purified with column chromatography (ethyl acetate/hexane=1:1→2:1) to give 2-(4-aminobenzyl)-3-methoxymethoxypyridine (2.92g) as orange oil.
¹H-NMR (200MHz, CDCl₃) δ 3.37 (3H, s), 4.08 (2H, s), 5.16 (2H, s), 6.59 (2H, dd, J=8.4, 2.0 Hz), 7.04-7.19 (3H, m), 7.33 (1H, dd, J=8.4, 1.2 Hz), 8.18 (1H, dd, J=4.8, 1.2 Hz). IR (neat) 3433, 3352, 3219, 1620, 1514, 1446, 1265, 1153, 1082, 985, 922, 798 cm⁻¹

### Reference Example 121

Under nitrogen atmosphere, to a mixture of magnesium (3.2g) and a piece of iodine in diethylether (20ml) was dropwise added to a solution of 1,5-dibromopentane (13.21g) and 1,2-dibromoethane (1.21ml) in diethylether (80ml) at 40°C for 1 hour. The mixture was refluxed for 1 hour, cooled to room temperature and allowed to stand for 2 hours. The upper layer of diethylether was removed through cannula, to obtain the di-Grignard reagent, which was dissolved in dichloromethane (250ml). The resulting di-Grignardreagent as it is was used for the following reaction. To benzylphosphonic acid (10.0g) was added thionyl chloride (30ml) and then a drop of DMF, and the mixture was refluxed for 3 hours and concentrated under reduced pressure. The residue was dissolved in dichloromethane (210ml), and the mixture was cooled to 0°C. To the mixture was dropwise added a solution of the above di-Grignard reagent in dichloromethane, which was cooled to 0°C, through cannula for 1 hour, and the mixture was stirred at room temperature for 20 hours. To the reaction mixture were added 10% ammonium chloride solution (100ml) and saturated sodium chloride solution, and the mixture was extracted with dichloromethane. The organic layer was washed with saturated sodium chloride solution, dried with magnesium sulfate and concentrated under reduced pressure. The residue was separated and purified with column chromatography (ethanol/ethyl acetate=1:3→1:2) to give 1-benzylphosphorinane-1-oxide (5.39g) as colorless crystals.
¹H-NMR (200MHz, CDCl₃) δ 1.36-2.18 (10H, m), 3.17 (2H, d, J=14.0 Hz), 7.23-7.42 (5H, m).
IR (KBr) 2939, 2912, 2886, 1493, 1452, 1404, 1232, 1161, 827, 700 cm⁻¹

### Reference Example 122

To a solution of diethyl benzylphosphonate (2.5g) in tetrahydrofuran (500ml) was added Red-Al (70% toluene solution) (3.8g) at room temperature, and the mixture was stirred until gas production stopped. To the reaction mixture was added 1,5-dibromopentane (25.18g), and the mixture was stirred at 50-60°C for 16 hours. To the reaction mixture was added water (20ml), and precipitate was removed by filtration. The filtrate was concentrated under reduced pressure, and the residue was separated and purified with column chromatography (ethyl acetate→ethanolethyl acetate=1:2) to give 1-benzylphosphorinane-1-oxide (8.41g) as colorless crystals.
¹H-NMR (200MHz, CDCl₃) δ 1.36-2.18 (10H, m), 3.17 (2H, d, J=14.0 Hz), 7.23-7.42 (5H, m).
IR (KBr) 2939, 2912, 2886, 1493, 1452, 1404, 1232, 1161, 827, 700 cm⁻¹

### Reference Example 123

To 1-benzylphosphorinane-1-oxide (5.39g) were added nitric acid (1.94ml) and sulfuric acid (15ml) at 0°C, and the mixture was stirred at 50-60°C for 2 hours. The reaction mixture was added to crushed ice-water, neutralized with ammonia solution and extracted with ethyl acetate. The organic layer was washed with saturated sodium chloride solution, dried with magnesium sulfate and concentrated under reduced pressure. The residue was separated and purified with column chromatography (ethanol/ethyl acetate=1:3→1:2) to give 1-(4-nitrobenzyl)-phosphorinane-1-oxide (2.47g)as pale yellow crystals.
¹H-NMR (200MHz, CDCl₃) δ 1.46-2.18 (10H, m), 3.28 (2H, d, J=13.6 Hz), 7.48 (2H, dd, J=8.8, 2.2 Hz), 8.21 (2H, d, J=8.8 Hz).
IR (KBr) 2926, 1599, 1516, 1348, 1230, 1159, 1132, 864, 822, 696 cm⁻¹

### Reference Example 124

A mixture of 1-(4-nitrobenzyl)phosphorinane-l-oxide (2.25g) and 10% Pd-C (0.2g) in ethanol (30ml) was vigorously stirred under hydrogen atmosphere for 24 hours. The catalyst was filtered off, and the filtrate was concentrated recrystallized from ethanol-diethylether to give 1-(4-aminobenzyl)-phosphorinane-1-oxide (1.5g) as pale yellow crystals.
¹H-NMR (200MHz, CDCl₃) δ 1.27-2.16 (10H, m), 3.06 (2H, d, J=13.8 Hz), 3.53-3.80 (2H, m), 6.65 (2H, d, J=8.3 Hz), 7.05 (2H, dd, J=8.3, 2.0 Hz).
IR (KBr) 3386, 3334, 3224, 2939, 1639, 1612, 1514, 1296, 1225, 1153, 1120, 841 cm⁻¹

### Reference Example 125

Under argon atmosphere, to a solution of 4-ethylbromobenzene (10.0g) in tetrahydrofuran (60ml) was added n-butyllithium (1.6M hexane solution) (37.2ml) at -78°C, and the mixture was stirred for 1 hour. To the reaction mixture was dropwise added a solution of tributyl borate (13.68g) in tetrahydrofuran (30ml), and the reaction mixture was warmed to room temperature and stirred at room temperature for 2 hours. To the reaction mixture was added 10% sulfuric acid (100ml), and the mixture was stirred for 1 hour. The mixture was extracted with ethyl acetate. The organic layer was washed with saturated sodium chloride solution, dried with magnesium sulfate and concentrated under reduced pressure. The residue was dissolved in acetone (30ml), and to the mixture was added 10% sulfuric acid (50ml). The mixture was stirred at room temperature for 16 hours, and under reduced pressure acetone was evaporated. The mixture was extracted with ethyl acetate. The organic layer was washed with saturated sodium chloride solution, dried with magnesium sulfate and concentrated under reduced pressure. The residue was separated and purified with column chromatography (ethyl acetate/hexane= 1 :2) to give crude 4-ethylphenyl borate (0.91g) as colorless solid. Under argon atmosphere, a solution of ethyl 7-bromo-2,3-dihydro-1-benzoxepine-4-carboxylate (500mg), the above crude 4-ethylphenyl borate (0.32g) and potassium carbonate (0.49g) in toluene-ethanol-water (20-2-2ml) was stirred at room temperature for 1 hour. To the reaction mixture was added tetrakistriphenyl-phosphinepalladium (0.06g), and the mixture was refluxed for 18 hours and cooled to room temperature. The organic layer was washed with saturated sodium chloride solution, dried with magnesium sulfate and concentrated under reduced pressure. The residue was separated and purified with column chromatography (ethyl acetate/hexane=1:15) to give ethyl 7-(4-ethylphenyl)-2,3-dihydro-1-benzoxepine-4-carboxyla te (464mg) as colorless crystals.
m.p. 81-83°C
¹H-NMR (200MHz, CDCl₃) δ 6 1.28 (3H, t, J=7.6 Hz), 1.36 (3H, t, J=7.2 Hz), 2.69 (2H, q, J=7.6 Hz), 3.00 (2H, t, J=5.2 Hz), 4.29 (2H ,q, J=7.2 Hz), 4.30 (2H, t, J=5.2 Hz), 7.04 (1H, d, J=8.4 Hz), 7.27 (2H, d, J=8.6 Hz), 7.44-7.51 (3H, m), 7.55 (1H, d, J=2.6 Hz), 7.65 (1H, br s).
IR (KBr) 1699, 1493, 1302, 1254, 1213, 1012, 822 cm⁻¹

| Elemental Analysis for C₂₁H₂₂O₃ | | |
|---|---|---|
| Calcd. | C, 78.23 ; | H, 6.88 : |
| Found. | C, 78.05 ; | H, 6.61. |

### Reference Example 126

To a solution of ethyl 7-(4-ethylphenyl)-2,3-dihydro-1-benzoxepine-4-carboxyla te (430mg) in ethanol (20ml) was added 1N sodium hydroxide (4.0ml) at room temperature, and the mixture was stirred for 24 hours and concentrated under reduced pressure. To the residue was added 1N hydrochloric acid (15ml), and the mixture was extracted with ethyl acetate. The organic layer was washed with saturated sodium chloride solution, dried with magnesium sulfate and concentrated to give crystals, which were collected by filtration to give 7-(4-ethylphenyl)-2,3-dihydro-1-benzoxepine-4-carboxyli c acid (328mg) as colorless crystals.
m.p. 241-243°C
¹H-NMR (200MHz, CDCl₃) δ 1.28 (3H, t, J=7.8 Hz), 2.70 (2H, q, J=7.8 Hz), 3.02 (2H, t, J=4.8 Hz), 4.33 (2H, t, J=4.8 Hz), 7.05 (1H, d, J=8.4 Hz), 7.27 (2H, d, J=8.0), 7.46-7.56 (4H, m), 7.78 (1H, br s) .
IR (KBr) 2966, 1689, 1491, 1437, 1263, 1230, 822 cm⁻¹

| Elemental Analysis for C₁₉H₁₈O₃ | | |
|---|---|---|
| Calcd. | C, 77.53 ; | H, 6.16 : |
| Found. | C, 77.52 ; | H, 6.27. |

### Reference Example 127

Under argon atmosphere, to a solution of 4-tert-butyl-bromobenzene (10.0g) in diethylether (50ml) was added n-butyllithium (1.6M, hexane solution) (32.3ml) at -78°C, and the mixture was stirred for 1 hour. To the reaction mixture was dropwise added trimethyl boric acid (16ml) in diethylether (30ml), and the mixture was warmed to room temperature and stirred at room temperature 16 hours. To the reaction mixture were added 1N hydrochloric acid (50ml) and water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane= 1:9) to give crude 4-tert-phenyl borate (0.84g) aspaleyellow oil. Under argon atmosphere, a solution of ethyl 7-bromo-2,3-dihydro-1-benzoxepine-4-carboxylate (500mg), the above crude 4-tert-butylphenyl borate(0.59g) and potassium carbonate (0.47g) in toluene-ethanol-water (20-2-2ml) was stirred at room temperature for 1 hour. To the reaction mixture was added tetrakistriphenylphosphine palladium (0.06g), and the mixture was refluxed for 20 hours and cooled to room temperature. The organic layer was washed with saturated sodium chloride solution, dried with magnesium sulfate and concentrated under reduced pressure. The residue was separated and purified with column chromatography (ethyl acetate/hexane=1:19) to give ethyl 7-(4-tert-butyl-phenyl)-2,3-dihydro-1-benzoxepine-4-car boxylate (504mg) as colorless oil.
¹H-NMR (200MHz, CDCl₃) δ 1.36 (9H, s), 1.36 (3H, t, J=7.2 Hz), 3.00 (2H, t, J=4.7 Hz), 4.29 (2H, q, J=7.2 Hz), 4.30 (2H, t, J=4.7 Hz), 7.04 (1H, d, J=8.2 Hz), 7.42-7.56 (6H, m), 7.65 (1H, br s).
IR (neat) 1731, 1491, 1298, 1246, 1211, 1184, 1090, 1018, 824 cm⁻¹

### Reference Example 128

To a solution of ethyl 7-(4-tert-butylphenyl)-2,3-dihydro-1-benzoxepine-4-carb oxylate (503.8mg) in ethanol (10ml)was added 1N sodium hydroxide (2.Om) at room temperature, and the mixture was stirred for 64 hours and concentrated under reduced pressure. To the residue was added 1N hydrochloric acid (15ml), and the mixture was extracted with ethyl acetate. The organic layer was washed with saturated sodium chloride solution, dried with magnesium sulfate and concentrated. The resulting crystal was collected by filtration to give 7-(4-tert-butyl-phenyl)-2,3-dihydro-1-benzoxepine-4-car boxylic acid (396mg) as colorless crystals.
m.p. 259-261°C
¹H-NMR (200MHz, CDCl₃) δ 1.37 (9H, s), 3.03 (2H, t, J=4.4 Hz), 4.34 (2H, t, J=4.4Hz), 7.06 (1H, d, J=8.4 Hz), 7.41-7.58 (6H, m), 7.79 (1H, br s).
IR (KBr) 2951, 1678, 1489, 1263, 829, 820 cm⁻¹

| Elemental Analysis for C₂₁H₂₂O₃ | | |
|---|---|---|
| Calcd. | C, 78.23 ; | H, 6.88 _{:} |
| Found. | C, 78.10 ; | H, 6.81. |

### Reference Example 129

Under argon atmosphere, a solution of ethyl 7-bromo-2,3-dihydro-1-benzoxepine-4-carboxylate (500mg), 4-chloro-phenyl borate (289mg) and potassium carbonate (464mg) in toluene-ethanol-water (20-2-2ml) was stirred at room temperature for 1 hour. To the reaction mixture was added tetrakistriphenyl-phosphinepalladium (0.06g), and the mixture was refluxed for 24 hours and cooled to room temperature. The organic layer was washed with saturated sodium chloride solution, dried with magnesium sulfate and concentrated under reduced pressure. The residue was separated and purified with column chromatography (ethyl acetate/hexane=1:19) to give ethyl 7-(4-chlorophenyl)-2,3-dihydro-1-benzoxepine-4-carboxyl ate (459mg) as colorless crystals.
m.p. 131-134°C
¹H-NMR (200MHz, CDCl₃) δ 1.36 (3H, t, J=7.2 Hz), 3.01 (2H, t, J=5.0 Hz), 4.23-4.34 (4H, m), 7.05 (1H, d, J=8.4 Hz), 7.37-7.52 (6H, m), 7.64 (1H, s).
IR (KBr) 1705, 1485, 1302, 1255, 1213, 820 cm⁻¹

| Elemental Analysis for C₁₉H₁₇O₃Cl | | | |
|---|---|---|---|
| Calcd. | C, 69.41 ; | H, 5.21 ; | Cl, 10.78 : |
| Found. | C, 69.16 ; | H, 5.12 ; | Cl, 10.85. |

### Reference Example 130

To a solution of ethyl 7-(4-chlorophenyl)-2,3-dihydro-1-benzoxepine-4-carboxyl ate (400mg) in tetrahydrofuran-ethanol (10-10ml) was added 1N sodium hydroxide (2.0ml) at room temperature, and the mixture was stirred for 42 hours and concentrated under reduced pressure. To the residue was added 1N hydrochloric acid (15ml), and the mixture was extracted with ethyl acetate. The organic layer was washed with saturated sodium chloride solution, dried with magnesium sulfate and concentrated. The resulting crystal was collected by filtration to give 7-(4-chlorophenyl)-2,3-dihydro-1-benzoxepine-4-carboxyl ic acid (342mg) as colorless crystals.
m.p. 263-264°C
¹H-NMR (200MHz, CDCl₃) δ 3.03 (2H, t, J=4.7 Hz), 4.34 (2H, t, J=4.7 Hz), 7.07 (1H, d, J=8.4 Hz), 7.35-7.55 (6H, m), 7.76 (1H, br s).
IR (KBr) 2959, 1680, 1483, 1267, 1230, 818 cm⁻¹

| Elemental Analysis for C₁₇H₁₃O₃Cl | | | |
|---|---|---|---|
| Calcd. | C, 69.89 ; | H, 4.36 ; | Cl, 11.79 : |
| Found. | C, 67.55 ; | H, 4.19 ; | Cl, 11.46. |

### Reference Example 131

Under argon atmosphere, a solution of ethyl 7-bromo-2,3-dihydro-1-benzoxepine-4-carboxylate (500mg), 4-tri-fluoromethylphenyl borate (351.5mg) and potassium carbonate .(0.47g) in toluene-ethanol-water (20-2-2ml) was stirred at room temperature for 1 hour. To the reaction mixture was added tetrakistriphenylphosphinepalladium (0.06g), and the mixture was refluxed for 20 hours and cooled to room temperature. The organic layer was washed with saturated sodium chloride solution, dried with magnesium sulfate and concentrated under reduced pressure. The residue was separated and purified with column chromatography (ethyl acetate/hexane=1:10) to give ethyl 7-(4-trifluoromethylphenyl)-2,3-dihydro-1-benzoxepine-4 -carboxylate (489mg) as colorless crystals.
m.p. 107-110°C
¹H-NMR (200MHz, CDCl₃) δ 1.37 (3H, t, J=7.2 Hz), 2.99-3.05 (2H, m), 4.29 (2H, q, J=7.2 Hz), 4.33 (2H, t, J=4.8 Hz), 7.09 (1H, d, J=8.4 Hz), 7.49 (1H, dd, J=8.4, 2.4 Hz), 7.58 (1H, d, J=2.4 Hz), 7.62-7.73 (5H, m).
IR (KBr) 1701, 1329, 1257, 1126, 1107, 1068, 1012, 822 cm⁻¹

| Elemental Analysis for C₂₀H₁₇O₃F₃ | | | |
|---|---|---|---|
| Calcd. | C, 66.30 ; | H, 4.73 ; | F, 15.73 : |
| Found. | C, 66.40 ; | H, 4.63 ; | F, 15.44. |

### Reference Example 132

To a solution of ethyl 7-(4-trifluoromethylphenyl)-2,3-dihydro-1-benzoxepine-4-carboxylate (440mg) in tetrahydrofuran-ethanol (10-10ml) was added 1N sodium hydroxide (4.0ml) at room temperature, and the mixture was stirred for 20 hours and concentrated under reduced pressure. To the residue was added 1N hydrochloric acid (5ml), and the mixture was extracted with ethyl acetate. The organic layer was washed with saturated sodium chloride solution, dried with magnesium sulfate and concentrated. The resulting crystal was collected by filtration to give 7-(4-trifluoromethylphenyl)-2,3-dihydro-1-benzoxepine-4 -carboxylic acid (392mg) as colorless crystals.
m.p. 273-276°C
¹H-NMR (200MHz, DMSO-d₆) δ 2.89 (2H, t, J=4.4 Hz), 4.28 (2H, t, J=4.4 Hz), 7.09 (1H, d, J=8.4 Hz), 7.61-7.70 (2H, m), 7.78 (2H, d, J=8.4 Hz), 7.92-7.96 (3H, m).
IR (KBr) 2979, 1689, 1329, 1263, 1134, 1072, 831 cm⁻¹

| Elemental Analysis for C₁₈H₁₃O₃F₃ | | |
|---|---|---|
| Calcd. | C, 64.67 ; | H, 3.92 : |
| Found. | C, 64.62 ; | H, 3.89. |

### Reference Example 133

Under argon atmosphere, to a solution of 4-bromo-phenetole (26.4g) in tetrahydrofuran (200ml) was dropwise added n-butyl-lithium (1.6M, hexane solution) (90.3ml) at -78°C for 50 minutes, and the mixture was stirred for 30 minutes. To the reaction mixture was dropwise added a solution of trimethyl borate (40.8g) in tetrahydrofuran (40ml) for 30 minutes, and the mixture was stirred for 30 minutes, warmed to room temperature, and further stirred for 1.5 hours. To the reaction mixture was added 10% sulfuric acid (182ml) for 40 minutes or more, and the mixture was stirred 1.5 hours, extracted with ethyl acetate, washed with saturated sodium chloride solution, dried with magnesium sulfate and concentrated under reduced pressure. The residue was crystallized from diisopropylether-hexane to give 4-ethoxyphenyl borate (15.5g) as colorless crystals. Under argon atmosphere, a solution of ethyl 7-bromo-2,3-dihydro-1-benzoxepine-4-carboxylate (504.5mg), the above 4-ethoxyphenyl borate (310mg) and potassium carbonate (0.47g) in toluene-ethanol-water (20-2-2ml) was stirred at room temperature for 1 hour. To the reaction mixture was added tetrakistriphenylphosphinepalladium (0.06g), and the mixture was refluxed for 20 hours and cooled to room temperature. The organic layer was washed with saturated sodium chloride solution, dried with magnesium sulfate and concentrated under reduced pressure. The residue was separated and purified with column chromatography (ethyl acetate/hexane=1:9→1:5) to give ethyl 7-(4-ethoxy-phenyl)-2,3-dihydro-1-benzoxepine-4-carboxy late (468mg) as colorless crystals.
m.p. 124-127°C
¹H-NMR (200MHz, CDCl₃) δ 1.36 (3H, t, J=7.2 Hz), 1.44 (3H, t, J=7.0 Hz), 3.00 (2H, t, J=4.0 Hz), 4.08 (2H, q, J=7.0 Hz), 4.28 (2H, q, J=7.2 Hz), 4.30 (2H, t, J=4.0 Hz), 6.96 (2H, dd, J=6.6, 2.2 Hz), 7.02 (1H, d, J=8.4 Hz), 7.41 (1H, d, J=2.6 Hz), 7.44-7.51 (3H, m), 7.65 (1H, br s).
IR (KBr) 1701, 1493, 1254, 1215, 1014, 824 cm⁻¹

| Elemental Analysis for C₂₁H₂₂O₄ | | |
|---|---|---|
| Calcd. | C, 74.54 ; | H, 6.55 : |
| Found. | C, 74.42 ; | H, 6.47. |

### Reference Example 134

To a solution of ethyl 7-(4-ethoxyphenyl)-2,3-dihydro-1-benzoxepine-4-carboxyl ate (447.8mg) in ethanol (20ml) was added 2N sodium hydroxide (2.0ml) at room temperature, and the mixture was stirred for 20 hours and concentrated under reduced pressure. To the residue was added 1N hydrochloric acid (5ml), and the mixture was extracted with ethyl acetate and concentrated. The resulting crystal was collected by filtration to give 7-(4-ethoxyphenyl)-2,3-dihydro-1-benzoxepine-4-carboxylic acid (380mg) as colorless crystals.
m.p. 269-271°C
¹H-NMR (200MHz, DMSO-d₆) δ 1.35 (3H, t, J=7.0 Hz), 2.81-2.94 (2H, m), 4.06 (2H, q, J=7.0Hz), 4.18-4.31 (2H,m), 6.94-7.00 (3H, m), 7.49-7.79 (5H, m).
IR (KBr) 2980, 1678, 1610, 1493, 1431, 1265, 1232, 1182, 1049, 926, 829, 810 cm⁻¹

| Elemental Analysis for C₁₉H₁₈O₄ | | |
|---|---|---|
| Calcd. | C, 73.53 ; | H, 5.85 : |
| Found. | C, 73.44 ; | H, 5.77. |

### Reference Example 135

Under argon atmosphere, to a solution of 4-trifluoro-methoxybromobenzene (10.0g) in tetrahydrofuran (75ml) was dropwise added n-butyllithium (1.6M, hexane solution) (28.5ml) at -78°C for 20 minutes, and the mixture was stirred for 40 minutes. To the reaction mixture was dropwise added a solution of trimethyl borate (12.9g) in tetrahydrofuran (12ml) for 15 minutes, and the mixture was stirred at -78°C for 30 minutes and at room temperature for 1 hour. To the reaction mixture was added was dropwise added 10% sulfuric acid (57.6ml) for 15 minutes, and the mixture was stirred for 2 hours, extracted with ethyl acetate, washed with saturated sodium chloride solution, dried with magnesium sulfate and concentrated under reduced pressure. The residue was crystallized from hexane to give 4-trifluoromethoxyphenyl borate (2.7g) as colorless crystals. Under argon atmosphere, a solution of ethyl 7-bromo-2,3-dihydro-1-benzoxepine-4-carboxylate (500mg), the above 4-trifluoromethoxyphenyl borate (380mg) and potassium carbonate (0.46g) in toluene-ethanol-water (20-2-2ml) was stirred at room temperature for 1 hour. To the reaction mixture was added tetrakistriphenyl-phosphinepalladium (0.06g), and the mixture was refluxed for 18 hours and cooled to room temperature. The organic layer was washed with saturated sodium chloride solution, dried with magnesium sulfate and concentrated under reduced pressure. The residue was separated and purified with column chromatography (ethyl acetate/hexane=1:10) to give ethyl 7- (4-trifluoromethoxyphenyl) -2,3-dihydro-I-benzoxepine-4-carboxylate (379mg) as colorless crystals.
m.p. 59-63°C
¹H-NMR (200MHz, CDCl₃) δ 1.36 (3H, t, J=7.1 Hz), 3.01 (2H, t, J=4.8 Hz), 4.24-4.34 (4H, m), 7.06 (1H, d, J=8.4 Hz), 7.22-7.31 (2H, m), 7.44 (1H, dd, J=8.4, 2.2 Hz), 7.52 (1H, d, J=2.2 Hz), 7.57 (2H, d, J=8.8 Hz), 7.64 (1H, br s).
IR (KBr) 1701, 1489, 1304, 1257, 1227, 1211, 1182, 1134, 1014, 833, 808 cm⁻¹

| Elemental Analysis for C₂₀H₁₇O₄F₃ | | |
|---|---|---|
| Calcd. | C, 63.49 ; | H, 4.53 : |
| Found. | C, 63.68 ; | H, 4.47. |

### Reference Example 136

To a solution of ethyl 7-(4-trifluoromethoxy-phenyl)-2,3-dihydro-1-benzoxepine -4-carboxylate (323.9mg) in tetrahydrofuran-ethanol (5-5ml) was added 1N sodium hydroxide (2.0ml) at room temperature, and the mixture was stirred for 5 days and concentrated under reduced pressure. To the residue 1N hydrochloric acid (5ml) was added, and the mixture was extracted with ethyl acetate. The organic layer was washed with saturated sodium chloride solution, dried with magnesium sulfate and concentrated. The resulting crystal was collected by filtration to give 7-(4-trifluoromethoxyphenyl)-2,3-dihydro-1-benzoxepine-4-carboxylic acid (282mg) as colorless crystals.
m.p. 252-254°C
¹H-NMR (200MHz, CDCl₃) δ 3.03 (2H, t, J=4.6 Hz), 4.34 (2H, t, J=4.6 Hz), 7.08 (1H, d, J=8.4 Hz), 7.28 (2H, d, J=8.8 Hz), 7.47 (1H, dd, J=8.4, 2.2 Hz), 7.54 (1H, d, J=2.2 Hz), 7.59 (2H, d, J=8.8 Hz), 7.78 (1H, br s).
IR (KBr) 2981, 1691, 1493, 1290, 1261, 1213, 1169, 835 cm⁻¹

| Elemental Analysis for C₁₈H₁₃O₄F₃ | | | |
|---|---|---|---|
| Calcd. | C, 61.72 ; | H, 3.74 ; | F, 16.27 : |
| Found. | C, 61.61 ; | H, 3.72 ; | F, 16.06. |

### Reference Example 137

To a solution of 5-bromosalicylaldehyde (10.0g) and tert-butyl acrylate (17.5ml) in tert-butanol (100ml) was added potassium tert-butoxide (1.67g) at room temperature, and the mixture was refluxed for 66 hours and cooled to room temperature. To the mixture was added ethyl acetate, and the mixture was washed with water, 1N sodium hydroxide and saturated sodium chloride solution, dried with magnesium sulfate and concentrated. The residue was separated and purified with column chromatography (ethyl acetate/hexane= 1:19) to give tert-butyl 6-bromo-2H-1-benzopyran-3-carboxylate (10.86g) as pale yellow crystals.
m.p. 96-97°C
¹H-NMR (200MHz, CDCl₃) δ 1.53 (9H, s), 4.95 (2H, d, J=0.8 Hz), 6.72 (1H, d, J=8.4 Hz), 7.21-7.30 (3H, m).
IR (KBr) 1699, 1479, 1331, 1288, 1159, 1088, 816 cm⁻¹

| Elemental Analysis for C₁₄H₁₅O₃Br | | | |
|---|---|---|---|
| Calcd. | C, 54.04 ; | H, 4.86 ; | Br, 25.68 : |
| Found. | C, 53.98 ; | H, 4.86 ; | Br, 25.90. |

### Reference Example 138

Under argon atmosphere, a solution of tert-butyl 6-bromo-2H-1-benzopyran-3-carboxylate (5.00g), 4-methylphenyl borate (2.62g) and potassium carbonate (4,44g) in toluene-ethanol-water (160-16-16ml) was stirred at room temperature for 1 hour. To the reaction mixture was added tetrakistriphenylphosphinepalladium (0.56g), and the mixture was refluxed for 14 hours and cooled to room temperature. The organic layer was washed with saturated sodium chloride solution, dried with magnesium sulfate and concentrated. The residue was separated and purified with column chromatography (ethyl acetate/hexane=1:19) to give pale yellow crystals, which were recrystallized from ethanol to give tert-butyl 6-(4-methylphenyl)-2H-1-benzopyran-3-carboxylate (3.84g) as pale yellow crystals.
m.p. 80-82°C
¹H-NMR (200MHz, CDCl₃) δ 1.54 (9H, s), 2.39 (3H, s), 4.98 (2H, d, J=1.4 Hz), 6.94 (1H, d, J=8.2 Hz), 7.23 (2H, d, J=8.0 Hz), 7.33 (1H, d, J=2.2 Hz), 7.36-7.45 (4H, m).
IR (KBr) 1705, 1367, 1340, 1311, 1251, 1159, 1133, 1003, 808 cm⁻¹

| Elemental Analysis for C₂₁H₂₂O₃ | | |
|---|---|---|
| Calcd. | C, 78.23 ; | H, 6.88 : |
| Found. | C, 78.07 ; | H, 6.89. |

### Reference Example 139

To tert-butyl 6-(4-methylphenyl)-2H-1-benzopyran-3-carboxylate (3.00g) was added 4N hydrochloric acid-ethyl acetate (10ml) at room temperature, and the mixture was stirred for 16 hours. To the reaction mixture was added hexane, and crystal was collected by filtration and washed with hexane to give 6-(4-methylphenyl)-2H-1-benzopyran-3-carboxylic acid (2.14g) as pale yellow crystals.
m.p. 236-237°C
¹H-NMR (200MHz, CDCl₃) δ 2.40 (3H, s), 5.05 (2H, d, J=1.4 Hz), 6.94 (1H, d, J=8.2 Hz), 7.23-7.27 (2H, m), 7.37 (1H, d, J=2.2 Hz), 7.41-7.52 (3H, m), 7.63 (1H, br s).
IR (KBr) 3022, 1689, 1633, 1485, 1442, 1306, 1242, 812 cm⁻¹

| Elemental Analysis for C₁₇H₁₄O₃ | | |
|---|---|---|
| Calcd. | C, 76.68 ; | H, 5.30 : |
| Found. | C, 76.51 ; | H, 5.03. |

### Reference Example 140

To a solution of 5-bromo-salicylaldehyde (10.0g) and ethyl crotonate (11.36g) in tert-butanol (50ml) was added potassium tert-butoxide (1.12g) at room temperature, and the mixture was refluxed for 3 days. To the reaction mixture was added water, and the mixture was extracted with ethyl acetate. The organic layer was washed with saturated sodium chloride solution, dried with magnesium sulfate and concentrated. The residue was separated and purified with column chromatography (ethyl acetate/hexane=1:10→1:5) to give pale yellow liquid (5.75g). The resulting compound was used for the following reaction without subjecting to further purification. Under nitrogen atmosphere, to a solution of the above crude product (5. 5g) and triethylamine (7.3ml) in dichloro-methane (50ml) was added methane-sulfonyl chloride (2.0ml) at 0°C, and the mixture was stirred at 0°C for 10 minutes and then at room temperature for 18 hours. To the reaction mixture was added water, and the mixture was extracted with diethylether. The organic layer was washed with saturated sodium chloride solution, dried with magnesium sulfate and concentrated. The residue was separated and purified with column chromatography (ethyl acetate/hexane=1:15) to give crude product (4.85g) as pale yellowoil. The resulting compound was used for the following reaction without subjecting to further purification. Under argon atmosphere, a solution of the above crude product (4.7g), 4-methylphenyl borate (2.58g) and potassium carbonate (4.4g) in toluene-ethanol-water (160-16-16ml) was stirred at room temperature for 1 hour. To the reaction mixture was added tetrakistriphenyl-phosphinepalladium (0.54g), and the mixture was refluxed for 20 hours and cooled to room temperature. The organic layer was washed with saturated sodium chloride solution, dried with magnesium sulfate and concentrated. The residue was separated and purified with column chromatography (ethyl acetate/hexane=1:15) to give ethyl 6-(4-methylphenyl)-2-methyl-2H-1-benzopyran-3-carboxyla te (3.63g) as pale yellow crystals.
m.p. 82-84°C
¹H-NMR (200MHz, CDCl₃) δ 1.35 (3H, t, J=7.2 Hz), 1.40 (3H, d, J=6.6 Hz), 2.39 (3H, s), 4.29 (2H, q, J=7.2 Hz), 5.40 (1H, q, J=6.6 Hz), 6.92 (1H, d, J=8.4 Hz), 7.24 (2H, d, J=8.2 Hz), 7.36 (1H, d, J=2.2 Hz), 7.40-7.49 (4H, m).
IR (KBr) 1699, 1485, 1296, 1244, 1217, 1190, 1136, 1047, 804, 764, 511 cm⁻¹

| Elemental Analysis for C₂₀H₂₀O₃ | | |
|---|---|---|
| Calcd. | C, 77.90 ; | H, 6.54 : |
| Found. | C, 77.79 ; | H, 6.46. |

### Reference Example 141

To a solution of ethyl 6-(4-methylphenyl)-2-methyl-2H-1-benzopyran-3-carboxylate (3.0g) in ethanol-tetra-hydrofuran (30-30ml) was added 1N sodium hydroxide (12ml) at room temperature, and the mixture was stirred for 16 hours. Under reduced pressure, the solvent was evaporated and acidified with 1N hydrochloric acid. The mixture was extracted with ethyl acetate. The organic layer was washed with saturated sodium chloride solution and dried with magnesium sulfate. Under reduced pressure, the solvent was evaporated to give 6-(4-methylphenyl)-2-methyl-2H-1-benzopyran-3-carboxyli c acid (2.15g) as yellow crystals.
m.p. 190-192°C
¹H-NMR (200MHz, CDCl₃) δ 1.43 (3H, d, J=6.6 Hz), 2.39 (3H, s), 5.40 (1H, q, J=6.6 Hz), 6.94 (1H, d, J=8.4 Hz), 7.24 (2H, d, J=8.0 Hz), 7.38 (1H, d, J=2.2 Hz), 7.44 (2H, d, J=8.0 Hz), 7.50 (1H, dd, J=8.4, 2.2 Hz), 7.60 (1H, s).
IR (KBr) 2983, 1680, 1635, 1485, 1421, 1298, 1261, 1190, 808 cm⁻¹

| Elemental Analysis for C₁₈H₁₆O₃ | | |
|---|---|---|
| Calcd. | C, 77.12 ; | H, 5.75 : |
| Found. | C, 77.25 ; | H, 5.63. |

### Reference Example 142

A solution of 5-bromo-2-thiophenecarboxyaldehyde (6.08g) and methyl (triphenylphosphoranilidene)acetate (11.12g) in toluene (60ml) was refluxed under nitrogen atmosphere for 2 hours and cooled. To the mixture was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane= 1:15→1:9) and recrystallized from ethyl acetate to give methyl (E)-3-(5-bromothiophen-2-yl)-acrylate (7.44g) as pale yellow crystals.
m.p. 60-62°C
¹H-NMR (200MHz, CDCl₃) δ 3.79 (3H, s), 6.13 (1H, d, J=15.8 Hz), 6.96-7.05 (2H, m), 7.66 (1H, d, J=15.8 Hz).
IR (KBr) 1724, 1624, 1417, 1257, 1203, 1165, 968, 802, 486 cm⁻¹

| Elemental Analysis for C₈H₇O₂SBr | | | | |
|---|---|---|---|---|
| Calcd. | C, 38.88 ; | H, 2.86 ; | S, 12.98 ; | Br, 32.34 : |
| Found. | C, 38.95 ; | H, 2.83 ; | S, 13.13 ; | Br, 32.36. |

### Reference Example 143

Under argon atmosphere, a solution of methyl (E) -3- (5-bromothiophen-2-yl) acrylate (4.0g), 4-methylphenyl borate (2.64g) and potassium carbonate (4.48g) in toluene-ethanol-water (160-16-16ml) was stirred at room temperature for 1 hour. To the reaction mixture was added tetrakistriphenylphosphinepalladium (0.56g), and the mixture was refluxed for 16 hours and cooled to room temperature. The organic layer was washed with saturated sodium chloride solution, dried with magnesium sulfate and concentrated under reduced pressure to give crude product (5.24g). To a solution of the resulting carboxylic acid ester (5.24g) in tetrahydrofuran (100ml) was added 1N sodium hydroxide (20ml) at room temperature, and the mixture was stirred for 5 days. To the reaction mixture was added water, and the mixture was washed with ethyl acetate. The aqueous layer was acidified with concentrated hydrochloric acid, and the mixture was extracted with ethyl acetate, washed with saturated sodium chloride solution, dried with magnesium sulfate and concentrated under reduced pressure to give (E)-3-[5-(4-methylphenyl)-thiophen-2-yl]acrylic acid (1.9g) as yellow crystals.
m.p.223-225°C
¹H-NMR (200MHz, CDCl₃) δ 2.38 (3H, s), 6.21 (1H, d, J=15.8 Hz), 7.16-7.27 (4H, m), 7.52 (2H, d, J=8.0 Hz), 7.84 (1H, d, J=15.8 Hz).
IR (KBr) 2968, 1666, 1606, 1413, 1261, 1230, 804 cm⁻¹

| Elemental Analysis for C₁₄H₁₂O₂S | | | |
|---|---|---|---|
| Calcd. | C, 38.83 ; | H, 4.95 ; | S, 13.12 : |
| Found. | C, 68.76 ; | H, 5.07 ; | S, 13.28. |

### Reference Example 144

To a suspension of 5-bromo-2-furancarboxylic acid (5.00g) and N-hydroxysuccinimide (3.31g) in acetonitrile (50ml) was added 1-ethyl-3-(3'-dimethylaminopropyl)-carbodiimide hydrochloride (5.52g) at room temperature, and the mixture was stirred for 2 hours. To the reaction mixture was added a suspension of N,O-dimethylhydroxyl-amine hydrochloride (2.81g) and triethylamine (10ml) in acetonitrile (20ml), and the mixture was stirred for 1 hour. To the reaction mixture were added 1,8-diazabicyclo-[5.4.0]-7-undecene (4.3ml) and DMF (50ml), and the mixture was stirred for 3 hours and concentrated under reduced pressure. To the residue was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=1:4→1:3→1:2) to give N-methyl-N-methoxy-5-bromofuran-2-carboxamide (2.77g) as pale yellow oil.
¹H-NMR (200MHz, CDCl₃) δ 3.34 (3H, s), 3.77 (3H, s), 6.45 (1H, d, J=3.6 Hz), 7.09 (1H, d, J=3.6 Hz).
IR (neat) 2974, 2937, 1647, 1475, 1416, 1385, 1211, 1024, 985, 926, 796, 739 cm⁻¹

### Reference Example 145

Under argon atmosphere, a solution of N-methyl-N-methoxy-5-bromofuran-2-carboxamide (2.77g), 4-methyl-phenyl borate (1.93g) and potassium carbonate (3.27g) in toluene-ethanol-water (110-11-11ml) was stirred at room temperature for 1 hour. To the reaction mixture was added tetrakistriphenylphosphinepalladium (0.41g), and the mixture was refluxed for 20 hours and cooled to room temperature. The organic layer was washed with saturated sodium chloride solution, dried with magnesium sulfate and concentrated under reduced pressure. The residue was separated and purified with column chromatography (ethyl acetate/hexane=1:5→1:2→1:1) to give N-methyl-N-methoxy-5-(4-methylphenyl)furan-2-carboxamide(2.65g) as colorless crystals.
m.p.54-58°C
¹H-NMR (200MHz, CDCl₃) δ 2.38 (3H, s), 3.38 (3H, s), 3.82 (3H, s), 6.69 (1H, d, J=3.8 Hz), 7.20-7.26 (3H, m), 7.68 (2H, d, J=8.6 Hz).
IR (neat) 1632, 1487, 1381, 1032, 987, 798, 739, 557, 494 cm⁻¹

| Elemental Analysis for C₁₄H₁₅NO₃ | | | |
|---|---|---|---|
| Calcd. | C, 68.56 ; | H, 6.16 ; | N, 5.71 : |
| Found. | C, 68.22 ; | H, 6.02 ; | N, 5.47. |

### Reference Example 146

Under nitrogen atmosphere, to a solution of N-methyl-N-methoxy-5-(4-methylphenyl)furan-2-carboxamid e (2.5g) in tetrahydrofuran (20ml) was added diisobutyl-aluminumhydride (1.01M toluene solution) (15ml) at-78°C, and the mixture was stirred at -78°C for 10 minutes and then at 0°C for 15 minutes. To the reaction mixture was added 1N hydrochloric acid to stop the reaction, and the mixture was extracted with ethyl acetate. The organic layer was washed with saturated sodium chloride solution, dried with magnesium sulfate and concentrated. The residue was separated and purified with column chromatography (ethyl acetate/hexane=1:5→1:4) to give crude product (1.49g). A solution of the crude aldehyde (1.49g) and methyl (triphenylphosphoranilidene)acetate (2.67g) in toluene (30ml) was refluxed under nitrogen atmosphere for 1 hour and cooled. To the mixture was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=1:9→1:5) to give methyl (E)-3-[5-(4-methylphenyl)furan-2-yl]acrylate (1.63g) as pale yellow crystals.
m.p. 113-115°C
¹H-NMR (200MHz, CDCl₃) δ 2.38 (3H, s), 3.80 (3H, s), 6.39 (1H, d, J=15.5 Hz), 6.68 (2H, s), 7.22 (2H, d, J=8.4 Hz), 7.44 (1H, d, J=15.5 Hz), 7.62 (2H, d, J=8.4 Hz) .
IR (KBr) 1716, 1632, 1304, 1201, 1161, 798 cm⁻¹

| Elemental Analysis for C₁₅H₁₄O₃ | | |
|---|---|---|
| Calcd. | C, 74.36 ; | H, 5.82 : |
| Found. | C, 74.36 ; | H, 5.75. |

### Reference Example 147

To a solution of methyl (E)-3-[5-(4-methylphenyl)-furan-2-yl]acrylate (1.49g) in tetrahydrofuran-ethanol (10-10ml) was added 2N sodium hydroxide (4ml) at room temperature, and the mixture was stirred for 24 hours. The reaction mixture was acidified with 1N hydrochloric acid, and the mixture was extracted with ethyl acetate. The organic layer was washed with saturated sodium chloride solution, dried with magnesium sulfate and concentrated under reduced pressure to give (E)-3-[5-(4-methylphenyl)-furan-2-yl]acrylic acid (0.93g) as colorless crystals.
m.p. 183-184°C
¹H-NMR (200MHz, CDCl₃) δ 2.39 (3H, s), 6.39 (1H, d, J=15.4 Hz), 6.70 (1H, d, J=3.4 Hz), 6.75 (1H, d, J=3.4 Hz), 7.23 (2H, d, J=8.2 Hz), 7.52 (1H, d, J=15.4 Hz), 7.64 (1H, d, J=8.2 Hz).
IR (KBr) 2964, 1678, 1624, 1419, 1308, 1261, 785 cm⁻¹

| Elemental Analysis for C₁₄H₁₂O₃ | | |
|---|---|---|
| Calcd. | C, 73.67 ; | H, 5.30 : |
| Found. | C, 73.42 ; | H, 5.15. |

### Reference Example 148

A solution of 4-bromo-2-thiophenecarboxyaldehyde (4.77g) and methyl (triphenylphosphoranilidene)acetate (8.44g) in toluene (50ml) was refluxed under nitrogen atmosphere for 3 hours and cooled. To the mixture was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane= 1:15) to give methyl (E)-3-(4-bromothiophen-2-yl)acrylate (5.55g) as pale yellow crystals.
m.p. 63-67°C
¹H-NMR (200MHz, CDCl₃) δ 3.80 (3H, s), 6.25 (1H, d, J=15.8 Hz), 7.16 (1H, d, J=0.8 Hz), 7.26 (1H, d, J=0.8 Hz), 7.68 (1H, d, J=15.8 Hz).
IR (KBr) 1713, 1630, 1304, 1257, 1165, 958, 828 cm⁻¹

| Elemental Analysis for C₈H₇O₂SBr | | | | |
|---|---|---|---|---|
| Calcd. | C, 38.88 ; | H, 2.86 ; | S, 12.98 ; | Br, 32.34 : |
| Found. | C, 38.78 ; | H, 2.83 ; | S, 12.98 ; | Br, 32.27. |

### Reference Example 149

Under argon atmosphere, a solution of methyl (E)-3-(4-bromothiophen-2-yl)acrylic acid (3.0g), 4-methyl-phenyl borate (1.82g) and potassium carbonate (3.36g) in toluene-ethanol-water (120-12-12ml) was stirred at room temperature for 1 hour. To the reaction mixture was added tetrakistriphenylphosphinepalladium (0.42g), and the mixture was refluxed for 24 hours and cooled to room temperature. The organic layer was washed with saturated sodium chloride solution, dried with magnesium sulfate and concentrated under reduced pressure. The residue was separated and purified with column chromatography (ethyl acetate/hexane=1:9→1:5→1:2) to give methyl (E)-3-[4-(4-methylphenyl)thiophen-2-yl)acrylate (2.40g) as pale yellow crystals.
m.p. 116-118°C
¹H-NMR (200MHz, CDCl₃,) δ 2.38 (3H, s), 3.80 (3H, s), 6.27 (1H, d, J=15.8 Hz), 7.21 (2H, d, J=7.8 Hz), 7.43-7.50 (4H, m), 7.80 (1H, d, J=15.8 Hz).
IR (KBr) 1713, 1622, 1506, 1423, 1302, 1240, 1192, 1159, 966, 847, 916, 760 cm⁻¹

| Elemental Analysis for C₁₅H₁₄O₂S | | | |
|---|---|---|---|
| Calcd. | C, 69.74 ; | H, 5.46 ; | S, 12.41 : |
| Found. | C, 69.54 ; | H, 5.47 ; | S, 12.24. |

### Reference Example 150

To a solution of methyl (E)-3-[4-(4-methylphenyl)-thiophen-2-yl)acrylate (2.40g) in tetrahydrofuran (50ml) was added 2N sodium hydroxide (6.0ml) at room temperature, and the mixture was stirred for 6 days. Precipitated crystal was collected by filtration and washed with tetrahydrofuran. To the crystals was added 1N hydrochloric acid (20ml), and the mixture was extracted with ethyl acetate. The organic layer was washed with saturated sodium chloride solution, dried with magnesium sulfate and concentrated under reduced pressure to give (E)-3-[4-(4-methylphenyl)thiophen-2-yl]acrylic acid (1.24g) as pale yellow crystals.
m.p.206-207°C
¹H-NMR (200MHz, CDCl₃) δ 2.38 (3H, s), 6.28 (1H, d, J=15.6 Hz), 7.23 (2H, d, J=8.0 Hz), 7.47 (2H, d, J=8.0 Hz), 7.49 (1H, s), 7.55 (1H, d, J=1.4 Hz), 7.90 (1H, d, J=15.6 Hz).
IR (KBr) 2970, 2918, 1682, 1622, 1306, 1196, 966, 818, 764 cm⁻¹

| Elemental Analysis for C₁₄H₁₂O₂S | | | |
|---|---|---|---|
| Calcd. | C, 68.83 ; | H, 4.95 ; | S, 13.12 : |
| Found. | C, 68.66 ; | H, 4.77 ; | S, 13.08. |

### Reference Example 151

Under nitrogen atmosphere, to a solution of ethyl chloroformylbutyrate (25.0g) in 1,2-dichloroethane (150ml) was dropwise added a solution of tin tetrachloride (76.6g) in 1,2-dichloroethane (50ml) at 0°C and then a solution of 2-bromothiophene (22.8g) in 1,2-dichloroethane (20ml), and the mixture was stirred at room temperature for 2 hours. The reaction mixture was vigorously stirred and added to ice-concentrated hydrochloric acid to stop the reaction. The mixture was stirred for 30 minutes and extracted with dichloromethane. The organic layer was washed with saturated sodium bicarbonate solution and saturated sodium chloride solution, dried with magnesium sulfate and concentrated. The residue was separated and purified with column chromatography (ethyl acetate/hexane=1:5) to give ethyl 5-(5-bromothiophen-2-yl)-5-oxovalerate (28.1g) as colorless crystals.
m.p. 53-54°C
¹H-NMR (200MHz, CDCl₃) δ 1.26 (3H, t, J=7.2 Hz), 1.97-2.12 (2H, m), 2.41 (2H, t, J=7.2 Hz), 2.92 (2H, t, J=7.3 Hz), 4.14 (2H, q, J=7.2 Hz), 7.10 (1H, d, J=4.0 Hz), 7.47 (1H, d, J=4.0 Hz).
IR (KBr) 1726, 1664, 1419, 1281, 1184, 980, 812 cm⁻¹

| Elemental Analysis for C₁₁H₁₃O₃SBr | | | | |
|---|---|---|---|---|
| Calcd. | C, 43.29 ; | H, 4.29 ; | S, 10.51 ; | Br, 26.18 _{:} |
| Found. | C, 43.54 ; | H, 4.20 ; | S, 10.64 ; | Br, 26.24. |

### Reference Example 152

Under argon atmosphere, a solution of ethyl 5-(5-bromothiophen-2-yl)-5-oxovalerate (10.09g), 4-methyl-phenyl borate (5.39g) and potassium carbonate (9.14g) in toluene-ethanol-water (320-32-32ml) was stirred at room temperature for 1 hour. To the reaction mixture was added tetrakistriphenylphosphinepalladium (1.14g), and the mixture was refluxed for 8 hours and cooled to room temperature. The organic layer was washed with saturated sodium chloride solution, dried with magnesium sulfate and concentrated under reduced pressure. The residue was separated and purified with column chromatography (ethyl acetate/hexane=1:4→1:3→1:2→1:1) to give ethyl 5-[5-(4-methylphenyl)thiophen-2-yl]-5-oxovalerate (10.23g) as colorless crystals.
m.p. 120-121°C
¹H-NMR (200MHz, CDCl₃) δ 1.26 (3H, t, J=7.2 Hz), 2.01-2.15 (2H, m), 2.38 (3H, s), 2.44 (2H, t, J=7.4 Hz), 2.97 (2H, t, J=7.2 Hz), 4.15 (2H, q, J=7.2 Hz), 7.22 (2H, d, J=7.9 Hz), 7.27 (1H, d, J=4.1 Hz), 7.55 (2H, d, J=7.9 Hz), 7.68 (1H, d, J=4.1 Hz).
IR (KBr) 1722, 1647, 1448, 1286, 1173, 816 cm⁻¹

| Elemental Analysis for C₁₈H₂₀O₃S | | | |
|---|---|---|---|
| Calcd. | C, 68.33 ; | H, 6.37 ; | S, 10.13 : |
| Found. | C, 68.40 ; | H, 6.26 ; | S, 10.11. |

### Reference Example 153

To a solution of ethyl 5- [5- (4-methylphenyl) thiophen-2-yl]-5-oxovalerate (4.50g) in trifluoroacetic acid (7.66ml) was added triethylsilane (5.7ml) at room temperature, and the mixture was stirred for 4 days. To the reaction mixture was added ethyl acetate, and the mixture was made alkaline with saturated sodium bicarbonate solution. The organic layer was washed with saturated sodium chloride solution, dried with magnesium sulfate and concentrated under reduced pressure. The residue was separated and purified with column chromatography (ethyl acetate/hexane= 1:9) to give crude ethyl 5-[5-(4-methyl-phenyl)thiophen-2-yl]valerate. To a solution of the crude ethyl 5-[5-(4-methylphenyl)thiophen-2-yl]valerate in tetrahydrofuran (50ml) was added 1N sodium hydroxide (20ml) at room temperature, and the mixture was stirred for 24 hours. To the reaction mixture was added water, and the mixture was washed with diethylether. The aqueous layer was acidified with 1N hydrochloric acid, and the mixture was extracted with ethyl acetate. The organic layer was washed with saturated sodium chloride solution, dried with magnesium sulfate and concentrated under reduced pressure to precipitate crystals, which were collected by filtration and washed with hexane to give 5-[5-(4-methylphenyl)-thiophen-2-yl]valeric acid (2.88g) as colorless crystals.
m.p.124-127°C
¹H-NMR (200MHz, CDCl₃) δ 1.67-1.82 (4H, m), 2.35 (3H, s), 2.36-2.45 (2H, m), 2.78-2.90 (2H, m), 6.73 (1H, d, J=3.6 Hz), 7.07 (1H, d, J=3.6 Hz), 7.15 (2H, d, J=8.4 Hz), 7.44 (2H, d, J=8.4 Hz).
IR (KBr) 2941, 1693, 1512, 1429, 1408, 1317, 1267, 1203, 945, 797, 771 cm⁻¹

| Elemental Analysis for C₁₆H₁₈O₂S | | | |
|---|---|---|---|
| Calcd. | C, 70.04 ; | H, 6.61 ; | S, 11.69 : |
| Found. | C, 69.79 ; | H, 6.37 ; | N, 11.62. |

### Reference Example 154

Under nitrogen atmosphere, to a solution of 5-[5-(4-methylphenyl)thiophen-2-yl]valeric acid (2.60g) in tetrahydrofuran (30ml) was added oxalyl chloride (1. 24ml) at room temperature and then a drop of DMF, and the mixture was stirred 1 hour. Under reduced pressure, the solvent was evaporated, and the residue was dissolved in dichloro-methane (30ml). To the mixture was added tin tetra-chloride (1.5ml) at 0°C , and the mixture was stirred at room temperature for 3 hours. The reaction mixture was added to water to stop the reaction, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=1:9→1:5) to give 2-(4-methylphenyl)-4-oxo-5,6,7,8-tetrahydro-4H-cyclo-he pta[b]thiophene (2.07g) as pale yellow crystals.
m.p. 82-84°C
¹H-NMR (200MHz, CDCl₃) δ 1.82-2.06 (4H, m), 2.35 (3H, s), 2.71-2.78 (2H, m), 3.06-3.12 (2H, m), 7.17 (2H, d, J=8.2 Hz), 7.44 (2H, d, J=8.2 Hz), 7.57 (1H, s).
IR (KBr) 2927, 1662, 1390, 1176, 810cm⁻¹

| Elemental Analysis for C₁₆H₁₆OS | | | |
|---|---|---|---|
| Calcd. | C, 74.96 ; | H, 6.29 ; | S, 12.51 : |
| Found. | C, 74.89 ; | H, 6.20 ; | S, 12.53. |

### Reference Example 155

To a solution of 2-(4-methylphenyl)-4-oxo-5,6,7,8-tetrahydro-4H-cyclohep ta [b] thiophene (2.62g) and dimethyl carbonate (2.6ml) in tetrahydrofuran (50ml) was added potassium tert-butoxide (1.38g) at room temperature, and the mixture was refluxed for 1 hour. To the reaction mixture were added potassium tert-butoxide (1.4g) and dimethyl carbonate (5ml), and the mixture was refluxed for 2 hours and cooled to room temperature. To the mixture was added 1N hydrochloric acid (150ml) at 0°C, and the mixture was extracted with ethyl acetate. The organic layer was washed with saturated sodium chloride solution, dried with magnesium sulfate and concentrated under reduced pressure to give crude products (3.30g).

To the crude products (3.30g) in dichloromethane (50ml) was added sodium boron hydride (0.77g) at room temperature and then methanol (8ml) at -15°C for 30 minutes, and the mixture was stirred for 2 hours. To the reaction mixture was added water, and the mixture was extracted with ethyl acetate. The organic layer was washed with saturated sodium chloride solution, dried with magnesium sulfate and concentrated under reduced pressure to give crude product (2.95g). To a solution of the crude product (2.95g) and triethylamine (7ml) in dichloromethane (20ml) was added methanesulfonyl chloride (1.2ml) at 0°C, and the mixture was stirred at room temperature for 17 hours. To the reaction mixture was added water, and the mixture was extracted with ethyl acetate. The organic layer was washed with saturated sodium chloride solution, dried with magnesium sulfate and concentrated under reduced pressure. The concentrate was purified with column chromatography (ethyl acetate/hexane= 1:9) to give methyl 2-(4-methyl-phenyl)-7,8-dihydro-6H-cyclohepta[b]thiophe ne-5-carboxylate (884mg) as yellow crystals.
¹H-NMR (200MHz, CDCl₃) δ 1.98-2.11 (2H, m), 2.36 (3H, s), 2.79 (2H, t, J=5.5 Hz), 3.09 (2H, t, J=5.6 Hz), 3.79 (3H, s), 7.08 (1H, s), 7.17 (2H, d, J=8.1Hz), 7.42 (2H, d, J=8.1 Hz), 7.60 (1H, s).

### Reference Example 156

To a solution of methyl 2-(4-methylphenyl)-7,8-dihydro-6H-cyclohepta[b]thiophen e-5-carboxylate (803mg) in ethanol-tetrahydrofuran (5-10ml) was added 2N sodium hydroxide (2ml) at room temperature, and the mixture was stirred for 5 days and concentrated under reduced pressure. To the residue was added 1N hydrochloric acid (10ml), and the mixture was extracted with ethyl acetate. The organic layer was washed with saturated sodium chloride solution, dried with magnesium sulfate and concentrated under reduced pressure to precipitate crystals, which were collected by filtration and washed with diisopropylether to give 2-(4-methylphenyl)-7,8-dihydro-6H-cyclohepta[b]thiophen e-5-carboxylic acid (650mg) as pale yellow crystals.
m.p.250-251°C
¹H-NMR (200MHz, CDCl₃) δ 2.00-2.14 (2H, m), 2.36 (3H, s), 2.75-2.85 (2H, m), 3.07-3.16 (2H, m), 7.10 (1H, s), 7.18 (2H, d, J=8.0 Hz), 7.43 (2H, d, J=8.0 Hz), 7.72 (1H, s).
IR (KBr) 2910, 2831, 1670, 1614, 1423, 1287, 1242, 810 cm⁻¹

| Elemental Analysis for C₁₇H₁₆O₂S | | | |
|---|---|---|---|
| Calcd. | C, 71.80 ; | H, 5.67 ; | S, 11.28 : |
| Found. | C, 71.74 ; | H, 5.64 ; | S, 11.06. |

### Reference Example 157

To a suspension of 5-bromonicotinic acid (5.0g) and N-hydroxysuccinimide (4.27g) in acetonitrile (60ml) was added 1-ethyl-3-(3'-dimethylaminopropyl)carbodiimide hydrochloride (7.12g) at room temperature, and the mixture was stirred for 30 minutes. To the reaction mixture were added N,O-dimethyl-hydroxylamine hydrochloride (2.66g) and triethylamine (10ml), and the mixture was stirred for 64 hours and concentrated under reduced pressure. To the residue was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=2:1) to give N-methyl-N-methoxy-5-bromo-pyridine-3-carboxamide (3.71g) as pale yellow oil.
¹H-NMR (200MHz, CDCl₃) δ 3.40 (3H, s), 3.58 (3H, s), 8.19 (1H, dd, J=2.2, 1.8 Hz), 8.76 (1H, d, J=2.2 Hz), 8.88 (1H, d, J=1.8 Hz).
IR (neat) 1647, 1412, 1381, 1221, 1099, 1020, 982, 897, 773, 739, 969, 667, 575, 461 cm⁻¹

### Reference Example 158

Under argon atmosphere, a solution of N-methyl-N-methoxy-5-bromopyridine-3-carboxamide (3.70g), 4-methylphenyl borate (2.26g) and potassium carbonate (4.17g) in toluene-ethanol-water (100-10-10ml) was stirred at room temperature for 1 hour. To the reaction mixture was added tetrakistriphenylphosphinepalladium (0.52g), and the mixture was refluxed for 16 hours and cooled to room temperature. The organic layer was washed with saturated sodium chloride solution, dried with magnesium sulfate and concentrated under reduced pressure. The residue was separated and purified with column chromatography (ethyl acetate/hexane=1:2→1:1) to give N-methyl-N-methoxy-5-(4-methylphenyl)pyridine-3-carboxa mide (3.97g) as yellow oil.
¹H-NMR (200MHz, CDCl₃) δ 2.42 (3H, s), 3.42 (3H, s), 3.60 (3H, s), 7.30 (2H, d, J=8.3 Hz), 7.51 (2H, d, J=8.3 Hz), 8.20 (1H, t, J=2.1 Hz), 8.89-8.81 (2H, m).
IR (neat) 1647, 1431, 1379, 1203, 982, 818, 743, 540, 426 cm⁻¹

### Reference Example 159

Under nitrogen atmosphere, to a solution of N-methyl-N-methoxy-5-(4-methylphenyl)pyridine-3-carboxamide (3.95g) in tetrahydrofuran (30ml) was dropwise added diisobutylaluminum hydride (1.01M toluene solution) (30ml) at -78°C, and the mixture was stirred at the same temperature for 2 hours . To the react ionmixture was added 1N hydrochloric acid to stop the reaction. To the mixture was added ethyl acetate, and the mixture was made alkaline with 1N sodium hydroxide. The organic layer was washed with saturated sodium chloride solution, dried with magnesium sulfate and concentrated under reduced pressure. The residue was separated and purified with column chromatography (ethyl acetate/hexane=1:2→1:1) to give 5-(4-methylphenyl)pyridine-3-carboxyaldehyde (1.82g) as colorless crystals.
m.p. 60-61°C
¹H-NMR (200MHz, CDCl₃) δ 2.43 (3H, s), 7.33 (2H, d, J=7.8 Hz), 7.54 (2H, d, J=7.8 Hz), 8.33 (1H, dd, J=2.2, 2.0 Hz), 9.03 (1H, d, J=2.0 Hz), 9.07 (1H, d, J=2.2 Hz), 10.19 (1H, s).
IR (KBr) 1701, 1186, 818, 725, 806 cm⁻¹

| Elemental Analysis for C₁₃H₁₁NO | | | |
|---|---|---|---|
| Calcd. | C, 79.17 ; | H, 5.62 ; | N, 7.10 : |
| Found. | C, 79.24 ; | H, 5.64 ; | N, 7.01. |

### Reference Example 160

A solution of 5-(4-methylphenyl)pyridine-3-carboxyaldehyde (1.82g) and methyl (triphenylphosphoranilidene)-acetate (3.46g) in toluene (20ml) was refluxed under nitrogen atmosphere for 4 hours and cooled. To the mixture was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate/hexane=1:2→1:1) to give methyl (E)-3-[5-(4-methylphenyl)pyridin-3-yl]acrylate (2.34g) as colorless crystals.
m.p. 141-144°C
¹H-NMR (200MHz, CDCl₃) δ 2.43 (3H, s), 3.84 (3H, s), 6.59 (1H, d, J=16.0 Hz), 7.32 (2H, d, J=7.9 Hz), 7.50 (2H, d, J=7.9 Hz), 7.76 (1H, d, J=16.0 Hz), 7.98 (1H, dd, J=2.2, 2.0 Hz), 8.70 (1H, d, J=2.0 Hz), 8.82 (1H, d, J=2.2 Hz).
IR (KBr) 1718, 1639, 1431, 1335, 1196, 1176, 995, 816 cm⁻¹

| Elemental Analysis for C₁₆H₁₅NO₂ | | | |
|---|---|---|---|
| Calcd. | C, 75.87 ; | H, 5.97 ; | N, 5.53 : |
| Found. | C, 75.82 ; | H, 5.86 ; | N, 5.47. |

### Reference Example 161

To a solution of methyl (E)-3-[5-(4-methylphenyl)-pyridin-3-yl]acrylate (2.25g) in tetrahydrofuran (20ml) was added 1N sodium hydroxide (11ml) at room temperature, and the mixture was stirred for 5 days. To the reaction mixture was added 1N hydrochloric acid (12ml), and the mixture was concentrated under reduced pressure to precipitate crystals, which were collected by filtration and washed with water and diethylether to give (E)-3-[5-(4-methylphenyl)pyridin-3-yl]acrylic acid (1.92g) as colorless crystals.
m.p. 208-211°C
¹H-NMR (200MHz, DMSO-d₆) δ 2.37 (3H, s), 6.85 (1H, d, J=16.2 Hz), 7.33 (2H, d, J=8.6 Hz), 7.66-7.74 (3H, m), 8.40-8.45 (1H, m), 8.81 (1H, d, J=1.8 Hz), 8.89 (1H, d, J=2.2 Hz).
IR (KBr) 3030, 1672, 1635, 1435, 1331, 1302, 987, 820 cm⁻¹

| Elemental Analysis for C₁₅H₁₃NO₂ | | | |
|---|---|---|---|
| Calcd. | C, 75.30 ; | H, 5.48 ; | N, 5.85 : |
| Found. | C, 74.99 ; | H, 5.39 ; | N, 5.94. |

### Reference Example 162

To DMF (7.18ml) was dropwise added phosphoryl chloride (8.64ml) at 0°C, and the mixture was stirred at room temperature for 30 minutes. To the mixture was added methyl acetoacetate (10ml) at 0°C, and the mixture was stirred at room temperature for 2 hours. The mixture was cooled to 0°C, and to the mixture was added 4-bromoaniline (16.78g), and the mixture was stirred at 90°C for 4 hours. To the reaction mixture was added chloroform, and the mixture was neutralized with 8N sodium hydroxide. The organic layer was washed with water and saturated sodium chloride solution, dried with magnesium sulfate and concentrated under reduced pressure. The residue was separated and purified with column chromatography (ethyl acetate/hexane=1:2) and was recrystallized from ethyl acetate-hexane to give methyl 6-bromo-2-methylquinoline-3-carboxylate (6.02g) as pale yellow crystals.
m.p. 150-151°C
¹H-NMR (200MHz, CDCl₃) δ 2.97 (3H, s), 3.99 (3H, s), 7.84 (1H, dd, J=9.0, 2.0 Hz), 7.92 (1H, d, J=9.0 Hz), 8.02 (1H, d, J=2.0 Hz), 8.65 (1H, s).
IR (KBr) 1726, 1423, 1396, 1277, 1238, 1219, 1134, 1074, 829 cm⁻¹

| Elemental Analysis for C₁₂H₁₀NO₂Br | | | |
|---|---|---|---|
| Calcd. | C, 51.45 ; | H, 3.60 ; | N, 5.00 : |
| Found. | C, 51.57 ; | H, 3.55 ; | N, 5.17. |

### Reference Example 163

Under argon atmosphere, a solution of methyl 6-bromo-2-methylquinoline-3-carboxylate (1.22g), 4-methylphenyl borate (0.65g) and potassium carbonate (1.18g) in toluene-ethanol-water (40-4-4ml) was stirred at room temperature for 1 hour. To the reaction mixture was added tetrakistriphenylphosphinepalladium (0.15g), and the mixture was refluxed for 18 hours and cooled to room temperature. The organic layer was washed with saturated sodium chloride solution, dried with magnesium sulfate and concentrated under reduced pressure. The residue was separated and purified with column chromatography (ethyl acetate/hexane= 1:1) to give methyl 6- (4-methylphenyl) -2-methylquinoline-3-carboxylate (1.27g) as colorless crystals.
m.p. 84-87°C
¹H-NMR (200MHz, CDCl₃) δ 2.43 (3H, s), 3.01 (3H, s), 4.00 (3H, s), 7.32 (2H, d, J=8.0 Hz), 7.61 (2H, d, J=8.0 Hz), 8.01-8.12 (3H, m), 8.79 (1H, s).
IR (KBr) 1732, 1440, 1277, 1213, 1068, 814 cm⁻¹

| Elemental Analysis for C₁₉H₁₇NO₂ | | | |
|---|---|---|---|
| Calcd. | C, 78.33 ; | H, 5.88 ; | N, 4.81 : |
| Found. | C, 77.98 ; | H, 6.02 ; | N, 4.75. |

### Reference Example 164

To a solution of methyl 6-(4-methylphenyl)-2-methylquinoline-3-carboxylate (0.99g) intetrahydrofuran-ethanol (5-5ml) was added 2N sodium hydroxide (2ml) at room temperature, and the mixture was stirred for 2 days. To the reaction mixture was added 1N hydrochloric acid (4ml), and the mixture was concentrated under reduced pressure to precipitate crystals, which were collected by filtration and washed with ethanol and diethylether to give 6-(4-methylphenyl)-2-methylquinoline-3-carboxylic acid (648mg) as colorless crystals.
m.p. 273°C (dec.)
¹H-NMR (200MHz, DMSO-d₆) δ 2.38 (3H, s), 2.89 (3H, s), 7.34 (2H, d, J=8.3 Hz), 7.74 (2H, d, J=8.3 Hz), 8.02 (1H, d, J=8.8 Hz), 8.15 (1H, dd, J=8.8, 2.1 Hz), 8.37 (1H, d, J=2.1 Hz), 8.90 (1H, s).
IR (KBr) 2918, 1703, 1570, 1495, 1257, 1227, 1180, 1151, 1065, 812, 770 cm⁻¹

| Elemental Analysis for C₁₈H₁₅NO₂ | | | |
|---|---|---|---|
| Calcd. | C, 77.96 ; | H, 5.45 ; | N, 5.05 : |
| Found. | C, 77.74 ; | H, 5.34 ; | N, 5.12. |

### Reference Example 165

Under argon atmosphere, a solution of ethyl 7-bromo-2,3-dihydro-1-benzoxepine-4-carboxylate (1.0g), 4-methylthiophenyl borate (622mg) and potassium carbonate (0.93g) in toluene-ethanol-water (30-3-3ml) was stirred at room temperature for 1 hour. To the reaction mixture was added tetrakistriphenyl-phosphinepalladium (117mg), and the mixture was refluxed for 16 hours. To the reaction mixture was added tetrakistriphenyl-phosphinepalladium (0.13g), and the mixture was refluxed for 24 hours and cooled to room temperature. The organic layer was washed with saturated sodium chloride solution, dried with magnesium sulfate and concentrated under reduced pressure. The residue was separated and purified with column chromatography (ethyl acetate/hexane=1:10) to give ethyl 7-(4-methylthio-phenyl)-2,3-dihydro-1-benzoxepine-4-car boxylate (442mg) as colorless crystals.
¹H-NMR (200MHz, CDCl₃) δ 1.36 (3H, t, J=7.0 Hz), 2.52 (3H, s), 3.00 (2H, t, J=4.8 Hz), 4.29 (2H, q, J=7.0 Hz), 4.30 (2H, t, J=4.8 Hz), 7.04 (1H, d, J=8.4 Hz), 7.32 (2H, d, J=8.8 Hz), 7.42-7.54 (4H, m), 7.65 (1H, br s).
IR (KBr) 1705, 1489, 1302, 1250, 1230, 1200, 1090, 1063, 1011, 813 cm⁻¹

### Reference Example 166

To a solution of ethyl 7-(4-methylthiophenyl)-2,3-dihydro-1-benzoxepine-4-carb oxylate (132mg) in ethanol-tetrahydrofuran (5ml-5ml) was added 1N sodium hydroxide (1.0ml) at room temperature, and the mixture was stirred for 20 hours and concentrated under reduced pressure. To the residue was added 1N hydrochloric acid (2ml) and the mixture was extracted with ethyl acetate. The organic layer was washed with saturated sodium chloride solution, dried with magnesium sulfate and concentrated under reduced pressure. The resulting crystal was collected by filtration to give 7-(4-methylthiophenyl)-2,3-dihydro-1-benzoxepine-4-carb oxylic acid (113mg) as colorless crystals.
¹H-NMR (200MHz, DMSO-d₆) δ 2.51 (3H, s,), 2.89 (2H, t, J=4.4 Hz), 4.25 (2H, t, J=4.4 Hz), 7.04 (1H, d, J=8.4 Hz), 7.33 (2H, d, J=8.4 Hz), 7.58 (1H, dd, J=8.4, 2.4 Hz), 7.61-7.70 (3H, m), 7.80 (1H, d, J=2.4 Hz).
IR (KBr) 2974, 1689, 1493, 1263, 1213, 1169, 1020, 833 cm⁻¹

### Reference Example 167

To a solution of 4-nitrobenzylalcohol (50 g, 0.326 mol) in ethyl acetate (EtOAc) (200 ml) were added 3,4-dihydropyran (35.7 ml, 0.392 mol) and CSA (camphor sulfonic acid) (379 mg, 1.63 mmol) under stirring at room temperature, and the mixture was stirred at room temperature for 1 hour. After the reaction completed, the reaction mixture was neutralized with saturated NaHCO₃ solution and separated ethyl acetate layer was dried with MgSO₄ and concentrated under reduced pressure. The residue was purified with silica gel column chromatography to give 4-(2-tetrahydro-pyranyloxymethyl)nitrobenzene (74.5 g, 96%) as syrup. ¹H-NMR (200 MHz, CDCl₃) δ : 1.55-2.05 (6H, m), 3.51-3.62 (1H, m), 3.83-3.94 (1H, m), 4.61 (1H, d, J=13.6Hz), 4.74 (1H, t, J=3.2Hz), 4.93 (1H, d, J=13.4Hz), 7.51-7.56 (2H, d, J=8.8Hz), 8.18-8.24 (2H, m).

### Reference Example 168

To a solution of 4-(2-tetrahydropyranyloxymethyl)-nitrobenzene (59.7 g, 0.256mol) in ethanol (EtOH) (300 ml) was added under nitrogen atmosphere at room temperature 10% Pd/C (5.97 g), and catalytic hydrogenation was carried out. The mixture was stirred at room temperature for 24 hours. After the reaction completed, the catalyst was filtered off, and the organic layer was concentrated under reduced pressure. The residue was purified with silica gel column chromatography to give 4-(2-tetrahydropyranyloxymethyl)-aniline (39.7 g, 76%) as syrup.
¹H-NMR (200 MHz, CDCl₃) δ : 1.45-1.95 (6H, m), 3.00-3.60 (3H, br m), 3.87-4.14 (1H, m), 4.39 (1H, d, J=11.4Hz), 4.68 (1H, d, J=11.4Hz), 4.71 (1H, m), 6.65-6.69 (2H, m), 7.15-7.19 (2H , m).

### Reference Example 169

To a solution of 2-(4-methylphenyl)-6,7-dihydro-5H-benzocycloheptene-8-c arboxylic acid (35.0 g, 0.126 mol) in tetrahydrofuran (THF) (280 ml) were added (COCl)₂ (21.9 ml, 0.251 mol) and DMF (0.7 ml) at 0°C. Under nitrogen atmosphere, the mixture was stirred at room temperature for 4 hours. After the reaction completed, The solvent was evaporated, and to the residue was added THF (315 ml). To a solution of the acid chloride was added a solution of 4-(2-tetrahydropyranyloxymethyl)aniline (28.1 g, 0.138 mol) and triethylamine (Et₃N) (26.3 ml, 0.189 mol) in THF (105 ml) at 0°C, and the mixture was stirred under nitrogen atmosphere, at room temperature for 2 hours. After the reaction completed, to the mixture was added water, and the mixture was extracted with ethyl acetate. The organic layer was washed with saturated NaCl solution and dried with MgSO₄. The solvent was evaporated and the residue was dissolved in methanol (MeOH) (470 ml). To the mixture was dropwise added 6N HCl (5.9 ml) at room temperature, and the mixture was stirred for 1 hour. After the reaction completed, the mixture was neutralized with saturated NaHCO₃ solution, and the solvent was removed. The residue was washed with water and then acetone/isopropylether (10:1; 60 ml), and the resulting precipitate was filtered, which was dissolved in THF. The mixture was dried with MgSO₄, and the solvent was evaporated. The resulting powder was washed twice with hexane:ethyl acetate (10:1; 50 ml) to give N- (4-hydroxymethylphenyl) -3- (4-methylphenyl) -6,7-dihydr o-5H-benzocycloheptene-6-carboxamide (26.8 g, 56%) as white powder.
¹H-NMR (200 MHz, CDCl₃) δ : 2.10-2.22 (2H, m), 2.39 (3H, s), 2.71 (2H, br t, J=6.4), 2.84-2.91 (2H, m), 4.67 (2H, s), 7.20-7.26 (2H, m), 7.33-7.51 (7H, m), 7.61 (2H, d, J=8.4), 7.71 (1H, br s).

### Reference Example 170

To a solution of N-(4-hydroxymethylphenyl)-2-(4-methylphenyl)-6,7-dihydr o-5H-benzocycloheptene-8-carboxamide (10.0 g, 26.1 mmol) and pyridine (0.1 ml) in chloroform (150 ml) was dropwise added a solution of thionyl chloride (3.4 ml, 39.2 mmol) in chloroform (90 ml), and the mixture was stirred under nitrogen atmosphere at room temperature for 17 hours. After the reaction completed, water was added to the mixture, and the mixture was extracted with chloroform. The organic layer was washed with saturated sodium chloride solution and dried with anhydrous magnesium sulfate. The solvent was evaporated, and the resulting powder was washed with hexane to give N-(4-chloromethylphenyl)-2-(4-methylphenyl)-6,7-dihydro -5H-benzocycloheptene-8-carboxamide (10.2 g, 97%) as colorless powder.
¹H-NMR (200 MHz, CDCl₃) δ : 2.05-2.21 (2H, m), 2.40 (3H, s), 2.71 (2H, br t, J=6.4), 2.84-2.91 (2H, m), 4.58 (2H, s), 7.20-7.27 (2H, m), 7.35-7.52 (7H, m), 7.59-7.65 (2H, m), 7.71 (1H, br s).

| Anal. | for C₂₆H₂₄NOCl·0.25H₂O: | | |
|---|---|---|---|
| Calcd: | C; 76.83, | H; 6.08, | N; 3.45. |
| Found: | C; 76.55, | H; 6.00, | N; 3.53. |

### Reference Example 171

To a solution of tetrahydro-4H-pyran-4-one (60 g, 0.6 mol) and water (5 ml) in DMF (70 ml, 0.90 mol) was added formic acid (46 ml, 1.2 mol), and the mixture was stirred at 140°C for 23 hours. After the reaction completed, reflux apparatus was changed to evaporation apparatus, crude amine was obtained by evaporation (74.6 g). b.p. 117 - 123 °C (27 mm).

To an aqueous solution (100 ml) of the crude amine (30 g) was dropwise added 6N HCl (5 drops), and the mixture was washed twice with dichloromethane. The aqueous layer was adjusted to pH 11 with sodium hydroxide. To the mixture was added NaCl, and the mixture was extracted with dichloromethane three times. The organic layer was dried with potassium carbonate, and the solvent was evaporated. The residue was purified with evaporation to give N,N-dimethyl-N-tetrahydropyran-4-ylamine (10.4 g, 29%) as colorless oil. b.p. 75-82 °C(29 mm) .
¹H-NMR (200 MHz, CDCl₃) δ : 1.40-1.82 (4H, m), 2.28 (6H, s), 2.25-2.40 (1H, m), 3.37 (2H, ddd, J=11.8, 11.8 and 2.2), 3.97-4.05 (2H, m) .

### Reference Example 172

To a suspension of 7-(4-methylphenyl)-2,3-dihydro-1-benzoxepine-4-carboxyl ic acid (0.6 g, 2.1 mmol) in tetrahydrofuran (10 ml) were added oxalyl chloride (0.33 ml, 4.3 mmol) and N,N-dimethylformamide (1 drop) at 0°C, and the mixture was stirred at room temperature for 2.5 hours. The solvent was evaporated, and the residue was dissolved in tetrahydrofuran (6 ml). To the mixture was dropwise added 4-(tert-butyldimethylsilyloxymethyl)aniline (0.56 g, 2.4 mmol) and triethylamine (0.36 ml, 2.6 mmol) in tetrahydrofuran (2 ml) at 0°C, and the mixture was stirred at room temperature for 16 hours. To the reaction mixture was added water, and the mixture was extracted with ethyl acetate. The extract was washed with saturated sodium chloride solution and dried with magnesium sulfate. The solvent was evaporated, and the residue was subjected to silica gel column chromatography. Crude amide (1.1 g) was obtained from fractions of hexane:ethyl acetate=5:1. This product was dissolved in acetone (8 ml), and to the mixture was dropwise added 6N hydrochloric acid. The mixture was stirred for 1 hour. To the mixture were added 1% sodium hydrogen carbonate (100 ml) and diisopropylether (100 ml), and precipitate was filtered, which were dissolved in acetone. The mixture was dried with magnesium sulfate, and the solvent was evaporated. The resulting powder was recrystallized from acetone-diisopropyl-ether to give N-(4-hydroxymethylphenyl)-7-(4-methylphenyl)-2,3-dihydr o-1-benzoxepine-4-carboxamide (0.87 g) as colorless crystals.
¹H-NMR (CDCl₃) δ : 2.39 (3H, s), 3.08 (2H, br t, J=4.4), 4.36 (2H, t, J=4.4), 4.68 (2H, s), 7.06 (2H, d, J=8.4), 7.18-7.61 (10H, m), 7.24 (2H, d, J=8.4).

| Anal. for C₂₅H₂₃NO₃: | | | |
|---|---|---|---|
| Calcd: | C; 77.90, | H; 6.01, | N; 3.63. |
| Found: | C; 77.91, | H; 6.10, | N; 3.55. |

### Reference Example 173

To a solution of N-(4-hydroxymethylphenyl)-7-(4-methylphenyl)-2,3-dihydr o-1-benzoxepine-4-carboxamide (412 mg, 1.07 mmol) and pyridine (1 drop) in chloroform (5 ml) was dropwise added thionyl chloride (0.14 ml, 1.61 mmol), and the mixture was stirred for 2 hours. The mixture was diluted with water and extracted with chloroform. The extract was washed with saturated sodium chloride solution and dried with magnesium sulfate. The solvent was evaporated, and the resulting powder was washed with hexane-ethyl acetate (1:1) to give N-(4-chloromethyl-phenyl)-7-(4-methylphenyl)-2,3-dihydr o-1-benzoxepine-4-carboxamide (380 mg, 88%) as colorless powder.
m.p. 164°C
¹H-NMR (CDCl₃) δ : 3.29 (3H, s), 3.07 (2H, t, J=4.8), 4.36 (2H, t, J=4.8), 4.59 (2H, s), 7.05 (1H, d, J=8.2), 7.22-7.26 (2H, m), 7.36-7.52 (6H, m), 7.57-7.62 (3H, m).

| Anal. for C₂₅H₂₂NO₂Cl: | | | |
|---|---|---|---|
| Calcd: | C; 74.34, | H; 5.49, | N; 3.47. |
| Found: | C; 74.00, | H; 5.42, | N; 3.29. |

### Reference Example 174

To a suspension of 1,4-cyclohexanedione monoethyleneketal (3.82 g, 24.6 mmol) and dimethylamine hydrochloride (2.00 g, 24.6 mmol) in 1,2-dichloroethane (50 ml) were dropwise added triethylamine (4.2 ml, 29.6 mmol) and DBU (1,8-diazabicyclo-[5.4.0]-7-undecene) (4.4 ml), and the mixture was stirred for 10 minutes. To the mixture was added triacetoxyborohydride (7.68 g, 34.4 mmol), and the mixture was stirred for 4.5 hours. Precipitate was filtered off, and the filtrate was concentrated to give crude product (6.34 g), which was dissolved in water (10 ml). To the mixture was dropwise added concentrated hydro-chloric acid (6 ml), and the mixture was stirred for 48 hours. The reaction mixture was diluted with water and washed twice with ether. The aqueous layer was made basic with sodium hydroxide and extracted with ether twice. The extract was washed with saturated sodium chloride solution, dried with potassium carbonate and purified by evaporation to give 4-dimethylaminocyclohexanone (0.59 g, 17%).
b.p.142-5°C
¹H-NMR (CDCl₃) δ: 1.69-2.13 (4H, m), 2.32 (6H, s), 2.20-2.41 (2H, m), 2.44-2.64 (3H, m) .

### Reference Example 175

To a solution of 7-(4-ethoxyphenyl)-2,3-dihydro-1-benzoxepine-4-carboxylic acid (2.38 g) in THF (50 ml) were added oxalyl chloride (1.4 ml) and DMF (2 drops) at room temperature, and the mixture was stirred for 1 hour. Under reduced pressure, the solvent was evaporated, and the residue was dissolved in THF (50 ml). To the mixture was dropwise added a solution of triethylamine (2.1 ml) and 4-aminobenzyloxy-tert-butyldimethylsilane (2.00 g) in THF (10 ml) at 0°C, and the mixture was stirred at room temperature for 18 hours. To the reaction mixture was added water, and the mixture was extracted with ethyl acetate. The organic layer was washed with saturated sodium chloride solution, dried with magnesium sulfate and concentrated under reduced pressure. The residue was separated and purified with column chromatography (ethyl acetate /hexane =1:4) to give pale yellow crystals (3.99 g), which were dissolved in acetone (50 ml). To the mixture was added 6N hydrochloric acid (1.3 ml) at room temperature, and the mixture was stirred for 1 hour. To the reaction mixture were added 5% sodium hydrogen carbonate solution (15 ml) and diisopropylether (100 ml). Precipitate was collected by filtration and washed with water and diisopropylether. The resulting solid was dissolved in THF, dried with magnesium sulfate and concentrated under reduced pressure to give crystals, which were recrystallized from THF to give 7-(4-ethoxyphenyl)-N-(4-hydroxymethylphenyl)-2,3-dihydr o-1-benzoxepine-4-carboxamide (2.65 g) as colorless crystals.
m.p. 208-210 °C
¹H-NMR (200MHz, DMSO-d₆) δ : 1.35 (3H, t, J=7 .0 Hz), 2.93-3.03 (2H, m), 4.06 (2H, q, J=7.0 Hz), 4.45 (2H, br s), 5.01-5.18 (1H, m), 6.98-7.05 (3H, m), 7.25-7.34 (3H, m), 7.49-7.71 (6H, m), 9.92 (1H, s).
IR (KBr) ν : 3363, 3290, 1659, 1612, 1525, 1493, 1242, 1227, 825 cm⁻¹

| Anal. for C₂₆H₂₅NO₄ | | | |
|---|---|---|---|
| Calcd: | C, 75.16 ; | H, 6.06 ; | N, 3.37 |
| Found: | C, 75.16 ; | H, 6.08 ; | N, 3.31. |

### Reference Example 176

To a suspension of 7-(4-ethoxyphenyl)-N-(4-hydroxymethylphenyl)-2,3-dihydro-1-benzoxepine-4-carboxamide (2.55 g) and pyridine (2 drops) in chloroform (50 ml) was added thionyl chloride (0.8 ml) at room temperature, and the mixture was stirred for 20 hours. To the reaction mixture was added water and then THF, and the mixture was extracted with ethyl acetate. The organic layer was washed with saturated sodium chloride solution, dried with magnesium sulfate and concentrated under reduced pressure to give solid, which was dissolved in THF and ethyl acetate. The mixture was concentrated under reduced pressure to give crystals, which were collected by filtration and washed with diisopropylether to give N-(4-chloromethylphenyl)-7-(4-ethoxyphenyl)-2,3-dihydro -1-benzoxepine-4-carboxamide (2.42 g) as colorless crystals.
m.p. 187-189 °C
¹H-NMR (200MHz, DMSO-d₆) δ : 1.35 (3H, t, J=7.0 Hz), 2.93-3.04 (2H, m), 4.06 (2H, q, J=7.0 Hz), 4.23-4.34 (2H, m), 4.74 (2H, s), 6.98-7.06 (3H, m), 7.35-7.42 (3H, m), 7.52 (1H, dd, J=8.4, 2.2 Hz), 7.59 (2H, d, J=8.8 Hz), 7.70-7.74 (3H, m), 10.04 (1H, s).
IR (KBr) ν : 3400, 1659, 1610, 1525, 1493, 1242, 1047, 822 cm⁻¹

| Anal. for C₂₆H₂₄NO₃Cl | | | |
|---|---|---|---|
| Calcd: | C, 71.97 ; | H, 5.57 ; | N, 3.23 |
| Found: | C, 71.96 ; | H, 5.54 ; | N, 3.04. |

### Working Example 194 (Production of Compound 194)

To solution of 7-(4-ethoxyphenyl)-N-[4-[N-methyl-N-(tetrahydropyran-4-yl)aminomethyl]phenyl]-2,3-dihydro-1-benzoxepine-4-carb oxamide (111 mg) in DMF (5 ml) was added methyl iodide (0.04 ml) at room temperature, and the mixture was stirred for 8 hours. Under reduced pressure, the mixture was concentrated, and to the residue was added ethyl acetate to precipitate solid, which was collected by filtration and recrystallized from ethanol-ethyl acetate to give dimethyl-[4-N-[7-(4-ethoxyphenyl)-2,3-dihydro-1-benzoxe pin-4-carbonyl]aminobenzyl]-4-tetrahydro-pyranylammoniu m iodide (Compound 194) (97 mg) as pale yellow crystals. m.p. 152-158 °C
¹H-NMR (200MHz, CDCl₃) δ : 1.41 (3H, t, J=7.0 Hz), 1.68-1.98 (2H, m), 2.10-2.26 (2H, m), 2.94 (6H, s), 2.98-3.08 (2H, m), 3.35-3.59 (3H, m), 3.96-4.16 (2H, m), 4.03 (2H, q, J=7.0 Hz), 4.19-4.31 (2H, m), 4.84 (2H, s), 6.91 (2H, d, J=8.8 Hz), 6.97 (1H, d, J=8.4 Hz), 7.38 (1H, dd, J=8.4, 2.2 Hz), 7.44-7.57 (5H, m), 7.69 (1H, d, J=2.2 Hz), 7.80 (2H, d, J=8.4 Hz), 8.01 (1H, s).
IR (KBr) ν : 3440, 1657, 1605, 1520, 1491, 1317, 1240 cm⁻¹

| Anal. for C₃₃H₃₉N₂O₄I·1.0H₂O | | | |
|---|---|---|---|
| Calcd: | C, 58.93 ; | H, 6.14 ; | N, 4.16 |
| Found: | C, 58.86 ; | H, 6.18 ; | N, 4.19. |

### Working Example 195 (Production of Compound 195)

To a solution of 7-(4-ethylphenyl)-N-[4-[N-methyl-N-(tetrahydropyran-4-y 1)aminomethyl]phenyl]-2,3-dihydro-1-benzoxepine-4-carbo xamide (125 mg) in DMF (5 ml) was added methyl iodide (0.04 ml) at room temperature, and the mixture was stirred for 20 hours. Under reduced pressure, the mixture was concentrated, and to the residue was added ethyl acetate to precipitate solid, which was collected by filtration and recrystallized from acetone-diethylether→ethanol-diethylether) to give dimethyl-[4-N-[7-(4-ethylphenyl)-2,3-dihydro-1-benzoxep in-4-carbonyl]aminobenzyl]-4-tetrahydropyranylammonium iodide (Compound 195) (68 mg) as pale yellow crystals.
m.p. 156-160 °C
¹H-NMR (200MHz, CDCl₃) δ : 1.25 (3H, t, J=7.6 Hz), 1.69-1.93 (2H, m), 2.13-2.28 (2H, m), 2.66 (2H, q, J=7.6 Hz), 2.95 (6H, s), 3.00-3.09 (2H, m), 3.39-3.56 (2H, m), 4.02-4.34 (5H, m), 4.86 (2H, s), 6.99 (1H, d, J=8.4 Hz), 7.18-7.28 (3H, m), 7.39-7.56 (5H, m), 7.69-7.73 (1H, m), 7.79 (2H, d, J=8.8 Hz), 8.78 (1H, s).
IR (KBr) ν : 3429, 1657, 1301, 1520, 1491, 1412, 1319, 1244, 827 cm⁻¹

| Anal. for C₃₃H₃₉N₂O₃I·1.0H₂O | | | |
|---|---|---|---|
| Calcd: | C, 60.37 ; | H, 6.29 ; | N, 4.27 |
| Found: | C, 60.40 ; | H, 6.24 ; | N, 4.10. |

### Working Example 196 (Production of Compound 196)

To a solution of N-[4-[N-methyl-N-(tetrahydropyran-4-yl)aminomethyl]phenyl]-7-(4-trifluoromethylphenyl)-2, 3-dihydro-1-benzoxepine-4-carboxamide (113.6 mg) in DMF (5 ml) was added methyl iodide (0.04 ml) at room temperature, and the mixture was stirred for 24 hours. Under reduced pressure, the mixture was concentrated, and to the residue was added ethyl acetate to precipitate solid, which was collected by filtration and recrystallized from acetone-diethylether→ethanol-diethyl-ether) to give dimethyl-[4-N-[7-(4-trifluoromethylphenyl)-2,3-dihydro-1-benzoxepin-4-carbonyl]aminobenzyl]-4-tetrahydro-pyran ylammonium iodide (Compound 196) (99 mg) as pale yellow crystals.
m.p. 213 °C (dec.)
¹H-NMR (200MHz, DMSO-d₆) δ : 1.42-1. 66 (2H, m), 1.75-1.88 (2H, m), 2.55 (6H, s), 2.62-2.72 (2H, m), 2.94-3.35 (3H, m), 3.68-3.81 (2H, m), 3.96-4.08 (2H, m), 4.13 (2H, s), 6.80 (1H, d, J=8.8 Hz), 7.05 (1H, s), 7.21 (2H, d, J=8.4 Hz), 7.34-7.40 (1H, m), 7.44-7.63 (7H, m), 9.89 (1H, s).
IR (KBr) ν : 3277, 1649, 1510, 1520, 1491, 1325, 1255, 1120, 843 cm⁻¹

| Anal. for C₃₂H₃₄N₂O₃F₃I·0.2H₂O | | | |
|---|---|---|---|
| Calcd: | C, 56.35 ; | H, 5.08 ; | N, 4.11 |
| Found: | C, 56.21 ; | H, 5.16 ; | N, 4.11. |

### Reference Example 177

In 1,2-dichloroethane(400 ml) was suspended p-nitrobenzylamine hydrochloride (30.8 g), 1,4-cyclohexane-dione monoethyleneketal (25.4 g) and triethylamine (23 ml), and to the suspension was added sodium triacetoxy boron hydride (50.9 g) under ice-cooling. Under nitrogen atmosphere, the mixture was stirred at room temperature for 2.5 hours . Under ice-cooling , 37% formalin (14.6 ml) and sodium triacetoxy boron hydride (50.9 g) were added to the mixture. Under nitrogen atmosphere, the mixture was stirred at room temperature overnight. The mixture was neutralized with sodium hydrogen carbonate and extracted with 1,2-dichloroethane. The organic layer was washed with sodium chloride solution and dried with anhydrous magnesium sulfate. Under reduced pressure, the solvent was evaporated to give yellow solid (47.5 g), 44 g of which was dissolved in (660 ml). To the mixture was added reduced iron (32 g) little by little, and the mixture was stirred at room temperature overnight. The solvent was evaporated, and to the residue was added ethyl acetate. The precipitate was filtered off, and the filtrate was made alkaline with potassium carbonate and extracted with ethyl acetate. The organic layer was washed with water and saturated sodium chloride solution and driedwith anhydrous magnesium sulfate. Under reduced pressure, the solvent was evaporated, and the residue was purified with silica gel column chromatography (ethyl acetate/triethylamine/methanol) to give 4-((N-(4,4-ethylenedioxycyclohexyl)-N-methyl)aminomethy 1)aniline (34.1 g) as brown oil.
¹H-NMR (CDCl₃) δ : 1.36-1.93 (8H, m), 2.17 (3H, s), 2.43-2.57 (1H, m), 3.46 (2H, s), 3.60 (2H, br), 3.94 (4H, s), 6.64 (2H, d, J=8.4Hz), 7.09 (2H, d, J=8.4Hz).
IR(neat) ν : 2946, 1615cm⁻¹.

### Working Example 197 (Production of Compound 197)

In dichloromethane (400 ml) was suspended 7-(4-methylphenyl)-2,3-dihydro-1-benzoxepine-4-carboxyl ic acid (17.0 g), and to the suspension were added oxalyl chloride (10.3 ml) and dimethylformamide (catalytic amount) under ice-cooling. The mixture was stirred at room temperature for 2 hours, and the solvent was evaporated. The residue was dissolved in tetrahydrofuran (300 ml), and the mixture was dropwise added to a solution of 4-((N-(4,4-ethylenedioxycyclohexyl)-N-methyl)aminomethy 1)-aniline (16.75 g) and triethylamine (25 ml) in tetrahydrofuran (200 ml), under ice-cooling. Under nitrogen atmosphere, the mixture was stirred at room temperature overnight, and the solvent was evaporated. To the residue was added water, and the mixture was extracted with ethyl acetate. The organic layer was washed with water and saturated sodium chloride solution and dried with anhydrous magnesium sulfate. Under reduced pressure, the solvent was evaporated to give crude crystals, which were recrystallized from ethyl acetate to give N-(4-((N-(4,4-ethylenedioxy-cyclohexyl)-N-methyl)aminom ethyl)phenyl)-7-(4-methyl-phenyl)-2,3-dihydro-1-benzoxe pine-4-carboxamide (Compound 197) (17.1 g) as colorless crystals.
mp 192-193°C.
¹H-NMR(CDCl₃) δ : 1.48-1.86 (8H, m), 2.20 (3H, s), 2.39 (3H, s), 2.45-2.60 (1H, m), 3.08 (2H, t, J=4.5Hz), 3.56 (2H, s), 3.95 (4H, s), 4.36 (2H, t, J=4.5Hz), 7.06 (1H, d, J=8.4Hz), 7.23-7.33 (4H, m), 7.44-7.56 (7H, m).
IR(KBr) ν : 2948, 1651cm⁻¹.

| Anal. for C₃₄H₃₈N₂O₄: | | | |
|---|---|---|---|
| Calcd: | C, 75.81; | H, 7.11; | N, 5.20. |
| Found: | C, 75.51; | H, 6.99; | N, 5.29. |

### Working Example 198 (Production of Compound 198)

In acetic acid (100 ml) and 1N hydrochloric acid (200 ml) was dissolved N-(4-((N-(4,4-ethylenedioxycyclo-hexyl)-N-methyl)aminom ethyl)phenyl)-7-(4-methylphenyl)-2,3-dihydro-1-benzoxep ine-4-carboxamide (17.1 g), and the mixture was stirred at 100°C for 1.5 hours and concentrated. The residue was neutralized with 1N sodium hydroxide and extracted with ethyl acetate. The organic layer was washed with water and saturated sodium chloride solution and dried with anhydrous magnesium sulfate. Under reduced pressure, the solvent was evaporated to give crude crystals, which were recrystallized from ethyl acetate-methanol to give N-(4-((N-(4-oxocyclohexyl)-N-methyl)aminomethyl)-phenyl )-7-(4-methylphenyl)-2,3-dihydro-1-benzoxepine-4-carbox amide (Compound 198) (12 g) as colorless crystals.
mp 149-150°C.
¹H-NMR (CDCl₃) δ: 1.78-2.13 (4H, m), 2.23 (3H, s), 2.25-2.35 (2H, m), 2.39 (3H, s), 2.45-2.57 (2H, m), 2.84-2.94 (1H, m), 3.08 (2H, t, J=4.4Hz), 3.59 (2H, s), 4.35 (2H, t, J=4.4Hz), 7.06 (1H, d, J=8.0Hz), 7.22-7.34 (4H, m), 7.43-7.57 (6H, m), 7.65 (1H, s).
IR(KBr) ν : 2946, 1713cm⁻¹.

| Anal. for C₃₂H₃₄N₂O₃ | | | |
|---|---|---|---|
| Calcd: | C, 77.70; | H, 6.93; | N, 5.66. |
| Found: | C, 77.45; | H, 6.78; | N, 5.65. |

### Reference Example 178

To a mixture of methyl 2-bromo-6,7-dihydro-5H-benzocycloheptene-8-carboxylate (0.5 g), 4-(1-pyrrolidinyl)phenyl borate(0.37 g), 1M potassium carbonate (6ml) and ethanol (6ml) was added toluene (50 ml), and the mixture was stirred under argon atmosphere at room temperature for 30 minutes. To the mixture was added tetrakistriphenylphosphinepalladium (0.08 g), and the mixture was refluxed for 6 hours and extracted with ethyl acetate. The organic layer was washed with water and saturated sodium chloride solution and dried with anhydrous magnesium sulfate. Under reduced pressure, the solvent was evaporated, and the residue was purified with silica gel column (ethyl acetate/hexane) to give colorless crystals (0.48 g), which were dissolved in 1N sodium hydroxide (15 ml), methanol (50 ml) and tetrahydrofuran (50 ml). The mixture was stirred at room temperature overnight, concentrated and neutralized with hydrochloric acid to precipitate 2-(4-(1-pyrrolidinyl)phenyl)-6,7-dihydro-5H-benzocycloheptene-8-carboxylic acid (0.46 g) as pale yellow crystals.
mp 242-243°C (dec.).
¹H-NMR (DMSO-d₆) δ : 1.93-2.00 (6H, m), 2.56 (2H, t, J=5.8Hz), 2.76-2.82 (2H, m), 3.23-3.35 (4H, m), 6.60 (2H, d, J=8.8Hz), 7.20 (1H, d, J=8.2Hz), 7.44 (1H, dd, J=1.0, 8.2Hz), 7.53 (2H, d, J=8.8Hz), 7.56 (1H, d, J=1.0Hz), 7.69 (1H, s).

| Anal. for C₂₂H₂₃NO₂·0.1H₂O : | | | |
|---|---|---|---|
| Calcd: | C, 78.82; | H, 6.98; | N, 4.18. |
| Found: | C, 78.92; | H, 6.95; | N, 4.15. |

### Working Example 199 (Production of Compound 199)

To a solution of 2-(4-(1-pyrrolidinyl)phenyl)-6,7-dihydro-5H-benzocycloh eptene-8-carboxylic acid (0.45 g), 4-(N-methyl-N-(tetrahydropyran-4-yl)aminomethyl)aniline (0.33 g) and 1-hydroxybenzotriazole (0.18 g) in dimethyl-formamide (20 ml) was added 1-ethyl-3-(3-dimethylamino-propyl)carbodiimide hydrochloride (0.39 g) under ice-cooling. Under nitrogen atmosphere, the reaction mixture was cooled to room temperature, and to the mixture were added 4-dimethylaminopyridine (catalytic amount) and triethylamine (0.56 ml). The mixture was stirred overnight, poured into water and extracted with ethyl acetate. The organic layer was washed with water and saturated sodium chloride solution and dried with anhydrous magnesium sulfate. Under reduced pressure, the solvent was evaporated, and the residue was purified with silica gel column (ethyl acetate/methanol/triethylamine) to give crude crystals, which were recrystallized from ethyl acetate-hexane to give 2-(4-(1-pyrrolidinyl)phenyl)-N-(4-((N-tetrahydropyran-4 -yl-N-methyl)aminomethyl)phenyl)-6,7-dihydro-5H-benzocy cloheptene-8-carboxamide (Compound 199) (0.28 g) as colorless crystals.
mp 124-125°C.
¹H-NMR(CDCl₃) δ : 1.66-1.77 (4H, m), 1.99-2.06 (4H, m), 2.11-2.18 (2H, m), 2.21 (3H, s), 2.55-2.75 (3H, m), 2.84-2.90 (2H, m), 3.30-3.44 (6H, m), 3.58 (2H, s), 4.00-4.14 (2H, m), 6.64 (2H, d, J=9.0Hz), 7.19 (1H, d, J=8.0Hz), 7.31 (2H, d, J=8.5Hz), 7.39-7.51 (4H, m), 7.57 (2H, d, J=8.5Hz), 7.64 (1H, s).
IR(KBr) ν : 2946, 2843, 1651, 1611cm⁻¹.

| Anal. for C₃₅H₄₁N₃O₂·0.2H₂O | | | |
|---|---|---|---|
| Calcd: | C, 77.95; | H, 7.74; | N, 7.79. |
| Found: | C, 77.76; | H, 7.59; | N, 7.79. |

### Reference Example 179

In 1,2-dichloroethane (50 ml) were dissolved p-nitrobenzaldehyde (5 g) and 3-amino-1-propanol (2.5 g), and to the mixture was added sodium triacetoxy boron hydride (9.8 g) under ice-cooling. Under nitrogen atmosphere, the mixture was stirred at room temperature for 5 hours. Under ice-cooling ,to the mixture was added 37% formalin(3 ml) and sodium triacetoxy boron hydride (9.8 g). Under nitrogen atmosphere, the mixture was stirred at room temperature overnight. To the mixture was added water, and the mixture was concentrated, neutralized with aqueous sodium hydroxide and extracted with ethyl acetate. The organic layer was washed with water and sodium chloride solution and dried with anhydrous magnesium sulfate. Under reduced pressure, the solvent was evaporated, and the residue was purified with silica gel column (ethyl acetate/methanol/triethylamine) to give yellow oil (5.0 g), 2.5g of which was dissolved in ethanol (50 ml) and catalytic hydrogenation was carried out with 5% palladium on carbon (0.2 g) for 1.5 hours. The catalyst was filtered off, and the solvent was evaporated. The residue was purified with silica gel column (ethyl acetate/methanol/triethylamine) to give 4-((N-3-hydroxypropyl-N-methyl)aminomethyl)-aniline (1.5 g) as pale yellow oil.
¹H-NMR (CDCl₃) δ: 1.67-1.78 (2H, m), 2.21 (3H, s), 2 . 62 (2H, t, J=5.5Hz), 3.41 (2H, s), 3.65 (2H, br), 3. 77 (2H, t, J=5.1Hz), 6.65 (2H, d, J=8.4Hz), 7.07 (2H, d, J=8.4Hz).
IR(neat) ν : 3347, 2948, 2799, 1615cm-¹.

### Working Example 200 (Production of Compound 200)

In dichloromethane (5 ml) was suspended 2-(4-methylphenyl)-6,7-dihydro-5H-benzocycloheptene-8-carboxylic acid (0.3 g), and to the suspension were added oxalyl chloride (0.28 ml) and dimethylformamide (catalytic amount) under ice-cooling. The mixture was stirred at room temperature for 1.5 hours, and the solvent was evaporated. The residue was dissolved in tetrahydrofuran (15 ml), and the mixture was dropwise added to a solution of 4-((N-3-hydroxypropyl-N-methyl)aminomethyl)aniline (0.23 g) and triethylamine (0.45 ml) in tetrahydrofuran (15 ml) under ice-cooling. Under nitrogen atmosphere, the mixture was stirred at room temperature overnight, and the solvent was evaporated. To the residue was added water, and the mixture was extracted with ethyl acetate. The organic layer was washed with water and saturated sodium chloride solution and dried with anhydrous magnesium sulfate. Under reduced pressure, the solvent was evaporated, and the residue was purified with silica gel column (ethyl acetate/methanol/triethylamine) to give crude crystals, which were recrystallized from ethyl acetate-hexane to give N-(4-((N-3-hydroxypropyl-N-methyl)aminomethyl)phenyl)-2 -(4-methylphenyl)-6,7-dihydro-5H-benzocycloheptene-8-ca rboxamide (Compound 200) (0.32 g) as colorless crystals.
mp 139-140°C.
¹H-NMR(CDCl₃) δ : 1.72-1.81 (2H, m), 2.13-2.19 (2H, m), 2.25 (3H, s), 2.40 (3H, s), 2.63-2.75 (4H, m), 2.86-2.92 (2H, m), 3.53 (2H, s), 3.79 (2H, t, J=5.4Hz), 7.21-7.32 (3H, m), 7.42-7.52 (6H, m), 7.58 (2H, d, J=8.4Hz), 7.66 (1H, s) .
IR(KBr) ν : 2936, 1651cm⁻¹.

| Anal. for C₃₀H₃₄N₂O₂·0.5H₂O : | | | |
|---|---|---|---|
| Calcd: | C, 77.72; | H, 7.61; | N, 6.04. |
| Found: | C, 77.94; | H, 7.62; | N, 6.15. |

### Working Example 201 (Production of Compound 201)

In dichloromethane(12 ml) was suspended 7-(4-methylphenyl)-2,3-dihydro-1-benzoxepine-4-carboxylic acid (0.4 g), and to the suspension were added oxalyl chloride (0.37 ml) and dimethylformamide (catalytic amount) under ice-cooling. The mixture was stirred at room temperature for 2 hours, and the solvent was evaporated. The residue was dissolved in tetrahydrofuran (15 ml), and the mixture was dropwise added to a solution of 4-((N-3-hydroxy-propyl-N-methyl)aminomethyl)aniline (0.33 g) and tri-ethylamine (0.6 ml) in tetrahydrofuran (15 ml) under ice-cooling. Under nitrogen atmosphere, the mixture was stirred at room temperature overnight, and the solvent was evaporated. To the residue was added water, and the mixture was extracted with ethyl acetate. The organic layer was washed with water and saturated sodium chloride solution and dried with anhydrous magnesium sulfate. Under reduced pressure, the solvent was evaporated, and the residue was purified with silica gel column (ethyl acetate/methanol/triethylamine) to give crude crystals, which were recrystallized from ethyl acetate-hexane to give N-(4-((N-3-hydroxypropyl-N-methyl)aminomethyl)phenyl)-7 -(4-methylphenyl)-2,3-dihydro-1-benzoxepine-4-carboxami de (Compound 201) (0.39 g) as colorless crystals.
mp 119-120°C .
¹H-NMR(CDCl₃) δ : 1.68-1.80 (2H, m), 2.24 (3H, s), 2.39 (3H, s), 2.65 (2H, t, J=5.8Hz), 3.07 (2H, t, J=4.6Hz), 3.52 (2H, s), 3.77 (2H, t, J=5.2Hz), 4.35 (2H, t, J=4.6Hz), 7.05 (1H, d, J=8.4Hz), 7.22-7.31 (3H, m), 7.43-7.52 (5H, m), 7.57 (2H, d, J=8.4Hz), 7.78 (1H,s).
IR(KBr) ν: 3287, 2948, 1649cm⁻¹.

| Anal. for C₂₉H₃₂N₂O₃·0.2H₂O: | | | |
|---|---|---|---|
| Calcd: | C, 75.69; | H, 7.10; | N, 6.09. |
| Found: | C, 75.58; | H, 6.93; | N, 6.08. |

### Working Example 202 (Production of Compound 202)

In dichloromethane (10 ml) was suspended 7-(4-methylphenyl)-2,3-dihydro-1-benzothiepine-4-carboxylic acid (0.3 g), and to the suspension were added oxalyl chloride (0.27 ml) and dimethylformamide (catalytic amount) under ice-cooling. The mixture was stirred at room temperature for 2 hours, and the solvent was evaporated. The residue was dissolved in tetrahydrofuran (15 ml), and the mixture was dropwise added to a solution of 4-(N-methyl-N-(tetrahydropyran-4-yl)aminomethyl)aniline (0.25 g) and triethylamine (0.42 ml) in tetrahydrofuran (15 ml) under ice-cooling. Under nitrogen atmosphere, the mixture was stirred at room temperature overnight, and the solvent was evaporated. To the residue was added water, and the mixture was extracted with ethyl acetate. The organic layer was washed with water and saturated sodium chloride solution and dried with anhydrous magnesium sulfate. Under reduced pressure, the solvent was evaporated to give crude crystals, which were recrystallized from ethyl acetate-hexane to give 7-(4-methylphenyl)-N-(4-((N-tetrahydropyran-4-yl-N-meth yl)aminomethyl)phenyl)-2,3-dihydro-1-benzothiepine-4-ca rboxamide (Compound 202) (0.45 g) as colorless crystals.
mp 177-178°C.
¹H-NMR (CDCl₃) δ : 1.63-1.77 (4H, m), 2.21 (3H, s), 2.40 (3H, s), 2.57-2.70 (1H, m), 3.08 (2H, t, J=5.8Hz), 3.26-3.44 (4H, m), 3.57 (2H, s), 4.01-4.11 (2H, m), 7.24-7.34 (3H, m), 7.40-7.57 (8H, m), 7.70 (1H, s).
IR(KBr) ν: 2949, 1651cm⁻¹.

| Anal. for C₃₁H₃₄N₂O₂S·0.3H₂O : | | | |
|---|---|---|---|
| Calcd: | C, 73.86; | H, 6.92; | N, 5.56. |
| Found: | C, 73.93; | H, 6.73; | N, 5.82. |

### Working Example 203 (Production of Compound 203)

In dichloromethane (6 ml) was suspended 2-(4-methylphenyl)-6,7-dihydro-5H-benzocycloheptene-8-c arboxylic acid (0.25 g), and to the suspension were added oxalyl chloride (0.24 ml) and dimethylformamide (catalytic amount) under ice-cooling. The mixture was stirred at room temperature for 1.5 hours, and the solvent was evaporated. The residue was dissolved in tetrahydrofuran (15 ml, and the mixture was dropwise added to a solution of 4-((N-methyl-N-(pentan-3-yl))aminomethyl)aniline (0.2 g) and triethylamine (0.38 ml) in tetrahydrofuran (15 ml) under ice-cooling. Under nitrogen atmosphere, the mixture was stirred at room temperature for 5 hours, and the solvent was evaporated. To the residue was added water, and the mixture was extracted with ethyl acetate. The organic layer was washed with water and saturated sodium chloride solution and dried with anhydrous magnesium sulfate. Under reduced pressure, the solvent was evaporated to give crude crystals, which were recrystallized from ethyl acetate-hexane to give N-(4-((N-methyl-N-(pentan-3-yl))aminomethyl)phenyl)-2-( 4-methylphenyl)-6,7-dihydro-5H-benzocycloheptene-8-carb oxamide (Compound 203) (0.23 g) as colorless crystals.
mp 112-113°C.
¹H-NMR (CDCl₃) δ : 0.94 (6H, t, J=7.3Hz), 1.26-1.54 (4H, m), 2.14 (3H, s), 2.14-2.32 (3H, m), 2.40 (3H, s), 2.72 (2H, t, J=6.4Hz), 2.86-2.91 (2H, m), 3.55 (2H, s), 7.21-7.27 (3H, m), 7.31-7.56 (8H, m), 7.62 (1H, s).
IR(KBr) ν : 2930, 1651cm⁻¹.

| Anal. for C₃₂H₃₈N₂O: | | | |
|---|---|---|---|
| Calcd: | C, 82.36; | H, 8.21; | N, 6.00. |
| Found: | C, 82.30; | H, 8.05; | N, 5.90. |

### Reference Example 180

To a mixture of 3-(4-methylphenyl)-6,7,8,9-tetra-hydro-5H-benzocyclohep tan-5-one (0.5 g), potassium carbonate (1.65 g) and 18-crown-6 (1.05 g) was added dimethylsulfoxide (10ml) . Undercarbondioxideatmosphere, the mixture was stirred at room temperature for 20 hours, poured into water, acidified with hydrochloric acid and extracted with ethyl acetate. The organic layer was washed with water and subjected to back extraction with sodium hydroxide and water. The aqueous layer was collected, acidified with hydrochloric acid and extracted with ethyl acetate. The organic layer was washed with water and saturated sodium chloride solution and dried with anhydrous magnesium sulfate. The solvent was evaporated to precipitate colorless crystals (0.42 g), which were filtered with hexane and dissolved in ethanol (40 ml). To the mixture was added sodium boron hydride (0.54 g), and the mixture was stirred at room temperature for 1 hour. To the mixture was added water, and the mixture was concentrated, was acidified with hydrochloric acid and extracted with ethyl acetate. The organic layer was washed with water and saturated sodium chloride solution and dried with anhydrous magnesium sulfate. The solvent was evaporated to give colorless crystals (0.41 g), which were dissolved in 80% formic acid (40 ml) . The mixture was stirred at 100°C for 2.5 hours and concentrated. To the residue was added water, and the mixture was extracted with ethyl acetate. The organic layer was washed with water and saturated sodium chloride solution and dried with anhydrous magnesium sulfate. The solvent was evaporated, and the residue was purified with silica gel column (ethyl acetate/hexane) to give 2-(4-methylphenyl)-6,7-dihydro-5H-benzocycloheptene-8-c arboxylic acid (0.14 g) as colorless crystals.
¹H-NMR (CDCl₃) δ : 2.04-2.18 (2H, m), 2.40 (3H, s), 2.70 (2H, t, J=6.8Hz), 2.86-2.91 (2H, m), 7.21-7.28 (3H, m), 7.44-7.56 (4H, m), 7.91 (1H, s).

### Reference Example 181

In dimethylsulfoxide (15 ml) were dissolved 3-(4-methylphenyl)-6,7,8,9-tetrahydro-5H-benzocyclohept an-5-one (0.5g) and 18-crown-6 (1.05g). Under ice-cooling, potassium t-butoxide (1.65 g) was added to the solution. Under carbon dioxide atmosphere, the mixture was stirred at room temperature for 3 hours, poured into water, acidified with hydrochloric acid and extracted with ethyl acetate. The organic layer was washed with water and subjected to back extraction with sodium hydroxide and water. The aqueous layer was collected, acidified with hydrochloric acid and extracted with ethyl acetate. The organic layer was washed with water and saturated sodium chloride solution and dried with anhydrous magnesium sulfate. The solvent was evaporated to precipitate colorless crystals (0.47 g), which were filtered with hexane and dissolved in ethanol (40 ml). To the mixture was added sodium boron hydride (0.58 g), and the mixture was stirred at room temperature for 1 hour. To the mixture was added water, and the mixture was concentrated, acidified with hydrochloric acid and extracted with ethyl acetate. The organic layer was washed with water and saturated sodium chloride solution and dried with anhydrous magnesium sulfate. The solvent was evaporated to precipitate colorless crystals (0.46 g), which were filtered with hexane. To the crystals was added 80% formic acid (10ml), and the mixture was refluxed for 1.5 hours. To the mixture was added water, and the mixture was extracted with ethyl acetate. The organic layer was washed with water and subjected to back extraction with sodium hydroxide and water. The aqueous layer was collected, acidified with hydrochloric acid and extracted with ethyl acetate. The organic layer was washed with water and saturated sodium chloride solution and dried with anhydrous magnesium sulfate. The solvent was evaporated to precipitate 2-(4-methyl-phenyl)-6,7-dihydro-5H-benzocycloheptene-8-carboxylic acid (0.22 g) as colorless crystals.
¹H-NMR (CDCl₃) δ : 2.04-2.16 (2H, m), 2.40 (3H, s), 2.69 (2H, t, J=6.7Hz), 2.86-2.91 (2H, m), 7.21-7.278 (3H, m), 7.44-7.56 (4H, m), 7.89 (1H, s).

### Working Example 204 (Production of Compound 204)

In dimethylformamide (100 ml) was dissolved 7-(4-methylphenyl)-N-(4-((N-(4-oxocyclohexyl)-N-methyl) -aminomethyl)-phenyl)-2,3-dihydro-1-benzoxepine-4-carbo xamide (7.5 g), and to the mixture was added methyl iodide (4.7ml). Under nitrogen atmosphere, the mixture was stirred at room temperature overnight. The solvent was evaporated, and to the residue was added acetone to give dimethyl-(N-(7-(4-methylphenyl)-2,3-dihydro-1-benzoxepin-4-carbonyl)-4-aminobenzyl)-N-(4-oxocyclo-hex yl)ammonium iodide (Compound 204) (8.9 g) as colorless crystals.
¹H-NMR(DMSO-d₆) δ: 2.09-2.24 (2H, m), 2.34 (3H, s), 2.41-2.61 (6H, m), 2.97 (6H, s), 2.97-3.00 (2H, m), 3.79-3.90 (1H, m), 4.31 (2H, t, J=4.4Hz), 4.56 (2H, s), 7.07 (1H, d, J=8.4Hz), 7.27 (2H, d, J=8.2Hz), 7.37 (1H, s), 7.55-7.60 (5H, m), 7.75 (1H, d, J=2.2Hz), 7.88 (2H, d, J=8.8Hz), 10.20 (1H, s) .

### Working Example 205 (Production of Compound 205)

In dimethylformamide (5 ml) was dissolved in 2-(4-(1-pyrrolidinyl)phenyl)-N-(4-((N-tetrahydropyran-4 -yl-N-methyl)aminomethyl)phenyl)-6,7-dihydro-5H-benzo-c ycloheptene-8-carboxamide (0.15 g), and to the mixture was added methyl iodide (0.02 ml). Under nitrogen atmosphere, the mixture was stirred at room temperature overnight. To the mixture was added ethyl acetate, and crude crystal was filtered. The crude crystal was recrystallized from ethanol-ethyl acetate to give dimethyl-(N-(2-(4-(1-pyrrolidinyl)phenyl)-6,7-dihydro-5 H-benzocycloheptene-8-carbonyl)-4-aminobenzyl)-4-tetrah ydropyranylammonium iodide (Compound 205) (0.05 g) as pale brown powder.
¹H-NMR (DMSO-d₆) δ : 1. 80-2.20 (10H, m), 2.63 (2H, t, J=5.6Hz), 2 . 81-2.84 (2H, m), 2.88 (6H, s), 3.24-3.44 (6H, m) ; 3 .54-3 .65 (1H, m), 4.02-4.11 (2H, m), 4.46 (2H, s), 6.62 (2H, d, J=9.0Hz), 7.25 (1H, d, J=7.8Hz), 7.36-7.60 (7H, m), 7.88 (2H, d,J=8.4Hz), 10.22 (1H, s).
IR(KBr) ν : 2967, 1663, 1609cm⁻¹.

| Anal. for C₃₆H₄₄IN₃O₂·H₂O: | | | |
|---|---|---|---|
| Calcd: | C, 62.15; | H, 6.66; | N, 6.04. |
| Found: | C, 61.89; | H, 6.30; | N, 5.97. |

### Working Example 206 (Production of Compound 206)

In dimethylformamide (5 ml) was dissolved N- (4- ((N-3-hydroxypropyl-N-methyl)aminomethyl)phenyl)-2-(4-methylphenyl)-6,7-dihydro-5H-benzocycloheptene-8-carboxamide (0.2 g), and to the mixture was added methyl iodide (0.04 ml). Under nitrogen atmosphere, the mixture was stirred at room temperature overnight. The solvent was evaporated, and to the residue was added ethyl acetate to give crude crystals, which were filtered and recrystallized from ethanol-ethyl acetate to give N-(3-hydroxypropyl)-N,N-dimethyl-(N-(2-(4-methylphenyl) -6,7-dihydro-5H-benzo-cycloheptene-8-carbonyl)-4-aminob enzyl)ammonium iodide (Compound 206) (0.05 g) as colorless crystals.
mp 210-213°C.
¹H-NMR(CDCl₃+CD₃OD) δ: 2.00-2.20 (4H, m), 2.40 (3H, s), 2.71 (2H, t, J=6.6Hz), 2.87-2.92 (2H, m), 3.10 (6H, s), 3.54-3.65 (2H, m), 3.73 (2H, t, J=5.3Hz), 4.63 (2H, s), 7.22-7.27 (3H, m), 7.43-7.58 (7H, m), 7.80 (2H, d, J=8.4Hz), 9.21 (1H, s).
IR(KBr) ν : 3337, 2934, 1653cm⁻¹.

| Anal. for C₃₁H₃₇IN₂O₂·0.5H₂O : | | | |
|---|---|---|---|
| Calcd: | C, 61.49; | H, 6.33; | N, 4.63. |
| Found: | C, 61.55; | H, 6.22; | N, 4.74. |

### Working Example 207 (Production of Compound 207)

In dimethylformamide (5 ml) was dissolved N- (4- ((N-3-hydroxypropyl-N-methyl)aminomethyl)phenyl)-7-(4-methylphenyl)-2,3-dihydro-1-benzoxepine-4-carboxamide (0.14 g), and to the mixture was added methyl iodide (0.04 ml). Under nitrogen atmosphere, the mixture was stirred at room temperature overnight. The solvent was evaporated, and to the residue was added ethyl acetate to give crude crystals, which were filtered and recrystallized from ethanol-ethyl acetate to give dimethyl-3-hydroxypropyl-(N-(7-(4-methylphenyl)-2,3-dihydro-1-benzoxepin-4-carbonyl)-4-am inobenzyl)ammonium iodide (Compound 207) (0.15 g) as colorless crystals.
mp 216-219°C.
¹H-NMR(CDCl₃+CD₃OD) δ: 2 . 00-2 .20 (2H, m), 2.40 (3H, s), 3.06-3.10 (2H, m), 3.10 (6H, s), 3.51-3.61 (2H, m), 3.73 (2H, t, J=5.4Hz), 4.37 (2H, t, J=4.6Hz), 4.61 (2H, s), 7.07 (1H, d, J=8.4Hz), 7.25 (2H, d, J=8.2Hz), 7.46-7.59 (7H, m), 7.81 (2H, d, J=8.2Hz), 9.54 (1H, s).
IR (KBr) ν : 3306, 1651cm⁻¹.

| Anal. for C₃₀H₃₅IN₂O₃·0.5H₂O: | | | |
|---|---|---|---|
| Calcd: | C, 59.31; | H, 5.97; | N, 4.61. |
| Found: | C, 59.36; | H, 5.95; | N, 4.75. |

### Working Example 208 (Production of Compound 208)

In dimethylformamide (5 ml) was dissolved 7-(4-methylphenyl)-N-(4-((N-tetrahydropyran-4-yl-N-methyl)-a minomethyl)-phenyl)-2,3-dihydro-1-benzothiepine-4-carbo xamide (0.19 g), and to the mixture was added methyl iodide (0.03 ml). Under nitrogen atmosphere, the mixture was stirred at room temperature overnight. The solvent was evaporated, and to the residue was added ethyl acetate to give crude crystals, which were filtered and recrystallized from ethanol-hexane to give dimethyl-(N-(7-(4-methyl-phenyl)-2,3-dihydro-1-benzothi epine-4-carbonyl)-4-aminobenzyl)-N-(4-tetrahydropyranyl)ammonium iodide (Compound 208) (0.2 g) as colorless crystals.
mp 220-222°C (dec.).
¹H-NMR(DMSO-d₆) δ : 1.78-1.95 (2H, m), 2.05-2.20 (2H, m), 2.35 (3H, s), 2.88 (6H, s), 2.95-3.05 (2H, m), 3.21-3.32 (4H, m), 3.50-3.65 (1H, m), 4.05-4.15 (2H, m), 4.46 (2H, s), 7.29 (2H, d, J=8.0Hz), 7.46-7.63 (7H, m), 7.81-7.90 (3H, m), 10.34 (1H, s).
IR(KBr) ν : 2924, 1657cm⁻¹.

### Working Example 209 (Production of Compound 209)

In dimethylformamide (5 ml) was dissolved N-(4-((N-methyl-N-(pentan-3-yl))aminomethyl)phenyl)-2-( 4-methylphenyl)-6,7-dihydro-5H-benzocycloheptene-8-carb oxamide (0.17 g), and to the mixture was added methyl iodide (0.08 ml). Under nitrogen atmosphere, the mixture was stirred at 45°C overnight. The solvent was evaporated, and to the residue was added ethyl acetate to give crude crystals, which were filtered and recrystallized from ethanol-ethyl acetate to give dimethyl-(N-(2-(4-methyl-phenyl)-6,7-dihydro-5H-benzocy cloheptene-8-carbonyl)-4-aminobenzyl)-N-(pentan-3-yl)am monium iodide (Compound 209) (0.15 g) as colorless crystals.
mp 190-194°C (dec.).
¹H-NMR (CDCl₃) δ : 1.15 (6H, t, J=7.4Hz), 1.67-1.82 (2H, m), 2.05-2.25 (4H, m), 2.39 (3H, s), 2.73 (2H, t, J=6.6Hz), 2.80-2.90 (2H, m), 3.11 (6H, s), 3.40-3.51 (1H, m), 4.91 (2H, s), 7.18-7.26 (3H, m), 7.44 (1H, dd, J=1.8, 8.4Hz), 7.49 (2H, d, J=8.4Hz), 7.57-7.62 (4H, m), 7.80 (2H, d, J=8.4Hz), 8.35 (1H, s).
IR (KBr) ν : 2936, 1659cm⁻¹.

| Anal. for C₃₃H₄₁IN₂O·0.5H₂O: | | | |
|---|---|---|---|
| Calcd: | C, 64.18; | H, 6.85; | N, 4.54. |
| Found: | C, 63.84; | H, 6.73; | N, 4.47. |

### Reference Example 182

In DMF (50 ml) was dissolved N-cyclohexyl-N-methylamine (12.5 g, 0.11 mol), and to the solution were added potassium carbonate (27.6 g, 0.20 mol) and 4-nitrobenzylbromide (21.6 g, 0.10 mol). The mixture was stirred at room temperature for 5 hours. Under reduced pressure, the reaction mixture was concentrated. To the residue was added ethyl acetate, and the mixture was extracted with water. The ethyl acetate layer was washed with saturated sodium chloride solution, dried with MgSO₄ and concentrated under reduced pressure. The residue was purified with silica gel column chromatography (ethyl acetate/hexane) to give N-cyclohexyl-N-methyl-N-(4-nitrobenzyl)amine (24.8 g).
¹H-NMR (200 MHz, CDCl₃) δ : 1.0-1.95 (10H, m), 2.19 (3H, s), 3.66 (2H, s), 7.51 (2H, d, J=8.8Hz), 8.17 (2H, d, J=8.8Hz) .

### Reference Example 183

To a solution of N-cyclohexyl-N-methyl-N-(4-nitrobenzyl)amine (12.4 g, 50.0 mmol) in methanol (250 ml) were added nickel bromide (1.09 g, 5.0 mmol) and then sodium boron hydride (7.57 g, 200 mmol) at 0°C, and the mixture was stirred at room temperature for 30 minutes. To the mixture were added nickel bromide (0.55 g, 2.5 mmol) and then sodium boron hydride (3.78 g, 100 mmol) at 0°C, and the mixture was stirred at room temperature for 30 minutes. To the reaction mixture was added water (100 ml), and the mixture was concentrated under reduced pressure. To the residue was added ethyl acetate, and insoluble material was filtered off with Celite. The filtrate was washed with ethyl acetate, and the ethyl acetate layer was dried with MgSO₄ and concentrated under reduced pressure. The residue was washed with hexane to give 4-(N-cyclohexyl-N-methylaminomethyl)aniline (3.99 g, 37%).
¹H-NMR (200 MHz, CDCl₃) δ: 1.0-1.95 (10H, m), 2.17 (3H, s), 2.3-2.55 (1H, m), 3.46 (2H, s), 3.59 (2H, br s), 6.65 (2H, d, J=8.5Hz), 7.10 (2H, d, J=8.5Hz).

### Working Example 210 (Production of Compound 210)

To a solution of 7-(4-methylphenyl)-2,3-dihydro-1-benzoxepine-4-carboxylic acid (0.28 g), 4-(N-cyclohexyl-N-methylaminomethyl)aniline (0.24 g) and 1-hydroxybenzotriazole (0.15 g) in dimethylformamide (10 ml) was added 1-ethyl-3-(3-dimethylaminopropyl)carbodiimide hydrochloride (0.29 g) under ice-cooling. Under nitrogen atmosphere, the mixture was cooled to room temperature, and to the mixture were added 4-dimethylaminopyridine (3 mg) and triethylamine (0.42 ml). The mixture was stirred for 20 hours, poured into water, and extracted with ethyl acetate. The organic layer was washed with water and saturated sodium chloride solution and dried with anhydrous magnesium sulfate. Under reduced pressure, the solvent was evaporated, and the residue was washed with ethyl acetate and dried to give N-(4-(N-cyclohexyl-N-methylaminomethyl)phenyl)-7-(4-met hylphenyl)-2,3-dihydro-1-benzoxepine-4-carboxamide (Compound 210) (0.40 g).
¹H-NMR(CDCl₃) δ : 1.0-1.95 (10H, m), 2.20 (3H, s), 2.35-2.55 (1H, m), 2.40 (3H, s), 3.0-3.15 (2H, m), 3.56 (2H, s), 4.3-4.45 (2H, m), 7.06 (1H, d, J=8.4Hz), 7.2-7.6 (11H, m).

### Working Example 211 (Production of Compound 211)

In dimethylformamide (7 ml) was dissolved N-(4-(N-cyclohexyl-N-methylaminomethyl)phenyl)-7-(4-methyl-phen yl)-2,3-dihydro-1-benzoxepine-4-carboxamide (0.15 g), and to the mixture was added methyl iodide (0.06 ml). Under nitrogen atmosphere, the mixture was stirred at room temperature for 20 hours. The solvent was evaporated, and to the residue was added ethyl acetate to give crude crystals, which were filtered and recrystallized from ethanol to give N-cyclohexyl-N,N-dimethyl-N-((7-(4-methylphenyl)-2,3-di hydro-1-benzoxepin-4-carbonyl)-4-aminobenzyl)ammonium iodide (Compound 211) (0.15 g).
¹H-NMR(CDCl₃) δ : 1.0-1.8 (6H, m), 1.9-2.05 (2H, m), 2.25-2.45 (2H, m), 2.36 (3H, s), 2.95-3.15 (8H, m), 3.45-3.7 (1H, m), 4.2-4.35 (2H, m), 4.83 (2H, s), 6.99 (1H, d, J=8.4Hz), 7.21 (2H, d, J=7.6Hz), 7.35-7.6 (6H, m), 7.74 (1H, d, J=2.2Hz), 7.85 (2H, d, J=8.6Hz), 8.79 (1H, s).
IR(KBr) ν : 1659, 1609, 1593, 1518, 1493cm⁻¹.

### Working Example 212 (Production of Compound 212)

In dimethylformamide (5 ml) was dissolved N-(4-(N-methyl-N-(tetrahydropyran-4-yl)aminomethyl)phen yl)-7-(4-morpholino-phenyl)-2,3-dihydro-1-benzoxepine-4 -carboxamide (0.20 g), and to the mixture was added methyl iodide (0.03 ml). Under nitrogen atmosphere, the mixture was stirred at room temperature for 32 hours. The solvent was evaporated, and the residue was purified with silica gel column chromatography (dichloromethane/methanol). The desired fraction was concentrated, and to the residue was added ethyl acetate. Insoluble material was filtered and recrystallized from ethanol to give dimethyl-N-(7-(4-morpholinophenyl)-2,3-dihydro-1-benzox epin-4-carbonyl)-4-aminobenzyl-N-(4-tetrahydropyranyl)a mmonium iodide
(Compound 212) (0.18 g).
¹H-NMR (CDCl₃) δ : 1.6-2.0 (2H, m), 2.1-2.3 (2H, m), 2.92 (6H, s), 2.95-3.2 (6H, m), 3.35-3.55 (2H, m), 3.8-3.9 (4H, m), 4.0-4.35 (5H, m), 4.84 (2H, s), 6. 85-7.05 (3H, m), 7.35-7.85 (9H, m), 8.92 (1H, s).
IR(KBr) ν : 1659, 1609, 1520, 1495cm⁻¹.

### Reference Example 184

In tetrahydrofuran(100 ml) was dissolved 1,2-methlenedioxy-4-bromobenzene (24.0 g), and to the mixture was dropwise added n-butyllithium (1.6M hexane solution, 82 ml) at -55°C or less. The mixture was stirred at -70°C or less for 30 minutes. The resulting solution was dropwise added to a solution of trimethyl borate (18.6 g) in tetrahydrofuran (50 ml) at -60°C or less through cannula, and the mixture was stirred at -70°C or less for 1 hour and then for 2 hours while warming the mixture to room temperature. To the reaction mixture were added 1N hydrochloric acid (130 ml) and diethylether (150 ml), and the organic layer was separated. The organic layer was washed with water and saturated sodium chloride solution and dried with anhydrous magnesium sulfate. Under reduced pressure, the solvent was evaporated. The residue was washed with diisopropylether to give 3,4-methlene-dioxyphenyl borate (6.79 g).
¹H-NMR(DMSO-d₆) δ : 5.99 (2H, s), 6.8-6.95 (1H, m), 7.25-7.45 (2H, m).

### Reference Example 185

To a mixture of methyl 7-bromo-2,3-dihydro-1-benzoxepine-4-carboxylate (0.57 g), 3,4-methlenedioxy-phenyl borate (0.47 g) and sodium carbonate (0.42 g) were added water (2 ml) and 1,2-dimethoxyethane(12 ml). Under argon atmosphere, the mixture was stirred at room temperature for 30 minutes, and to the mixture was added tetrakistriphenylphosphinepalladium (0.16 g). The mixture was stirred at 80°C for 14 hours and extracted with ethyl acetate. The organic layer was washed with water and saturated sodium chloride solution and dried with anhydrous magnesium sulfate. Under reduced pressure, the solvent was evaporated, and the residue was purified with silica gel column (ethyl acetate/hexane) to give methyl 7-(3,4-methlenedioxyphenyl)-2,3-dihydro-1-benzoxepine-4 -carboxylate (0.43 g).
¹H-NMR(CDCl₃) δ : 2.95-3.10 (2H, m), 3.83 (3H, s), 4.25-4.35 (2H, m), 6.01 (2H, s), 6.87 (1H, d, J=8.6Hz), 6.95-7.10 (3H, m), 7.40 (1H, dd, J=8.4, 2.4Hz), 7.47 (1H, d, J=2.2Hz), 7.65 (1H, s).

### Reference Example 186

To methyl 7-(3,4-methlenedioxyphenyl)-2,3-dihydro-1-benzoxepine-4 -carboxylate (0.40 g) were added methanol (5 ml) and 1N sodium hydroxide (3.7 ml), and the mixture was stirred at room temperature for 20 hours. To the mixture was added 1N hydrochloric acid (3.7 ml), and the mixture was concentrated under reduced pressure. Precipitate was washed with water and diethylether and dried under reduced pressure to give 7-(3,4-methylene-dioxyphenyl)-2,3-dihydro-1-benzoxepine -4-carboxylic acid (0.32 g).
¹H-NMR(DMSO-d₆) δ : 2.80-2.95 (2H, m), 4.15-4.35 (2H, m), 6.05 (2H, s), 6. 97 (1H, d, J=8.1Hz), 7. 01 (1H, d, J=8.4Hz), 7.16 (1H, dd, J=8.1, 1.7Hz), 7.29 (1H, d, J=1.7Hz), 7.53 (2H, dd, J=8.4, 2.3Hz), 7.63 (1H, s), 7.74 (1H, d, J=2.3Hz). Working Example 213 (Production of Compound 213)

To a solution of 7-(3,4-methlenedioxyphenyl)-2,3-dihydro-1-benzoxepine-4 -carboxylic acid (0.14 g), 4-(N-methyl-N-(tetrahydropyran-4-yl)aminomethyl)aniline (0.11 g) and 1-hydroxy-benzotriazole (0.15 g) in dimethylformamide (10 ml) was added 1-ethyl-3-(3-dimethyl-aminopropyl)carbodiimide hydrochloride (0.13 g) under ice-cooling. Under nitrogen atmosphere, the reaction mixture was warmed to room temperature. To the mixture were added 4-dimethylaminopyridine (3 mg) and triethylamine (0.19 ml), and the mixture was stirred for 18 hours, poured into water, and extracted with ethyl acetate. The organic layer was washed with water and saturated sodium chloride solution, and dried with anhydrous magnesium sulfate. Under reduced pressure, the solvent was evaporated, and the residue was purified with silica gel column (ethyl acetate) to give 7-(3,4-methlenedioxyphenyl)-4-(N-methyl-N-(tetrahydropyran-4-yl)aminomethyl)phenyl)-2,3-dihydro-1 -benzoxepine-4-carboxamide (Compound 213) (0.19 g).
¹H-NMR (CDCl₃) δ: 1.55-1.85 (4H, m), 2.21 (3H, s), 2.55-2.80 (1H, m), 3.00-3.15 (2H, m), 3.30-3.45 (2H, m), 3.58 (2H, s), 3.95-4.15 (2H, m), 4.30-4.45 (2H, m), 6.01 (2H, s), 6.88 (1H, d, J=8.6Hz), 6.95-7.10 (3H, m), 7.20-7.65 (7H, m).
IR (KBr) ν : 1653, 1597, 1514, 1483cm⁻¹.

### Working Example 214 (Production of Compound 214)

In dimethylformamide (5 ml) was dissolved 7-(3,4-methlenedioxyphenyl)-4-(N-methyl-N-(tetrahydropy ran-4-yl)aminomethyl)phenyl)-2,3-dihydro-1-benzoxepine-4-carboxamide (95 mg), and to the mixture was added methyl iodide (0.012 ml). Under nitrogen atmosphere, the mixture was stirred at room temperature for 18 hours. The solvent was evaporated, and to the residue was added ethyl acetate. Insoluble material was filtered and recrystallized from ethanol to give dimethyl-N-(7-(3,4-methylenedioxyphenyl)-2,3-dihydro-1-benzoxepin-4-carbonyl)-4-amino-be nzyl-N-(4-tetrahydropyranyl)ammoniumiodide (Compound 214) (101 mg).
¹H-NMR (CDCl₃) δ : 1.7-2.0 (2H, m), 2.15-2.3 (2H, m), 2.85-3.1 (8H, m), 3.4-3.55 (2H, m), 4.0-4.35 (5H, m), 4.85 (2H, s), 5.96 (2H, s), 6.81 (1H, d, J=7.8Hz), 6.9-7.1 (3H, m), 7.25-7.7 (5H, m), 7.83 (2H, d, J=8.2 Hz), 8.89 (1H, s).
IR (KBr) ν : 1659, 1609, 1520, 1495cm⁻¹.

### Working Example 215 (Production of Compound 215)

In aqueous methanol was dissolved N,N-dimethyl-N-(4-(((2-(4-methylphenyl)-6,7-dihydro-5H-benzocyclo-hepten-8-yl)carbonyl)amino)benzyl)-N-(4-tetr ahydro-pyranyl)ammonium iodide (19 g), and the mixture was subjected to ion exchange resin (DOWEX1-x8, 100-200 mesh, Cl⁻ type) column, which was eluted with aqueous methanol. The solvent of the desired fractions was evaporated, and to the residue was added acetone to give crude crystals, which were recrystallized from ethanol to give N,N-dimethyl-N-(4-(((2-(4-methylphenyl)-6,7-dihydro-5H-benzocyclohepten-8-yl)carbonyl)amino)benzyl)-N-(4-tetra hydropyranyl) ammonium chloride (Compound 215) (10.1 g) as colorless crystals.
mp 226-232°C(dec.).
¹H-NMR(CDCl₃+CD₃OD) δ: 1.80-2.00 (2H, m), 2.07-2.26 (4H, m), 2.39 (3H, s), 2.72 (2H, t, J=6.6Hz), 2.85-2.91 (2H, m), 3.00 (6H, s), 3.54 (2H, t, J=11.3Hz), 4.00-4.21 (3H, m), 4.70 (2H, s), 7.21-7.29 (3H, m), 7.42-7.56 (7H, m), 7.81 (2H, d, J=8.4Hz), 9.06 (1H, s).
IR (KBr) ν : 2934, 1655 cm⁻¹.

| Anal. for C₃₃H₃₉ClN₂O₂: | | | | |
|---|---|---|---|---|
| Calcd: | C, 74.62; | H, 7.40; | N, 5.27; | Cl, 6.67. |
| Found: | C, 74.35; | H, 7.33; | N, 5.20; | Cl, 6.80. |

### Working Example 215a (Production of Compound 215)

To a solution of N-(4-chloromethylphenyl)-2-(4-methylphenyl)-6,7-dihydro -5H-benzocycloheptene-8-carboxamide (9.38 g, 23.3 mmol) in DMF (50 ml) was dropwise added a solution of N,N-dimethyl-N-tetrahydropyran-4-ylamine (4.5 g, 35.0 mmol) inDMF (50 ml). Under nitrogen atmosphere, the mixture was stirred for 23 hours. The solvent was evaporated to give powder, which was washed with acetone and dried. The resulting colorless powder was recrystallized from ethanol to give N,N-dimethyl-N-(4-(((2-(4-methylphenyl)-6,7-dihydro-5H-benzocyclohepten-8-yl)carbonyl)amino)benzyl)-N-(4-tetra hydropyranyl)-ammonium chloride (Compound 215) (10.6 g, 86%) as colorless powder.

### Working Example 216 (Production of Compound 216)

In aqueous acetonitrile was dissolved N,N-dimethyl-N-(4-(((7-(4-methylphenyl)-2,3-dihydro-1-benzoxepin-4-y l)carbonyl)amino)benzyl)-N-(4-oxocyclohexyl)ammonium iodide (22.8 g), and the mixture was subjected to ion exchange resin (DOWEX-SBR, Cl⁻ type) column, which was eluted with aqueous acetonitrile. The solvent of the desired fractions was evaporated, and the residue was dissolved in water. The mixture was subjected to freeze-drying to give N,N-dimethyl-N-(4-(((7-(4-methylphenyl)-2,3-dihydro-1-b enzoxepin-4-yl)carbonyl)amino)benzyl)-N-(4-oxocyclo-hex yl)ammonium chloride (Compound 216) (16.1 g) as colorless powder.
¹H-NMR(DMSO-d₆) δ : 2.05-2.25 (2H, m), 2.34 (3H, s), 2.41-2.61 (6H, m), 2.97 (6H, s), 2.97-3.00 (2H, m), 3.75-3.90 (1H, m), 4.30 (2H, t, J=4.4Hz), 4.57 (2H, s), 7.06 (1H, d, J=8.4Hz), 7.27 (2H, d, J=7.8Hz), 7.45 (1H, s), 7.53-7.60 (5H, m), 7.78 (1H, d, J=2.2Hz), 7.92 (2H, d, J=8.4Hz), 10.34 (1H, s).
IR(KBr) ν: 3025, 2967, 1717, 1655cm⁻¹.

| Anal. for C₃₃H₃₇ClN₂O₃·0.5H₂O: | | | | |
|---|---|---|---|---|
| Calcd: | C, 71.53; | H, 6.91; | N, 5.06; | Cl, 6.40. |
| Found: | C, 71.21; | H, 6.94; | N, 4.94; | Cl, 6.24. |

### Working Example 216a (Production of Compound 216)

To a solution of N-(4-chloromethylphenyl)-7-(4-methylphenyl)-2,3-dihydro -1-benzoxepine-4-carboxamide (214 mg, 0.530 mmol) in N,N-dimethylformamide (1 ml) was dropwise added a solution of 4-dimethylaminocyclohexanone (112 mg, 0.795 mmol) in N,N-dimethylformamide (1 ml). Under nitrogen atmosphere, the mixture was stirred for 14 hours. The solvent was evaporated to give crude product, which was washed with ether to give N,N-dimethyl-N-(4-(((7-(4-methylphenyl)-2,3-dihydro-1-b enzoxepin-4-yl)carbonyl)-amino)benzyl)-N-(4-oxocyclohex yl)ammonium chloride (Compound 216) (305 mg) as colorless powder.

### Working Example 217 (Production of Compound 217)

To a solution of N-(4-chloromethylphenyl)-7-(4-ethoxyphenyl)-2,3-dihydro -1-benzoxepine-4-carboxamide (2.38 g) in DMF (20 ml) was added N,N-dimethyl-N-tetrahydropyran-4-ylamine (1.42 g) at room temperature, and the mixture was stirred for 14 hours. To the reaction mixture was added ethyl acetate (100 ml) to precipitate crystals, which were collected by filtration. The crystal was washed with ethyl acetate to give crude product as pale yellow crystals, which were recrystallized from ethanol to give as N-(4-(((7-(4-ethoxyphenyl)-2,3-dihydro-1-benzoxepin-4-y l)carbonyl)amino)benzyl)-N,N-dimethyl-N-(4-tetrahydropy ranyl)ammonium chloride (Compound 217) (1.29 g) colorless crystals.
m.p. 200-204 °C
¹H-NMR (200MHz, DMSO-d₆) δ:1.35 (3H, t, J=7.0 Hz), 1.75-1.98 (2H, m), 2.06-2.24 (2H, m), 2.88 (6H, s), 2.94-3.05 (2H, m), 3.28-3.43 (2H, m), 3.49-3.69 (1H, m), 3.99-4.13 (2H, m), 4.07 (2H, q, J=7.0 Hz), 4.23-4.35 (2H, m), 4.47 (2H, s), 6.98-7.07 (3H, m), 7.37 (1H, s), 7.50-7.61 (5H, m), 7.72 (1H, d, J=2.2 Hz), 7.87 (2H, d, J=8.4 Hz), 10.22 (1H, s).
IR (KBr) ν : 3425, 1647, 1603, 1520, 1489, 1407, 1317, 1294, 1240, 831 cm⁻¹

| Anal. for C₃₃H₃₉N₂O₄Cl | | | | |
|---|---|---|---|---|
| Calcd: | C, 70.38 ; | H, 6.98 ; | N, 4.97 ; | Cl, 6.30 |
| Found: | C, 70.49 ; | H, 7.08 ; | N, 4.94 ; | Cl, 6.19. |

### Working Example 217a (Production of Compound 217)

In aqueous methanol was dissolved N-(4-(((7-(4-ethoxyphenyl)-2,3-dihydro-1-benzoxepin-4-y l)carbonyl)-amino)benzyl)-N,N-dimethyl-N-(4-tetrahydrop yranyl)-ammonium iodide (26.6 g), and the mixture was subjected to ion exchange resin (DOWEX-SBR, Cl⁻ type) column, which was eluted with aqueous methanol. The solvent of the desired fractions was evaporated, and to the residue was added acetone to give crude crystals, which were recrystallized from ethanol to give N-(4-(((7-(4-ethoxyphenyl)-2,3-dihydro-1-benzoxepin-4-y 1)carbonyl)amino)benzyl)-N,N-dimethyl-N-(4-tetrahydropy ranyl) ammonium chloride (Compound 217) (16.6g) as colorless crystals.

### Working Example 218 (Production of Compound 218)

To a solution of N-(4-((N-tetrahydrothiopyran-4-yl-N-methyl)aminomethyl) phenyl)-7-(4-methylphenyl)-2,3-dihydro-1-benzoxepine-4-carboxamide (0.2g) in dichloromethane (10ml) was added mCPBA (0.1g) at -10 to -20°C, and the mixture was stirred for 30 minutes. To the mixture was added sodium thiosulfate solution, and the mixture was concentrated and extracted with ethyl acetate. The organic layer was washed with sodium hydrogen carbonate solution, water and saturated brine and dried with anhydrous magnesium sulfate. Under reduced pressure, the solvent was evaporated, and the residue was purified with silica gel column (methanol/triethylamine/ethyl acetate) to give N-(4-((N-(1-oxotetrahydrothiopyran-4-yl)-N-methyl)-amin omethyl)phenyl)7-(4-methylphenyl)-2,3-dihydro-1-benzoxe pine-4-carboxamide (Compound 218) (E,Z mixture: 0.12g) as colorless powder.
¹H-NMR(δ ppm, CDCl₃) 1.80-1.97 (2H, m), 2.17 (1.4H, S), 2.28 (1.6H, s), 2.37-2.51 (3H, m), 2.39 (3H, S), 2.56-2.73 (2H, m), 3.08 (2H, t, J=4.7Hz), 3.15-3.28 (2H, m), 3.54 (0.9H, s), 3.63 (1.1H, s), 4.36 (2H, t, J=4.7Hz), 7.06 (1H, d, J=8.4Hz), 7.23-7.34 (4H, m), 7.44-7.57 (6H, m), 7.64 (1H, s).
IR (KBr) ν : 3279, 2946, 1651cm⁻¹.

| | | | |
|---|---|---|---|
| Anal. Calcd. for C₃₁H₃₄N₂O₃S : | C,72.34; | H,6.66; | N,5.44. |
| Found | C,72.31; | H,6.66; | N,5.35. |

### Working Example 219 (Production of Compound 219)

To a suspension of 2-(4-methylphenyl)-6,7-dihydro-5H-benzocycloheptene-8-c arboxylic acid (0.15g) in dichloromethane (5ml) were added under ice-cooling oxalyl chloride (0.15ml) and dimethylformamide (catalytic amount), and the mixture was stirred at room temperature for 2 hours. The solvent was evaporated, and the residue was dissolved in tetrahydrofuran (15ml). The mixture was added dropwise, under ice-cooling, to a mixture of 1-(4-aminobenzyl)phosphorinane-1-oxide (0.13g) and triethylamine (0.23ml) in tetrahydrofuran (15ml). Under nitrogen atmosphere, the mixture was stirred at room temperature overnight. The mixture was poured into water and extracted with ethyl acetate. The organic layer was washed with water and saturated brine, and dried with anhydrous magnesium sulfate. Under reduced pressure, the solvent was evaporated to give crude crystals, which were recrystallized from ethanol/hexane to give 2-(4-methyl-phenyl)-N-(4-((1-oxophosphorinane-1-yl)meth yl)-phenyl)-6,7-dihydro-5H-benzocycloheptene-8-carboxam ide (Compound 219) (0.16g) as colorless crystals.
mp 282-283°C(dec.).
¹H-NMR (δ ppm, CDCl₃) 1.40-1.60 (2H, m), 1.70-1.80 (6H, m), 1.80-2.20. (4H, m), 2.40 (3H, s), 2.72 (2H, t, J=6.6Hz), 2.86-2.95 (2H, m), 3.16 (2H, d, J=13.6Hz), 7.15-7.26 (4H, m), 7.42-7.52 (5H, m), 7.60 (2H, d, J=8.0Hz), 7.80 (1H, s).
IR (KBr) ν : 2932, 1659cm⁻¹.

| Anal. Calcd. for C₃₁H₃₄NO₂P·0.2H₂O : | | | |
|---|---|---|---|
| | C, 76.43 ; | H, 7.12 ; | N,2.87. |
| Found | C,76.20; | H,7.31; | N,3.00. |

### Working Example 220 (Production of Compound 220)

To a suspension of 2-(4-methylphenyl)-6,7-dihydro-5H-benzocycloheptene-8-c arboxylic acid (0.3g) in dichloromethane (5ml) were added under ice-cooling oxalyl chloride (0.3ml) and dimethylformamide (catalytic amount), and the mixture was stirred at room temperature for 2 hours. The solvent was evaporated, and the residue was dissolved in tetrahydrofuran (10ml). The mixture was added dropwise, under ice-cooling, to a mixture of 4-(N-methyl-N-(tetrahydrothiopyran-4-yl)-aminomethyl)aniline (0.27g) and triethylamine (0.45ml) in tetrahydrofuran (10ml). Under nitrogen atmosphere, the mixture was stirred at room temperature for 4 hours. The solvent was evaporated, and to the residue was added water. The mixture was extracted with ethyl acetate, and the organic layer was washed with water and saturated brine, and dried with anhydrous magnesium sulfate. Under reduced pressure, the solvent was evaporated to give crude crystals, which were recrystallized from ethyl acetate/hexane to give N-(4-((N-tetrahydrothiopyran-4-yl-N-methyl)aminomethyl) -phenyl)-2-(4-methylphenyl)-6,7-dihydro-5H-benzocyclo-h eptene-8-carboxamide (Compound 220) (0.45g) as colorless crystals.
mp 177-178°C.
¹H-NMR (δ ppm, CDCl₃) 1.65-1.85 (2H, m), 2.14-2.20 (2H, m), 2.22 (3H, s), 2.40 (3H, s), 2.47-2.53 (1H, m), 2.68-2.72 (6H, m), 2.86-2.92 (2H, m), 3.58 (2H, s), 7.21-7.27 (2H, m), 7.31 (2H, d, J=8.4Hz), 7.42-7.52 (5H, m), 7.56 (2H, d, J=8.4Hz), 7.63 (1H, s).
IR(KBr) ν : 2932, 1651cm⁻¹.

| Anal. Calcd. for C₃₂H₃₆N₂OS·0.2H₂O: | | | |
|---|---|---|---|
| | C,76.82; | H,7.33; | N,5.60. |
| Found | C,76.89; | H,7.35; | N,5.64. |

### Working Example 221 (Production of Compound 221a and 221b)

To a solution of N-(4-((N-tetrahydrothiopyran-4-yl-N-methyl)aminomethyl) phenyl)-2-(4-methylphenyl)-6,7-dihydro-5H-benzocyclohep tene-8-carboxamide (0.3g) in dichloromethane (20ml) was added mCPBA (0.18g) at -10 to -20°C, and the mixture was stirred for 1.5 hours. To the mixture was added sodium thiosulfate solution, and the mixture was concentrated and extracted with ethyl acetate. The organic layer was washed with sodium hydrogen carbonate solution, water and saturated brine, and dried with anhydrous magnesium sulfate. Under reduced pressure, the solvent was evaporated, and the residue was purified with silica gel column (methanol/triethylamine/ethyl acetate) to give two kinds of crude crystals, each of which was recrystallized from ethyl acetate/ethanol/hexane to give (E) or (Z)-N-(4-((N-(1-oxotetrahydrothiopyran-4-yl)-N-methyl)a minomethyl)phenyl)-2-(4-methylphenyl)-6,7-dihydro-5H-be nzocycloheptene-8-carboxamide (Compound 221a) (76mg) and (Z) or (E)-N-(4-((N-(1-oxotetrahydro-thiopyran-4-yl)-N-methyl) aminomethyl)phenyl)-2-(4-methylphenyl)-6,7-dihydro-5H-b enzocycloheptene-8-carboxamide (Compound 221b) (0.11g) as colorless crystals, respectively.

### Compound 2214a:

mp 218-219°C.
¹H-NMR(δ ppm, CDCl₃) 1.80-2.00 (2H, m), 2.10-2.20 (2H, m), 2.19 (3H, s), 2.25-2.39 (2H, m), 2.40 (3H, S), 2.61-2.76 (5H, m), 2.86-2.92 (2H, m), 3.23-3.33 (2H, m), 3.57 (2H, s), 7.22-7.31 (4H, m), 7.42-7.52 (5H, m), 7.58 (2H, d, J=8.4Hz), 7.66 (1H, s).

| Anal. Calcd. for C₃₂H₃₆N₂O₂S·0.2H₂O : | | | |
|---|---|---|---|
| | C, 74.44; | H,7.11; | N,5.43. |
| Found | C,74.43; | H,7.18; | N,5.66. |

### Compound 221b:

mp 216-218°C.
¹H-NMR (δ ppm, CDCl₃) 1.80-2.00 (3H, m), 2.10-2.25 (3H, m), 2.35 (3H, s), 2.40 (3H, S), 2.44-2.53 (2H, m), 2.69-2.76 (3H, m), 2.86-2.92 (2H, m), 3.07-3.17 (2H, m), 3.71 (2H, s), 7.22-7.27 (2H, m), 7.35-7.52 (7H, m), 7.60 (2H, d, J=8.4Hz), 7.73 (1H, s).

### Working Example 222 (Production of Compound 222)

In dichloromethane (5ml) was suspended 2-(4-methylphenyl)-6,7-dihydro-5H-benzocycloheptene-8-carboxylic acid (0. 3g), and to the mixture were added, under ice-cooling, oxalyl chloride (0.3ml) and dimethylformamide (catalytic amount). The mixture was stirred at room temperature for 2 hours, and the solvent was evaporated. The residue was dissolved in tetrahydrofuran (15ml), and the solution was added dropwise, under ice-cooling, to a solution of 4-(N-ethyl-N-(tetrahydropyran-4-yl)amino-methyl)aniline (0.27g) and triethylamine (0.45ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated, and the residue was purified with silica gel column (ethyl acetate) to give crude crystals, which were recrystallized from ethyl acetate/hexane to give N-(4-((N-ethyl-N-tetrahydropyran-4-yl)aminomethyl)-phen yl)-2-(4-methylphenyl)-6,7-dihydro-5H-benzocycloheptene -8-carboxamide (Compound222) (0.38g) as colorless crystals.
mp 122-123°C.
¹H-NMR (δ ppm, CDCl₃) 1.01 (3H, t, J=7.1Hz), 1.62-1.72 (4H, m), 2.13-2.19 (2H, m), 2.40 (3H, s), 2.57 (2H, q, J=7.1Hz), 2.69-2.76 (3H, m), 2.86-2.92 (2H, m), 3.34 (2H, dt, J=3.4, 10.9Hz), 3.62 (2H, s), 3.97-4.04 (2H, m), 7.21-7.28 (3H, m), 7.35 (2H, d, J=8.6Hz), 7.42-7.57 (6H, m), 7.62 (1H, s) .
IR(KBr) ν : 2936, 1651cm⁻¹.

| | | | |
|---|---|---|---|
| Anal. Calcd. for C₃₃H₃₈N₂O₂: | C, 80.13; | H, 7.74; | N, 5.66. |
| Found | C,79.96; | H,7.77; | N,5.38. |

### Working Example 223 (Production of Compound 223)

To a suspension of 7-(4-methylphenyl)-2,3-dihydro-1-benzothiepine-4-carbox ylic acid (0.3g) in dichloromethane (6ml) were added, under ice-cooling, oxalyl chloride (0.25ml) anddimethylformamide (catalytic amount), and the mixture was stirred at room temperature for 1.5 hours. The solvent was evaporated, and the residue was dissolved in tetrahydrofuran (15ml). The mixture was added dropwise, under ice-cooling, to a solution of 4-((N-methyl-N-(pentan-3-yl))aminomethyl)-aniline (0.23g) and triethylamine (0.42ml) in tetrahydrofuran (15ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated to give crude crystals, which were recrystallized from ethyl acetate/hexane to give N-(4-((N-methyl-N-(pentan-3-yl)amino)methyl)-phenyl)-7-(4-methylphenyl)-2,3-dihydro-1-benzothiepine-4-carboxam ide (Compound 223) (0.34g) as colorless crystals.
mp 136-137°C.
¹H-NMR(δ ppm, CDCl₃) 0.94 (6H, t, J=7.3Hz), 1.26-1.54 (4H, m), 2.13 (3H, s), 2.17-2.32 (1H, m), 2.40 (3H, s), 3.08 (2H, t, J=5.9Hz), 3.29 (2H, t, J=5.9Hz), 3.55 (2H, s), 7.24-7.28 (2H, m), 7.31-7.40 (3H, m), 7.44-7.57 (6H, m), 7.66 (1H, s).
IR (KBr) ν : 2959, 2928, 1651cm⁻¹.

| | | | |
|---|---|---|---|
| Anal. Calcd. for C₃₁H₃₆N₂OS: | C, 76.82; | H, 7.49; | N,5.78. |
| Found | C,76.77; | H,7.21; | N,5.63. |

### Working Example 224 (Production of Compound 224)

In dichloromethane (5ml) was suspended 7-(4-methylphenyl)-2,3-dihydro-1-benzoxepine-4-carboxylic acid (0.25g), and to the mixture were added, under ice-cooling, oxalyl chloride (0.23ml) and dimethylformamide (catalytic amount).
The mixture was stirred at room temperature for 2 hours, and the solvent was evaporated. The residue was dissolved in tetrahydrofuran (20ml), and the mixture was added dropwise, under ice-cooling, to a solution of 2-(N-(4-aminobenzyl)-N-methylamino)-1,3-propanediol (0.21g) and triethylamine (0.37ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate/ethanol/hexane to give N-(4-((N-bis(hydroxy-methyl)methyl-N-methyl)aminomethyl )phenyl)-7-(4-methyl-phenyl)-2,3-dihydro-1-benzoxepine-4-carboxamide (Compound 224) (0.22g) as colorless crystals.
mp 199-201°C .
¹H-NMR(δppm, CDCl₃) 2.30 (3H, s), 2.39 (3H, s), 2.96-3.03 (1H, m), 3.08 (2H, t, J=4.5Hz), 3.61-3.73 (4H, m), 3.78 (2H, s), 4.36 (2H, t, J=4.5Hz), 7.06 (1H, d, J=8.4Hz), 7.23-7.32 (4H, m), 7.44-7.58 (6H, m), 7.62 (1H, s).
IR (KBr) ν : 3260, 2928, 1653cm⁻¹.

| Anal. Calcd. for C₂₉H₃₂N₂O₄·0.2H₂O: | | | |
|---|---|---|---|
| | C,73.15; | H,6.86; | N,5.88. |
| Found | C,73.20; | H,6.86; | N,5.91. |

### Working Example 225 (Production of Compound 225)

In dichloromethane (5ml) was suspended 7-(4-methylphenyl)-2,3-dihydro-1-benzoxepine-4-carboxylic acid (0.3g), and to the mixture were added, under ice-cooling, oxalyl chloride (0.28ml) and dimethylformamide (catalytic amount). The mixture was stirred at room temperature for 2 hours, and the solvent was evaporated. The residue was dissolved in tetrahydrofuran (20ml), and the mixture was added dropwise, under ice-cooling, to a solution of N-(4-aminobenzyl)sarcosine methyl ester (0.25g) and triethylamine (0.45ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated, and the residue was purified with silica gel column (ethyl acetate/hexane) to give crude crystals, which were recrystallized from ethyl acetate/hexane to give N-(4-((N-methoxycarbonylmethyl-N-methyl)aminomethyl)-ph enyl)-7-(4-methylphenyl)-2,3-dihydro-1-benzoxepine-4-ca rboxamide (Compound 225) (0.38g) as colorless crystals.
mp 135-136°C.
¹H-NMR (δ ppm, CDCl₃) 2.39 (3H, s), 2.39 (3H, s), 3.08 (2H, t, J=4.4Hz), 3.26 (2H, s), 3.65 (2H, s), 3.72 (3H, s), 4.36 (2H, t, J=4.4Hz), 7.06 (1H, d, J=8.4Hz), 7.22-7.36 (4H, m), 7.43-7.60 (7H, m).
IR(KBr) ν : 3262, 2951, 1740cm⁻¹.

| | | | |
|---|---|---|---|
| Anal. Calcd. for C₂₉H₃₀N₂O₄: | C, 74.02; | H,6.43; | N, 5.95. |
| Found | C,74.07; | H,6.47; | N,5.94. |

### Working Example 226 (Production of Compound 226)

In methanol (20ml) and THF (10ml) was dissolved N-(4-((N-methoxycarbonylmethyl-N-methyl)aminomethyl)-ph enyl)-7-(4-methylpheriyl)-2,3-dihydro-1-benzoxepine-4-ca rboxamide (0.24g), and to the mixture was added 1N sodium hydroxide solution (3.0ml). The mixture was stirred at room temperature overnight and concentrated. The residue was neutralized with 1N hydrochloric acid, and precipitated materials were filtered and dissolved in methanol. The mixture was filtered, and to the filtrate was added 4N hydrochloric acid-ethyl acetate. The solvent was evaporated, and the residue was purified with methanol/diethylether to give N-(4-((N-carboxymethyl-N-methyl)-aminomethyl)phenyl)-7-(4-methylphenyl)-2,3-dihydro-1-benzoxepine-4-carboxamid e hydrochloride (Compound 226) (0.21g) as pale yellow amorphous.
¹H-NMR (δ ppm, DMSO-d₆) 2.34 (3H, s), 2.76 (3H, s), 2.99 (2H, br), 3.36 (2H, br), 4.02 (2H, s), 4.30 (2H, br), 7.06 (1H, d, J=8.4Hz), 7.27 (2H, d, J=7.8Hz), 7.38 (1H, s), 7.48 (2H, d, J=8.6Hz), 7.55-7.59 (3H, m), 7.76 (1H, d, J=2.2Hz), 7.82 (2H, d, J=8.6Hz), 10.18 (1H, s).
IR (KBr) ν : 1744 cm⁻¹.

| Anal. Calcd. for C₂₈H₂₉ClN₂O₄·0.5H₂O:] | | | |
|---|---|---|---|
| | C,66.99; | H,6.02; | N,5.58. |
| Found | C,66.93; | H, 5.87; | N,5.11. |

### Working Example 227 (Production of Compound 227)

In dichloromethane (10ml) was suspended 7-(4-methylphenyl)-2,3-dihydro-1-benzothiepine-4-carboxylic acid (0.3g), and to the mixture were added, under ice-cooling, oxalyl chloride (0.25ml) and dimethylformamide (catalytic amount). The mixture was stirred at room temperature for 2 hours, and the solvent was evaporated. The residue was dissolved in tetrahydrofuran (20ml), and the mixture was added dropwise, under ice-cooling, to a solution of N-(4-aminobenzyl)sarcosine methyl ester (0.23g) and triethylamine (0.42ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated to give crude crystals, which were recrystallized from ethyl acetate/hexane to give N-(4-((N-methoxycarbonylmethyl-N-methyl)aminomethyl)phe nyl)-7-(4-methylphenyl)-2,3-dihydro-1-benzothiepine-4-c arboxamide (Compound 227) (0.43g) as colorless crystals.
mp 148-150°C.
¹H-NMR(δppm, CDCl₃) 2.39 (3H, s), 2.40 (3H, s), 3.08 (2H, t, J=6.0Hz), 3.26 (2H, s), 3.29 (2H, t, J=6.0Hz), 3.66 (2H, s), 3.72 (3H, s), 7.24-7.58 (11H, m), 7.67 (1H, s).
IR (KBr) ν : 1738cm⁻¹.

| | | | |
|---|---|---|---|
| Anal. Calcd. for C₂₉H₃₀N₂O₃S: | C, 71.58 ; | H, 6.21 ; | N,5.76. |
| Found | C,71.75; | H,5.95; | N,5.60. |

### Working Example 228 (Production of Compound 228)

In methanol (20ml) and THF (10ml) was dissolved N-(4-((N-methoxycarbonylmethyl-N-methyl)aminomethyl)-ph enyl)-7-(4-methylphenyl)-2,3-dihydro-1-benzothiepine-4-carboxamide (0.23g), and to the mixture was added 1N sodium hydroxide solution (2.4ml). The mixture was stirred at room temperature overnight, concentrated and neutralized with 1N hydrochloric acid. Precipitated materials were filtered, washed with water and recrystallized from ethanol/hexane to give N-(4-((N-carboxymethyl-N-methyl)aminomethyl)phenyl)-7-( 4-methyl-phenyl)-2,3-dihydro-1-benzothiepine-4-carboxam ide (Compound 228) (0.16g) as colorless crystals.
mp 243-245°C.
¹H-NMR (δ ppm, DMSO-d₆) 2.34 (6H, br), 3.00 (2H, br), 3.16 (2H, br), 3.22 (2H, br), 3.80 (2H, br), 7.20-7.35 (4H, m), 7.45-7.72 (7H, m), 7.82 (1H, s), 10.14 (1H, s).

| Anal. Calcd. for C₂₈H₂₈N₂O₃S·0.5H₂O: | | | |
|---|---|---|---|
| | C,69.83; | H,6.07; | N,5.82. |
| Found | C,69.62; | H,5.92; | N,5.58. |

### Working Example 229 (Production of Compound 229)

In dichloromethane (5ml) was suspended 7-(4-methylphenyl)-2,3-dihydro-1-benzothiepine-4-carbox ylic acid (0.2g), and to the mixture were added, under ice-cooling, oxalyl chloride (0.18ml) and dimethylformamide (catalytic amount). The mixture was stirred at room temperature for 2 hours, and the solvent was evaporated. The residue was dissolved in tetrahydrofuran (20ml), and the mixture was added dropwise, under ice-cooling, to a solution of 1-(N-(4-aminobenzyl)-N-methylamino)-3-propanol (0.15g) and triethylamine (0.28ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated, and the residue was purified with silica gel column (methanol/triethylamine/ethylacetate) to give crude crystals, which were recrystallized from ethyl acetate/hexane to give N-(4-((N-3-hydroxypropyl-N-methyl)aminomethyl)phenyl)-7 -(4-methylphenyl)-2,3-dihydro-1-benzothiepine-4-carboxa mide (Compound 229) (0.16g) as colorless crystals.
mp 147-148°C.
¹H-NMR (δ ppm, CDCl₃) 1.69-1.80 (2H, m), 2.25 (3H, s), 2.40 (3H, s), 2.67 (2H, t, J=5.6Hz), 3.08 (2H, t, J=5.9Hz), 3.28 (2H, t, J=5.9Hz), 3.53 (2H, s), 3.78 (2H, t, J=5.3Hz), 7.24-7.32 (3H, m), 7.41-7.50 (4H, m), 7.53-7.60 (4H, m), 7.81 (1H, s).
IR(KBr) ν : 3266, 2948, 1649cm⁻¹.

| Anal. Calcd. for C₂₉H₃₂N₂O₂S·0-3H₂O : | | | |
|---|---|---|---|
| | C, 72.86 ; | H,6.87; | N,5.86. |
| Found | C, 72.90 ; | H,6.70; | N,6.05. |

### Working Example 230 (Production of Compound 230)

In dichloromethane (5ml) was suspended 7-(4-methylphenyl)-2,3-dihydro-1-benzoxepine-4-carboxyl ic acid (0.2g), and to the mixture were added, under ice-cooling, oxalyl chloride (0.19ml) anddimethylformamide (catalytic amount). The mixture was stirred at room temperature for 2 hours, and the solvent was evaporated. The residue was dissolved in tetrahydrofuran (20ml), and the mixture was added dropwise, under ice-cooling, to a solution of 4-((N-3-methoxypropyl-N-methyl)amino-methyl)aniline (0.16g) and triethylamine (0.3ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated to give crude crystals, which were recrystallized from ethyl acetate/hexane to give N-(4-((N-3-methoxypropyl-N-methyl)aminomethyl)phenyl)-7 -(4-methyl-phenyl)-2,3-dihydro-1-benzoxepine-4-carboxam ide (Compound 230) (0.28g) as colorless crystals.
mp 121-123°C.
¹H-NMR(δppm, CDCl₃) 1.75-1.84 (2H, m), 2.19 (3H, s), 2.40 (3H, s), 2.45 (2H, t, J=7.3Hz), 3.09 (2H, t, J=4.6Hz), 3.33 (3H, s), 3.43 (2H, t, J=6.6Hz), 3.47 (2H, s), 4.37 (2H, t, J=4.6Hz), 7.06 (1H, d, J=8.2Hz), 7.23-7.33 (4H, m), 7.44-7.56 (7H, m).
IR (KBr) ν : 2934, 1653cm⁻¹.

| | | | |
|---|---|---|---|
| Anal. Calcd. for C₃₀H₃₄N₂O₃: | C,76.57; | H, 7.28; | N, 5.95. |
| Found | C,76.41; | H,7.24; | N,6.02. |

### Working Example 231 (Production of Compound 231)

In dichloromethane (5ml) was suspended 7-(4-methylphenyl)-2,3-dihydro-1-benzothiepine-4-carbox ylic acid (0.15g), and to the mixture were added, under ice-cooling, oxalyl chloride (0.15ml) and dimethylformamide (catalytic amount). The mixture was stirred at room temperature for 2 hours, and the solvent was evaporated. The residue was dissolved in tetrahydrofuran (15ml), and the mixture was added dropwise, under ice-cooling, to a solution of 4-((N-3-methoxypropyl-N-methyl)amino-methyl)aniline (0.12g) and triethylamine (0.21ml) in tetrahydrofuran (10ml). Under nitrogen atmosphere, the mixture was stirred at room temperature overnight. The solvent was evaporated, and to the residue was added water. The mixture was extracted with ethyl acetate, and the organic layer was with water and saturated brine, and dried with anhydrous magnesium sulfate. Under reduced pressure, the solvent was evaporated to give crude crystals, which were recrystallized from ethyl acetate/hexane to give N-(4-((N-3-methoxypropyl-N-methyl)aminomethyl)phenyl)-7 -(4-methylphenyl)-2,3-dihydro-1-benzothiepine-4-carboxa mide (Compound 231) (0.18g) as colorless crystals.
mp 128-129°C.
¹H-NMR (δ ppm, CDCl₃) 1.70-1.87 (2H, m), 2.19 (3H, s), 2.40 (3H, s), 2.45 (2H, t, J=8.4Hz), 3.08 (2H, t, J=5. 6Hz), 3.29 (2H, t, J=5.6Hz), 3.33 (3H, s), 3.43 (2H, t, J=6.4Hz), 3.47 (2H, s), 7.24-7.33 (3H, m), 7.40-7.58 (8H, m), 7.68 (1H, s).
IR(KBr) ν : 2924, 1651cm⁻¹.

| | | | |
|---|---|---|---|
| Anal. Calcd. for C₃₀H₃₄N₂O₂S: | C, 74 . 04 ; | H, 7.04; | N,5.76. |
| Found | C,73.80; | H,6.95; | N,5.87. |

### Working Example 232 (Production of Compound 232)

To a solution of 2-methyl-6-(4-methylphenyl)-quinoline-3-carboxylic acid (120mg) and 1-hydroxy-benzotriazole (88mg) in DMF (5ml) was added at room temperature 1-ethyl-3-(3'-dimethylaminopropyl)-carbodiimide hydrochloride (125mg), and the mixture was stirred for 1 hour. To the mixture was added a solution of 1-(4-aminobenzyl)phosphorinane-1-oxide (109mg) and triethylamine (0.1ml) in DMF (3ml), and the mixture was stirred for 3 days. Under reduced pressure, the mixture was concentrated, and to the residue was added water. The mixture was extracted with chloroform, and the organic layer was washed with saturated brine and dried with magnesium sulfate. Under reduced pressure, the mixture was concentrated, and the residue was separated and purified with column chromatography (ethanol/ethyl acetate=1:2) and recrystallized from (ethanol/ethyl acetate) to give pale yellow crystals of 2-methyl-6-(4-methylphenyl)-N-(pentamethylenephosphoryl methylphenyl)quinoline-3-carboxamide (Compound 232) (116.1mg).
m.p. 273-275 °C
¹H-NMR (200MHz, CDCl₃) δ 1.01-1.84 (10H, m), 2.44 (3H, s), 2.90 (3H, s), 3.04 (2H, d, J=12.6 Hz), 7.17-7.25 (2H, m), 7.32 (2H, d, J=7.9 Hz), 7.61 (2H, d, J=7.9 Hz), 7.69 (2H, d, J=8.2 Hz), 7.99-8.13 (3H, m), 8.30 (1H, s), 9.44 (1H, br s).
IR (KBr) 3024, 1664, 1601, 1539, 1516, 1319, 1159, 847, 816 cm⁻¹

| Anal. Calcd. for C₃₀H₃₁N₂O₂P·0.3H₂O | | | | |
|---|---|---|---|---|
| Calcd. | C, 73.84 ; | H, 6.53 ; | N, 5.74 ; | P, 6.35. |
| Found. | C, 73.67 ; | H, 6.58 ; | N, 5.67 ; | P, 6.27. |

### Working Example 233 (Production of Compound 233)

Under nitrogen atmosphere, to a solution of (E)-3-[5-(4-isopropylphenyl)thiophen-2-yl]acrylic acid (130mg) in THF (10ml) was added at room temperature oxalyl chloride (0.07ml) and then a drop of DMF, and the mixture was stirred for 1 hour. Under reduced pressure, the solvent was evaporated, and the residue was dissolved in THF (20ml). To the mixture were added 1-(4-aminobenzyl)-phosphorinane-1-oxide (117mg) and triethylamine (0.15ml) at 0°C, and the mixture was stirred at room temperature for 4 hours. The mixture was added tovigorously stirredwater to stop the reaction and extracted with ethyl acetate. The organic layer was washed with saturated brine, dried with magnesium sulfate, concentrated and purified with column chromatography (ethanol/ethyl acetate=1:4) and recrystallized from ethanol/ethyl acetate to give yellow crystals of (E)-3-[5-(4-methylphenyl)thiophen-2-yl]-N-(pentaethylen ephosphorylmethylphenyl)acrylamide (Compound 233) (60.5mg).
m.p. 295 °C (dec.)
¹H-NMR (200MHz, CDCl₃) δ 1.28 (6H, d, J=7.0 Hz), 1.51-2.10 (10H, m), 2.89-3.00 (1H, m), 3.15 (2H, d, J=13.2 Hz), 6.48 (1H, d, J=15.0 Hz), 7.15-7.33 (6H, m), 7.50-7.62 (4H, m), 7.82 (1H, d, J=15.0 Hz), 8.37-8.59 (1H, m).
IR (KBr) 3057, 1672, 1618, 1543, 1510, 1412, 1356, 1327, 1250, 1232, 1165, 960, 852, 829, 793 cm⁻¹

| Anal. Calcd. For C₂₈H₃₂NO₂SP | | | |
|---|---|---|---|
| Calcd. | C, 70.41 ; | H, 6.75 ; | N, 2.93. |
| Found. | C, 70.06 ; | H, 6.82 ; | N, 2.98. |

### Working Example 234 (Production of Compound 234)

Under nitrogen atmosphere, to a solution of (E)-3-[5-(4-tert-butylphenyl)thiophen-2-yl]acrylic acid (120mg) in THF (10ml) were added at room temperature oxalyl chloride (0.06ml) and a drop of DMF, and the mixture was stirred for 1 hour. Under reduced pressure, the solvent was evaporated, and the residue was dissolved in THF (20ml). To the mixture were added at 0°C 1-(4-aminobenzyl)phosphorinane-1-oxide (104mg) and triethylamine (0.12ml), and the mixture was stirred at room temperature for 18 hours. The mixture was added to vigorously stirred water to stop the reaction and extracted with ethyl acetate. The organic layer was washed with saturated brine and dried with magnesium sulfate. Under reduced pressure, the mixture was concentrated, and the residue was purified with column chromatography (ethanol/ethyl acetate=1:4) and recrystallized from ethanol to give yellow crystals of (E)-N-(4-pentamethylene phosphorylmethylphenyl)-3-[5-(4-tert-butylphenyl)-thiop hen-2-yl]acrylamide (Compound 234) (82.1mg).
m.p. >300 °C
¹H-NMR (200MHz, CDCl₃) δ 1.35 (9H, s), 1.50-2.22 (10H, m), 3.15 (2H, d, J=13.2 Hz), 6.53 (1H, d, J=15.4 Hz), 7.12-7.30 (4H, m), 7.42 (2H, d, J=8.4 Hz), 7.49-7.60 (4H, m), 7.82 (1H, d, J=15.4 Hz), 8.79-8.98 (1H, m).
IR (KBr) 3238, 1672, 1618, 1543, 1514, 1358, 1252, 1167, 852, 793 cm⁻¹

| Anal. Calcd. For C₂₉H₃₄NO₂SP | | | | |
|---|---|---|---|---|
| Calcd. | C, 70.85 ; | H, 6.97 ; | N, 2.85 ; | P, 6.30. |
| Found. | C, 70.61 ; | H, 6.90 ; | N, 2.89 ; | P, 6.17. |

### Working Example 235 (Production of Compound 235)

Under nitrogen atmosphere, to a solution of 2-(4-methylphenyl)benzofuran-5-carboxylic acid (130mg) in THF (10ml) were added at room temperature oxalyl chloride (0.07ml) and a drop of DMF, and the mixture was stirred for 1 hour. Under reduced pressure, the solvent was evaporated, and the residue was dissolved in THF (20ml). To the mixture were added at 0°C 1-(4-aminobenzyl)phosphorinane-1-oxide (126mg) and triethyl-amine (0.15ml), and the mixture was stirred at room temperature for 3 hour. The mixture was added to vigorously stirred water to stop the reaction and extracted with ethyl acetate. The organic layer was washed with saturated brine, dried with magnesium sulfate and concentrated. The resulting crystals were recrystallized from ethanol to give colorless crystals of 2-(4-methylphenyl)-N-(4-pentamethylenephosphorylmethylphenyl)benzofuran-5-carboxamide (Compound 235) (134.6mg). m.p. 297-296 °C
¹H-NMR (200MHz, CDCl₃) δ 1.42-2.16 (10H, m), 2.42 (3H, s), 3.17 (2H, d, J=13.2 Hz), 7.04 (1H, s), 7.24-7.33 (4H, m), 7.58 (1H, d, J=8.6 Hz), 7.67 (2H, d, J=8.4 Hz), 7.76-7.85 (3H, m), 8.14 (1H, d, J=1.8 Hz), 8.15-8.19 (1H, m).
IR (KBr) 3390, 2929, 1657, 1524, 1323, 1230, 1161, 1132, 849, 824, 800, 760 cm⁻¹

| Anal. Calcd. For C₂₈H₂₈NO₃P | | | |
|---|---|---|---|
| Calcd. | C, 73.51 ; | H, 6.17 ; | N, 3.06. |
| Found. | C, 73.45 ; | H, 5.89 ; | N, 2.83. |

### Working Example 236 (Production of Compound 236)

To a solution of 2-(4-methylphenyl)benzofuran-6-carboxylic acid (130mg) in THF (10ml) were added oxalyl chloride (0.07ml) and a drop of dimethylformamide at room temperature, and the mixture was stirred for 1 hour. Under reduced pressure, the solvent was evaporated, and the residue was dissolved in THF (20ml). To the mixture were added at 0°C 1-(4-aminobenzyl)phosphorinane-1-oxide (126mg) and triethylamine (0.15ml), and the mixture was stirred at room temperature for 20 hours. The mixture was added to vigorously stirred water to stop the reaction and extracted with dichloromethane, and the organic layer was washed with saturated brine. Under reduced pressure, the mixture was concentrated, and the residue was recrystallized fromethanol to give pale yellow crystals of 2-(4-methyl-phenyl)-N-(4-pentamethylenephosphoryl-methy lphenyl)benzofuran-6-carboxamide(Compound236) (149.9mg). m.p. >300 °C
IR (KBr) 3224, 1651, 1535, 1512, 1323, 1165, 845, 820 cm⁻¹

| Anal. Calcd. For C₂₈H₂₈NO₃P | | | |
|---|---|---|---|
| Calcd. | C, 73.51 ; | H, 6.17 ; | N, 3.06. |
| Found. | C, 73.50 ; | H, 6.17 ; | N, 2.92. |

### Working Example 237 (Production of Compound 237)

To a solution of 7-(4-methylsulfonylphenyl)-2,3-dihydro-1-benzoxepine-4-carboxylic acid (100mg) in THF (10ml) were added at room temperature oxalyl chloride (0.05ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)-aminomethyl]anilin e (71mg) and triethylamine (0.1ml), and the mixture was stirred at room temperature for 16 hours. The mixture was added to vigorously stirred water to stop the reaction and extracted with ethyl acetate. The organic layer was washed with saturated brine and dried with magnesium sulfate. Under reduced pressure, the mixture was concentrated, and the residue was purified with column chromatography (ethanol/ethylacetate=1:3) and recrystallized from ethanol to give colorless crystals of 7-(4-methylsulfonylphenyl)-N-[4-[N-methyl-N-(tetra-hydr opyran-4-yl)aminomethyl]phenyl]-2,3-dihydro-1-benzoxepi ne-4-carboxamide (Compound 237) (123mg).
m.p. 233-235 °C
¹H-NMR (200MHz, CDCl₃) δ 1.62-1.82 (4H, m), 2.21 (3H, s), 2.56-2.73 (1H, m), 3.04-3.15 (2H, m), 3.10 (3H, s), 3.31-3.43 (2H, m), 3.57 (2H, s), 3.99-4.09 (2H, m), 4.39 (2H, t, J=4.5 Hz), 7.12 (1H, d, J=8.4 Hz), 7.24-7.35 (3H, m), 7.46-7.60 (5H, m), 7.74 (2H, d, J=8.6 Hz), 8.00 (2H, d, J=8.6 Hz).
IR (KBr) 3292, 1645, 1524, 1308, 1144 cm⁻¹

| Anal. Calcd. for C₃₁H₃₄N₂O₅S | | | | |
|---|---|---|---|---|
| Calcd. | C, 68.11 ; | H, 6.27 ; | N, 5.12 ; | S, 5.87. |
| Found. | C, 67.94 ; | H, 6.40 ; | N, 5.09 ; | S, 5.90. |

### Working Example 238 (Production of Compound 238)

Under nitrogen atmosphere, to a solution of (E)-3-[5-(4-isopropylphenyl)thiophen-2-yl]acrylic acid (130mg) in THF (10ml) were added at room temperature oxalyl chloride (0.07ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)aminomethyl]aniline (116mg) and triethylamine (0.15ml), and the mixture was stirred at room temperature for 4 hour. The mixture was added to vigorously stirred water to stop the reaction and extracted with ethyl acetate. The organic layer was washed with saturated brine, dried with magnesium sulfate, concentrated and purified with column chromatography (ethanol/ethyl acetate=1:4) and recrystallized from ethyl acetate/hexane to give yellow crystals of (E)-3-[5-(4-isopropylphenyl)thiophen-2-yl]-N-[4-[N-meth yl-N-(tetrahydropyran-4-yl)aminomethyl]-phenyl]acrylami de (Compound 238) (162.9mg).
m.p. 187-189 °C ¹H-NMR (200MHz, CDCl₃) δ 1.27 (6H, d, J=6.8 Hz), 1.54-1.84 (4H, m), 2.21 (3H, s), 2.55-2.72 (1H, m), 2.84-3.01 (1H, m), 3.30-3.44 (2H, m), 3.56 (2H, s), 3.97-4.10 (2H, m), 6.31 (1H, d, J=15.4 Hz), 7.19-7.35 (7H, m), 7.49-7.61 (4H, m), 7.84 (1H, d, J=15.4 Hz).
IR (KBr) 3315, 1664, 1606, 1535, 1512, 1408, 1335, 1169, 829, 804 cm⁻¹

| Anal. Calcd. for C₂₉H₃₄N₂O₂S | | | | |
|---|---|---|---|---|
| Calcd. | C, 73.38 ; | H, 7.22 ; | N, 5.90 ; | S, 6.76. |
| Found. | C, 73.12 ; | H, 7.34 ; | N, 5.88 ; | S, 6.83. |

### Working Example 239 (Production of Compound 239)

A solution of 7-(4-methylthiophenyl)-N-[4-[N-methyl-N-(4-tetrahydropy ran-4-yl)aminomethyl]phenyl]-2,3-dihydro-1-benzoxepine-4-carboxamide (110mg) and sodium periodate (48mg) in methanol/water (40/15ml) was stirred at room temperature for 2 days. Under reduced pressure, the mixture was concentrated, and to the residue was added water. The mixture was extracted with chloroform. The organic layer was washed with saturated brine and dried with magnesium sulfate. Under reduced pressure, the mixture was concentrated, and the residue was purified with column chromatography (ethanol/ethyl acetate=1:1) and recrystallized from ethanol/ethyl acetate to give colorless crystals of 7-(4-methylsulfinylphenyl)-N-[4-[N-methyl-N-(4-tetrahyd ropyran-4-yl)aminomethyl]phenyl]-2,3-dihydro-1-benzoxep ine-4-carboxamide (Compound 239) (15.5mg).
¹H-NMR (200MHz, CDCl₃) δ 1.52-1 .83 (4H, m), 2.21 (3H, s), 2.52-2.74 (1H, m), 2.77 (3H, s), 3.10 (2H, t, J=4.4 Hz), 3.29-3.43 (2H, m), 3.57 (2H, s), 3.98-4.10 (2H, m), 4.39 (2H, t, J=4.4 Hz), 7.11 (1H, d, J=8.0 Hz), 7.23-7.35 (3H, m), 7.44-7.63 (5H, m), 7.71 (4H, s).
IR (KBr) 3327, 1649, 1515, 1410, 1315, 1240, 1038, 822 cm⁻¹

### Working Example 240 (Production of Compound 240)

Under nitrogen atmosphere, to a solution of (E)-3-[5-(4-tert-butylphenyl)thiophen-2-yl]acrylic acid (130mg) in THF (10ml) were added at room temperature oxalyl chloride (0.06ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)aminomethyl]aniline (109mg) and triethylamine (0.13ml), and the mixture was stirred at room temperature for 6 days. The mixture was added to vigorously stirred water to stop the reaction and extracted with ethyl acetate. The organic layer was washed with saturated brine, dried with magnesium sulfate and concentrated. The residue was purified with column chromatography (ethanol/ethyl acetate=1:4) and recrystallized from ethyl acetate/hexane to give yellow crystals of (E)-3-[5-(4-tert-butylphenyl)thiophen-2-yl]-N-[4-[N-met hyl-N-(tetrahydropyran-4-yl)amino-methyl]phenyl]acrylam ide (Compound 240) (107.3mg).
m.p. 216-220 °C
¹H-NMR (200MHz, CDCl₃) δ 1.35 (9H, s), 1.50-1.86 (4H, m), 2.21 (3H, s), 2.51-2.76 (1H, m), 3.30-3.45 (2H, m), 3.57 (2H, s), 3.99-4.10 (2H, m), 6.32 (1H, d, J=14.8Hz), 7.21-7.35 (5H, m), 7.43 (2H, d, J=8.4 Hz), 7.51-7.61 (4H, m), 7.84 (1H, d, J=14.8 Hz).
IR (KBr) 3320, 1666, 1606, 1535, 1335, 831 cm⁻¹

| Anal. Calcd. for C₃₀H₃₆N₂O₂S·0.1H₂O | | | |
|---|---|---|---|
| Calcd. | C, 73.46 ; | H, 7.44 ; | N, 5.71. |
| Found. | C, 73.41 ; | H, 7.41 ; | N, 5.83. |

### Working Example 241 (Production of Compound 241)

Under nitrogen atmosphere, to a solution of 2-(4-methylphenyl)benzofuran-5-carboxylic acid (200mg) in THF (10ml) were added at room temperature oxalyl chloride (0.1ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)aminomethyl]aniline (192mg) and triethylamine (0.22ml), and the mixture was stirred at room temperature for 18 hours. The mixture was added to vigorously stirred water to stop the reaction and extracted with chloroform. The organic layer was washed with saturated brine, dried with magnesium sulfate and concentrated. The resulting crystals were recrystallized from ethanol to give colorless crystals of 2-(4-methylphenyl)-N-[4-(N-methyl-N-(tetrahydropyran-4-yl) aminomethyl) phenyl] benzofuran-5-carboxamide (Compound 241) (295.8mg) .
m.p. 233-236 °C
¹H-N_{M}R (200MHz, CDCl₃) δ 1.62-1.83 (4H, m), 2.22 (3H, s), 2.42 (3H, s), 2.57-2.72 (1H, m), 3.32-3.44 (2H, m), 3.59 (2H, s), 3.99-4.09 (2H, m), 7.03 (1H, s), 7.31-7.36 (4H, m), 7.56-7.64 (3H, m), 7.76-7.82 (3H, m), 7.87 (1H, s), 8.11 (1H, d, J=1.4 Hz).
IR (KBr) 3388, 2943, 1647, 1597, 1525, 1408, 1319, 1148, 794 cm⁻¹

| Anal. Calcd. For C₂₉H₃₀N₂O₃ | | | |
|---|---|---|---|
| Calcd. | C, 76.63 ; | H, 6.65 ; | N, 6.16, |
| Found. | C, 76.61 ; | H, 6.47 ; | N, 6.00. |

### Working Example 242 (Production of Compound 242)

To a solution of 2-(4-methylphenyl)benzofuran-6-carboxylic acid (200mg) in THF (10ml) were added at room temperature oxalyl chloride (0.1ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)aminomethyl]aniline (192mg) and triethylamine (0.22ml), and the mixture was stirred at room temperature for 4 hour. The mixture was added to vigorously stirred water to stop the reaction and extracted with dichloromethane. The organic layer was washed with saturated brine and dried with magnesium sulfate. Under reduced pressure, the mixture was concentrated, and the residue was purified with column chromatography (ethanol/ethyl acetate=1:4→1:2→2:1) and recrystallized from ethanol to give pale yellow crystals of 2-(4-methylphenyl)-N-[4-[N-methyl-N-(tetrahydro-pyran-4 -yl)aminomethyl]phenyl]benzofuran-6-carboxamide (Compound 242) (280mg).
m.p. 224-227 °C
¹H-NMR (200MHz, CDCl₃) δ 1.41-1.82 (4H, m), 2.22 (3H, s), 2.42 (3H, s), 2.56-2.74 (1H, m), 3.32-3.44 (2H, m), 3.59 (2H, s), 3.98-4.12 (2H, m), 7.02 (1H, s), 7.25-7.37 (4H, m), 7.61-7.66 (3H, m), 7.72-7.81 (3H, m), 7.92 (1H, s), 8.07 (1H, s).
IR (KBr) 3304, 1647, 1520, 1313, 822 cm⁻¹

| Anal. Calcd. for C₂₉H₃₀N₂O₃ | | | |
|---|---|---|---|
| Calcd. | C, 76.63 ; | H, 6.65 ; | N, 6.16. |
| Found. | C, 76.79 ; | H, 6.39 ; | N, 6.13. |

### Working Example 243 (Production of Compound 243)

To a solution of (E)-3-[5-(4-methylphenyl)thiophen-2-yl]-N-[4-[N-methyl-N-(tetrahydropyran-4-yl)amino-meth yl]phenyl]acrylamide (100mg) in DMF (3ml) was added at room temperature methyl iodide (0.5ml), and the mixture was stirred for 2 days. Under reduced pressure, the mixture was concentrated, and to the residue was added acetonitrile. The resulting crystals were collected by filtration to give yellow crystals of N,N-dimethyl-N- [4- [[(E) -3- [5- (4-methylphenyl) thiophen-2 -yl]-2-propenoyl]amino]benzyl]-4-tetrahydropyranyl ammonium iodide (Compound 243) (101.1mg).
m.p. 212-216 °C
¹H-NMR (200MHz, DMSO-d₆) δ 1.74-1.99 (2H, m), 2.09-2.22 (2H, m), 2.34 (3H, s), 2.87 (6H,brs), 3.24-3.42 (2H, m), 3.48-3.66 (1H, m), 4.00-4.11 (2H, m), 4.46 (2H, s), 6.58 (1H, d, J=15.4 Hz), 7.27 (2H, d, J=7.9 Hz), 7.44-7.58 (4H, m), 7.61 (2H, d, J=7.9 Hz), 7.76 (1H, d, J=15.4 Hz), 7.82 (2H, d, J=8.8 Hz), 10.43 (1H, s).
IR (KBr) 3165, 1675, 1606, 1525, 1155, 814 cm⁻¹

| Anal. Calcd. for C₂₈H₃₃N₂O₂SI·0.5H₂O | | | |
|---|---|---|---|
| Calcd. | C, 56.28 ; | H, 5.74 ; | N, 4.69. |
| Found. | C, 56.04 ; | H, 5.71 ; | N, 4.71. |

### Working Example 244 (Production of Compound 244)

To a solution of (E)-N-[4-[N-methyl-N-(tetrahydro-pyran-4-yl)aminomethyl ]phenyl]-3-[5-(4-isopropyl-phenyl)thiophen-2-yl]acrylam ide (80mg) in DMF (5ml) was added at room temperature methyl iodide (0.04ml), and the mixture was stirred for 3 days. Under reduced pressure, the solvent was evaporated, and to the residue was added acetonitrile. The resulting crystals were collected by filtration to give yellow crystals of N,N-dimethyl-N-[4-[[(E)-3-[5-(4-isopropylphenyl)thiophe n-2-yl]-2-propenoyl]amino]benzyl]-4-tetrahydropyranyl ammonium iodide (Compound 244) (76.9mg).
m.p. 217-220 °C
¹H-NMR (200MHz, DMSO-d₆) δ 1.23 (6H, d, J=7.0 Hz), 1.72-2.01 (2H, m), 2.08-2.23 (2H, m), 2.79-3.01 (1H, m), 2.87 (6H, s), 3.25-3.44 (2H, m), 3.49-3.68 (1H, m), 3.99-4.12 (2H, m), 4.46 (2H, s), 6.58 (1H, d, J=15.4 Hz), 7.33 (2H, d J=8.5 Hz), 7.44-7.57 (4H, m), 7.63 (2H, d, J=8.5 Hz), 7.76 (1H, d, J=15.4 Hz), 7.82 (2H, d, J=8.8 Hz), 10.42 (1H, s).
IR (KBr) 3298, 1654, 1608, 1527, 1452, 1417, 1323, 1252, 1163, 843, 802 cm⁻¹

| Anal. Calcd. for C₃₀H₃₇N₂O₂SI | | | |
|---|---|---|---|
| Calcd. | C, 58.44 ; | H, 6.05 ; | N, 4.54. |
| Found. | C, 58.24 ; | H, 5.83 ; | N, 4.27. |

### Working Example 245 (Production of Compound 245)

To a solution of 2-(4-methylphenyl)-N-[4-(N-methyl-N-(tetrahydropyran-4-yl)aminomethyl)phenyl]-benzofuran-5-carboxamide (120mg) in DMF (20ml) was added at room temperature methyl iodide (0.04ml), and the mixture was stirred for 24 hours. Under reduced pressure, the solvent was evaporated, and to the residue was added ethanol. The resulting crystals were collected by filtration to give yellow crystals of N,N-dimethyl-N-[4-[[2-(4-methyl-phenyl)benzofuran-5-car bonyl]amino]-benzyl]-4-tetra-hydropyranyl ammonium iodide (Compound 245) (142.1mg).
m.p. 208-212 °C
¹H-NMR (200MHz, DMSO-d₆) δ 1.71-2.01 (2H, m), 2.12-2.23 (2H, m), 2.39 (3H, s), 2.89 (6H, s), 3.10-3.43 (2H, m), 3.48-3.69 (1H, m), 4.03-4.15 (2H, m), 4.48 (2H, s), 7.36 (2H, d, J=8.0 Hz), 7.53-7.59 (3H, m), 7.77 (1H, d J=8.4 Hz), 7.85-7.99 (5H, m), 8.29 (1H, d, J=1.8 Hz), 10.52 (1H, s).
IR (KBr) 3277, 1643, 1595, 1525, 1468, 1416, 1325, 842, 820, 789, 762 cm⁻¹

| Anal. Calcd. for C₃₀H₃₃N₂O₃I·1.0H₂O | | | |
|---|---|---|---|
| Calcd. | C, 58.64 ; | H, 5.74 ; | N, 4.56. |
| Found. | C, 58.98 ; | H, 5.62 ; | N, 4.55. |

### Working Example 246 (Production of Compound 246)

To a solution of 7-(4-methoxyphenyl)-2,3-dihydro-1-benzothiepine-4-carbo xylicacid (150mg) inTHF (10ml) were added at room temperature oxalyl chloride (0.13ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)amino-methyl]anilin e (116mg) and triethylamine (0.2ml), and the mixture was stirred at room temperature for 4 hours. The mixture was added to vigorously stirred water to stop the reaction and extracted with ethyl acetate. The organic layer was washed with saturated brine and dried with magnesium sulfate. Under reduced pressure, the mixture was concentrated, and the residue was purified with column chromatography (ethanol/ethyl acetate=1:4) and recrystallized from ethanol/diethylether to give pale yellow crystals of 7-(4-methoxyphenyl)-N-[4-[N-methyl-N-(tetrahydropyran-4 -yl)aminomethyl]phenyl]-2,3-dihydro-1-benzothiepine-4-c arboxamide (Compound 246) (128.5mg).
m.p.162-164 °C
¹H-NMR (200MHz, CDCl₃) δ 1.61-1.83 (4H, m), 2.21 (3H, s), 2.55-2.72 (1H, m), 3.05-3.10 (2H, m), 3.26-3.44 (4H, m), 3.57 (2H, s), 3.86 (3H, s), 3.96-4.09 (2H, m), 6.98 (2H, d, J=8.8 Hz), 7.32 (2H, d, J=8.4 Hz), 7.35-7.43 (2H, m), 7.48-7.57 (6H, m), 7.68 (1H, br s).
IR (KBr) 3332, 1647, 1515, 1248, 818 cm⁻¹

| Anal. Calcd. for C₃₁H₃₄N₂O₃S | | | |
|---|---|---|---|
| Calcd. | C, 72.34 ; | H, 6.66 ; | N, 5.44. |
| Found. | C, 72.25 ; | H, 6.67 ; | N, 5.43. |

### Working Example 247 (Production of Compound 247)

To a solution of 7-(4-methoxyphenyl)-2,3-dihydro-1-benzothiepine-4-carbo xylicacid (200mg) inTHF (10ml) were added at room temperature oxalyl chloride (0.30ml) and a drop of DMF, and the mixture was stirred for 1 hour. Under reduced pressure, the solvent was evaporated, and the residue was dissolved in THF (20ml). To the mixture were added at 0°C 4-[N-(4,4-ethylenedioxycyclohexyl)-N-methylaminomethyl] aniline (0.20g) and triethylamine (0.3ml), and the mixture was stirred at room temperature for 4 hours. The mixture was added to vigorously stirred water to stop the reaction and extracted with ethyl acetate. The organic layer was washed with saturated brine and dried with magnesium sulfate. Under reduced pressure, the mixture was concentrated, and the residue solid was recrystallized from acetone/diethylether to give pale yellow crystals of N-[4-[N-(4,4-ethylenedioxy-cyclohexyl)-N-methylaminomet hyl]phenyl]-7-(4-methoxy-phenyl)-2,3-dihydro-1-benzothi epine-4-carboxamide (Compound 247) (226.4mg).
m.p. 198-201 °C
¹H-NMR (200MHz, CDCl₃) δ 1.45-1.91 (8H, m), 2.21 (3H, s), 2.44-2.65 (1H, m), 3.03-3.10 (2H, m), 3.26-3.31 (2H, m), 3.57 (2H, s), 3.86 (3H, s), 3.95 (4H, s), 6.98 (2H, d, J=8.8 Hz), 7.32 (2H, d, J=8.4 Hz), 7.37-7.43 (2H, m), 7.46-7.60 (6H, m), 7.68 (1H, br s).
IR (KBr) 3359, 1651, 1514, 1252, 1103, 1030, 926, 830 cm⁻¹

| Anal. Calcd. for C₃₄H₃₈N₂O₄S·0.3H₂O | | | |
|---|---|---|---|
| Calcd. | C, 70.88 ; | H, 6.75 ; | N, 4.86. |
| Found. | C, 70.86 ; | H, 6.70 ; | N, 4.77. |

### Working Example 248 (Production of Compound 248)

To a solution of N-[4-[N-(4,4-ethylenedioxy-cyclohexyl)-N-methylaminomet hyl]phenyl]-7-(4-methoxy-phenyl)-2,3-dihydro-1-benzothi epine-4-carboxamide (130mg) in THF (15ml) was added at room temperature 6N hydrochloric acid (1ml), and the mixture was stirred for 66 hours. To the mixture was added sodium bicarbonate solution, and extracted with ethyl acetate. The organic layer was washed with saturated brine and magnesium sulfate. Under reduced pressure, the mixture was concentrated, and the resulting solid was recrystallized from ethyl acetate/hexane to give pale yellow crystals of 7-(4-methoxyphenyl)-N-[4-[N-methyl-N-(4-oxocyclohexyl) aminomethyl]phenyl]-2,3-dihydro-1-benzothiepine-4-carbo xamide (Compound 248) (78.3mg).
m.p. 133-139 °C
¹H-NMR (200MHz, CDCl₃) δ 1.74-2.19 (4H, m), 2.23 (3H, s), 2.30-2.59 (4H, m), 2.81-2.97 (1H, m), 3.04-3.10 (2H, m), 3.26-3.32 (2H, m), 3.60 (2H, s), 3.86 (3H, s), 6.98 (2H, d, J=9.2 Hz), 7.33 (2H, d, J=8.4 Hz), 7.38-7.43 (2H, m), 7.48-7.58 (6H, m), 7.71 (1H, br s).
IR (KBr) 3273, 1711, 1651, 1605, 1515, 1408, 1317, 1248, 1180, 820 cm⁻¹

| Anal. Calcd. for C₃₂H₃₄N₂O₃S·0.2H₂O | | | |
|---|---|---|---|
| Calcd. | C, 72.48 ; | H, 6.54 ; | N, 5.28. |
| Found. | C, 72.33 ; | H, 6.42 ; | N, 5.13. |

### Working Example 249 (Production of Compound 249)

To a solution of 7-(4-morpholinophenyl)-2,3-dihydro-1-benzothiepine-4-carboxylic acid (150mg) and 1-hydroxy-benzotriazole (0.11g) in DMF (5ml) was added at room temperature 1-ethyl-3-(3-dimethylaminopropyl)carbo-diimide hydrochloride (0.16g), and the mixture was stirred for 1 hour. To the mixture was added a solution of 4-[N-methyl-N-(tetrahydropyran-4-yl)aminomethyl]aniline (135mg) and triethylamine (0.11ml) in DMF (5ml), and the mixture was stirred for 18 hours. Under reduced pressure, the mixture was concentrated, and to the mixture was added water. The mixture was extracted with ethyl acetate, and the organic layer was washed with saturated brine and dried with magnesium sulfate. Under reduced pressure, the solvent was evaporated, and the residue was purified with column chromatography (ethanol/ethyl acetate=1:2) to give yellow crystals of N-[4-[N-methyl-N-(tetrahydropyran-4-yl)aminomethyl]phen yl]-7-(4-morpholinophenyl)-2,3-dihydro-1-benzothiepine-4-carboxamide (Compound 249) (113.9mg).
m.p. 255-259 °C
¹H-NMR (200MHz, CDCL₃) δ 1.63-1.84 (4H, m), 2.21 (3H, s), 2.55-2.76 (1H, m), 3.02-3.10 (2H, m), 3.19-3.46 (8H, m), 3.58 (2H, s), 3.85-3.93 (4H, m), 3.98-4.10 (2H, m), 6.99 (2H, d, J=9.2 Hz), 7.32 (2H, d, J=8.4 Hz), 7.37-7.45 (2H, m), 7.49-7.58 (6H, m), 7.67 (1H, br s).
IR (KBr) 3288, 1653, 1606, 1522, 1232, 1119, 928, 816 cm⁻¹

| Anal. Calcd. for C₃₄H₃₉N₃O₃S·0.5H₂O | | | |
|---|---|---|---|
| Calcd. | C, 70.56 ; | H, 6.97 ; | N, 7.26. |
| Found. | C, 70.43 ; | H, 6.83 ; | N, 7.22. |

### Working Example 250 (Production of Compound 250)

To a solution of 7-(3,4-methylenedioxyphenyl)-2,3-dihydro-1-benzothiepin e-4-carboxylic acid (150mg) in THF (10ml) was added at room temperature oxalyl chloride (0.08ml) and a drop of DMF, and the mixture was stirred for 1 hour. Under reduced pressure, the solvent was evaporated, and the residue was dissolved in THF (20ml). To the mixture were added at 0°C 4-[N-methyl-N-(tetrahydropyran-4-yl)aminomethyl]aniline (112mg) and triethylamine (0.13ml), and the mixture was stirred at room temperature for 18 hours. The mixture was added to vigorously stirred water to stop the reaction and extracted with ethyl acetate. The organic layer was washed with saturated brine and dried with magnesium sulfate. Under reduced pressure, the mixture was concentrated, and the residue was purified with column chromatography (ethanol/ethyl acetate=1:3) and recrystallized from ethanol to give colorless crystals of 7-(3,4-methylenedioxyphenyl)-N-[4-[N-methyl-N-(tetra-hy dropyran-4-yl)aminomethyl]phenyl]-2,3-dihydro-1-benzoth iepine-4-carboxamide (Compound 250) (183.2mg).
m.p. 193-194 °C
¹H-NMR (200MHz, CDCl₃) δ 1.52-1.83 (4H, m), 2.21 (3H, s), 2.54-2.72 (1H, m), 3.04-3.10 (2H, m), 3.23-3.44 (4H, m), 3.57 (2H, s), 3.98-4.09 (2H, m), 6.01 (2H, s), 6.88 (1H, d, J=8.8 Hz), 7.01-7.07 (2H, m), 7.29-7.38 (4H, m), 7.46-7.58 (4H, m), 7.68 (1H, br s).
IR (KBr) 3334, 1647, 1506, 1475, 1408, 1313, 1232, 1041, 818 cm⁻¹

| Anal. Calcd. for C₃₁H₃₂N₂O₄S | | | |
|---|---|---|---|
| Calcd. | C, 70.43 ; | H, 6.10 ; | N, 5.30. |
| Found. | C, 70.28 ; | H, 5.94 ; | N, 5.14. |

### Working Example 251 (Production of Compound 251)

To a solution of 7-(4-ethoxyphenyl)-2,3-dihydro-1-benzoxepine-4-carboxyl ic acid (200mg) in THF (10ml) were added at room temperature oxalyl chloride (0.11ml) and a drop of DMF, and the mixture was stirred for 1 hour. Under reduced pressure, the mixture was concentrated, and the residue was dissolved inTHF (20ml) . To the mixture was added a solution of added at 0°C 4-[N-(4,4-ethylenedioxycyclohexyl)-N-methylaminomethyl]aniline (0.19g) and triethylamine (0.18ml) in THF (5ml), and the mixture was stirred at room temperature for 16 hours. To the mixture was added water, and the mixture was extracted with ethyl acetate. The organic layer was washed with saturated brine and dried with magnesium sulfate. Under reduced pressure, the solvent was evaporated, and the residue was purified with column chromatography (ethanol/ethyl acetate=1:3) and recrystallized from ethyl acetate/ diisopropylether) to give colorless crystals of 7-(4-ethoxyphenyl)-N-[4-[N-(4,4-ethylenedioxycyclohexyl )-N-methylaminomethyl]-phenyl]-2,3-dihydro-1-benzoxepin e-4-carboxamide (Compound 251) (119.1mg). The mother liquor was concentrated to give crude product (91.5mg).
m.p. 172-174 °C
¹H-NMR (200MHz, CDCl₃) δ 1.44 (3H, t, J=7.0 Hz), 1.51-1.88 (8H, m), 2.20 (3H, s), 2.44-2.64 (1H, m), 3.08 (2H, t, J=4.6 Hz), 3.56 (2H, s), 3.95 (4H, s), 4.08 (2H, q, J=7.0 Hz), 4.36 (2H, t, J=4.6 Hz), 6.96 (2H, d, J=9.0 Hz), 7.05 (1H, d, J=8.4 Hz), 7.32 (2H, d, J=8.4 Hz), 7.40-7.56 (8H, m).
IR (KBr) 3350, 1651, 1515, 1493, 1242, 1101, 922, 829, 802 cm⁻¹

| Anal. Calcd. for C₃₅H₄₀N₂O₅ | | | |
|---|---|---|---|
| Calcd. | C, 73.92 ; | H, 7.09 ; | N, 4.93. |
| Found. | C, 73.82 ; | H, 7.01 ; | N, 4.90. |

### Working Example 252 (Production of Compound 252)

To a solution of 7-(4-ethoxyphenyl)-N-[4-[N-(4,4-ethylenedioxycyclohexyl )-N-methylaminomethyl]phenyl]-2,3-dihydro-1-benzoxepine -4-carboxamide (151.5mg) in THF (10ml) was added at room temperature 3N hydrochloric acid (2ml), and the mixture was stirred for 22 hours. To the mixture was added saturated sodium bicarbonate solution, and the mixture was extracted with ethyl acetate. The organic layer was washed with saturated brine and dried with magnesium sulfate. Under reduced pressure, the mixture was concentrated to give colorless solid, which was recrystallized from ethyl acetate/diisopropylether to give colorless crystals of 7-(4-ethoxyphenyl)-N-[4-[N-methyl-N-(4-oxocyclohexyl) aminomethyl]phenyl]-2,3-dihydro-1-benzoxepine-4-carboxa mide (Compound 252) (103.5mg).
m.p. 146-148°C
¹H-NMR (200MHz, CDCl₃) δ 1.44 (3H, t, J=7.0 Hz), 1.80-2.19 (4H, m), 2.23 (3H, s), 2.29-2.59 (4H, m), 2.83-2.98 (1H, m), 3.04-3.12 (2H, m), 3.61 (2H, s), 4.08 (2H, q, J=7.0 Hz), 4.34-4.39 (2H, m), 6.96 (2H, d, J=8.8 Hz), 7.05 (1H, d, J=8.4 Hz), 7.33 (2H, d, J=8.0 Hz), 7.41-7.57 (8H, m).
IR (KBr) 3329, 1709, 1645, 1518, 1495, 1242, 825 cm⁻¹

| Anal. Calcd. for C₃₃H₃₆N₂O₄·0.25H₂O | | | |
|---|---|---|---|
| Calcd. | C, 74.91 ; | H, 6.95 ; | N, 5.29. |
| Found. | C, 74.68 ; | H, 6.92 ; | N, 5.28. |

### Working Example 253 (Production of Compound 253)

In THF (3.4ml) was dissolved 7-(5-methyl-2-thienyl)-2,3-dihydro-1-benzoxepine-4-carb oxylic acid (340mg), and to the mixture were added oxalyl chloride (0.198ml) and DMF (one drop) while stirring at room temperature. The mixture was stirred at room temperature for 2 hours. Under reduced pressure, the solvent was removed, and the resulting residue was dissolved in THF (5.1ml). The mixture was added dropwise to a solution of 4-[N-methyl-N-(tetrahydropyran-4-yl)aminomethyl]aniline (308mg) and triethylamine (0.473ml) in THF (5.1ml), under ice-cooling, and the mixture was stirred at room temperature for 13 hours. The mixture was poured into water, extracted with ethyl acetate, washed with saturated brine and dried with magnesium sulfate. Under reduced pressure, the solvent was removed, and the resulting residue was purified with silica gel column chromatography (ethyl acetate/ethanol=2/1) and recrystallized from hexane/ethyl acetate to give N-[4-[N-methyl-N-(tetrahydropyran-4-yl)amino-methyl]phe nyl]-7-(5-methyl-2-thienyl)-2,3-dihydro-1-benzoxepine-4 -carboxamide (Compound 253) (20mg).
m.p. 129-130°C
¹H-NMR (200MHz, CDCl₃) δ 1.50-1.82 (4H, m),2.21 (3H, s), 2.31 (3H, s), 2.65 (1H, m), 3.08 (2H, t, J=4.6Hz), 3.37 (2H, dt, J=11.2, 3.2Hz), 3.58 (2H, s), 4.04 (2H, m), 4.37 (2H, t, J=4.6Hz),6.92 (1H, d, J=5.2Hz), 7.04 (1H, d, J=5.2Hz), 7.18-7.52 (7H, m), 7.51-7.56 (2H, m)
IR (KBr)
3294, 1653, 1597, 1514, 1498, 1456, 1406, 1315, 1248, 733cm⁻¹

### Working Example 254 (Production of Compound 254)

In THF (10ml) was dissolved 7-(3-thienyl)-2,3-dihydro-1-benzoxepine-4-carboxylic acid (240mg), and to the mixture were added oxalyl chloride (0.15ml) and DMF (one drop) while stirring at room temperature, and the mixture was stirred at room temperature for 1.5 hours. Under reduced pressure, the solvent was removed, and the resulting residue in THF (6ml) was added dropwise to a solution of 4-[N-methyl-N-(tetrahydropyran-4-yl)aminomethyl]aniline (247mg) and triethylamine (0.35ml) in THF (10ml), under ice-cooling, and the mixture was stirred at room temperature for 14 hours. The mixture was poured into water, extracted with ethyl acetate, washed with saturated brine and dried with magnesium sulfate. Under reduced pressure, the solvent was removed, and the resulting residue was purified with silica gel column chromatography (ethyl acetate/ethanol=2/1) and recrystallized from hexane/ethyl acetate to give N- [4- [N-methyl-N- (tetrahydropyran-4-yl) amino-methyl]phenyl]-7-(3-thienyl)-2,3-dihydro-1-benzoxepine-4-carboxam ide (Compound 254) (180mg).
m.p. 194-195°C
¹H-NMR (200MHz, CDCl₃) δ 1.60-1.84 (4H, m), 2 . 22 (3H, s), 2.69 (1H, m), 3.09 (2H, t, J=4.6Hz), 3.36 (2H, dt, J=11.2, 2.6Hz), 3.60 (2H, s), 4.04 (2H, m), 4.34 (2H, t, J=4.6Hz), 7.03 (1H, d, J=8.4Hz), 7.25-7.42 (7H, m), 7.47 (1H, dd, J=8.4, 2.2Hz), 7.54 (1H, s), 7.58 (1H, s), 7.67 (1H, s)
IR (KBr)
3306, 1645, 1604, 1514, 1496, 1456, 1408, 1321, 1230, 781cm⁻¹

| Anal. Calcd. for C₂₈H₃₀N₂O₃S | | | |
|---|---|---|---|
| Calcd. | C,70.86; | H,6.37; | N,5.90. |
| Found. | C,70.74; | H,6.16; | N,5.92 |

### Working Example 255 (Production of Compound 255)

In THF 10ml was dissolved in 7-(4-methyl-2-thienyl)-2,3-dihydro-1-benzoxepine-4-carb oxylic acid (250mg), and to the mixture were added oxalyl chloride (0.145ml) and DMF (one drop) while stirring at room temperature, and the mixture was stirred at room temperature for 2 hours. Under reduced pressure, the solvent was removed, and the resulting residue in methylene chloride (10ml) was added dropwise to a solution of 4-[N-methyl-N-(tetrahydropyran-4-yl)aminomethyl]aniline (250mg) and triethylamine (0.35ml) in THF(5ml), under ice-cooling, and the mixture was stirred at room temperature for 13 hours. The mixture was poured into water, extracted with ethyl acetate, washed with saturated brine and dried with magnesium sulfate. Under reduced pressure, the solvent was removed, and the resulting residue was purified with silica gel column chromatography (ethyl acetate/ethanol=2/1) and recrystallized from hexane/ethyl acetate to give N-[4-[N-methyl-N-(tetra-hydropyran-4-yl)aminomethyl]phe nyl]-7-(4-methyl-2-thienyl)-2,3-dihydro-1-benzoxepine-4 -carboxamide (Compound 255) (185mg).
m.p. 147-148°C
¹H-NMR (200MHz, CDCl₃) δ 1.60-1.80 (4H, m), 2.21 (3H, s), 2.31 (3H, s), 2.64 (1H, m), 3.06 (2H, t, J=4.2Hz), 3.37 (2H, dt, J=11.4, 2.8Hz), 3.57 (2H, s), 4.04 (2H, m), 4.33 (2H, t, J=4.2Hz), 6.82 (1H, d, J=1.2Hz), 6.99 (1H, d, J=8.4Hz), 7.04 (1H, d, J=1.2Hz), 7.19 (1H, s), 7.41-7.57 (5H, m), 7.67 (1H, s) '
IR (KBr) 3292, 1653, 1597, , 1514, 1456, 1406, 1315, 1246, 733cm⁻¹

| Anal. Calcd. for C₂₉H₃₂N₂O₃S · 0.5H₂O | | | |
|---|---|---|---|
| Calcd. | C,69.99; | H,6.68; | N,5.63. |
| Found. | C,69.85; | H,6.43; | N,5.68. |

### Working Example 256 (Production of Compound 256)

In THF (5.0ml) was dissolved 7-(4-fluorophenyl)-2,3-dihydro-1-benzoxepine-4-carboxyl ic acid (137mg), and to the mixture were added DMF (one drop) and oxalyl chloride (0.085ml). The mixture was stirred at room temperature for 1 hour, and the solvent was removed under reduced pressure. The residue was dissolved in THF (5.0ml), and to the mixture was added a solution of 4-[(N-methyl-N-tetrahydropyran-4-yl)aminomethyl]aniline (117mg) and triethylamine (0.135ml) in THF (5.0ml). The mixture was stirred at room temperature for 1 hour, and to the mixture was added water (50ml). The mixture was extracted with ethyl acetate (100ml and 50ml), and the organic layer was dried with anhydrous magnesium sulfate. The solvent was removed under reduced pressure, and the residue was purified with silica gel column chromatography and recrystallized to give 7-(4-fluorophenyl)-N-[4-[(N-methyl-N-tetrahydropyran-4-yl)amino-me thyl]-phenyl]-2,3-dihydro-1-benzoxepine-4-carboxamide (Compound 256) (149mg, 64%) as pale yellow needle crystals. mp 177-178 °C.
IR (KBr) 3351, 2938, 1649, 1632, 1595, 1518, 1491, 1412, 1316, 1219, 829 cm⁻¹.
¹H NMR (200MHz, CDCl₃) δ 1.69-1.77 (4H, m), 2.21 (3H, s), 2.60-2.70 (1H, m), 3.09 (2H, t, J=4.2Hz), 3.37 (2H, td, J=11.1, 2.9Hz), 3.58 (2H, s), 4.04 (2H, d, J=10.6Hz), 4.37 (2H, t, J=4.7Hz), 7.04-7.16 (3H, m), 7.29-7.56 (8H, m).

| | | | |
|---|---|---|---|
| Anal. Calcd. for C₃₀H₃₁FN₂O₃; | C, 74.05, | H, 6.42, | N, 5.76. |
| Found; | C, 73.90, | H, 6.35, | N, 5.53. |

### Working Example 257 (Production of Compound 257)

To a suspension of 6-(4-methylphenyl)-2H-thiochromene-3-carboxylic acid (0.36 g, 1.28 mmol) in dichloromethane (5 ml) were added at 0°C oxalate chloride (0.33 ml, 3.84 mmol) and N,N-dimethylformamide (one drop), and the mixture was stirred at room temperature for 1 hour. The solvent was evaporated, and the residue was dissolved in tetrahydrofuran (3 ml). To the mixture was added dropwise a solution of aniline (0.31 g, 1.41 mmol) and triethylamine (0.54 ml, 3.84 mmol) in tetrahydrofuran (2 ml), and the mixture was stirred for 3 hours. To the mixture was added water, and the mixture was extracted with ethyl acetate. The organic layer was washed with saturated brine and dried with magnesium sulfate. The solvent was evaporated, and the resulting powder was washed with hexane to give 6-(4-methylphenyl)-N-(4-((N-methyl-N-tetra-hydropyran-4 -yl)amino)-methyl)phenyl-2H-thiochromene-3-carboxamide (Compound 257) (0.45 g, 72%) as pale yellow powder. m.p. 200°C.
¹H-NMR (DMSO-d₆) δ : 7.32-7.36 (3H, m), 7.21-7.28 (4H, m), 7.07 (1H, d, J=8.2), 6.92-6.99 (4H, m), 3.50-3.66 (2H, m), 3.48 (2H, s), 3.20 (2H, s), 2.86-3.00 (2H, m), 2.20-2.37 (1H, m), 2.03 (3H, s), 1.78 (3H, s), 1.08-1.46 (4H, m).

| Anal. Calcd for C₃₀H₃₂N₂O₂S · 0.25H₂O : | | | |
|---|---|---|---|
| | C; 73.66, | H; 6.70, | N; 5.73. |
| Found : | C; 73.84, | H; 6.60, | N; 5.84. |

### Working Example 258 (Production of Compound 258)

To a suspension of 6-(4-methylphenyl)-2H-thiochromene-3-carboxylic acid (226 mg, 0.785 mmol) in tetrahydrofuran (7 ml) were added oxalyl chloride (0.21 ml, 2.35 mmol) and N,N-dimethylformamide (one drop), and the mixture was stirred at room temperature for 1 hour. Under reduced pressure, the solvent was evaporated, and the residue was dissolved in tetrahydrofuran (5ml). To the mixture was added dropwise a solution of (E)-4-((N-(4-hydroxycyclohexyl)-N-methyl)aminomethyl)an iline (202 mg, 0.864 mmol) and triethylamine (0.33 ml, 2.35 mmol) in tetrahydrofuran (2 ml), and the mixture was stirred for 15 hours. To the mixture was added water, and the mixture was extracted with ethyl acetate. The extract was washed with saturated brine and dried with magnesium sulfate. The solvent was evaporated, and the residue was purified with silica gel column chromatography [ethylacetate:ethanol (2:1)] to give (E)-N-(4-((N-(4-hydroxycyclohexyl)-N-methyl)amino) methyl)phenyl-6-(4-methylphenyl)-2H-thiochromene-3-carb oxamide (Compound 258) (160 mg, 41%), which was recrystallized from ethyl acetate/hexane to give yellow crystals.
m.p. 149°C
¹H-NMR(CDCl₃) δ : 7.73 (1H, brs), 7.42-7.58 (6H, m), 7.22-7.38 (5H, m), 3.81 (2H, d, J=0.8), 3.59 (2H, s), 3.55-3.68 (1H, m), 2.42-2.61 (1H, m), 2.40 (3H, s), 2.21 (3H, s), 1.86-2.20 (4H, m), 1.23-1.57 (4H, m).

| Anal. Calcd for C₃₁H₃₄N₂O₄S · 1.25H₂O: | | | |
|---|---|---|---|
| | C; 71.44, | H; 7.06, | N; 5.37. |
| Found: | C; 71.12, | H; 6.53, | N;5.51. |

### Working Example 259 (Production of Compound 259)

To a suspension of 6-(4-methylphenyl)-2H-thiochromene-3-carboxylic acid (204 mg, 0.708 mmol) in tetrahydrofuran (6 ml) were added oxalyl chloride (0.19 ml) and N,N-dimethylformamide (one drop), and the mixture was stirred at room temperature for 1 hour. Under reduced pressure, the solvent was evaporated, and the residue was dissolved in tetrahydrofuran (5 ml). To the mixture was added dropwise a solution of 4-((N-(2-methoxy-ethyl)-N-methyl)aminomethyl)aniline (153 mg, 0.802 mmol) and triethylamine (0.30 ml) in tetrahydrofuran (2 ml), and the mixture was stirred for 15 hours. To the mixture was added water, and the mixture was extracted with ethyl acetate. The extract was washed with saturated brine and dried with magnesium sulfate. The solvent was evaporated, and the residue was purified with silica gel column chromatography [ethyl acetate:ethanol (2:1)] to give N-(4-(N-(2-methoxyethyl)-N-methyl)aminomethyl)-phenyl-6 -(4-methylphenyl)-2H-thiochromene-3-carboxamide (Compound 259) (170 mg, 52%), which was recrystallized from ethyl acetate/hexane to give yellow crystals.
m.p. 101°C
¹H-NMR (CDCl₃) δ : 7.67 (1H, brs), 7.41-7.57 (6H, m), 7.20-7.38 (5H, m), 3.82 (2H, t, J=0.8), 3.56 (2H, s), 3.53 (2H, t, J=5.8), 3.35 (3H, s), 2.61 (2H, t, J=5.8), 2.40 (3H, s), 2.28 (3H, s).

| Anal. Calcd for C₂₈H₃₀N₂O₂S · 0.25H₂O: | | | |
|---|---|---|---|
| | C; 72.62, | H; 6.64, | N; 6.05. |
| Found: | C; 72.43, | H; 6.39, | N; 6.36. |

### Working Example 260 (Production of Compound 260)

To a suspension of 7-(4-methylphenyl)-2,3-dihydro-1-benzothiepine-4-carbox ylic acid (292 mg, 0.987 mmol) in tetrahydrofuran (10 ml) were added at 0°C oxalyl chloride (0.26 ml) and N,N-dimethylformamide (one drop), and the mixture was stirred at room temperature for 1.5 hours. The solvent was evaporated, and the residue was dissolved in tetrahydrofuran ,(8 ml). To the residue was added dropwise a solution of 4-((N-(3-ethoxycarbonylethyl)-N-methyl)-aminomethyl)ani line (233 mg, 0.987 mmol) and triethylamine (0.42 ml) in tetrahydrofuran (2 ml) at 0°C, and the mixture was stirred at room temperature for 17 hours. To the mixture was added water, and the mixture was extracted with ethyl acetate. The extract was washed with saturated brine and dried with magnesium sulfate. The solvent was evaporated, and the residue was purified with silica gel column chromatography [ethyl acetate] to give N-(4-(N-(3-ethoxycarbonylethyl)-N-methyl)aminomethyl)phenyl-7-(4-methylphenyl)-2,3-dihydro-1-benzothiepine-4-carboxamide (Compound 260) (408 mg, 80%), which was recrystallized from acetone/ethanol to give colorless crystals. m.p. 124°C.
¹H-NMR (CDCl₃) δ 7.89 (1H, brs), 7.38-7.58 (7H, m), 7.22-7.30 (4H, m), 4.14 (2H, q, J=7.4), 3.48 (2H, s), 3.25 (2H, dt, J=5.4, 1.4) 3.05 (2H, t, J=5.4), 2.74 (2H, t, J=6.8), 2.51 (2H, t, J=6.8), 2.39 (3H, s), 2.19 (3H, s), 1.25 (3H, t, J=7.4).

| | | | |
|---|---|---|---|
| Anal. Calcd for C₃₁H₃₄N₂O₃S: | C; 72.34, | H; 6.66, | N; 5.44. |
| Found: | C ; 72.32, | H; 6.43, | N; 5.45. |

### Working Example 261 (Production of Compound 261)

To a suspension of 7-(4-methylphenyl)-2,3-dihydro-1-benzothiepine-4-carbox ylic acid (222 mg, 0.750 mmol) in tetrahydrofuran (7 ml) was added at 0°C oxalyl chloride (0.26 ml, 2.97 mmol) and N,N-dimethylformamide (one drop), and the mixture was stirred at room temperature for 2 hours. The solvent was evaporated, and the residue was dissolved in tetrahydrofuran (5 ml). To the residue was added dropwise a solution of aniline (149 mg, 0.825 mmol) and triethylamine (0.31 ml, 2.25 mmol) in tetrahydrofuran (2 ml) at 0°C, and the mixture was stirred at room temperature for 3 days. To the mixture was added water, and the mixture was extracted with ethyl acetate. The extract was washed with saturated brine and dried with magnesium sulfate. The solvent was evaporated, and the residue was purified with silica gel column chromatography [ethyl acetate:methanol: triethylamine (5:1:0.6)] to give N-(4-(N-(2-hydroxy-ethyl)-N-methyl)aminomethyl)phenyl-7 -(4-methylphenyl)-2,3-dihydro-1-benzothiepine-4-carboxa mide (Compound 261) (310 mg, 90%).
m.p. 138°C.
¹H-NMR (CDCl₃) δ : 7.74 (1H, brs), 7.40-7.59 (7H, m), 7.23-7.32 (4H, m), 3.64 (2H, t, J=5.2), 3.58 (2H, s), 3.28 (2H, t, J=5.6), 3.07 (2H, t, J=5.6), 2.62 (2H, t, J=5.2).

| | | | |
|---|---|---|---|
| Anal. Calcd for C₃₁H₃₄N₂O₃S: | C; 72.34, | H; 6.66, | N; 5.44. |
| Found: | C; 72.32, | H; 6.43, | N; 5.45. |

### Working Example 262 (Production of Compound 262)

To a suspension of 6-(4-methylphenyl)-2-pyridine-acrylic acid (160mg, 0.67mmol) in DMF (5ml) were added at 0°C 1-hydroxybenzotriazole (99mg, 0.73mmol), 4-[N-methyl-N-(4-tetrahydropyranyl)aminomethyl]aniline (162mg, 0.74 mmol), 1-ethyl-3-(3-dimethylaminopropyl)carbodiimide hydrochloride (192mg, 1.00mmol), triethylamine (0.28ml, 2.01mmol) and 4-dimethylaminopyridine (10mg) in this order, and the mixture was stirred at room temperature for 17 hours. The mixture was concentrated under reduced pressure, and to the residue was added ethyl acetate (40ml). The mixture was washed with water (5ml, 3ml × 2), saturated sodium bicarbonate solution (3ml×3) and saturated brine (3ml) in this order. The organic layer was dried with anhydrous sodium sulfate and concentrated under reduced pressure, and the residue was purified with column chromatography (silica gel 15g, ethyl acetate/methanol=9/1). The desired fraction was concentrated under reduced pressure to give N-[4-[N-methyl-N-(4-tetrahydropyranyl)aminomethyl]pheny 1]-6-(4-methylphenyl)-2-pyridineacrylamide (Compound 262) (259mg, 0.59mmol, 88%).
IR (KBr): 1667, 1634, 1601, 1537, 1514 cm⁻¹.
¹H-NMR (CDCl₃) δ : 1.55-1.85 (4H, m), 2.21 (3H, s), 2.43 (3H, s), 2.55-2.75 (1H, m), 3.30-3.45 (2H, m), 3.58 (2H, s), 3.95-4.10 (2H, m), 7.20-7.50 (5H, m), 7.45-7.85 (6H, m), 7.98 (2H, d, J=8.2Hz).

### Working Example 263 (Production of Compound 263)

In DMF (5ml) was dissolved 7-(3,4-methylene-dioxyphenyl)-2,3-dihydro-1-benzoxepine -4-carboxylic acid, and to the mixture were added 1-hydroxybenzotriazole (67mg, 0.50mmol), 4-[N-methyl-N-(4-tetrahydropyranyl)-aminomethyl]aniline (109mg, 0.49mmol), 1-ethyl-3-(3-dimethylaminopropyl)-carbodiimide hydrochloride (130mg, 0.68mmol), triethylamine (0.189ml, 1.36mmol) and 4-dimethylaminopyridine (3mg). The mixture was stirred at room temperature for 18 hours and concentrated under reduced pressure. To the residue was added ethyl acetate (60m), and the mixture was washed with water (5ml ×3), saturated sodium bicarbonate solution (3ml ×3) and saturated brine (5ml) in this order. The organic layer was dried with anhydrous sodium sulfate and concentrated under reduced pressure. The residue was purified with column chromatography (silica gel 15g, ethyl acetate). The desired fraction was concentrated under reduced pressure, and to the residue was added ethyl acetate. Insoluble materials were filtered, and the insoluble materials were washed with ethyl acetate and dried under reduced pressure to give 7-(3,4-methylenedioxyphenyl)-N-[4-[N-methyl-N-(4-tetrah ydro-pyranyl)aminomethyl]phenyl]-2,3-dihydro-1-benzoxep ine-4-carboxamide (Compound 263) (187mg, 0.36mmol, 81%).
IR (KBr): 1653, 1597, 1514 cm⁻¹.
¹H-NMR (CDCl₃) δ: 1.55-1.85 (4H, m), 2.21 (3H, s), 2.55-2.80 (1H, m), 3.00-3.15 (2H, m), 3.30-3.45 (2H, m), 3.58 (2H, s), 3.95-4.15 (2H, m), 4.30-4.45 (2H, m), 6.01 (2H, s), 6.88 (1H, d, J=8.6Hz), 6.95-7.10 (3H, m), 7.20-7.65 (7H, m).

### Working Example 264 (Production of Compound 264)

In DMF (6ml) was dissolved 7-morpholino-2,3-dihydro-1-benzoxepine-4-carboxylic acid (200mg, 0.73mmol), and to the mixture were added at 0°C 1-hydroxybenzotriazole (108mg, 0.80mmol), 4-[N-methyl-N-(4-tetrahydropyranyl)-aminomethyl]aniline (176mg, 0.80mmol), 1-ethyl-3-(3-dimethylaminopropyl)carbodiimide hydrochloride (209mg, 1.09mmol), triethylamine (0.304ml, 2.18mmol) and 4-dimethylaminopyridine (3mg). The mixture was stirred at room temperature for 13 hours and concentrated under reduced pressure. To the residue was added ethyl acetate (40ml), and the mixture was washed with water (5ml ×3), saturated sodium bicarbonate solution (5ml×3) and saturated brine (5ml) in this order. The organic layer was dried with anhydrous sodium sulfate and concentrated under reduced pressure. The residue was purified with column chromatography (silica gel 15g, ethyl acetate/methanol=1/0 → 9/1). The desired fraction was concentrated under reduced pressure, and to the residue was added diethylether. Insoluble materials were filtered, and the insoluble materials were washed with diethylether and dried under reduced pressure to give N-[4-[N-methyl-N-(4-tetra-hydropyranyl)aminomethyl]phen yl]-7-morpholino-2,3-dihydro-1-benzoxepine-4-carboxamid e (Compound 264) (248mg, 0.52mmol, 71%).
IR (KBr): 1655, 1597, 1507 cm⁻¹.
¹H-NMR (CDCl₃) δ : 1.5-1.85 (4H, m), 2.21 (3H, s), 2.55-2.75 (1H, m), 3.0-3.15 (6H, m), 3.3-3.45 (2H, m), 3.57 (2H, s), 3.8-3.9 (4H, m), 3.95-4.1 (2H, m), 4.29 (2H, t, J=4.7Hz), 6.8-7.0 (3H, m), 7.15-7.35 (3H, m), 7.5-7.6 (2H+1H (amide-H), m) .

### Working Example 265 (Production of Compound 265)

In DMF (6ml) was dissolved 7-(4-methylphenyl)-2,3-dihydro-1-benzoxepine-4-carboxyl ic acid (140mg, 0.50 mmol), and to the mixture were added at 0°C 1-hydroxy-benzotriazole (74mg, 0.55mmol), 4-[N-(2-pyrimidinyl)-aminomethyl]aniline (100mg, 0.50mmol) and 1-ethyl-3-(3-dimethylaminopropyl)-carbodiimide hydrochloride (144mg, 0.75mmol). The mixture was stirred at room temperature for 22 hours and concentrated under reduced pressure. To the residue was added ethyl acetate (40ml), and the mixture was washed with water (5ml), saturated sodium bicarbonate solution (5ml×3) and saturated brine (5ml) in this order. The organic layer was driedwith anhydrous sodium sulfate and concentrated to about 3ml under reduced pressure. Precipitated insoluble materials were filtered and the insoluble materials were washed with ethyl acetate and dried under reduced pressure to give N-[4-[N-(2-pyrimidinyl)-aminomethyl]phenyl]-7-(4-methyl phenyl)-2,3-dihydro-1-benzoxepine-4-carboxamide (Compound 265) (129mg, 0.28mmol, 56%).
IR (KBr): 1647, 1591, 1518 cm⁻¹.
¹H-NMR (DMSO-d₆) δ: 2.34 (3H, s), 2.9-3.05 (2H, m), 4.2-4.35 (2H, m), 4.46 (2H, d, J=6.6Hz), 6.57 (1H, t, J=4.8Hz), 7.04 (1H, d, J=8.4Hz), 7.2-7.35 (5H, m), 7.5-7.75 (7H, m), 8.27 (2H, d, J=4.8Hz), 9.91 (1H, s).

### Working Example 266 (Production of Compound 266)

To a mixture of 7-(2-methyl-1H-tetrazol-5-yl)-2,3-dihydro-1-benzoxepine -4-carboxylic acid (180mg, 0.66 mmol), 4-[N-methyl-N-(tetrahydropyran-4-yl)aminomethyl]-aniline (160mg, 0.73mmol), 1-hydroxybenzotriazole (98mg, 0.73mmol) and DMF (10ml) were added at 0°C 1-[3-(dimethylamino)propyl]-3-ethylcarbodiimide hydrochloride (190mg, 0.99mmol) and triethylamine (0.276ml, 1.98mmol), and the mixture was stirred at room temperature for 24 hours. The mixture was concentrated under reduced pressure, and to the residue was added ethyl acetate (40ml). The mixture was washed with saturated sodium bicarbonate solution (5ml ×3) and saturated brine (5ml) in this order. The organic layer was dried with anhydrous sodium sulfate and concentrated under reduced pressure, and the residue was purified with column chromatography (silica gel 15g, ethyl acetate). The desired fraction was concentrated under reduced pressure, and to the residue was added ethyl acetate. Insoluble materials were filtered, and the insoluble materials were washed with ethyl acetate and dried under reduced pressure to give 7-(2-methyl-1H-tetrazol-5-yl)-N-[4-[N-methyl-N-(tetrahy dropyran-4-yl)aminomethyl]-phenyl]-2,3-dihydro-1-benzox epine-4-carboxamide (Compound 266) (217mg, 0.46 mmol, 69%).
IR (KBr): 1647, 1628, 1611, 1595, 1522 cm⁻¹.
¹H-NMR (DMSO-d₆) δ : 1.35-1.8 (4H, m), 2.10 (3H, s), 2.4-2.7 (1H, m), 2.9-3.1 (2H, m), 3.15-3.4 (2H, m), 3.52 (2H, s), 3.8-4.0 (2H, m), 4.25-4.45 (2H, m), 4.42 (3H, s), 7.16 (1H, d, J=8.4Hz), 7.26 (2H, d, J=8.4Hz), 7.40 (1H, s), 7.66 (2H, d, J=8.4Hz), 7.92 (1H, dd, J=1.9, 8.4Hz), 8.19 (1H, d, J=1.9Hz).

### Working Example 267 (Production of Compound 267)

To a mixture of 7-(1-methyl-1H-tetrazol-5-yl)-2,3-dihydro-1-benzoxepine -4-carboxylic acid (69mg, 0.25 mmol), 4-[N-methyl-N-(tetrahydropyran-4-yl)aminomethyl]aniline (61mg, 0.28mmol), 1-hydroxybenzotriazole (38mg, 0.28mmol) and DMF (4ml) were added at 0°C 1-[3-(dimethylamino)-propyl]-3-ethylcarbodiimide hydrochloride (97mg, 0.51mmol) and triethylamine (0.106ml, 0.76mmol), and the mixture was stirred at room temperature for 2 days. The mixture was concentrated under reduced pressure, and to the residue was added ethyl acetate. The mixture was washed with saturated sodium bicarbonate solution. The organic layer was dried with anhydrous sodium sulfate and concentrated under reduced pressure. The residue was purified with column chromatography (silica gel 10g, ethyl acetate). The desired fraction was concentrated under reduced pressure, and to the residue was added ethyl acetate. Insoluble materials were filtered and the insoluble materials were washed with ethyl acetate and dried under reduced pressure to give 7-(1-methyl-1H-tetrazol-5-yl)-N-[4-[N-methyl-N-(tetrahy dropyran-4-yl)aminomethyl]-phenyl]-2,3-dihydro-1-benzox epine-4-carboxamide (Compound 267) (84mg, 0.18mmol, 70%).
IR (KBr): 1649, 1630, 1597, 1518 cm⁻¹.
¹H-NMR (DMSO-d₆) δ : 1.35-1.8 (4H, m), 2.10 (3H, s), 2.45-2.7 (1H, m), 2.95-3.1 (2H, m), 3.15-3.4 (2H, m), 3.51 (2H, s), 3.8-4.0 (2H, m), 4.20 (3H, s), 4.3-4.45 (2H, m), 7.22 (1H, d, J=8.4Hz), 7.26 (2H, d, J=8.6Hz), 7.35 (1H, s), 7.64 (2H, d, J=8.6Hz), 7.76 (1H, dd, J=2.2, 8.4Hz), 7.99 (1H, d, J=2.2Hz).

### Working Example 268 (Production of Compound 268)

In DMF (12.0ml) was dissolved 1-methyl-7-(4-methylphenyl)-2,3-dihydro-1-benzoazepine-4-carboxylic acid hydrochloride (386mg), and to the mixture was added thionyl chloride (0.26ml). The mixture was stirred at room temperature for 30 minutes, and the solvent was evaporated under reduced pressure. The residue was dissolved in dichloromethane (10.0ml). Thus prepared acid chloride solution was added dropwise at 0°C to a solution of 4-[[N-methyl-N-(tetrahydropyran-4-yl)amino]methyl]anili ne (310mg) and triethylamine (0.82ml) in dichloromethane (4.0ml) . The mixture was stirred at 0°C for 10 minutes and then at room temperature for 22 hours. To the mixture was added water (100ml), and the mixture was extracted with dichloromethane (100ml ; twice). The organic layer was dried with anhydrous magnesium sulfate, and the solvent was evaporated under reduced pressure. The residue was purified with silica gel column chromatography (75g, ethyl acetate:ethanol=9:1) and recrystallized from ethanol to give 1-methyl-7-(4-methylphenyl)-N-[4-[[N-methyl-N-(tetrahyd ropyran-4-yl)amino]methyl]phenyl]-2,3-dihydro-1-benzoaz epine-4-carboxamide (Compound 268) (250mg, 43%). mp 178-181°C.
¹H NMR (200MHz, CDCl₃) δ 1.64-1.76 (4H, m), 2.21 (3H, s), 2.38 (3H, s), 2.66 (1H, septet, J=5.3Hz), 2.96 (2H, t, J=4.4Hz), 3.09 (3H, s), 3.30-3.43 (2H + 2H, m), 3.58 (2H, s), 4.01-4.06 (2H, m), 6.88 (1H, d, J=8.6Hz), 7.23 (2H, d, J=8.0Hz), 7.30 (2H, d, J=8.4Hz), 7.42, (1H, s), 7.461 (2H, d, J=8.2Hz), 7.466 (1H, dd, J=8.3, 2.3Hz), 7.535 (2H, d, J=8.4Hz), 7.539 (1H, d, J=2.6Hz), 7.58 (1H, s).
IR (KBr) 3337, 2949, 2851, 1653, 1516, 1501, 1341, 1304, 1238, 818, 521 cm⁻¹.

| | | | |
|---|---|---|---|
| Anal. Calcd. for C₃₂H₂₇N₃O₂: | C, 77.54; | H, 7.52; | N, 8.48. |
| Found: | C,77.51; | H,7.43; | N,8.44. |

### Working Example 269 (Production of Compound 269)

In water : ethanol : toluene (1:1:10, 18.0ml) were dissolved 4-ethoxyphenyl borate (252mg) and 7-bromo-1-methyl-N-[4-[[N-methyl-N-(tetrahydropyran-4-y 1)amino]-methyl]phenyl]-2,3-dihydro-1-benzoazepine-4-ca rboxamide (613mg), and to the mixture was added potassium carbonate (420mg). The mixture was stirred under argon atmosphere for 30 minutes, and to the mixture was added tetrakis-triphenylphosphine palladium (59mg). Under argon atmosphere, the mixture was refluxed for 17 hours. The mixture was diluted with ethyl acetate (200ml) and washed with water (50ml) and saturated brine (50ml). The organic layer was dried with anhydrous magnesium sulfate, and the solvent was evaporated under reduced pressure. The residue was purified with silica gel column chromatography (75g, ethyl acetate:ethanol=9:1) and recrystallized from ethanol to give 7-(4-ethoxyphenyl)-1-methyl-N-[4-[[N-methyl-N-(tetrahyd ropyran-4-yl)amino]methyl]phenyl]-2,3-dihydro-1-benzoaz epine-4-carboxamide (Compound 269) (230mg, 35%).
mp 150.5-152°C.
¹H NMR (200MHz, CDCl₃) δ 1.44 (3H, t, J=7.0Hz), 1.64-1.77 (4H, m), 2.21 (3H, s), 2.57-2.72 (1H, m), 2.96 (2H, t, J=4.5Hz), 3.08 (3H, s), 3.31-3.43 (2H + 2H, m), 3.57 (2H, s), 4.01-4.09 (2H, m), 4.07 (2H, q, J=7.0Hz), 6.88 (1H, d, J=8.4Hz), 6.95 (2H, d, J=8.8Hz), 7.30 (2H, d, J=8.6Hz), 7.40-7.55 (1H + 1H + 1H + 1H, concealed under 7.45 and 7.53), 7.47 (2H, d, J=9.0Hz), 7.53 (2H, d, J=8.8Hz).
IR (KBr) 3372, 2955, 2847, 1680, 1605, 1595, 1518, 1503, 1314, 1240, 1194, 812 cm⁻¹.

| | | | |
|---|---|---|---|
| Anal. Calcd. for C₃₃H₃₉N₃O₃•0.5H₂O: | C, 74.13 ; | H, 7.54 ; | N, 7.86. |
| Found: | C,74.34; | H,7.31; | N,7.96. |

### Working Example 270 (Production of Compound 270)

In water:ethanol:toluene (1:1:10, 18.0ml) were dissolved 4-ethylphenyl borate (227mg) and 7-bromo-1-methyl-N-[4-[[N-methyl-N-(tetrahydropyran-4-y 1)amino]-methyl]phenyl]-2,3-dihydro-1-benzoazepine-4-ca rboxamide (611mg), and to the mixture was added potassium carbonate (418mg). The mixture was stirred under argon atmosphere for 30 minutes, and to the mixture was added tetrakis-triphenylphosphine palladium (59mg). Under argon atmosphere, the mixture was refluxed for 17 hours, and the mixture was diluted with ethyl acetate (200ml) and washed with water (50ml) and saturated brine (50ml). The organic layer was dried with anhydrous magnesium sulfate, and the solvent was evaporated under reduced pressure. The residue was purified with silica gel column chromatography (75g, ethyl acetate:ethanol=9:1) and recrystallized from ethanol to give 7-(4-ethylphenyl)-1-methyl-N-[4-[[N-methyl-N-(tetrahydr opyran-4-yl)amino]methyl]phenyl]-2,3-dihydro-1-benzoaze pine-4-carboxamide (Compound 270) (252mg, 39%).
mp 164-165°C.
¹H NMR (200MHz, CDCl₃) δ 1.27 (3H, t, J=7.6Hz), 1.66-1.76 (4H, m), 2.21 (3H, s), 2.54-2.70 (1H, m), 2.69 (2H, q, J=7.7Hz), 2.96 (2H, t, J=4.7Hz), 3.09 (3H, s), 3.29-3.43 (4H, m), 3.57 (2H, s), 4.01-4.06 (2H, m), 6.89 (1H, d, J=8.6Hz), 7.26 (2H, d, J=8.4Hz), 7.30 (2H, d, J=8.8Hz), 7.40 (1H, s), 7.48 (1H, dd, J=8.6, 2.2Hz), 7.49 (2H, d, J=9.2Hz), 7.54 (2H, d, J=8.8Hz), 7.55 (1H, d, J=2.2Hz), 1H was concealed under 7.40-7.56.
IR (KBr) 3364, 2946, 2851, 1653, 1514, 1341, 1304, 1233, 1188, 824, 575, 519 cm⁻¹.

| | | | |
|---|---|---|---|
| Anal. Calcd. for C₃₃H₃₉N₃O₂: | C, 77.76; | H, 7.71; | N, 8.24. |
| Found: | C, 77.81; | H, 7.64; | N, 8.27. |

### Working Example 271 (Production of Compound 271)

In water:ethanol:toluene (1:1:10, 18.0ml) were dissolved 4-trifluorophenyl borate (190mg) and 7-bromo-1-methyl-N-[4-[[N-methyl-N-(tetrahydropyran-4-y l)-amino]methyl]phenyl]-2,3-dihydro-1-benzoazepine-4-ca rboxamide (403mg), and to the mixture was added potassium carbonate (276mg). The mixture was stirred under argon atmosphere for 30 minutes, and to the mixture was added tetrakistriphenylphosphine palladium (39mg). Under argon atmosphere, the mixture was refluxed for 17 hours, and the mixture was diluted with ethyl acetate (200ml) and washed with water (50ml) and saturated brine (50ml). The organic layer was dried with anhydrous magnesium sulfate, and the solvent was evaporated under reduced pressure. The residue was purified with silica gel column chromatography (75g, ethyl acetate: ethanol = 9:1) and recrystallized from ethanol to give 1-methyl-N-[4-[[N-methyl-N-(tetrahydropyran-4-yl)amino] -methyl]phenyl]-7-(4-trifluoromethylphenyl)-2,3-dihydro -1-benzoazepine-4-carboxamide (Compound 271) (177mg, 39%).
mp 187.5-188.5°C.
¹H NMR (200MHz, CDCl₃) δ 1.69-1.77 (4H, m), 2.21 (3H, s), 2.57-2.72 (1H, m), 2.98 (2H, t, J=4.6Hz), 3.12 (3H, s), 3.37 (2H, td, J=11.2, 3.3Hz), 3.38 (2H, t, J=4.7Hz), 3.57 (2H, s), 4.01-4.06 (2H, m), 6.91 (1H, d, J=8.4Hz), 7.30 (2H, d, J=8.4Hz), 7.42 (1H, s), 7.49 (1H, dd, J=8.4, 2.2Hz), 7.54 (2H, d, J=8.4Hz), 7.55 (1H, s), 7.58 (1H, d, J=2.2Hz), 7.66 (4H, s).
IR (KBr) 2949, 2847, 1651, 1603, 1516, 1325, 1163, 1115, 1073, 847, 812cm⁻¹.

| | | | |
|---|---|---|---|
| Anal. Calcd. for C₃₂H₃₃F₃N₃O₂: | C, 69.93; | H, 6.24; | N, 7.65. |
| Found: | C, 69.66; | H, 6.20; | N, 7.71. |

### Working Example 272 (Production of Compound 272)

In water:ethanol:toluene (1:1:10, 18.0ml) were dissolved 4-(4-morpholino)phenyl borate (208mg) and 7-bromo-1-methyl-N-[4-[[N-methyl-N-(tetrahydropyran-4-y 1)amino]methyl]phenyl]-2,3-dihydro-1-benzoazepine-4-car boxamide (406mg), and to the mixture was added potassium carbonate (278mg). The mixture was stirred under argon atmosphere for 30 minutes, and to the mixture was added tetrakistriphenylphosphine palladium (39mg). Under argon atmosphere, the mixture was refluxed for 17 hours, and the mixture was diluted with ethyl acetate (200ml) and washed with water (50ml) and saturated brine (50ml). The organic layer was dried with anhydrous magnesium sulfate, and the solvent was evaporated under reduced pressure. The residue was purified with silica gel column chromatography (75g, ethyl acetate:ethanol=9:1) and recrystallized from ethanol to give 1-methyl-N-[4-[[N-methyl-N-(tetrahydro-pyran-4-yl)amino ]methyl]phenyl]-[4-(4-morpholino)-phenyl]-2,3-dihydro-1 -benzoazepine-4-carboxamide (Compound 272) (247mg, 52%).
mp 209-211°C.
¹H NMR (200MHz, CDCl₃) δ 1.64-1.77 (4H, m), 2.21 (3H, s), 2.57-2.75 (1H, m), 2.96 (2H, t, J=5.2Hz), 3.09 (3H, s), 3.20 (2H, t, J=4.8Hz), 3.18-3.22 (2H, m), 3.33-3.43 (4H, m), 3.58 (2H, s), 3.89 (4H, t, J=4.8Hz), 4.01-4.06 (2H, m), 6.88 (1H, d, J=8.4Hz), 6.97 (2H, d, J=8.8Hz), 7.30 (2H, d, J=8.8Hz), 7.41-7.56 (8H, m).
IR (KBr) 2953, 2847, 1653, 1607, 1514, 1505, 1311, 1232, 1119, 926, 814, 735cm⁻¹.

| | | | |
|---|---|---|---|
| Anal. Calcd. for C₃₅H₄₂N₄O₅: | C, 74.18; | H, 7.47; | N, 9.89. |
| Found: | C, 74.17; | H, 7.39; | N, 9.98. |

### Reference Example 187

In 1,2-dichloroethane (50ml) were suspended p-nitro-benzylaminehydrochloride (3.77g), 4H-tetrahydropyran-4-one (2g) and triethylamine (2.8ml), and to the mixture was added, under ice-cooling, triacetoxy sodium boron hydride (5.92g). Under nitrogen atmosphere, the mixture was stirred at room temperature for 4 hours, and to the mixture were added, under ice-cooling, acetaldehyde (1.5ml) and triacetoxy sodium boron hydride (5.92g). Under nitrogen atmosphere, the mixture was stirred at room temperature overnight. The solvent was evaporated, and the residue was neutralized with sodium hydroxide solution. The mixture was extracted with ethyl acetate. The organic layer was washed with water and saturated brine and dried with anhydrous magnesium sulfate. Under reduced pressure, the solvent was evaporated, and the residue was purified with silica gel column (ethyl acetate/hexane) to give N-(4-nitrobenzyl)-N-(tetrahydropyran-4-yl)ethylamine (4.0g) as yellow oil.
¹H-NMR (δ ppm, CDCl₃) 1.01 (3H, t, J=6.9Hz), 1.52-1.73 (4H, m), 2.59 (2H, q, J=6.9Hz), 2.68-2.83 (1H, m), 3.34 (2H, dt, J=3.6, 11.2Hz), 3.73 (2H, s), 3.99-4.06 (2H, m), 7.54 (2H, d, J=9.0Hz), 8.16 (2H, d, J=9.0Hz).
IR(neat) ν : 2951, 2841, 1599, 1520cm⁻¹.

### Reference Example 188

In acetic acid (100ml) was dissolved N-(4-nitro-benzyl)-N-(tetrahydropyran-4-yl)ethylamine (4.0g), and to the mixture was added reduced iron (4.2g). The mixture was stirred at room temperature overnight. The solvent was evaporated, and to the residue was added ethyl acetate. The precipitates were filtered off, and the filtrate was washed with sodium hydroxide solution, water and saturated brine, and dried with anhydrous magnesium sulfate. Under reduced pressure, the solvent was evaporated, and the residue was purified with silica gel column (methanol/triethylamine/ethyl acetate) to give 4-(N-ethyl-N-(tetrahydropyran-4-yl)aminomethyl)aniline (2.3g) as red oil.
¹H-NMR(δ ppm, CDCl₃) 1.00 (3H, t, J=7.1Hz), 1.52-1.70 (4H, m), 2.54 (2H, q, J=7.1Hz), 2.66-2.82 (1H, m), 3.26-3.39 (2H, m), 3.52 (2H, s), 3.59 (2H, br), 3.95-4.04 (2H, m), 6.64 (2H, d, J=8.5Hz), 7.12 (2H, d, J=8.5Hz).

### Reference Example 189

In 1,2-dichloroethane (75ml) were suspended p-nitrobenzaldehyde (5g) and 2-amino-1,3-propanediol (3.0g), and to the mixture was added, under ice-cooling, triacetoxy sodium boron hydride (9.8g). Under nitrogen atmosphere, the mixture was stirred at room temperature for 3.5 hours. To the mixture were added, under ice-cooling, 37% formalin (3ml) and triacetoxy sodium boron hydride (9.8g), and the mixture was stirred, under nitrogen atmosphere, at room temperature overnight. To the mixture was added water, and the mixture was concentrated. The residue was neutralized with sodium hydroxide solution, saturated with sodium hydrochloride and extracted with ethyl acetate. The organic layer was dried with anhydrous magnesium sul fate, and the solvent was evaporated under reduced pressure. The residue was purified with silica gel column (ethyl acetate) to give 2-(N-methyl-N-(4-nitro-benzyl)amino)-1,3-propanediol (3.0g) as pale yellow crystals.
mp 65-66°C.
¹H-NMR(δ ppm, CDCl₃) 2.31 (3H, s), 2.93-3.06 (1H, m), 3.64-3.80 (4H, m), 3.92 (2H, s), 7.49 (2H, d, J=8.8Hz), 8.20 (2H, d, J=8.8Hz).
IR(KBr) ν: 3349, 2942, 2884, 1520cm⁻¹.

| | | | |
|---|---|---|---|
| Anal. Calcd. for C₁₁H₁₆N₂O₄: | C, 54.99; | H,6.71; | N,11.66. |
| Found: | C, 55.14; | H,6.61; | N,11.55. |

### Reference Example 190

In ethanol (50ml) was dissolved 2-(N-methyl-N-(4-nitrobenzyl)amino)-1,3-propanediol (2.9g), and catalytic reduction was carried out with 5% palladium carbon (0.15g) at room temperature for 2 hours. The catalyst was filteredoff, and the solvent of the filtrate was evaporated. The residue was purified with silica gel column (methanol/triethylamine/ethyl acetate) to give 2- (N- (4-aminobenzyl) - N-methylamino)-1,3-propanediol (0.6g) as pale yellow amorphous.
¹H-NMR(δppm, CDCl₃) 2.26 (3H, s), 2.37 (2H, br), 2.91-2.99 (1H, m), 3.55-3.73 (6H, m), 6.65 (2H, d, J=8.4Hz), 7.08 (2H, d, J=8.4Hz).
IR(KBr) ν: 3347, 2942, 2880, 1615cm⁻¹.

| Anal. Calcd. for C₁₁H₁₈N₂O₂·0.1H₂O: | | | |
|---|---|---|---|
| | C,62.30; | H,8.65; | N,13.21. |
| Found: | C,62.37; | H, 8.79; | N,13.24. |

### Reference Example 191

In 1,2-dichloroethane (50ml) were suspended p-nitrobenzaldehyde (5g), sarcosine methyl ester hydrochloride (4.6g) and triethylamine (4.6ml), and to the mixture was added, under ice-cooling, triacetoxy sodium boron hydride (9.8g). Under nitrogen atmosphere, the mixture was stirred at room temperature for 4 hours. To the mixture was added water, and the mixture was concentrated, neutralized with sodium hydroxide solution and extracted with ethyl acetate. The organic layer was washed with water and brine, and dried with anhydrous magnesium sulfate. Under reduced pressure, the solvent was evaporated, and the residue was purified with silica gel column (ethyl acetate/hexane) to give N- (4-nitrobenzyl) sarcosine methyl ester (6. 3g) as colorless oil.
¹H-NMR(δ ppm, CDCl₃) 2.39 (3H, m), 3.33 (2H, s), 3.73 (3H, s), 3.80 (2H, s), 7.55 (2H, d, J=8.8Hz), 8.19 (2H, d, J=8.8Hz). IR(neat) ν : 2951, 2847, 1748cm⁻¹.

### Reference Example 192

In acetic acid (100ml) was dissolved N-(4-nitro-benzyl)sarcosine methyl ester (5.96g), and to the mixture was added little by little reduced iron (7g). The mixture was stirred at room temperature overnight. The solvent was evaporated, and to the residue was added ethyl acetate. The precipitates were filtered off, and the filtrate was washed with sodium hydroxide solution, water and saturated brine, and dried with anhydrous magnesium sulfate. Under reduced pressure, the solvent was evaporated, and the resulting residue was purified with silica gel column chromatography (ethyl acetate/hexane) to give N-(4-aminobenzyl)sarcosine methyl ester (3.0g) as red oil.
¹H-NMR(δppm, CDCl₃) 2.36 (3H, m), 3.22 (2H, s), 3.55 (2H, s), 3.65 (2H, br), 3.70 (3H, s), 6.65 (2H, d, J=8.6Hz), 7.11 (2H, d, J=8.6Hz).
IR(neat) ν :3364, 2949, 1744cm⁻¹.

### Reference Example 193

In 1,2-dichloroethane (50ml) were dissolved p-nitrobenzaldehyde (5g) and 3-methoxypropylamine (3.1g), and to the mixture was added, under ice-cooling, triacetoxy sodium boron hydride (9.8g). Under nitrogen atmosphere, the mixture was stirred at room temperature for 3 hours, and to the mixture were added, under ice-cooling, 37% formalin (3ml) and triacetoxy sodium boron hydride (9.8g). Under nitrogen atmosphere, the mixture was stirred at room temperature for 3 hours, and to the mixture was added water. The mixture was concentrated, neutralized with sodium hydroxide solution and extracted with ethyl acetate. The organic layer was washed with water and subjected to back extraction with 1N hydrochloric acid. The aqueous layer was washed with ethyl acetate, neutralized with 1N sodium hydroxide solution and extracted with ethyl acetate. The organic layer was washed with water and saturated brine, and dried with anhydrous magnesium sulfate. Under reduced pressure, the solvent was evaporated to give N- (3-methoxypropyl)-N-methyl-4-nitrobenzylamine (5.6g) as yellow oil. ¹H-NMR(δppm, CDCl₃) 1.72-1.85 (2H, m), 2.20 (3H, s), 2.47 (2H, t, J=7.3Hz), 3.33 (3H, s), 3.43 (2H, t, J=6.4Hz), 3.58 (2H, s), 7.50 (2H, d, J=9.0Hz), 8.18 (2H, d, J=9.0Hz).
IR(neat) ν : 2805, 1605, 1520cm⁻¹.

### Reference Example 194

In acetic acid (70ml) was dissolved N-(3-methoxy-propyl)-N-methyl-4-nitrobenzylamine (5.5g), and to the mixture was added little by little reduced iron (6.4g). The mixture was stirred at room temperature overnight. The solvent was evaporated, and to the residue was added ethyl acetate. The precipitates were filtered off , the filtrate was washed with sodium hydroxide solution, water and saturated brine, and dried with anhydrous magnesium sulfate. Under reduced pressure, the solvent was evaporated to give 4-((N-3-methoxypropyl-N-methyl)amino-methyl)aniline (4.4g) as red oil.
¹H-NMR (δ ppm, CDCl₃) 1.71-1.85 (2H, m), 2.16 (3H, s), 2.42 (2H, t, J=7.4Hz), 3.32 (3H, s), 3.37 (2H, s), 3.41 (2H, t, J=6.6Hz), 3.61 (2H, br), 6.64 (2H, d, J=8.4Hz), 7.08 (2H, d, J=8.4Hz).
IR(neat) ν : 2946, 2795, 1615cm⁻¹.

### Reference Example 195

In ethanol (50ml) was dissolved 7-(4-methylphenyl)-2,3,4,5-tetrahydro-1-benzoxepin-5-one (1g), and to the mixture was added, under ice-cooling, sodium boron hydride (0.3g). The mixture was stirred at room temperature for 30 minutes, and to the mixture was added water. The mixture was concentrated and extracted with ethyl acetate. The organic layer was washed with water and concentrated. The residue was dissolved in bis(2-methoxyethyl)ether (20ml), and to the mixture was added hydrochloric acid (5ml). The mixture was stirred at 75°C for 1 hour, poured into water and extracted with ethyl acetate. The organic layer was washed with water and saturated brine, and dried with anhydrous magnesium sulfate. The solvent was evaporated, and the precipitated 7-(4-methylphenyl)-2,3-dihydro-1-benzoxepine (0.78g) was filtered with hexane to give colorless crystals.
mp 98-100°C.
¹H-NMR(δppm, CDCl₃) 2.38 (3H, s), 2.65-2.74 (2H, m), 4.27 (2H, t, J=4.9Hz), 6.01 (1H, dt, J=11.7, 4.4Hz), 6.39 (1H, d, J=11.7Hz), 7.01 (1H, d, J=8.0Hz), 7.23 (2H, d, J=8.2Hz), 7.31-7.38 (2H, m), 7.45 (2H, d, J=8.0Hz).
IR(KBr) ν : 3025, 1491cm⁻¹.

| | | |
|---|---|---|
| Anal. Calcd. for C₁₇H₁₆O: | C, 86.41; | H, 6.82. |
| Found: | C,86.17; | H,6.61. |

### Reference Example 196

Under ice-cooling, to dimethylformamide (0.2ml) was added dropwise sulfuryl chloride (0.17ml), and the mixture was stirred, under nitrogen atmosphere, at room temperature for 10 minutes. To the mixture was added 7-(4-methyl-phenyl)-2,3-dihydro-1-benzoxepine (0.3g), and the mixture was stirred, under nitrogen atmosphere, at 90°C for 3 hours. To the mixture was added ice-water, and the mixture was extracted with ethyl acetate. The organic layer was washed with water and saturated brine, and dried with anhydrous magnesium sulfate. The solvent was evaporated to give 7-(4-methylphenyl)-2,3-dihydro-1-benzoxepine-4-sulfonyl chloride (0.36g) as pale yellow crystals.
mp 162-166°C.
¹H-NMR(δ ppm, CDCl₃) 2.40 (3H, s), 3.27 (2H, t, J=4.7Hz), 4.41 (2H, t, J=4.7Hz), 7.11 (1H, d, J=9.6Hz), 7.26 (2H, d, J=8.2Hz), 7.44 (2H, d, J=8.2Hz), 7.57-7.62 (2H, m), 7.70 (1H, s).
IR(KBr) ν: 3027, 1634, 1493cm⁻¹.

| | | |
|---|---|---|
| Anal. Calcd. for C₁₇H₁₅ClO₃S: | C, 60.98; | H,4.52. |
| Found: | C,61.14; | H,4.26. |

### Reference Example 197

Under argon atmosphere, a solution of ethyl (E)-3-(5-bromothiophen-2-yl)acrylate (1.00g), 4-isopropylphenyl borate (0.86g) and potassium carbonate (1.12g) in toluene/ethanol/water (40/4/4ml) was stirred at room temperature for 1 hour. To the mixture was added tetrakistriphenylphosphine palladium (0.14g), and the mixture was refluxed for 18 hours and then cooled to room temperature. The organic layer was washed with saturated brine and dried with magnesium sulfate. Under reduced pressure, the mixture was concentrated, and the residue was purified with column chromatography (ethyl acetate/hexane= 1:9) to give pale yellow crystals of methyl (E)-3-[5-(4-isopropylphenyl)-thiophen-2-yl]acrylate (0.83g) .
m.p. 117-119°C
¹H-NMR (200MHz, CDCl₃) δ 1.27 (6H, d, J=6.8 Hz), 2.94-3.00 (1H, m), 3.80 (3H, s), 6.22 (1H, d, J=15.8 Hz), 7.24-7.28 (4H, m), 7.54 (2H, d, J=7.8 Hz), 7.76 (1H, d, J=15.8 Hz).
IR (KBr) 1718, 1622, 1436, 1306, 1230, 1203, 1165, 806 cm⁻¹

| Anal. Calcd. for C₁₇H₁₈O₂S | | | |
|---|---|---|---|
| Calcd. | C, 71.30 ; | H, 6.33 ; | S, 11.20. |
| Found. | C, 71.22 ; | H, 6.33 ; | S, 11.23. |

### Reference Example 198

To a solution of methyl (E) -3- [5- (4-isopropylphenyl)-thiophen-2-yl]acrylate (0.75mg) in THF/ethanol (10/10ml) was added at room temperature 2N sodium hydroxide solution (2.0ml), and the mixture was stirred for 20 hours. Under reduced pressure, the mixture was concentrated, and to the residue was added 1N hydrochloric acid (10ml). The mixture was extracted with ethyl acetate, and the organic layer was washed with saturated brine, dried with magnesium sulfate and concentrated. The resulting crystals were collected by filtration to give pale yellow crystals of (E)-3-[5-(4-isopropylphenyl)thiophen-2-yl]acrylic acid (639.7mg).
m.p. 216-219°C
¹H-NMR (200MHz, CDCl₃) δ 1.28 (6H, d, J=7.0 Hz), 2.86-3.01 (1H, m), 6.22 (1H, d, J=15.7 Hz), 7.23-7.33 (4H, m), 7.56 (2H, d, J=8.4 Hz), 7.85 (1H, d, J=15.7 Hz).
IR (KBr) 2966, 1668, 1608, 1414, 1302, 1263, 1228, 804 cm⁻¹

| Anal. Calcd. for C₁₆H₁₆O₂S | | | |
|---|---|---|---|
| Calcd. | C, 70.56; | H, 5.92; | S, 11.77. |
| Found. | C, 70.23; | H, 5.94 ; | S, 11.62. |

### Reference Example 199

Under argon atmosphere, a solution of methyl (E)-3-(5-bromothiophen-2-yl)acrylate (0.23g), 4-tert-butyl-phenyl borate (0.3g) and potassium carbonate (0.26g) in toluene/ethanol/water (20/2/2ml) was stirred at room temperature for 1 hour. To the mixture was added tetrakistriphenylphosphine palladium (32mg), and the mixture was refluxed for 18 hours and then cooled to room temperature. To the organic layer was added ethyl acetate, and the mixture was washed with saturated brine and dried with magnesium sulfate. Under reduced pressure, the mixture was concentrated, and the residue was purified with column chromatography (ethyl acetate/hexane=1:9) to give pale yellow crystals of methyl (E)-3-[5-(4-tert-butyl-phenyl)thiophen-2-yl]acrylate (240mg). This compound was used for the following reaction, without subjecting further purification.
¹H-NMR (200MHz, CDCl₃) δ 1.34 (9H, s), 3.80 (3H, s), 6.22 (1H, d, J=15.8 Hz), 7.21-7.30 (2H, m), 7.42 (2H, d, J=8.7 Hz), 7.55 (2H, d, J=8.7 Hz), 7.76 (1H, d, J=15.8 Hz).
IR (KBr) 1716, 1622, 1436, 1302, 1232, 1207, 1165, 972, 806 cm⁻¹

### Reference Example 200

To a solution of methyl (E)-3-[5-(4-tert-butyl-phenyl)-thiophen-2-yl]acrylate (190mg) of THF/ethanol (15/15ml) was added at room temperature 2N sodium hydroxide solution (2.0ml), and the mixture was stirred 18 hours. To the mixture was added 1N hydrochloric acid (5ml), and the mixture was extracted with ethyl acetate. The organic layer was washed with saturated brine and dried with magnesium sulfate. Under reduced pressure, the mixture was concentrated, and the precipitated crystals were collected by filtration, which were washed with hexane to give yellow crystals of (E)-3-[5-(4-tert-butylphenyl)thiophen-2-yl]acrylic acid (149.7mg). This compound was used for the following reaction, without subjecting further purification.
¹H-NMR (200MHz, CDCl) δ 1.35 (9H, s), 6.22 (1H, d, J=15.6 Hz), 7.20-7.29 (2H, m), 7.43 (2H, d, J=8.8 Hz), 7.56 (2H, d, J=8.8 Hz), 7.85 (1H, d, J=15.6 Hz).
IR (KBr) 2962, 1678, 1612, 1414, 1302, 1232, 806 cm⁻¹

### Reference Example 201

To a solution of 4'-methylacetophenone (10.0g) in ethanol (100ml) were added at room temperature an aqueous solution (50ml) of hydroxyamine hydrochloride (7.77g) and sodium acetate (9.63g), and the mixture was refluxed for 24 hours and then cooled. The mixture was concentrated, and to the residue was added 1N hydrochloric acid (150ml). The mixture was extracted with ethyl acetate, washed with saturated brine and dried with magnesium sulfate. Under reduced pressure, the mixture was concentrated, and the residue was purified with column chromatography (ethyl acetate/hexane=1:3) to give colorless crystals of 4'-methylacetophenonoxime (10.89g).
¹H-NMR (200MHz, CDCl₃) δ 2.28 (3H, s), 2.37 (3H, s), 7.19 (2H, d, J=8.1 Hz), 7.53 (2H, d, J=8.1 Hz), 8.55-8.69 (1H, m) .

### Reference Example 202

To a solution of 4'-methylacetophenonoxime (10.46g) in DMF (250ml) was added at 0°C sodium hydride (60%, 3.08g), and the mixture was stirred at room temperature for 1 hour. To the mixture was added a solution of 4-fluorobenzaldehyde (9.57g) in THF (300ml), and the mixture was stirred for 5 days. To the mixture was added 1N hydrochloric acid (200ml), and the mixture was extracted with ethyl acetate. The organic layer was washed with saturated brine and dried with magnesium sulfate. Under reduced pressure, the mixture was concentrated, and the residue was purified with column chromatography (ethyl acetate/hexane=1:5) to give colorless crystals of 4-(4'-methyl-α-methylbenzylidene-aminoxy)benzaldehyde (11.23g).
m.p. 96-98 °C
¹H-NMR (200MHz, CDCl₃) δ 2.41 (3H, s), 2.47 (3H, s), 7.25 (2H, d, J=7.8 Hz), 7.43 (2H, d, J=8.8 Hz), 7.69 (2H, d, J=7.8 Hz), 7.88 (2H, d, J=8.8 Hz), 9.93 (1H, s).
IR (KBr) 1699, 1597, 1576, 1498, 1232, 1207, 1149, 916, 820 cm⁻¹

| Anal. Calcd. for C₁₆H₁₅NO₂ | | | |
|---|---|---|---|
| Calcd. | C, 75.87 ; | H, 5.97 ; | N, 5.53. |
| Found. | C, 75.73 ; | H, 6.04 ; | N, 5.48. |

### Reference Example 203

A solution of 4-(4'-methyl-α -methylbenzylidene-aminoxy)benzaldehyde (5.0g) in 1N hydrochloric acid/acetic acid (80ml) was stirred at 100-110°C for 24 hours and then cooled to room temperature. To the mixture was added water, and the mixture was extracted with ethyl acetate. The organic layer was washed with saturated brine and dried with magnesium sulfate. Under reduced pressure, the mixture was concentrated, and the residue was purified with column chromatography (ethyl acetate/hexane=1:9) to give colorless crystals of 2-(4-methylphenyl)benzofuran-5-aldehyde (1.50g).
m.p. 162-164 °C
¹H-NMR (200MHz, CDCl₃) δ 2.41 (3H, s), 7.06 (1H, s), 7.28 (2H, d, J=8.0 Hz), 7.62 (1H, d, J=8.4 Hz), 7.77 (2H, d, J=8.0 Hz), 7.84 (1H, dd, J=8.4, 1.8 Hz), 8.11 (1H, d, J=1.8 Hz), 10.06 (1H, s).
IR (KBr) 1697, 1292, 1271, 824, 798 cm⁻¹

| Anal. Calcd. For C₁₆H₁₂O₂ | | |
|---|---|---|
| Calcd. | C, 81.34 ; | H, 5.12. |
| Found. | C, 81.21 ; | H, 5.11. |

### Reference Example 204

To a solution of 2-(4-methylphenyl)benzofuran-5-carbaldehyde (500mg) and 1-methylcyclohexene (1.2ml) in DMF (15ml) was added a solution (9ml) of sodium chlorite (80%, 1.5g) and sodium dihydrogenphosphate (1.5g) at room temperature, and the mixture was stirred for 3 hours. To the mixture was added 1N hydrochloric acid, and the mixture was extracted with ethyl acetate. The organic layer was washed with sodium thiosulfate and saturated brine, and dried with magnesium sulfate. Under reduced pressure, the mixture was concentrated, and the precipitated crystals were collected by filtration, which were washed with diethylether to give colorless crystals of 2-(4-methylphenyl)benzofuran-5-carboxylic acid (395mg).
m.p. 279-283 °C
¹H-NMR (200MHz, CDCl₃) δ 2.38 (3H, s), 7.34 (2H, d, J=8.2 Hz), 7.48 (1H, s), 7.70 (1H, d, J=8.8 Hz), 7.84 (2H, d, J=8.2 Hz), 7.92 (1H, dd, J=8.8, 1.2 Hz), 8.26 (1H, d, J=1.2 Hz).
IR (KBr) 2989, 1689, 1416, 1291, 768 cm⁻¹

| Anal. Calcd. for C₁₆H₁₂O₃ | | |
|---|---|---|
| Calcd. | C, 76.18 ; | H, 4.79. |
| Found. | C, 76.11 ; | H, 4.74. |

### Reference Example 205

To a solution of ethyl vanillate (2.50g) and triethylamine (3.6ml) in dichloromethane (50ml) was added at 0°C trifluoromethanesulfonic acid anhydride (2.6ml), and the mixture was stirred for 1.5 hours. To the mixture was added water (15ml), and the mixture was extracted with ethyl acetate. The organic layer was washed with saturated brine and dried with magnesium sulfate. Under reduced pressure, the mixture was concentrated, and the residue was purified with column chromatography (ethyl acetate/hexane=1:15) to give yellow oil of ethyl 3-methoxy-4-trifluoromethane-sulfonyloxybenzoate (3.96g).
¹H-NMR (200MHz, CDCl₃) δ 1.41 (3H, t, J=7.1 Hz), 3.99 (3H, s), 4.41 (2H, q, J=7.1 Hz), 7.28 (1H, d, J=7.6 Hz), 7.67-7.72 (2H, m).
IR (neat) 1726, 1606, 1502, 1466, 1427, 1292, 1246, 1207, 1142, 1109, 1030, 833, 768, 617 cm⁻¹

### Reference Example 206

To a solution of ethyl 3-methoxy-4-trifluoromethane-sulfonyloxybenzoate (3.95g), 4-methylphenylacetylene (1.54g) and triethylamine (5.0ml) in DMF (40ml) was added bistriphenylphosphine palladium dichloride (0.25g), and the mixture was stirred at 100°C for 3 hours and then cooled to room temperature. To the mixture was added water, and the mixture was extracted with diethylether. The organic layer was washed with saturated brine and dried with magnesium sulfate. Under reduced pressure, the mixture was concentrated, and the residue was purified with column chromatography (ethyl acetate/hexane=1:9) and recrystallized from ethyl acetate/hexane to give pale yellow crystals of ethyl 3-methoxy-4-[2-(4-methylphenyl)-ethynyl]-benzoate (2.02g).
m.p. 71-73 °C
¹H-NMR (200MHz, CDCl₃) δ 1.41 (3H, t, J=7.1 Hz), 2.37 (3H, s), 3.97 (3H, s), 4.39 (2H, q, J=7.1 Hz), 7.16 (2H, d, J=7.9 Hz), 7.47 (2H, d, J=7.9 Hz), 7.53 (1H, d, J=8.0 Hz), 7.57 (1H, d, J=1.6 Hz), 7.63 (1H, dd, J=8.0, 1.6 Hz).
IR (KBr) 1711, 1410, 1294, 1236, 1099, 1036, 812, 762 cm⁻¹

| Anal. Calcd. for C₁₉H₁₈O₃ | | |
|---|---|---|
| Calcd. | C, 77.53 ; | H, 6.16. |
| Found. | C, 77.48 ; | H, 6.01. |

### Reference Example 207

A mixture of ethyl 3-methoxy-4-(4-methylphenyl)-ethynylbenzoate (1.5g) and pyridinium chloride (9.0g) was stirred at 200°C for 2 hours, and then cooled to 100°C . To the mixture was added DMF (20ml), and the mixture was cooled to room temperature. To the mixture was added 1N hydrochloric acid, and the mixture was extracted with ethyl acetate. The organic layer was washed with saturated brine and dried with magnesium sulfate . Under reduced pressure, the mixture was concentrated, and the precipitated crystals were collected by filtration, which were washed with diethylether and hexane to give pale yellow crystals of 2-(4-methylphenyl)benzofuran-6-carboxylic acid (0.84g).
m.p. 270-272 °C
¹H-NMR (200MHz, DMSO-d₆) δ 2.38 (3H, s), 7.35 (2H, d, J=8.2 Hz), 7.47 (1H, s), 7.72 (1H, d, J=8.0 Hz), 7.85-7.89 (3H, m), 8.11 (1H, s).
IR (KBr) 2972, 1677, 1612, 1498, 1413, 1300, 1230, 798 cm⁻¹

| Anal. Calcd. For C₁₆H₁₂O₃ | | |
|---|---|---|
| Calcd. | C, 76.18 ; | H, 4.79. |
| Found. | C, 76.05 ; | H, 4.54. |

### Reference Example 208

To a solution of ethyl 7-(4-methylthiophenyl)-2,3-dihydro-1-benzoxepine-4-carb oxylate (198.5mg) in THF (20ml) was added at 0°C 70% 3-chloroperbenzoic acid (317mg), and the mixture was stirred at 0°C for 30 minutes and then at room temperature for 1 hour. To the mixture was added sodium thiosul fate solution, and the mixture was stirred for a f ewminutes and then extracted with ethyl acetate. The organic layer was washed with saturated sodium bicarbonate solution and saturated brine, and dried with magnesium sulfate. Under reduced pressure, the mixture was concentrated, and the residue was purified with column chromatography (ethyl acetate/hexane=1:1) to give colorless crystals of ethyl 7-(4-methylsulfonylphenyl)-2,3-dihydro-1-benzoxepine-4-carboxylate (221.8mg).
m.p. 150-153 °C
¹H-NMR (200MHz, CDCl₃) δ 1.37 (3H, t, J=7.2 Hz), 3.03 (2H, t, J=4.5 Hz), 3.10 (3H, s), 4.30 (2H, q, J=7.2 Hz), 4.33 (2H, t, J=4.5 Hz), 7.10 (1H, d, J=8.4 Hz), 7.50 (1H, dd, J=8.4, 2.2 Hz), 7.60 (1H, d, J=2.2 Hz), 7.65 (1H, s), 7.75 (2H, d, J=8.4 Hz), 8.01 (2H, d, J=8.4 Hz).
IR (KBr) 1693, 1595, 1485, 1302, 1252, 1230, 1213, 1146, 1092, 825 cm⁻¹

| Anal. Calcd. for C₂₀H₂₀O₅S | | | |
|---|---|---|---|
| Calcd. | C, 64.50 ; | H, 5.41 ; | S, 8.61. |
| Found. | C, 64.36 ; | H, 5.40 ; | S, 8.53. |

### Reference Example 209

To a solution of ethyl 7-(4-methylsulfonylphenyl)-2,3-dihydro-1-benzoxepine-4-carboxylate (180mg) in THF/ethanol (5/5ml) was added at room temperature 1N sodium hydroxide solution (1ml), and the mixture was stirred for 4 days. To the mixture was added 1N hydrochloric acid (10ml), and the mixture was concentrated under reduced pressure. The residue was extracted with ethyl acetate. Under reduced pressure, the mixture was concentrated. The resulting crystals were collected by filtration, which were washed with water, ethanol and diethylether to give colorless crystals of 7-(4-methyl-sulfonylphenyl)-2,3-dihydrobenzoxepine-4-ca rboxylic acid (148.2mg).
m.p. 275 °C (dec.)
¹H-NMR (200MHz, DMSO-d₆) δ 2.84-2.94 (2H, m), 3.25 (3H, s), 4.23-4.34 (2H, m), 7.10 (1H, d, J=8.4 Hz), 7.64-7.75 (2H, m), 7.92-8.04 (5H, m).
IR (KBr) 3018, 1674, 1308, 1267, 1147, 829, 783, 760, 636, 546cm⁻¹

| Anal. Calcd. for C₁₈H₁₆O₅S·0.2H₂O | | | |
|---|---|---|---|
| Calcd. | C, 62.13 ; | H, 4.75 ; | S, 9.21. |
| Found. | C, 62.19 ; | H, 4.69 ; | S, 9.06. |

### Reference Example 210

A mixture of 4-bromothiophenol (24,8g), ethyl 4-bromo-butyrate (30.7g) and potassium carbonate (36.2g) in DMF (100ml) was stirred at room temperature overnight. Under reduced pressure, the solvent was evaporated, and to the residue was added water. The mixture was extracted with ethyl acetate, and the organic layer was washed with saturated brine and dried with magnesium sulfate. Under reduced pressure, the mixture was concentrated, and to the residue was were added methanol (120ml) and 1N sodium hydroxide solution (240ml). The mixture was stirred at room temperature overnight, and to the mixture was added water. The mixture was washed with ethyl acetate, and to the aqueous layer was added concentrated hydrochloric acid to make the solution acidic. The mixture was extracted with ethyl acetate. The organic layer was washed with saturated brine and dried with magnesium sulfate. Under reduced pressure, the solvent was evaporated to colorless prism of 4-(4-bromophenylthio)butyric acid (31.8g).
¹H-NMR (200MHz, CDCl₃) δ 1.87-2.02 (2H, m), 2.53 (2H, t, J=7.1 Hz), 2.96 (2H, t, J=7.2 Hz), 7.21 (2H, d, J=8.8 Hz), 7.41 (2H, d, J=8.8 Hz).
IR (KBr) 1699 cm⁻¹

| Anal. Calcd. for C₁₀H₁₁O₂BrS | | |
|---|---|---|
| Calcd. | C, 43.65 ; | H, 4.03. |
| Found. | C, 43.70 ; | H, 3.93. |

### Reference Example 211

A mixture of 4-(4-bromophenylthio)butyric acid(31.8g) and polyphosphoric acid (250g) was stirred at 100°Cfor 1 hour. The mixture was added to ice/water and extracted with ethyl acetate. The organic layer was washed with saturated brine and dried with magnesium sulfate. Under reduced pressure, the solvent was evaporated to give brown prism of 7-bromo-2,3,4,5-tetrahydro-1-benzo-thiepin-5-one (13.6g).
¹H-NMR (200MHz, CDCl₃) δ 2.22-2.35 (2H, m), 2.94-3.08 (4H, m), 7.33 (1H, d, J=8.0 Hz), 7.44 (1H, dd, J=8.0, 2.6 Hz), 7.96 (1H, d, J=2.6 Hz).
IR (KBr) 1682 cm⁻¹

| Anal. Calcd. for C₁₀H₉OBrS | | |
|---|---|---|
| Calcd. | C, 46.71 ; | H, 3.53. |
| Found. | C, 46.71 ; | H, 3.45. |

### Reference Example 212

To a solution of 7-bromo-2,3,4,5-tetrahydro-1-benzothiepin-5-one (13.5g) in dimethyl carbonate (200ml) was added at room temperature sodium methoxide (14.2g), and the mixture was refluxed for 8 hours under nitrogen atmosphere. To the mixture was added 1N hydrochloric acid, and the mixture was extracted with ethyl acetate. The organic layer was washed with water and saturated brine, and dried with magnesium sulfate. Under reduced pressure, the solvent was evaporated to give brown prism of methyl 7-bromo-5-oxo-2,3,4,5-tetrahydro-1-benzothiepine-4-carb oxylate (11.5g) .
¹H-NMR (200MHz, CDCl₃) δ 2.40-2.84 (6H, m), 3.16-3.27 (2H, m), 3.75 (3H, s), 4.47-4.56 (1H, m), 7.33 (1H, d, J=8.4 Hz), 7.47 (1H, dd, J=8.4, 2.6 Hz), 7.99 (1H, d, J=2.6 Hz).
IR (KBr) 1750 cm⁻¹

| Anal. Calcd. for C₁₂H₁₁O₃BrS | | |
|---|---|---|
| Calcd. | C, 45.73 ; | H, 3.52. |
| Found. | C, 46.01 ; | H, 3.48. |

### Reference Example 213

A solution of methyl 7-bromo-5-oxo-2,3,4,5-tetrahydro-1-benzothiepine-4-carb oxylate (24.94g) in THF (200ml) was cooled to -20°C, and to the mixture was added dropwise a solution of sodium boro hydride (2.99g) in methanol (30ml). While the temperature of the mixture was kept at -15 to 20°C, the mixture was stirred for 1 hour. To the mixture was added water, and the mixture was extracted with ethyl acetate. The organic layer was washed with saturated brine and dried with magnesium sulfate. Under reduced pressure, the solvent was evaporated, and the residue (24.38g) was dissolved in THF (200ml). To the mixture was added triethylamine (26ml) and then to the mixture was added dropwise at 0°C methanesulfonyl chloride (9.2ml). The mixture was stirred at 0°C for 30 minutes and then at room temperature for 15 hours. To the mixture was added dropwise
1,8-diaza-bicyclo[5,4,0]-7-undecene (17.9g), and the mixture was stirred for 3 hours. To the mixture was added water, and the mixture was extracted with ethyl acetate. The organic layer was washed with water and saturated brine and dried with magnesium sulfate. Under reduced pressure, the mixture was concentrated, and the residue was purified with column chromatography (ethyl acetate/ hexane=1:10).
Under reduced pressure, the mixture was concentrated, and the resulting crystals were recrystallized from ethyl acetate/hexane to give pale yellow crystals of methyl 7-bromo-2,3-dihydro-1-benzothiepine-4-carboxylate (11.00g).
m.p. 94-95 °C
¹H-NMR (200MHz, CDCl₃) δ 2.94-3.00 (2H, m), 3.15-3.21 (2H, m), 3.83 (3H, s), 7.28-7.33 (2H, m), 7.51 (1H, d, J=1.2 Hz), 7.70 (1H, s).

| Anal. Calcd. for C₁₂H₁₁O₂BrS | | |
|---|---|---|
| Calcd. | C, 48.17 ; | H, 3.71. |
| Found. | C, 48.37 ; | H, 3.77. |

### Reference Example 214

Under argon atmosphere, a mixture of methyl 7-bromo-2,3-dihydro-1-benzothiepine-4-carboxylate (1.5g), 4-methoxyphenyl borate (0.84g) and potassium carbonate (1.39g) in toluene/ethanol/water (50/5/5ml) was stirred at room temperature for 1 hour. To the mixture was added tetrakistriphenylphosphine palladium (0.17g), and the mixture was refluxed for 24 hours and then cooled. The mixture was extracted with ethyl acetate, washed with saturated brine and dried with magnesium sulfate. Under reduced pressure, the mixture was concentrated, and the residue was purified with column chromatography (ethyl acetate/hexane=1:15→1:9→1:4→1:2) to give pale yellow crystals of methyl 7-(4-methoxyphenyl)-2,3-dihydro-1-benzothiepine-4-carbo xylate (1.40g).
m.p. 117-120 °C
¹H-NMR (200MHz, CDCl₃) δ 2.97-3.04 (2H, m), 3. 19-3.25 (2H, m), 3.84 (3H, s), 3.86 (3H, s), 6.98 (2H, d, J=8.8 Hz), 7.39 (1H, dd, J=8.0, 2.2 Hz), 7.48-7.54 (3H, m), 7.57 (1H, d, J=2.2 Hz), 7.88 (1H, br s).
IR (KBr) 1716, 1630, 1606, 1520, 1479, 1431, 1281, 1250, 1221, 1186, 1020, 835, 814 cm⁻¹

| Anal. Calcd. for C₁₉H₁₈O₃S | | |
|---|---|---|
| Calcd. | C, 69.91 ; | H, 5.56. |
| Found. | C, 70.22 ; | H, 5.65. |

### Reference Example 215

To a solution of methyl 7-(4-methoxyphenyl)-2,3-dihydro-1-benzothiepine-4-carbo xylate (0.50g) in ethanol/THF (10/10ml) was added at room temperature 1N sodium hydroxide solution (2ml), and the mixture was stirred for 18 hours. To the mixture was added 1N hydrochloric acid (2ml). Under reduced pressure, the mixture was concentrated. To the mixture was added water, and the precipitates were collected by filtration, which were washed with 2-propanol, diethylether and hexane to give pale yellow solid of 7-(4-methoxyphenyl)-2,3-dihydro-1-benzo-thiepine-4-carb oxylicacid (508mg). This compoundwas used for the following reaction, without subjecting further purification.
¹H-NMR (200MHz, DMSO-d₆) δ 2.87 (2H, t, J=5.7 Hz), 3.11 (2H, t, J=5.7Hz), 3.80 (3H, s), 7.01 (2H, d, J=8.8 Hz), 7.33-7.42 (2H, m), 7.50-7.55 (2H, m), 7.62 (2H, d, J=8.8 Hz).
IR (KBr) 3356, 1633, 1608, 1518, 1358, 1246, 1178, 1020, 825 cm⁻¹

### Reference Example 216

Under argon atmosphere, a mixture of methyl 7-bromo-2,3-dihydro-1-benzothiepine-4-carboxylate (0.70g), 4-morpholinophenyl borate (581.3mg) and potassium carbonate (0.65g) in toluene/ethanol/water (20/2/2ml) was stirred at room temperature for 1 hour. To the mixture was added tetrakistriphenylphosphine palladium (0.14g), and the mixture was refluxed for 20 hours and then cooled. The mixture was extracted with ethyl acetate, washed with saturated brine and dried with magnesium sulfate. Under reduced pressure, the mixture was concentrated, and the residue was purified with column chromatography (ethyl acetate/dichloromethane=1:4) to give yellow crystals of methyl 7-(4-morpholinophenyl)-2,3-dihydro-1-benzo-thiepine-4-c arboxylate (664.4mg).
m.p. 154-156 °C
¹H-NMR (200MHz, CDCl₃) δ 2.97-3.02 (2H, m), 3.20-3.25 (6H, m), 3.84 (3H, s), 3.87-3.91 (4H, m), 6.98 (2H, d, J=8.8 Hz), 7.35-7.43 (1H, m), 7.49-7.58 (4H, m), 7.88 (1H, s).
IR (KBr) 1709, 1606, 1520, 1448, 1274, 1242, 1232, 120, 926, 816 cm⁻¹

| Anal. Calcd. for C₂₂H₂₃NO₃S | | | |
|---|---|---|---|
| Calcd. | C, 69.26 ; | H, 6.08 ; | N, 3.67. |
| Found. | C, 69.43 ; | H, 6.01 ; | N, 3.81. |

### Reference Example 217

To a solution of methyl 7-(4-morpholinophenyl)-2,3-dihydro-1-benzothiepine-4-ca rboxylate (0.55g) in ethanol/THF (30/30ml) was added at room temperature 1N sodium hydroxide solution (1.8ml), and the mixture was stirred for 6 days and then refluxed for 2 hours. To the mixture was added 1N hydrochloric acid (1.8ml). The resulting solid was collected by filtration, which was washed with ethanol and diethylether to give yellow powder of 7-(4-morpholinophenyl)-2,3-dihydro-1-benzo-thiepine-4-c arboxylic acid (502.2mg).
m.p. 280 °C (dec.)
¹H-NMR (200MHz, DMSO-d₆) δ 2.88 (2H, t, J=5.3 Hz), 3.05-3.25 (6H, m), 3.67-3.82 (4H, m), 7.02 (2H, d, J=8.7Hz), 7.43-7.54 (2H, m), 7.61 (2H, d, J=8.7 Hz), 7.75 (1H, s), 7.81 (1H, s).
IR (KBr) 2967, 1709, 1684, 1608, 1520, 1232, 1120, 926, 814 cm⁻¹

| Anal. Calcd. for C₂₁H₂₁NO₃S | | | |
|---|---|---|---|
| Calcd. | C, 68.64 ; | H, 5.76 ; | N, 3.81. |
| Found. | C, 68.68 ; | H, 5.62 ; | N, 3.69. |

### Reference Example 218

Under argon atmosphere, a mixture of methyl 7-bromo-2,3-dihydro-1-benzothiepine-4-carboxylate (1.5g), 3,4-methylenedioxyphenyl borate (0.92g) and potassium carbonate (1.39g) in toluene/ethanol/water (50/5/5ml) was stirred at room temperature1 hours. To the mixture was added tetrakistriphenylphosphine palladium (0.29g), and the mixture was refluxed for 16 hours and cooled. The mixture was extracted with ethyl acetate, washed with saturated brine and dried with magnesium sulfate. Under reduced pressure, the mixture was concentrated, and the residue was purified with column chromatography (ethyl acetate/hexane=1:2) to give pale yellow crystals of methyl 7-(3,4-methylenedioxyphenyl)-2,3-dihydro-1-benzo-thiepi ne-4-carboxylate (1.55g).
m.p. 126-129 °C
¹H-NMR (200MHz, CDCl₃) δ 2.97-3.06 (2H, m), 3. 19-3. 24 (2H, m), 3.84 (3H, s), 6.01 (2H, s), 6.88 (1H, d, J=8.8 Hz), 7.02-7.08 (2H, m), 7.35 (1H, dd, J=8.0, 1.8 Hz), 7.50 (1H, d, J=8.4 Hz), 7.53 (1H, d, J=1.8 Hz), 7.87 (1H, br s).
IR (KBr) 1709, 1471, 1435, 1248, 1223, 1186, 1034, 928, 804 cm⁻¹

| Anal. Calcd. for C₁₉H₁₆O₄S | | |
|---|---|---|
| Calcd. | C, 67.04 ; | H, 4.74. |
| Found. | C, 67.19 ; | H, 4.61. |

### Reference Example 219

To a solution of methyl 7-(3,4-methylenedioxyphenyl)-2,3-dihydro-1-benzothiepin e-4-carboxylate (0.6g) in ethanol/ THF (10/10ml) was added at room temperature 1N sodium hydroxide solution (2ml), and the mixture was stirred for 64 hours. To the mixture was added 1N hydrochloric acid (3ml), and the mixture was concentrated. The resulting solid was collected by filtration, which was washed with water, 2-propanol and diisopropylether to give pale yellow powder of 7-(3,4-methylenedioxyphenyl)-2,3-dihydro-1-benzothiepin e-4-carboxylic acid (510.6mg).
m.p. 227-229 °C
¹H-NMR (200MHz, DMSO-d₆) δ 2.86-2.92 (2H, m), 3.14-3.20 (2H, m), 6.07 (2H, s), 6.99 (1H, d, J=8.2 Hz), 7.21 (1H, dd, J=8.2, 1.8 Hz), 7.33 (1H, d, J=1.8 Hz), 7.44-7.53 (2H, m), 7.77-7.82 (2H, m).
IR (KBr) 2895, 1672, 1473, 1288, 1252, 1225, 1039, 933, 806 cm⁻¹

| Anal. Calcd. for C₁₈H₁₄O₄S | | |
|---|---|---|
| Calcd. | C, 66.24 ; | H, 4.32. |
| Found. | C, 66.01 ; | H, 4.44. |

### Reference Example 220

To a suspension of 4-phenylpiperidine (5.0g) in acetonitrile (100ml) was added triethylamine (8.64ml) and then was added dropwise at 0°C a solution of p-toluene-sulfonyl chloride (6.50g) in acetonitrile (30ml) . The mixture was stirred at 0°C for 2 hours. Under reduced pressure, the solvent was evaporated, and to the residue was water. The mixture was extracted with ethyl acetate, and the organic layer was washed with saturated brine and dried with magnesium sulfate. Under reduced pressure, the mixture was concentrated, and the resulting crystals were collected by filtration, which were washed with hexane to give colorless crystals of 1-(4-methylphenylsulfonyl)-4-phenylpiperidine (8.93g).
m.p. 153-154 °C
¹H-NMR (200MHz, CDCl₃) δ 1.83-1.95 (4H, m), 2.26-2.43 (3H, m), 2.45 (3H, s), 3.87-3.99 (2H, m), 7.13-7.30 (5H, m), 7.35 (2H, d, J=8.0 Hz), 7.69 (2H, d, J=8.0 Hz).
IR (KBr) 1336, 1165, 1092, 933, 725, 700, 651, 577, 546 cm⁻¹

| Anal. Calcd. for C₁₈H₂₁NO₂S | | | |
|---|---|---|---|
| Calcd. | C, 68.54 ; | H, 6.71 ; | N, 4.44. |
| Found. | C, 68.31 ; | H, 6.64 ; | N, 4.40. |

### Reference Example 221

To a solution of 1-(4-methylphenylsulfonyl)-4-phenylpiperidine (1.0g) and 1,1-dichloromethylmethylether (0.57ml) in dichloromethane (5ml) was added at 0°C a solution of titanium tetrachloride (0.7ml) in dichloromethane (5ml), and the mixture was stirred at room temperature for 2 hours. The mixture was added to stirred ice/water to stop the reaction. The mixture was extracted with ethyl acetate.
The organic layer was washed with sodium bicarbonate solution and saturated brine and dried with magnesium sulfate. Under reduced pressure, the mixture was concentrated, and the residue was purified with column chromatography (ethyl acetate/hexane=1:4→1:2) to give pale yellow crystals of 4-[1-(4-methylphenylsulfonyl)-piperidin-4-yl]benzaldehy de (0.381g). (469.4mg of the starting materials were collected)
m.p. 134-137 °C ¹H-NMR (200MHz, CDCl₃) δ 1.75-1.96 (4H, m), 2.29-2.58 (3H, m), 2.46 (3H, s), 3.90-4.03 (2H, m), 7.29-7.37 (4H, m), 7.69 (2H, d, J=8.4 Hz), 7.82 (2H, d, J=8.4 Hz), 9.97 (1H, s).
IR (KBr) 1697, 1603, 1333, 1159, 937, 721, 581, 546 cm⁻¹

| Anal. Calcd. for C₁₉H₂₁NO₃S | | | |
|---|---|---|---|
| Calcd. | C, 66.45 ; | H, 6.16 ; | N, 4.08. |
| Found. | C, 66.31 ; | H, 6.08 ; | N, 4.38. |

### Reference Example 222

To a suspension of (3-carboxypropyl)triphenyl-phosphonium bromide (16.5g) in THF (170ml) was added at room temperature potassium t-butoxide (8.63g), and the mixture was stirred at 60°C for 10 minutes and then cooled to room temperature. To the mixture was added a solution of 4-[1-(4-methylphenylsulfonyl)piperidin-4-yl]benzaldehyd e (4.40g) in THF (20ml), and the mixture was stirred at 60°Cfor 1 hour. To the mixture was added water (80ml) and the mixture was extracted with toluene (80ml). To the aqueous layer was added 1N hydrochloric acid to make the solution pH 3, and the mixture was extracted with ethyl acetate. The organic layer was washed three times with 2% sodium bicarbonate solution, and then with 1N hydrochloric acid and saturated brine (×3). Under reduced pressure, the mixture was concentrated, and the residue was dissolved in THF (150ml). To the mixture was added Pd-C (0.5g), and the mixture was stirred under hydrogen atmosphere for 5 hours. By filtration Pd-C was removed, and the filtrate was concentrated under reduced pressure. The resulting crystals were collected by filtration, which were washed with hexane to give colorless crystals of 5-[4-[1-(4-methylphenylsulfonyl)piperidin-4-yl]phenyl]p entanoic acid (4.63g).
m.p. 164-170 °C
¹H-NMR (200MHz, CDCl₃) δ 1.58-1.70 (4H, m), 1.79-1.91 (4H, m), 2.25-2.42 (5H, m), 2.45 (3H, s), 2.54-2.65 (2H, m), 3.84-3.97 (2H, m), 7.04 (2H, d, J=8.2 Hz), 7.10 (2H, d, J=8.2 Hz), 7.34 (2H, d, J=8.3 Hz), 7.68 (2H, d, J=8.3 Hz).
IR (KBr) 2937, 1703, 1335, 1163, 926, 725, 546 cm⁻¹

| Anal. Calcd. for C₂₃H₂₉NO₄S | | | |
|---|---|---|---|
| Calcd. | C, 66.48 ; | H, 7.03 ; | N, 3.37. |
| Found. | C, 66.66 ; | H, 7.00 ; | N, 3.50. |

### Reference Example 223

To a solution of 5-[4-[1-(4-methylphenylsulfonyl)-piperidin-4-yl]phenyl] pentanoic acid (0.50g) in THF (10ml) were added at room temperature oxalyl chloride (0.21ml) and a drop of DMF, and the mixture was stirred for 1 hour. Under reduced pressure, the mixture was concentrated, and the residue was dissolved in dichloromethane (10ml). To the mixture was added at 0°C aluminum chloride (0.35g), and the mixture was stirred at 0°C for 30 minutes and then at room temperature for 5 minutes. The mixture was added to ice/water, and the mixture was extracted with ethyl acetate. The organic layer was washed with 1N hydrochloric acid, saturated sodium bicarbonate solution and saturated brine, and dried with magnesium sulfate. Under reduced pressure, the mixture was concentrated, and the residue was purified with column chromatography (ethylacetate/hexane=1:2) to give colorless crystals of 3-[1-(4-methylphenylsulfonyl)-piperidin-4-yl]-6,7,8,9-tetrahydro-5H-benzocyclohepten-5-one (0.32g) .
m.p. 165-169 °C
¹H-NMR (200MHz, CDCl₃) δ 1.74-1.93 (8H, m), 2.24-2.43 (3H, m), 2.46 (3H, s), 2.68-2.76 (2H, m), 2.85-2.95 (2H, m), 3.85-4.00 (2H, m), 7.14 (1H, d, J=8.0 Hz), 7.22 (1H, dd, J=8.0, 1.8 Hz), 7.35 (2H, d, J=8.2 Hz), 7.50 (1H, d, J=1.8 Hz), 7.68 (2H, d, J=8.2 Hz).
IR (KBr) 1674, 1333, 1242, 1161, 1093, 933, 721, 546 cm⁻¹

| Anal. Calcd. for C₂₃H₂₇NO₃S | | | |
|---|---|---|---|
| Calcd. | C, 69.49 ; | H, 6.85 ; | N, 3.52. |
| Found. | C, 69.10; | H, 6.62 ; | N, 3.71. |

### Reference Example 224

To a solution of 3-[1-(4-methylphenylsulfonyl)-piperidin-4-yl]-6,7,8,9-t etrahydro-5H-benzocyclohepten-5-one (3.25g) in dimethyl carbonate (50ml) was added at room temperature sodium methoxide (2.21g), and the mixture was refluxed for 4.5 hours and cooled to room temperature. To the mixture was added 1N hydrochloric acid (100ml), and the mixture was extracted with ethyl acetate. The organic layer was washed with saturated brine and dried with magnesium sulfate. Under reduced pressure, the mixture was concentrated to give crude product (3.91g). The resulting crude product was dissolved in THF (150ml), and to the mixture was added at -40°C a solutionof sodiumborohydride (0.31g) inmethanol (10ml) . The mixture was stirred at -10 to -20°C for 1 hour. To the mixture was added a solution of sodium boro hydride (0.31g) in methanol (10ml), and the mixture was stirred for 1.5 hours. To the mixture was added acetone (2ml), and the mixture was stirred for 30 minutes. To the mixture was added water, and the mixture was extracted with ethyl acetate. The organic layer was washed with saturated brine and dried with magnesium sulfate. Under reduced pressure, the mixture was concentrated, and the residue was dissolved inTHF (40ml). To the mixture was added triethylamine (3.42ml). To the mixture was added at 0°Cmethanesulfonyl chloride (0.95ml), and the mixture was stirred at 0°C for 30 minutes and then at room temperature for 30 minutes. To the mixture was added 1,8-diaza-bicyclo[5,4,0]-7-undecene (3.7ml), and the mixture was stirred for 14 hours. To the mixture was added, and the mixture was extracted with ethyl acetate. The organic layer was washed with saturated brine and dried with magnesium sulfate. Under reduced pressure, the mixture was concentrated, and the residue was purified with column chromatography (ethyl acetate/hexane=1:2) to give colorless crystals of methyl 4-[1-(4-methylphenyl-sulfonyl)piperidin-4-yl]-6,7-dihyd ro-5H-benzocyclo-heptene-8-carboxylate (2.01g).
m.p. 169-173 °C
¹H-NMR (200MHz, CDCl₃) δ 1.75-1.92 (2H, m), 1.95-2.09 (2H, m), 2.26-2.43 (3H, m), 2.45 (3H, s), 2.62 (2H, t, J=6.2 Hz), 2.75-2.80 (2H, m), 3.81 (3H, s), 3.87-3.98 (2H, m), 6.98-7.10 (3H, m), 7.35 (2H, d, J=8.6 Hz), 7.65 (1H, s), 7.68 (2H, d, J=8.6 Hz).
IR (KBr) 1709, 1433, 1336, 1234, 1198, 1161, 1092, 933, 721, 548 cm⁻¹

| Anal. Calcd. for C₂₅H₂₉NO₄S | | | |
|---|---|---|---|
| Calcd. | C, 68.31 ; | H, 6.65 ; | N, 3.19. |
| Found. | C, 68.23 ; | H, 6.60 ; | N, 3.04. |

### Reference Example 225

To a solution of methyl 4-[1-(4-methylphenyl-sulfonyl)piperidin-4-yl]-6,7-dihyd ro-5H-benzocyclo-heptene-8-carboxylate (1.0g) in ethanol/THF (20/40ml) was added at room temperature 1N sodium hydroxide solution (2.7ml), and the mixture was stirred for 13 hours. Under reduced pressure, the mixture was concentrated. To the mixture was added water, and the mixture was washed with ethyl acetate. To the aqueous layer was added 1N hydrochloric acid (5ml), and the mixture was extracted with ethyl acetate/THF. The organic layer was washed with saturated brine and dried with magnesium sulfate. Under reduced pressure, the mixture was concentrated, and the resulting colorless crystals were collected by filtration, which were washed with hexane to give colorless crystals of 4-[1-(4-methylphenylsulfonyl)piperidin-4-yl]-6,7-dihydr o-5H-benzocycloheptene-8-carboxylic acid (565.4mg).
m.p. 255-257 °C
¹H-NMR (200MHz, CDCl₃) δ 1 .74-1 . 94 (4H, m), 1 .96-2 . 11 (2H, m), 2.28-2.48 (3H, m), 2.46 (3H, s), 2.65 (2H, t, J=6.6 Hz), 2.78-2.84 (2H, m), 3.87-4.01 (2H, m), 7.00-7.12 (3H, m), 7.35 (2H, d, J=8.2 Hz), 7.72 (2H, d, J=8.2 Hz), 7.77 (1H, s).
IR (KBr) 3008, 1674, 1352, 1294, 1273, 1255, 1163, 931, 721, 548 cm⁻¹

| Anal. Calcd. for C₂₄H₂₇NO₄S | | | |
|---|---|---|---|
| Calcd. | C, 67.74 ; | H, 6.40 ; | N, 3.29. |
| Found. | C, 67.97 ; | H, 6.69 ; | N, 311. |

### Reference Example 226

In THF (126ml) was dissolved 5-bromo-2-methyl-thiophene (10.5g), and to the mixture was added dropwise at -78°C 1.6N n-butyl lithium/hexane (40.8ml). The mixture was stirred for 1 hour, and to the mixture was added dropwise a solution of trimethyl borate (18.5g) inTHF (40ml) . The mixture was stirred for 15 minutes and warmed to room temperature. To the mixture was added 10% sulfuric acid (63ml), and the mixture was stirred for 15 minutes. The mixture was extracted with ethyl acetate, washed with saturated brine and dried with magnesium sulfate. Under reduced pressure, the solvent was removed, and the resulting residue was washed with isopropylether to give 5-methyl-2-thienyl borate (4.6g).
¹H-NMR (200MHz, CDCl₃) δ 2.59 (3H, s),6.93 (1H, d, J=3.4Hz), 7.79 (1H, d, J=3.4Hz)

### Reference Example 227

In toluene/ethanol/water (10/1/1) (24ml) was dissolved methyl 7-bromo-2,3-dihydro-1-benzoxepine-4-carboxylate (560mg), and to the mixture were added 5-methyl-2-thienyl borate (875mg) and potassium carbonate (1.56g). The mixture was stirred at room temperature for 30 minutes. To the mixture was added tetrakistriphenyl-phosphine palladium (260mg), and the mixture was stirred at 100°C for 24 hours and cooled to room temperature. The mixture was extracted with ethyl acetate, washed with saturated brine and dried with magnesium sulfate. Under reduced pressure, the solvent was removed, and the resulting residue was purified with silica gel column chromatography (hexane/acetone=12/1) to give methyl 7-(5-methyl-2-thienyl)-2,3-dihydro-1-benzoxepine-4-carb oxylate (345mg).
¹H-NMR (200MHz, CDCl₃) δ 2.28 (3H, s), 2.99 (2H, t, J=4.8Hz), 3.83 (3H, s), 4.28 (2H, t, J=4.8Hz), 6.82 (1H, d, J=1.2Hz), 7.05 (1H, d, J=8.4Hz), 7.45 (1H, dd, J=8.4, 2.4), 7.54 (1H, d, J=2.4Hz), 7.61 (1H, s)

### Reference Example 228

In THF (10.5ml) and methanol (5.2ml) was dissolved methyl 7-(5-methyl-2-thienyl)-2,3-dihydro-1-benzoxepine-4-carboxylate (525mg), and to the mixture was added 1N sodium hydroxide (10.5ml). The mixture was stirred at room temperature for 2 hours. Under reduced pressure,the organic solvent was removed, and to the residue was added ethyl acetate. The mixture was extracted with water, and to the aqueous layer was added 6N hydrochloric acid to make the solution pH 4-5, which was extracted with ethyl acetate, washed with saturated brine and dried with magnesium sulfate. Under reduced pressure, the solvent was removed to give 7-(5-methyl-2-thienyl)-2,3-dihydro-1-benzoxepine-4-carboxylic acid (410mg).
¹H-NMR (200 MHz,DMSO-d₆) δ 2.23 (3H, s), 2.87 (2H, t, J=4.4Hz), 4.24 (2H, t, J=4.4Hz), 6.99 (1H, d, J=8.4Hz), 7.07 (1H, s), 7.31 (1H, d, J=1.4Hz), 7.49 (1H, dd, J=8.4, 2.2Hz), 7.58 (1H, s), 7.74 (1H, d, J=2.2Hz).

### Reference Example 229

In toluene/ethanol/water (10/1/1) (12ml) was dissolved methyl 7-bromo-2,3-dihydro-1-benzoxepine-4-carboxylate (700mg), and to the mixture were added 3-thienyl borate (422mg) and potassium carbonate (0.98g). The mixture was stirred at room temperature for 30 minutes, and to the mixture was added tetrakistriphenylphosphine palladium (136mg). The mixture was stirred at 100°C for 13 hours and cooled to room temperature, and the mixture was extracted with ethyl acetate, washed with saturated brine and dried with magnesium sulfate. Under reduced pressure, the solvent was removed, and the resulting residue was purified with silica gel column chromatography (hexane/acetone=3/1) to give methyl 7-(3-thienyl)-2,3-dihydro-1-benzoxepine-4-carboxylate (610mg).
¹H-NMR (200MHz, CDCl₃) δ 3.00 (2H, t, J=4.2Hz), 3.83 (3H, s), 4.30 (2H, t, J=4.2Hz), 7.01 (1H, d, J=8.4Hz), 7.33-7.40 (3H, m), 7.49 (1H, dd, J=8.4, 2.4), 7.66 (1H, d, J=2.4Hz), 7.64 (1H, s)

### Reference Example 230

In THF (24ml) and methanol (6ml) was dissolved methyl 7-(3-thienyl)-2,3-dihydro-1-benzoxepine-4-carboxylate (610mg), and to the mixture was added 1N sodium hydroxide (12ml). The mixture was stirred at room temperature for 3 hours. Under reduced pressure, the organic solvent was removed, and to the residue was added ethyl acetate. The mixture was extracted with water, and to the aqueous layer was added 6N hydrochloric acid to make the solution pH 4-5, which was extracted with ethyl acetate, washed with saturated brine and dried with magnesium sulfate. Under reduced pressure, the solvent was removed to give 7-(3-thienyl)-2,3-dihydro-1-benzoxepine-4-carboxylic acid (500mg).
¹H-NMR (200MHz, DMSO-d₆) δ 2.87 (2H, t, J=4.6Hz), 4.24 (2H, t, J=4.6Hz), 7.00 (1H, d, J=8.4Hz), 7.60-7.85 (4H, m), 7.84-7.89 (2H, m)

### Reference Example 231

In ether (160ml) was dissolved 3-methylthiophene (20g), and to the mixture was added N,N,N,N-tetramethylethylenediamine (26g). To the mixture was added dropwise at room temperature 1.6N n-butyl lithium/hexane (140ml), and the mixture was refluxed for 30 minutes. The mixture was cooled to -70°C, and to the mixture was added dropwise a solution of trimethyl borate (63.5g) in THF (64ml). The mixture was stirred for 30 minutes and warmed to room temperature. To the mixture was added 10% sulfuric acid (285ml), and the mixture was stirred for 15 minutes. The mixture was washed with water and dried with magnesium sulfate. Under reduced pressure., the solvent was removed, and the resulting residue was washed with isopropylether to give 4-methyl-2-thienyl borate (6.0g).
¹H-NMR(200MHz, CDCl₃) δ 2.36 (3H, s), 7.35 (1H), 7.78 (1H, s)

### Reference Example 232

In toluene/ethanol/water (10/1/1) (8.4ml) was dissolved methyl 7-bromo-2,3-dihydro-1-benzoxepine-4-carboxylate (500mg), and to the mixture were added 4-methyl-2-thienyl borate (334mg) and potassium carbonate (651g). The mixture was stirred at room temperature for 30 minutes, and to the mixture was added tetrakistriphenylphosphine palladium (97mg). The mixture was stirred at 100°C for 24 hours and cooled to room temperature. The mixture was extracted with ethyl acetate, washed with saturated brine and dried with magnesium sulfate. Under reduced pressure, the solvent was removed, and the resulting residue was purified with silica gel column chromatography (hexane/acetone=8/1) to give methyl 7-(4-methyl-2-thienyl)-2,3-dihydro-1-benzoxepine-4-carb oxylate (432mg).
¹H-NMR (200MHz, CDCl₃) δ 2.28 (3H, s), 2.99 (2H, t, J=4.8Hz), 3.83 (3H, s), 4.28 (2H, t, J=4.8Hz), 6.82 (1H, d, J=1.2Hz), 7.05 (1H, d,,J=8.4Hz), 7.45 (1H, dd, J=8.4,2.4Hz), 7.54 (1H, d, J=2.4Hz), 7.61 (1H, s)

### Reference Example 233

In THF (10ml) was dissolved methyl 7-(4-methyl-2-thienyl)-2,3-dihydro-1-benzoxepine-4-carb oxylate (420mg), and to the mixture was added 1N sodium hydroxide (8.4ml). The mixture was stirred at room temperature for 15 hours. Under reduced pressure, the organic solvent was removed, and to the residue was added ethyl acetate. The mixture was extracted with water, and to the aqueous layer was added 6N hydrochloric acid to make the solution pH 4-5, which was extracted with ethyl acetate, washed with saturated brine and dried with magnesium sulfate. Under reduced pressure, the solvent was removed to give 7-(4-methyl-2-thienyl)-2,3-dihydro-1-benzoxepine-4-carb oxylic acid (320mg).
¹H-NMR (200MHz,DMSO-d₆) δ 2.23 (3H, s), 2.87 (2H, t, J=4.4Hz), 4.24 (2H, t, J=4.4Hz), 6.99 (1H, d, J=8.4Hz), 7.07 (1H, s), 7.31 (1H, d, J=1.4Hz), 7.49 (1H, dd, J=8.4,2.2Hz), 7. 58 (1H, s), 7.74 (1H, d, J=2.2Hz)

### Reference Example 234

To methyl 7-bromo-2,3-dihydro-1-benzoxepine-4-carboxylate (500mg) were added 4-fluorophenyl borate (272mg), potassium carbonate (537mg), water (1.5ml), ethanol (1.5ml) and toluene (15ml). Under argon atmosphere, the mixture was stirred at room temperature for 1 hour, and to the mixture was added tetrakistriphenylphosphine palladium (61mg, 3mol%). Under argon atmosphere, the mixture was refluxed for 21 hours, and to the mixture was added ethyl acetate (100ml). The mixture was washed with water (50ml) and saturated brine (50ml), and dried with anhydrous magnesium sulfate. Under reduced pressure, the solvent was removed, and the residue was purified with silica gel column chromatography to give methyl 7-(4-fluoro-phenyl)-2,3-dihydro-1-benzoxepine-4-carboxy late (310mg, 59%) as pale yellow crystals. ¹H NMR (200MHz, CDCl₃) δ 3.01 (2H, t, J=4.1Hz), 3.83 (3H, s), 4.31 (2H, t, J=4.8Hz), 7.03-7.17 (3H, m), 7.40-7.54 (4H, m), 7.66 (1H, s).

### Reference Example 235

To methyl 7-(4-fluorophenyl)-2,3-dihydro-1-benzoxepine-4-carboxyl ate (0.27g) were added THF (5.0ml), ethanol (10.0ml) and 2N sodium hydroxide solution (1.0ml), and the mixture was stirred at room temperature for 19 hours. Under reduced pressure, the solvent was removed, and the residue was diluted with water (100ml) . The aqueous layer was made acidic with hydrochloric acid, and the mixture was extracted with ethyl acetate (100ml). The organic layer was dried with anhydrous magnesium sulfate, and the solvent was removed under reduced pressure. The residue was crystallized and washed with hexane to give 7-(4-fluorophenyl)-2,3-dihydro-1-benzoxepine-4-carboxyl ic acid (0.22g, 86%) as white crystals.
¹H NMR (200MHz, CDCl₃) δ 3.03 (2H, t, J=4.8Hz), 4.33 (2H, t, J=4.6Hz), 7.05-7.17 (3H, m), 7.43-7.55 (4H, m), 7.76 (1H, s).

### Reference Example 236

To 4-bromophenoxybutyric acid (75.0g) was added polyphosphoric acid (873g), and the mixture was stirred at 100°C for 45 minutes. The mixture was poured into ice (about 1.5kg), and the mixture was extracted with ethyl acetate (1.5L and 0.5L) . The organic layer was washed with water (400ml×3), 1N sodium hydroxide solution (400ml×2), saturated sodium hydrogen carbonate solution (400ml×2), water (400ml×3) and saturated brine (400ml×3), and dried with anhydrous magnesium sulfate. The solvent was removed under reduced pressure to give 7-bromo-2,3,4,5-tetrahydro-1-benzoxepin-5-one (38.6g, 55%, 132.5°C/0.33mmHg) as pale yellow oil.

### Reference Example 237

To a solution of 5-bromo-2-fluorobenzaldehyde (0.49 g, 2.62 mmol) and ethyl 3-mercaptopropionate (0.37 ml, 2.88 mmol) in N,N-dimethylformamide (10 ml) was added potassium carbonate (0.90 g, 6.55 mmol), and the mixture was stirred at room temperature for 1 hour and then at 70°C for 15 hours. The mixture was poured into ice-water, and made pH 4 with 1N hydrochloric acid. The aqueous layer was extracted with ethyl acetate, and the organic layer was washed with water and saturated brine, and dried with magnesium sulfate. The solvent was evaporated, and the residue was purified with silica gel column chromatography [hexane: ethyl acetate (5:1)] to give ethyl 6-bromo-2H-thiochromene-3-carboxylate (0.45 g, 58%) as yellow powder, a part of which was recrystallized from ethanol to give pale yellow needles. m.p. 87°C
¹H-NMR (CDCl₃) δ : 7.47 (1H, br s), 7.26-7.38 (2H, m), 7.14 (1H, d, J=8.0), 4.31 (2H, q, J=7.4), 3.73 (2H, d, J=1.2), 1.36 (3H, d, J=7.4).

| | | |
|---|---|---|
| Anal. Calcd for C₁₂H₁₁BrO₂S: | C; 48.17, | H; 3.71. |
| Found: | C; 48.07, | H; 3.77. |

### Reference Example 238

A solution of ethyl 6-bromo-2H-thiochromene-3-carboxylate (1.00 g, 3.34 mmol), 4-methylphenyl borate (0.55 g, 4.01 mmol) and tetrakistriphenylphosphine palladium (0.19 g, 0.167 mmol) in 2M sodium carbonate (3.5 ml), ethanol (3 ml) and toluene (25 ml) was stirred at 80°C for 24 hours. To the mixture was added water, and the mixture was extracted with ethyl acetate. The organic layer was washed with0.5N hydrochloric acid and saturated brine, and dried with magnesium sulfate. The solvent was evaporated, and the residue was purified with silica gel column chromatography [hexane:ethyl acetate (5:1)] to give ethyl 6-(4-methylphenyl)-2H-thiochromene-3-carboxylate (1.02 g, 99%) as yellow powder.
m.p. 87°C
¹H-NMR (CDCl₃) δ : 7.62 (1H, br s), 7.40-7.46 (4H, m), 7.22-7.31 (3H, m), 4.31 (2H, q, J=7.0), 3.77 (2H, d, J=1.0), 2.40 (3H, s), 1.37 (3H, t, J=7.0).

| | | |
|---|---|---|
| Anal. Calcd for C₁₉H₁₈O₂S : | C; 73.52, | H; 5.84. |
| Found: | C; 73.51, | H; 5.65. |

### Reference Example 239

To a solution of ethyl 6-(4-methylphenyl)-2H-thio-chromene-3-carboxylate (2.12 g, 6.84 mmol) in tetrahydrofuran (20 ml) and acetonitrile (20 ml) was added dropwise 1N sodium hydroxide (7 ml), and the mixture was stirred at 60°C for 2.5 hours. The solvent was evaporated, and the residue was dissolved in diethylether. The mixture was extracted with water. The organic layer was extracted with 0.5N sodium hydroxide, and both of the aqueous layers were made pH 3 with 6N hydrochloric acid. The mixture was extracted with ethyl acetate, and the organic layer was washed with saturated brine and dried with magnesium sulfate. The solvent was evaporated to give 6-(4-methyl-phenyl)-2H-thiochromene-3-carboxylic acid (1.83 g, 95%) as yellow powder.
m.p. 244°C
¹H-NMR (DMSO-d₆) δ : 7.44 (1H, d, J=1.8), 7.21-7.32 (4H, m), 7.05 (1H, d, J=8.4), 6.95 (2H, d, J=8.2), 3.41 (2H, d, J=1.0), 2.02 (3H, s).

| | | |
|---|---|---|
| Anal. Calcd for C₁₇H₁₄O₂S · 0.25H₂O: | C ; 71.18, | H; 5.09. |
| Found: | C; 70.90, | H; 4.80. |

### Reference Example 240

To a solution of 4-nitrobenzaldehyde (6.0 g, 37.7 mmol) and ethyl β-aminopropionate hydrochloride (6.1 g, 37.7 mmol) in 1,2-dichloroethane (120 ml) was added triethylamine (5.3 ml, 37.7 mmol) and at 0°C was added little by little triacetoxy boro hydride (11.8 g, 52.8 mmol). The mixture was stirred at room temperature for 1 hour, and to the mixture was added 37% formalin (4.0 ml, 49.0 mmol) and then at 0°C triacetoxy boro hydride (11.8 g, 52.8 mmol). The mixture was stirred at room temperature for 14 hours, and the mixture was neutralized with saturated sodium hydrogen carbonate and extracted with dichloromethane. The extract was washed with saturated brine and dried with magnesium sulfate. The solvent was evaporated to give crude product, which was purified with silica gel column chromatography [hexane:ethyl acetate (3:2)] to give ethyl 3-(N-methyl-N-(4-nitrobenzyl))aminopropionate (9.34 g, 93%) as pale yellow oil.
¹H-NMR (CDCl₃) δ : 8.17 (2H, dd, J=8.8, 1.8), 7.49 (2H, d, J=8.8), 4.15 (2H, q, J=7.4), 3.61 (2H, s), 2.76 (2H, t, J=7.2), 2.52 (2H, t, J=7.2), 2.22 (3H, s), 1.26 (3H, t, J=7.4).

| | | | |
|---|---|---|---|
| Anal. Calcd for C₁₃H₁₈N₂O₄: | C; 58.63, | H; 6.81, | N; 10.52. |
| Found: | C; 58.24, | H; 6.78, | N; 10.23. |

### Reference Example 241

To a solution of 4-nitrobenzaldehyde (2.0g, 13.2 mmol) and 2-methoxyethylamine (1.15 ml, 13.2 mmol) in 1,2-dichloroethane (40 ml) was added triethylamine (1.9 ml), and at 0°C was added little by little triacetoxy boro hydride (4 . 1 g) . The mixture was stirred at room temperature for 1 hour was stirred, and to the mixture was added 37% formalin (1.4 ml) and then at 0°C triacetoxy boro hydride (4.1 g). The mixture was stirred at room temperature for 14 hours, neutralized with saturated sodium hydrogen carbonate solution and extracted with dichloromethane. The extract was washed with saturated brine and dried with magnesium sulfate. The solvent was evaporated to give crude product which was purified with silica gel column chromatography [hexane:ethyl acetate (1: 2)] to give 4-((N-(2-methoxy-ethyl)-N-methyl)aminomethyl)nitrobenze ne (2.75 g, 93%) as pale yellow oil.
¹H-NMR (CDCl₃) δ : 8.18 (2H, d, J=8.8), 7.53 (2H, d, J=8.8), 3.66 (2H, s), 3.53 (2H, t, J=5.6), 3.35 (3H, s), 2.63 (2H, t, J=5.6), 2.28 (3H, s).

| | | | |
|---|---|---|---|
| Anal. Calcd for C₁₄H₂₀N₂O₃: | C; 63.62, | H; 7.63, | N; 10.60. |
| Found: | C; 63.54, | H; 7.59, | N; 10.51. |

### Reference Example 242

To a solution of 4-nitrobenzaldehyde (1.76 g, 11.7 mmol) and 4-aminocyclohexanol (1.34 g, 13.2 mmol) in 1,2-dichloroethane (30 ml) was added triethylamine (1.6 ml) and at 0°C was added little by little triacetoxy boro hydride (3.7 g) . The mixture was stirred at room temperature for 1 hour, and to the mixture was added 37% formalin (1.2ml) and then at 0°C triacetoxy boro hydride (3.7 g). The mixture was stirred at room temperature for 14 hours, neutralized with saturated sodium hydrogen carbonate and extracted with dichloromethane. The extract was washed with saturated brine and dried with magnesium sulfate. The solvent was evaporated to give crude product, which was purified with silica gel column chromatography [ethyl acetate:ethanol (2:1)] to give (E)-4-((N-(4-hydroxy-cyclohexyl)-N-methyl)aminomethyl)n itrobenzene (2.08 g, 67%) as pale yellow crystals, a part of which was recrystallized from ether/hexane to give pale yellow needles.
m.p. 87°C
¹H-NMR (CDCl₃) δ : 8.17 (2H, d, J=8.6), 7.51 (2H, d, J=8.6), 3.51-3.65 (1H, m), 2.39-2.56 (1H, m), 2.18 (3H, s), 1.83-2.12 (4H, m), 1.20-1.51 (4H, m).

| | | | |
|---|---|---|---|
| Anal. Calcd for C₁₄H₂₀N₂O₃: | C; 63.62, | H; 7.63, | N; 10.68. |
| Found: | C; 63.54, | H; 7.59, | N; 10.51. |

### Reference Example 243

To a solution of (E)-4-((N-(4-hydroxycyclohexyl)-N-methyl)aminomethyl)ni trobenzene (1.07 g, 4.05 mmol) in ethyl acetate (30 ml) was added 5%-Pd/C (0.43 g), and the mixture was stirred under hydrogen atmosphere for 3.5 hours. The mixture was filtered with sellaite, and the filtrate was concentrated. The resulting residue was purified with silica gel column chromatography [ethyl acetate:methanol: triethylamine (9:1: 0.02) to give (E)-4-((N-(4-hydroxy-cyclohexyl)-N-methyl)aminomethyl)a niline (0.27 g, 28%) as yellow powder.
m.p. 105°C.
¹H-NMR (CDCl₃) δ : 7.09 (2H, d, J=8.6), 6.65 (2H, d, J=8.6), 3.46-3.70 (1H, m), 3.45 (2H, s), 2.35-2.53 (1H, m), 2.16 (3H, s), 1.84-2.10 (4H, m), 1.19-1.51 (4H, m).

### Reference Example 244

To a solution of ethyl 3-(N-methyl-N-(4-nitro-benzyl))aminopropionate (1.51g, 5.68mmol) in acetic acid (30ml) was added iron (1.27g, 22.7mmol), and the mixture was stirred for 14 hours. The solvent was evaporated, and the precipitates were filtered with sellaite and washed with ethyl acetate. The filtrate was diluted with water, made basic with potassium carbonate and extracted with ethyl acetate. The extracted was washed with saturated brine and dried with magnesium sulfate. The solvent was evaporated, and the residue was purified with silica gel column chromatography [ethyl acetate:ethanol (2:1)] to give ethyl 3-(N-methyl-N-(4-aminobenzyl))aminopropianate (0.70g, 52%) as brown oil.
¹H-NMR (CDCl₃) δ: 7.07 (2H, d, J=8.6), 6.64 (2H, d, J=8.6), 4.13 (2H, q, J=6.8), 3.41 (2H, s), 3.30-3.60 (2H, m), 2.73 (2H, t, J=7.4), 2.51 (2H, t, J=7.4), 2.19 (3H, s), 1.25 (3H, t, J=6.8).

### Reference Example 245

To a solution of 4-((N-(2-methoxyethyl)-N-methyl)-aminomethyl)nitrobenze ne (1.1 g, 4.91 mmol) in acetic acid (20 ml) was added iron (1.1g, 19.6 mmol), and the mixture was stirred for 15 hours. The solvent was evaporated, and the precipitates were filtered with sellaite and washed with ethyl acetate. The filtrate was diluted with water, made basic with potassium carbonate and extracted with ethyl acetate. The extract was washed with saturated brine and dried with magnesium sulfate. The solvent was evaporated, and the residue was purified with silica gel column chromatography [ethyl acetate:methanol: triethylamine (7:1:0.02)] to give 4-((N-(2-methoxyethyl)-N-methyl)-aminomethyl)aniline(88 0 mg, 92%) as brown oil.
¹H-NMR (CDCl₃) δ : 7.09 (2H, d, J=8.4), 6.64 (2H, d, J=8.4), 3.50 (2H, t, J=5.8), 3.45 (2H, s), 3.33 (3H, s), 2.57 (2H, t, J=5.8), 2.24 (3H, s).

### Reference Example 246

To a solution of 4-nitrobenzaldehyde (6.04 g, 40.0 mmol), N-methylethanolamine (3.00 g, 40.0 mmol) and triethylamine (5.6 ml, 40.0 mmol) in tetrahydrofuran (200 ml) was added triacetoxy boro hydride (26.8 g, 120 mmmol), and the mixture was stirred for 21 hours. The mixture was diluted with ethyl acetate, and washed with saturated sodium hydrogen carbonate and saturated brine. The extract was dried, and the solvent was evaporated to give crude product, which was purified with silica gel column chromatography [ethyl acetate:ethanol (4:1)] to give 4-((N-(2-hydroxy-ethyl)-N-methyl)aminomethyl)nitrobenze ne (7.08 g, 84%) as yellow oil.
¹H-NMR (CDCl₃) δ : 8.20 (2H, d, J=8.8), 7.50 (2H, d, J=8.8), 3.68 (2H, s), 3.68 (2H, t, J=5.6), 2.64 (2H, t, J=5.6), 2.52-2.70 (1H, m), 2.26 (3H, s).

### Reference Example 247

To a solution of 4-((N-(2-hydroxyethyl)-N-methyl)aminomethyl)nitrobenzen e (2.95 g, 14.1 mmol) in acetic acid (60 ml) was added iron (3.14 g, 56.2 mmol), and the mixture was stirred for 23 hours. The solvent was evaporated, and the precipitates were filtered with sellaite and washed with ethyl acetate. The filtrate was diluted with water, madepH 10 with potassium carbonate and extracted with ethyl acetate. The extract was washed with saturated brine and dried with magnesium sulfate. The solvent was evaporated, and the residue was purified with silica gel column chromatography [ethyl acetate:methanol: triethylamine (5:1:0.3)] to give 4-((N-(2-hydroxyethyl)-N-methyl)aminomethyl)aniline (1.25 g, 49%) as brown oil. ¹H-NMR (CDCl₃) δ : 7.07 (2H, d, J=8.4), 6.65 (2H, d, J=8.4), 3.61 (2H, t, J=5.2), 3.46 (2H, s), 2.57 (2H, t, J=5.2), 2.20 (3H, s).

### Reference Example 248

To THF(60ml) was added at -70°C n-butyllithium (1.59M hexane solution, 63ml, 100mmol) . To the mixture was added dropwise (taking about 1 hour) a solution of 2,6-dibromopyridine (23.69g, 100mmol) in THF (140ml) at -60°C, and the mixture was stirred at -70°C for 15 minutes. To the mixture was added DMF (12ml), and the mixture was stirred at the same temperature for 15 minutes. To the mixture was added 20% ammonium chloride solution (100ml), and the organic layer was separated. The aqueous layer extracted with ethyl acetate (100ml), and the organic layer was mixed with the previous organic layer. The organic layer was dried with anhydrous sodium sulfate and concentrated under reduced pressure. The residue was purified with column chromatography (silica gel 150g, ethyl acetate/hexane= 1/20), and the desired fraction was concentrated under reduced pressure. To the residue was added diisopropyl-ether (15ml), and insoluble materials were filtered, which were washed with diisopropylether (5ml × 3) and dried under reduced pressure to give 6-bromo-2-pyridinecarbaldehyde (2.05g, 11.0mmol, 11%).
IR (KBr): 1732 cm⁻¹.
¹H-NMR (CDCl₃) δ: 7.65-8.00 (3H, m), 10.01 (1H, s).

### Reference Example 249

InTHF (10ml) was suspended sodiumhydride (60%, 440mg, 11.0mmol), and to the mixture was added at -30°C a solution of diethylphosphonoethyl acetate (2.47g, 11.0mmol) in THF (10ml). The mixture was stirred at the same temperature for 30 minutes, and to the mixture was added at -30°C a solution of 6-bromo-2-pyridinecarbaldehyde (1.86g, 10.0mmol) in THF (10ml). While warming the temperature of the mixture from -30°C to -10°C, the mixture was stirred for 1.5hours. To the mixture was added diethylether (40ml), and the mixture was washed with water (20ml, 5ml×2) and saturated brine (5ml). The organic layer was dried with anhydrous magnesium sulfate and concentrated under reduced pressure. To the residue was added hexane (10ml), and the mixture was cooled to 0°C. The precipitated insoluble materials were filtered, which were washed with hexane cooled to 0°C, and dried under reduced pressure to give ethyl 6-bromo-2-pyridine-acrylate (2.00g, 7.81mmol, 78%).
IR (KBr): 1717, 1703 cm⁻¹.
¹H-NMR (CDCl₃) δ : 1.34 (3H, t, J=7.1Hz), 4.28 (2H, q, J=7.1Hz), 6.96 (1H, d, 15.8Hz), 7.30-7.65 (4H, m).

### Reference Example 250

In 1,2-dimethoxyethane (4ml) were dissolved ethyl 6-bromo-2-pyridineacrylate (512mg, 2.00mmol) and 4-methylphenyl borate (299mg, 2.20mmol), and to the mixture were added sodium carbonate (424mg, 4.00 mmol), water (2ml) and tetrakis-(triphenylphosphine)palladium (116mg, 0.10mmol). The mixture was stirred at 80°C for 10 hours. To complete the reaction, 4-tolyl borate (150mg, 1.10mmol) and tetrakis(triphenyl-phosphine)palladium (116mg, 0.10mmol) were added at 80°C to the mixture, and the mixture was stirred for 14 hours. To the mixture was added ethyl acetate (30ml), and the mixture was water (5ml×2) and saturated brine (5ml). The organic layer was dried with anhydrous magnesium sulfate and concentrated under reduced pressure. The residue was purified with column chromatography (silica gel 15g, ethyl acetate/hexane= 1/19), and the desired fraction was concentrated under reduced pressure to give ethyl 6-(4-methylphenyl)-2-pyridineacrylate (495mg, 1.85mmol, 93%).
IR (KBr): 1713 cm⁻¹.
¹H-NMR (CDCl₃) δ : 1.36 (3H, t, J=7.1Hz), 2.42 (3H, s), 4.30 (2H, q, J=7.1Hz), 7.10 (1H, d, 15.6Hz), 7.25-7.35 (3H, m), 7.65-7.85 (3H, m), 7.99 (2H, d, J=8.2Hz).

### Reference Example 251

In methanol (5ml) was suspended ethyl 6-(4-methyl-phenyl)-2-pyridineacrylate (465mg, 1.74mmol), and to the mixture was added at 0°C 1N sodium hydroxide solution (5.22ml). The mixture was stirred at room temperature for 20 hours. To the mixture was added at 0°C 1N hydrochloric acid (5.22ml), and methanol was evaporated under reduced pressure. The aqueous layer extracted with ethyl acetate (30ml, 20ml). The organic layer was dried with anhydrous sodium sulfate and concentrated under reduced pressure. To the residue was added diisopropylether (5ml), and Insoluble materials were filtered, which were washed with diisopropylether and dried under reduced pressure to give 6-(4-methylphenyl)-2-pyridineacrylic acid (344mg, 1.44mmol, 83%).
¹H-NMR (CDCl₃) δ 2.43 (3H, s), 7.15 (1H, d, 15.5Hz), 7.25-7.40 (1H, m), 7.31 (2H, d, J=8.5Hz), 7.70-7.85 (2H, m), 7.84 (1H, d, J=15.5Hz), 8.00 (2H, d, J=8.5Hz).

### Reference Example 252

In 1,2-dimethoxyethane(12ml) were dissolved methyl 7-bromo-2,3-dihydro-1-benzoxepine-4-carboxylate (566mg, 2.00mmol) and 3,4-methylenedioxyphenyl borate (465mg, 2.80mmol). To the mixture were added sodium carbonate (424mg, 4.00mmol), water (2ml) and tetrakis(triphenyl-phosphine)palladium (162mg, 0.14mmol), and the mixture was stirred at 80°C for 14 hours. To the mixture was added ethyl acetate (30ml), and the mixture was extracted with water (5ml × 2) and saturated brine (5ml). The organic layer was dried with anhydrous magnesium sulfate and concentrated under reduced pressure. The residue was purified with column chromatography (silica gel 15g, ethyl acetate/hexane=1/19), and the desired fraction was concentrated under reduced pressure. To the residue was added diisopropylether, and the insoluble materials were filtered, which were washed with diisopropylether and dried under reduced pressure to give methyl 7-(3,4-methylene-dioxyphenyl)-2,3-dihydro-1-benzoxepine -4-carboxylate (434mg, 1.34mmol, 67%).
IR (KBr): 1705 cm-1.
¹H-NMR (CDCl₃) δ : 2.95-3.10 (2H, m), 3.83 (3H, s), 4.25-4.35 (2H, m), 6.01 (2H, s), 6.87 (1H, d, J=8.6Hz), 6.95-7.10 (3H, m), 7.40 (1H, dd, J=8.4, 2.4Hz), 7.47 (1H, d, J=2.2Hz), 7.65 (1H, s).

### Reference Example 253

In methanol (5ml) was suspended 7-(3,4-methylenedioxy-phenyl)-2,3-dihydro-1-benzoxepine -4-carboxylate (399mg, 1.23mmol), and to the mixture was added 1N sodium hydroxide solution (3.69ml). The mixture was stirred at room temperature for 20 hours, and to the mixture was added 1N hydrochloric acid (3.69ml). The mixture was concentrated under reduced pressure, and to the residue was added water. Insoluble materials were filtered, which were washed with water and diethylether and dried under reduced pressure to give 7-(3,4-methylenedioxyphenyl)-2,3-dihydro-1-benzoxepine-4-carboxylic acid(321mg, 1.03mmol, 84%).
¹H-NMR (DMSO-d₆) δ : 2 .80-2. 95 (2H, m), 4.15-4.35 (2H, m), 6.05 (2H, s), 6.97 (1H, d, J=8.1Hz), 7.01 (1H, d, J=8.4Hz), 7.16 (1H, dd, J=8.1, 1.7Hz), 7.29 (1H, d, J=1.7Hz), 7.53 (1H, dd, J=8.4, 2.3Hz), 7.63 (1H, s), 7.74 (1H, d, J=2.3Hz).

### Reference Example 254

In THF (100ml) was dissolved 1,2-methylenedioxy-4-bromobenzene (24.00g, 119mmol), and to the mixture was added dropwise at -55°C or less n-butyllithium (1.6M hexane solution, 82ml, 131mmol). The mixture was stirred at -70°C for 30 minutes, and the resulting mixture was added dropwise at -60°C or less to a solution of trimethyl borate (18.61g, 179mmol) in tetrahydrofuran (50ml) with using cannula. The mixture was stirred at -70°C for 1 hour and then for 2 hours with warming to room temperature. To the mixture were added 1N hydrochloric acid (130ml) and diethylether (150ml), and the organic layer was separated. The organic layer was washed with water (50×2ml) and saturated brine (50ml), dried with anhydrous magnesium sulfate and concentrated under reduced pressure. To the residue was added diisopropylether (40ml), and insoluble materials were filtered, which were washed with diisopropylether (30ml × 4) and dried under reduced pressure to give 3,4-methylenedioxyphenyl borate (6.79g, 40.9mmol, 34%).
¹H-NMR (DMSO-d₆) δ : 5.99 (2H, s), 6.8-6.95 (1H, m), 7.25-7.45 (2H, m).

### Reference Example 255

In methanol (250ml) was suspended 5-nitrosalicylic acid (50.0g, 273 mmol), and to the mixture was added sulfuric acid (6ml). The mixture was stirred at 100°C for 24 hours and the cooled to room temperature. The precipitated insoluble materials were filtered, which were washed with hydrous methanol (containing 20% of water) and methanol, and dried under reduced pressure to give methyl 5-nitrosalicylate (38.5g, 195mmol, 72%).
¹H-NMR (CDCl₃) δ : 4.04 (3H, s), 7.10 (1H, d, J=9.5Hz), 8.35 (1H, dd, J=2.7, 9.5Hz), 8.81 (1H, d, J=2.7Hz), 11.45 (1H, s, OH).

### Reference Example 256

In DMF (50ml) was dissolved methyl 5-nitrosalicylate (1.97g, 10.0mmol), and to the mixture were added ethyl 4-bromobutyrate (1.57ml, 11.0mmol) and potassium carbonate (2.76g, 20.0mmol). The mixture was stirred at 110°C for 5 hours, and the mixture was concentrated under reduced pressure. To the residue was added ethyl acetate, and the mixture was washed with water and 10% potassium carbonate solution. The organic layer was dried with anhydrous magnesium sulfate and concentrated under reduced pressure. The residue was purified with column chromatography (silica gel 30g, ethyl acetate/hexane=1/5→1/3), and the desired fraction was concentrated under reduced pressure to give ethyl 4-(2-methoxycarbonyl-4-nitrophenoxy)butyrate (2.51g, 8.06mmol, 81%).
¹H-NMR (CDCl₃) δ : 1.26 (3H, t, J=7,2Hz), 2.1-2.3 (2H, m), 2.60 (2H, t, J=7.1Hz), 3.93 (3H, s), 4.15 (2H, q, J=7.2Hz), 4.23 (2H, t, J=6.1Hz), 7.06 (1H, d, J=9.4Hz), 8.35 (1H, dd, J=2.8, 9.4Hz), 8.71 (1H, d, J=2.8Hz).

### Reference Example 257

In THF (25ml) was dissolved ethyl 4-(2-methoxy-carbonyl-4-nitrophenoxy)butyrate (2.37g, 7.61mmol), and to the mixture was added 10% palladium-carbon (containing 50% water, 0.94g). The mixture was subjected to catalytic reduction at room temperature for 4 hours. Insoluble materials were filtered off, and the filtrate was dried with anhydrous magnesium sulfate and concentrated under reduced pressure to give ethyl 4-(4-amino-2-methoxycarbonyl-phenoxy)butyrate (2.20g). IR (KBr): 1730 cm⁻¹.
¹H-NMR (CDCl₃) δ : 1.25 (3H, t, J=7.2Hz), 2.0-2.2 (2H, m), 2.56 (2H, t, J=7.3Hz), 3.88 (3H, s), 4.00 (2H, t, J=6.0Hz), 4.14 (2H, q, J=7.2Hz), 6.75-6.9 (2H, m), 7.1-7.2 (1H, m).

### Reference Example 258

A mixture of ethyl 4-(4-amino-2-methoxycarbonyl-phenoxy)butyrate (2.20g), bis(2-chloroethyl)ether (0.915ml, 7.81mmol), potassium carbonate(3.24g, 23.4mmol), sodium iodide (2.34g, 15.6mmol) and DMF (20ml) was stirred at 70°C for 24 hours, and the mixture was concentrated under reduced pressure. To the residue was added water, and the mixture was extracted with ethyl acetate. The organic layer was dried with anhydrous sodium sulfate and concentrated under reduced pressure. The residue was purified with column chromatography (silica gel 30g, ethyl acetate/hexane=1/4), and the desired fraction was concentrated under reduced pressure to give ethyl 4-(2-methoxy-carbonyl-4-morpholinophenoxy)butyrate (2.18g).
IR (KBr): 1732 cm⁻¹.
¹H-NMR (CDCl₃) δ : 1.25 (3H, t, J=7.1Hz), 2.0-2.2 (2H, m), 2.57 (2H, t, J=7.1Hz), 3.0-3.15 (4H, m), 3.8-3.9 (4H, m), 3.89 (3H, s), 4.04 (2H, t, J=6.0Hz), 4.14 (2H, q, J=7.1Hz), 6.92 (1H, d, J=9.0Hz), 7.04 (1H, dd, J=3.1, 9.0Hz), 7.36 (1H, d, J=3.1Hz).

### Reference Example 259

In THF (15ml) was dissolved diisopropylamine (1.018ml), and to the mixture was added dropwise at 0°C n-butyl lithium (4.2ml). The mixture was stirred at the same temperature for 30 minutes. To the mixture was added dropwise a solution of ethyl 4-(2-methoxycarbonyl-4-morpholinophenoxy)butyrate (1829mg, 5.18mmol) inTHF (5ml) at-78°C, ice bath was removed, and the mixture was stirred for 7 hours. To the mixture was added at 0°C 10% ammonium chloride solution (30ml), and the mixture was extracted with ethyl acetate (30ml×3). The organic layer was dried with anhydrous sodium sulfate and concentrated under reduced pressure. The residue was purified with column chromatography (silica gel 50g, ethyl acetate/hexane=1/5), and the desired fraction was concentrated under reduced pressure to give ethyl 7-morpholino-5-oxo-2,3,4,5-tetrahydro-1-benzoxepine-4-c arboxylate (924mg, 2.89mmol, 56%).

### Reference Example 260

In THF (10ml) was dissolved ethyl 7-morpholino-5-oxo-2,3,4,5-tetrahydro-1-benzoxepine-4-c arboxylate (924mg, 2.89mmol), and to the mixture was added at -30°C a solution of sodium boro hydride (164mg, 4.34mmol) in methanol (3ml). The mixture was stirred at -20°C to -15°C for 30 minutes, and the mixture was cooled to -50°C, to which was added water (15ml). The mixture was extracted with ethyl acetate (15ml×3), and the organic layer was dried with anhydrous magnesium sulfate and concentrated under reduced pressure. The residue was dissolved in THF (10ml), and to the mixture were added at 0°C triethylamine (2.02ml, 14.5mmol) and methanesulfonylchloride (0.336ml, 4.34mmol) . The mixture was stirred at room temperature for 17 hours and concentrated under reduced pressure. To the residue was added water (15ml), and the mixture was extracted with ethyl acetate (20ml×3). The organic layer was dried with anhydrous sodium sulfate and concentrated under reduced pressure. The residue was purified with column chromatography (silica gel 30g, ethyl acetate/ hexane=1/5), and the desired fraction was concentrated under reduced pressure to give ethyl 7-morpholino-2,3-dihydro-1-benzoxepine-4-carboxylate (691mg, 2.28mmol, 79%).
IR (KBr): 1703 cm⁻¹.
¹H-NMR (CDCl₃) δ: 1.35 (3H, t, J=7.2Hz), 2.9-3.0 (2H, m), 3.05-3.15 (4H, m), 3.8-3.9 (4H, m), 4.22 (2H, t, J=4.8Hz), 4.28 (2H, q, J=7.2Hz), 6.8-7.0 (3H, m), 7.54 (1H, s).

### Reference Example 261

In methanol (8ml) was dissolved ethyl 7-morpholino-2,3-dihydro-1-benzoxepine-4-carboxylate (800mg, 2.64mmol), and to the mixture was added 1N sodium hydroxide solution (8ml). The mixture was stirred at room temperature for 12 hours, and to the mixture was added 1N hydrochloric acid (8ml). The organic solvent was evaporated under reduced pressure, and the precipitated insoluble materials were filtered, which were washed with water and diisopropylether and dried under reduced pressure to give 7-morpholino-2,3-dihydro-1-benzoxepine-4-carboxylic acid (649mg, 2.36mmol, 89%).
¹H-NMR (CDCl₃) δ : 2.97 (2H, t, J=4.5Hz), 3.05-3.15 (4H, m), 3.8-3.95 (4H, m), 4.25 (2H, t, J=4.5Hz), 6.8-7.0 (3H, m), 7.67 (1H, s).

### Reference Example 262

A mixture of 4-nitrobenzylamine (6.09g, 40.0mmol), 2-chloropyrimidine (4.82g, 42.1mmol), triethylamine (11.2ml, 80.4mmol) and ethanol (120ml) was stirred at 110°C for 24 hours, and the mixture was concentrated under reduced pressure. To the residue was added water, and the mixture was extracted with ethyl acetate-THF. The organic layer was dried with anhydrous sodium sulfate and concentrated under reduced pressure. The residue was recrystallized from ethyl acetate-ethanol to give N-(4-nitrobenzyl)-N-(2-pyrimidinyl)amine (0.99g, 4.3mmol, 11%) .
¹H-NMR (CDCl₃) δ: 4.77 (2H, d, J=6.4Hz), 5.59 (1H, m), 6.62 (1H, t, J=4.9Hz), 7.51 (2H, d, J=8.6Hz), 8.19 (2H, d, J=8.6Hz), 8.30 (2H, d, J=4.9Hz).

### Reference Example 263

In THF (20ml) and methanol (20ml) was dissolved N-(4-nitrobenzyl)-N-(2-pyrimidinyl)amine (921mg, 4.00mmol), and to the mixture were added at 0°C nickel bromide (137mg) and sodium boro hydride(955mg). The mixture was stirred at room temperature for 30 minutes and concentrated under reduced pressure. To the residue were added ethyl acetate, THF and water, and the insoluble materials were filtered off. The aqueous layer was extracted with ethyl acetate-THF, and the organic layer was dried with anhydrous sodium sulfate and concentrated under reduced pressure. The residue was purified with column chromatography (silica gel 30g, ethyl acetate/hexane=1/1), and the desired fraction was concentrated under reduced pressure. To the residue was added diethylether, and the insoluble materials were filtered, which were washed with diethylether and dried under reduced pressure to give 4- [N- (2-pyrimidinyl) aminomethyl] aniline (208mg, 1.04mmol, 26%).
¹H-NMR (CDCl₃) δ: 4.50 (2H, d, J=5.4Hz), 5.32 (1H, m), 6.54 (1H, t, J=4.7Hz), 6.66 (2H, d, J=8.3Hz), 7.15 (2H, d, J=8.3Hz), 8.29 (2H, d, J=4.7Hz).

### Reference Example 264

A mixture of methyl 7-bromo-2,3-dihydro-1-benzoxepine-4-carboxylate (1416mg, 5.00 mmol), zinc cyanide (352mg, 3.00mmol), tetrakis(triphenylphosphine)-palladium (347mg, 0.30mmol) and DMF(10ml) was stirred at 80°C for 3 hours. The mixture was concentrated under reduced pressure, and to the residue was added ethyl acetate. Insoluble materials were filtered off, which were washed with ethyl acetate. The filtrate was concentrated under reduced pressure. The resulting crude product was recrystallized from ethyl acetate to give methyl 7-cyano-2,3-dihydro-1-benzoxepine-4-carboxylate (800mg, 3.49mmol, 70%).
IR (KBr): 2222, 1721 cm⁻¹.
¹H-NMR (CDCl₃) δ : 2.95-3.1 (2H, m), 3.84 (3H, s), 4.3-4.4 (2H, m), 7.05 (1H, d, J=8.8Hz), 7.50 (1H, dd, J=2.0, 8.8Hz), 7.52 (1H, s), 7.66 (1H, d, J=2.0Hz).

### Reference Example 265

In toluene (15ml) was suspended methyl 7-cyano-2,3-dihydro-1-benzoxepine-4-carboxylate (642mg, 2.80mmol), and to the mixture were added trimethylsilylazide (0.929ml, 7.00mmol) and dibutyl tin oxide (70mg, 0.28mmol). The mixture was stirred at 100°C for 24 hours and concentrated under reduced pressure. To the residue was added methanol, and the mixture was concentrated under reduced pressure. To the residue was added ethyl acetate, and the mixture was extracted with saturated sodium bicarbonate solution (30ml, 10ml×2). To the aqueous layer was added 6N hydrochloric acid to make the solution about pH 1, and the mixture was extracted with ethyl acetate and THF ((30ml50ml) and (10ml/10ml) × 2) . The organic layer was dried with anhydrous magnesium sulfate and concentrated under reduced pressure, to the residue was added ethyl acetate. Insoluble materials were filtered, which were washed with ethyl acetate and dried under reduced pressure to give methyl 7-(1H-tetrazol-5-yl)-2,3-dihydro-1-benzoxepine-4-carbox ylate (662mg, 2.43mmol, 87%).
¹H-NMR (DMSO-d₆) δ : 2 . 85-3 .0 (2H, m), 3.78 (3H, s), 4 .25-4 .4 (2H, m), 7.21 (1H, d, J=8.6Hz), 7.60 (1H, s), 7.94 (1H, dd, J=2.1, 8.6Hz), 8.16 (1H, d, J=2.1Hz).

### Reference Example 266

In DMF (6ml) was dissolved methyl 7-(1H-tetrazol-5-yl)-2,3-dihydro-1-benzoxepine-4-carbox ylate (400mg, 1.47mmol), and to the mixture was added at 0°C sodium hydride (60%, 90mg, 2.3mmol). The mixture was stirred at the same temperature for 15 minutes, and to the mixture was added at 0°C methyl iodide (0.28ml, 4.4mmol). While the temperature of the mixture was warmed from 0°C to room temperature, the mixture was stirred for 3 hours. To the mixture was added at 0°C water (30ml), and the mixture was extracted with ethyl acetate. The organic layer was dried with anhydrous sodium sulfate and concentrated under reduced pressure. The residue was purified with column chromatography (silica gel 40g, ethyl acetate/hexane=1/8 →1/2), and the first eluted desired fraction was concentrated under reduced pressure to give methyl 7-(2-methyl-1H-tetrazol-5-yl)-2,3-dihydro-1-benzoxepine -4-carboxylate (334mg, 1.17mmol, 79%). The second eluted desired fraction was concentrated under reduced pressure to give methyl 7-(1-methyl-1H-tetrazol-5-yl)-2,3-dihydro-1-benzoxepine -4-carboxylate (76mg, 0.27mmol, 18%).
Methyl
7-(2-methyl-1H-tetrazol-5-yl)-2,3-dihydro-1-benzoxepine -4-carboxylate;
IR (KBr): 1705 cm⁻¹.
¹H-NMR (CDCl₃) δ : 2.95-3.1 (2H, m), 3.83 (3H, s), 4.25-4.4 (2H, m), 4.39 (3H, s), 7.09 (1H, d, J=8.4Hz), 7.69 (1H, s), 8.00 (1H, dd, J=2.2, 8.4Hz), 8.15 (1H, d, J=2.2Hz).
Methyl
7-(1-methyl-1H-tetrazol-5-yl)-2,3-dihydro-1-benzoxepine -4-carboxylate;
IR (KBr): 1705 cm⁻¹.
¹H-NMR (CDCl₃) δ : 3.0-3.1 (2H, m), 3.84 (3H, s), 4.3-4.45 (2H, m), 4.20 (3H, s), 7.17 (1H, d, J=8.4Hz), 7.61 (1H, s), 7.63 (1H, dd, J=2.2, 8.4Hz), 7.75 (1H, d, J=2.2Hz).

### Reference Example 267

In methanol (7ml) and THF (7ml) was suspended methyl 7-(2-methyl-1H-tetrazol-5-yl)-2,3-dihydro-1-benzoxepine -4-carboxylate (324mg, 1.13mmol), and to the mixture was added 1N sodium hydroxide solution (3.4ml). The mixture was stirred at 50°C for 4 hours, and to the mixture was added, under ice-cooling, 1N hydrochloric acid (3.4ml). The mixture was concentrated under reduced pressure, and to the residue was added water. Insoluble materials were filtered, which were washed with water and dried under reduced pressure to give 7-(2-methyl-1H-tetrazol-5-yl)-2,3-dihydro-1-benzoxepine -4-carboxylic acid (295mg, 1.08mmol, 96%).

### Reference Example 268

In methanol (3ml) and THF (3ml) was dissolved methyl 7-(2-methyl-1H-tetrazol-5-yl)-2,3-dihydro-1-benzoxepine -4-carboxylate (76mg, 0.27mmol), and to the mixture was added 1N sodium hydroxide solution (0.8ml). The mixture was stirred at 50°C for 4 hours, and to the mixture was added, under ice-cooling, 1N hydrochloric acid (0.8ml). The mixture was concentrated under reduced pressure, and to the residue was added water. Insoluble materials were filtered, which were washed with water and dried under reduced pressure to give 7-(1-methyl-1H-tetrazol-5-yl)-2,3-dihydro-1-benzoxepine -4-carboxylic acid (69mg, 0.25 mmol, 95%).

### Reference Example 269

In THF (500ml) was dissolved 4- [(benzyloxy) carbonyl] - aminobutyric acid (25.0g), and to the mixture was gradually added at -5°C methyl iodide (37.4g). Under nitrogen atmosphere, the mixture was stirred at 0°C for 15 minutes and then at room temperature for 24 hours. To the mixture was added ethyl acetate (300ml) and then water (800ml). The mixture was made pH 11 with sodium hydroxide and washed with ether (400ml ×2). The aqueous layer was made pH 2 with concentrated hydrochloric acid and extracted with ethyl acetate (1000ml and 500ml × 3). The organic layer was washed with 1M sodium thiosulfate solution (300ml) and dried with magnesium sulfate. The solvent was evaporated under reduced pressure to give 4-[(benzyloxy)carbonyl]-4-methyl-aminobutyric acid (26.3g).
¹H NMR (200MHz, CDCl₃) δ 1.88 (2H, m), 2.35-2.37 (2H, m), 2.93 (3H, s), 3.36 (2H, t, J=6.6Hz), 5.13 (2H, s), 7.35 (5H, s) .

### Reference Example 270

To dichloromethane (1000ml) was added at room temperature anhydrous magnesium sulfate (50.6g) and then concentrated sulfuric acid (6.0ml). The mixture was stirred at room temperature for 15 minutes, and to the mixture was added 4-[(benzyloxy)carbonyl]-4-methyl-aminobutyric acid (26.3g) and then tert-butanol (50.5ml). The mixture was sealed completely and stirred at room temperature for 18 hours. To the mixture was added saturated sodium hydrogen carbonate solution to dissolve all of the magnesium sulfate, and the mixture was stirred. The organic layer was separated, washed with saturated brine (400ml) and dried with anhydrous magnesium sulfate. The solvent was evaporated under reduced pressure, and the residue was purified with silica gel column chromatography (250g, hexane:ethyl acetate=5:1) to give tert-butyl 4-[(benzyloxy)-carbonyl]-4-methylaminobutyrate (17.2g, 53%).
¹H NMR (200MHz, CDCl₃) δ 1.44 (9H, s), 1.82 (2H, quint, J=6.6Hz), 2.21 (2H, t, J=6.2Hz), 2.93 (3H, s), 3.31 (2H, t, J=7.1Hz), 5.13 (2H, s), 7.35 (5H, s).

### Reference Example 271

In methanol (70ml) was dissolved tert-butyl 4-[(benzyloxy)carbonyl]-4-methylaminobutyrate (6.06g), and to the mixture was added 10% palladium-carbon (580mg) . Under hydrogen atmosphere, the mixture was stirred at room temperature for 3 hours, and 10% palladium-carbon was removed. The solvent was evaporated under reduced pressure to give tert-butyl 4-methylaminobutyrate (3.35g, 98%). ¹H NMR (200MHz, CDCl₃) δ 1.45 (9H, s), 1.72 (1H, brs), 1.77 (2H, quint, J=7.2Hz), 2.27 (2H, t, J=7.3Hz), 2.43 (3H, s), 2.61 (2H, t, J=7.1Hz).

### Reference Example 272

In DMF (5.0ml) was dissolved tert-butyl 4-methyl-aminobutyrate (1050mg), and to the mixture was added at room temperature a solution of 5-bromo-2-fluorobenzaldehyde (1025mg) in DMF (1.0ml) and then potassium carbonate (837mg). The mixture was stirred at 70°C for 60 hours, and to the mixture was added at room temperature water (50ml). The mixture was extracted with ethyl acetate (50ml×3), and the organic layer was washed with saturated brine (50ml×3) and dried with anhydrous magnesium sulfate. The solvent was evaporated under reduced pressure, and the residue was purified with silica gel column chromatography (75g, hexane:ethyl acetate=10:1) to give tert-butyl 4-(4-bromo-2-formyl-N-methylanilino) butyrate (1620mg, 90%).
¹H NMR (200MHz, CDCl₃) δ 1.42 (9H, s), 1.88 (2H, quint, J=7.4Hz), 2.22 (2H, t, J=7.3Hz), 2.88 (3H, s), 3.14 (2H, t, J=7.3Hz), 7.01 (1H, d, J=8.6Hz), 7.55 (1H, dd, J=8.7, 2.5Hz), 7.88 (1H, d, J=2.6Hz), 10.19 (1H, s).

### Reference Example 273

In tert-butanol (250ml) was dissolved tert-butyl 4-(4-bromo-2-formyl-N-methylanilino)butyrate (4.54g) and tert-butoxy potassium (1.43g), and the mixture was refluxed for 1 hour and cooled. To the mixture was added water (500ml), and the mixture was extracted with ethyl acetate (500ml ×2). The aqueous layer was made weakly acidic with 1N hydrochloric acid (about 12.5ml), and the mixture was extracted with ethyl acetate (500ml) . Both of these organic layer was washed with saturated brine (250ml) and dried with anhydrous magnesium sulfate. The solvent was evaporated under reduced pressure, and the residue was purified with silica gel column chromatography (200g, hexane:ethyl acetate=10:1→1:1) to give tert-butyl 7-bromo-1-methyl-2,3-dihydro-1-benzoazepine-4-carboxyla te (3.33g, 77%) and 7-bromo-1-methyl-2,3-dihydro-1H-1-benzoazepine-4-carbox ylic acid (0.60g, 17%). tert-butyl 7-bromo-1-methyl-2,3-dihydro-1-benzoazepine-4-carboxyla te;
¹H NMR (200MHz, CDCl₃) δ 1.53 (9H, s), 2.80 (2H, t, J=4.8Hz), 3.00 (3H, s), 3.21 (2H, t, J=4.7Hz), 6.65 (1H, d, J=8.8Hz), 7.25 (1H, dd, J=8.8, 2.2Hz), 7.39 (1H, d, J=2.6Hz), 7.46 (1H, s).
7-bromo-1-methyl-2,3-dihydro-1H-1-benzoazepine-4-carbox ylic acid;
¹H NMR (200MHz, CDCl₃) δ 2.85 (2H, t, J=4.8Hz), 3.03 (3H, s), 3.25 (2H, t, J=4.9Hz), 6.67 (1H, d, J=9.2Hz), 7.29 (1H, dd, J=8.8, 2.2Hz), 7.44 (1H, d, J=2.6Hz), 7.67 (1H, s).

### Reference Example 274

In water:ethanol:toluene (1:1:10, 18.0ml) were dissolved 4-methylphenyl borate (276mg) and tert-butyl 7-bromo-l-methyl-2,3-dihydro-l-benzoazepine-4-carboxyla te (571mg), and to the mixture was added potassium carbonate (560mg). The mixture was stirred under argon atmosphere for 30 minutes, and to the mixture was added tetrakistriphenylphosphine palladium (78mg). Under argon atmosphere, the mixture was refluxed for 19.5 hours. The mixture was diluted with ethyl acetate (300ml) and washed with water (100ml) and saturated brine (100ml). The organic layer was dried with anhydrous magnesium sulfate. The solvent was evaporated under reduced pressure, and the residue was purified with silica gel column chromatography (120g, hexane→ hexane : ethyl acetate=10:1) to give tert-butyl 1-methyl-7-(4-methylphenyl)-2,3-dihydro-1-benzoazepine-4-carboxylate (422mg, 72%).
¹H NMR (200MHz, CDCl₃) δ 1.54 (9H, s), 2.38 (3H, s), 2.83 (2H, t, J=4.9Hz), 3.06 (3H, s), 3.28 (2H, t, J=4.9Hz), 6.85 (1H, d, J=8.4Hz), 7.23 (2H, d, J=8.0Hz), 7.447 (1H, dd, J=8.6, 2.4Hz), 7.463 (2H,d, J=8.2Hz),7.53 (1H, d, J=2.2Hz), 7.67 (1H, s).

### Reference Example 275

In ethyl acetate (7.0ml) was dissolved tert-butyl 1-methyl-7-(4-methylphenyl)-2,3-dihydro-1-benzoazepine-4-carboxylate (490mg), and to the mixture was added 4N hydrochloric acid (ethyl acetate) (7.0ml). The mixture was stirred at room temperature for 20 hours. The solvent was evaporated under reduced pressure, and the residue was washed with hexane (10ml×3) to give 1-methyl-7-(4-methylphenyl)-2,3-dihydro-1-benzoazepine-4-carboxylic acid hydrochloride (443mg, 96%).
mp 249-252°C (decomp.).
¹HNMR (200MHz, DMSO-d₆) δ 2.32 (3H, s), 2.75 (2H, t, J=4.6Hz), 3.03 (3H, s), 3.25 (2H, t, J=4.9Hz), 6.92 (1H, d, J=8.6Hz), 7.22 (2H, d, J=8.2Hz), 7.53 (1H, dd, J=8.8, 2.4Hz), 7.55 (2H, d, J=8.2Hz), 7.65 (1H, d, J=2.4Hz), 7.68 (1H, s).
IR (KBr) 3021, 2469, 1707, 1466, 1190, 1107, 810, 530 cm⁻¹.

| Anal. Calcd. for C₁₉H₁₉NO₂•HCl•0.3H₂O: | | | |
|---|---|---|---|
| | C, 68.08; | H, 6.19; | N, 4.18. |
| Found: | C, 67.97; | H, 6.13; | N, 4.05. |

### Reference Example 276

In DMF (12.0ml) was dissolved 7-bromo-1-methyl-2,3-dihydro-1-benzoazepine-4-carboxyli c acid hydrochloride (600mg), and to the mixture was added thionyl chloride (0.39ml). The mixture was stirred at room temperature for 15 minutes. The solvent was evaporated under reduced pressure, and the residue was dissolved in dichloromethane (14.0ml). The thus obtained acid chloride solution was added dropwise at 0°C to a solution of 4-[[N-methyl-N-(tetrahydropyran-4-yl)amino]methyl]anili ne (562mg) and triethylamine (1.48ml) in dichloromethane (5.5ml). The mixture was stirred at 0°C for 10 minutes and then at room temperature for 5 hours. To the mixture was added water (100ml), and the mixture was extracted with dichloromethane (100ml×3). The organic layer was dried with anhydrous magnesium sulfate. The solvent was evaporated under reduced pressure, and the residue was purified with silica gel column chromatography (150g, ethyl acetate:ethanol=10:1) to give 7-bromo-1-methyl-N-[4-[[N-methyl-N-(tetrahydropyran-4-y 1)amino]methyl]-phenyl]-2,3-dihydro-1-benzoazepine-4-ca rboxamide (767mg, 75%). mp 62-64°C.
¹H NMR (200MHz, CDCl₃) δ 1.63-1.79 (4H, m), 2.21 (3H, s), 2.57-2.72 (1H, m), 2.94 (2H, t, J=4.2Hz), 3.03 (3H, s), 3.27-3.44 (2H + 2H, m), 3.57 (2H, s), 4.00-4.07 (2H, m), 6.70 (1H, d, J=8.8Hz), 7.20 (1H, s), 7.26-7.303 (2H, m), 7.301 (1H, dd, J=8.6, 2.4Hz), 7.42 (1H, d, J=2.6Hz), 7.50-7.55 (1H + 2H, m).
IR (KBr) 3264, 2949, 2843, 1655, 1597, 1514, 1499, 1406, 1314, 1246, 1182, 810 cm⁻¹.

| Anal. Calcd. for C₂₅H₃₀N₃O₂Br•0.25H₂O: | | | |
|---|---|---|---|
| | C, 61.41; | H, 6.29; | N, 8.59. |
| Found: | C, 61.45; | H, 6.25; | N, 8.32. |

### Working Example 273 (Production of Compound 273)

In hydrous methanol was dissolved N,N-dimethyl-N-(4-(((7-(4-methylphenyl)-2,3-dihydro-1-b enzoxepin-4-yl)carbonyl)amino)benzyl)tetrahydro-2H-pyra n-4-aminium iodide (14.2g), and the mixture was subjected to ion exchange resin (DOWEX SBR, 20-50 mesh, Cl⁻ type) column and eluted with hydrous methanol. The solvent of the resulting fraction was evaporated, and to the residue was added acetone to give crude crystals, which were recrystallized from ethanol to give N,N-dimethyl-N-(4-(((7-(4-methylphenyl)-2,3-dihydro-1-benzoxepin-4-yl)carbonyl)-amino)benzyl)-t etrahydro-2H-pyran-4-aminium chloride (Compound 273) (10.4g) as colorless crystals.
mp 232-237°C(dec.).
¹H-NMR(δ ppm, DMSO-d₆) 1.76-2.00 (2H, m), 2.14-2.20 (2H, m), 2.35 (3H, s), 2 .89 (6H, s), 3. 01 (2H, t, J=4. 5Hz), 3.29-3.46 (2H, m), 3.55-3.69 (1H, m), 4.04-4.09 (2H, m), 4.31 (2H, t, J=4.5Hz), 4.50 (2H, s), 7.06 (1H, d, J=8.4Hz), 7.27 (2H, d, J=8.4Hz), 7.46 (1H, s), 7.53-7.59 (5H, m), 7.79 (1H, d, J=2.2Hz), 7.92 (2H, d, J=8.4Hz), 10.34 (1H, s).
IR(KBr) ν : 2973, 2849, 1645, 1516cm⁻¹.

| Anal. Calcd. for C₃₂H₃₇ClN₂O₃: | | | | |
|---|---|---|---|---|
| | C,72.10; | H,7.00; | N,5.25; | Cl, 6.65. |
| Found | C,72.03; | H,6.83; | N,5.38; | Cl, 6.47. |

### Working Example 274 (Production of Compound 274)

In dichloromethane (5ml) was suspended 7-(4-methyl-phenyl)-2,3-dihydro-1-benzoxepine-4-carboxy lic acid (0.25g), and to the mixture were added, under ice-cooling, oxalyl chloride (0.16ml) anddimethylformamide (catalytic amount). The mixture was stirred at room temperature for 2 hours, and the solvent was evaporated. The residue was dissolved in tetrahydrofuran (20ml), and the mixture was added dropwise to a solution of 4-((N,N-bis(2-methoxy-ethyl)amino)methyl)aniline (0.24g) and triethylamine (0.4ml) in tetrahydrofuran (10ml) under ice-cooling. Under nitrogen atmosphere, the mixture was stirred at room temperature overnight, and the solvent was evaporated. To the residue was added water, and the mixture was extracted with ethyl acetate. The organic layer washed with water and saturated brine, and dried with anhydrous magnesium sulfate. Under reduced pressure, the solvent was evaporated, and the residue was purified with silica gel column (ethyl acetate) to give crude crystals, which were recrystallized from ethyl acetate-hexane to give N-(4-((N,N-bis(2-methoxyethyl)-amino)methyl)phenyl)-7-( 4-methylphenyl)-2,3-dihydro-1-benzoxepine-4-carboxamide (Compound 274) (0.25g) as colorless crystals.
mp 110-112°C.
¹H-NMR(δppm, CDCl₃) 2.39 (3H, s), 2.74 (4H, t, J=6.0Hz), 3.07 (2H, t, J=4.4Hz), 3.32 (6H, s), 3.48 (4H, t, J=6.0Hz), 3.69 (2H, s), 4.35 (2H, t, J=4.4Hz), 7.05 (1H, d, J=8.0Hz), 7.24 (2H, d, J=8.4Hz), 7.33 (2H, d, J=8.8Hz), 7.43-7.55 (6H, m), 7.61 (1H, s).
IR(KBr) *ν :* 3287, 2876, 1651cm⁻¹.

| Anal. Calcd. for C₃₁H₃₆N₂O₄: | | | |
|---|---|---|---|
| | C,74.37; | H,7.25; | N,5.60. |
| Found | C,74.33; | H,7.15; | N,5.45. |

### Working Example 275 (Production of Compound 275)

In dichloromethane (5ml) was suspended 7-(4-methyl-phenyl)-2,3-dihydro-1-benzoxepine-4-carboxy lic acid (0.25g), and to the mixture were added, under ice-cooling, oxalyl chloride (0.23ml) and dimethylformamide (catalytic amount). The mixture was stirred at room temperature for 2 hours, and the solvent was evaporated. The residue was dissolved in tetrahydrofuran (20ml), and the mixture was added dropwise to a solution of 4-((N-(3-ethoxypropyl)-N-methylamino)methyl)aniline dihydrochloride (0.3g) and triethylamine (0.62ml) in tetrahydrofuran (10ml), under ice-cooling. Under nitrogen atmosphere, the mixture was stirred at room temperature overnight, and the solvent was evaporated. To the residue was added water, and the mixture was extracted with ethyl acetate. The organic layer washed with water and saturated brine, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give N-(4-((N-(3-ethoxypropyl)-N-methylamino)methyl)phenyl)-7-(4-methylphenyl)-2,3-dihydro-1-benzoxepine-4-carboxam ide (Compound 275) (0.3g) as colorless crystals.
mp 119-122°C.
¹H-NMR(δppm, CDCl₃) 1.19 (3H, t, J=7.1Hz), 1.65-1.85 (2H, m), 2.19 (3H, s), 2.39 (3H, s), 2.46 (2H, t, J=7.2Hz), 3.08 (2H, t, J=4.8Hz), 3.42-3.52 (6H, m), 4.36 (2H, t, J=4.8Hz), 7.06 (1H, d, J=8.4Hz), 7.24 (2H, d, J=8.0Hz), 7.30 (2H, d, J=8.8Hz), 7.44-7.58 (7H, m).
IR (KBr) ν : 2975, 2872, 1647, 1516cm⁻¹.

| Anal . Calcd. for C₃₁H₃₆N₂O₃: | | | |
|---|---|---|---|
| | C,76.83; | H,7.49; | N,5.78. |
| Found | C,76.73; | H,7.31; | N,5.95. |

### Working Example 276 (Production of Compound 276)

In THF (5ml) was dissolved 7-(4-methylphenyl)-2,3-dihydro-1-benzoxepine-4-carboxyl ic acid (0.25g), and to the mixture were added, under ice-cooling, oxalyl chloride (0.16ml) anddimethylformamide (catalytic amount). The mixture was stirred at room temperature for 2 hours, and the solvent was evaporated. The residue was dissolved in tetrahydrofuran (15ml), and the mixture was added dropwise to a solution of 4-((N-(1,3-dimethoxypropan-2-yl)-N-methylamino)methyl)a niline (0.23g) and triethylamine (0.5ml) in tetrahydrofuran (10ml), under ice-cooling. Under nitrogen atmosphere, the mixture was stirred at room temperature overnight, and the solvent was evaporated. To the residue was added water, and the mixture was extracted with ethyl acetate. The organic layer washed with water and saturated brine, and dried with anhydrous magnesium sulfate. Under reduced pressure, the solvent was evaporated, and the residue was purified with silica gel column (ethyl acetate/hexane) to give crude crystals, which were recrystallized from ethyl acetate-hexane to give N-(4-((N-(1,3-dimethoxypropan-2-yl)-N-methylamino)methy 1)-phenyl)-7-(4-methylphenyl)-2,3-dihydro-1-benzoxepine -4-carboxamide (Compound276) (0.25g) as colorless crystals.
mp 128-132°C.
¹H-NMR(δ ppm, CDCl₃) 2.31 (3H, s), 2.39 (3H, s), 3.00-3.09 (3H, m), 3.35 (6H, s), 3.44-3.63 (4H, m), 3.71 (2H, s), 4.35 (2H, t, J=4.7Hz), 7.05 (1H, d, J=8.4Hz), 7.24 (2H, d, J=8 .0HZ), 7.33 (2H, d, J=8.8Hz), 7.43-7.58 (7H, m). IR(KBr) ν : 3285, 2882, 1651, 1516cm⁻¹.

| Anal. Calcd. for C₃₁H₃₆N₂O₄: | | | |
|---|---|---|---|
| | C,74.37; | H, 7.25; | N,5.60. |
| Found | C,74.17; | H,7.05; | N,5.75. |

### Working Example 277 (Production of Compound 277)

In THF (5ml) was dissolved 7-(4-methylphenyl)-2,3-dihydro-1-benzoxepine-4-carboxyl ic acid (0.25g), and to the mixture were added, under ice-cooling, oxalyl chloride (0.16ml) anddimethylformamide (catalytic amount). The mixture was stirred at room temperature for 2 hours, and the solvent was evaporated. The residue was dissolved in tetrahydrofuran (15ml), and the mixture was added dropwise to a solution of 4-((N-(2-methoxyethyl)-N-methylamino)-methyl)aniline (0.21g) and triethylamine (0.37ml)intetrahydrofuran (10ml), under ice-cooling. Under nitrogen atmosphere, the mixture was stirred at room temperature overnight, and the solvent was evaporated. To the residue was added water, and the mixture was extracted with ethyl acetate. The organic layer was washed with water and saturated brine, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give N-(4-((N-(2-methoxyethyl)-N-methylamino)methyl)phenyl)-7-(4-methylphenyl)-2,3-dihydro-1-benzoxepine-4-carboxam ide (Compound 277) (0.24g) as colorless crystals.
mp 121-122°C .
¹H-NMR (δ ppm,CDCl₃) 2.26 (3H, s), 2.39(3H, s), 2.60 (2H, t, J=5.8Hz), 3.07 (2H, t, J=4.5Hz), 3.35 (3H, s), 3.49-3.54 (4H, m), 4.35 (2H, t, J=4.5Hz), 7.05 (1H, d, J=8.4Hz), 7.24 (2H, d, J=8.8Hz), 7.31 (2H, d, J=8.8Hz), 7.43-7.56 (6H, m), 7.62 (1H, s).
IR(KBr) ν: 3287, 2926, 1651, 1516cm⁻¹.

| Anal. Calcd. for C₂₉H₃₂N₂O₃: | | | |
|---|---|---|---|
| | C,76.29; | H,7.06; | N,6.14. |
| Found | C, 75.99; | H,7.02; | N,6.22. |

### Working Example 278 (Production of Compound 278)

In water/ethanol/toluene(1:1:10, 18.0ml) were dissolved 4-trifluoromethoxyphenyl borate (208mg) and 7-bromo-1-methyl-N-[4-[[N-methyl-N-(tetrahydro-2H-pyran -4-yl)amino]methyl]phenyl]-2,3-dihydro-1-benzazepine-4-carboxamide (407mg), and to the mixture was added potassium carbonate (279mg). Under argon atmosphere, the mixture was stirred for 30 minutes, and the mixture was added tetrakistriphenylphosphine palladium (39mg). Under argon atmosphere, the mixture was refluxed for 16 hours, and the mixture was diluted with ethyl acetate (200ml). The mixture was washed with water (50ml) and saturated brine (50ml), and the organic layer was dried with anhydrous magnesium sulfate. The solvent was evaporated under reduced pressure, and the residue was purified with silica gel column chromatography (75g, ethyl acetate→ethyl acetate/ethanol=20:1) and recrystallized from ethanol to give 1-methyl-N-[4-[[N-methyl-N-(tetrahydro-2H-pyran-4-yl)am ino]methyl]phenyl]-7-(4-trifluoromethoxyphenyl)-2,3-dih ydro-1-benzazepine-4-carboxamide (Compound 278) (148mg, 31%).
mp 182-183°C.
¹H NMR (200MHz, CDCl₃) δ 1.63-1.76 (4H, m), 2.20 (3H, s), 2.56-2.72 (1H, m), 2.96 (2H, t, J=4.6Hz), 3.09 (3H, s), 3.30-3.43 (4H, m), 3.56 (2H, s), 4.01-4.06 (2H, m), 6.89 (1H, d, J=8.6Hz), 7.25 (2H, d, J=8.2Hz), 7.30 (2H, d, J=8.6Hz), 7.40 (1H, s), 7.48 (1H, dd, J=8.6, 2.4Hz), 7.51-7.58 (6H, m) .
IR (KBr) 2951, 2847, 1651, 1514, 1501, 1260, 1221, 1163, 806, 733 cm⁻¹.

| | | | |
|---|---|---|---|
| Anal. Calcd. for C₃₂H₃₄N₃O₃F₃ : | C, 67.95; | H, 6.06; | N, 7.43. |
| Found: | C, 67.74; | H, 5.87; | N, 7.68. |

### Working Example 279 (Production of Compound 279)

In water/ethanol/toluene (1:1:10, 18.0ml) were dissolved 4-(1-piperidinyl)phenyl borate (179mg) and 7-bromo-1-methyl-N-[4-[[N-methyl-N-(tetrahydro-2H-pyran -4-yl)amino]methyl]phenyl]-2,3-dihydro-1-benzazepine-4-carboxamide (353mg), and to the mixture was added potassium carbonate (242mg). Under argon atmosphere, the mixture was stirred for 40 minutes, and to the mixture was added tetrakistriphenylphosphine palladium (34mg). Under argon atmosphere, the mixture was refluxed for 15 hours, and the mixture was dilute with ethyl acetate (200ml) . The mixture was washed with water (50ml) and saturated brine (50ml), and the organic layer was dried with anhydrous magnesium sulfate. The solvent was evaporated under reduced pressure, and the residue was purified with silica gel column chromatography (75g, ethyl acetate/ethanol=9:1) and recrystallized from ethanol to give 1-methyl-N-[4-[[N-methyl-N-(tetrahydro-2H-pyran-4-yl)am ino]methyl]-phenyl]-7-[4-(1-piperidinyl)phenyl]-2,3-dih ydro-1-benzazepine-4-carboxamide (Compound 279) (79mg, 19%).
mp 202-204°C.
¹H NMR (200MHz, CDCl₃) δ 1.59-1.77 (10H, m), 2.21 (3H, s), 2.57-2.73 (1H, m), 2.95 (2H, t, J=4.4Hz), 3.07 (3H, s), 3.19 (4H, t, J=5.1Hz), 3.31-3.43 (4H, m), 3.57 (2H, s), 4.01-4.06 (2H, m), 6.86 (1H, d, J=8.4Hz), 6.99 (2H, d, J=8.8Hz), 7.30 (2H, d, J=8.6Hz), 7.39-7.50 (5H, m), 7.54 (2H, d, J=8.4Hz), 7.57 (1H, s).
IR (KBr) 2938, 2849, 1645, 1607, 1505, 1314, 1235, 910, 812, 733cm⁻¹.

| | | | |
|---|---|---|---|
| Anal. Calcd. for C₃₆H₄₄N₄O₂: | C, 76.56; | H, 7. 85; | N, 9.92. |
| Found: | C, 76.53; | H, 7.79; | N, 10.01. |

### Working Example 280 (Production of Compound 280)

In water/ethanol/toluene (1:1:10, 60.0ml) were dissolved 4-methylphenyl borate (658mg) and 7-bromo-1-formyl-N-[4-[[N-methyl-N-(tetrahydro-2H-pyran -4-yl)amino]methyl]phenyl]-2,3-dihydro-1-benzazepine-4-carboxamide (2.01g), and to the mixture was added potassium carbonate (1.34g). Under argon atmosphere, the mixture was stirred for 30 minutes, and to the mixture was added tetrakistriphenylphosphine palladium (186mg). Under argon atmosphere, the mixture was refluxed for 17 hours, and the mixture was dilute with ethyl acetate (750ml). The mixture was washed with water (200ml) and saturated brine (100ml), and the organic layer was dried with anhydrous magnesium sulfate. The solvent was evaporated under reduced pressure, and the residue was purified with silica gel column chromatography (150g, ethyl acetate→ethyl acetate/ethanol=20:1) and recrystallized from ethanol to give 1-formyl-7-(4-methylphenyl)-N-[4-[[N-methyl-N-(tetrahyd ro-2H-pyran-4-yl)amino]methyl]phenyl]-2,3-dihydro-1-ben zazepine-4-carboxamide (Compound 280) (669mg, 33%).
mp 229-230. 5°C.
¹H NMR (200MHz, CDCl₃) δ 1.69-1.79 (4H, m), 2.21 (3H, s), 2.41 (3H, s), 2.57-2.72 (1H, m), 3.04 (2H, t, J=4.9Hz), 3.37 (2H, td, J=10.2, 3.1Hz), 3.57 (2H, s), 3.93 (2H, t, J=5.5Hz), 4.01-4.07 (2H, m), 7.21 (1H, d, J=8.2Hz), 7.29 (2H, d, J=7. 6Hz), 7.32 (2H, d, J=8.4Hz), 7.50 (2H, d, J=8.8Hz), 7.54 (2H, d, J=8.8Hz), 7.58 (1H, s), 7.59 (1H, dd, J=8.2, 2.2Hz), 1H was concealed under 7.55-7.58, 7.71 (1H, d, J=2.2Hz), 8.56 (1H, s).
IR (KBr) 2946, 2847, 1667, 1597, 1516, 1497, 1360, 1316, 814, 733 cm⁻¹.

| | | | |
|---|---|---|---|
| Anal. Calcd. for C₃₂H₃₅N₃O₃ : | C, 75.41; | H, 6.92; | N, 8.25. |
| Found: | C, 75.45; | H, 6.95; | N, 8.18. |

### Working Example 281 (Production of Compound 281)

To 1-formyl-7-(4-methylphenyl)-N-[4-[[N-methyl-N-(tetrahyd ro-2H-pyran-4-yl)amino]methyl]phenyl]-2,3-dihydro-1-ben zazepine-4-carboxamide (1177mg) was added 1N hydrochloric acid (20ml), and the mixture was stirred at 100°Cfor 1 hour. The mixture was dilute with ethyl acetate(50ml) and made weakly basic with saturated sodium hydrogen carbonate solution (45ml). To the mixture were added ethyl acetate (250ml) and water (100ml), and separated. The organic layer was dried with anhydrous magnesium sulfate. The solvent was evaporated under reduced pressure, and the residue was purified with silica gel column chromatography (75g, ethyl acetate/ethanol=9:1) to give 7-(4-methyl-phenyl)-N-[4-[[N-methyl-N-(tetrahydro-2H-py ran-4-yl)amino]methyl]phenyl]-2,3-dihydro-1-benzazepine -4-carboxamide (Compound 281) (804mg, 72%) as amorphous.
¹H NMR (200MHz, CDCl₃) δ 1.69-1.80 (4H, m), 2.21 (3H, s), 2.38 (3H, s), 2.58-2.72 (1H, m), 2.96 (2H, t, J=4.4Hz), 3.37 (2H, td, J=11.4, 3.1Hz), 3.47 (2H, t, J=4.8Hz), 3.57 (2H, s), 4.01-4.07 (2H, m), 4.53-4.70 (1H, br), 6.71 (1H, d, J=8.4Hz), 7.22 (2H, d, J=7.8Hz), 7.28-7.32 (4H, m), 7.35 (1H, dd, J=8.4, 2.2Hz), 7.42 (1H, s), 7.46 (1H, s), 7.48 (1H, d, J=2.0Hz), 7.54 (2H, d, J=8.6Hz).
IR (KBr) 3330, 2949, 2847, 1651, 1609, 1514, 1507, 1408, 1316, 910, 812, 735 cm⁻¹.

| | | | |
|---|---|---|---|
| Anal. Calcd. for C₃₁H₃₅N₃O₂: | C, 77.31; | H, 7.32; | N, 8.72. |
| Found: | C, 77.44; | H, 7.12; | N, 8.78. |

### Working Example 282 (Production of Compound 282)

In dimethylformamide (5ml) was dissolved 7-(4-ethoxyphenyl)-1-methyl-2,3-dihydro-1-benzazepine-4 -carboxylic acid hydrochloride (0.5g), and to the mixture was added, under ice-cooling, thionyl chloride (0.25ml). The mixture was stirred at room temperature for 45 minutes, and the solvent was evaporated. The residue was dissolved in tetrahydrofuran (15ml), and the mixture was added dropwise to a suspension of 4-((N-(3-ethoxypropyl)-N-methylamino)methyl)aniline dihydrochloride (0.41g) and triethylamine (1.2ml) in tetrahydrofuran (10ml), under ice-cooling. Under nitrogen atmosphere, the mixture was stirred at room temperature overnight, and the solvent was evaporated. To the residue was added water, and the mixture was extracted with ethyl acetate. The organic layer was washed with water and saturated brine, and dried with anhydrous magnesium sulfate. Under reduced pressure, the solvent was evaporated, and the residue was purified with silica gel column (methanol/triethylamine/ethyl acetate) to give crude crystals, which were recrystallized from ethyl acetate-hexane to give N-(4-((N-(3-ethoxypropyl)-N-methylamino)methyl)phenyl)-7-(4-ethoxyphenyl)-1-methyl-2,3-dihydro-1-benzazepine-4 -carboxamide (Compound 282) (0.39g) as pale yellow crystals.
mp 129-131°C.
¹H-NMR(δppm, CDCl₃) 1.19 (3H, t, J=6.9Hz), 1.44 (3H, t, J=7.1Hz), 1.76-1.84 (2H, m), 2.19 (3H, s), 2.46 (2H, t, J=7.4Hz), 2.97 (2H, t, J=4.6Hz), 3.09 (3H, s), 3.35 (2H, t, J=4.8Hz), 3.41-3.52 (6H, m), 4.07 (2H,q, J=7.1Hz), 6.88 (1H, d, J=8.4Hz), 6.95 (2H, d, J=8.8Hz), 7.29 (2H, d, J=8.8Hz), 7.40-7.55 (8H, m).
IR(KBr) ν : 2978, 2868, 1651, 1607, 1516, 1503cm⁻¹.

| Anal. Calcd. for C₃₃H₄₁N₃O₃: | | | |
|---|---|---|---|
| | C, 75.11; | H,7.83; | N,7.96. |
| Found | C,74.90; | H,7.98; | N,7.97. |

### Working Example 283 (Production of Compound 283)

In water/ethanol/toluene (1:1:10, 18.0ml) were dissolved 4-ethylthiophenyl borate (264mg) and 7-bromo-1-methyl-N-[4-[[N-methyl-N-(tetrahydro-2H-pyran -4-yl)amino]methyl]phenyl]-2,3-dihydro-1-benzazepine-4-carboxamide (439mg), and to the mixture was added potassium carbonate (301mg). Under argon atmosphere, the mixture was stirred for 30 minutes, and to the mixture was added tetrakistriphenylphosphine palladium (42mg). Under argon atmosphere, the mixture was refluxed for 17.5 hours, and the mixture was dilute with ethyl acetate (200ml). The mixture was washed with water (50ml) and saturated brine (50ml), and the organic layer was dried with anhydrous magnesium sulfate. The solvent was evaporated under reduced pressure, and the residue was purified with silica gel column chromatography (75g, ethyl acetate→ethyl acetate/ethanol=9:1) and recrystallized from ethanol to give 7-(4-ethylthiophenyl)-1-methyl-N-[4-[[N-methyl-N-(tetra hydro-2H-pyran-4-yl)amino]methyl]phenyl]-2,3-dihydro-1-benzazepine-4-carboxamide (Compound 283) (168mg, 34%).
mp 139-141°C.
¹H NMR (200MHz, CDCl₃) δ 1.34 (3H, t, J=7.3Hz), 1.63-1.76 (4H, m), 2.21 (3H, s), 2.57-2.72 (1H, m), 2.98 (2H, q, J=7.3Hz), 2H around d 2.96 was concealed by d 2.98, 3.10 (3H, s), 3.31-3.43 (4H, m), 3.57 (2H, s), 4.00-4.07 (2H, m), 6.89 (1H, d, J=8.6Hz), 7.28-7.40 (6H, m), 7.466 (1H, dd, J=8.5, 2.3Hz), 7.473 (1H, s), 7.52-7.56 (4H, m).
IR (KBr) 2948, 2845, 1645, 1597, 1514, 1489, 1408, 1314, 1244, 1188, 812 cm⁻¹.

| | | | |
|---|---|---|---|
| Anal. Calcd. for C₃₃H₃₉N₃O₂S : | C, 73.16; | H, 7.26; | N, 7.76. |
| Found: | C, 72.96; | H, 7.08; | N, 7.64. |

### Working Example 284 (Production of Compound 284)

In DMF (10.0ml) was dissolved 7-(4-methylphenyl)-1-[(trifluoromethyl)sulfonyl]-2,3-di hydro-1-benzazepine-4-carboxylic acid (387mg), and to the mixture was added thionyl chloride (0.175ml). The mixture was stirred at room temperature for 1 hour, and excess thionyl chloride and DMF were evaporated under reduced pressure. The residue was dissolved in dichloromethane (10.0ml), and the mixture was added dropwise to a solution of 4-[[N-methyl-N-(tetrahydro-2H-pyran-4-yl)amino]methyl]a niline dihydrochloride (331mg) and triethylamine (0.98ml) in dichloromethane (15.0ml) at 0°C . The mixture was stirred at room temperature for 4 hours, and to the mixture was added water (50ml). The mixture was extracted with dichloromethane (100ml × 3), and the organic layer was dried with anhydrous magnesium sulfate. The solvent was evaporated under reduced pressure, and the residue was purified with silica gel column chromatography (35g, ethyl acetate→ethylacetate/ethanol=9:1) and recrystallized from ethanol to give 7-(4-methylphenyl)-N-[4-[[N-methyl-N-(tetrahydro-2H-pyr an-4-yl)amino]methyl]-phenyl]-1-[(trifluoromethyl)sulfo nyl]-2,3-dihydro-1-benzazepine-4-carboxamide (Compound 284) (251mg, 43%).
mp 185-187°C.
¹H NMR (200MHz, CDCl₃) δ 1.70-1.77 (4H, m), 2.21 (3H, s), 2.41 (3H, s), 2.57-2.72 (1H, m), 3.11 (2H, t, J=5.9Hz), 3.37 (2H, td, J=11.3, 2.9Hz), 3.58 (2H, s), 4.02-4.08 (4H, m), 7.26-7.35 (4H, m), 7.46-7.61 (8H, m), 7.64 (1H, s).
IR (KBr) 1661, 1516, 1497, 1393, 1314, 1223, 1194, 1142, 812 cm⁻¹.

| | | | |
|---|---|---|---|
| Anal. Calcd. for C₃₂H₃₄F₃N₃O₄S: | C, 62.63; | H, 5.58; | N, 6.85. |
| Found: | C, 62.58; | H, 5.57; | N, 6.91. |

### Working Example 285 (Production of Compound 285)

To a solution of 7-(4-methylphenyl)-2,3-dihydrobenzoxepine-4-carboxylic acid (280mg) and 2-[(4-aminophenyl)methylamino]pyridine (199mg) in DMF (4ml) was added, under ice-cooling, diethyl cyanophosphate (0.18ml) and triethylamine (0.17ml), and the mixture was stirred at 0 °C for 30 minutes and then at room temperature for 1 hour. To the mixture was added DMAP (1 piece), and the mixture was stirred at room temperature for 18 hours. Under ice-cooling, to the mixture was added sodium bicarbonate solution, and the mixture was extracted with ethyl acetate, washed with brine, dried (anhydrous magnesium sulfate) and concentrated. The residue was purified with silica gel column chromatography (ethyl acetate/hexane =1/1) and recrystallized from ethyl acetate/hexane to give N- [4-[(pyrid-2-yl)aminomethyl]phenyl]-7-(4-methylphenyl )-2,3-dihydro-1-benzoxepine-4-carboxamide (Compound 285) (97mg) as colorless crystals.
m.p. 189-190°C ¹H-NMR (200MHz, CDCl₃) δ : 2.39 (3H, s), 3.07 (2H, t, J = 4.6), 4.36 (2H, t, J = 4.6), 4.49 (2H, d, J = 4.6), 4.9-5.0 (1H, brm), 6.38 (1H, d, J = 8.4), 6.60 (1H, dd, J = 5.2, 7.2), 7.06 (1H, d, J = 8.4), 7.2-7.6 (12H, m), 8.05-8.15 (1H, m).
IR (KBr) 1651, 1597, 1522, 1491, 1439, 1316, 1254, 812, 772cm⁻¹

| Anal. for C₃₀H₂₇N₃O₂·0.2H₂O | | | |
|---|---|---|---|
| Calcd. | C, 77.46; | H, 5.94; | N, 9.03: |
| Found. | C, 77.24; | H, 5.96; | N, 8.91. |

### Reference Example 277

A solution of p-nitrobenzyl bromide (10g) in THF (50ml) was added dropwise to a solution of bis(2-methoxyethyl)-amine (6.8g) and triethylamine (10ml) in THF (50ml). Under nitrogen atmosphere, the mixture was stirred at room temperature overnight, and the solvent was evaporated. To the residue was added water, and the mixture was extracted with ethyl acetate. The organic layer was washed with water and saturated brine, and dried with anhydrous magnesium sulfate. Under reduced pressure, the solvent was evaporated to give N,N-bis(2-methoxyethyl)-4-nitrobenzylamine (10.8g) as yellow oil.
¹H-NMR(δppm, CDCl₃) 2.76 (4H, t, J=5.6Hz), 3.31 (6H, s), 3.48 (4H, t, J=5.6Hz), 3.83 (2H, s), 7.54 (2H, d, J=8.8Hz), 8.17 (2H, d, J=8.8Hz).
IR(neat) ν : 2878, 1599, 1520cm⁻¹.

### Reference Example 278

In acetic acid (200ml) was dissolved N,N-bis(2-methoxyethyl)-4-nitrobenzylamine (10.5g), and to the mixture was added reduced iron (11g) little by little. The mixture was stirred at room temperature overnight, and the solvent was evaporated. To the residue was added ethyl acetate and precipitates were filtered off. The filtrate was washed with sodium hydroxide solution, water and saturated brine, and dried with anhydrous magnesium sulfate. Under reduced pressure, the solvent was evaporated, and the residue was purified with silica gel column chromatography (ethyl acetate) to give 4-((N,N-bis(2-methoxyethyl)amino)-methyl)aniline (6.2g) as red oil.
¹H-NMR (δ ppm, CDCl₃) 2.71 (4H, t, J=6.3Hz), 3.31 (6H, s), 3.46 (4H, t, J=6.3Hz), 3.59 (2H, s), 6.63 (2H, d, J=8.4Hz), 7.10 (2H, d, J=8.4Hz).
IR(neat) ν : 3353, 2874, 2818, 1615cm⁻¹.

### Reference Example 279

In 1,2-dichloroethane (50ml) were dissolved p-nitrobenzaldehyde (5g) and 3-ethoxypropylamine (3.75g), and to the mixture was added, under ice-cooling, triacetoxy sodium boro hydride (9.8g). Under nitrogen atmosphere, the mixture was stirred at room temperature overnight, and to the mixture were added, under ice-cooling, 37% formalin (3.5ml) and triacetoxy sodium boro hydride (9.8g). Under nitrogen atmosphere, the mixture was stirred at room temperature for 8 hours, and the solvent was evaporated. The residue was neutralized with 1N sodium hydroxide solution, and the mixture was extracted with ethyl acetate. The organic layer was washed with water and subjected to back extraction with 1N hydrochloric acid. The mixture was washed with ethyl acetate, neutralized with 1N sodium hydroxide and extracted with ethyl acetate. The organic layer was washed with water and saturated brine, and dried with anhydrous magnesium sulfate. Under reduced pressure, the solvent was evaporated to give N-(3-ethoxypropyl)-N-methyl-4-nitrobenzylamine (6.6g) as yellow oil.
¹H-NMR(δppm, CDCl₃) 1.18 (3H, t, J=7.0Hz), 1.72-1.86 (2H, m), 2.20 (3H, s), 2.48 (2H, t, J=7.6Hz) 3.41-3.52 (4H, m), 3.58 (2H, s), 7.50 (2H, d, J=8.8Hz), 8.17 (2H, d, J=8.8Hz). IR(neat) ν : 2859, 1520, 1346cm⁻¹.

### Reference Example 280

In THF (60ml) were suspended N-(3-ethoxypropyl)-N-methyl-4-nitrobenzylamine (6.0g), iron chloride (III) (0.06g) and active charcoal (0.6g), and to the suspension was added dropwise hydrazine monohydrate (4.1ml) at 60-65°C. The mixture was stirred at 65°C for 4 hours, and to the mixture was added hydrazine monohydrate (15ml). The mixture was stirred at 65°C for 4 hours and filtered. The solvent of the filtrate was evaporated, and the residue was extracted with ethyl acetate. The organic layer was washed with saturated brine and dried with anhydrous magnesium sulfate, and the solvent was evaporated. The residue was dissolved in 2-propanol, and to the mixture was added hydrochloric acid (6ml). The solvent was evaporated, and the precipitated 4-((N-(3-ethoxypropyl)-N-methylamino)-methyl)aniline dihydrochloride (5.8g) was filtered with ethyl acetate and washed with ethyl acetate-hexane to give yellow powder.
mp 173-175°C.
¹H-NMR(δppm, CDCl₃+CD₃OD) 1.16 (3H, t, J=7.0Hz), 2.18 (2H, br), 2.72 (3H, s), 3.05-3.29 (2H, m), 3.40-3.52 (4H, m), 4.22-4.43 (2H, m), 7.58 (2H, d, J=8.2Hz), 7.78 (2H, d, J=8.2Hz), 11.86 (1H, br).
IR (KBr) ν : 1651cm⁻¹.

| Anal. Calcd. for C₁₃H₂₂N₂O·2HCl : | | | |
|---|---|---|---|
| | C,52.88; | H,8.19; | N,9.49. |
| Found | C,52.61; | H,8.05; | N,9.55. |

### Reference Example 281

In 1,2-dichloroethane (50ml) were suspended p-nitrobenzylamine hydrochloride (3g), 1,3-dimethoxyacetone (1.9g) and triethylamine (2.2ml), and to the mixture was added, under ice-cooling, triacetoxy sodium boro hydride (4.7g). Under nitrogen atmosphere, the mixture was stirred at room temperature for 5 hours, and to the mixture were added, under ice-cooling, 37%formalin (1.8ml) and triacetoxy sodium boro hydride (5g). Under nitrogen atmosphere, the mixture was stirred at room temperature overnight, and the solvent was evaporated. The residue was neutralized with 1N sodium hydroxide solution and extracted with ethyl acetate. The organic layer was washed with water and saturated brine, and dried with anhydrous magnesium sulfate. Under reduced pressure, the solvent was evaporated, and the residue was purified with silica gel column (ethyl acetate/hexane) to give N-(1,3-dimethoxy-propan-2-yl)-N-methyl-4-nitrobenzylami ne (3.2g) as yellow oil. ¹H-NMR (δ ppm, CDCl₃) 2.32 (3H, s), 2. 97-3 .09 (1H, m), 3.36 (6H, s) 3.44-3.63 (4H, m), 3.85 (2H, s), 7.53 (2H, d, J=9.0Hz), 8.17 (2H, d, J=9.0Hz).
IR (neat) ν : 2880, 1520, 1346cm⁻¹.

### Reference Example 282

In acetic acid (100ml) was dissolved N-(1,3-dimethoxypropan-2-yl)-N-methyl-4-nitrobenzylamine (3.1g), and to the mixture was added reduced iron (3.2g) little by little. The mixture was stirred at room temperature overnight, and the solvent was evaporated. To the residue was added ethyl acetate, and precipitates were filtered off. The filtrate was washed with sodium hydroxide solution, water and saturated brine, and dried with anhydrous magnesium sulfate. Under reduced pressure, the solvent was evaporated, and the residue dissolved in ethyl acetate. To the mixture was added 4N hydrochloric acid-ethyl acetate, and precipitates were filtered and washed with diethylether. The mixture was dissolved in water, and the mixture was neutralized with 1N sodium hydroxide solution and extracted with ethyl acetate. The organic layer was washed with water and saturated brine, and dried with anhydrous magnesium sulfate. Under reduced pressure, the solvent was evaporated to give 4-((N-(1,3-dimethoxypropan-2-yl)-N-methylamino)methyl)-aniline (2.4g) as red oil.
¹H-NMR(δ ppm, CDCl₃) 2.29 (3H, s), 2.95-3.07 (1H, m), 3.34 (6H, s), 3.42-3.58 (4H, m), 3.61 (2H, s), 6.64 (2H, d, J=8.4Hz), 7.11 (2H, d, J=8.4Hz).
IR(neat) ν : 3357, 2880, 1615, 1518cm⁻¹.

### Reference Example 283

In 1,2-dichloroethane (50ml) were dissolved p-nitrobenzaldehyde (5g) and 2-methoxyethylamine (2.7g), and to the mixture was added, under ice-cooling, triacetoxy sodium boro hydride (9.8g). Under nitrogen atmosphere, the mixture was stirred at room temperature for 4 hours, and to the mixture were added, under ice-cooling, 37% formalin (3.8ml) and triacetoxy sodium boro hydride (10g). Under nitrogen atmosphere, the mixture was stirred at room temperature overnight, and the solvent was evaporated. The residue was neutralized with 1N sodium hydroxide solution and extracted with ethyl acetate. The organic layer was washed with water and saturated brine, and dried with anhydrous magnesium sulfate. Under reduced pressure, the solvent was evaporated, and the residue was purified with silica gel column (ethyl acetate/hexane) to give N-(2-methoxyethyl)-N-methyl-4-nitrobenzylamine (5.9g) as yellow oil.
¹H-NMR(δ ppm, CDCl₃) 2.28 (3H, s), 2.63 (2H, t, J=5. 6Hz) 3.35 (3H, s), 3.52 (2H, t, J=5.6Hz), 3.65 (2H, s) 7.52 (2H, d, J=8.8Hz), 8.18 (2H, d, J=8.8Hz).
IR(neat) ν: 2814, 1605, 1520, 1346cm⁻¹.

### Reference Example 284

In acetic acid (100ml) was dissolved N-(2-methoxy-ethyl)-N-methyl-4-nitrobenzylamine (5.9g), and to the mixture was added reduced iron (7.5g) little by little. The mixture was stirred at room temperature overnight, and the solvent was evaporated. To the residue was added ethyl acetate, and precipitates were filtered off. The filtrate was washed with sodiumhydroxide solution, water and saturated brine, and dried with anhydrous magnesium sulfate. Under reduced pressure, the solvent was evaporated to give 4-((N-(2-methoxyethyl)-N-methylamino)methyl)aniline (3.4g) as red oil.
¹H-NMR (δ ppm, CDCl₃) 2.24 (3H, s), 2.57 (2H, t, J=6.0Hz), 3.33 (3H, s), 3.44 (2H, s), 3.50 (2H, t, J=6.0Hz), 6.64 (2H, d, J=8 .4Hz), 7.09 (2H, d, J=8.4Hz).
IR(neat) ν : 3349, 2813, 1615, 1518cm⁻¹.

### Reference Example 285

In THF (350ml) was dissolved 5-bromoanthranilic acid (40.06g), and the mixture was cooled to 0°C. To the mixture was added dropwise a solution of 10.0M borane dimethyl-sulfide in THF (54.5ml), and the mixture was stirred at room temperature for 4.5 hours. The mixture was cooled to 0°C, and to the mixture was added dropwise 3N sodium hydroxide solution. The mixture was stirred at room temperature overnight, and to the mixture was added granulated sodium hydroxide to adjust the mixture to pH 11. The aqueous layer was saturated with potassium carbonate, and the THF layer was separated. The aqueous layer was extracted with ether (100ml×5). The organic layers were combined and dried with magnesium sulfate. The solvent was evaporated under reduced pressure to give (2-amino-5-bromophenyl)methanol (36.66g, 100%).
¹H NMR (200MHz, CDCl₃) δ 4.62 (2H, s), 7.20 (1H, s), 7.23-7.26 (1H, m).

### Reference Example 286

To acetone (300ml) were added (2-amino-5-bromophenyl)methanol (23.32g) and active manganese dioxide (58.5g), and the mixture was stirred at room temperature for 17.5 hours and filtered. The solvent was evaporated under reduced pressure to give 2-amino-5-bromobenzaldehyde (16.41g, 71%).
¹H NMR (200MHz, CDCl₃) δ 6.10-6.20 (2H, br), 6.57 (1H, d, J=8.8Hz), 7.38 (1H, dd, J=8.8, 2.4Hz), 7.59 (1H, d, J=2.4Hz), 9.81 (1H, s).

### Reference Example 287

To acetic acid anhydride (34.8ml) was added formic acid (17.0ml) at 0°C, and the mixture was stirred at 60°C for 2 hours, cooled and diluted with THF (200ml). In THF (100ml) was dissolved 2-amino-5-bromobenzaldehyde (16.40g), and the mixture was added dropwise to the previously prepared solution of formic acid anhydride in THF at 0°C. The mixture was stirred at 0°C for 2 hours, and the solvent was evaporated under reduced pressure. The residue was washed with hexane and filtered to give 4-bromo-2-formylphenylformamide (15.24g, 82%).
¹H NMR (200MHz, CDCl₃) δ 7.72 (1H, dd, J=8.8, 2.6Hz), 7.83 (1H, d, J=2.6Hz), 8.53 (1H, s), 8.68 (1H, d, J=9.2Hz), 9.88 (1H, s), 10.94 (1H, br).

### Reference Example 288

To 4-bromo-2-formylphenylformamide (18.07g), ethyl 4-bromobutyrate (30.9g) and potassium carbonate (21.9g) was added DMF (160ml), and the mixture was stirred at 70°C for 24 hours. The mixture was dilute with ethyl acetate (1400ml), washed with water (300ml ×3) and saturated brine (150ml), and dried with magnesium sulfate. The solvent was evaporated under reduced pressure, and the residue was purified with silica gel column chromatography (300g, hexane:ethyl acetate=4:1→1:1) to give ethyl 4-(4-bromo-2,N-diformylanilino)butyrate (21.56g, 80%). ¹H NMR (200MHz, CDCl₃) (syn:anti=5:2 or 2:5) δ 1.23 (2.1H, t, J=7.2Hz), 1.25 (0.9H, t, J=7.2Hz), 1.87 (2H, quint, J=7.5Hz), 2.35 (1.4H, t, J=7.3Hz), 2.36 (0.6H, t, J=6.8Hz), 3.78 (0.6H, t, J=7.5Hz), 3.85 (1.4H, t, J=7.6Hz), 4.10 (1.4H, q, J=6. 9Hz), 4.15 (0.6H, q, J=7.2Hz), 7.17 (0.3H, d, J=8.4Hz), 7.24 (0.7H, d, J=8.6Hz), 7.81 (0.3H, dd, J=8.4, 2.4Hz), 7.82 (0.7H, dd, J=8.4, 2.4Hz), 8.09 (0.3H, d, J=2.4Hz), 8.10 (0.7H, d, J=2.4Hz), 8.19 (0.7H, s), 8.39 (0.3H, s), 9.92 (0.3H, s), 10.04 (0.7H, s).

### Reference Example 289

In t-butanol (500ml) were dissolved ethyl 4-(4-bromo-2,N-diformylanilino)butyrate (15.32g) and potassium t-butoxide (5.53g), and the mixture was refluxed for 30 minutes. To the mixture were added water (500ml) and 1N hydrochloric acid (50ml), and the mixture was extracted with ethyl acetate (1000ml). The organic layer was washed with saturated brine (200ml) and dried with magnesium sulfate. The solvent was evaporated under reduced pressure, and the residue was purified with silica gel column chromatography (300g, hexane:ethyl acetate=4:1→1:1) to give ethyl 7-bromo-1-formyl-2,3-dihydro-1-benzazepine-4-carboxylat e (3.13g, 22%) and 7-bromo-1-formyl-2,3-dihydro-1-benzazepine-4-carboxylic acid (1.39g, 10%).
Ethyl
7-bromo-1-formyl-2,3-dihydro-1-benzazepine-4-carboxylat e;
mp 150.5-152°C.
¹H NMR (200MHz, CDCl₃) δ 1.34 (3H, t, J=7.1Hz), 2.93 (2H, t, J=4.9Hz), 3.80 (2H, t, J=5.7Hz), 4.28 (2H, q, J=7.2Hz), 7.00 (1H, d, J=8.4Hz), 7.50 (1H, dd, J=8.4, 2.2Hz), 7.57 (1H, s), 7.66 (1H, d, J=2.2Hz), 8.46 (1H, s).
IR (KBr) 1707, 1678, 1491, 1358, 1265, 1235, 1194, 1088 cm⁻¹.

| | | | |
|---|---|---|---|
| Anal. Calcd. for C₁₉H₁₄NO₃Br: | C, 51.87; | H, 4.35; | N, 4.32. |
| Found: | C, 51.81; | H, 4.35; | N, 4.19. |

7-Bromo-1-formyl-2,3-dihydro-1-benzazepine-4-carboxylic acid;
mp 248-249.5°C.
¹H NMR (200MHz, DMSO-d₆) δ 2.73 (2H, td, J=5.1, 1.2Hz), 3.67 (2H, t, J=5.9Hz), 7.33 (1H, d, J=8.4Hz), 7.57 (1H, s), 7.61 (1H, dd, J=8.4, 2.6Hz), 7.91 (1H, d, J=2.4Hz), 8.48 (1H, s).
IR (KBr) 1665, 1491, 1431, 1360, 1300, 1281, 1252, 1196, 999, 918, 841, 754 cm⁻¹.

| | | | |
|---|---|---|---|
| Anal. Calcd. for C₁₂H₁₀NO₃Br: | C, 48.67; | H, 3.41; | N, 4.73. |
| Found: | C, 48.70; | H, 3.56; | N, 4.54. |

### Reference Example 290

In 1N sodium hydroxide (13.0ml) and THF:ethanol (1:1, 50ml) was dissolved ethyl 7-bromo-1-formyl-2,3-dihydro-1-benzazepine-4-carboxylat e (2.77g), and the mixture was stirred at room temperature for 15 hours. To the mixture was added 1N hydrochloric acid (12.5ml), and the mixture was concentrated. To the residue was added water (200ml), and the mixture was adjusted to pH 2 with 1N hydrochloric acid. The mixture was extracted with ethyl acetate (300 ml×3), and the organic layer was dried with magnesium sulfate. The solvent was evaporated under reduced pressure to give 7-bromo-1-formyl-2,3-dihydro-1-benzazepine-4-carboxylic acid (2.52g, 100%).

### Reference Example 291

To a solution of 7-bromo-1-formyl-2,3-dihydro-1-benzazepine-4-carboxylic acid (3 .28g) inDMF (30ml) was addeddropwise thionyl chloride (2.0ml) at 0°C, and the mixture was stirred at room temperature for 30 minutes.
Under reduced pressure, thionyl chloride and DMF were evaporated, and the residue was dissolved in dichloromethane (40ml). To a solution of 4-[[N-methyl-N-(tetrahydro-2H-pyran-4-yl)amino]methyl]a niline (3.90g) and triethylamine (11.6ml) in dichloromethane (40ml) was added dropwise the previously prepared chloride solution at 0°C, and the mixture was stirred at room temperature for 7 hours. The mixture was concentrated under reduced pressure, and the residue was diluted with ethyl acetate (400ml), washed with water (100m1X2) and saturated brine (50ml), and dried with magnesium sulfate. The solvent was evaporated under reduced pressure, and the residue was purified with silica gel column chromatography (200g, ethyl acetate→ethyl acetate/ethanol=10:1) to give 7-bromo-1-formyl-N-[4-[[N-methyl-N-(tetrahydro-2H-pyran -4-yl)amino]methyl]-phenyl]-2,3-dihydro-1-benzazepine-4 -carboxamide (2.13g, 39%).
mp 173-175°C.
¹H NMR (200MHz, CDCl₃) δ 1.66-1.77 (4H, m), 2.21 (3H, s), 2.58-2.73 (1H, m), 3.02 (2H, t, J=4.8Hz), 3.37 (2H, td, J=10.3, 2.9Hz), 3.58 (2H, s), 3.87 (2H, t, J=5.5Hz), 4.02-4.08 (2H, m), 7.03 (1H, d, J=8.4Hz), 7.32 (2H, d, J=8.4Hz), 1H was concealed under 7.27-7.34, 7.50 (1H, s), 7.51 (1H, dd, J=8.5, 2.3Hz), 7.52 (2H, d, J=8.4Hz), 7.65 (1H, d, J=2.2Hz), 8.49 (1H, s).
IR (KBr) 2953, 2845, 1669, 1599, 1520, 1358, 1316, 1260, 1192, 733 cm⁻¹.

| | | | |
|---|---|---|---|
| Anal. Calcd. for C₂₅H₂₈N₃O₃Br: | C, 60.24; | H, 5.66; | N, 8.43. |
| Found: | C, 60.15; | H, 5.69; | N, 8.49. |

### Reference Example 292

To t-butyl 7-bromo-1-methyl-2,3-dihydro-1-benzazepine-4-carboxylat e (4.0g), 4-ethoxyphenyl borate (2.35g), 1M potassium carbonate solution (25ml) and ethanol (25ml) was added toluene (100ml), and the mixture was stirred under argon atmosphere at room temperature for 30 minutes. To the mixture was added tetrakistriphenylphosphine palladium (0.55g), and the mixture was refluxed under argon atmosphere overnight. The organic layer was washed with water and saturated brine, and dried with anhydrous magnesium sulfate. Under reduced pressure, the solvent was evaporated, and the residue was purified with silica gel column (ethyl acetate/hexane) to give t-butyl 7-(4-ethoxyphenyl)-1-methyl-2,3-dihydro-1-benzazepine-4 -carboxylate (4.0g) as yellow crystals.
mp 140-142°C.
¹H-NMR(δ ppm, CDCl₃) 1.43 (3H, t, J=7.0Hz), 1.54 (9H, s), 2.82 (2H, t, J=4.8Hz), 3.05 (3H, s), 3.27 (2H, t, J=4.8Hz), 4.07 (2H,q,J=7.0Hz), 6.83 (1H, d, J=8.4Hz), 6.95 (2H, d, J=8.8Hz), 7.38-7.49 (4H, m), 7.66 (1H, s).
IR (KBr) n: 2978, 1694cm⁻¹.

| Anal. Calcd. for C₂₄H₂₉NO₃: | | | |
|---|---|---|---|
| | C, 75.96 ; | H,7.70; | N,3.69. |
| Found | C,75.91; | H,7.89; | N,3.49. |

### Reference Example 293

In dimethoxyethane (100ml) was dissolved t-butyl 7-(4-ethoxyphenyl)-1-methyl-2,3-dihydro-1-benzazepine-4 -carboxylate (4.0g), and to the mixture was added 6N hydrochloric acid (25ml). The mixture was refluxed for 3 hours, and the solvent was evaporated. Precipitated yellow powder was filtered and washed with ethyl acetate-hexane to give 7-(4-ethoxyphenyl)-1-methyl-2,3-dihydro-1-benzazepine-4 -carboxylic acid hydrochloride (3.8g).
mp 245-254°C (dec.).
¹H-NMR (δ ppm, DMSO-d₆) 1.35 (3H, t, J=7.0Hz), 2.77 (2H,br), 3.02 (3H, s), 3.25 (2H,br), 4.05 (2H,q,J=7.0Hz), 6.94-6.98 (3H, m), 7.49-7.68 (5H, m).
IR(KBr) ν : 2976, 2880, 2475, 1701cm⁻¹.

### Reference Example 294

In 1N hydrochloric acid (25ml) and ethanol (20ml) was dissolved ethyl 7-bromo-1-formyl-2,3-dihydro-1-benzazepine-4-carboxylat e (1165mg), and the mixture was refluxed for 2 hours. The mixture was neutralized with saturated sodium hydrogen carbonate solution, and the mixture was extracted with ethyl acetate (300ml). The organic layer was washed with water (100ml) and dried with anhydrous magnesium sulfate. The solvent was evaporated under reduced pressure, and the residue was purified with silica gel column chromatography (150g, hexane/ethyl acetate=9:1) to give ethyl 7-bromo-2,3-dihydro-1-benzazepine-4-carboxylate (628mg, 59%).
mp 120-121°C.
¹H NMR (200MHz, CDCl₃) δ 1.34 (3H, t, J=7.1Hz), 2.86 (2H, td, J=4.8, 1.2Hz), 3.36 (2H, t, J=4.8Hz), 4.25 (2H, q, J=7.1Hz), 4.51-4.66 (1H, br), 6.49 (1H, d, J=8.8Hz), 7.15 (1H, dd, J=8.7, 2.3Hz), 7.39 (1H, d, J=2.2Hz), 7.53 (1H, s).
IR (KBr) 3377, 2978, 1694, 1493, 1248, 1209, 1173, 1090, 812 cm⁻¹.

| | | | |
|---|---|---|---|
| Anal. Calcd. for C₁₃H₁₄BrNO₂ : | C, 52.72; | H, 4.76; | N, 4.73. |
| Found: | C, 52.54; | H, 4.88; | N, 4.60. |

### Reference Example 295

In dichloromethane (30ml) were dissolved 7-bromo-2,3-dihydro-1-benzazepine-4-carboxylic acid ethyl (457mg) and triethylamine (1.29ml), and to the mixture was added dropwise at 0°C trifluoromethanesulfonic acid anhydride (1.56ml). The mixture was stirred at 0°C for 4 hours, and to the mixture was added water (50ml) at 0°C. The mixture was extracted with dichloromethane (100ml), and the organic layer was dried with anhydrous magnesium sulfate. The solvent was evaporated under reduced pressure, and the residue was purified with silica gel column chromatography (50g, hexane/ethylacetate=9:1) to give ethyl 7-bromo-1-[(trifluoromethyl)sulfonyl]-2,3-dihydro-1-ben zazepine-4-carboxylate (516mg, 78%).
¹H NMR (200MHz, CDCl₃) δ 1.36 (3H, t, J=7.5Hz), 3.00 (2H, t, J=6.0Hz), 3.91-4.03 (2H, m), 4.30 (2H, q, J=7.2Hz), 7.38 (1H, d, J=8.4Hz), 7.45 (1H, dd, J=8.8, 2.2Hz), 7.63 (1H+1H, s).
IR (KBr) 2982, 1713, 1487, 1397, 1252, 1227, 1194, 1142, 1100, 1090, 700, 627 cm-¹.

### Reference Example 296

In water/ethanol/toluene (1:1:10, 36.0ml) 4-methylphenyl borate (194mg) and ethyl 7-bromo-1-[(trifluoromethyl)sulfonyl]-2,3-dihydro-1-ben zazepine-4-carboxylate (510mg) were dissolved, and to the mixture was added potassium carbonate (395mg). The mixture was stirred under argon atmosphere for 30 minutes, and to the mixture was added tetrakistriphenylphosphine palladium (138mg). Under argon atmosphere, the mixture was refluxed for 17 hours, and the mixture was diluted with ethyl acetate (150ml) and washed with water (50ml) and saturated brine (50ml) . The organic layer was dried with anhydrous magnesium sulfate. The solvent was evaporated under reduced pressure, and the residue was purified with silica gel column chromatography (50g, hexane/ethylacetate=9:1) to give ethyl 7-(4-methylphenyl)-1-[(trifluoromethyl)-sulfonyl]-2,3-d ihydro-l-benzazepine-4-carboxylate (469mg, 90%).
¹H NMR (200MHz, CDCl₃) δ 1.37 (3H, t, J=7.2Hz), 2.41 (3H, s), 3.02 (2H, t, J=6.0Hz), 3.99-4.05 (2H, m), 4.31 (2H, q, J=7.1Hz), 7.27 (2H, d, J=8.0Hz), 7.43-7.56 (4H, m), 7.60-7.68 (1H, m), 7.77 (1H, s).
IR (KBr) 2982, 1709, 1495, 1395, 1246, 1225, 1192, 1152, 1096, 812, 642, 588 cm⁻¹.

### Reference Example 297

In 1N sodium hydroxide solution (3.0ml) and THF/ethanol (1:1, 12.0ml) was dissolved 7-(4-methylphenyl)-1-[(trifluoromethyl)sulfonyl]-2,3-di hydro-l-benzazepine-4-carboxylic acid ethyl(463mg), and the mixture was stirred at room temperature for 14 hours. The mixture was neutralized with 1N hydrochloric acid (3.5ml) and concentrated. To the residue was added water (40ml), and the mixture was extracted with ethyl acetate (100ml ×3).
The organic layer was dried with anhydrous magnesium sulfate, and the solvent was evaporated under reduced pressure to give 7-(4-methylphenyl)-1-[(trifluoromethyl)sulfonyl]-2,3-di hydro-1-benzazepine-4-carboxylic acid (393mg, 91%). ¹HNMR (200MHz, DMSO-d₆) δ 2.39 (3H, s), 2. 94 (2H, t, J=6.2Hz), 4.00-4.08 (2H, m), 7.28 (2H, d, J=8.6Hz), 7.41-7.49 (1H, m), 7.56 (2H, d, J=8.4Hz), 7.61-7.66 (1H, m), 7.73-7.77 (1H, m), 8.00 (1H, s).

### Reference Example 298

To a solution of 4-nitrobenzaldehyde (3.02g) and 2-aminopyridine (1.88g) in 1,2-dichloroethane (70ml) were added triacetoxy sodium boro hydride (5.93g) and acetic acid (1.14ml), and the mixture was stirred under nitrogen atmosphere at room temperature for 2 hours and concentrated. To the residue was added sodium bicarbonate solution, and the mixture was extracted with ethyl acetate, washed with brine, dried (anhydrous magnesium sulfate) and concentrated. The residue was purified with silica gel column chromatography (ethyl acetate/hexane =1/1), and to the purified materials were added ethyl acetate/diethylether and 1N hydrochloric acid. The aqueous layer was extracted and washed with diethylether, and to the mixture was added sodium carbonate. The mixture was extracted with ethyl acetate, and the extract was dried (anhydrous magnesium sulfate), concentrated and recrystallized from ethyl acetate/hexane to give 2-[(4-nitrophenyl)methylamino]-pyridine (1.63g) as pale yellow crystals.
m.p. 131-132°C
¹H-NMR (200MHz, CDCl₃) δ : 4.67 (2H, d, J = 6.0), 4.9-5.1 (1H, brm), 6.37 (1H, d, J = 8.4), 6.63 (1H, dd, J = 5.1, 6.9), 7.35-7.45 (1H, m), 7.52 (2H, d, J = 8.8), 8.15-8.25 (1H, m), 8.18 (2H, d, J = 8.8).
IR (KBr) 1601, 1516, 1460, 1348, 1281, 1159, 999, 772cm⁻¹

| Anal for C₁₂H₁₁N₃O₂ | | | |
|---|---|---|---|
| Calcd. | C, 62.87; | H, 4.84; | N, 18.33: |
| Found. | C, 62.69; | H, 4.69; | N, 18.20. |

### Reference Example 299

To a solution of nickel bromide (44mg) in methanol (4ml)/THF (4ml) was added sodium boro hydride (40mg), and the mixture was stirred. To the mixture was added 2-[(4-nitrophenyl)methylamino]pyridine (0.92g) and then sodium boro hydride (414mg), and the mixture was stirred at room temperature for 1 hour. To the mixture was added nickel bromide (44mg)and sodium boro hydride (454mg), and the mixture was stirred at room temperature for 2 hours. Insoluble materials were filtered off with sellaite, and to the filtrate was added sodium bicarbonate solution. The mixture was extracted with ethyl acetate and washed with brine. The extract was dried (anhydrous magnesium sulfate) and concentrated, and the residue was purified twice with silica gel column chromatography (ethyl acetate/hexane =1/1) to give 2- [(4-aminophenyl)methylamino]pyridine (369mg) as pale red solid.
¹H-NMR (200MHz, CDCl₃) δ : 3.4-3.8 (2H, br), 4.36 (2H, d, J = 5.2), 4.7-4.85 (1H, br), 6.37 (1H, d, J = 8.4), 6.58 (1H, dd, J = 5.2, 8.0), 6.66 (2H, d, J = 8.4), 7.15 (2H, d, J = 8.4), 7.35-7.45 (1H, m), 8.05-8.15 (1H, m).
IR (KBr) 1603, 1578, 1514, 1443, 1335, 1294, 1159, 818, 770 cm⁻¹

### Industrial Applicability

The compound of the formula (I) or a salt thereof of the present invention has potent antagonistic activity on MCP-1 receptor and can be advantageously used for the treatment or prophylaxis of various inflammatory diseases in human and animals, cardiac infarction, myocarditis, etc.

## Claims

1. A compound of the formula: 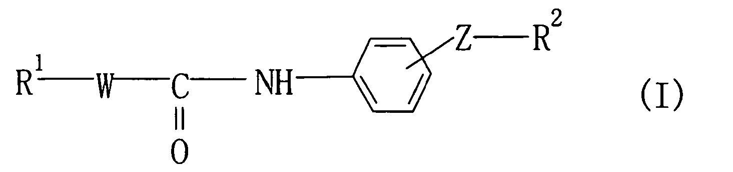 wherein R¹ is an optionally substituted 5- to 6-membered ring, W is a divalent group of the formula: 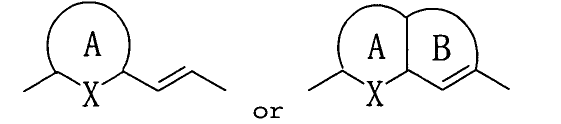 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring, Z is a methylene group, R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 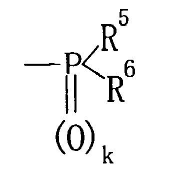 wherein k is 0 or 1, and when k is 0, a phosphorus atom may form a phosphonium; and R⁵ and R⁶ are independently an optionally substituted hydrocarbon group or an optionally substituted amino group, and R⁵ and R⁶ may bind to each other to form a cyclic group together with the adjacent phosphorus atom, or a salt thereof.

2. A compound according to claim 1, wherein R¹ is benzene, furan, thiophene, pyridine, cyclopentane, cyclohexane, pyrrolidine, piperidine, piperazine, morpholine, thiomorpholine or tetrahydropyran, each of which may be substituted.

3. A compound according to claim 1, wherein R¹ is an optionally substituted benzene.

4. A compound according to claim 1, wherein the ring A is furan, thiophene, pyrrole, pyridine or benzene, each of which may be substituted.

5. A compound according to claim 1, wherein the ring A is an optionally substituted benzene.

6. A compound according to claim 1, wherein W is a group of the formula: 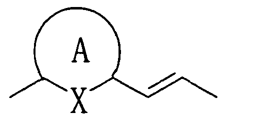 wherein each symbol is as defined in claim 1.

7. A compound according to claim 1, wherein W is a group of the formula: 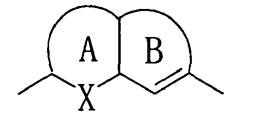 wherein each symbol is as defined in claim 1.

8. A compound according to claim 7, wherein the ring B is a 5- to 7-membered ring group of the formula: 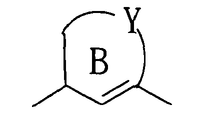 wherein Y is -Y'-(CH₂)ₘ- (Y' is -S-, -O-, -NH- or -CH₂-, and m is an integer of 0-2), -CH=CH- or -N=CH-, which may have a substituent at any possible position.

9. A compound according to claim 8, wherein Y is -Y'- (CH₂)₂-(Y' is -S-, -O-, -NH- or -CH₂-).

10. A compound according to claim 8, wherein Y is - (CH₂)₂-, - (CH₂)₃- or -O-(CH₂)₂-.

11. A compound according to claim 10, wherein the ring A is an optionally substituted benzene.

12. A compound according to claim 1, wherein Z is substituted at para position of the benzene ring.

13. A compound according to claim 1, wherein R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium, or (3) a group of the formula: 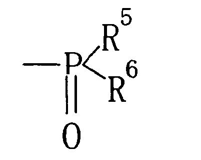 wherein R⁵ and R⁶ are independently an optionally substituted hydrocarbon group, and R⁵ and R⁶ may bind to each other to form a cyclic group together with the adjacent phosphorus atom.

14. A compound of the formula: 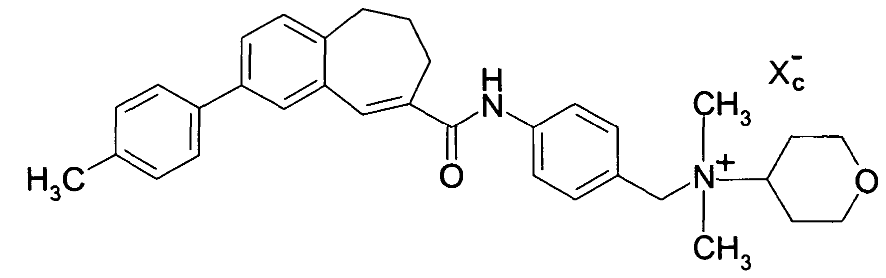 according to claim 1 wherein X_{c}⁻ is an anion.

15. A compound according to claim 14, wherein X_{c} is a halogen atom.

16. A compound according to claim 1 selected from the class consisting of
N-methyl-N-[4-[[[2-(4-methylphenyl)-6,7-dihydro-5H-benz ocyclohepten-8-yl] carbonyl] amino] benzyl] -piperidinium iodide,
N-methyl-N-[4-[[[7-(4-methylphenyl)-2,3-dihydro-1-benzo xepin-4-yl]carbonyl]amino]benzyl]piperidinium iodide, N-[4-[N-methyl-N-(tetrahydropyran-4-yl)aminomethyl]-phe nyl]-7-(4-methylphenyl)-2,3-dihydro-1-benzoxepine-4-car boxmide,
N-[4-[N-methyl-N-(tetrahydropyran-4-yl)aminomethyl]-phe nyl]-7-(4-morpholinophenyl)-2,3-dihydro-1-benzoxepine-4 -carboxmide,
7-(4-ethoxyphenyl)-N-[4-[N-methyl-N-(tetrahydropyran-4-yl)aminomethyl]phenyl]-2,3-dihydro-1-benzoxepine-4-carb oxmide,
N,N-dimethyl-N-[4-[[[2-(4-methylphenyl)-6,7-dihydro-5H-benzocyclohepten-8-yl]carbonyl]amino]benzyl]-N-(tetrahy dropyran-4-yl)ammonium iodide and
N-methyl-N-[4-[[[7-(4-methylphenyl)-3,4-dihydro-naphtha len-2-yl]carbonyl]amino]benzyl]piperidinium iodide, or a salt thereof.

17. A method for producing a compound of the formula: 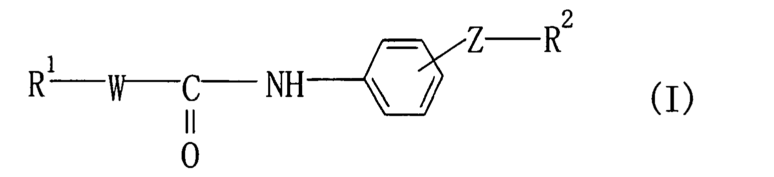 according to claim 1 wherein each symbol is as defined in claim 1 or a salt thereof, which comprises subjecting a compound of the formula:
R¹-W-COOH (II)
wherein each symbol is as defined in claim 1, a salt or a reactive derivative thereof to condensation reaction with a compound of the formula: 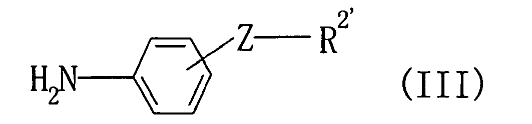 wherein Z is as defined in claim 1 and R²' is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 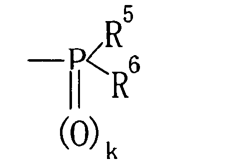 wherein k is 0 or 1, and when k is 0, a phosphorus atom may form a phosphonium; and R⁵ and R⁶ are independently an optionally substituted hydrocarbon group or an optionally substituted amino group, and R⁵ and R⁶ may bind to each other to form a cyclic group together with the adjacent phosphorus atom, the above groups (1) - (3) being optionally protected, or a salt thereof, and, if desired, subjecting the obtained product to deprotection, oxidation, reduction and/or ammoniumation.

18. A method according to claim 17 wherein the compound of the formula R¹-W-COOH is 3-(4-methylphenyl)-8,9-dihydro-7H-benzocyclo-heptene-6-carboxylic acid or a salt thereof.

19. A pharmaceutical composition comprising a compound according to claim 1 or a salt thereof.

20. A composition according to claim 19, which is for antagonizing MCP-1 receptor.

21. A composition according to claim 19, which is for the treatment or prophylaxis of cardiac infarction or myocarditis.

22. A pharmaceutical composition for antagonizing MCP-1 receptor, which comprises a compound of the formula: 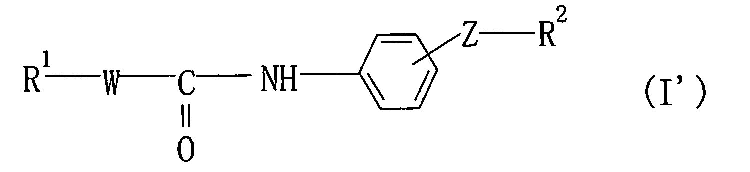 wherein R¹ is an optionally substituted 5- to 6-membered ring, W is a divalent group of the formula: 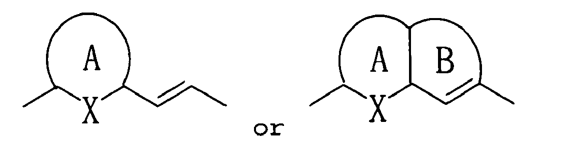 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring, Z is a methylene group, R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 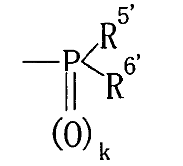 wherein k is 0 or 1, and when k is 0, a phosphorus atom may form a phosphonium; and R^{5'} and R^{6'} are independently an optionally substituted hydrocarbon group, an optionally substituted hydroxy group or an optionally substituted amino group, and R⁵' and R⁶' may bind to each other to form a cyclic group together with the adjacent phosphorus atom, or a salt thereof.

23. Use of a compound of the formula: 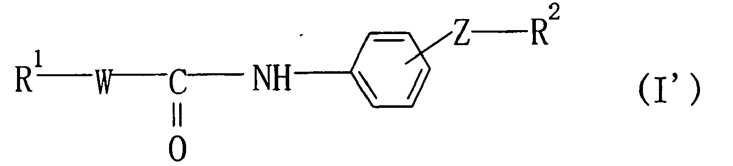 wherein R¹ is an optionally substituted 5- to 6-membered ring; W is a divalent group of the formula: 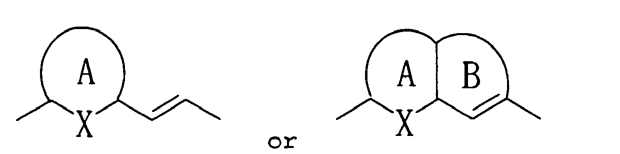 wherein the ring A is an optionally substituted 5- to 6-membered aromatic ring, X is an optionally substituted carbon atom, an optionally substituted nitrogen atom, sulfur atom or oxygen atom, the ring B is an optionally substituted 5- to 7-membered ring; Z is a methylene group; R² is (1) an optionally substituted amino group in which a nitrogen atom may form a quaternary ammonium, (2) an optionally substituted nitrogen-containing heterocyclic ring group which may contain a sulfur atom or an oxygen atom as ring constituting atoms and wherein a nitrogen atom may form a quaternary ammonium or (3) a group of the formula: 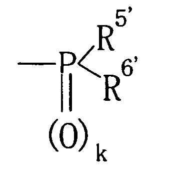 wherein k is 0 or 1, and when k is 0, a phosphorus atom may form a phosphonium; and R⁵' and R⁶' are independently an optionally substituted hydrocarbon group, an optionally substituted hydroxy group or an optionally substituted amino group, and R⁵' and R⁶' may bind to each other to form a cyclic group together with the adjacent phosphorus atom, or a salt thereof, for the manufacture of a medicament for the treatment or prophylaxis of inflammatory disease, cardiac infarction or myocarditis.

24. Use of a compound according to claim 1 or a salt thereof for the manufacture of a medicament for the treatment or prophylaxis of inflammatory disease, cardiac infarction or myocarditis.

## Patentansprüche

1. Verbindung der Formel: 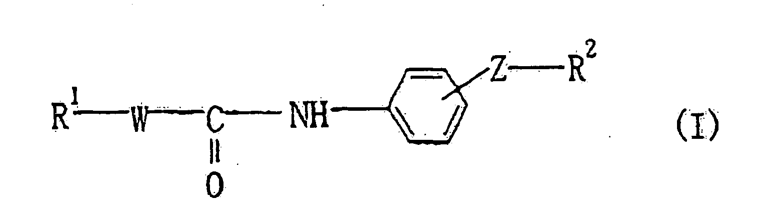 wobei R¹ ein gegebenenfalls substituierter 5- bis 6-gliedriger Ring ist, W eine zweiwertige Gruppe der Formel: 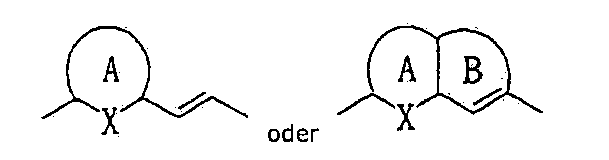 ist, wobei der Ring A ein gegebenenfalls substituierter 5- bis 6-gliedriger aromatischer Ring ist, X ein gegebenenfalls substituiertes Kohlenstoffatom, ein gegebenenfalls substituiertes Stickstoffatom, ein Schwefelatom oder ein Sauerstoffatom ist, der Ring B ein gegebenenfalls substituierter 5- bis 7-gliedriger Ring ist, Z eine Methylengruppe ist, R² (1) eine gegebenenfalls substituierte Aminogruppe, bei der ein Stickstoffatom ein quaternäres Ammonium bilden kann, (2) eine gegebenenfalls substituierte stickstoffhaltige heterocyclische Ringgruppe, die ein Schwefelatom oder ein Sauerstoffatom als ringbildende Atome enthalten kann und wobei ein Stickstoffatom ein quaternäres Ammonium bilden kann, oder (3) eine Gruppe der Formel: 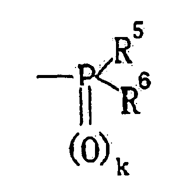 ist, wobei k 0 oder 1 ist, und wenn k 0 ist, ein Phosphoratom ein Phosphonium bilden kann, und R⁵ und R⁶ unabhängig eine gegebenenfalls substituierte Kohlenwasserstoffgruppe oder eine gegebenenfalls substituierte Aminogruppe sind und R⁵ und R⁶ zusammen mit dem benachbarten Phosphoratom unter Bildung einer cyclischen Gruppe miteinander verbunden sein können, oder ein Salz davon.

2. Verbindung nach Anspruch 1, wobei R¹ Benzol, Furan, Thiophen, Pyridin, Cyclopentan, Cyclohexan, Pyrrolidin, Piperidin, Piperazin, Morpholin, Thiomorpholin oder Tetrahydropyran ist, die jeweils substituiert sein können.

3. Verbindung nach Anspruch 1, wobei R¹ ein gegebenenfalls substituiertes Benzol ist.

4. Verbindung nach Anspruch 1, wobei der Ring A Furan, Thiophen, Pyrrol, Pyridin oder Benzol ist, die jeweils substituiert sein können.

5. Verbindung nach Anspruch 1, wobei der Ring A ein gegebenenfalls substituiertes Benzol ist.

6. Verbindung nach Anspruch 1, wobei W eine Gruppe der Formel 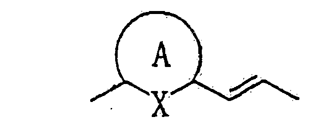 ist, wobei jedes Symbol wie in Anspruch 1 definiert ist.

7. Verbindung nach Anspruch 1, wobei W eine Gruppe der Formel 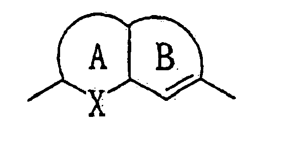 ist, wobei jedes Symbol wie in Anspruch 1 definiert ist.

8. Verbindung nach Anspruch 7, wobei der Ring B eine 5- bis 7-gliedrige Ringgruppe der Formel: 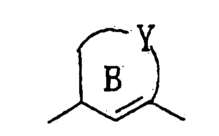 ist, wobei Y -Y'-(CH₂)ₘ- (Y' ist -S-, -O-, -NH- oder -CH₂-, und m ist eine ganze Zahl von 0 - 2), -CH=CH- oder -N=CH- ist, das einen Substituenten in jeder möglichen Position haben kann.

9. Verbindung nach Anspruch 8, wobei Y -Y'-(CH₂)₂- ist (Y' ist -S-, -O-, -NH- oder -CH₂-).

10. Verbindung nach Anspruch 8, wobei Y -(CH₂)₂-, -(CH₂)₃- oder -O-(CH₂)₂- ist.

11. Verbindung nach Anspruch 10, wobei der Ring A ein gegebenenfalls substituiertes Benzol ist.

12. Verbindung nach Anspruch 1, wobei Z in der para-Position des Benzolrings substituiert ist.

13. Verbindung nach Anspruch 1, wobei R² (1) eine gegebenenfalls substituierte Aminogruppe, bei der ein Stickstoffatom ein quaternäres Ammonium bilden kann, (2) eine gegebenenfalls substituierte stickstoffhaltige heterocyclische Ringgruppe, die ein Schwefelatom oder ein Sauerstoffatom als ringbildende Atome enthalten kann und wobei ein Stickstoffatom ein quaternäres Ammonium bilden kann, oder (3) eine Gruppe der Formel: 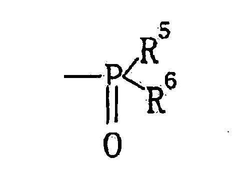 ist, wobei R⁵ und R⁶ unabhängig eine gegebenenfalls substituierte Kohlenwasserstoffgruppe sind und R⁵ und R⁶ zusammen mit dem benachbarten Phosphoratom unter Bildung einer cyclischen Gruppe miteinander verbunden sein können.

14. Verbindung der Formel: 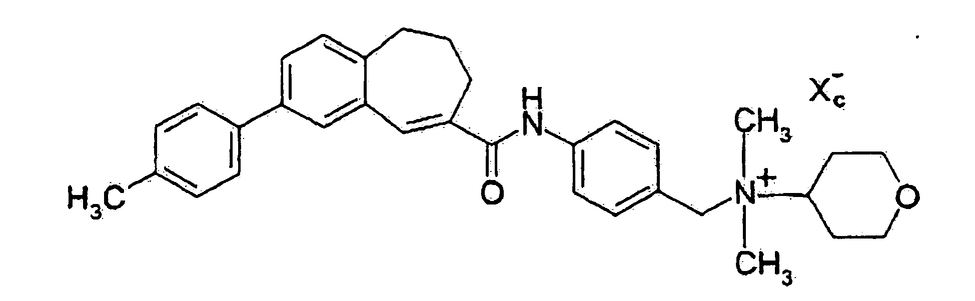 nach Anspruch 1, wobei X_{c}⁻ ein Anion ist.

15. Verbindung nach Anspruch 14, wobei X_{c} ein Halogenatom ist.

16. Verbindung nach Anspruch 1, ausgewählt aus der Klasse bestehend aus
N-Methyl-N-[4-[[[2-(4-methylphenyl)-6,7-dihydro-5H-benzocyclohepten-8-yl]carbonyl]amino]benzyl]piperidiniumiodid,
N-Methyl-N-[4-[[[7-(4-methylphenyl)-2,3-dihydro-1-benzoxepin-4-yl]-carbonyl]amino]benzyl]piperidiniumiodid,
N-[4-[N-Methyl-N-(tetrahydropyran-4-yl)aminomethyl]phenyl]-7-(4-methylphenyl)-2,3-dihydro-1-benzoxepin-4-carboxamid,
N-[4-[N-Methyl-N-(tetrahydropyran-4-yl)aminomethyl]phenyl]-7-(4-morpholinophenyl)-2,3-dihydro-1-benzoxepin-4-carboxamid,
7-(4-Ethoxyphenyl)-N-[4-[N-methyl-N-(tetrahydropyran-4-yl)aminomethyl]phenyl]-2,3-dihydro-1-benzoxepin-4-carboxamid,
N,N-Dimethyl-N-[4-[[[2-(4-methylphenyl)-6,7-dihydro-5H-benzocyclohepten-8-yl]carbonyl]amino]benzyl]-N-(tetrahydropyran-4-yl)-ammoniumiodid und
N-Methyl-N-[4-[[[7-(4-methylphenyl)-3,4-dihydronaphthalin-2-yl]-carbonyl]amino]benzyl]piperidiniumiodid
oder ein Salz davon.

17. Verfahren zur Herstellung einer Verbindung der Formel : 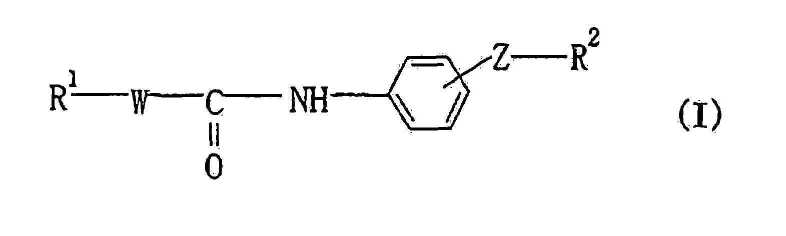 nach Anspruch 1, wobei jedes Symbol wie in Anspruch 1 definiert ist, oder eines Salzes davon, umfassend das Einwirkenlassen einer Verbindung der Formel:
R¹-W-COOH (II)
wobei jedes Symbol wie in Anspruch 1 definiert ist, eines Salzes oder eines reaktiven Derivats davon einer Kondensationsreaktion mit einer Verbindung der Formel: 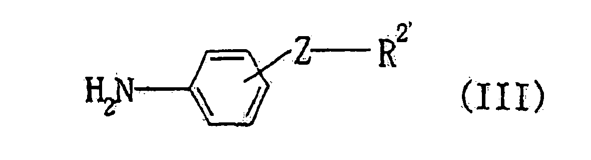 wobei Z wie in Anspruch 1 definiert ist und R²' (1) eine gegebenenfalls substituierte Aminogruppe, bei der ein Stickstoffatom ein quaternäres Ammonium bilden kann, (2) eine gegebenenfalls substituierte stickstoffhaltige heterocyclische Ringgruppe, die ein Schwefelatom oder ein Sauerstoffatom als ringbildende Atome enthalten kann und bei der ein Stickstoffatom ein quaternäres Ammonium bilden kann, oder (3) eine Gruppe der Formel: 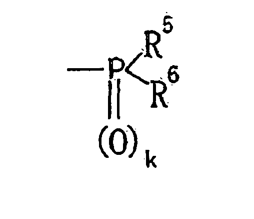 ist, wobei k 0 oder 1 ist, und wenn k 0 ist, ein Phosphoratom ein Phosphonium bilden kann, und R⁵ und R⁶ unabhängig eine gegebenenfalls substituierte Kohlenwasserstoffgruppe oder eine gegebenenfalls substituierte Aminogruppe sind und R⁵ und R⁶ zusammen mit dem benachbarten Phosphoratom unter Bildung einer cyclischen Gruppe miteinander verbunden sein können, wobei die obigen Gruppen (1) - (3) gegebenenfalls geschützt sind, oder einem Salz davon, und bei Bedarf das Einwirkenlassen einer Entfernung von Schutzgruppen, einer Oxidation, Reduktion und/oder Ammonisierung auf das erhaltene Produkt.

18. Verfahren nach Anspruch 17, wobei die Verbindung der Formel R¹-W-COOH 3-(4-Methylphenyl)-8,9-dihydro-7H-benzocyclohepten-6-carbonsäure oder ein Salz davon ist.

19. Pharmazeutische Zusammensetzung, umfassend eine Verbindung nach Anspruch 1 oder ein Salz davon.

20. Verbindung nach Anspruch 19, die zur Antagonisierung des MCP-1-Rezeptors dient.

21. Zusammensetzung nach Anspruch 19, die zur Behandlung oder Prophylaxe eines Herzinfarkts oder einer Myokarditis dient.

22. Pharmazeutische Zusammensetzung zur Antagonisierung des MCP-1-Rezeptors, umfassend eine Verbindung der Formel: 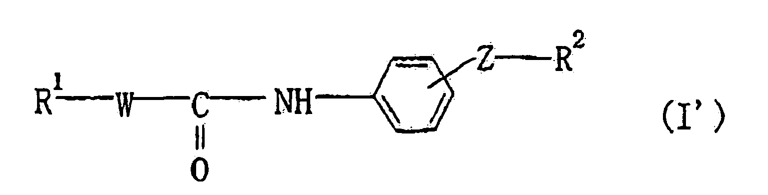 wobei R¹ ein gegebenenfalls substituierter 5- bis 6-gliedriger Ring ist, W eine zweiwertige Gruppe der Formel: 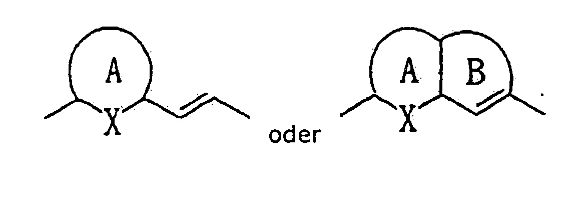 ist, wobei der Ring A ein gegebenenfalls substituierter 5- bis 6-gliedriger aromatischer Ring ist, X ein gegebenenfalls substituiertes Kohlenstoffatom, ein gegebenenfalls substituiertes Stickstoffatom, ein Schwefelatom oder ein Sauerstoffatom ist, der Ring B ein gegebenenfalls substituierter 5- bis 7-gliedriger Ring ist, Z eine Methylengruppe ist, R² (1) eine gegebenenfalls substituierte Aminogruppe, bei der ein Stickstoffatom ein quaternäres Ammonium bilden kann, (2) eine gegebenenfalls substituierte stickstoffhaltige heterocyclische Ringgruppe, die ein Schwefelatom oder ein Sauerstoffatom als ringbildende Atome enthalten kann und wobei ein Stickstoffatom ein quaternäres Ammonium bilden kann, oder (3) eine Gruppe der Formel: 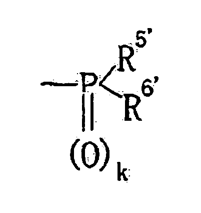 ist, wobei k 0 oder 1 ist, und wenn k 0 ist, ein Phosphoratom ein Phosphonium bilden kann, und R^{5'} und R^{6'} unabhängig eine gegebenenfalls substituierte Kohlenwasserstoffgruppe, eine gegebenenfalls substituierte Hydroxygruppe oder eine gegebenenfalls substituierte Aminogruppe sind und R^{5'} und R^{6'} zusammen mit dem benachbarten Phosphoratom unter Bildung einer cyclischen Gruppe miteinander verbunden sein können, oder ein Salz davon.

23. Verwendung einer Verbindung der Formel: 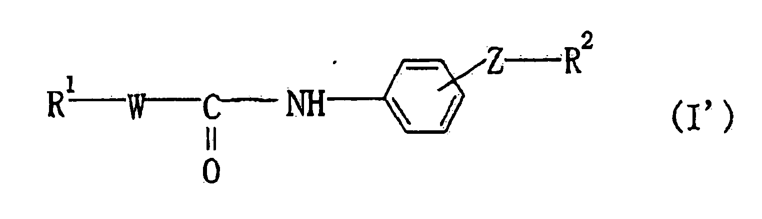 wobei R¹ ein gegebenenfalls substituierter 5- bis 6-gliedriger Ring ist;
W eine zweiwertige Gruppe der Formel: 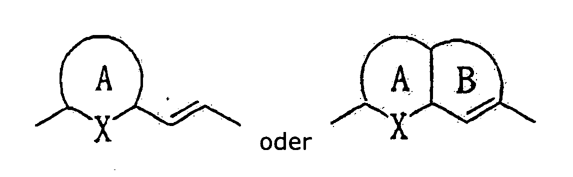 ist, wobei der Ring A ein gegebenenfalls substituierter 5- bis 6-gliedriger aromatischer Ring ist, X ein gegebenenfalls substituiertes Kohlenstoffatom, ein gegebenenfalls substituiertes Stickstoffatom, ein Schwefelatom oder ein Sauerstoffatom ist, der Ring B ein gegebenenfalls substituierter 5- bis 7-gliedriger Ring ist, Z eine Methylengruppe ist, R² (1) eine gegebenenfalls substituierte Aminogruppe, bei der ein Stickstoffatom ein quaternäres Ammonium bilden kann, (2) eine gegebenenfalls substituierte stickstoffhaltige heterocyclische Ringgruppe, die ein Schwefelatom oder ein Sauerstoffatom als ringbildende Atome enthalten kann und wobei ein Stickstoffatom ein quaternäres Ammonium bilden kann, oder (3) eine Gruppe der Formel: 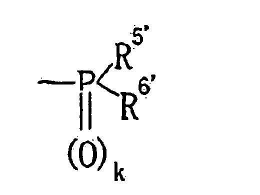 ist, wobei k 0 oder 1 ist, und wenn k 0 ist, ein Phosphoratom ein Phosphonium bilden kann, und R^{5'} und R^{6'} unabhängig eine gegebenenfalls substituierte Kohlenwasserstoffgruppe, eine gegebenenfalls substituierte Hydroxygruppe oder eine gegebenenfalls substituierte Aminogruppe sind und R^{5'} und R^{6'} zusammen mit dem benachbarten Phosphoratom unter Bildung einer cyclischen Gruppe miteinander verbunden sein können, oder eines Salzes davon zur Herstellung eines Medikaments zur Behandlung oder Prophylaxe einer Entzündungskrankheit, eines Herzinfarkts oder einer Myokarditis.

24. Verwendung einer Verbindung nach Anspruch 1 oder eines Salzes davon zur Herstellung eines Medikaments zur Behandlung oder Prophylaxe einer Entzündungskrankheit, eines Herzinfarkts oder einer Myokarditis.

## Revendications

1. Composé de formule : 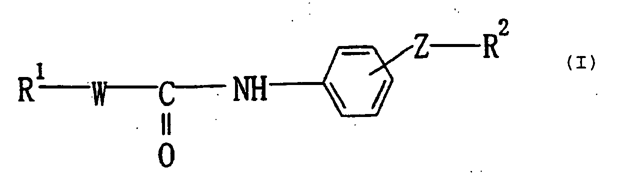 dans laquelle R¹ représente un cycle de 5 à 6 chaînons, éventuellement substitué, w représente un groupe divalent de formule : 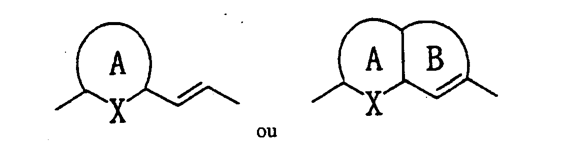 dans laquelle le cycle A est un cycle aromatique de 5 à 6 chaînons, éventuellement substitué, X représente un atome de carbone éventuellement substitué, un atome d'oxygène, un atome de soufre ou un atome d'azote éventuellement substitué, le cycle B représente un cycle de 5 à 7 chaînons, éventuellement substitué, Z représente un groupe méthylène, R² est (1) un groupe amino éventuellement substitué dans lequel un atome d'azote peut former un ammonium quaternaire, (2) un groupe hétérocyclique azoté éventuellement substitué qui peut contenir un atome de soufre ou un atome d'oxygène à titre d'atome constituant le cycle et dans lequel un atome d'azote peut former un ammonium quaternaire, ou (3) un groupe de formule 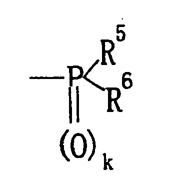 dans laquelle k vaut 0 ou 1, et lorsque k vaut 0, un atome de phosphore peut former un phosphonium ; et R⁵ et R⁶ représentent indépendamment un groupe hydrocarboné éventuellement substitué ou un groupe amino éventuellement substitué, et R⁵ et R⁶ peuvent être liés l'un à l'autre pour former un groupe cyclique conjointement avec l'atome de phosphore adjacent, ou un sel d'un tel composé.

2. Composé selon la revendication 1, dans lequel R¹ représente un groupe benzène, furanne, thiophène, pyridine, cyclopentane, cyclohexane, pyrrolidine, pipéridine, pipérazine, morpholine, thiomorpholine ou tétrahydropyranne, chacun d'eux pouvant être substitué.

3. Composé selon la revendication 1, dans lequel R¹ représente un groupe benzène éventuellement substitué.

4. Composé selon la revendication 1, dans lequel le cycle A est un groupe furanne, thiophène, pyrrole, pyridine ou benzène, chacun d'eux pouvant être substitué.

5. Composé selon la revendication 1, dans lequel le cycle A est un groupe benzène éventuellement substitué.

6. Composé selon la revendication 1, dans lequel W est un groupe de formule : 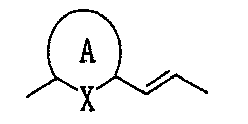 dans laquelle chaque symbole est tel que défini dans la revendication 1.

7. Composé selon la revendication 1, dans lequel W est un groupe de formule : 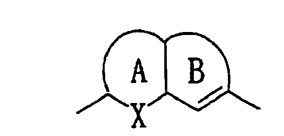 dans laquelle chaque symbole est tel que défini dans la revendication 1.

8. Composé selon la revendication 7, dans lequel le cycle B est un groupe cyclique de 5 à 7 chaînons de formule : 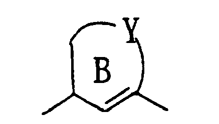 dans laquelle Y représente -Y'-(CH₂)ₘ- (Y' est -S-, -O-, -NH- ou -CH₂- et m est un nombre entier valant 0-2), -CH=CH- ou -N=CH-, qui peut porter un substituant en n'importe quelle position possible.

9. Composé selon la revendication 8, dans lequel Y représente -Y'-(CH₂)₂- (Y' est -S-, -O-, -NH- ou -CH₂-).

10. Composé selon la revendication 8, dans lequel Y est -(CH₂)₂-, -(CH₂)₃- ou -O-(CH₂)₂-.

11. Composé selon la revendication 10, dans lequel le cycle A est un benzène éventuellement substitué.

12. Composé selon la revendication 1, dans lequel Z porte un substituant en position para du cycle benzènique.

13. Composé selon la revendication 1, dans lequel R² est (1) un groupe amino éventuellement substitué dans lequel un atome d'azote peut former un ammonium quaternaire, (2) un groupe hétérocyclique azoté éventuellement substitué qui peut contenir un atome de soufre ou un atome d'oxygène à titre d'atome constitutif du cycle et dans lequel un atome d'azote peut former un ammonium quaternaire, ou (3) un groupe de formule : 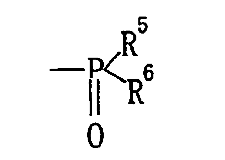 dans laquelle R⁵ et R⁶ représentent indépendamment un groupe hydrocarboné éventuellement substitué, et R⁵ et R⁶ peuvent être liés l'un à l'autre pour former un groupe cyclique conjointement avec l'atome de phosphore adjacent.

14. Composé de formule : 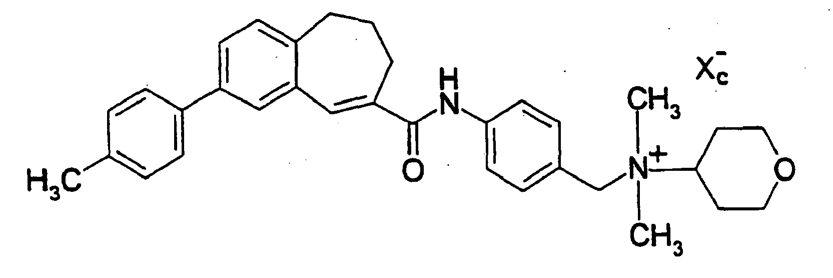 selon la revendication 1, dans laquelle X_{c}⁻ est un anion.

15. Composé selon la revendication 14, dans lequel X_{c} représente un atome d'halogène.

16. Composé selon la revendication 1, choisi dans l'ensemble comprenant :
l'iodure de N-méthyl-N-[4-[[[2-(4-méthylphényl)-6,7-dihydro-5H-benzo-cycloheptèn-8-yl]carbonyl]amino]benzyl]pipéridinium,
l'iodure de N-méthyl-N-[4-[[[7-(4-méthylphényl)-2,3-dihydro-1-benzo-xépin-4-yl]carbonyl]amino]benzyl]pipéridinium,
le N-[4-[N-méthyl-N-[tétrahydropyran-4-yl]aminométhyl]phényl]-7-(4-méthylphényl)-2,3-dihydro-1-benzoxépine-4-carboxamide,
le N-[4-[N-méthyl-N-[tétrahydropyran-4-yl]aminométhyl]phényl] -7-(4-morpholinophényl)-2,3-dihydro-1-benzoxépine-4-carboxamide,
le 7-(4-éthoxyphényl)-N-[4-[N-méthyl-N-(tétrahydropyran-4-yl)aminométhyl]phényl]-2,3-dihydro-1-benzoxépine-carboxamide,
l'iodure de N,N-diméthyl-N-[4-[[[2-(4-méthylphényl)-6,7-dihydro-5H-benzocycloheptèn-8-yl]carbonyl]amino]benzyl]-N-(tétrahydropyran-4-yl] ammonium, et
l'iodure de N-méthyl-N-[4-[[[7-(4-méthylphényl)-3,4-dihydronaphtalèn-2-yl]carbonyl] amino] benzyl] pipéridinium,
ou un sel d'un tel composé.

17. Procédé pour la production d'un composé de formule : 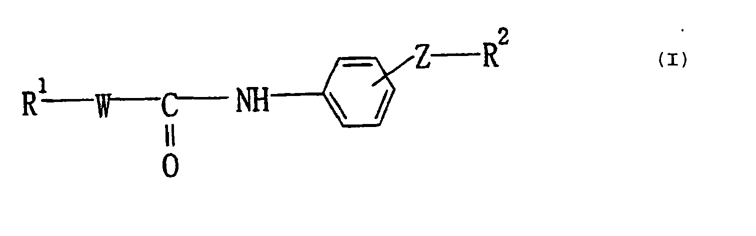 selon la revendication 1, formule dans laquelle chaque symbole est tel que défini dans la revendication 1, ou d'un de ses sels, qui comprend le fait de soumettre un composé de formule
R¹-W-COOH (II)
dans laquelle chaque symbole est tel que défini dans la revendication 1, ou un sel ou un dérivé réactif d'un tel composé, à une réaction de condensation avec un composé de formule : 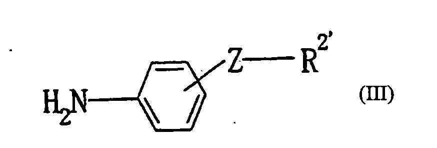 dans laquelle Z est tel que défini dans la revendication 1, et R²' est (1) un groupe amino éventuellement substitué dans lequel un atome d'azote peut former un ammonium quaternaire, (2) un groupe hétérocyclique azoté, éventuellement substitué qui peut contenir un atome de soufre ou un atome d'oxygène à titre d'atome constitutif du noyau et dans lequel un atome d'azote peut former un ammonium quaternaire, ou (3) un groupe de formule : 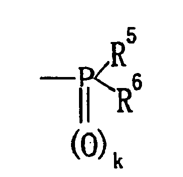 dans laquelle k vaut O ou 1, et lorsque k vaut 0, un atome de phosphore peut former un phosphonium ; et R⁵ et R⁶ représentent indépendamment un groupe hydrocarboné éventuellement substitué ou un groupe amino éventuellement substitué, et R⁵ et R⁶ peuvent être liés l'un à l'autre pour former un groupe cyclique conjointement avec l'atome de phosphore adjacent, les groupes (1) - (3) ci-dessus étant éventuellement protégés, ou un sel d'un tel composé, et si nécessaire, de soumettre le produit obtenu à une déprotection, une oxydation, une réduction et/ou une transformation en ammonium.

18. Procédé selon la revendication 17, dans lequel le composé de formule R¹-W-COOH est l'acide 3-(4-méthylphényl)-8,9-dihydro-7H-benzocyclo-heptène-6-carboxylique ou un de ses sels.

19. Composition pharmaceutique qui comprend un composé selon la revendication 1 ou un de ses sels.

20. Composition selon la revendication 19, qui est un antagoniste du récepteur de MCP-1.

21. Composition selon la revendication 19, qui concerne le traitement ou la prophylaxie de l'infarctus du myocarde ou de la myocardite.

22. Composition pharmaceutique pour s'opposer au récepteur de MCP-1, qui comprend un composé de formule : 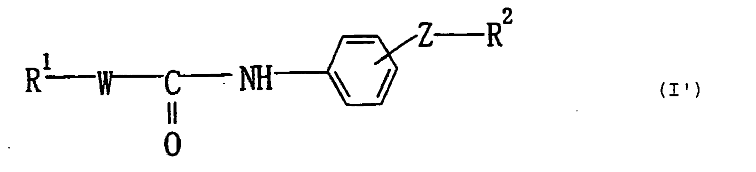 dans laquelle R¹ représente un cycle de 5 à 6 chaînons, éventuellement substitué, W représente un groupe divalent de formule : 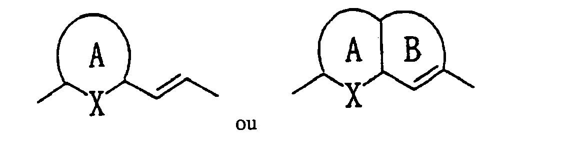 dans laquelle le cycle A est un cycle aromatique de 5 à 6 chaînons, éventuellement substitué, X représente un atome de carbone éventuellement substitué, un atome d'oxygène, un atome de soufre ou un atome d'azote éventuellement substitué, le cycle B représente un cycle de 5 à 7 chaînons, éventuellement substitué, Z représente un groupe méthylène, R² est (1) un groupe amino éventuellement substitué dans lequel un atome d'azote peut former un ammonium quaternaire, (2) un groupe hétérocyclique azoté éventuellement substitué qui peut contenir un atome de soufre ou un atome d'oxygène à titre d'atome constituant le cycle et dans lequel un atome d'azote peut former un ammonium quaternaire, ou (3) un groupe de formule 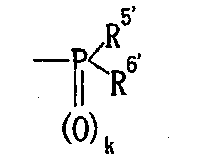 dans laquelle k vaut 0 ou 1, et lorsque k vaut 0, un atome de phosphore peut former un phosphonium ; et R⁵' et R⁶' représentent indépendamment un groupe hydrocarboné éventuellement substitué, un groupe hydroxy éventuellement substitué ou un groupe amino éventuellement substitué, et R⁵' et R⁶' peuvent être liés l'un à l'autre pour former un groupe cyclique conjointement avec l'atome de phosphore adjacent, ou un sel d'un tel composé.

23. Utilisation d'un composé de formule 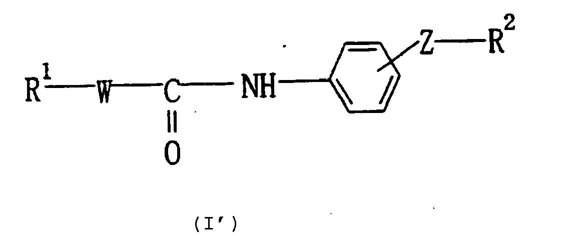 dans laquelle R¹ représente un cycle de 5 à 6 chaînons, éventuellement substitué, W représente un groupe divalent de formule : 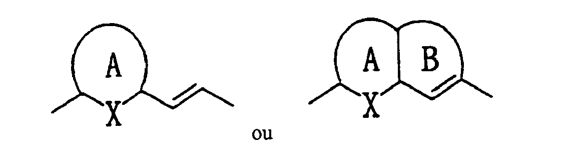 dans laquelle le cycle A est un cycle aromatique de 5 à 6 chaînons, éventuellement substitué, X représente un atome de carbone éventuellement substitué, un atome d'oxygène, un atome de soufre ou un atome d'azote éventuellement substitué, le cycle B représente un cycle de 5 à 7 chaînons, éventuellement substitué, Z représente un groupe méthylène, R² est (1) un groupe amino éventuellement substitué dans lequel un atome d'azote peut former un ammonium quaternaire, (2) un groupe hétérocyclique azoté éventuellement substitué qui peut contenir un atome de soufre ou un atome d'oxygène à titre d'atome constituant le cycle et dans lequel un atome d'azote peut former un ammonium quaternaire, ou (3) un groupe de formule 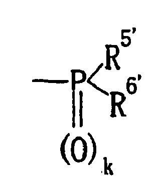 dans laquelle k vaut 0 ou 1, et lorsque k vaut 0, un atome de phosphore peut former un phosphonium ; et R^{5'} et R^{6'} représentent indépendamment un groupe hydrocarboné éventuellement substitué, un groupe hydroxy éventuellement substitué ou un groupe amino éventuellement substitué, et R⁵' et R⁶' peuvent être liés l'un à l'autre pour former un groupe cyclique conjointement avec l'atome de phosphore adjacent, ou d'un de ses sels, pour la fabrication d'un médicament pour le traitement ou la prophylaxie d'une maladie inflammatoire, de l'infarctus du myocarde ou de la myocardite.

24. Utilisation d'un composé selon la revendication 1 ou d'un de ses sels, pour la fabrication d'un médicament pour le traitement ou la prophylaxie d'une maladie inflammatoire, d'un infarctus du myocarde ou d'une myocardite.
